# EUROPEAN PATENT APPLICATION

(11) **EP 4 711 365 A1**
(43) Date of publication of application: **18.03.2026**
(21) Application number: 24806556.7
(22) Date of filing: 13.05.2024
(51) Int. Cl.: C07D 471/04, C07D 417/14, A61K 31/437, A61K 31/513, A61K 31/519, C07D 417/06, A61P 37/00

(54) **THIAZOLE DERIVATIVE INHIBITOR, AND PREPARATION METHOD THEREFOR AND USE THEREOF**

(30) Priority: 12.05.2023 CN 202310541987; 29.05.2023 CN 202310619175; 28.06.2023 CN 202310782590; 13.07.2023 CN 202310863775; 20.07.2023 CN 202310902187; 23.08.2023 CN 202311070938; 19.10.2023 CN 202311362376; 09.11.2023 CN 202311495885; 01.12.2023 CN 202311643127; 25.01.2024 CN 202410110620; 28.02.2024 CN 202410225909
(71) Applicant: Shanghai Hansoh Biomedical Co., Ltd., Shanghai 201203 (CN); Jiangsu Hansoh Pharmaceutical Group Co., Ltd., Lianyungang, Jiangsu 222069 (CN)
(72) Inventor: GAO, Peng, Shanghai 201203 (CN); CHENG, Yu, Shanghai 201203 (CN); SUN, Guangjun, Shanghai 201203 (CN); ZHANG, Chao, Shanghai 201203 (CN); YU, Wensheng, Shanghai 201203 (CN)
(74) Representative: De Clercq & Partners
(86) International application number: PCT/CN2024/092928
(87) International publication number: WO 2024/235205

(57) **Abstract**

The present invention relates to a thiazole derivative inhibitor, and a preparation method therefor and the use thereof. Specifically, the present invention relates to a compound, a preparation method therefor, a pharmaceutical composition containing the compound, and the use thereof as an inhibitor in the treatment of autoimmune diseases.

## Description

**The present application claims the right of priority for**
application number: CN 2023105419877, filed on May 12, 2023;
application number: CN 202310619175X, filed on May 29, 2023;
application number: CN 2023107825907, filed on June 28, 2023;
application number: CN 2023108637750, filed on July 13, 2023;
application number: CN 2023109021873, filed on July 20, 2023;
application number: CN 2023110709386, filed on August 23, 2023;
application number: CN 2023113623762, filed on October 19, 2023;
application number: CN 2023114958852, filed on November 9, 2023;
application number: CN 2023116431270, filed on December 1, 2023;
application number: CN 2024101106204, filed on January 25, 2024;
application number: CN 2024102259090, filed on February 28, 2024.

### Technical Field

The present invention belongs to the field of drug synthesis and specifically relates to a thiazole derivative inhibitor, and a preparation method therefor and the use thereof.

### Background Art

Human IL-17 (Interleukin 17) is a major class of proinflammatory cytokines. Human IL-17 comprises six family members: IL-17A (also known as CTLA-8), IL-17B, IL-17C, IL-17D, IL-17E (also known as IL-25) and IL-17F. The human IL-17 receptor family consists of five members: IL-17RA, IL-17RB, IL-17RC, IL-17RD and IL-17RE. Human IL-17 initiates downstream signalling pathways by binding to heterodimeric IL-17 receptors.

IL-17A is the most extensively studied cytokine. IL-17A is produced by a variety of cells, such as Th17 (T helper 17 cells), Tc17 (T cytotoxic 17 cells), NKT (natural killer T cells), γδT (γδT cells) and epithelial cells. IL-17 activates Act1 (TRAF3 interacting protein 2)-dependent MAPK and NF-κb signalling pathways by binding to IL-17 receptors on skin cells, etc., which upregulates the expression of various cytokines (such as IL-6, TNF-α, G-CSF and GM-CSF), chemokines (such as CXCL1, CXCL2, CXCL5, CXCL8 and CXCL10), antimicrobial peptides (such as Defensin, MUCSAC and S100A7) and matrix metalloproteinases (e.g., MMP1) at the transcriptional level. IL-17A is involved in regulating many important biological processes, such as bacterial and fungal infections, wound repair and the promotion of inflammation.

The IL-17A pathway also plays an important role in autoimmune/inflammatory diseases. Preclinical studies have shown that IL-23 or IL-17RA knockout mice are resistant to imiquimod (IMQ)-induced psoriasis, and IL-17 monoclonal antibodies can effectively inhibit the psoriasis-related scores induced by IMQ or IL-23. IL-17-related therapeutic antibodies (Secukinumab, Ixekizumab, Brodalumab, Bimekizumab, etc.) have also been approved for the treatment of diseases such as psoriasis and ankylosing spondylitis, demonstrating excellent clinical efficacy and mild and controllable toxic side effects.

Nevertheless, IL-17-related therapeutic antibodies are associated with high costs and require administration via subcutaneous or intravenous injection, making them less accessible and convenient for patients with autoimmune/inflammatory diseases who require long-term medication. The development of highly active oral IL-17 small-molecule inhibitors can greatly improve the accessibility and convenience of medication for patients, offering considerable clinical and commercial values.

### Summary of the Invention

An object of the present invention is to provide a compound as represented by formula (I-A), or a stereoisomer or pharmaceutically acceptable salt thereof: 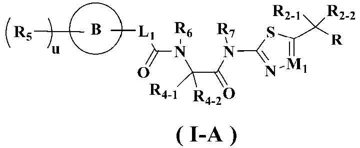
wherein: M₁ is selected from N or CR₃;
L₁ is selected from a bond, -O-, -NH-, -CO-, -CONH-, C₁₋₃ alkylene, C₂₋₄ alkenylene, C₂₋₄ alkynylene, -O-C₁₋₃ alkylene-, -S-C₁₋₃ alkylene-, -C₁₋₃ alkylene-NH-, -C₁₋₃ alkylene-C(O)NH-, -C₁₋₃ alkylene-NHC(O)-, -C₁₋₃ alkylene-S(O)ᵣ₁-, -C₁₋₃ alkylene-NHS(O)ᵣ₂- or -C₁₋₃ alkylene-S(O)ᵣ₃NH-; the -NH-, -CONH-, C₁₋₃ alkylene, C₂₋₄ alkenylene, C₂₋₄ alkynylene, -O-C₁₋₃ alkylene-, -S-C₁₋₃ alkylene-, -C₁₋₃ alkylene-NH-, -C₁₋₃ alkylene-C(O)NH-, -C₁₋₃ alkylene-NHC(O)-, -C₁₋₃ alkylene-S(O)ᵣ₁-, -C₁₋₃ alkylene-NHS(O)ᵣ₂- or -C₁₋₃ alkylene-S(O)ᵣ₃NH- is optionally substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl; a bond is preferred;
ring B is selected from C₃₋₁₂ cycloalkyl, 3- to 12-membered heterocyclyl, C₆₋₁₄ aryl or 5- to 14-membered heteroaryl; or ring B is absent;
R is selected from C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₁₂ cycloalkyl, 3- to 12-membered heterocyclyl, C₆₋₁₄ aryl or 5- to 14-membered heteroaryl, wherein the C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₁₂ cycloalkyl, 3- to 12-membered heterocyclyl, C₆₋₁₄ aryl or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₁₂ cycloalkyl, 3- to 12-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₁₂ cycloalkyl, 3- to 12-membered heterocyclyl, C₆₋₁₀ aryl or 5- to 10-membered heteroaryl may optionally be further substituted with one or more of hydrogen, deuterium, halogen, amino, hydroxyl, cyano, nitro, carboxyl, oxo, thio, =CF₂, =CHF, =NOC₁₋₆ alkyl, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₁₂ cycloalkyl, 3- to 12-membered heterocyclyl, C₆₋₁₀ aryl and 5- to 10-membered heteroaryl; the C₃₋₁₂ cycloalkyl, 3- to 12-membered heterocyclyl, C₆₋₁₀ aryl and 5- to 10-membered heteroaryl substituents are optionally further substituted with one or more of hydrogen, deuterium, halogen, amino, hydroxyl, cyano, nitro, carboxyl, oxo, thio, =CF₂, =CHF, =NOC₁₋₆ alkyl, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ heteroalkyl, C₁₋₆ deuteroheteroalkyl, C₁₋₆ haloheteroalkyl, C₁₋₆ hydroxyalkyl, C₃₋₈ cycloalkyl, 3- to 8-membered heterocyclyl, C₆₋₁₀ aryl and 5- to 10-membered heteroaryl; the C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ heteroalkyl, C₁₋₆ deuteroheteroalkyl, C₁₋₆ haloheteroalkyl, C₁₋₆ hydroxyalkyl, C₃₋₈ cycloalkyl, 3- to 8-membered heterocyclyl, C₆₋₁₀ aryl and 5- to 10-membered heteroaryl are optionally further substituted with one or more of hydrogen, deuterium, halogen, amino, hydroxyl, cyano, nitro, carboxyl, oxo, thio, =CF₂, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ heteroalkyl, C₁₋₆ deuteroheteroalkyl, C₁₋₆ haloheteroalkyl, C₁₋₆ hydroxyalkyl, C₃₋₈ cycloalkyl, 3- to 8-membered heterocyclyl, C₆₋₁₀ aryl and 5- to 10-membered heteroaryl; preferably, R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₙ₁OR_{b1}, -(CH₂)ₙ₂C(O)R_{b2}, -(CH₂)ₙ₃NHC(O)R_{b3}, - (CH₂)ₙ₄C(O)NHR_{b4}, -(CH₂)ₙ₅NR_{b5}R_{b6}, -(CH₂)ₙ₆S(O)ₙ₇R_{b7}, -(CH₂)ₙ₈S(O)₂NHR_{b8} or - (CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, carboxyl, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl, wherein the C₃₋₈ cycloalkyl, 3- to 8-membered heterocyclyl, C₆₋₁₀ aryl and 5- to 10-membered heteroaryl substituents are optionally further substituted with one or more of hydrogen, deuterium, halogen, amino, hydroxyl, cyano, nitro, carboxyl, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
preferably, R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, carboxyl, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
preferably, R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
alternatively, R₂₋₁ and R₂₋₂ are connected to form C₃₋₈ cycloalkyl, 3- to 8-membered heterocyclyl, C₆₋₁₀ aryl or 5- to 10-membered heteroaryl, wherein the C₃₋₈ cycloalkyl, 3- to 8-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
alternatively, R and R₂₋₂ are connected to form C₃₋₈ cycloalkyl, 3- to 8-membered heterocyclyl, C₆₋₁₀ aryl or 5- to 10-membered heteroaryl, wherein the C₃₋₈ cycloalkyl, 3- to 8-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₃ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, - (CH₂)ₒ₁OR_{c1}, -(CH₂)ₒ₂C(O)R_{c2}, -(CH₂)ₒ₃NHC(O)R_{c3}, -(CH₂)ₒ₄C(O)NHR_{c4}, - (CH₂)ₒ₅NR_{c5}R_{c6}, -(CH₂)ₒ₆S(O)ₒ₇R_{c7}, -(CH₂)ₒ₈S(O)₂NHR_{c8} or -(CH₂)ₒ₉NHS(O)₂R_{c9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₄₋₁ and R₄₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CHR_{d10})ₚ₁OR_{d1}, -(CHR_{d10})ₚ₂C(O)R_{d2}, - (CHR_{d10})ₚ₃NHC(O)R_{d3}, -(CHR_{d10})ₚ₄C(O)NHR_{d4}, -(CHR_{d10})ₚ₅NR_{d5}R_{d6}, - (CHR_{d10})ₚ₆S(O)ₚ₇R_{d7}, -(CHR_{d10})ₚ₈S(O)₂NHR_{d8} or -(CHR_{d10})ₚ₉NHS(O)₂R_{d9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
preferably, R₄₋₁ and R₄₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₚ₁OR_{d1}, -(CH₂)ₚ₂C(O)R_{d2}, - (CH₂)ₚ₃NHC(O)R_{d3}, -(CH₂)ₚ₄C(O)NHR_{d4}, -(CH₂)ₚ₅NR_{d5}R_{d6}, -(CH₂)ₚ₆S(O)ₚ₇P_{d7}, - (CH₂)ₚ₈S(O)₂NHR_{d8} or -(CH₂)ₚ₉NHS(O)₂R_{d9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
alternatively, R₄₋₁ and R₄₋₂ are connected to form C₃₋₈ cycloalkyl, 3- to 8-membered heterocyclyl, C₆₋₁₀ aryl or 5- to 10-membered heteroaryl, wherein the C₃₋₈ cycloalkyl, 3- to 8-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₅ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, - (CH₂)_{q1}ORₑ₁, -(CH₂)_{q2}C(O)Rₑ₂, -(CH₂)_{q3}NHC(O)Rₑ₃, -(CH₂)_{q4}C(O)NHRₑ₄, - (CH₂)_{q5}NRₑ₅Rₑ₆, -(CH₂)_{q6}S(O)_{q7}Rₑ₇, -(CH₂)_{q8}S(O)₂NHRₑ₈ or -(CH₂)_{q9}NHS(O)₂Rₑ₉, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₆ and R₇ are each independently selected from hydrogen, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₁₂ cycloalkyl, 3- to 12-membered heterocyclyl, C₆₋₁₄ aryl or 5- to 14-membered heteroaryl, wherein the C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₁₂ cycloalkyl, 3- to 12-membered heterocyclyl, C₆₋₁₄ aryl or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
Rₐ₁, Rₐ₂, Rₐ₃, Rₐ₄, Rₐ₅, Rₐ₆, Rₐ₇, Rₐ₈ and Rₐ₉ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{b1}, R_{b2}, R_{b3}, R_{b4}, R_{b5}, R_{b6}, R_{b7}, R_{b8} and R_{b9} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{c1}, R_{c2}, R_{c3}, R_{c4}, R_{c5}, R_{c6}, R_{c7}, R_{c8} and R_{c9} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{d1}, R_{d2}, R_{d3}, R_{d4}, R_{d5}, R_{d6}, R_{d7}, R_{d8}, R_{d9} and R_{d10} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
Rₑ₁, Rₑ₂, Rₑ₃, Rₑ₄, Rₑ₅, Rₑ₆, Rₑ₇, Rₑ₈ and Rₑ₉ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
u is 0, 1, 2, 3, 4 or 5;
m1, m2, m3, m4, m5, m6, m8 and m9 are each independently selected from 0, 1, 2, 3, 4, 5 or 6;
n1, n2, n3, n4, n5, n6, n8 and n9 are each independently selected from 0, 1, 2, 3, 4, 5 or 6;
o1, o2, o3, o4, o5, o6, o8 and o9 are each independently selected from 0, 1, 2, 3, 4, 5 or 6;
p1, p2, p3, p4, p5, p6, p8 and p9 are each independently selected from 0, 1, 2, 3, 4, 5 or 6;
q1, q2, q3, q4, q5, q6, q8 and q9 are each independently selected from 0, 1, 2, 3, 4, 5 or 6;
m7, n7, o7, p7 and q7 are each independently selected from 0, 1, 2 or 3;
r1, r2 and r3 are each independently selected from 0, 1 or 2.

In certain embodiments of the present invention, the C₃₋₈ cycloalkyl, 3- to 8-membered heterocyclyl, C₆₋₁₀ aryl and 5- to 10-membered heteroaryl substituents described for each group may optionally be further substituted with one or more of hydrogen, deuterium, halogen, amino, hydroxyl, cyano, nitro, carboxyl, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl.

In certain embodiments of the present invention, R is

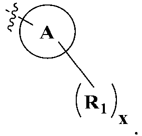

In certain embodiments of the present invention, the compound is as represented by formula (I-A-1) 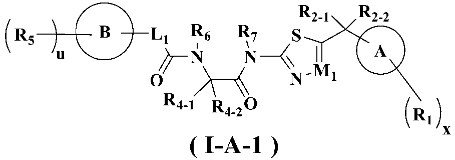
wherein ring A is selected from C₃₋₁₂ cycloalkyl, 3- to 12-membered heterocyclyl, C₆₋₁₄ aryl or 5- to 14-membered heteroaryl;
R₁ is independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₘ₁ORₐ₁, -(CH₂)ₘ₂C(O)Rₐ₂, -(CH₂)ₘ₃NHC(O)Rₐ₃, - (CH₂)ₘ₄C(O)NHRₐ₄, -(CH₂)ₘ₅NRₐ₅Rₐ₆, -(CH₂)ₘ₆S(O)ₘ₇Rₐ₇, -(CH₂)ₘ₈S(O)₂NHRₐ₈ or -(CH₂)ₘ₉NHS(O)₂Rₐ₉, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5-to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl; x is 0, 1, 2, 3, 4 or 5.

In certain embodiments of the present invention, the compound as represented by formula (I-A) is as represented by formula (I): 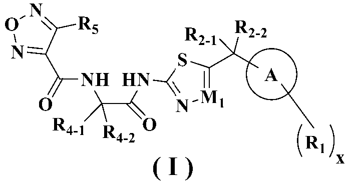
wherein ring A is selected from C₃₋₁₂ cycloalkyl, 3- to 12-membered heterocyclyl, C₆₋₁₄ aryl or 5- to 14-membered heteroaryl;
R₁ is independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₘ₁ORₐ₁, -(CH₂)ₘ₂C(O)Rₐ₂, -(CH₂)ₘ₃NHC(O)Rₐ₃, - (CH₂)ₘ₄C(O)NHRₐ₄, -(CH₂)ₘ₅NRₐ₅Rₐ₆, -(CH₂)ₘ₆S(O)ₘ₇Rₐ₇, -(CH₂)ₘ₈S(O)₂NHRₐ₈ or -(CH₂)ₘ₉NHS(O)₂Rₐ₉, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5-to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, carboxyl, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
preferably, R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
alternatively, R₂₋₁ and R₂₋₂ are connected to form C₃₋₈ cycloalkyl, 3- to 8-membered heterocyclyl, C₆₋₁₀ aryl or 5- to 10-membered heteroaryl, wherein the C₃₋₈ cycloalkyl, 3- to 8-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₃ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, - (CH₂)ₒ₁OR_{c1}, -(CH₂)ₒ₂C(O)R_{c2}, -(CH₂)ₒ₃NHC(O)R_{c3}, -(CH₂)ₒ₄C(O)NHR_{c4}, - (CH₂)ₒ₅NR_{c5}R_{c6}, -(CH₂)ₒ₆S(O)ₒ₇R_{c7}, -(CH₂)ₒ₈S(O)₂NHR_{c8} or -(CH₂)ₒ₉NHS(O)₂R_{c9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₄₋₁ and R₄₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CHR_{d10})ₚ₁OR_{d1}, -(CHR_{d10})ₚ₂C(O)R_{d2}, - (CHR_{d10})ₚ₃NHC(O)R_{d3}, -(CHR_{d10})ₚ₄C(O)NHR_{d4}, -(CHR_{d10})ₚ₅NR_{d5}R_{d6}, - (CHR_{d10})ₚ₆S(O)ₚ₇R_{d7}, -(CHR_{d10})ₚ₈S(O)₂NHR_{d8} or (CHR_{d10})ₚ₉NHS(O)₂R_{d9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
preferably, R₄₋₁ and R₄₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₚ₁OR_{d1}, -(CH₂)ₚ₂C(O)R_{d2}, - (CH₂)ₚ₃NHC(O)R_{d3}, -(CH₂)ₚ₄C(O)NHR_{d4}, -(CH₂)ₚ₅NR_{d5}R_{d6}, -(CH₂)ₚ₆S(O)ₚ₇P_{d7}, - (CH₂)ₚ₈S(O)₂NHR_{d8} or -(CH₂)ₚ₉NHS(O)₂R_{d9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
alternatively, R₄₋₁ and R₄₋₂ are connected to form C₃₋₈ cycloalkyl, 3- to 8-membered heterocyclyl, C₆₋₁₀ aryl or 5- to 10-membered heteroaryl, wherein the C₃₋₈ cycloalkyl, 3- to 8-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₅ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, - (CH₂)_{q1}ORₑ₁, -(CH₂)_{q2}C(O)Rₑ₂, -(CH₂)_{q3}NHC(O)Rₑ₃, -(CH₂)_{q4}C(O)NHRₑ₄, - (CH₂)_{q5}NRₑ₅Rₑ₆, -(CH₂)_{q6}S(O)_{q7}Rₑ₇, -(CH₂)_{q8}S(O)₂NHRₑ₈ or -(CH₂)_{q9}NHS(O)₂Rₑ₉, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
Rₐ₁, Rₐ₂, Rₐ₃, Rₐ₄, Rₐ₅, Rₐ₆, Rₐ₇, Rₐ₈ and Rₐ₉ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{b1}, R_{b2}, R_{b3}, R_{b4}, R_{b5}, R_{b6}, R_{b7}, R_{b8} and R_{b9} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{c1}, R_{c2}, R_{c3}, R_{c4}, R_{c5}, R_{c6}, R_{c7}, R_{c8} and R_{c9} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{d1}, R_{d2}, R_{d3}, R_{d4}, Rₐ₅, R_{d6}, R_{d7}, R_{d8}, R_{d9} and R_{d10} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
Rₑ₁, R_{c2}, Rₑ₃, Rₑ₄, R_{c5}, Rₑ₆, R_{c7}, Rₑ₈ and Rₑ₉ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
x is 0, 1, 2, 3, 4 or 5;
m1, m2, m3, m4, m5, m6, m8 and m9 are each independently selected from 0, 1, 2, 3, 4, 5 or 6;
n1, n2, n3, n4, n5, n6, n8 and n9 are each independently selected from 0, 1, 2, 3, 4, 5 or 6;
o1, o2, o3, o4, o5, o6, o8 and o9 are each independently selected from 0, 1, 2, 3, 4, 5 or 6;
p1, p2, p3, p4, p5, p6, p8 and p9 are each independently selected from 0, 1, 2, 3, 4, 5 or 6;
q1, q2, q3, q4, q5, q6, q8 and q9 are each independently selected from 0, 1, 2, 3, 4, 5 or 6;
m7, n7, o7, p7 and q7 are each independently selected from 0, 1, 2 or 3.

In certain embodiments of the present invention, the compound as represented by formula (I) is as represented by formula (I-B),

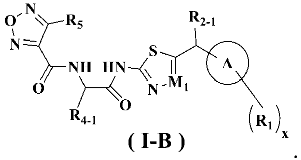

In certain embodiments of the present invention, the compound as represented by formula (I) is as represented by formula (I-B-1) or (I-B-2),

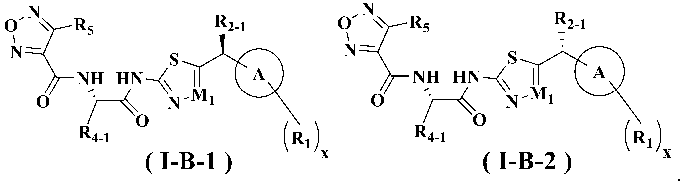

In certain embodiments of the present invention, the compound as represented by formula (I) is as represented by formula (I-1) or (I-2), 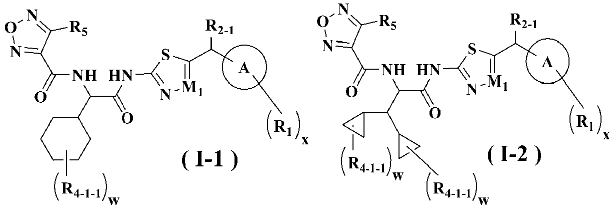 wherein R₄₋₁₋₁ is independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl; w is selected from 0, 1, 2, 3, 4, 5 or 6.

In certain embodiments of the present invention, the compound as represented by formula (I-1) is as represented by formula (I-1-1), (I-1-2), (I-2-1) or (I-2-2),

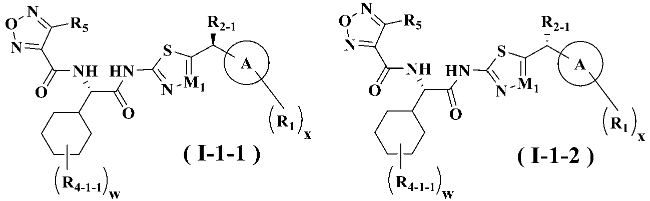

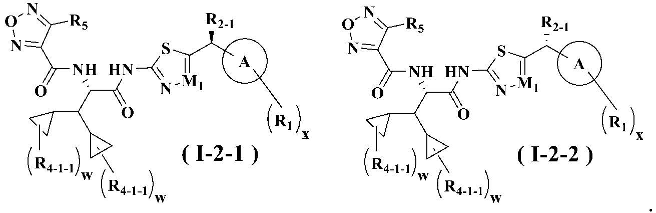

In certain embodiments of the present invention, the compound as represented by formula (I-1-1) is further as represented by formula (I-1-1-1),

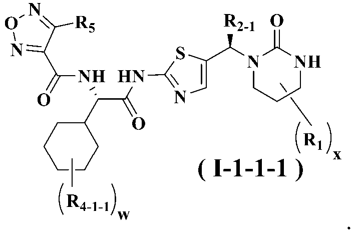

In certain embodiments of the present invention, the compound as represented by formula (I-A) is as represented by formula (VII): 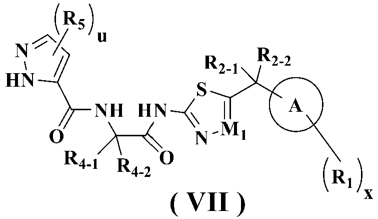 wherein M₁ is selected from N or CR₃;
ring A is selected from C₃₋₁₂ cycloalkyl, 3- to 12-membered heterocyclyl, C₆₋₁₄ aryl or 5- to 14-membered heteroaryl;
R₁ is independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₘ₁ORₐ₁, -(CH₂)ₘ₂C(O)Rₐ₂, -(CH₂)ₘ₃NHC(O)Rₐ₃, - (CH₂)ₘ₄C(O)NHRₐ₄, -(CH₂)ₘ₅NRₐ₅Rₐ₆, -(CH₂)ₘ₆S(O)ₘ₇Rₐ₇, -(CH₂)ₘ₈S(O)₂NHRₐ₈ or -(CH₂)ₘ₉NHS(O)₂Rₐ₉, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5-to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, carboxyl, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl, wherein the C₃₋₈ cycloalkyl, 3- to 8-membered heterocyclyl, C₆₋₁₀ aryl and 5- to 10-membered heteroaryl substituents are optionally further substituted with one or more of hydrogen, deuterium, halogen, amino, hydroxyl, cyano, nitro, carboxyl, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
preferably, R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{d7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, carboxyl, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
preferably, R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
alternatively, R₂₋₁ and R₂₋₂ are connected to form C₃₋₈ cycloalkyl, 3- to 8-membered heterocyclyl, C₆₋₁₀ aryl or 5- to 10-membered heteroaryl, wherein the C₃₋₈ cycloalkyl, 3- to 8-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₃ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, - (CH₂)ₒ₁OR_{c1}, -(CH₂)ₒ₂C(O)R_{c2}, -(CH₂)ₒ₃NHC(O)R_{c3}, -(CH₂)ₒ₄C(O)NHR_{c4}, - (CH₂)ₒ₅NR_{c5}R_{c6}, -(CH₂)ₒ₆S(O)ₒ₇R_{c7}, -(CH₂)ₒ₈S(O)₂NHR_{c8} or -(CH₂)ₒ₉NHS(O)₂R_{c9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₄₋₁ and R₄₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CHR_{d10})ₚ₁OR_{d1}, -(CHR_{d10})ₚ₂C(O)R_{d2}, - (CHR_{d10})ₚ₃NHC(O)R_{d3}, -(CHR_{d10})ₚ₄C(O)NHR_{d4}, -(CHR_{d10})ₚ₅NR_{d5}R_{d6}, - (CHR_{d10})ₚ₆(O)ₚ₇R_{d7}, -(CHR_{d10})ₚ₈S(O)₂NHR_{d8} or (CHR_{d10})ₚ₉NHS(O)₂R_{d9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
preferably, R₄₋₁ and R₄₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₚ₁OR₄₁, -(CH₂)ₚ₂C(O)R_{d2}, - (CH₂)ₚ₃NHC(O)R_{d3}, -(CH₂)ₚ₄C(O)NHR_{d4}, -(CH₂)ₚ₅NR_{d5}R_{d6}, -(CH₂)ₚ₆S(O)ₚ₇R_{d7}, - (CH₂)ₚ₈S(O)₂NHR_{d8} or -(CH₂)ₚ₉NHS(O)₂R_{d9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
alternatively, R₄₋₁ and R₄₋₂ are connected to form C₃₋₈ cycloalkyl, 3- to 8-membered heterocyclyl, C₆₋₁₀ aryl or 5- to 10-membered heteroaryl, wherein the C₃₋₈ cycloalkyl, 3- to 8-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₅ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, - (CH₂)_{q1}ORₑ₁, -(CH₂)_{q2}C(O)Rₑ₂, -(CH₂)_{q3}NHC(O)Rₑ₃, -(CH₂)_{q4}C(O)NHRₑ₄, - (CH₂)_{q5}NRₑ₅Rₑ₆, -(CH₂)_{q6}S(O)_{q7}Rₑ₇, -(CH₂)_{q8}S(O)₂NHRₑ₈ or -(CH₂)_{q9}NHS(O)₂Rₑ₉, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
Rₐ₁, Rₐ₂, Rₐ₃, Rₐ₄, Rₐ₅, Rₐ₆, Rₐ₇, Rₐ₈ and Rₐ₉ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{b1}, R_{b2}, R_{b3}, R_{b4}, R_{b5}, R_{b6}, R_{b7}, R_{b8} and R_{b9} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{c1}, R_{c2}, Rₑ₃, R_{c4}, R_{c5}, R_{c6}, R_{c7}, R_{c8} and R_{c9} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{d1}, R_{d2}, R_{d3}, R_{d4}, Rₐ₅, R_{d6}, R_{d7}, R_{d9} and R_{d10} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
Rₑ₁, R_{c2}, Rₑ₃, Rₑ₄, R_{c5}, Rₑ₆, R_{c7}, Rₑ₈ and Rₑ₉ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
x is 0, 1, 2, 3, 4 or 5;
u is 0, 1, 2, 3, 4 or 5;
m1, m2, m3, m4, m5, m6, m8 and m9 are each independently selected from 0, 1, 2, 3, 4, 5 or 6;
n1, n2, n3, n4, n5, n6, n8 and n9 are each independently selected from 0, 1, 2, 3, 4, 5 or 6;
o1, o2, o3, o4, o5, o6, o8 and o9 are each independently selected from 0, 1, 2, 3, 4, 5 or 6;
p1, p2, p3, p4, p5, p6, p8 and p9 are each independently selected from 0, 1, 2, 3, 4, 5 or 6;
q1, q2, q3, q4, q5, q6, q8 and q9 are each independently selected from 0, 1, 2, 3, 4, 5 or 6;
m7, n7, o7, p7 and q7 are each independently selected from 0, 1, 2 or 3.

In certain embodiments of the present invention, the compound as represented by formula (VII) is as represented by formula (VII-B),

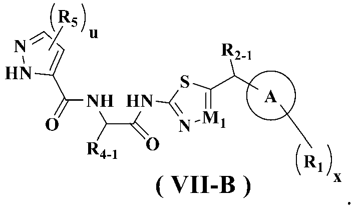

In certain embodiments of the present invention, the compound as represented by formula (VII) is as represented by formula (VII-B-1) or (VII-B-2),

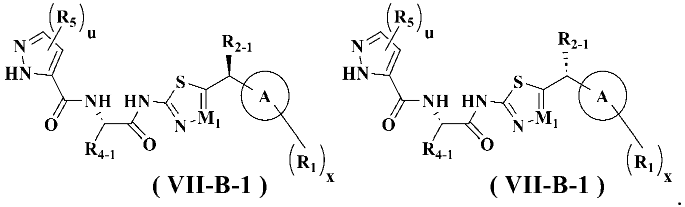

In certain embodiments of the present invention, the compound as represented by formula (VII) is as represented by formula (VII-1) or (VII-2), 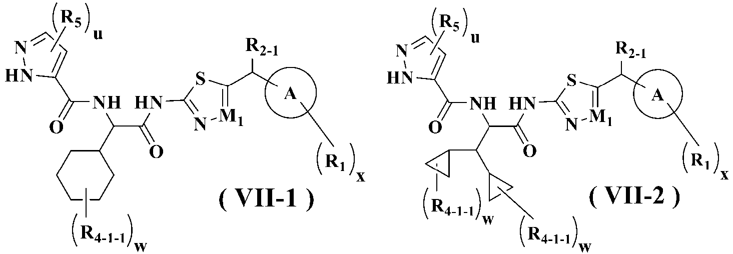 wherein R₄₋₁₋₁ is independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl; w is selected from 0, 1, 2, 3, 4, 5 or 6.

In certain embodiments of the present invention, the compound as represented by formula (VII-1) or (VII-2) is as represented by formula (VII-1-1), (VII-1-2), (VII-2-1) or (VII-2-2),

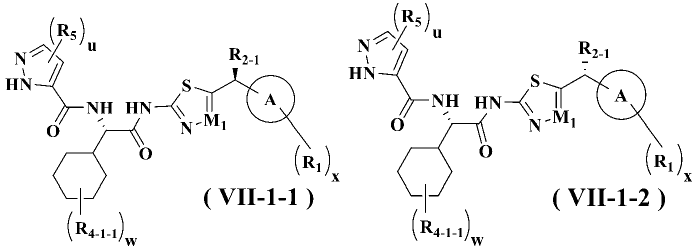

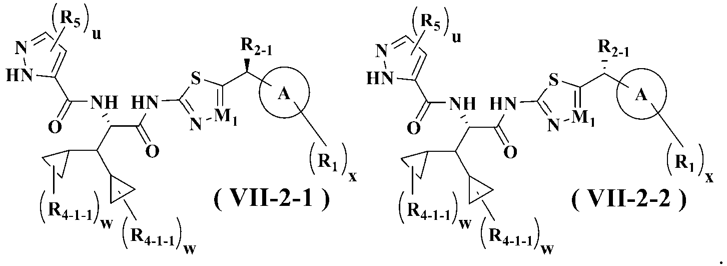

In certain embodiments of the present invention, ring A is selected from C₃₋₈ cycloalkyl, 3- to 10-membered heterocyclyl, C₆₋₁₀ aryl or 5- to 10-membered heteroaryl; preferably 5- to 6-membered heterocyclyl, 5- to 6-membered heteroaryl, 5- to 6-membered heteroaryl fused 5- to 6-membered heteroaryl, 5- to 6-membered heteroaryl fused phenyl, or 5- to 6-membered heteroaryl fused 5- to 6-membered heterocyclyl;
more preferably 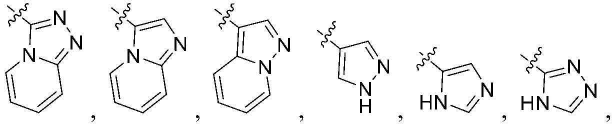 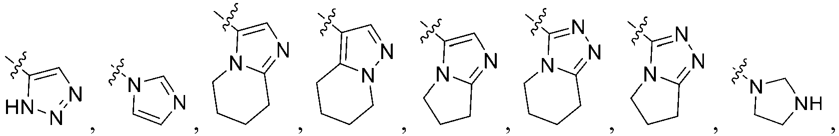 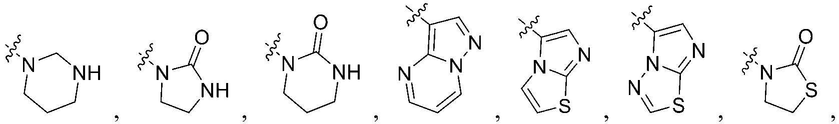 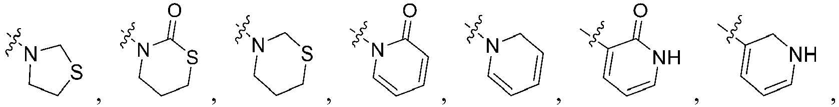 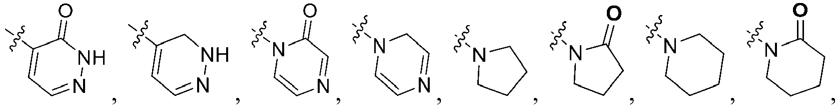 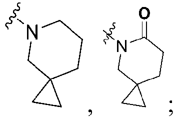
more preferably 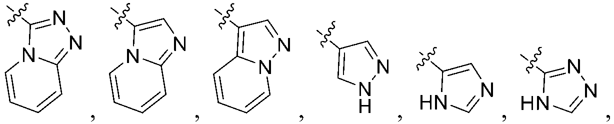 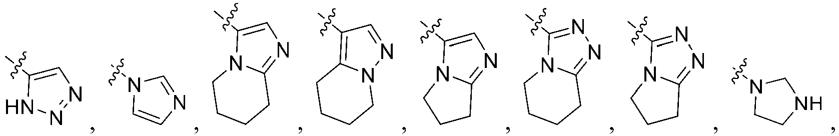 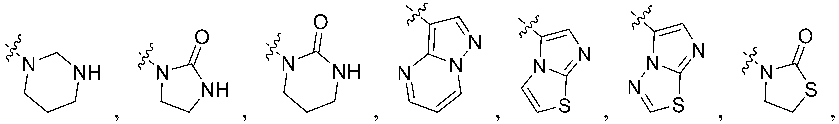 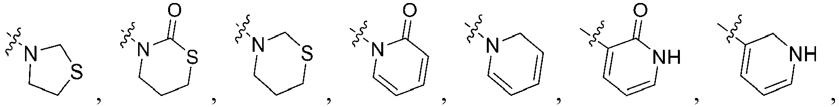 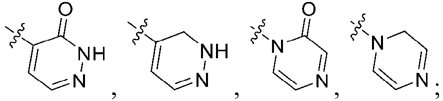
more preferably

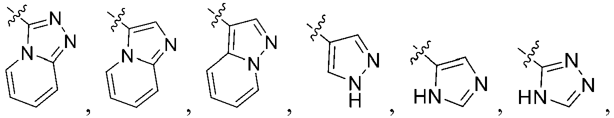

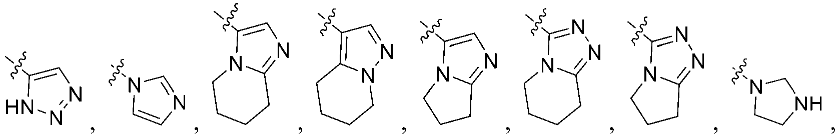

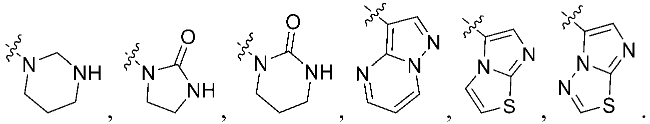

In certain embodiments of the present invention, ring A is selected from 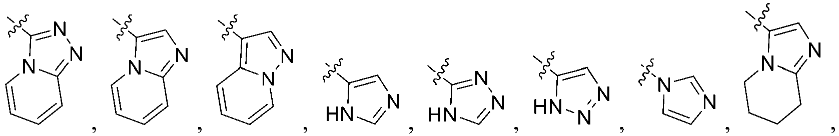 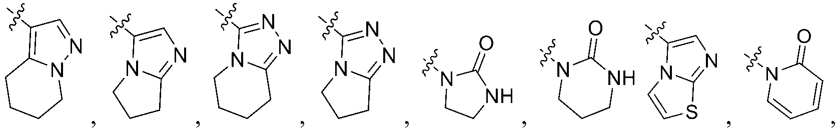 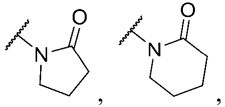 preferably selected from

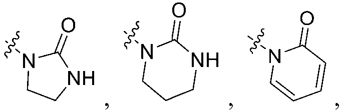

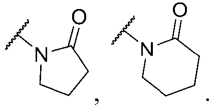

In certain embodiments of the present invention, R is selected from

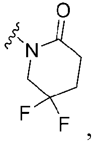

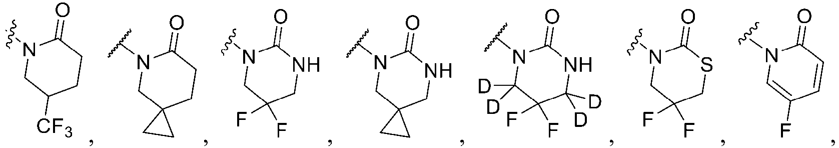

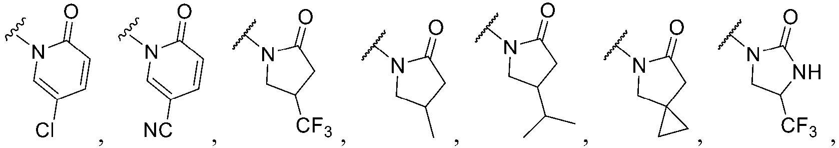

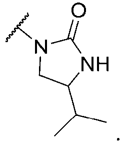

In certain embodiments of the present invention, the compound as represented by formula (I-A) is as represented by formula (II): 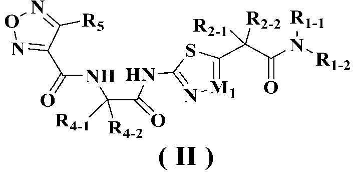 wherein M₁ is selected from N or CR₃;
R₁₋₁ or R₁₋₂ is each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₘ₁ORₐ₁, -(CH₂)ₘ₂C(O)Rₐ₂, -(CH₂)ₘ₃NHC(O)Rₐ₃, - (CH₂)ₘ₄C(O)NHRₐ₄, -(CH₂)ₘ₅NRₐ₅Rₐ₆, -(CH₂)ₘ₆S(O)ₘ₇Rₐ₇, -(CH₂)ₘ₈S(O)₂NHRₐ₈ or -(CH₂)ₘ₉NHS(O)₂Rₐ₉, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5-to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
alternatively, R₁₋₁ and R₁₋₂ are connected to form 3- to 8-membered heterocyclyl or 5- to 10-membered heteroaryl, wherein the 3- to 8-membered heterocyclyl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl and 5- to 10-membered heteroaryl;
R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, carboxyl, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
preferably, R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{d7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
alternatively, R₂₋₁ and R₂₋₂ are connected to form C₃₋₈ cycloalkyl, 3- to 8-membered heterocyclyl, C₆₋₁₀ aryl or 5- to 10-membered heteroaryl, wherein the C₃₋₈ cycloalkyl, 3- to 8-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₃ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, - (CH₂)ₒ₁OR_{c1}, -(CH₂)ₒ₂C(O)R_{c2}, -(CH₂)ₒ₃NHC(O)R_{c3}, -(CH₂)ₒ₄C(O)NHR_{c4}, - (CH₂)ₒ₅NR_{c5}R_{c6}, -(CH₂)ₒ₆S(O)ₒ₇R_{c7}, -(CH₂)ₒ₈S(O)₂NHR_{c8} or -(CH₂)ₒ₉NHS(O)₂R_{c9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₄₋₁ and R₄₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CHR_{d10})ₚ₁OR_{d1}, -(CHR_{d10})ₚ₂C(O)R_{d2}, - (CHR_{d10})ₚ₃NHC(O)R_{d3}, -(CHR_{d10})ₚ₄C(O)NHR_{d4}, -(CHR_{d10})ₚ₅NR_{d5}R_{d6}, - (CHR_{d10})ₚ₆S(O)ₚ₇R_{d7}, -(CHR_{d10})ₚ₈S(O)₂NHR_{d8} or (CHRₐ₁₀)ₚ₉NHS(O)₂R_{d9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
preferably, R₄₋₁ and R₄₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₚ₁OR_{d1}, -(CH₂)ₚ₂C(O)R_{d2}, - (CH₂)ₚ₃NHC(O)R_{d3}, -(CH₂)ₚ₄C(O)NHR_{d4}, -(CH₂)ₚ₅NR_{d5}R_{d6}, -(CH₂)ₚ₆S(O)ₚ₇R_{d7}, - (CH₂)ₚ₈S(O)₂NHR_{d8} or -(CH₂)ₚ₉NHS(O)₂R_{d9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
alternatively, R₄₋₁ and R₄₋₂ are connected to form C₃₋₈ cycloalkyl, 3- to 8-membered heterocyclyl, C₆₋₁₀ aryl or 5- to 10-membered heteroaryl, wherein the C₃₋₈ cycloalkyl, 3- to 8-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₅ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, - (CH₂)_{q1}ORₑ₁, -(CH₂)_{q2}C(O)Rₑ₂, -(CH₂)_{q3}NHC(O)Rₑ₃, -(CH₂)_{q4}C(O)NHRₑ₄, - (CH₂)_{q5}NRₑ₅Rₑ₆, -(CH₂)_{q6}S(O)_{q7}Rₑ₇, -(CH₂)_{q8}S(O)₂NHRₑ₈ or -(CH₂)_{q9}NHS(O)₂Rₑ₉, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
Rₐ₁, Rₐ₂, Rₐ₃, Rₐ₄, Rₐ₅, Rₐ₆, Rₐ₇, Rₐ₈ and Rₐ₉ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{b1}, R_{b2}, R_{b3}, R_{b4}, R_{b5}, R_{b6}, R_{b7}, R_{b8} and R_{b9} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{c1}, R_{c2}, R_{c3}, R_{c4}, R_{c5}, R_{c6}, R_{c7}, R_{c8} and R_{c9} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{d1}, R_{d2}, R_{d3}, R_{d4}, Rₐ₅, Rₐ₅, R_{d7}, Rₐ₅, Rₐ₉ and R_{d10} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
Rₑ₁, Rₑ₂, Rₑ₃, Rₑ₄, Rₑ₅, Rₑ₆, Rₑ₇, Rₑ₈ and Rₑ₉ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
x is 0, 1, 2, 3, 4 or 5;
m1, m2, m3, m4, m5, m6, m8 and m9 are each independently selected from 0, 1, 2, 3, 4, 5 or 6;
n1, n2, n3, n4, n5, n6, n8 and n9 are each independently selected from 0, 1, 2, 3, 4, 5 or 6;
o1, o2, o3, o4, o5, o6, o8 and o9 are each independently selected from 0, 1, 2, 3, 4, 5 or 6;
p1, p2, p3, p4, p5, p6, p8 and p9 are each independently selected from 0, 1, 2, 3, 4, 5 or 6;
q1, q2, q3, q4, q5, q6, q8 and q9 are each independently selected from 0, 1, 2, 3, 4, 5 or 6;
m7, n7, o7, p7 and q7 are each independently selected from 0, 1, 2 or 3.

In certain embodiments of the present invention, R is

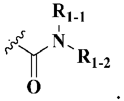

In certain embodiments of the present invention, the compound as represented by formula (II) is as represented by formula (II-1), 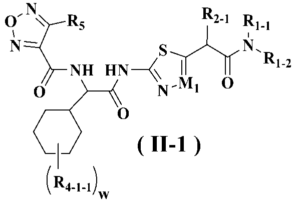
wherein R₁₋₁ or R₁₋₂ is each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₘ₁ORₐ₁, -(CH₂)ₘ₂C(O)Rₐ₂, - (CH₂)ₘ₃NHC(O)Rₐ₃, -(CH₂)ₘ₄C(O)NHRₐ₄, -(CH₂)ₘ₅NRₐ₅Rₐ₆, -(CH₂)ₘ₆S(O)ₘ₇Rₐ₇, - (CH₂)ₘ₈S(O)₂NHRₐ₈ or -(CH₂)ₘ₉NHS(O)₂Rₐ₉, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₄₋₁₋₁ is independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl; w is selected from 0, 1, 2, 3, 4, 5 or 6.

In certain embodiments of the present invention, the compound as represented by formula (II) is as represented by formula (II-1-1),

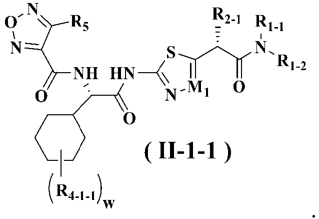

In certain embodiments of the present invention, the compound as represented by formula (II) is as represented by formula (II-2), 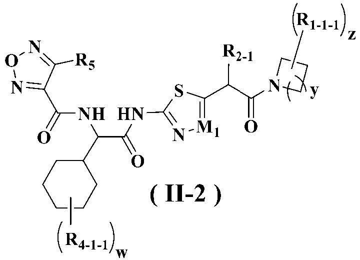
wherein R₁₋₁₋₁ is independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl;
R₄₋₁₋₁ is independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl; w is selected from 0, 1, 2, 3, 4 or 5; y is selected from 0, 1, 2, 3, 4, 5 or 6; z is selected from 0, 1, 2, 3, 4, 5 or 6.

In certain embodiments of the present invention, the compound as represented by formula (II-2) is as represented by formula (II-2-1),

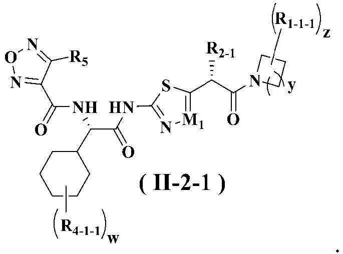

In certain embodiments of the present invention, the compound as represented by formula (I-A) is as represented by formula (III): 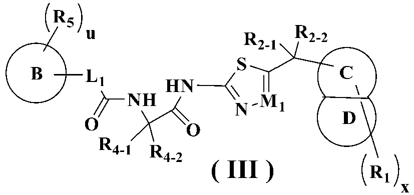 wherein M₁ is selected from N or CR₃;
ring B is selected from C₃₋₁₂ cycloalkyl, 3- to 12-membered heterocyclyl, C₆₋₁₄ aryl or 5- to 14-membered heteroaryl; or ring B is absent, and 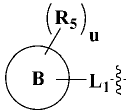 is R₅-L₁- 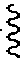 -;
ring C is selected from C₃₋₆ cycloalkyl, 3- to 6-membered heterocyclyl, phenyl or 5- to 6-membered heteroaryl;
ring D is selected from C₃₋₆ cycloalkyl, 3- to 6-membered heterocyclyl, phenyl or 5- to 6-membered heteroaryl;
L₁ is selected from a bond, -O-, -NH-, -CO-, -CONH-, C₁₋₃ alkylene, C₂₋₄ alkenylene, C₂₋₄ alkynylene, -O-C₁₋₃ alkylene-, -S-C₁₋₃ alkylene-, -C₁₋₃ alkylene-NH-, -C₁₋₃ alkylene-C(O)NH-, -C₁₋₃ alkylene-NHC(O)-, -C₁₋₃ alkylene-S(O)ᵣ₁-, -C₁₋₃ alkylene-NHS(O)ᵣ₂- or -C₁₋₃ alkylene-S(O)ᵣ₃NH-; the -NH-, -CONH-, C₁₋₃ alkylene, C₂₋₄ alkenylene, C₂₋₄ alkynylene, -O-C₁₋₃ alkylene-, -S-C₁₋₃ alkylene-, -C₁₋₃ alkylene-NH-, -C₁₋₃ alkylene-C(O)NH-, -C₁₋₃ alkylene-NHC(O)-, -C₁₋₃ alkylene-S(O)ᵣ₁-, -C₁₋₃ alkylene-NHS(O)ᵣ₂- or -C₁₋₃ alkylene-S(O)ᵣ₃NH- is optionally substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl;
R₁ is independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₘ₁ORₐ₁, -(CH₂)ₘ₂C(O)Rₐ₂, -(CH₂)ₘ₃NHC(O)Rₐ₃, - (CH₂)ₘ₄C(O)NHRₐ₄, -(CH₂)ₘ₅NRₐ₅Rₐ₆, -(CH₂)ₘ₆S(O)ₘ₇Rₐ₇, -(CH₂)ₘ₈S(O)₂NHRₐ₈ or -(CH₂)ₘ₉NHS(O)₂Rₐ₉, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5-to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{d7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, carboxyl, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl, wherein the C₃₋₈ cycloalkyl, 3- to 8-membered heterocyclyl, C₆₋₁₀ aryl and 5- to 10-membered heteroaryl substituents are optionally further substituted with one or more of hydrogen, deuterium, halogen, amino, hydroxyl, cyano, nitro, carboxyl, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
preferably, R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, carboxyl, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
preferably, R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
alternatively, R₂₋₁ and R₂₋₂ are connected to form C₃₋₈ cycloalkyl, 3- to 8-membered heterocyclyl, C₆₋₁₀ aryl or 5- to 10-membered heteroaryl, wherein the C₃₋₈ cycloalkyl, 3- to 8-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₃ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, - (CH₂)ₒ₁OR_{c1}, -(CH₂)ₒ₂C(O)R_{c2}, -(CH₂)ₒ₃NHC(O)R_{c3}, -(CH₂)ₒ₄C(O)NHR_{c4}, - (CH₂)ₒ₅NR_{c5}R_{c6}, -(CH₂)ₒ₆S(O)ₒ₇R_{c7}, -(CH₂)ₒ₈S(O)₂NHR_{c8} or -(CH₂)ₒ₉NHS(O)₂R_{c9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₄₋₁ and R₄₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CHRₐ₁₀)ₚ₁OR_{d1}, -(CHR_{d10})ₚ₂C(O)R_{d2}, - (CHR_{d10})ₚ₃NHC(O)R_{d3}, -(CHR_{d10})ₚ₄C(O)NHR_{d4}, -(CHR_{d10})ₚ₅NR_{d5}R_{d6}, - (CHR_{d10})ₚ₆S(O)ₚ₇R_{d7}, -(CHR_{d10})ₚ₈S(O)₂NHR_{d8} or (CHR_{d10})p₉NHS(O)₂R_{d9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
preferably, R₄₋₁ and R₄₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₚ₁OR_{d1}, -(CH₂)ₚ₂C(O)R_{d2}, - (CH₂)ₚ₃NHC(O)R_{d3}, -(CH₂)ₚ₄C(O)NHR_{d4}, -(CH₂)ₚ₅NR_{d5}R_{d6}, -(CH₂)ₚ₆S(O)ₚ₇R_{d7}, - (CH₂)ₚ₈S(O)₂NHR_{d8} or -(CH₂)ₚ₉NHS(O)₂R_{d9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
alternatively, R₄₋₁ and R₄₋₂ are connected to form C₃₋₈ cycloalkyl, 3- to 8-membered heterocyclyl, C₆₋₁₀ aryl or 5- to 10-membered heteroaryl, wherein the C₃₋₈ cycloalkyl, 3- to 8-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₅ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, - (CH₂)_{q1}ORₑ₁, -(CH₂)_{q2}C(O)Rₑ₂, -(CH₂)_{q3}NHC(O)Rₑ₃, -(CH₂)_{q4}C(O)NHRₑ₄, - (CH₂)_{q5}NRₑ₅Rₑ₆, -(CH₂)_{q6}S(O)_{q7}Rₑ₇, -(CH₂)_{q8}S(O)₂NHRₑ₈ or -(CH₂)_{q9}NHS(O)₂Rₑ₉, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
Rₐ₁, Rₐ₂, Rₐ₃, Rₐ₄, Rₐ₅, Rₐ₆, Rₐ₇, Rₐ₈ and Rₐ₉ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{b1}, R_{b2}, R_{b3}, R_{b4}, R_{b5}, R_{b6}, R_{b7}, R_{b8} and R_{b9} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{c1}, R_{c2}, R_{c3}, R_{c4}, R_{c5}, R_{c6}, R_{c7}, R_{c8} and R_{c9} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{d1}, R_{d2}, R_{d3}, R_{d4}, R_{d5}, R_{d6}, R_{d7}, R_{d8}, R_{d9} and R_{d10} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
Rₑ₁, Rₑ₂, Rₑ₃, Rₑ₄, Rₑ₅, Rₑ₆, Rₑ₇, Rₑ₈ and Rₑ₉ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
x is 0, 1, 2, 3, 4 or 5;
u is 0, 1, 2, 3, 4 or 5;
m1, m2, m3, m4, m5, m6, m8 and m9 are each independently selected from 0, 1, 2, 3, 4, 5 or 6;
n1, n2, n3, n4, n5, n6, n8 and n9 are each independently selected from 0, 1, 2, 3, 4, 5 or 6;
o1, o2, o3, o4, o5, o6, o8 and o9 are each independently selected from 0, 1, 2, 3, 4, 5 or 6;
p1, p2, p3, p4, p5, p6, p8 and p9 are each independently selected from 0, 1, 2, 3, 4, 5 or 6;
q1, q2, q3, q4, q5, q6, q8 and q9 are each independently selected from 0, 1, 2, 3, 4, 5 or 6;
m7, n7, o7, p7 and q7 are each independently selected from 0, 1, 2 or 3;
r1, r2 and r3 are each independently selected from 0, 1 or 2.

In certain embodiments of the present invention, the compound as represented by formula (III) is as represented by formula (III-B):

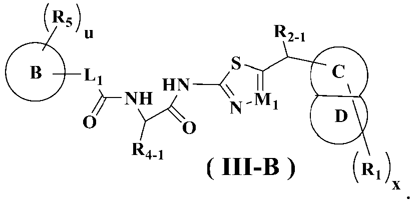

In certain embodiments of the present invention, the compound as represented by formula (III) is as represented by formula (III-B-1) or (III-B-2):

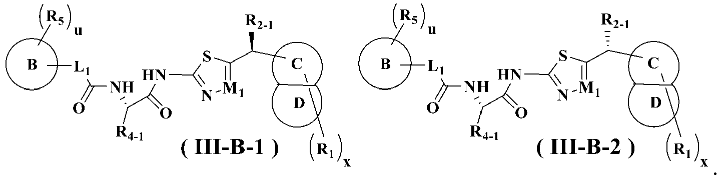

In certain embodiments of the present invention, ring C is 5- to 6-membered heteroaryl; preferably 5-membered heteroaryl; more preferably

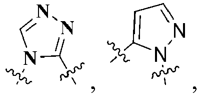

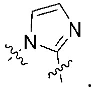

In certain embodiments of the present invention, ring D is 5- to 6-membered heteroaryl; preferably 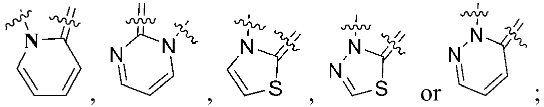 more preferably

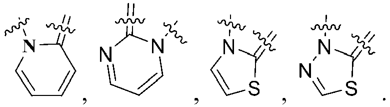

In certain embodiments of the present invention, the compound as represented by formula (III) is as represented by formula (III-1), (III-2), (III-3), (III-4), (III-5), (III-6), (III-7), (III-8), (III-9), (III-10) or (III-11), 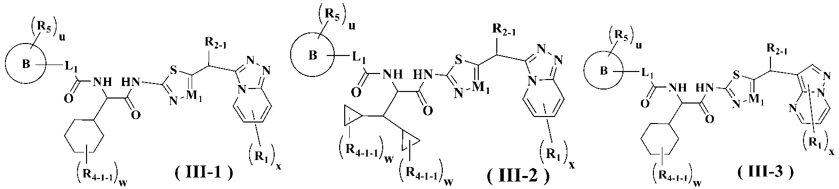 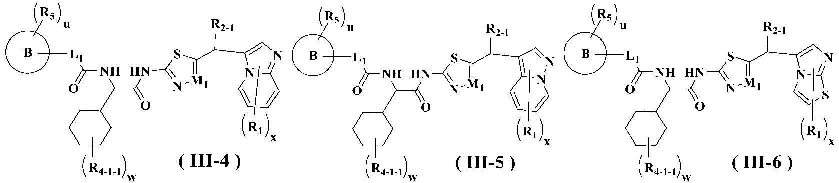 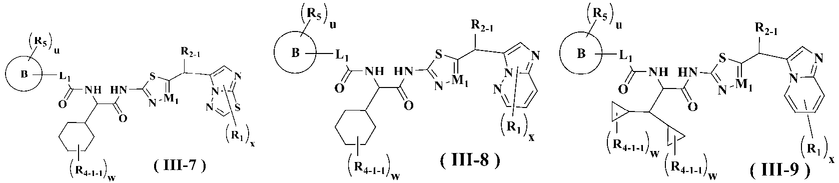 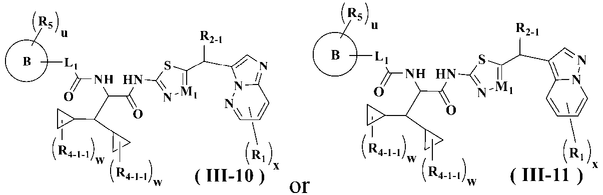 R₄₋₁₋₁ is independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl; w is selected from 0, 1, 2, 3, 4 or 5.

In certain embodiments of the present invention, the compound as represented by formula (III-1), (III-2), (III-3), (III-4), (III-5), (III-6), (III-7), (III-8), (III-9), (III-10) or (III-11) is respectively as represented by formula (III-1-1), (III-2-1), (III-3-1), (III-4-1), (III-5-1), (III-6-1), (III-7-1), (III-8-1), (III-9-1), (III-10-1) or (III-11-1),

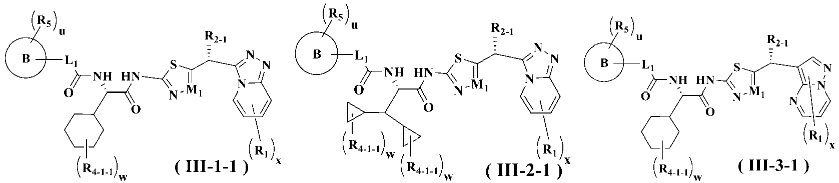

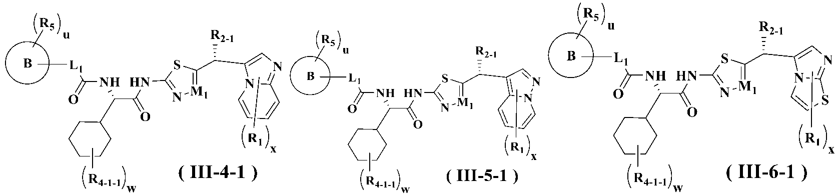

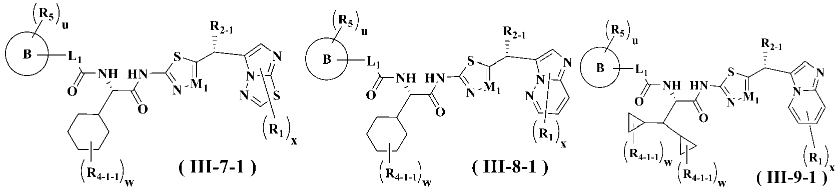

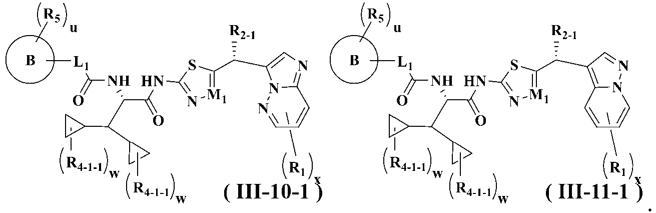

In certain embodiments of the present invention, the compound as represented by formula (I-A) is as represented by formula (IV) or formula (IV-A): 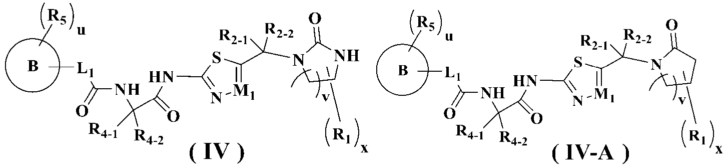 wherein M₁ is selected from N or CR₃;
ring B is selected from C₃₋₁₂ cycloalkyl, 3- to 12-membered heterocyclyl, C₆₋₁₄ aryl or 5- to 14-membered heteroaryl; or ring B is absent, and 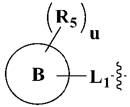 is R₅-L₁- 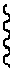 -;
L₁ is selected from a bond, -O-, -NH-, -CO-, -CONH-, C₁₋₃ alkylene, C₂₋₄ alkenylene, C₂₋₄ alkynylene, -O-C₁₋₃ alkylene-, -S-C₁₋₃ alkylene-, -C₁₋₃ alkylene-NH-, -C₁₋₃ alkylene-C(O)NH-, -C₁₋₃ alkylene-NHC(O)-, -C₁₋₃ alkylene-S(O)ᵣ₁-, -C₁₋₃ alkylene-NHS(O)ᵣ₂- or -C₁₋₃ alkylene-S(O)ᵣ₃NH-; the -NH-, -CONH-, C₁₋₃ alkylene, C₂₋₄ alkenylene, C₂₋₄ alkynylene, -O-C₁₋₃ alkylene-, -S-C₁₋₃ alkylene-, -C₁₋₃ alkylene-NH-, -C₁₋₃ alkylene-C(O)NH-, -C₁₋₃ alkylene-NHC(O)-, -C₁₋₃ alkylene-S(O)ᵣ₁-, -C₁₋₃ alkylene-NHS(O)ᵣ₂- or -C₁₋₃ alkylene-S(O)ᵣ₃NH- is optionally substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl;
R₁ is independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₘ₁ORₐ₁, -(CH₂)ₘ₂C(O)Rₐ₂, -(CH₂)ₘ₃NHC(O)Rₐ₃, - (CH₂)ₘ₄C(O)NHRₐ₄, -(CH₂)ₘ₅NRₐ₅Rₐ₆, -(CH₂)ₘ₆S(O)ₘ₇Rₐ₇, -(CH₂)ₘ₈S(O)₂NHRₐ₈ or -(CH₂)ₘ₉NHS(O)₂Rₐ₉, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5-to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₁₂ cycloalkyl, 3- to 12-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₁₂ cycloalkyl, 3- to 12-membered heterocyclyl, C₆₋₁₀ aryl or 5- to 10-membered heteroaryl may optionally be further substituted with one or more of hydrogen, deuterium, halogen, amino, hydroxyl, cyano, nitro, carboxyl, oxo, thio, =CF₂, =CHF, =NOC₁₋₆ alkyl, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₁₂ cycloalkyl, 3- to 12-membered heterocyclyl, C₆₋₁₀ aryl and 5- to 10-membered heteroaryl; the C₃₋₁₂ cycloalkyl, 3- to 12-membered heterocyclyl, C₆₋₁₀ aryl and 5- to 10-membered heteroaryl substituents are optionally further substituted with one or more of hydrogen, deuterium, halogen, amino, hydroxyl, cyano, nitro, carboxyl, oxo, thio, =CF₂, =CHF, =NOC₁₋₆ alkyl, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ heteroalkyl, C₁₋₆ deuteroheteroalkyl, C₁₋₆ haloheteroalkyl, C₁₋₆ hydroxyalkyl, C₃₋₈ cycloalkyl, 3- to 8-membered heterocyclyl, C₆₋₁₀ aryl and 5- to 10-membered heteroaryl; the C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ heteroalkyl, C₁₋₆ deuteroheteroalkyl, C₁₋₆ haloheteroalkyl, C₁₋₆ hydroxyalkyl, C₃₋₈ cycloalkyl, 3- to 8-membered heterocyclyl, C₆₋₁₀ aryl and 5- to 10-membered heteroaryl are optionally further substituted with one or more of hydrogen, deuterium, halogen, amino, hydroxyl, cyano, nitro, carboxyl, oxo, thio, =CF₂, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ heteroalkyl, C₁₋₆ deuteroheteroalkyl, C₁₋₆ haloheteroalkyl, C₁₋₆ hydroxyalkyl, C₃₋₈ cycloalkyl, 3- to 8-membered heterocyclyl, C₆₋₁₀ aryl and 5- to 10-membered heteroaryl;
preferably, R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, carboxyl, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl, wherein the C₃₋₈ cycloalkyl, 3- to 8-membered heterocyclyl, C₆₋₁₀ aryl and 5- to 10-membered heteroaryl substituents are optionally further substituted with one or more of hydrogen, deuterium, halogen, amino, hydroxyl, cyano, nitro, carboxyl, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
preferably, R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, carboxyl, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
preferably, R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
alternatively, R₂₋₁ and R₂₋₂ are connected to form C₃₋₈ cycloalkyl, 3- to 8-membered heterocyclyl, C₆₋₁₀ aryl or 5- to 10-membered heteroaryl, wherein the C₃₋₈ cycloalkyl, 3- to 8-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₃ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, - (CH₂)ₒ₁OR_{c1}, -(CH₂)ₒ₂C(O)R_{c2}, -(CH₂)ₒ₃NHC(O)R_{c3}, -(CH₂)ₒ₄C(O)NHR_{c4}, - (CH₂)ₒ₅NR_{c5}R_{c6}, -(CH₂)ₒ₆S(O)ₒ₇R_{c7}, -(CH₂)ₒ₈S(O)₂NHR_{c8} or -(CH₂)ₒ₉NHS(O)₂R_{c9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₄₋₁ and R₄₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CHR_{d10})ₚ₁OR_{d1}, -(CHR_{d10})ₚ₂C(O)R_{d2}, - (CHR_{d10})ₚ₃NHC(O)R_{d3}, -(CHR_{d10})ₚ₄C(O)NHR_{d4}, -(CHR_{d10})ₚ₅NR_{d5}R_{d6}, - (CHR_{d10})ₚ₆S(O)ₚ₇R_{d7}, -(CHR_{d10})ₚ₈S(O)₂NHR_{d8} or -(CHR_{d10})ₚ₉NHS(O)₂R_{d9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
preferably, R₄₋₁ and R₄₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₚ₁OR_{d1}, -(CH₂)ₚ₂C(O)R_{d2}, - (CH₂)ₚ₃NHC(O)R_{d3}, -(CH₂)ₚ₄C(O)NHR_{d4}, -(CH₂)ₚ₅NR_{d5}R_{d6}, -(CH₂)ₚ₆S(O)ₚ₇R_{d7}, - (CH₂)ₚ₈S(O)₂NHR_{d8} or -(CH₂)ₚ₉NHS(O)₂R_{d9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
alternatively, R₄₋₁ and R₄₋₂ are connected to form C₃₋₈ cycloalkyl, 3- to 8-membered heterocyclyl, C₆₋₁₀ aryl or 5- to 10-membered heteroaryl, wherein the C₃₋₈ cycloalkyl, 3- to 8-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₅ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, - (CH₂)_{q1}ORₑ₁, -(CH₂)_{q2}C(O)Rₑ₂, -(CH₂)_{q3}NHC(O)Rₑ₃, -(CH₂)_{q4}C(O)NHRₑ₄, - (CH₂)_{q5}NRₑ₅Rₑ₆, -(CH₂)_{q6}S(O)_{q7}Rₑ₇, -(CH₂)_{q8}S(O)₂NHRₑ₈ or -(CH₂)_{q9}NHS(O)₂Rₑ₉, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
Rₐ₁, Rₐ₂, Rₐ₃, Rₐ₄, Rₐ₅, Rₐ₆, Rₐ₇, Rₐ₈ and Rₐ₉ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{b1}, R_{b2}, R_{b3}, R_{b4}, R_{b5}, R_{b6}, R_{b7}, R_{b8} and R_{b9} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{c1}, R_{c2}, R_{c3}, R_{c4}, R_{c5}, R_{c6}, R_{c7}, R_{c8} and R_{c9} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{d1}, R_{d2}, R_{d3}, R_{d4}, R_{d5}, R_{d6}, R_{d7}, R_{d8} and R_{d9} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
Rₑ₁, Rₑ₂, Rₑ₃, Rₑ₄, Rₑ₅, Rₑ₆, Rₑ₇, Rₑ₈ and Rₑ₉ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
x is 0, 1, 2, 3, 4 or 5; v is 1, 2, 3 or 4; u is 0, 1, 2, 3, 4 or 5;
m1, m2, m3, m4, m5, m6, m8 and m9 are each independently selected from 0, 1, 2, 3, 4, 5 or 6;
n1, n2, n3, n4, n5, n6, n8 and n9 are each independently selected from 0, 1, 2, 3, 4, 5 or 6;
o1, o2, o3, o4, o5, o6, o8 and o9 are each independently selected from 0, 1, 2, 3, 4, 5 or 6;
p1, p2, p3, p4, p5, p6, p8 and p9 are each independently selected from 0, 1, 2, 3, 4, 5 or 6;
q1, q2, q3, q4, q5, q6, q8 and q9 are each independently selected from 0, 1, 2, 3, 4, 5 or 6;
m7, n7, o7, p7 and q7 are each independently selected from 0, 1, 2 or 3;
r1, r2 and r3 are each independently selected from 0, 1 or 2.

In certain embodiments of the present invention, the compound as represented by formula (IV) is as represented by formula (IV-1) or formula (IV-2), and the compound as represented by formula (IV-A) is as represented by formula (IV-A-1) or formula (IV-A-2),

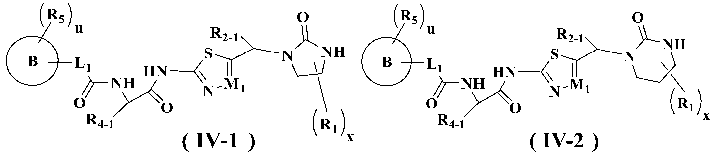

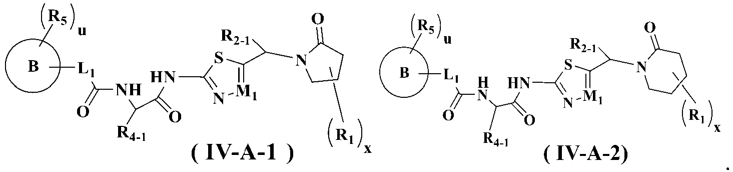

In certain embodiments of the present invention, the compound as represented by formula (IV-1), formula (IV-2), formula (IV-A-1) or formula (IV-A-2) is as represented by formula (IV-1-1), formula (IV-2-1), formula (IV-A-3) or formula (IV-A-4),

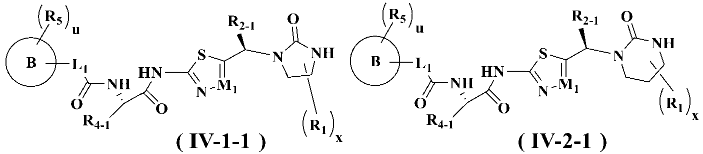

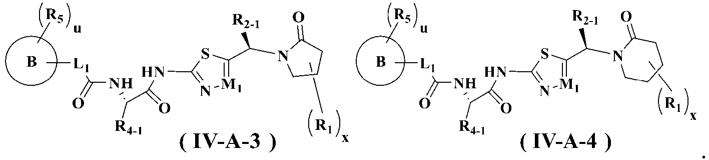

In certain embodiments of the present invention, the compound as represented by formula (I-A) is as represented by formula (V), 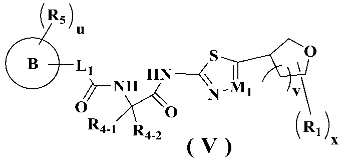 wherein M₁ is selected from N or CR₃;
ring B is selected from C₃₋₁₂ cycloalkyl, 3- to 12-membered heterocyclyl, C₆₋₁₄ aryl or 5- to 14-membered heteroaryl; or ring B is absent, and 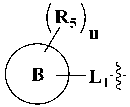 is R₅-L₁- 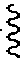 -;
L₁ is selected from a bond, -O-, -NH-, -CO-, -CONH-, C₁₋₃ alkylene, C₂₋₄ alkenylene, C₂₋₄ alkynylene, -O-C₁₋₃ alkylene-, -S-C₁₋₃ alkylene-, -C₁₋₃ alkylene-NH-, -C₁₋₃ alkylene-C(O)NH-, -C₁₋₃ alkylene-NHC(O)-, -C₁₋₃ alkylene-S(O)ᵣ₁-, -C₁₋₃ alkylene-NHS(O)ᵣ₂- or -C₁₋₃ alkylene-S(O)ᵣ₃NH-; the -NH-, -CONH-, C₁₋₃ alkylene, C₂₋₄ alkenylene, C₂₋₄ alkynylene, -O-C₁₋₃ alkylene-, -S-C₁₋₃ alkylene-, -C₁₋₃ alkylene-NH-, -C₁₋₃ alkylene-C(O)NH-, -C₁₋₃ alkylene-NHC(O)-, -C₁₋₃ alkylene-S(O)ᵣ₁-, -C₁₋₃ alkylene-NHS(O)ᵣ₂- or -C₁₋₃ alkylene-S(O)ᵣ₃NH- is optionally substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl;
R₁ is independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₘ₁ORₐ₁, -(CH₂)ₘ₂C(O)Rₐ₂, -(CH₂)ₘ₃NHC(O)Rₐ₃, - (CH₂)ₘ₄C(O)NHRₐ₄, -(CH₂)ₘ₅NRₐ₅Rₐ₆, -(CH₂)ₘ₆S(O)ₘ₇Rₐ₇, -(CH₂)ₘ₈S(O)₂NHRₐ₈ or -(CH₂)ₘ₉NHS(O)₂Rₐ₉, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5-to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₃ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, - (CH₂)ₒ₁OR_{c1}, -(CH₂)ₒ₂C(O)R_{c2}, -(CH₂)ₒ₃NHC(O)R_{c3}, -(CH₂)ₒ₄C(O)NHR_{c4}, - (CH₂)ₒ₅NR_{c5}R_{c6}, -(CH₂)ₒ₆S(O)ₒ₇R_{c7}, -(CH₂)ₒ₈S(O)₂NHR_{c8} or -(CH₂)ₒ₉NHS(O)₂R_{c9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₄₋₁ and R₄₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CHR_{d10})ₚ₁OR_{d1}, -(CHR_{d10})ₚ₂C(O)Rₐ₂, - (CHR_{d10})ₚ₃NHC(O)R_{d3}, -(CHR_{d10})ₚ₄C(O)NHR_{d4}, -(CHR_{d10})ₚ₅NR_{d5}R_{d6}, - (CHR_{d10})ₚ₆S(O)ₚ₇R_{d7}, -(CHR_{d10})ₚ₈S(O)₂NHR_{d8} or -(CHR_{d10})ₚ₉NHS(O)₂R_{d9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
preferably, R₄₋₁ and R₄₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₚ₁OR_{d1}, -(CH₂)ₚ₂C(O)R_{d2}, - (CH₂)ₚ₃NHC(O)R_{d3}, -(CH₂)ₚ₄C(O)NHR_{d4}, -(CH₂)ₚ₅NR_{d5}R_{d6}, -(CH₂)ₚ₆S(O)ₚ₇R_{d7}, - (CH₂)ₚ₈S(O)₂NHR_{d8} or -(CH₂)ₚ₉NHS(O)₂R_{d9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
alternatively, R₄₋₁ and R₄₋₂ are connected to form C₃₋₈ cycloalkyl, 3- to 8-membered heterocyclyl, C₆₋₁₀ aryl or 5- to 10-membered heteroaryl, wherein the C₃₋₈ cycloalkyl, 3- to 8-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₅ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, - (CH₂)_{q1}ORₑ₁, -(CH₂)_{q2}C(O)Rₑ₂, -(CH₂)_{q3}NHC(O)Rₑ₃, -(CH₂)_{q4}C(O)NHRₑ₄, - (CH₂)_{q5}NRₑ₅Rₑ₆, -(CH₂)_{q6}S(O)_{q7}Rₑ₇, -(CH₂)_{q8}S(O)₂NHRₑ₈ or -(CH₂)_{q9}NHS(O)₂Rₑ₉, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
Rₐ₁, Rₐ₂, Rₐ₃, Rₐ₄, Rₐ₅, Rₐ₆, Rₐ₇, Rₐ₈ and Rₐ₉ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{b1}, R_{b2}, R_{b3}, R_{b4}, R_{b5}, R_{b6}, R_{b7}, R_{b8} and R_{b9} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{c1}, R_{c2}, R_{c3}, R_{c4}, R_{c5}, R_{c6}, R_{c7}, R_{c8} and R_{c9} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{d1}, R_{d2}, R_{d3}, R_{d4}, Rₐ₅, Rₐ₆, R_{d7}, R_{d8} and R_{d9} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
Rₑ₁, Rₑ₂, Rₑ₃, Rₑ₄, Rₑ₅, Rₑ₆, Rₑ₇, Rₑ₈ and Rₑ₉ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
x is 0, 1, 2, 3, 4 or 5; v is 1, 2, 3 or 4; u is 0, 1, 2, 3, 4 or 5;
m1, m2, m3, m4, m5, m6, m8 and m9 are each independently selected from 0, 1, 2, 3, 4, 5 or 6;
n1, n2, n3, n4, n5, n6, n8 and n9 are each independently selected from 0, 1, 2, 3, 4, 5 or 6;
o1, o2, o3, o4, o5, o6, o8 and o9 are each independently selected from 0, 1, 2, 3, 4, 5 or 6;
p1, p2, p3, p4, p5, p6, p8 and p9 are each independently selected from 0, 1, 2, 3, 4, 5 or 6;
q1, q2, q3, q4, q5, q6, q8 and q9 are each independently selected from 0, 1, 2, 3, 4, 5 or 6;
m7, n7, o7, p7 and q7 are each independently selected from 0, 1, 2 or 3;
r1, r2 and r3 are each independently selected from 0, 1 or 2.

In certain embodiments of the present invention, the compound as represented by formula (V) is as represented by formula (V-1),

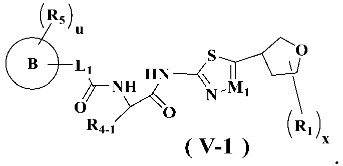

In certain embodiments of the present invention, the compound as represented by formula (V-1) is as represented by formula (V-1-1),

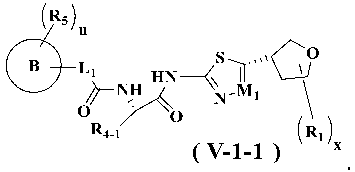

In certain embodiments of the present invention, the compound as represented by formula (I) is as represented by formula (VII), 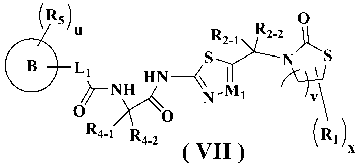 wherein M₁ is selected from N or CR₃;
ring B is selected from C₃₋₁₂ cycloalkyl, 3- to 12-membered heterocyclyl, C₆₋₁₄ aryl or 5- to 14-membered heteroaryl; or ring B is absent, and 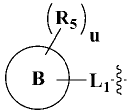 is R₅-L₁- 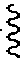 -;
L₁ is selected from a bond, -O-, -NH-, -CO-, -CONH-, C₁₋₃ alkylene, C₂₋₄ alkenylene, C₂₋₄ alkynylene, -O-C₁₋₃ alkylene-, -S-C₁₋₃ alkylene-, -C₁₋₃ alkylene-NH-, -C₁₋₃ alkylene-C(O)NH-, -C₁₋₃ alkylene-NHC(O)-, -C₁₋₃ alkylene-S(O)ᵣ₁-, -C₁₋₃ alkylene-NHS(O)ᵣ₂- or -C₁₋₃ alkylene-S(O)ᵣ₃NH-; the -NH-, -CONH-, C₁₋₃ alkylene, C₂₋₄ alkenylene, C₂₋₄ alkynylene, -O-C₁₋₃ alkylene-, -S-C₁₋₃ alkylene-, -C₁₋₃ alkylene-NH-, -C₁₋₃ alkylene-C(O)NH-, -C₁₋₃ alkylene-NHC(O)-, -C₁₋₃ alkylene-S(O)ᵣ₁-, -C₁₋₃ alkylene-NHS(O)ᵣ₂- or -C₁₋₃ alkylene-S(O)ᵣ₃NH- is optionally substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl;
R₁ is independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₘ₁ORₐ₁, -(CH₂)ₘ₂C(O)Rₐ₂, -(CH₂)ₘ₃NHC(O)Rₐ₃, - (CH₂)ₘ₄C(O)NHRₐ₄, -(CH₂)ₘ₅NRₐ₅Rₐ₆, -(CH₂)ₘ₆S(O)ₘ₇Rₐ₇, -(CH₂)ₘ₈S(O)₂NHRₐ₈ or -(CH₂)ₘ₉NHS(O)₂Rₐ₉, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5-to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, carboxyl, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl, wherein the C₃₋₈ cycloalkyl, 3- to 8-membered heterocyclyl, C₆₋₁₀ aryl and 5- to 10-membered heteroaryl substituents are optionally further substituted with one or more of hydrogen, deuterium, halogen, amino, hydroxyl, cyano, nitro, carboxyl, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
preferably, R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, carboxyl, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
preferably, R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
alternatively, R₂₋₁ and R₂₋₂ are connected to form C₃₋₈ cycloalkyl, 3- to 8-membered heterocyclyl, C₆₋₁₀ aryl or 5- to 10-membered heteroaryl, wherein the C₃₋₈ cycloalkyl, 3- to 8-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₃ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, - (CH₂)ₒ₁OR_{c1}, -(CH₂)ₒ₂C(O)R_{c2}, -(CH₂)ₒ₃NHC(O)R_{c3}, -(CH₂)ₒ₄C(O)NHR_{c4}, - (CH₂)ₒ₅NR_{c5}R_{c6}, -(CH₂)ₒ₆S(O)ₒ₇R_{c7}, -(CH₂)ₒ₈S(O)₂NHR_{c8} or -(CH₂)ₒ₉NHS(O)₂R_{c9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₄₋₁ and R₄₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CHR_{d10})ₚ₁OR_{d1}, -(CHR_{d10})ₚ₂C(O)R_{d2}, - (CHR_{d10})ₚ₃NHC(O)R_{d3}, -(CHR_{d10})ₚ₄C(O)NHR_{d4}, -(CHR_{d10})ₚ₅NR_{d5}R_{d6}, - (CHR_{d10})ₚ₆S(O)ₚ₇R_{d7}, -(CHR_{d10})ₚ₈S(O)₂NHR_{d8} or -(CHR_{d10})ₚ₉NHS(O)₂R_{d9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
alternatively, R₄₋₁ and R₄₋₂ are connected to form C₃₋₈ cycloalkyl, 3- to 8-membered heterocyclyl, C₆₋₁₀ aryl or 5- to 10-membered heteroaryl, wherein the C₃₋₈ cycloalkyl, 3- to 8-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₅ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, - (CH₂)_{q1}ORₑ₁, -(CH₂)_{q2}C(O)Rₑ₂, -(CH₂)_{q3}NHC(O)Rₑ₃, -(CH₂)_{q4}C(O)NHRₑ₄, - (CH₂)_{q5}NRₑ₅Rₑ₆, -(CH₂)_{q6}S(O)_{q7}Rₑ₇, -(CH₂)_{q8}S(O)₂NHRₑ₈ or -(CH₂)_{q9}NHS(O)₂Rₑ₉, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
Rₐ₁, Rₐ₂, Rₐ₃, Rₐ₄, Rₐ₅, Rₐ₆, Rₐ₇, Rₐ₈ and Rₐ₉ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{b1}, R_{b2}, R_{b3}, R_{b4}, R_{b5}, R_{b6}, R_{b7}, R_{b8} and R_{b9} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{c1}, R_{c2}, R_{c3}, R_{c4}, R_{c5}, R_{c6}, R_{c7}, R_{c8} and R_{c9} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{d1}, R_{d2}, R_{d3}, R_{d4}, R_{d5}, R_{d5}, R_{d7}, R_{d5}, R_{d9} and R_{d10} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
Rₑ₁, Rₑ₂, Rₑ₃, Rₑ₄, Rₑ₅, Rₑ₆, Rₑ₇, Rₑ₈ and Rₑ₉ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
x is 0, 1, 2, 3, 4 or 5; v is 1, 2, 3 or 4; u is 0, 1, 2, 3, 4 or 5;
m1, m2, m3, m4, m5, m6, m8 and m9 are each independently selected from 0, 1, 2, 3, 4, 5 or 6;
n1, n2, n3, n4, n5, n6, n8 and n9 are each independently selected from 0, 1, 2, 3, 4, 5 or 6;
o1, o2, o3, o4, o5, o6, o8 and o9 are each independently selected from 0, 1, 2, 3, 4, 5 or 6;
p1, p2, p3, p4, p5, p6, p8 and p9 are each independently selected from 0, 1, 2, 3, 4, 5 or 6;
q1, q2, q3, q4, q5, q6, q8 and q9 are each independently selected from 0, 1, 2, 3, 4, 5 or 6;
m7, n7, o7, p7 and q7 are each independently selected from 0, 1, 2 or 3;
r1, r2 and r3 are each independently selected from 0, 1 or 2.

In certain embodiments of the present invention, the compound as represented by formula (VII) is as represented by formula (VII-1) or formula (VII-2),

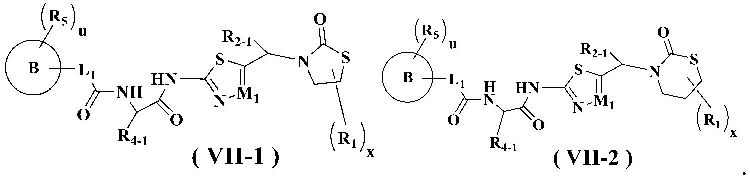

In certain embodiments of the present invention, the compound as represented by formula (VII) is as represented by formula (VII-1-1) or formula (VII-2-1),

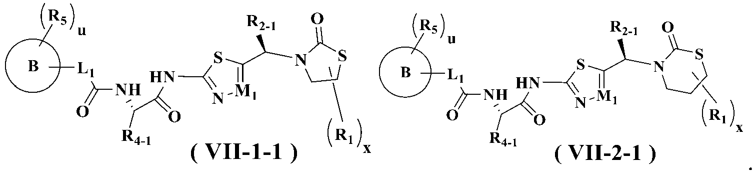

In certain embodiments of the present invention, the compound as represented by formula (I) is as represented by formula (VIII) or (IX), 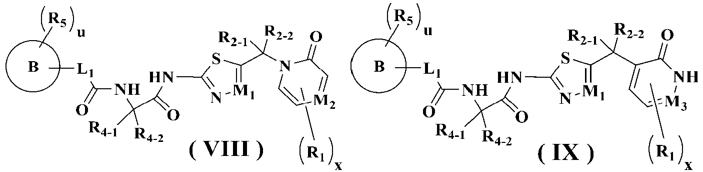 wherein
M₁ is selected from N or CR₃; M₂ is selected from N or CH; M₃ is selected from N or CH;
ring B is selected from C₃₋₁₂ cycloalkyl, 3- to 12-membered heterocyclyl, C₆₋₁₄ aryl or 5- to 14-membered heteroaryl; or ring B is absent, and 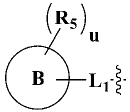 is **R₅**-**L₁** 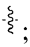
L₁ is selected from a bond, -O-, -NH-, -CO-, -CONH-, C₁₋₃ alkylene, C₂₋₄ alkenylene, C₂₋₄ alkynylene, -O-C₁₋₃ alkylene-, -S-C₁₋₃ alkylene-, -C₁₋₃ alkylene-NH-, -C₁₋₃ alkylene-C(O)NH-, -C₁₋₃ alkylene-NHC(O)-, -C₁₋₃ alkylene-S(O)ᵣ₁-, -C₁₋₃ alkylene-NHS(O)ᵣ₂- or -C₁₋₃ alkylene-S(O)ᵣ₃NH-; the -NH-, -CONH-, C₁₋₃ alkylene, C₂₋₄ alkenylene, C₂₋₄ alkynylene, -O-C₁₋₃ alkylene-, -S-C₁₋₃ alkylene-, -C₁₋₃ alkylene-NH-, -C₁₋₃ alkylene-C(O)NH-, -C₁₋₃ alkylene-NHC(O)-, -C₁₋₃ alkylene-S(O)ᵣ₁-, -C₁₋₃ alkylene-NHS(O)ᵣ₂- or -C₁₋₃ alkylene-S(O)ᵣ₃NH- is optionally substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl;
R₁ is independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₘ₁ORₐ₁, -(CH₂)ₘ₂C(O)Rₐ₂, -(CH₂)ₘ₃NHC(O)Rₐ₃, - (CH₂)ₘ₄C(O)NHRₐ₄, -(CH₂)ₘ₅NRₐ₅Rₐ₆, -(CH₂)ₘ₆S(O)ₘ₇Rₐ₇, -(CH₂)ₘ₈S(O)₂NHRₐ₈ or -(CH₂)ₘ₉NHS(O)₂Rₐ₉, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5-to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, carboxyl, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl, wherein the C₃₋₈ cycloalkyl, 3- to 8-membered heterocyclyl, C₆₋₁₀ aryl and 5- to 10-membered heteroaryl substituents are optionally further substituted with one or more of hydrogen, deuterium, halogen, amino, hydroxyl, cyano, nitro, carboxyl, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
preferably, R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, carboxyl, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
preferably, R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
alternatively, R₂₋₁ and R₂₋₂ are connected to form C₃₋₈ cycloalkyl, 3- to 8-membered heterocyclyl, C₆₋₁₀ aryl or 5- to 10-membered heteroaryl, wherein the C₃₋₈ cycloalkyl, 3- to 8-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₃ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, - (CH₂)ₒ₁OR_{c1}, -(CH₂)ₒ₂C(O)R_{c2}, -(CH₂)ₒ₃NHC(O)R_{c3}, -(CH₂)ₒ₄C(O)NHR_{c4}, - (CH₂)ₒ₅NR_{c5}R_{c6}, -(CH₂)ₒ₆S(O)ₒ₇R_{c7}, -(CH₂)ₒ₈S(O)₂NHR_{c8} or -(CH₂)ₒ₉NHS(O)₂R_{c9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₄₋₁ and R₄₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CHR_{d10})ₚ₁OR_{d1}, -(CHR_{d10})ₚ₂C(O)R_{d2}, - (CHR_{d10})ₚ₃NHC(O)R_{d3}, -(CHR_{d10})ₚ₄C(O)NHR_{d4}, -(CHR_{d10})ₚ₅NR_{d5}R_{d6}, - (CHR_{d10})ₚ₆S(O)ₚ₇R_{d7}, -(CHR_{d10})ₚ₈S(O)₂NHR_{d8} or (CHR_{d10})ₚ₉NHS(O)₂R_{d9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
alternatively, R₄₋₁ and R₄₋₂ are connected to form C₃₋₈ cycloalkyl, 3- to 8-membered heterocyclyl, C₆₋₁₀ aryl or 5- to 10-membered heteroaryl, wherein the C₃₋₈ cycloalkyl, 3- to 8-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₅ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, - (CH₂)_{q1}ORₑ₁, -(CH₂)_{q2}C(O)Rₑ₂, -(CH₂)_{q3}NHC(O)Rₑ₃, -(CH₂)_{q4}C(O)NHRₑ₄, - (CH₂)_{q5}NRₑ₅Rₑ₆, -(CH₂)_{q6}S(O)_{q7}Rₑ₇, -(CH₂)_{q8}S(O)₂NHRₑ₈ or -(CH₂)_{q9}NHS(O)₂Rₑ₉, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
Rₐ₁, Rₐ₂, Rₐ₃, Rₐ₄, R_{d5}, Rₐ₆, Rₐ₇, Rₐ₈ and R_{d9} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{b1}, R_{b2}, R_{b3}, R_{b4}, R_{b5}, R_{b6}, R_{b7}, R_{b8} and R_{b9} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{c1}, R_{c2}, R_{c3}, R_{c4}, R_{c5}, R_{c6}, R_{c7}, R_{c8} and R_{c9} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{d1}, R_{d2}, R_{d3}, R_{d4}, R_{d5}, R_{d5}, R_{d7}, R_{d8}, R_{d9} and R_{d10} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
Rₑ₁, Rₑ₂, Rₑ₃, Rₑ₄, Rₑ₅, Rₑ₆, Rₑ₇, Rₑ₈ and Rₑ₉ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
x is 0, 1, 2, 3, 4 or 5; u is 0, 1, 2, 3, 4 or 5;
m1, m2, m3, m4, m5, m6, m8 and m9 are each independently selected from 0, 1, 2, 3, 4, 5 or 6;
n1, n2, n3, n4, n5, n6, n8 and n9 are each independently selected from 0, 1, 2, 3, 4, 5 or 6;
o1, o2, o3, o4, o5, o6, o8 and o9 are each independently selected from 0, 1, 2, 3, 4, 5 or 6;
p1, p2, p3, p4, p5, p6, p8 and p9 are each independently selected from 0, 1, 2, 3, 4, 5 or 6;
q1, q2, q3, q4, q5, q6, q8 and q9 are each independently selected from 0, 1, 2, 3, 4, 5 or 6;
m7, n7, o7, p7 and q7 are each independently selected from 0, 1, 2 or 3;
r1, r2 and r3 are each independently selected from 0, 1 or 2.

In certain embodiments of the present invention, the compound as represented by formula (VIII) or (IX) is as represented by formula (VIII-1) or formula (IX-1),

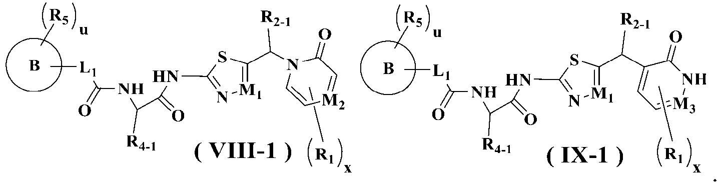

In certain embodiments of the present invention, the compound as represented by formula (VIII-1) or formula (IX-1) is as represented by formula (VIII-1-1) or formula (IX-1-1),

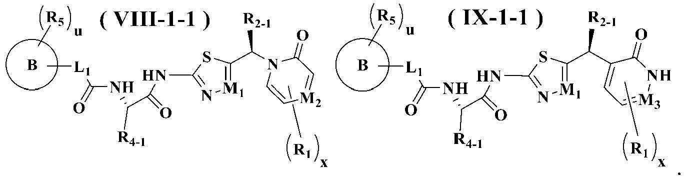

In certain embodiments of the present invention, the compound is as represented by formula (I-AAA) 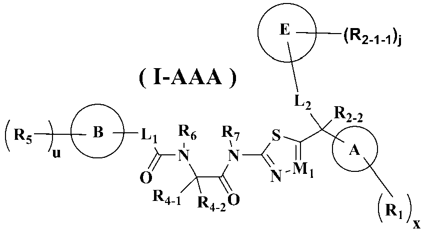
L₂ is selected from a bond, -O-, -NH-, -CO-, -CONH-, C₁₋₃ alkylene, C₂₋₄ alkenylene, C₂₋₄ alkynylene, -O-C₁₋₃ alkylene-, -S-C₁₋₃ alkylene-, -C₁₋₃ alkylene-NH-, -C₁₋₃ alkylene-C(O)NH-, -C₁₋₃ alkylene-NHC(O)-, -C₁₋₃ alkylene-S(O)ᵣ₄-, -C₁₋₃ alkylene-NHS(O)ᵣ₅- or -C₁₋₃ alkylene-S(O)ᵣ₆NH-; the -NH-, -CONH-, C₁₋₃ alkylene, C₂₋₄ alkenylene, C₂₋₄ alkynylene, -O-C₁₋₃ alkylene-, -S-C₁₋₃ alkylene-, -C₁₋₃ alkylene-NH-, -C₁₋₃ alkylene-C(O)NH-, -C₁₋₃ alkylene-NHC(O)-, -C₁₋₃ alkylene-S(O)ᵣ₄-, -C₁₋₃ alkylene-NHS(O)ᵣ₅- or -C₁₋₃ alkylene-S(O)ᵣ₆NH- is optionally substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl;
ring E is selected from C₃₋₁₂ cycloalkyl, 3- to 12-membered heterocyclyl, C₆₋₁₀ aryl, or 5- to 10-membered heteroaryl, preferably 3- to 12-membered heterocyclyl;
each R₂₋₁₋₁ is independently selected from hydrogen, deuterium, halogen, amino, hydroxyl, cyano, nitro, carboxyl, oxo, thio, =CF₂, =CHF, =NOC₁₋₆ alkyl, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ heteroalkyl, C₁₋₆ deuteroheteroalkyl, C₁₋₆ haloheteroalkyl, C₁₋₆ hydroxyalkyl, C₃₋₈ cycloalkyl, 3- to 8-membered heterocyclyl, C₆₋₁₀ aryl or 5- to 10-membered heteroaryl, wherein the C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ heteroalkyl, C₁₋₆ deuteroheteroalkyl, C₁₋₆ haloheteroalkyl, C₁₋₆ hydroxyalkyl, C₃₋₈ cycloalkyl, 3- to 8-membered heterocyclyl, C₆₋₁₀ aryl and 5- to 10-membered heteroaryl are optionally further substituted with one or more of hydrogen, deuterium, halogen, amino, hydroxyl, cyano, nitro, carboxyl, oxo, thio, =CF₂, =CHF, =NOC₁₋₆ alkyl, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ heteroalkyl, C₁₋₆ deuteroheteroalkyl, C₁₋₆ haloheteroalkyl, C₁₋₆ hydroxyalkyl, C₃₋₈ cycloalkyl, 3- to 8-membered heterocyclyl, C₆₋₁₀ aryl and 5- to 10-membered heteroaryl, and R₂₋₁₋₁ is preferably methyl, ethyl, methoxy, F, =CF₂, -CHF₂, -CH₂F, -CF₃, -CH₂CHF₂, - CHF₂CH₃, -CH₂CF₃, -CH₂OCH₃, -CH₂OCD₃, -CH₂OCF₃, -CH₂O cyclopropyl, - CH₂SCF₃, cyclopropyl, cyclobutyl, OCD₃ or OCF₃;
j is 0, 1, 2, 3, 4 or 5; r4, r5 and r6 are each independently selected from 0, 1 or 2;
v is 1, 2, 3 or 4.

In certain embodiments of the present invention, the compound is as represented by formula (I-AAA): 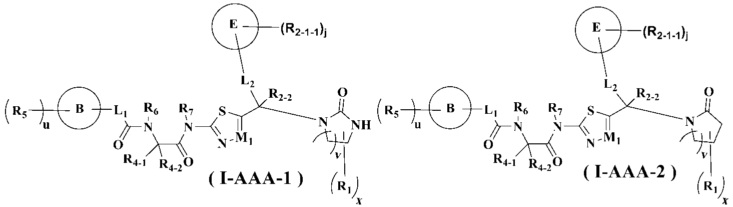 v is 1, 2, 3 or 4.

In certain embodiments of the present invention, the compound is as represented by formula (I-AA-1) or (I-AA-2): 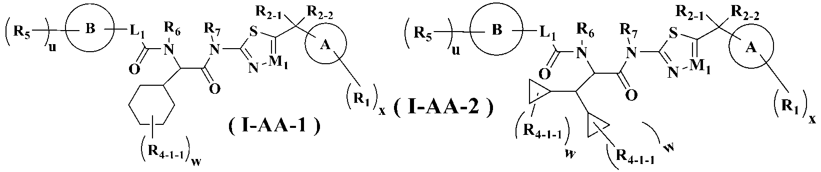 wherein R₄₋₁₋₁ is independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl; w is selected from 0, 1, 2, 3, 4, 5 or 6.

In certain embodiments of the present invention, ring B is selected from C₃₋₆ cycloalkyl, 3- to 8-membered heterocyclyl, C₆₋₁₀ aryl or 5- to 10-membered heteroaryl; or ring B is absent; preferably, ring B is selected from C₃₋₆ cycloalkyl, phenyl or 5- to 6-membered heteroaryl; or ring B is absent; more preferably, ring B is selected from 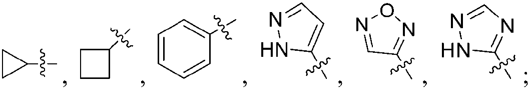 or ring B is absent; more preferably, ring B is selected from 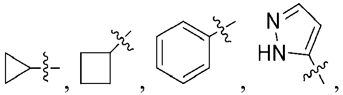 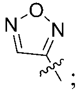 or ring B is absent. In certain embodiments of the present invention, ring B is 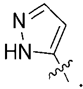 In certain embodiments of the present invention, ring B is

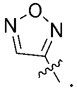

In certain embodiments of the present invention, the compound is as represented by formula (I-AA), (I-AA-3) or (I-AA-4): 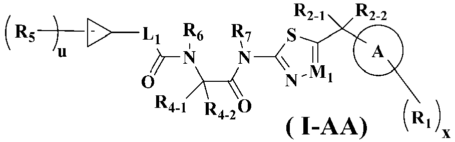 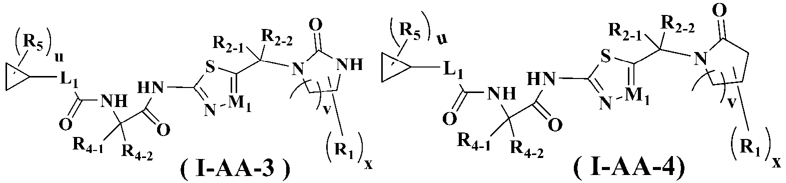 v is 1, 2, 3 or 4.

In certain embodiments of the present invention, L₁ is selected from a bond, O or-CF₂-. In certain embodiments of the present invention, L₁ is a bond. In certain embodiments of the present invention, L₁ is O. In certain embodiments of the present invention, L₁ is selected from a bond or-CF₂-.

In certain embodiments of the present invention, R is

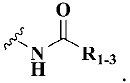

In certain embodiments of the present invention, the compound as represented by formula (I-A) is as represented by formula (VI): 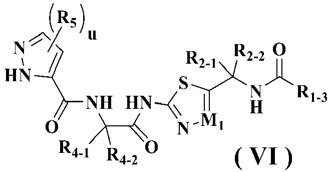 wherein M₁ is selected from N or CR₃;
R₁₋₃ is independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₘ₁ORₐ₁, -(CH₂)ₘ₂C(O)Rₐ₂, -(CH₂)ₘ₃NHC(O)Rₐ₃, - (CH₂)ₘ₄C(O)NHRₐ₄, -(CH₂)ₘ₅NRₐ₅Rₐ₆, -(CH₂)ₘ₆S(O)ₘ₇Rₐ₇, -(CH₂)ₘ₈S(O)₂NHRₐ₈, - (CH₂)ₘ₉NHS(O)₂Rₐ₉ or -(CH₂)ₘ₁₀Rₐ₁₀, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl and 5- to 10-membered heteroaryl;
R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, carboxyl, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl, wherein the C₃₋₈ cycloalkyl, 3- to 8-membered heterocyclyl, C₆₋₁₀ aryl and 5- to 10-membered heteroaryl substituents are optionally further substituted with one or more of hydrogen, deuterium, halogen, amino, hydroxyl, cyano, nitro, carboxyl, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
preferably, R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, carboxyl, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
preferably, R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
alternatively, R₂₋₁ and R₂₋₂ are connected to form C₃₋₈ cycloalkyl, 3- to 8-membered heterocyclyl, C₆₋₁₀ aryl or 5- to 10-membered heteroaryl, wherein the C₃₋₈ cycloalkyl, 3- to 8-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₃ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, - (CH₂)ₒ₁OR_{c1}, -(CH₂)ₒ₂C(O)R_{c2}, -(CH₂)ₒ₃NHC(O)R_{c3}, -(CH₂)ₒ₄C(O)NHR_{c4}, - (CH₂)ₒ₅NR_{c5}R_{c6}, -(CH₂)ₒ₆S(O)ₒ₇R_{c7}, -(CH₂)ₒ₈S(O)₂NHR_{c8} or -(CH₂)ₒ₉NHS(O)₂R_{c9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₄₋₁ and R₄₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CHR_{d10})ₚ₁OR_{d1}, -(CHR_{d10})ₚ₂C(O)R_{d2}, - (CHR_{d10})ₚ₃NHC(O)R_{d3}, -(CHR_{d10})ₚ₄C(O)NHR_{d4}, -(CHR_{d10})ₚ₅NR_{d5}R_{d6}, - (CHR_{d10})ₚ₆S(O)ₚ₇R_{d7}, -(CHR_{d10})ₚ₈S(O)₂NHR_{d8} or -(CHR_{d10})ₚ₉NHS(O)₂R_{d9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
preferably, R₄₋₁ and R₄₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₚ₁OR_{d1}, -(CH₂)ₚ₂C(O)R_{d2}, - (CH₂)ₚ₃NHC(O)R_{d3}, -(CH₂)ₚ₄C(O)NHR_{d4}, -(CH₂)ₚ₅NR_{d5}R_{d6}, -(CH₂)ₚ₆S(O)ₚ₇R_{d7}, - (CH₂)ₚ₈S(O)₂NHR_{d8} or -(CH₂)ₚ₉NHS(O)₂R_{d9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
alternatively, R₄₋₁ and R₄₋₂ are connected to form C₃₋₈ cycloalkyl, 3- to 8-membered heterocyclyl, C₆₋₁₀ aryl or 5- to 10-membered heteroaryl, wherein the C₃₋₈ cycloalkyl, 3- to 8-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₅ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, - (CH₂)_{q1}ORₑ₁, -(CH₂)_{q2}C(O)Rₑ₂, -(CH₂)_{q3}NHC(O)Rₑ₃, -(CH₂)_{q4}C(O)NHRₑ₄, - (CH₂)_{q5}NRₑ₅Rₑ₆, -(CH₂)_{q6}S(O)_{q7}Rₑ₇, -(CH₂)_{q8}S(O)₂NHRₑ₈ or -(CH₂)_{q9}NHS(O)₂Rₑ₉, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
Rₐ₁, Rₐ₂, Rₐ₃, Rₐ₄, Rₐ₅, Rₐ₆, Rₐ₇, Rₐ₈, Rₐ₉ and Rₐ₁₀ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{b1}, R_{b2}, R_{b3}, R_{b4}, R_{b5}, R_{b6}, R_{b7}, R_{b8} and R_{b9} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{c1}, R_{c2}, R_{c3}, R_{c4}, R_{c5}, R_{c6}, R_{c7}, R_{c8} and R_{c9} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{d1}, R_{d2}, R_{d3}, R_{d4}, Rₐ₅, R_{d6}, R_{d7}, R_{d8}, R_{d9} and R_{d10} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
Rₑ₁, Rₑ₂, Rₑ₃, Rₑ₄, Rₑ₅, Rₑ₆, Rₑ₇, Rₑ₈ and Rₑ₉ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
x is 0, 1, 2, 3, 4 or 5; u is 0, 1, 2, 3, 4 or 5;
m1, m2, m3, m4, m5, m6, m8, m9 and m10 are each independently selected from 0, 1, 2, 3, 4, 5 or 6;
n1, n2, n3, n4, n5, n6, n8 and n9 are each independently selected from 0, 1, 2, 3, 4, 5 or 6;
o1, o2, o3, o4, o5, o6, o8 and o9 are each independently selected from 0, 1, 2, 3, 4, 5 or 6;
p1, p2, p3, p4, p5, p6, p8 and p9 are each independently selected from 0, 1, 2, 3, 4, 5 or 6;
q1, q2, q3, q4, q5, q6, q8 and q9 are each independently selected from 0, 1, 2, 3, 4, 5 or 6;
m7, n7, o7, p7 and q7 are each independently selected from 0, 1, 2 or 3.

In certain embodiments of the present invention, the compound as represented by formula (VI) is as represented by formula (VI-1), and in certain embodiments of the present invention, the compound as represented by formula (VI-1) is as represented by formula (VI-1-1):

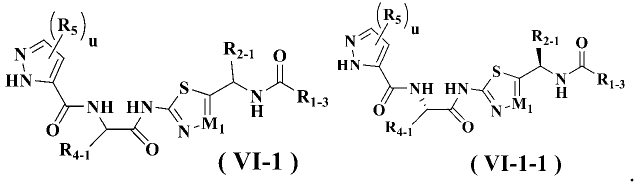

In certain embodiments of the present invention, R₁ is independently selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl, 5- to 6-membered heteroaryl, -(CH₂)ₘ₁ORₐ₁, - (CH₂)ₘ₂C(O)Rₐ₂, -(CH₂)ₘ₃NHC(O)Rₐ₃, -(CH₂)ₘ₄C(O)NHRₐ₄, -(CH₂)ₘ₅NRₐ₅Rₐ₆, - (CH₂)ₘ₆S(O)ₘ₇Rₐ₇, -(CH₂)ₘ₈S(O)₂NHRₐ₈ or -(CH₂)ₘ₉NHS(O)₂Rₐ₉, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3-to 6-membered heterocyclyl, C₆₋₁₀ aryl or 5- to 6-membered heteroaryl may optionally be further substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl;
Rₐ₁, Rₐ₂, Rₐ₃, Rₐ₄, Rₐ₅, Rₐ₆, Rₐ₇, Rₐ₈ and Rₐ₉ are each independently selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3-to 6-membered heterocyclyl, C₆₋₁₀ aryl or 5- to 6-membered heteroaryl may optionally be further substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl;
preferably, R₁ is hydrogen, deuterium, oxo, trifluoromethyl, fluoro, chloro, bromo, -CH₂CF₃, cyano, methoxy, -CH=CF₂ or -CH₂CHF₂; preferably, R₁ is hydrogen, deuterium, oxo, trifluoromethyl, fluoro, chloro, -CH₂CF₃, cyano, methoxy, -CH=CF₂ or -CH₂CHF₂; preferably, R₁ is hydrogen, deuterium, oxo, trifluoromethyl, fluoro, -CH₂CF₃, cyano, methoxy, -CH=CF₂ or -CH₂CHF₂; more preferably, R₁ is oxo, trifluoromethyl, fluoro, -CH₂CF₃ or cyano.

In certain embodiments of the present invention, R₂₋₁ and R₂₋₂ are each independently selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl, 5- to 6-membered heteroaryl, - (CH₂)ₙ₁OR_{b1}, -(CH₂)ₙ₂C(O)R_{b2}, -(CH₂)ₙ₃NHC(O)R_{b3}, -(CH₂)ₙ₄C(O)NHR_{b4}, - (CH₂)ₙ₅NR_{b5}R_{b6}, -(CH2)ₙ₆S(O)ₙ₇R_{b7}, -(CH2)ₙ₈S(O)₂NHR_{b8} or -(CH₂)ₙ₉NHS(O)₂R_{b9}, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl may optionally be further substituted with one or more of hydrogen, deuterium, halogen, amino, hydroxyl, cyano, nitro, carboxyl, oxo, thio, =CF₂, =CHF, =NOC₁₋₃ alkyl, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3-to 6-membered heterocyclyl, C₆₋₁₀ aryl and 5- to 6-membered heteroaryl, wherein the C₃₋₆ cycloalkyl, 3- to 6-membered heterocyclyl, C₆₋₁₀ aryl and 5- to 6-membered heteroaryl substituents are optionally further substituted with one or more of hydrogen, deuterium, halogen, amino, hydroxyl, cyano, nitro, carboxyl, oxo, thio, =CF₂, =CHF, =NOC₁₋₃ alkyl, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ heteroalkyl, C₁₋₃ deuteroheteroalkyl, C₁₋₃ haloheteroalkyl, C₁₋₃ hydroxyalkyl, C₃₋₆ cycloalkyl, 3- to 6-membered heterocyclyl, C₆₋₁₀ aryl and 5- to 6-membered heteroaryl.

In certain embodiments of the present invention, R₂₋₁ and R₂₋₂ are each independently selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl, 5- to 6-membered heteroaryl, - (CH₂)ₙ₁OR_{b1}, -(CH₂)ₙ₂C(O)R_{b2}, -(CH₂)ₙ₃NHC(O)R_{b3}, -(CH₂)ₙ₄C(O)NHR_{b4}, - (CH₂)ₙ₅NR_{b5}R_{d6}, -(CH2)ₙ₆S(O)ₙ₇R_{b7}, -(CH2)ₙ₈S(O)₂NHR_{b8} or -(CH₂)ₙ₉NHS(O)₂R_{b9}, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl may optionally be further substituted with one or more of hydrogen, deuterium, halogen, amino, hydroxyl, cyano, nitro, carboxyl, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl, wherein the C₃₋₆ cycloalkyl, 3- to 6-membered heterocyclyl, C₆₋₁₀ aryl and 5- to 6-membered heteroaryl substituents are optionally further substituted with one or more of hydrogen, deuterium, halogen, amino, hydroxyl, cyano, nitro, carboxyl, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl.

In certain embodiments of the present invention, R₂₋₁ and R₂₋₂ are each independently selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl, 5- to 6-membered heteroaryl, - (CH₂)ₙ₁OR_{b1}, -(CH₂)ₙ₂C(O)R_{b2}, -(CH₂)ₙ₃NHC(O)R_{b3}, -(CH₂)ₙ₄C(O)NHR_{b4}, - (CH₂)ₙ₅NR_{b5}R_{b6}, -(CH2)ₙ₆S(O)ₙ₇R_{b7}, -(CH2)ₙ₈S(O)₂NHR_{b8} or -(CH₂)ₙ₉NHS(O)₂R_{b9}, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl may optionally be further substituted with one or more of hydrogen, deuterium, halogen, amino, hydroxyl, cyano, nitro, carboxyl, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl;
preferably, R₂₋₁ and R₂₋₂ are each independently selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl, 5- to 6-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH2)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl may optionally be further substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl;
alternatively, R₂₋₁ and R₂₋₂ are connected to form C₃₋₈ cycloalkyl, 3- to 8-membered heterocyclyl, C₆₋₁₀ aryl or 5- to 10-membered heteroaryl, wherein the C₃₋₈ cycloalkyl, 3- to 8-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl;
R_{b1}, R_{b2}, R_{b3}, R_{b4}, R_{b5}, R_{b6}, R_{b7}, R_{b8} and R_{b9} are each independently selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3-to 6-membered heterocyclyl, C₆₋₁₀ aryl or 5- to 6-membered heteroaryl may optionally be further substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl.

In certain embodiments of the present invention, the C₃₋₆ cycloalkyl, 3- to 6-membered heterocyclyl, C₆₋₁₀ aryl and 5- to 6-membered heteroaryl substituents for the groups described in each general formula may optionally be further substituted with one or more of hydrogen, deuterium, halogen, amino, hydroxyl, cyano, nitro, carboxyl, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl.

In certain embodiments of the present invention, R₂₋₁ is 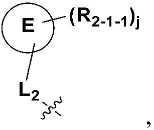 and preferably ring E is 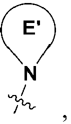 wherein ring E' is selected from 3- to 12-membered heterocyclyl, preferably 3- to 8-membered monocyclic heterocyclyl, 3- to 8-membered fused heterocyclyl, 3- to 8-membered bridged heterocyclyl or 3- to 8-membered spiroheterocyclyl, more preferably 3- to 8-membered monocyclic heterocyclyl, 3- to 8-membered fused heterocyclyl or 3- to 8-membered spiroheterocyclyl, more preferably 5- to 9-membered heterocyclyl, more preferably 5- to 6-membered heterocyclyl; preferably, ring E' is selected from

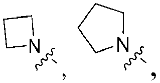

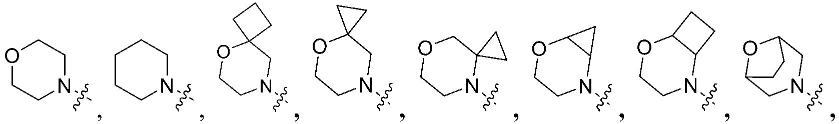

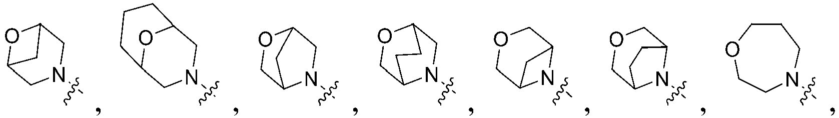

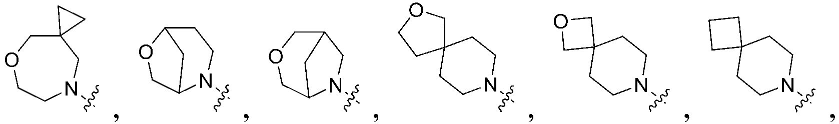

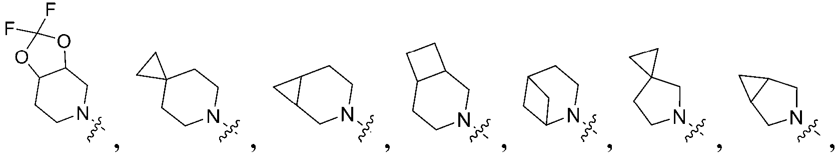

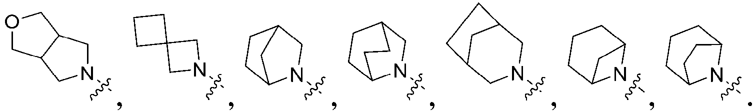

In certain embodiments of the present invention, L₂ is CH₂.

In certain embodiments of the present invention, R₂₋₁ is selected from 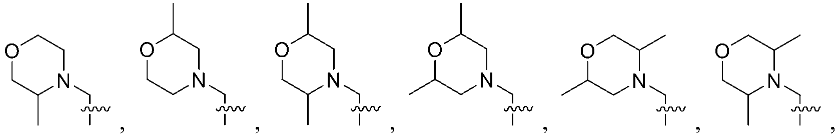 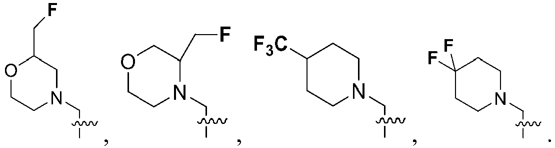 In certain embodiments of the present invention, preferably, R₂₋₁ is selected from hydrogen, methyl, 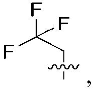 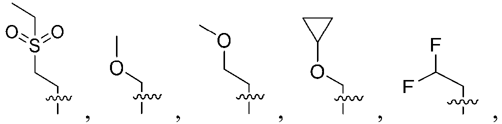 cyclopropyl, -CH=CF₂, -CH₂- cyclopropyl, -OCH₂OCD₃, -CH₂OCD₃, -CH₂OCHF₂, -CH₂COOCH₂CH₃, - CH₂COOH, -CH₂CH₂COOH, CH₂N (CH₃)₂, 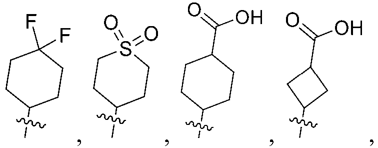 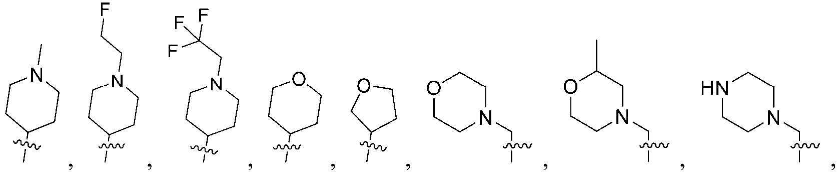 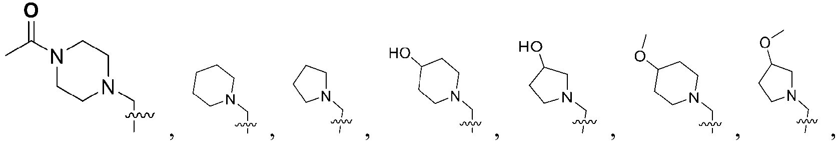 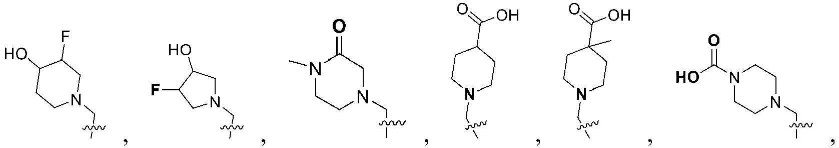 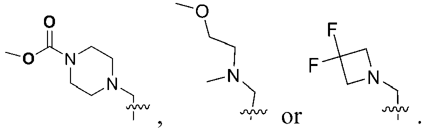 In certain embodiments of the present invention, R₂₋₁ is selected from hydrogen, methyl, 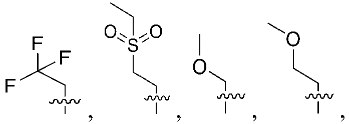 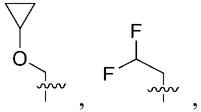 cyclopropyl, -CH=CF₂, -CH₂-cyclopropyl, -OCH₂OCD₃, - CH₂OCD₃, -CH₂OCHF₂, -CH₂COOCH₂CH₃, -CH₂COOH, -CH₂CH₂COOH, CH₂N (CH₃)₂, 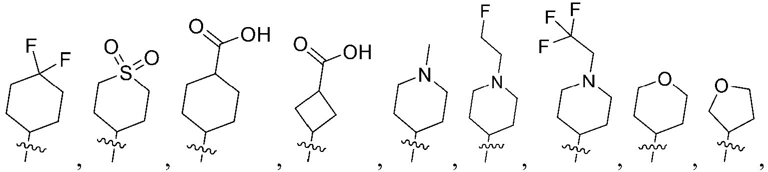 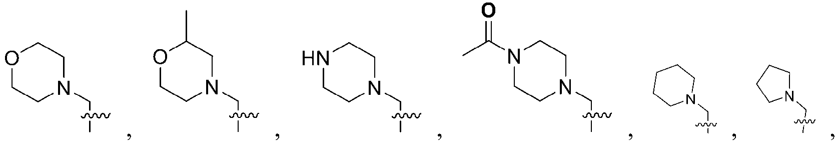 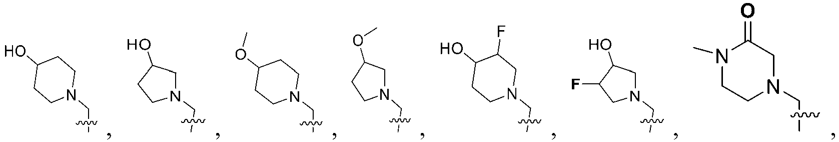 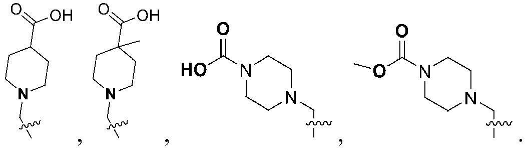 In certain embodiments of the present invention, R₂₋₁ is selected from hydrogen, methyl, 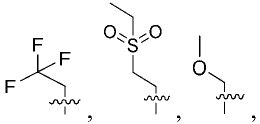 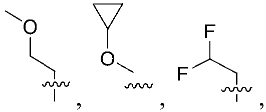 cyclopropyl, -CH=CF₂, -CH₂-cyclopropyl, -OCH₂OCD₃, -CH₂OCD₃, -CH₂OCHF₂, -CH₂COOCH₂CH₃, -CH₂COOH, -CH₂CH₂COOH, 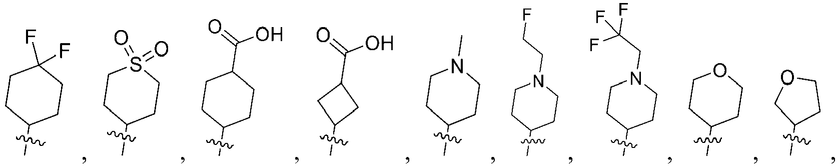 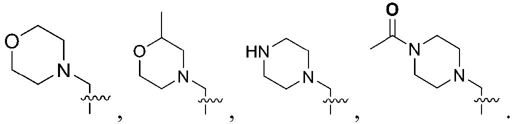 In certain embodiments of the present invention, R₂₋₁ is selected from hydrogen, methyl, 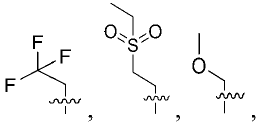 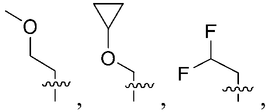 cyclopropyl, -CH=CF₂, -CH₂-cyclopropyl, -OCH₂OCD₃, -CH₂OCD₃, -CH₂OCHF₂, -CH₂COOCH₂CH₃, -CH₂COOH, -CH₂CH₂COOH,

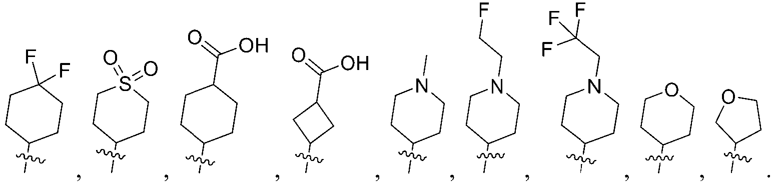

In certain embodiments of the present invention, R₂₋₁ is selected from hydrogen, methyl, 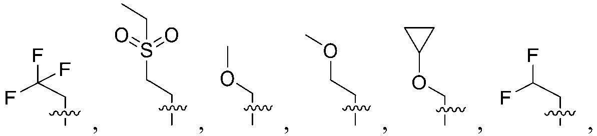 cyclopropyl, - CH=CF₂, -CH₂-cyclopropyl, -OCH₂OCD₃, -CH₂OCD₃ or -CH₂OCHF₂. In certain embodiments of the present invention, R₂₋₁ is selected from hydrogen, methyl, 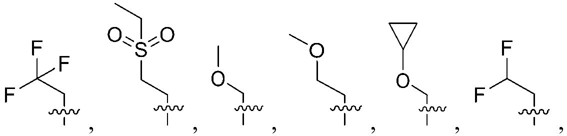 cyclopropyl, -CH=CF₂, - CH₂-cyclopropyl, -OCH₂OCD₃ or -CH₂OCD₃. In certain embodiments of the present invention, R₂₋₁ is selected from hydrogen, methyl, 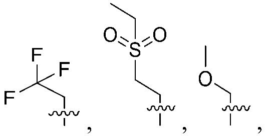 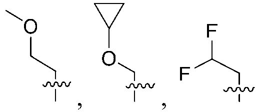 or cyclopropyl.

In certain embodiments of the present invention, R₂₋₂ is hydrogen. In certain embodiments of the present invention, R₂₋₂ is selected from hydrogen or methyl.

In certain embodiments of the present invention, R₃ is independently selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl, 5- to 6-membered heteroaryl, -(CH₂)ₒ₁OR_{c1}, - (CH₂)ₒ₂C(O)R_{c2}, -(CH₂)ₒ₃NHC(O)R_{c3}, -(CH₂)ₒ₄C(O)NHR_{c4}, -(CH₂)ₒ₅NR_{c5}R_{c6}, - (CH₂)ₒ₆S(O)ₒ₇R_{c7}, -(CH₂)ₒ₈S(O)₂NHR_{c8} or -(CH₂)ₒ₉NHS(O)₂R_{c9}, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl may optionally be further substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl;
R_{c1}, R_{c2}, R_{c3}, R_{c4}, R_{c5}, R_{c6}, R_{c7}, R_{c8} and R_{c9} are each independently selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3-to 6-membered heterocyclyl, C₆₋₁₀ aryl or 5- to 6-membered heteroaryl may optionally be further substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl;
preferably, R₃ is independently hydrogen.

In certain embodiments of the present invention, R₄₋₁ and R₄₋₂ are each independently selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₈ cycloalkyl, 3- to 6-membered heterocyclyl, C₆₋₁₀ aryl, 5- to 6-membered heteroaryl, - (CHR_{d10})ₚ₁0R_{d1}, -(CHR_{d10})ₚ₂C(O)R_{d2}, -(CHR_{d10})ₚ₃NHC(O)R_{d3}, - (CHR_{d10})ₚ₄C(O)NHR_{d4}, -(CHR_{d10})ₚ₅NR_{d5}R_{d6}, -(CHR_{d10})ₚ₆S(O)ₚ₇R_{d7}, - (CHR_{d10})ₚ₈S(O)₂NHR_{d8} or -(CHR_{d10})ₚ₉NHS(O)₂R_{d9}, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₈ cycloalkyl, 3- to 6-membered heterocyclyl, C₆₋₁₀ aryl or 5- to 6-membered heteroaryl may optionally be further substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl;
preferably, R₄₋₁ and R₄₋₂ are each independently selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₈ cycloalkyl, 3- to 6-membered heterocyclyl, C₆₋₁₀ aryl, 5- to 6-membered heteroaryl, -(CH₂)ₚ₁OR_{d1}, -(CH₂)ₚ₂C(O)R_{d2}, - (CH₂)ₚ₃NHC(O)R_{d3}, -(CH₂)ₚ₄C(O)NHR_{d4}, -(CH₂)ₚ₅NR_{d5}R_{d6}, -(CH₂)ₚ₆S(O)ₚ₇R_{d7}, - (CH₂)ₚ₈S(O)₂NHR_{d8} or -(CH₂)ₚ₉NHS(O)₂R_{d9}, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₈ cycloalkyl, 3- to 6-membered heterocyclyl, C₆₋₁₀ aryl or 5- to 6-membered heteroaryl may optionally be further substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl;
alternatively, R₄₋₁ and R₄₋₂ are connected to form C₃₋₈ cycloalkyl, 3- to 8-membered heterocyclyl, C₆₋₁₀ aryl or 5- to 10-membered heteroaryl, wherein the C₃₋₈ cycloalkyl, 3- to 8-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl;
R_{d1}, R_{d2}, R_{d3}, R_{d4}, R_{d5}, R_{d6}, R_{d7}, R_{d8} and R_{d9} are each independently selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3-to 6-membered heterocyclyl, C₆₋₁₀ aryl or 5- to 6-membered heteroaryl may optionally be further substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl.

In certain embodiments of the present invention, R₄₋₁ is selected from 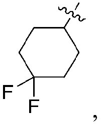 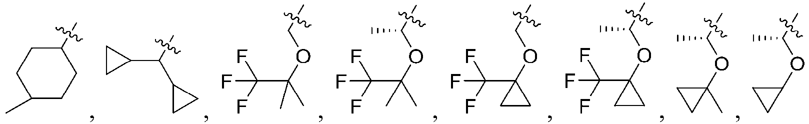 or 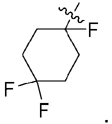 In certain embodiments of the present invention, R₄₋₁ is selected from 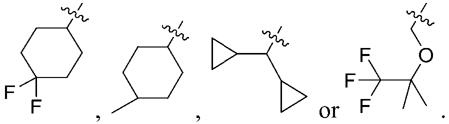 In certain embodiments of the present invention, R₄₋₁ is selected from 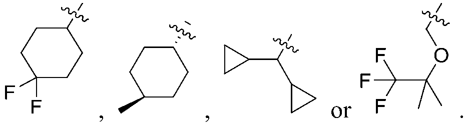 In certain embodiments of the present invention, R₄₋₁ is selected from 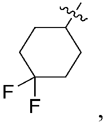 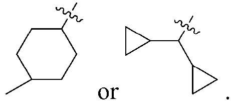 In certain embodiments of the present invention, R₄₋₁ is selected from 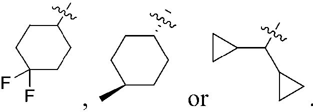 In certain embodiments of the present invention, R₄₋₁ is 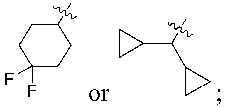 or in certain embodiments of the present invention, R₄₋₁ is 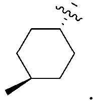 In certain embodiments of the present invention, R₄₋₁ is 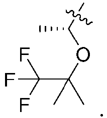 In certain embodiments of the present invention, R₄₋₁ is 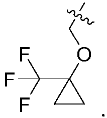 In certain embodiments of the present invention, R₄₋₁ is 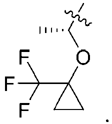 In certain embodiments of the present invention, R₄₋₁ is 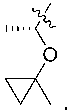 In certain embodiments of the present invention, R₄₋₁ is 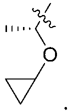 In certain embodiments of the present invention, R4_1 is

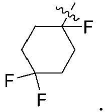

In certain embodiments of the present invention, R₄₋₂ is hydrogen.

In certain embodiments of the present invention, R₅ is selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl, 5- to 6-membered heteroaryl, -(CH₂)_{q1}ORₑ₁, -(CH₂)_{q2}C(O)Rₑ₂, - (CH₂)_{q3}NHC(O)Rₑ₃, -(CH₂)_{q4}C(O)NHRₑ₄, -(CH₂)_{q5}NRₑ₅Rₑ₆, -(CH₂)_{q6}S(O)_{q7}Rₑ₇, - (CH₂)_{q8}S(O)₂NHRₑ₈ or -(CH₂)_{q9}NHS(O)₂Rₑ₉, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl may optionally be further substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl;
Rₑ₁, Rₑ₂, Rₑ₃, Rₑ₄, Rₑ₅, Rₑ₆, Rₑ₇, Rₑ₈ and Rₑ₉ are each independently selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3-to 6-membered heterocyclyl, C₆₋₁₀ aryl or 5- to 6-membered heteroaryl may optionally be further substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl;
preferably, R₅ is selected from hydrogen, fluoro, methyl, isopropyl, 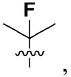 trifluoromethyl, monofluoromethyl, cyano, cyclopropyl or ethyl; more preferably, R₅ is selected from hydrogen, fluoro, methyl, isopropyl, 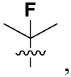 trifluoromethyl, monofluoromethyl or cyano. In certain embodiments of the present invention, preferably, R₅ is selected from hydrogen, fluoro, methyl, isopropyl, 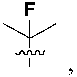 trifluoromethyl, monofluoromethyl, cyano, cyclopropyl, ethyl, deuterated ethyl, deuterated methyl, cyclobutyl, cyclopentyl, cyclohexyl,

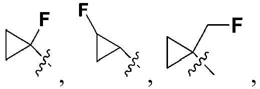

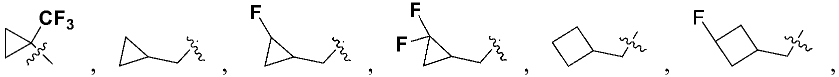

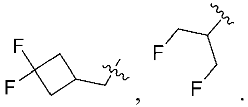

In certain embodiments of the present invention, R₄₋₁₋₁ is independently selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3-to 6-membered heterocyclyl, C₆₋₁₀ aryl and 5- to 6-membered heteroaryl;
preferably, R₄₋₁₋₁ is independently selected from hydrogen, fluoro or methyl.

In certain embodiments of the present invention, R₁₋₁ or R₁₋₂ is each independently hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3-to 6-membered heterocyclyl, C₆₋₁₀ aryl, 5- to 6-membered heteroaryl, -(CH₂)ₘ₁ORₐ₁, -(CH₂)ₘ₂C(O)Rₐ₂, -(CH₂)ₘ₃NHC(O)Rₐ₃, -(CH₂)ₘ₄C(O)NHRₐ₄, -(CH₂)ₘ₅NRₐ₅Rₐ₆, - (CH₂)ₘ₆S(O)ₘ₇Rₐ₇, -(CH₂)ₘ₈S(O)₂NHRₐ₈ or -(CH₂)ₘ₉NHS(O)₂Rₐ₉, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3-to 6-membered heterocyclyl, C₆₋₁₀ aryl or 5- to 6-membered heteroaryl may optionally be further substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl;
Rₐ₁, Rₐ₂, Rₐ₃, Rₐ₄, Rₐ₅, Rₐ₆, Rₐ₇, Rₐ₈ and Rₐ₉ are each independently selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3-to 6-membered heterocyclyl, C₆₋₁₀ aryl or 5- to 6-membered heteroaryl may optionally be further substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl.

In certain embodiments of the present invention, R₁₋₁ and R₁₋₂ are connected to form 3- to 6-membered heterocyclyl, wherein the 3- to 6-membered heterocyclyl may optionally be further substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl.

In certain embodiments of the present invention, R₁₋₁ or R₁₋₂ is each independently selected from methyl or

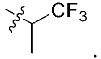

In certain embodiments of the present invention, R₁₋₁ and R₁₋₂ are connected to form

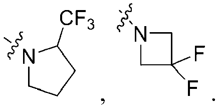

In certain embodiments of the present invention, R₁₋₃ is selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl, 5- to 6-membered heteroaryl, -(CH₂)ₘ₁ORₐ₁, -(CH₂)ₘ₂C(O)Rₐ₂, - (CH₂)ₘ₃NHC(O)Rₐ₃, -(CH₂)ₘ₄C(O)NHRₐ₄, -(CH₂)ₘ₅NRₐ₅Rₐ₆, -(CH₂)ₘ₆S(O)ₘ₇Rₐ₇, - (CH₂)ₘ₈S(O)₂NHRₐ₈, -(CH₂)ₘ₉NHS(O)₂Rₐ₉ or -(CH₂)ₘ₁₀Rₐ₁₀, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl may optionally be further substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl;
Rₐ₁, Rₐ₂, Rₐ₃, Rₐ₄, Rₐ₅, Rₐ₆, Rₐ₇, Rₐ₈, Rₐ₉ and Rₐ₁₀ are each independently selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3-to 6-membered heterocyclyl, C₆₋₁₀ aryl or 5- to 6-membered heteroaryl may optionally be further substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl;
preferably, R₁₋₃ is selected from

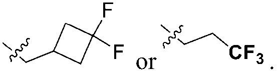

In certain embodiments of the present invention, R₁₋₁₋₁ is independently selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3-to 6-membered heterocyclyl, C₆₋₁₀ aryl or 5- to 6-membered heteroaryl; preferably, R₁₋₁₋₁ is independently selected from hydrogen, trifluoromethyl or fluoro.

In certain embodiments of the present invention, the compound is as represented by formula (I-E) 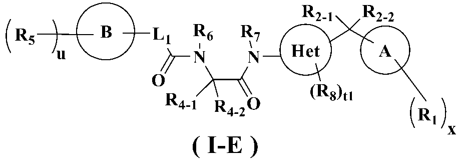 wherein
ring Het is selected from C₃₋₁₂ cycloalkyl, 3- to 12-membered heterocyclyl, C₆₋₁₄ aryl or 5- to 14-membered heteroaryl; preferably 5- to 6-membered heteroaryl; further preferably thiazolyl, pyrazolyl or oxadiazolyl;
each R₈ is independently selected from hydrogen, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₁₂ cycloalkyl, 3- to 12-membered heterocyclyl, C₆₋₁₄ aryl or 5- to 14-membered heteroaryl, wherein the C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₁₂ cycloalkyl, 3- to 12-membered heterocyclyl, C₆₋₁₄ aryl or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl and 5- to 10-membered heteroaryl;
t1 is 0, 1, 2, 3, 4 or 5;
ring A, ring B, R₁, R₂₋₁, R₂₋₂, R₄₋₁, R₄₋₂, R₅, R₆, R₇, L₁, u and x are as defined above, for example, in general formula (I-A-1).

The present invention also provides a compound as represented by the following general formulas, or a stereoisomer or pharmaceutically acceptable salt thereof, 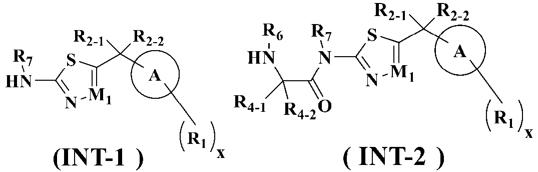 wherein ring A, R₁, R₂₋₁, R₂₋₂, R₄₋₁, R₄₋₂, R₆, R₇, x and M₁ are as defined above.

The present invention also provides a method for preparing the compound as represented by formula (I-A-1), or the stereoisomer or pharmaceutically acceptable salt thereof, wherein the method comprises reacting a compound as represented by formula (INT-3) with a compound as represented by formula (INT-2) to obtain the compound as represented by formula (I-A-1); 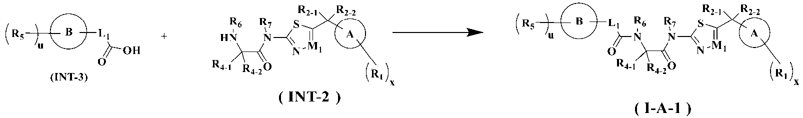
preferably, the reaction is carried out in the presence of a base and a catalyst; the base is an organic base, preferably DIPEA, and the catalyst is an amide condensing agent, preferably HATU;
ring A, R₁, R₂₋₁, R₂₋₂, R₄-₁, R₄₋₂, R₆, R₇, x and M₁ are as defined above.

The present invention also provides a pharmaceutical composition comprising a therapeutically effective dose of the above compound or the stereoisomer or pharmaceutically acceptable salt thereof, and one or more pharmaceutically acceptable carriers, diluents or excipients.

In certain embodiments of the present invention, the compound or the stereoisomer or pharmaceutically acceptable salt thereof is present in the composition in an amount of 0.1% to 95% by weight, preferably 0.5% to 85% by weight, more preferably 1% to 60% by weight, further preferably 10% to 50% by weight, further more preferably 15% to 40% by weight, further more preferably 20% to 30% by weight, and further more preferably 20% to 25% by weight (based on the total weight of the pharmaceutical composition).

In certain embodiments of the present invention, the pharmaceutical composition is selected from a tablet, a capsule, a liquid preparation or an injection, preferably further comprises a filler, optionally further comprises a disintegrant, or further comprises one or more of a glidant or a lubricant.

The present invention further relates to the use of the above compound or the stereoisomer or pharmaceutically acceptable salt thereof, or the pharmaceutical composition in the preparation of an IL-17 inhibitor drug, wherein the IL-17 is preferably IL-17A.

The present invention further relates to the use of the compound, or the stereoisomer or pharmaceutically acceptable salt thereof, or the pharmaceutical composition thereof in the preparation of a medicament for treating an autoimmune disease, wherein the autoimmune disease is preferably selected from psoriasis, plaque psoriasis, guttate psoriasis, inverse psoriasis, pustular psoriasis, erythroderma psoriaticum, psoriatic arthritis, ankylosing spondylitis, hidradenitis suppurativa, rheumatoid arthritis, palmoplantar psoriasis, spondyloarthritis and non-infectious uveitis.

The present invention also relates to a method for treating, preventing and/or treating an autoimmune disease, wherein the method comprises administering to a patient a therapeutically effective dose of the compound, or the stereoisomer or pharmaceutically acceptable salt thereof, or the pharmaceutical composition thereof.

In certain embodiments of the present invention, the autoimmune disease is selected from psoriasis, plaque psoriasis, guttate psoriasis, inverse psoriasis, pustular psoriasis, erythroderma psoriaticum, psoriatic arthritis, ankylosing spondylitis, hidradenitis suppurativa, rheumatoid arthritis, palmoplantar psoriasis, spondyloarthritis and non-infectious uveitis.

### Detailed Description of the Invention

Unless stated to the contrary, all technical and scientific terms used herein generally have the same meanings as commonly understood by one of ordinary skill in the art. Specifically, the terms used in the description and claims have the following meanings.

The term "alkyl" refers to a linear or branched saturated aliphatic hydrocarbon group which may be optionally substituted with one or more substituents. In particular embodiments, the alkyl refers to a linear saturated hydrocarbon group having 1 to 20 (C₁₋₂₀), 1 to 15 (C₁₋₁₅), 1 to 12 (C₁₋₁₂), 1 to 10 (C₁₋₁₀), 1 to 8 (C₁₋₈), 1 to 6 (C₁₋₆), or 1 to 3 (C₁₋₃) carbon atoms, or a branched saturated hydrocarbon group having 3 to 20 (C₃₋₂₀), 3 to 15 (C₃₋₁₅), 3 to 12 (C₃₋₁₂), 3 to 10 (C₃₋₁₀), 3 to 8 (C₃₋₈) or 3 to 6 (C₃₋₆) carbon atoms. As used herein, linear C₁₋₆ alkyl and branched C₃₋₆ alkyl groups are also referred to as "lower alkyl". For example, C₁₋₆ alkyl refers to a linear saturated monovalent hydrocarbon group having 1 to 6 carbon atoms or a branched saturated monovalent hydrocarbon group having 3 to 6 carbon atoms. In one embodiment, the C₁₋₆ alkyl contains 1 to 6 (e.g., 1, 2, 3, 4, 5 or 6) carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, various branched isomers thereof, etc. In one embodiment, the alkyl is optionally substituted alkyl as described elsewhere herein.

The term "alkylene" refers to alkyl in which one hydrogen atom is further substituted, wherein the "alkyl" is as defined above. Non-limiting examples of "alkylene" include: methylene (-CH₂-), ethylene (-(CH₂)₂-), propylene (-(CH₂)₃-) or butylene (-(CH₂)₄-). In one embodiment, the alkylene is optionally substituted alkyl as described elsewhere herein.

The term "alkenyl" refers to a linear or branched unsaturated aliphatic hydrocarbon group containing at least one carbon-carbon double bond which may be located at any position within the alkenyl, and the alkenyl may be optionally substituted with one or more substituents. In particular embodiments, the alkenyl is a linear unsaturated hydrocarbon group having 2 to 20 (C₂₋₂₀), 2 to 15 (C₂₋₁₅), 2 to 12 (C₂₋₁₂), 2 to 10 (C₂₋₁₀), 2 to 8 (C₂₋₈), 2 to 6 (C₂₋₆) or 2 to 4 (C₂₋₄) carbon atoms, or a branched unsaturated hydrocarbon group having 3 to 20 (C₃₋₂₀), 3 to 15 (C₃₋₁₅), 3 to 12 (C₃₋₁₂), 3 to 10 (C₃₋₁₀), 3 to 8 (C₃₋₈) or 3 to 6 (C₃₋₆) carbon atoms. Unless otherwise specified, the term "alkenyl" as used herein includes both linear and branched alkenyl. For example, C₂₋₆ alkenyl refers to a linear unsaturated hydrocarbon group having 2 to 6 carbon atoms or a branched unsaturated hydrocarbon group having 3 to 6 carbon atoms. In one embodiment, the C₂₋₆ alkenyl contains 2 to 6 (e.g., 2, 3, 4, 5 or 6) carbon atoms. Non-limiting examples of alkenyl include: 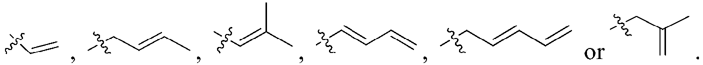 One of ordinary skill in the art can understand that the term "alkenyl" may also include groups having "cis" and "trans" configurations, or alternatively, groups having "E" and "Z" configurations. In one embodiment, the alkenyl is optionally substituted alkenyl as described elsewhere herein.

The term "alkynyl" refers to a linear or branched unsaturated aliphatic hydrocarbon group containing at least one carbon-carbon triple bond which may be located at any position within the alkynyl, and the alkynyl may be optionally substituted with one or more substituents. In particular embodiments, the alkynyl is a linear unsaturated hydrocarbon group having 2 to 20 (C₂₋₂₀), 2 to 15 (C₂₋₁₅), 2 to 12 (C₂₋₁₂), 2 to 10 (C₂₋₁₀), 2 to 8 (C₂₋₈), 2 to 6 (C₂₋₆), or 2 to 4 (C₂₋₄) carbon atoms, or a branched unsaturated hydrocarbon group having 3 to 20 (C₃₋₂₀), 3 to 15 (C₃₋₁₅), 3 to 12 (C₃₋₁₂), 3 to 10 (C₃₋₁₀), 3 to 8 (C₃₋₈) or 3 to 6 (C₃₋₆) carbon atoms. Unless otherwise specified, the term "alkynyl" as used herein includes both linear and branched alkynyl. For example, C₂₋₆ alkynyl refers to a linear unsaturated hydrocarbon group having 2 to 6 carbon atoms or a branched unsaturated hydrocarbon group having 3 to 6 carbon atoms. In one embodiment, the C₂₋₆ alkynyl contains 2 to 6 (e.g., 2, 3, 4, 5 or 6) carbon atoms. Non-limiting examples of alkynyl include: 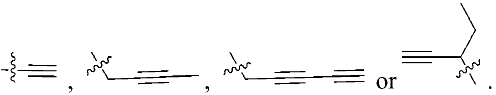 In one embodiment, the alkynyl is optionally substituted alkynyl as described elsewhere herein.

The term "cycloalkyl" refers to a saturated or partially unsaturated aliphatic hydrocarbon monocyclic, polycyclic (two or more rings) cyclic group, which may be optionally substituted with one or more substituents. In particular embodiments, the cycloalkyl ring contains 3 to 20 (C₃₋₂₀), 3 to 12 (C₃₋₁₂), 3 to 8 (C₃₋₈), or 3 to 6 (C₃₋₆) carbon atoms. In one embodiment, the cycloalkyl ring contains 6 to 14 (C₆₋₁₄) or 7 to 10 (C₇₋₁₀) carbon atoms; and it may contain one or more double bonds, but does not have a completely conjugated π electron system. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.; and the polycyclic cycloalkyl includes spirocycloalkyl, fused cycloalkyl and bridged cycloalkyl in one embodiment. In one embodiment, the cycloalkyl is optionally substituted cycloalkyl as described elsewhere herein, or cycloalkyl that is optionally fused to heterocyclyl, aryl or heteroaryl, and non-limiting examples include indanyl, tetrahydronaphthyl, benzocycloheptyl, etc.

The term "spirocycloalkyl" refers to an aliphatic hydrocarbon polycyclic group with monocyclic rings sharing one carbon atom (called a spiro atom), and it may contain one or more double bonds, but no ring has a completely conjugated π electron system. In particular embodiments, the spirocycloalkyl contains 5 to 20 (C₅₋₂₀), 6 to 14 (C₆₋₁₄) or 7 to 10 (C₇₋₁₀) (e.g., 7, 8, 9 or 10) carbon atoms. According to the number of shared spiro atoms between rings, the spirocycloalkyl is divided into monospirocycloalkyl, bispirocycloalkyl or polyspirocycloalkyl. In one embodiment, the spirocycloalkyl is monospirocycloalkyl or bispirocycloalkyl. In one embodiment, the spirocycloalkyl is 4-membered/4-membered, 3-membered/5-membered, 4-membered/5-membered, 4-membered/6-membered, 5-membered/5-membered or 5-membered/6-membered monospirocycloalkyl. In one embodiment, the spirocycloalkyl is optionally substituted spirocycloalkyl as described elsewhere herein. Non-limiting examples of spirocycloalkyl include:

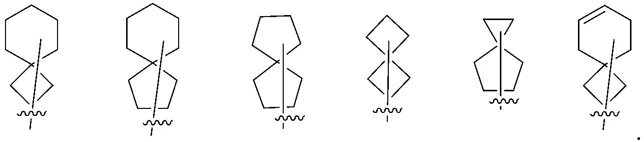

The term "fused cycloalkyl" refers to an all-carbon polycyclic group with each ring in the system sharing a pair of adjacent carbon atoms with another ring in the system, wherein one or more ring may contain one or more double bonds, but no ring has a completely conjugated π electron system. In particular embodiments, the fused cycloalkyl contains 5 to 20 (C₅₋₂₀), 6 to 14 (C₆₋₁₄) or 7 to 10 (C₇₋₁₀) (e.g., 7, 8, 9 or 10) carbon atoms. According to the number of constituent rings, the fused cycloalkyl can be divided into bicyclic, tricyclic, tetracyclic or polycyclic fused cycloalkyl. In one embodiment, the fused cycloalkyl is bicyclic or tricyclic, and in a further embodiment, the fused cycloalkyl is 3-membered/5-membered, 4-membered/5-membered, 5-membered/5-membered or 5-membered/6-membered bicyclic cycloalkyl. In one embodiment, the fused cycloalkyl is optionally substituted fused cycloalkyl as described elsewhere herein, or fused cycloalkyl that is optionally fused to heterocyclyl, aryl or heteroaryl. Non-limiting examples of fused cycloalkyl include: 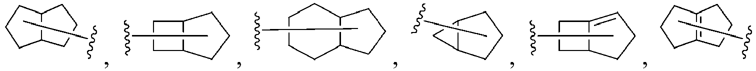 or

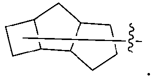

The term "bridged cycloalkyl" refers to an all-carbon polycyclic group in which any two rings share two carbon atoms that are not directly connected; and it may contain one or more double bonds, but no ring has a completely conjugated π electron system. In particular embodiments, the bridged cycloalkyl contains 5 to 20 (C₅₋₂₀), 6 to 14 (C₆₋₁₄) or 7 to 10 (C₇₋₁₀) (e.g., 7, 8, 9 or 10) carbon atoms. According to the number of constituent rings, the bridged cycloalkyl can be divided into bicyclic, tricyclic, tetracyclic or polycyclic bridged cycloalkyl, preferably bicyclic or tricyclic. In one embodiment, the bridged cycloalkyl is optionally substituted bridged cycloalkyl as described elsewhere herein. Non-limiting examples of bridged cycloalkyl include:

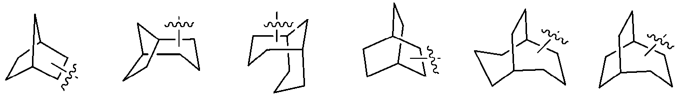

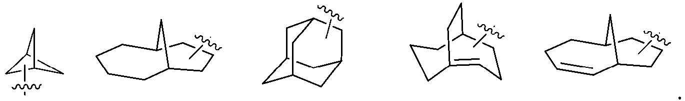

The term "heterocyclyl" refers to a saturated or partially unsaturated monocyclic or polycyclic cyclic hydrocarbon group, wherein one or more ring atoms are heteroatoms selected from nitrogen, oxygen, boron, phosphorus or sulphur, wherein the nitrogen, phosphorus or sulphur atom may be optionally oxidized, the nitrogen atom may be optionally quaternized, the ring carbon atoms may be optionally substituted with oxygen, but excluding ring moieties of -O-O- and -O-S-, and the remaining ring atoms are carbon atoms. The heterocyclyl may contain one or more double bonds but do not have a completely conjugated π electron system. In particular embodiments, the heterocyclyl contains 3 to 20, 3 to 12, 3 to 8 or 3 to 6 ring atoms, of which 1 to 4 are heteroatoms. In one embodiment, the heterocyclyl contains 3 to 6, 4 to 6, 3 to 8, 3 to 10, 6 to 10 or 7 to 11 ring atoms. In one embodiment, the heterocyclyl contains 3 to 8 (e.g., 3, 4, 5, 6, 7 or 8) ring atoms. Non-limiting examples of monocyclic heterocyclyl include tetrahydropyrrolyl, azetidinyl, oxetanyl, oxacyclohexyl, tetrahydrofuranyl, tetrahydropyranyl, tetrahydrothiophenyl, dihydroimidazolyl, dihydrofuryl, dihydropyrazolyl, dihydropyrrolyl, piperidinyl, piperazinyl, morpholinyl, thiomorpholinyl, homopiperazinyl, pyranyl, etc. Polycyclic heterocyclyl includes spiroheterocyclyl, fused heterocyclyl and bridged heterocyclyl. In one embodiment, the heterocyclyl is optionally substituted heterocyclyl as described elsewhere herein, or heterocyclyl that is further fused to other cycloalkyl, heterocyclyl, aryl and heteroaryl through any two or more atoms on the ring.

The term "spiroheterocyclyl" refers to a polycyclic heterocyclic group in which the rings share one atom (called a spiro atom), wherein one or more ring atoms are heteroatoms selected from nitrogen, oxygen, boron, phosphorus or sulphur, and the remaining ring atoms are carbon atoms. The spiroheterocyclyl may contain one or more double bonds, but no ring has a completely conjugated π electron system. In particular embodiments, the spiroheterocyclyl contains 5 to 20 or 6 to 14 ring atoms, and in one embodiment, the spiroheterocyclyl contains 7 to 11 (e.g., 7, 8, 9 10 or 11) ring atoms. According to the number of shared spiro atoms between rings, the spiroheterocyclyl is divided into monospiroheterocyclyl, bispiroheterocyclyl or polyspiroheterocyclyl, preferably monospiroheterocyclyl and bispiroheterocyclyl. In one embodiment, the spiroheterocyclyl is 4-membered/4-membered, 4-membered/5-membered, 4-membered/6-membered, 5-membered/5-membered or 5-membered/6-membered monospiroheterocyclyl. In one embodiment, the spiroheterocyclyl is optionally substituted spiroheterocyclyl as described elsewhere herein. Non-limiting examples of spiroheterocyclyl include:

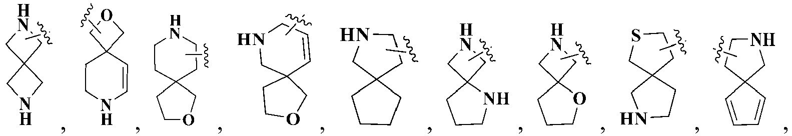

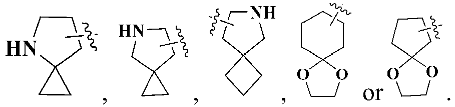

The term "fused heterocyclyl" refers to a polycyclic heterocyclic group with each ring in the system sharing a pair of adjacent atoms with another ring in the system, and one or more rings may contain one or more double bonds, but no ring has a completely conjugated π electron system, wherein one or more ring atoms are heteroatoms selected from nitrogen, oxygen, boron, phosphorus or sulphur, and the remaining ring atoms are carbon atoms. In particular embodiments, the fused heterocyclyl contains 5 to 20 or 6 to 14 ring atoms, and in one embodiment, the fused heterocyclyl contains 7 to 10 (e.g., 7, 8, 9 or 10) ring atoms. According to the number of constituent rings, the fused heterocyclyl can be divided into bicyclic, tricyclic, tetracyclic or polycyclic fused heterocyclyl, preferably bicyclic or tricyclic. In one embodiment, the fused heterocyclyl is 5-membered/5-membered or 5-membered/6-membered bicyclic fused heterocyclyl. In one embodiment, the fused heterocyclyl is optionally substituted fused heterocyclyl as described elsewhere herein, or fused heterocyclyl that can be fused to cycloalkyl, heterocyclyl, aryl or heteroaryl. Non-limiting examples of fused heterocyclyl include:

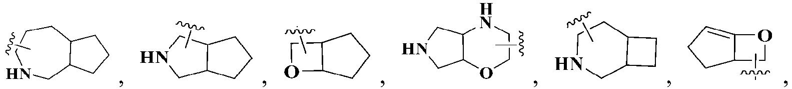

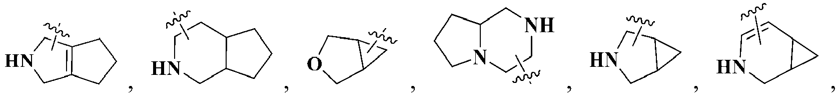

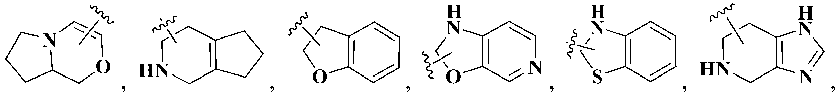

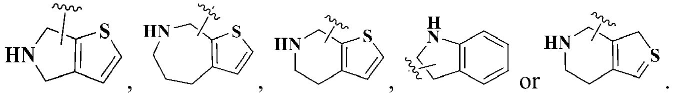

The term "bridged heterocyclyl" refers to a polycyclic heterocyclic group with any two rings sharing two atoms that are not directly connected. The bridged heterocyclyl may contain one or more double bonds, but no ring has a completely conjugated π electron system, wherein one or more ring atoms are heteroatoms selected from nitrogen, oxygen, boron, phosphorus or sulphur, and the remaining ring atoms are carbon atoms. In particular embodiments, the bridged heterocyclyl contains 5 to 20 or 6 to 14 ring atoms, and in one embodiment, the bridged heterocyclyl contains 7 to 10 (e.g., 7, 8, 9 or 10) ring atoms. According to the number of constituent rings, the bridged heterocyclyl can be divided into bicyclic, tricyclic, tetracyclic or polycyclic bridged heterocyclyl, preferably bicyclic, tricyclic or tetracyclic. In one embodiment, the bridged heterocyclyl is bicyclic or tricyclic. In one embodiment, the bridged heterocyclyl is optionally substituted bridged heterocyclyl as described elsewhere herein. Non-limiting examples of bridged heterocyclyl include:

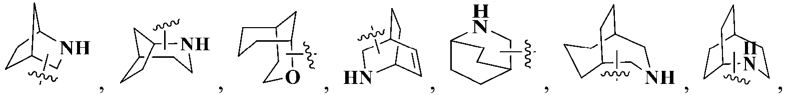

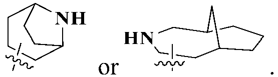

The term "aryl" refers to an all-carbon monocyclic or fused polycyclic (i.e., rings sharing a pair of adjacent carbon atoms) group containing at least one conjugated π electron system, which may be optionally substituted with one or more substituents. In particular embodiments, the aryl contains 6 to 20, 6 to 14, or 6 to 10 ring atoms. In one embodiment, the aryl may further refer to a bicyclic, tricyclic or tetracyclic ring system, in which at least one ring is an aromatic ring and the other rings may be saturated or partially unsaturated carbocyclic rings or rings containing one or more heteroatoms independently selected from O, S and N. In one embodiment, the aryl is selected from benzo 5- to 10-membered heteroaryl, benzo 3- to 10-membered cycloalkyl or benzo 3- to 10-membered heterocyclyl. In one embodiment, the aryl is selected from benzo 5- or 6-membered heteroaryl, benzo 3-to 6-membered cycloalkyl or benzo 3- to 6-membered heterocyclyl, wherein the heterocyclyl is heterocyclyl containing 1 to 3 nitrogen atoms, oxygen atoms or sulphur atoms. Non-limiting examples of aryl include phenyl, naphthyl, fluorenyl, azulenyl, anthryl, phenanthrenyl, pyrenyl, biphenyl, terphenyl, dihydronaphthyl, indenyl, tetrahydronaphthyl (tetralinyl), 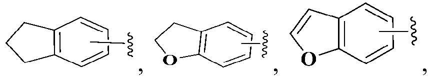 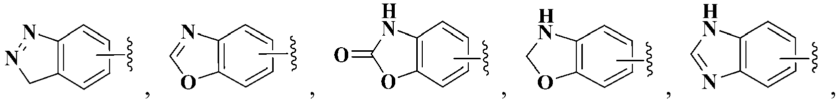 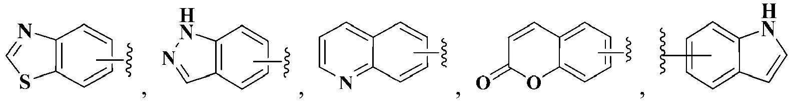 and

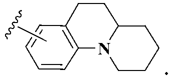

The term "arylene" refers to bivalent aryl formed by further substitution of one hydrogen atom on aryl, wherein the arylene is optionally substituted or unsubstituted, and the aryl is defined as above.

The term "heteroaryl" refers to an optionally substituted monocyclic or polycyclic group or ring system containing at least one aromatic ring, wherein the aromatic ring has one or more heteroatoms independently selected from O, S and N. In particular embodiments, the heteroaryl contains 5 to 20, 5 to 15, or 5 to 10 ring atoms, of which 1 to 4 are heteroatoms. In one embodiment, the heteroaryl contains 5 or 6 ring atoms. In particular embodiments, the heteroaryl may further refer to a bicyclic, tricyclic or tetracyclic ring, in which at least one ring is an aromatic ring having one or more heteroatoms independently selected from O, S and N, and the other rings may be saturated or partially unsaturated carbocyclic rings or rings containing one or more heteroatoms independently selected from O, S and N. In one embodiment, the heteroaryl is selected from heteroaryl fused 6- to 10-membered aryl, heteroaryl fused 3- to 10-membered cycloalkyl, or heteroaryl fused 3- to 10-membered heterocyclyl. Further in one embodiment, the heteroaryl is selected from 5- or 6-membered heteroaryl fused 6- to 10-membered aryl, 5- or 6-membered heteroaryl fused 3- to 6-membered cycloalkyl, or 5- or 6-membered heteroaryl fused 3- to 6-membered heterocyclyl, wherein the heterocyclyl is heterocyclyl containing 1 to 3 nitrogen atoms, oxygen atoms or sulphur atoms. Non-limiting examples include: furyl, imidazolyl, isothiazolyl, isoxazolyl, oxadiazolyl, oxazolyl, pyrazinyl, pyrazolyl, pyridazinyl, pyridyl, pyrimidyl, pyrrolyl, thiadiazolyl, thiazolyl, thienyl, tetrazolyl, triazinyl, triazolyl, benzofuryl, benzoimidazolyl, benzisoxazolyl, benzopyranyl, benzothiadiazolyl, benzothienyl, nenzophenylthio, benzothienyl, benzotriazolyl, imidazopyridyl, imidazothiazolyl, indolizinyl, indolyl, indazolyl, isobenzofuryl, isobenzothienyl, isoindolyl, isoquinolyl, naphthyridinyl, oxazolopyridyl, phthalazinyl, pteridyl, purinyl, pyridopyridyl, pyrrolopyridyl, quinolyl, quinoxalinyl, quinazolinyl, thiadiazolopyrimidyl, thienopyridyl, acridyl, benzoindolyl, carbazolyl, dibenzofuryl, phenanthrolinyl, phenanthridinyl, phenarsazinyl, phenazinyl, phenothiazinyl, phenoxazinyl, xanthenyl,

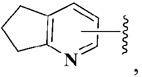

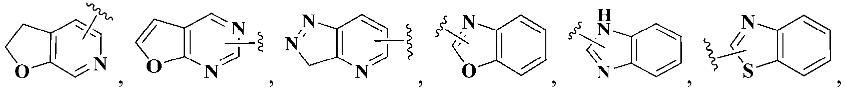

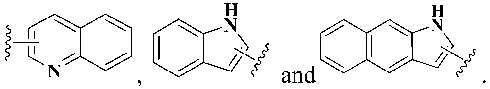

The term "heteroarylene" refers to bivalent heteroaryl formed by further substituting one hydrogen atom of cycloalkyl, wherein the heteroarylene is optionally substituted or unsubstituted, and the heteroaryl is as defined above.

The term "heteroalkyl" refers to a stable linear or branched, or cyclic hydrocarbon group, or a combination thereof, and is composed of the indicated number of carbon atoms and one or more (in one embodiment, one to three) heteroatoms selected from O, N, Si and S, wherein the nitrogen and sulphur atoms are optionally oxidized and the nitrogen heteroatom may be optionally quaternized. In one embodiment, the heteroatoms O, N and S may be placed at any interior position of the heteroalkyl group. In one embodiment, the heteroatom Si may be placed at any position (e.g., interior or terminal position) of the heteroalkyl group, including the position where the alkyl group is connected to the rest of the molecule. Non-limiting examples include: -CH₂-CH₂-O-CH₃, -CH₂-CH₂-NH-CH₃, -CH₂-CH₂-N (CH₃)-CH₃, -CH₂-S-CH₂-CH₃, -CH₂-CH₂-S(O)-CH₃, -CH₂-CH₂-S(O)₂-CH₃, -CH = CH-O-CH₃, -Si (CH₃)₃, -CH₂-CH = N-OCH₃ and-CH = CH-N (CH₃)-CH₃. Up to two heteroatoms can be consecutive, for example, -CH₂-NH-O-CH₃ and -CH₂-O-Si(CH₃)₃. In particular embodiments, the heteroalkyl is optionally substituted heteroalkyl described as elsewhere herein.

The term "alkoxy" refers to -O-(alkyl) and -O-(unsubstituted cycloalkyl), wherein the alkyl or cycloalkyl is as defined above. Non-limiting examples of alkoxy include: methoxy, ethoxy, propoxy, butoxy, cyclopropoxy, cyclobutoxy, cyclopentyloxy or cyclohexyloxy. In one embodiment, the alkoxy is optionally substituted alkoxy as described elsewhere herein.

The term "alkylacyl" refers to -C(O)-alkyl, wherein the alkyl is as defined above.

The term "haloalkyl" refers to alkyl substituted with one or more halogen, wherein the alkyl is as defined above. Non-limiting examples of haloalkyl include: trifluoromethyl, -CH₂CF₃,

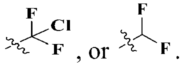

The term "haloalkoxy" refers to alkoxy substituted with one or more halogen, wherein the alkoxy is as defined above.

The term "hydroxyalkyl" refers to alkyl substituted with hydroxyl, wherein the alkyl is as defined above.

The term "alkylthio" refers to -S-(alkyl) and -S-(unsubstituted cycloalkyl), wherein the alkyl or cycloalkyl is as defined above. Non-limiting examples of alkylthio include: methylthio, ethylthio, propylthio, butylthio, cyclopropylthio, cyclobutylthio, cyclopentylthio or cyclohexylthio. In one embodiment, the alkylthio is optionally substituted alkylthio as described elsewhere herein.

The term "haloalkylthio" refers to alkylthio substituted with one or more halogen, wherein the alkylthio is as defined above.

The term "alkenylcarbonyl" refers to -C(O)-(alkenyl), wherein the alkenyl is as defined above. Non-limiting examples of alkenylcarbonyl include: ethenylcarbonyl, propenylcarbonyl, or butenylcarbonyl. In one embodiment, the alkenylcarbonyl is optionally substituted alkenylcarbonyl as described elsewhere herein.

The term "aminocarbonyl" refers to NH₂-C(O)-.

The term "alkylaminocarbonyl" refers to aminocarbonyl (NH₂-C(O)-) in which one or both of two hydrogen is substituted with alkyl, wherein the alkyl is as described above.

The term "alkylamino" refers to amino in which one or both of two hydrogen is substituted with alkyl, wherein the alkyl is as described above.

The term "carbonyl" refers to a -C(O)-, -(CO)- or -C(=O)- group. All the symbols are used interchangeably in the description.

The term "hydrogen" includes proton (¹H), deuterium (²H), tritium (³H) and/or a mixture thereof. In certain embodiments, one or more hydrogen-occupied positions in the compound may be enriched with deuterium and/or tritium. Such isotopically enriched analogues may be prepared from suitable isotopically labelled starting materials obtained from commercial sources or by known literature procedures.

The alkyl, alkylene, alkenyl, alkynyl, cycloalkyl, heterocyclyl, aryl, arylene, heteroaryl, heteroarylene, heteroalkyl, alkoxy, alkylthio, hydroxyalkyl, alkenylcarbonyl, aminocarbonyl, alkylaminocarbonyl, alkylamino or alkylacyl may be substituted or unsubstituted. In one embodiment, the substituent is selected from one or more of the following groups: alkyl, alkenyl, alkynyl, alkoxy, alkylthio, alkylamino, alkylacyl, halogen, mercapto, hydroxyl, nitro, cyano, azidyl, oximido, phosphate group, oxo, thio, carboxyl, carboxylate group, cycloalkyl, heterocyclyl, aryl, heteroaryl, heterocycloalkoxy, cycloalkylthio or heterocycloalkylthio.

Different expressions such as "X is selected from A, B or C", "X is selected from A, B and C", "X is A, B or C", and "X is A, B and C" all express the same meaning, i.e., X can be any one or more of A, B and C.

"Optional" or "optionally" means that the event or circumstance subsequently described may but need not to occur, and the description includes the occasions where the events or circumstances occur or do not occur. For example, "heterocyclic group optionally substituted with alkyl" means the alkyl may but need not be present, the description includes the case where the heterocyclic group is substituted with alkyl and the case where the heterocyclic group is not substituted with alkyl.

In various parts of the present invention, linking substituents are described. When it is clear that a linking group is required for the structure, the markush variables listed for that group should be understood as referring to the linking group. For example, if a linking group is required for the structure and the markush group definition for that variable lists "alkyl" or "aryl," it should be understood that the "alkyl" or "aryl" respectively represents the attached alkylene group or arylene group.

The term "substituted" means that any one or more hydrogen atoms on the designated atom are substituted with a substituent, provided that the valence state of the designated atom is normal, and the substituted compound is stable in one embodiment. When the substituent is oxo (i.e., =O), it means that two hydrogen atoms are substituted. The term "optionally substituted" means that a group may or may not be substituted. Unless otherwise specified, the type and number of substituents may be arbitrary on the basis of chemical practicability. It goes without saying, the substituents may be only in their possible chemical positions, a person skilled in the art can determine the possible or impossible substitutions (by experiment or theory) without paying too much effort. For example, amino having a free hydrogen or a hydroxy group may be unstable when combined the carbon atoms having an unsaturated (e.g., olefinic) bond.

Unless stated to the contrary, the indefinite articles "a" and "an" and the definite article "the" in the present description and claims include plural as well as singular forms.

The "pharmaceutical composition" denotes a mixture containing one or more of the compounds as described herein or physiologically/pharmaceutically acceptable salts or prodrug thereof and other chemical components, as well as other components, such as a physiologically/pharmaceutically acceptable carrier and an excipient. The purpose of pharmaceutical compositions is to facilitate administration to living organisms and facilitate the absorption of active ingredients to exert biological activity.

The "pharmaceutically acceptable salt" refers to salts of the compound of the present invention, which are safe and effective when used in mammals, and have appropriate biological activity.

The "stereoisomer" comprises all enantiomerically/diastereomerically/stereoisomerically pure and enantiomerically/diastereomerically/stereoisomerically enriched compounds of the present invention.

"Stereoisomerically pure" means a composition comprising one stereoisomer of a compound and being substantially free of another stereoisomer of the compound. For example, a stereoisomerically pure composition of a compound having one chiral centre will be substantially free of the opposite enantiomer of the compound. A stereoisomerically pure composition of a compound having two chiral centres will be substantially free of other diastereomers of the compound. A typical stereoisomerically pure compound comprises greater than about 80% by weight of one stereoisomer of the compound and less than about 20% by weight of another stereoisomer of the compound, greater than about 90% by weight of one stereoisomer of the compound and less than about 10% by weight of another stereoisomer of the compound, greater than about 95% by weight of one stereoisomer of the compound and less than about 5% by weight of another stereoisomer of the compound, greater than about 97% by weight of one stereoisomer of the compound and less than about 3% by weight of another stereoisomer of the compound, or greater than about 99% by weight of one stereoisomer of the compound and less than about 1% by weight of another stereoisomer of the compound.

"Stereoisomerically enriched" means a composition comprising greater than about 55% by weight, greater than about 60% by weight, greater than about 70% by weight, or greater than about 80% by weight of one stereoisomer of a compound.

"Enantiomerically pure" means a stereoisomerically pure composition of a compound having one chiral centre. Similarly, the term "enantiomerically enriched" refers to a stereomerically enriched composition of a compound having one chiral centre.

"Optical activity" and "enantiomeric activity" mean a combination of molecules having an enantiomeric or diastereomeric excess of no less than about 50%, no less than about 70%, no less than about 80%, no less than about 90%, no less than about 91%, no less than about 92%, no less than about 93% , no less than about 94%, no less than about 95%, no less than about 96%, no less than about 97%, no less than about 98%, no less than about 99%, no less than about 99.5%, or no less than about 99.8%. In particular embodiments, the compound comprises about 95% or more of the desired enantiomer or diastereomer and about 5% or less of the less preferred enantiomer or diastereomer, based on the total weight of the racemate.

When describing optically active compounds, the prefixes R and S are used to indicate the absolute configuration of the molecule relative to its chiral centre. (+) and (-) are used to represent the optical rotation of the compound, that is, the direction of the plane of polarized light rotated by the optically active compound. The prefix (-) indicates that the compound is levorotatory, that is, the compound rotates the plane of polarized light to the left or counterclockwise. The prefix (+) indicates that the compound is dextrorotatory, that is, the compound rotates the plane of polarized light to the right or clockwise. However, the signs of optical rotation (+) and (-) are independent of the absolute configurations R and S of the molecule.

### Detailed Description of Embodiments

The present invention will be further described below with reference to examples, but these examples do not limit the scope of the present invention.

### Example

The structure of the compound of the present invention is determined by nuclear magnetic resonance (NMR) or/and liquid chromatography-mass spectrometry (LC-MS). NMR chemical shift (δ) is given in parts per million (ppm). NMR is determined with Bruker AVANCE-400 nuclear magnetic resonance instrument; the solvents for determination are deuterated dimethyl sulfoxide (DMSO-*d₆*), deuterated methanol (CD₃OD) and deuterated chloroform (CDCl₃); and the internal standard is tetramethylsilane (TMS).

Liquid chromatography-mass spectrometry LC-MS is determined by Agilent 1200 Infinity Series mass spectrometer. For determination by HPLC, Agilent 1200DAD high-pressure liquid chromatograph (Sunfire C18 150 × 4.6 mm chromatographic column) and Waters 2695-2996 high-pressure liquid chromatograph (Gimini C₁₈ 150 × 4.6 mm chromatographic column) are used.

Yantai Huanghai HSGF254 or Qingdao GF254 silica gel plate is used as a thin layer chromatography silica plate; the specification used for the TLC is 0.15-0.20 mm; and the specification when separating and purifying a product by thin layer chromatography is 0.4-0.5 mm. Yantai Huanghai silica gel of 200-300 mesh silica gel is generally used as a carrier in column chromatography.

The starting materials in the examples of the present invention are known and can be purchased on the market, or can be synthesised using or according to methods known in the art.

Unless otherwise specified, all reactions in the present invention are carried out with continuous magnetic stirring in a dry nitrogen or argon atmosphere, using dried solvents and with the reaction temperature expressed in degrees Celsius.

### Example 1

### Step 1: preparation of diethyl 2-(2,2,2-trifluoroethyl)malonate

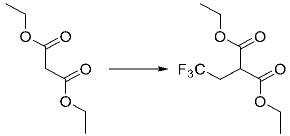

To a solution of diethyl malonate (45 g, 281.0 mmol) and 2,2,2-trifluoroethyl trifluoromethanesulfonate (71.7 g, 309.1 mmol) in acetonitrile (800 mL) was added potassium carbonate (60.2 g, 435.5 mmol) at room temperature. The reaction was heated to 90°C and stirred for 12 hours. The reaction was cooled to room temperature, filtered under reduced pressure through celite and concentrated under reduced pressure to remove the organic solvent. The residue was dissolved in dichloromethane and washed with saturated sodium chloride. The organic phase was separated, dried over anhydrous sodium sulphate and concentrated under reduced pressure to remove the organic solvent, and the residue was separated by column chromatography to obtain the title compound (19.6 g, 29%). MS m/z (ESI): 243.1 [M]⁺.

### Step 2: preparation of diethyl 2-(2,2-dimethoxyethyl)-2-(2,2,2-trifluoroethyl)malonate

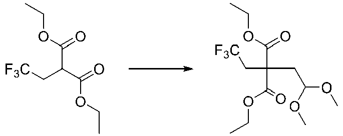

In an ice bath, to a solution of diethyl 2-(2,2,2-trifluoroethyl)malonate (14 g, 57.8 mmol) in DMF (150 mL) was added sodium hydride (2.89 g, 72.3 mmol, 60 wt%) in batches, and the mixture was stirred at room temperature for 30 minutes. 2-Bromo-1,1-dimethoxyethane (11.72 g, 69.4 mmol) and potassium iodide (1.92 g, 11.6 mmol) were added, and the mixture was heated to 125°C and stirred for 8 hours. The reaction was cooled to room temperature, diluted with ethyl acetate and washed three times with a saturated sodium chloride solution. The organic phase was separated, dried over anhydrous sodium sulphate, filtered and then concentrated under reduced pressure to remove the organic solvent, and the residue was separated by column chromatography to obtain the title compound (7.8 g, 41%). MS m/z (ESI): 331.1[M]⁺.

### Step 3: preparation of ethyl 2-(2,2-dimethoxyethyl)-4,4,4-trifluorobutanoate

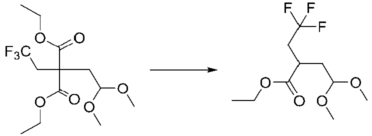

Diethyl 2-(2,2-dimethoxyethyl)-2-(2,2,2-trifluoroethyl)malonate (7.8 g 23.6 mmol) and sodium bromide (4.86 g, 47.2 mmol) were added to a mixed solvent of water (0.85 g, 47.2 mmol) and DMF (100 mL). The reaction was heated to 160°C and stirred in a sealed tube for 12 hours. The reaction was cooled to room temperature and diluted with ethyl acetate. The organic phase was washed three times with a saturated sodium chloride solution, dried over anhydrous sodium sulphate, filtered and then concentrated under reduced pressure to remove the organic solvent, and the residue was separated by column chromatography to obtain the title compound (3.5 g, 57%). MS m/z (ESI): 259.1 [M+H]⁺.

### Step 4: preparation of ethyl 4,4,4-trifluoro-2-(2-carbonylethyl)butynoate

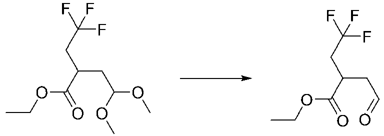

Ethyl 2-(2,2-dimethoxyethyl)-4,4,4-trifluorobutanoate (2.3 g, 8.91 mmol) and p-toluenesulphonic acid monohydrate (254 mg, 1.34 mmol) were dissolved in a mixed solvent of acetone (20 mL) and water (20 mL), and the reaction was heated to 70°C and stirred for 3 hours. The reaction solution was cooled to room temperature and diluted with dichloromethane. The organic phase was washed with a saturated sodium chloride solution, dried over anhydrous sodium sulphate, filtered and then concentrated to remove the organic solvent, and the residue was separated by column chromatography to obtain the title compound (1.6 g, 85%). MS m/z (ESI): 213.1 [M+H]⁺.

### Step 5: preparation of ethyl 2-(1-bromo-2-carbonylethyl)-4,4,4-trifluorobutanoate

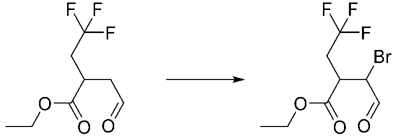

Ethyl 4,4,4-trifluoro-2-(2-carbonylethyl)butynoate (1.6 g, 7.54 mmol) was dissolved in a mixed solution of diethyl ether (20 mL) and 1,4-dioxane (20 mL), and the mixture was cooled to 0°C in an ice-water bath. To the reaction solution was slowly added bromine (1.21 g, 7.54 mmol). After the addition, the reaction solution was slowly warmed to room temperature and further stirred for 3 hours. The reaction solution was quenched with a saturated sodium thiosulphate solution and extracted three times with ethyl acetate. The organic phases were combined, dried over anhydrous sodium sulphate, filtered and then concentrated under reduced pressure to remove the organic solvent, so as to obtain the title compound (crude, 2.1 g), which was directly used in the next reaction. MS m/z (ESI): 291.1 [M+H]⁺.

### Step 6: preparation of ethyl 2-(2-aminothiazol-5-yl)-4,4,4-trifluorobutanoate

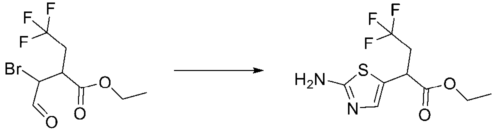

2-(1-bromo-2-carbonylethyl)-4,4,4-trifluorobutanoate (2.1 g, 7.2 mmol) and thiourea (0.55 g, 7.2 mmol) were added to ethanol (30 mL). The reaction solution was heated to 90°C, stirred for 12 hours and cooled to room temperature. The reaction solution was diluted with dichloromethane and washed with a saturated sodium chloride solution. The organic phase was separated, dried over anhydrous sodium sulphate, filtered and then concentrated under reduced pressure to remove the organic solvent, and the residue was separated by column chromatography to obtain the title compound, ethyl 2-(2-aminothiazol-5-yl)-4,4,4-trifluorobutanoate (0.76 g, 39%). MS m/z (ESI): 269.1 [M+H]⁺.

### Step 7: preparation of ethyl 2-(2-((S)-2-((tert-butoxycarbonyl)amino)-2-(4,4-difluorocyclohexyl)acetamido)thiazol-5-yl)-4,4,4-trifluorobutanoate

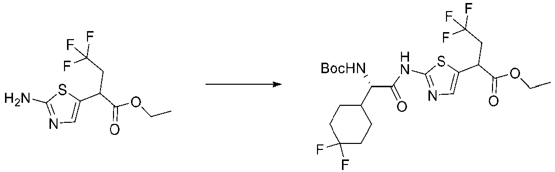

Ethyl 2-(2-aminothiazol-5-yl)-4,4,4-trifluorobutanoate (0.76 g, 2.8 mmol) and (2S)-2-(tert-butoxycarbonylamino)-2-(4,4-difluorocyclohexyl)acetic acid (0.97 g, 3.3 mmol) were dissolved in dichloromethane (15 mL). HATU (1.18 g, 3.1 mmol) and DIPEA (837 mg, 6.5 mmol) were sequentially added, and the mixture was stirred at room temperature for 3 hours. The reaction solution was diluted with ethyl acetate and washed with a saturated sodium chloride solution. The organic phase was separated, dried over anhydrous sodium sulphate, filtered and then concentrated under reduced pressure to remove the organic solvent, and the residue was separated by column chromatography to obtain the title compound (1.38 g, 89.6%). MS m/z (ESI): 544.1 [M+H]⁺.

### Step 8: preparation of 2-(2-((S)-2-((tert-butoxycarbonyl)amino)-2-(4,4-difluorocyclohexyl)acetamido)thiazol-5-yl)-4,4,4-trifluorobutanoic acid

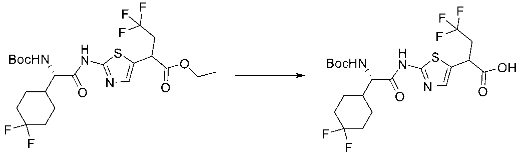

Ethyl 2-(2-((S)-2-((tert-butoxycarbonyl)amino)-2-(4,4-difluorocyclohexyl) acetamido)thiazol-5-yl)-4,4,4-trifluorobutanoate (1.6 g, 2.9 mmol) was dissolved in a mixed solvent of tetrahydrofuran (8 mL)-methanol (6 mL)-water (12 mL), and the mixture was cooled to 0°C in an ice-water bath. Under stirring, lithium hydroxide monohydrate (618 mg, 14.72 mmol) was added. The reaction was slowly warmed to room temperature, further stirred for 1 hour and concentrated under reduced pressure to remove the organic solvent, and the residue was neutralized to pH 5 with 2 N hydrochloric acid solution and extracted three times with ethyl acetate. The organic phases were combined, dried over anhydrous sodium sulphate , filtered and concentrated under reduced pressure to remove the organic solvent, so as to obtain the title compound (1.26 g, crude), which was directly used in the next reaction. MS m/z (ESI): 516.1 [M+H]⁺.

### Step 9: preparation of tert-butyl((1S)-2-((5-(1-(2-(5-cyanopyridin-2-yl)hydrazinyl)-4,4,4-trifluoro-1-carbonylbutan-2-yl)thiazol-2-yl)amino)-1-(4,4-difluorocyclohexyl)-2-carbonylethyl)carbamate

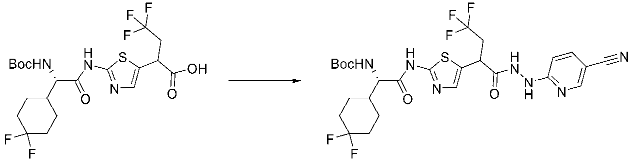

2-(2-((S)-2-((tert-butoxycarbonyl)amino)-2-(4,4-difluorocyclohexyl)acetamido)thiazol-5-yl)-4,4,4-trifluorobutanoic acid (1.26 g, 2.4 mmol) and 6-hydrazinylnicotinonitrile (492 mg, 3.67 mmol) were dissolved in a DMF solution (20 mL), and the mixture was cooled to 0°C in an ice-water bath. 1-Ethyl-(3-dimethylaminopropyl)carbodiimide hydrochloride (703 mg, 3.67 mmol), HOBt (495 mg, 3.67 mmol) and DIPEA (948 mg, 7.3 mmol) were added. The reaction was slowly warmed to room temperature and further stirred for 24 hours. The reaction solution was diluted with ethyl acetate and washed with a saturated sodium chloride solution. The organic phase was separated, dried over anhydrous sodium sulphate, filtered and then concentrated under reduced pressure to remove the organic solvent, and the residue was separated by column chromatography to obtain the title compound (1.16 g, 75%). MS m/z (ESI): 632.2 [M+H]⁺.

### Step 10: preparation of tert-butyl((1S)-2-((5-(1-(6-cyano-[1,2,4]triazolo[4,3-a]pyridin-3-yl)-3,3,3-trifluoropropyl)thiazol-2-yl)amino)-1-(4,4-difluorocyclohexyl)-2-carbonylethyl)carbamate

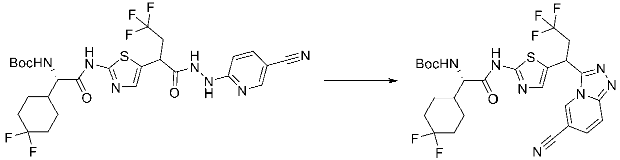

Tert-butyl((1S)-2-((5-(1-(2-(5-cyanopyridin-2-yl)hydrazinyl)-4,4,4-trifluoro-1-carbonylbutan-2-yl)thiazol-2-yl)amino)-1-(4,4-difluorocyclohexyl)-2-carbonylethyl)carbamate (0.11 g, 0.17 mmol) was dissolved in tetrahydrofuran (5 mL), and the mixture was cooled to 0°C in an ice-water bath. To the reaction solution were sequentially added triethylamine (0.31 g, 3.09 mmol) and triphenylphosphine dichloride (343 mg, 1.03 mmol). The reaction solution was transferred to room temperature and stirred for 1 hour, and then the reaction solution was heated to 90°C and stirred for 6 hours. The reaction solution was cooled to room temperature, diluted with dichloromethane and washed with a saturated sodium chloride solution. The organic phase was separated, dried over anhydrous sodium sulphate, filtered and then concentrated under reduced pressure to remove the organic solvent, and the residue was separated by column chromatography to obtain the title compound (81 mg, 76%). MS m/z (ESI): 614.1 [M+H]⁺.

### Step 11: preparation of (2S)-2-amino-N-(5-(1-(6-cyano-[1,2,4]triazolo[4,3-a]pyridin-3-yl)-3,3,3-trifluoropropyl)thiazol-2-yl)-2-(4,4-difluorocyclohexyl)acetamide

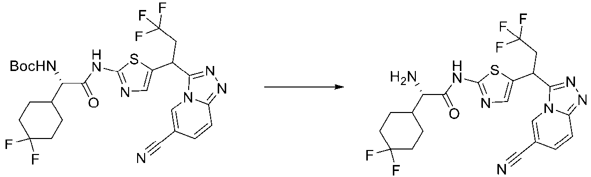

Tert-butyl((1S)-2-((5-(1-(6-cyano-[1,2,4]triazolo[4,3-a]pyridin-3-yl)-3,3,3-trifluoropropyl)thiazol-2-yl)amino)-1-(4,4-difluorocyclohexyl)-2-carbonylethyl)carbamate (81 mg, 0.13 mmol) was dissolved in dichloromethane (1 mL), and hydrogen chloride in dioxane (3 mL, 4 M) was added. The reaction was slowly warmed to room temperature, further stirred for 2 hours and concentrated under reduced pressure to obtain the title compound (68 mg, crude hydrochloride), which was directly used in the next step without purification. MS m/z (ESI): 514.1 [M+H]⁺.

### Step 12: preparation of N-((S)-2-((5-((R)-1-(6-cyano-[1,2,4]triazolo[4,3-a]pyridin-3-yl)-3,3,3-trifluoropropyl)thiazol-2-yl)amino)-1-(4,4-difluorocyclohexyl)-2-carbonylethyl)-4-methyl-1,2,5-oxadiazole-3-carboxamide

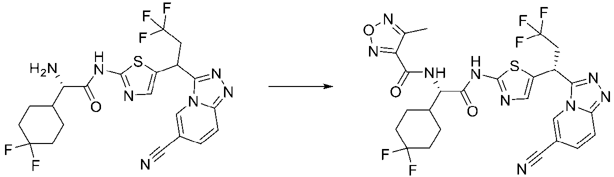

(2S)-2-amino-N-(5-(1-(6-cyano-[1,2,4]triazolo[4,3-a]pyridin-3-yl)-3,3,3-trifluoropropyl)thiazol-2-yl)-2-(4,4-difluorocyclohexyl)acetamide (crude hydrochloride, 30 mg, 0.054 mmol) and 4-methyl-1,2,5-oxadiazole-3-carboxylic acid (8.9 mg, 0.07 mmol) were added to dichloromethane (2 mL). At room temperature, with stirring, HATU (26.6 mg, 0.07 mmol) and DIPEA (181 mg, 0.14 mmol) were sequentially added. The mixture was stirred at room temperature for 3 hours. The reaction solution was diluted with dichloromethane and washed with a saturated sodium chloride solution. The organic phase was separated, dried over anhydrous sodium sulphate, filtered and then concentrated under reduced pressure to remove the organic solvent, and the residue was sequentially purified by HPLC and subjected to chiral resolution to obtain the title compound (8 mg, 23.7 %).

¹H NMR(400 MHz, DMSO-*d₆*) *δ* 9.56 (s, 1H), 9.42-9.37 (m, 1H), 7.96-7.93 (m, 1H), 7.65 (s, 1H), 7.62-7.59 (m, 1H), 7.40-7.34 (m, 1H), 5.63-5.51 (m, 1H), 4.57 (t, J= 7.8 Hz, 1H), 3.49-3.40 (m, 2H), 2.42 (s, 3H), 2.34-2.30 (m, 1H), 2.07-1.94 (m, 4H), 1.86-1.81 (m, 2H), 1.75-1.65 (m, 2H); MS m/z (ESI): 624.1 [M+H]⁺.

The following examples were prepared with reference to the method of Example 1.

| **Example** | **Structure** | **MS [M+H] ⁺** | **Example** | **Structure** | **MS [M+H] ⁺** |
|---|---|---|---|---|---|
| **2** | 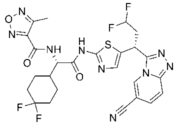 | 606.2 | **511** | 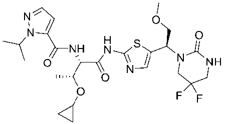 | 570.2 |
| **3** | 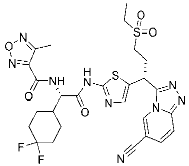 | 662.2 | **512** | 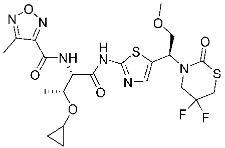 | 561.1 |
| **4** | 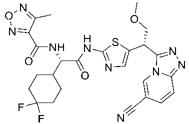 | 586.2 | **513** | 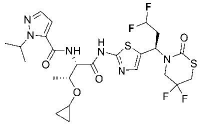 | 607.2 |
| **5** | 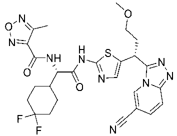 | 600.2 | **514** | 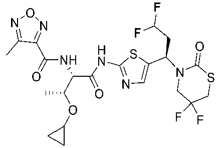 | 581.1 |
| **6** | 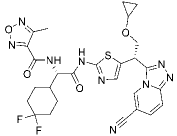 | 612.2 | **515** | 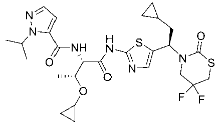 | 597.2 |
| **7** | 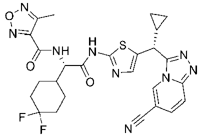 | 582.2 | **516** | 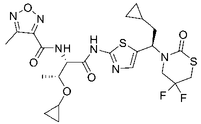 | 571.2 |
| **8** | 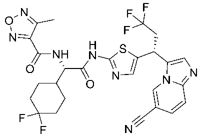 | 623.2 | **517** | 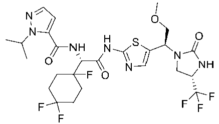 | 640.2 |
| **9** | 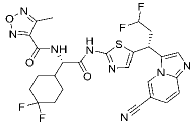 | 605.2 | **518** | 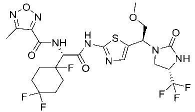 | 614.2 |
| **10** | 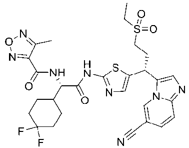 | 661.2 | **519** | 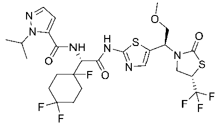 | 657.2 |
| **11** | 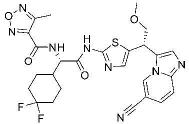 | 585.2 | **520** | 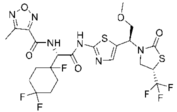 | 631.1 |
| **12** | 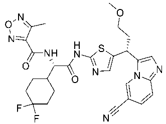 | 599.2 | **521** | 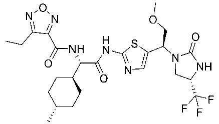 | 588.2 |
| **13** | 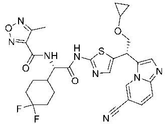 | 611.2 | **522** | 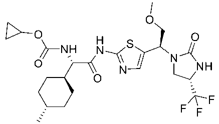 | 548.2 |
| **14** | 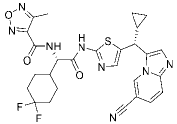 | 581.2 | **523** | 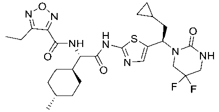 | 580.2 |
| **15** | 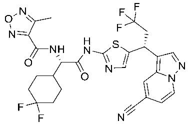 | 623.2 | **524** | 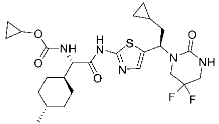 | 540.2 |
| **16** | 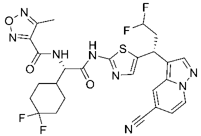 | 605.2 | **525** | 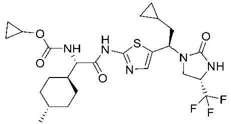 | 558.2 |
| **17** | 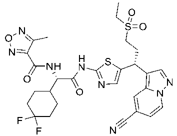 | 661.2 | **526** | 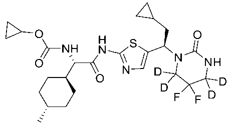 | 544.3 |
| **18** | 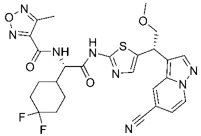 | 585.2 | **527** | 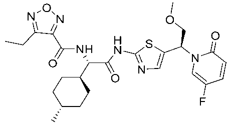 | 547.2 |
| **19** | 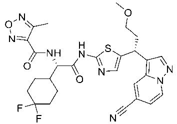 | 599.2 | **528** | 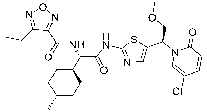 | 563.2 |
| **20** | 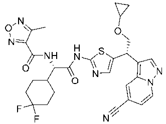 | 611.2 | **529** | 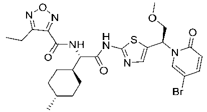 | 607.2 |
| **21** | 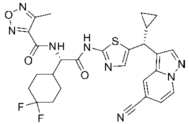 | 581.2 | **530** | 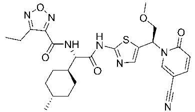 | 554.2 |
| **22** | 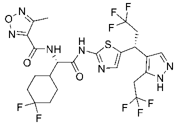 | 630.2 | **531** | 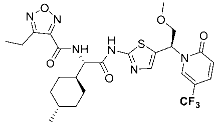 | 597.2 |
| **23** | 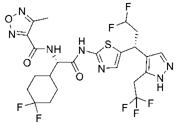 | 612.2 | **532** | 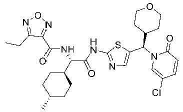 | 603.2 |
| **24** | 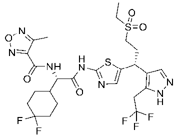 | 668.2 | **533** | 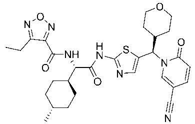 | 594.2 |
| **25** | 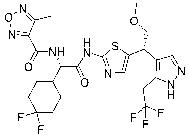 | 592.2 | **534** | 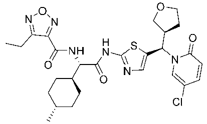 | 589.2 |
| **26** | 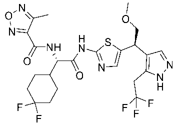 | 592.2 | **535** | 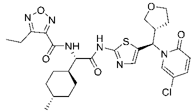 | 589.2 |
| **27** | 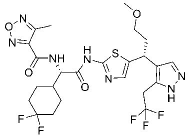 | 606.2 | **536** | 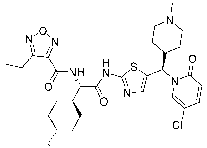 | 616.2 |
| **28** | 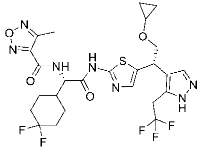 | 618.2 | **537** | 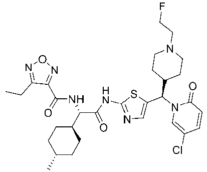 | 648.3 |
| **29** | 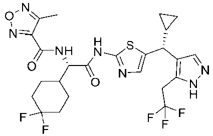 | 588.2 | **538** | 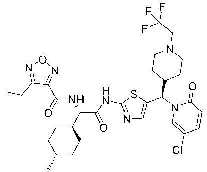 | 684.2 |
| **30** | 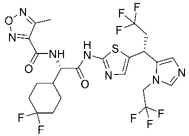 | 630.2 | **539** | 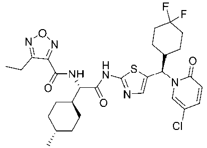 | 637.2 |
| **31** | 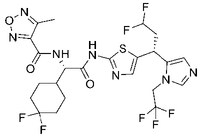 | 612.2 | **540** | 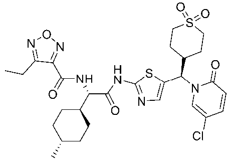 | 651.2 |
| **32** | 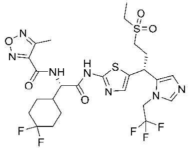 | 668.2 | **540-1** | 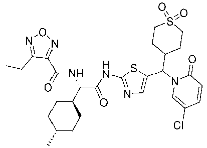 | 651.2 |
| **33** | 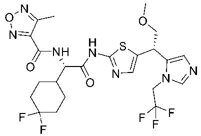 | 592.2 | **541** | 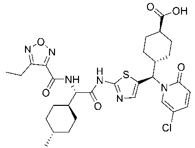 | 645.2 |
| **34** | 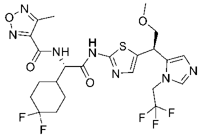 | 592.2 | **542** | 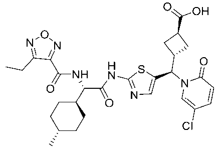 | 617.2 |
| **35** | 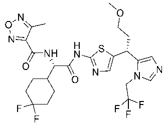 | 606.2 | **543** | 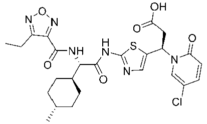 | 577.2 |
| **36** | 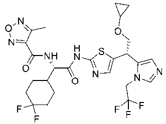 | 618.2 | **544** | 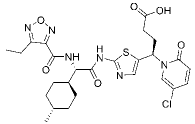 | 591.2 |
| **37** | 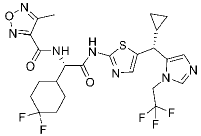 | 588.2 | **545** | 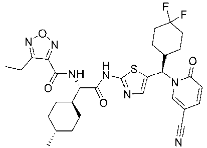 | 628.2 |
| **38** | 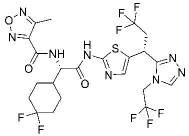 | 631.1 | **546** | 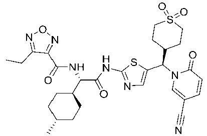 | 642.2 |
| **39** | 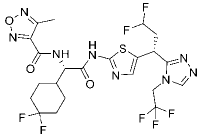 | 613.2 | **547** | 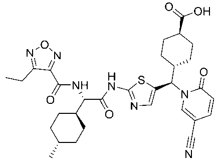 | 636.3 |
| **40** | 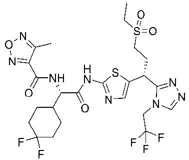 | 669.2 | **548** | 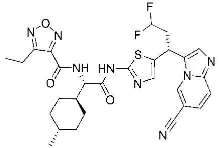 | 597.2 |
| **41** | 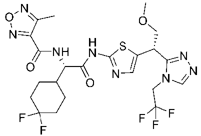 | 593.2 | **549** | 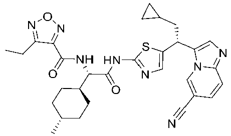 | 587.3 |
| **42** | 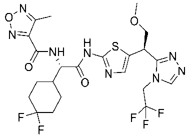 | 593.2 | **550** | 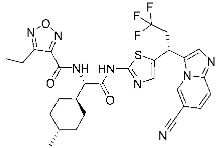 | 615.2 |
| **43** | 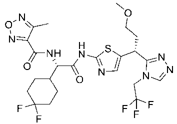 | 607.2 | **551** | 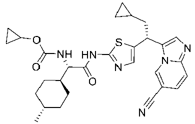 | 547.2 |
| **44** | 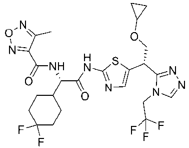 | 619.2 | **552** | 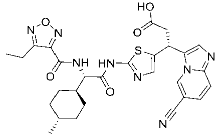 | 591.2 |
| **45** | 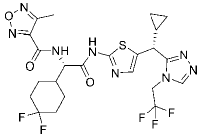 | 589.2 | **553** | 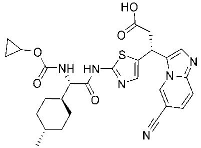 | 551.2 |
| **46** | 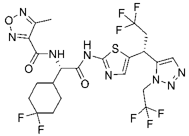 | 631.1 | **554** | 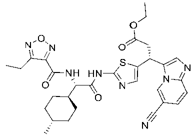 | 619.2 |
| **47** | 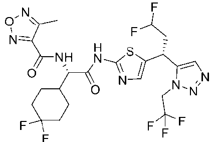 | 613.1 | **555** | 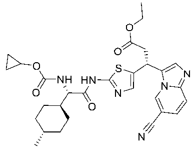 | 579.2 |
| **48** | 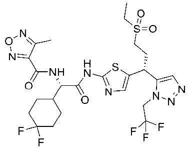 | 669.2 | **556** | 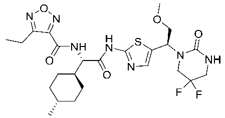 | 570.2 |
| **49** | 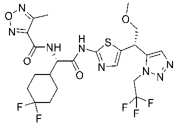 | 593.2 | **557** | 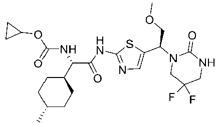 | 530.2 |
| **50** | 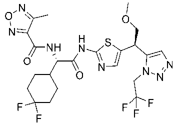 | 593.2 | **558** | 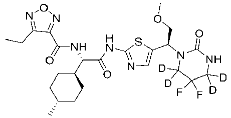 | 574.3 |
| **51** | 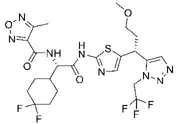 | 607.2 | **559** | 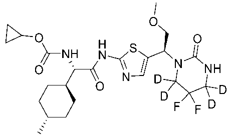 | 534.2 |
| **52** | 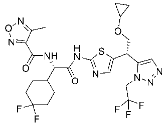 | 619.2 | **560** | 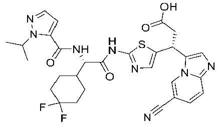 | 625.2 |
| **53** | 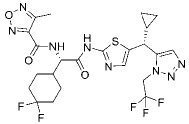 | 589.2 | **561** | 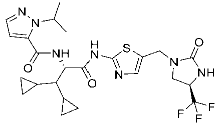 | 554.2 |
| **54** | 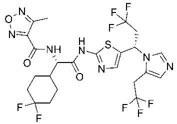 | 630.2 | **562** | 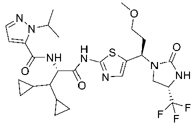 | 612.3 |
| **55** | 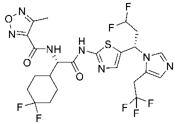 | 612.2 | **563** | 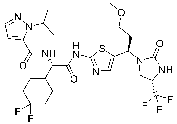 | 636.2 |
| **56** | 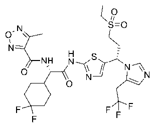 | 668.2 | **564** | 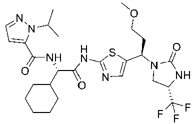 | 600.3 |
| **57** | 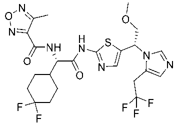 | 592.2 | **565** | 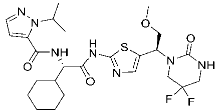 | 568.2 |
| **58** | 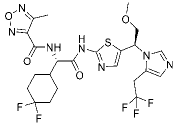 | 592.2 | **566** | 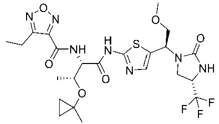 | 590.2 |
| **59** | 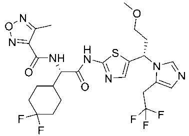 | 606.2 | **567** | 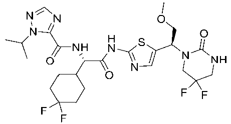 | 605.2 |
| **60** | 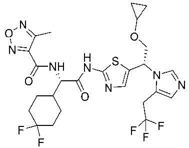 | 618.2 | **568** | 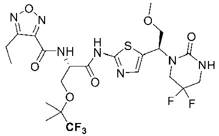 | 614.2 |
| **61** | 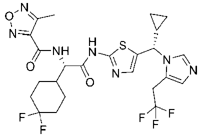 | 588.2 | **569** | 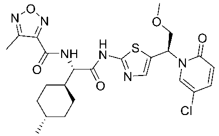 | 549.2 |
| **62** | 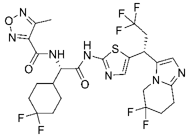 | 638.2 | **570** | 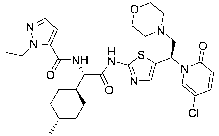 | 616.2 |
| **63** | 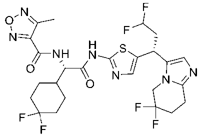 | 620.2 | **571** | 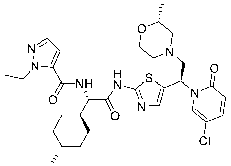 | 630.3 |
| **64** | 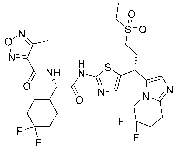 | 676.2 | **572** | 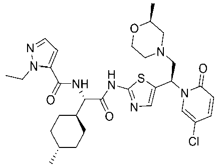 | 630.3 |
| **65** | 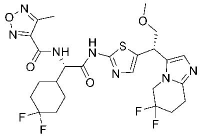 | 600.2 | **573** | 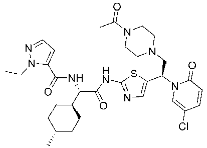 | 657.2 |
| **66** | 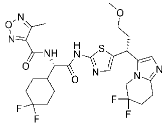 | 614.2 | **574** | 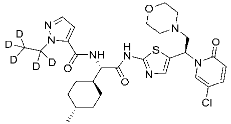 | 621.2 |
| **67** | 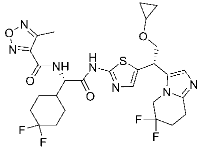 | 626.2 | **575** | 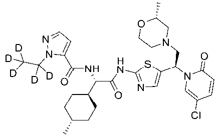 | 635.3 |
| **68** | 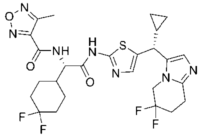 | 596.2 | **576** | 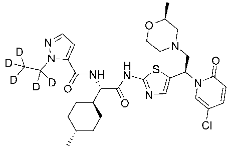 | 635.3 |
| **69** | 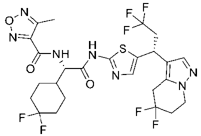 | 638.2 | **577** | 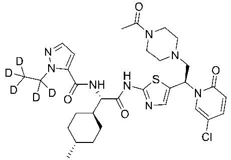 | 662.3 |
| **70** | 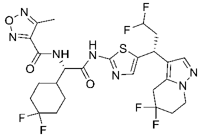 | 620.2 | **577-1** | 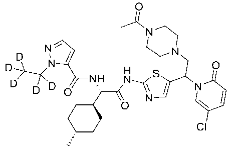 | 662.3 |
| **71** | 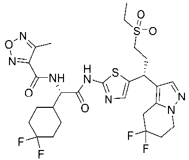 | 676.2 | **578** | 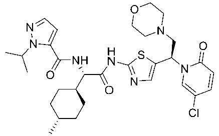 | 630.3 |
| **72** | 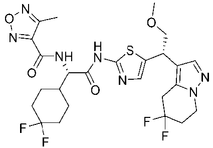 | 600.2 | **579** | 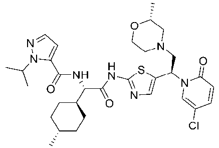 | 644.3 |
| **73** | 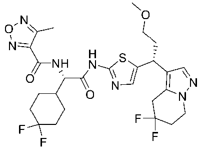 | 614.2 | **580** | 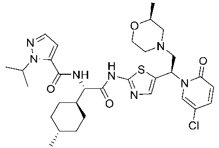 | 644.3 |
| **74** | 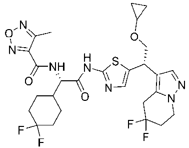 | 626.2 | **581** | 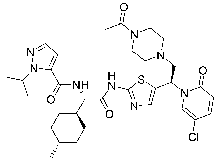 | 671.2 |
| **75** | 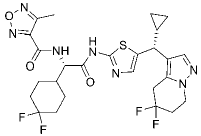 | 596.2 | **582** | 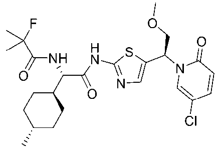 | 527.2 |
| **76** | 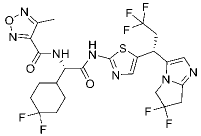 | 624.2 | **583** | 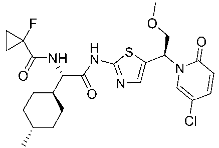 | 525.2 |
| **77** | 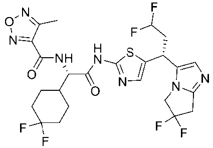 | 606.2 | **584** | 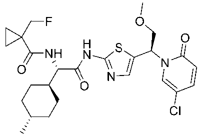 | 539.2 |
| **78** | 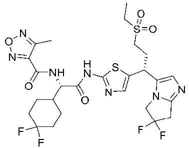 | 662.2 | **585** | 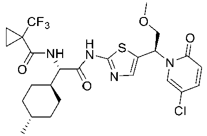 | 575.2 |
| **79** | 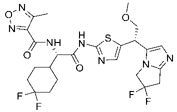 | 586.2 | **586** | 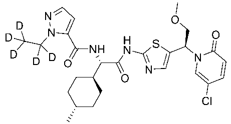 | 566.2 |
| **80** | 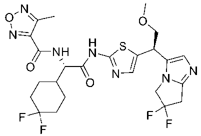 | 586.2 | **586-1** | 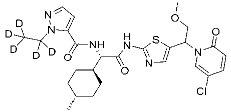 | 566.2 |
| **81** | 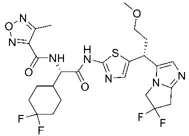 | 600.2 | **587** | 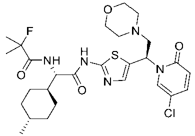 | 582.2 |
| **82** | 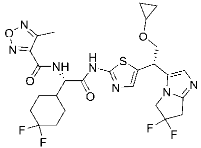 | 612.2 | **588** | 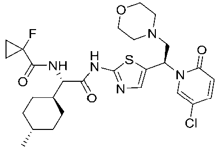 | 580.2 |
| **83** | 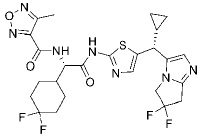 | 582.2 | **589** | 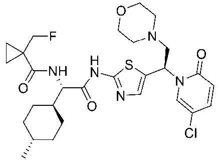 | 594.2 |
| **84** | 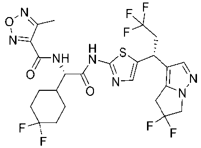 | 624.2 | **590** | 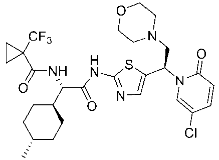 | 630.2 |
| **85** | 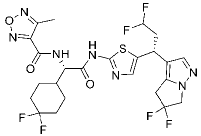 | 606.2 | **591** | 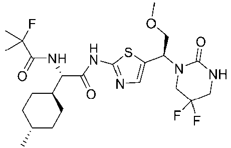 | 534.2 |
| **86** | 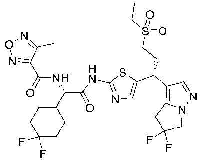 | 662.2 | **592** | 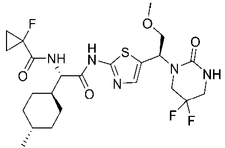 | 532.2 |
| **87** | 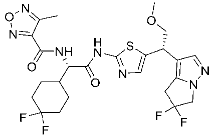 | 586.2 | **593** | 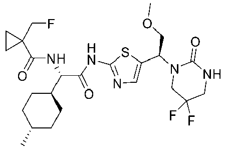 | 546.2 |
| **88** | 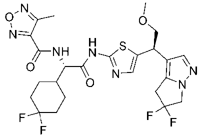 | 586.2 | **594** | 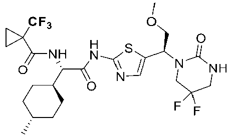 | 582.2 |
| **89** | 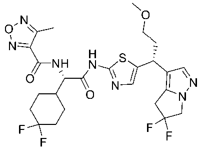 | 600.2 | **595** | 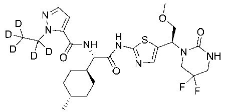 | 573.2 |
| **90** | 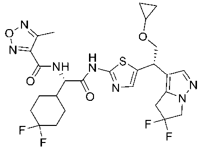 | 612.2 | **596** | 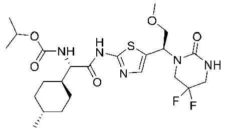 | 532.2 |
| **91** | 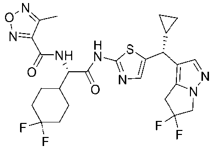 | 582.2 | **597** | 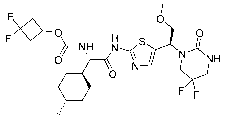 | 580.2 |
| **92** | 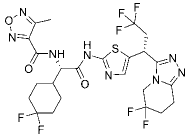 | 639.2 | **598** | 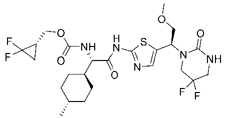 | 580.2 |
| **93** | 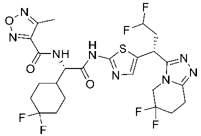 | 621.2 | **599** | 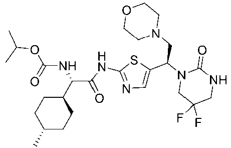 | 587.2 |
| **94** | 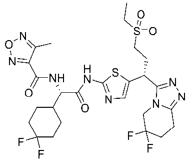 | 677.2 | **600** | 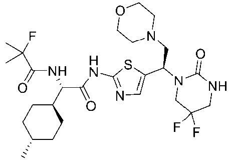 | 589.2 |
| **95** | 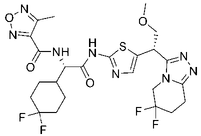 | 601.2 | **601** | 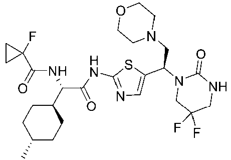 | 587.2 |
| **96** | 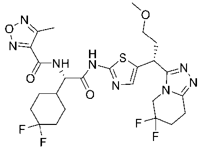 | 615.2 | **602** | 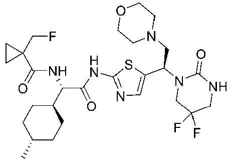 | 601.2 |
| **97** | 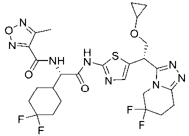 | 627.2 | **603** | 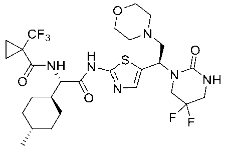 | 637.2 |
| **98** | 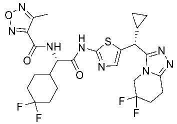 | 597.2 | **604** | 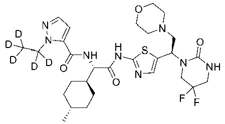 | 628.3 |
| **99** | 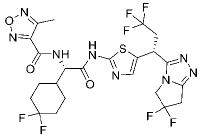 | 625.2 | **605** | 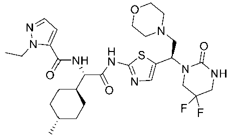 | 623.3 |
| **100** | 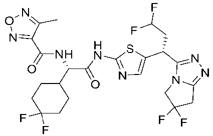 | 607.2 | **606** | 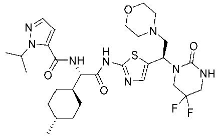 | 637.3 |
| **101** | 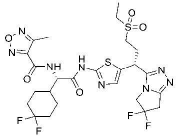 | 663.2 | **607** | 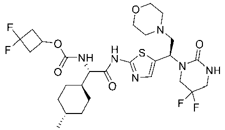 | 635.2 |
| **102** | 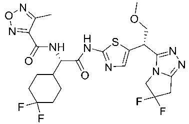 | 587.2 | **608** | 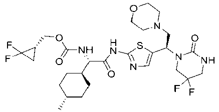 | 635.2 |
| **103** | 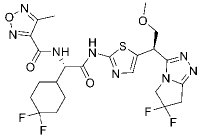 | 587.2 | **609** | 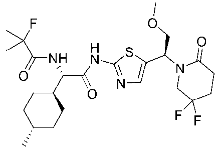 | 533.2 |
| **104** | 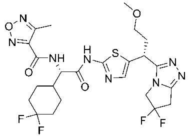 | 601.2 | **610** | 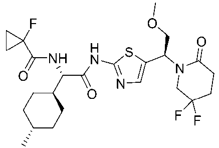 | 531.2 |
| **105** | 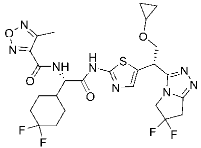 | 613.2 | **611** | 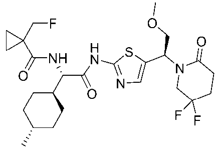 | 545.2 |
| **106** | 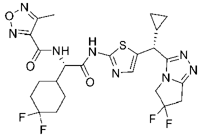 | 583.2 | **612** | 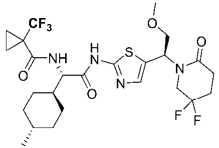 | 581.2 |
| **107** | 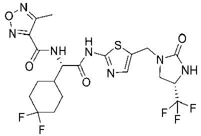 | 552.1 | **613** | 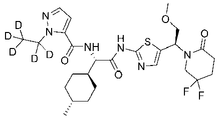 | 572.2 |
| **108** | 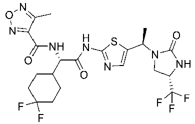 | 566.2 | **614** | 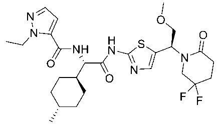 | 567.2 |
| **109** | 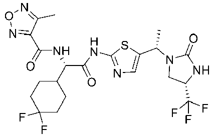 | 566.2 | **615** | 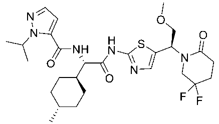 | 581.2 |
| **110** | 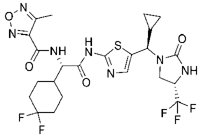 | 592.2 | **616** | 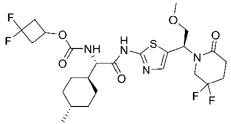 | 579.2 |
| **111** | 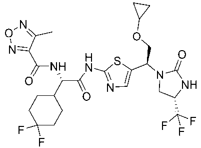 | 622.2 | **617** | 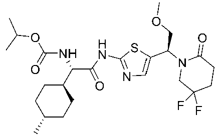 | 531.2 |
| **112** | 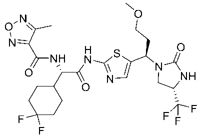 | 610.2 | **618** | 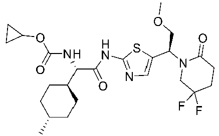 | 529.2 |
| **113** | 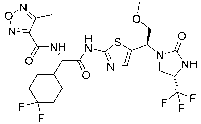 | 596.2 | **619** | 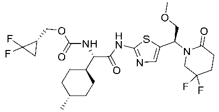 | 579.2 |
| **114** | 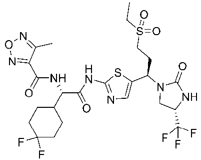 | 672.2 | **620** | 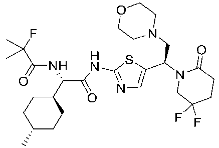 | 588.2 |
| **115** | 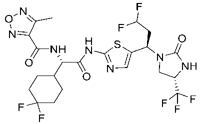 | 616.2 | **621** | 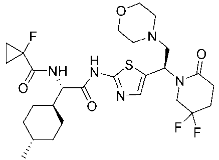 | 586.2 |
| **116** | 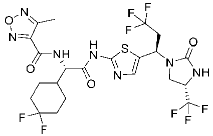 | 634.1 | **622** | 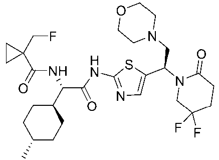 | 600.2 |
| **117** | 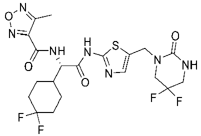 | 534.2 | **623** | 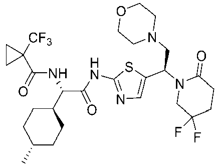 | 636.2 |
| **118** | 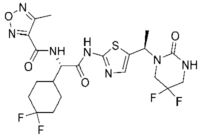 | 548.2 | **624** | 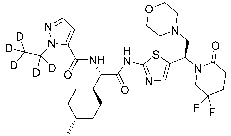 | 627.3 |
| **119** | 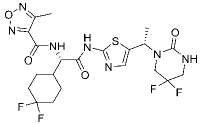 | 548.2 | **625** | 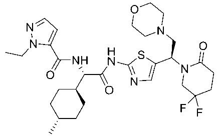 | 622.3 |
| **120** | 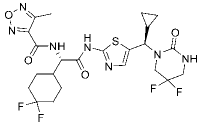 | 574.2 | **626** | 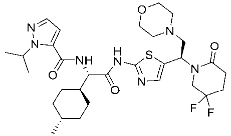 | 636.3 |
| **121** | 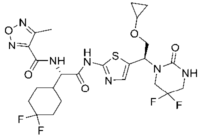 | 604.2 | **627** | 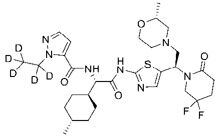 | 641.3 |
| **122** | 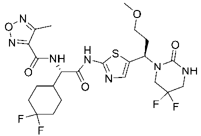 | 592.2 | **628** | 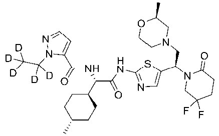 | 641.3 |
| **123** | 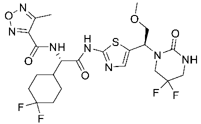 | 578.2 | **629** | 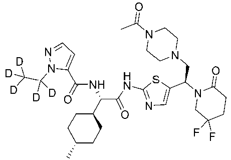 | 668.3 |
| **124** | 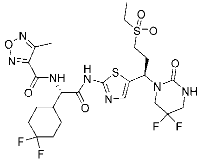 | 654.2 | **630** | 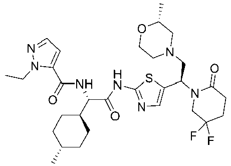 | 636.3 |
| **125** | 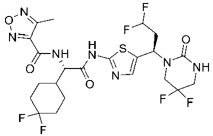 | 598.2 | **631** | 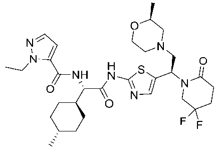 | 636.3 |
| **126** | 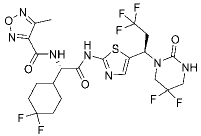 | 616.2 | **632** | 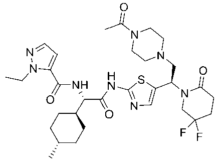 | 663.3 |
| **127** | 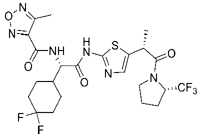 | 579.2 | **633** | 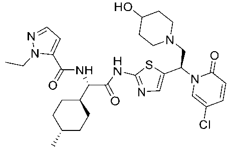 | 630.3 |
| **128** | 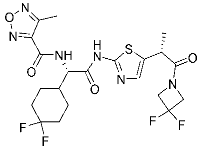 | 533.2 | **634** | 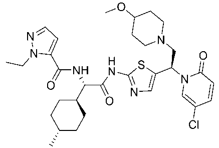 | 644.3 |
| **129** | 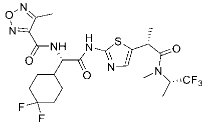 | 567.2 | **635** | 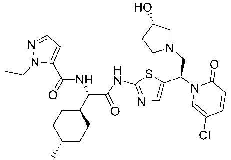 | 616.2 |
| **130** | 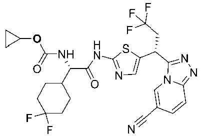 | 598.2 | **636** | 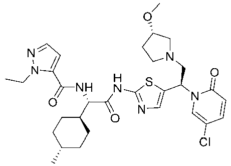 | 630.3 |
| **131** | 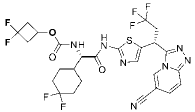 | 648.2 | **637** | 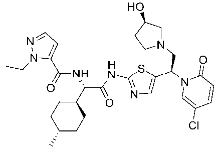 | 616.2 |
| **132** | 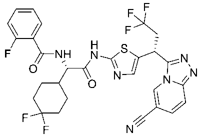 | 636.2 | **638** | 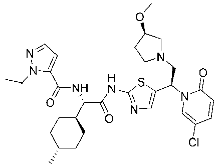 | 630.3 |
| **133** | 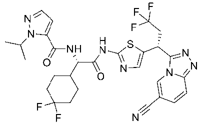 | 650.2 | **639** | 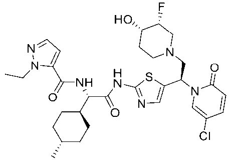 | 648.3 |
| **134** | 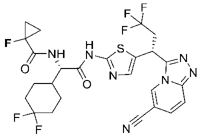 | 600.2 | **640** | 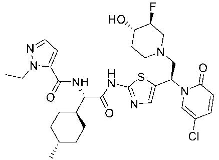 | 648.3 |
| **135** | 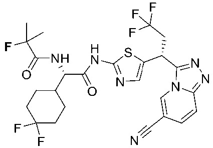 | 602.2 | **641** | 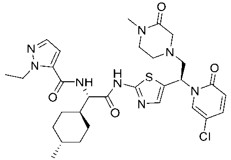 | 643.3 |
| **136** | 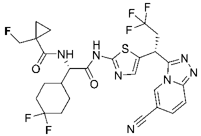 | 614.2 | **642** | 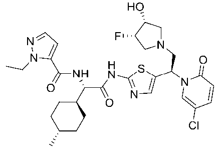 | 634.2 |
| **137** | 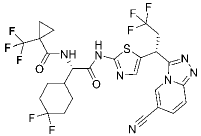 | 650.2 | **643** | 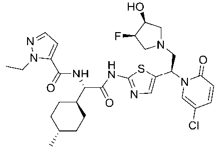 | 634.2 |
| **138** | 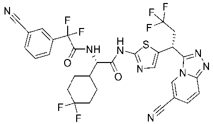 | 693.2 | **644** | 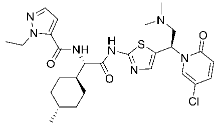 | 574.2 |
| **139** | 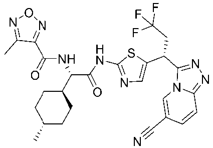 | 602.2 | **645** | 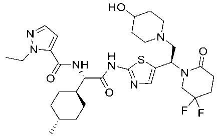 | 636.3 |
| **140** | 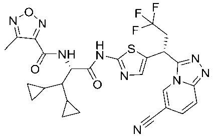 | 600.2 | **646** | 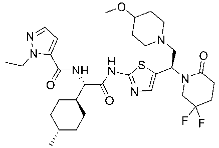 | 650.3 |
| **141** | 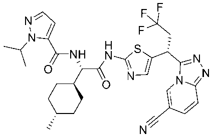 | 628.2 | **647** | 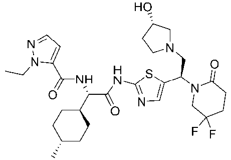 | 622.3 |
| **142** | 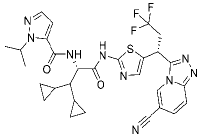 | 626.2 | **648** | 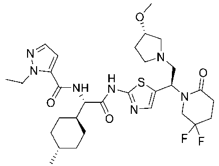 | 636.3 |
| **143** | 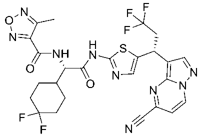 | 624.1 | **649** | 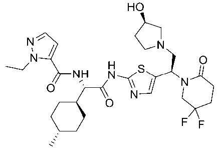 | 622.3 |
| **144** | 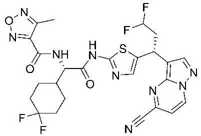 | 606.2 | **650** | 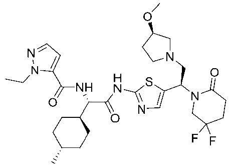 | 636.3 |
| **145** | 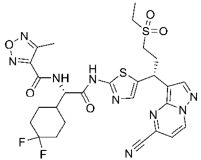 | 662.1 | **651** | 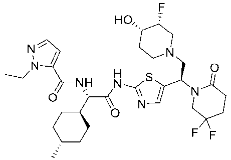 | 654.3 |
| **146** | 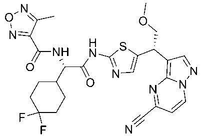 | 586.2 | **652** | 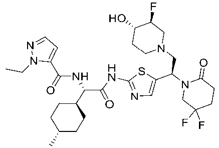 | 654.3 |
| **147** | 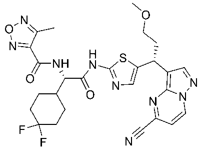 | 600.2 | **653** | 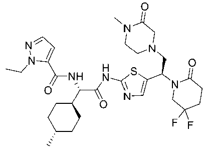 | 649.3 |
| **148** | 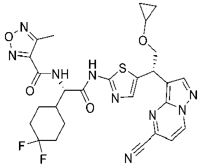 | 612.2 | **654** | 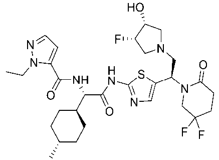 | 640.3 |
| **149** | 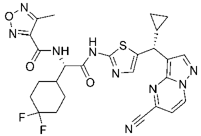 | 582.2 | **655** | 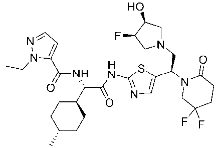 | 640.3 |
| **150** | 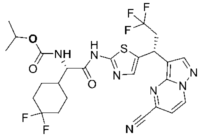 | 600.2 | **656** | 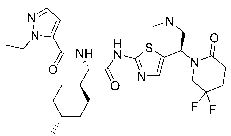 | 580.3 |
| **151** | 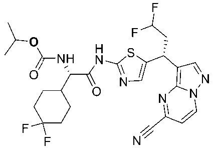 | 582.2 | **657** | 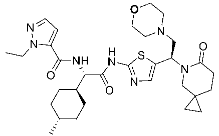 | 612.4 |
| **152** | 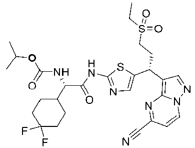 | 638.2 | **658** | 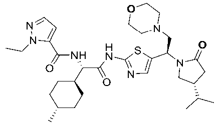 | 614.3 |
| **153** | 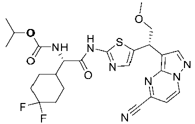 | 562.2 | **659** | 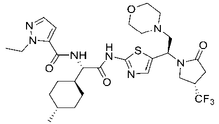 | 640.3 |
| **154** | 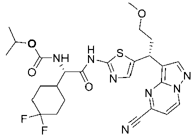 | 576.2 | **660** | 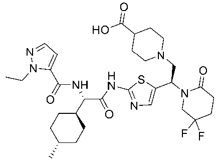 | 664.3 |
| **155** | 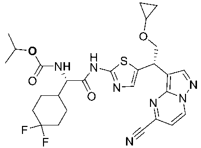 | 588.2 | **661** | 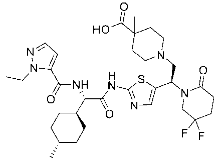 | 678.3 |
| **156** | 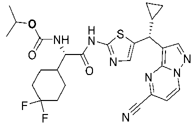 | 558.2 | **662** | 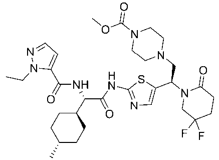 | 679.3 |
| **157** | 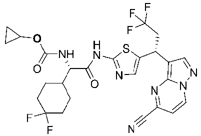 | 598.1 | **663** | 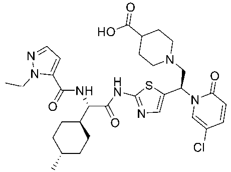 | 658.3 |
| **158** | 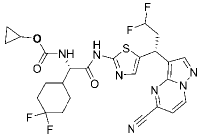 | 580.2 | **664** | 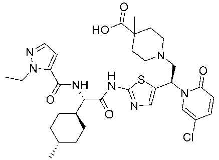 | 672.3 |
| **159** | 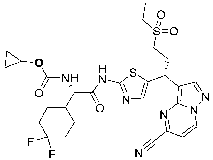 | 636.2 | **665** | 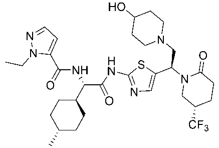 | 668.3 |
| **160** | 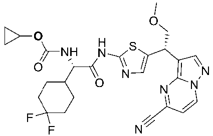 | 560.2 | **666** | 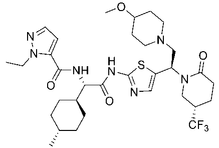 | 682.3 |
| **161** | 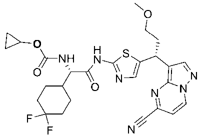 | 574.2 | **667** | 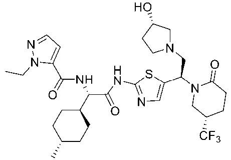 | 654.3 |
| **162** | 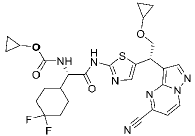 | 586.2 | **668** | 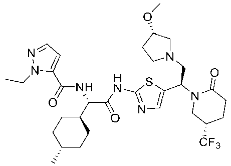 | 668.3 |
| **163** | 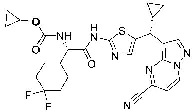 | 556.2 | **669** | 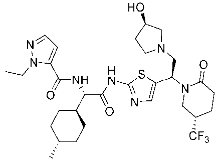 | 654.3 |
| **164** | 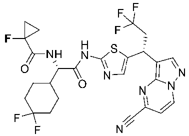 | 600.1 | **670** | 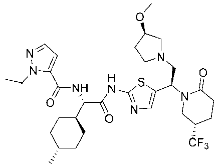 | 668.3 |
| **165** | 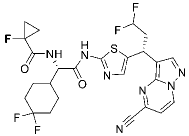 | 582.2 | **671** | 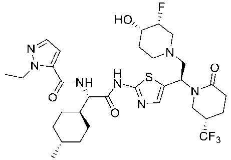 | 686.3 |
| **166** | 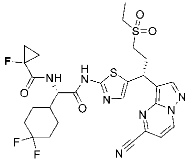 | 638.2 | **672** | 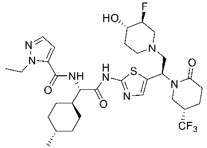 | 686.3 |
| **167** | 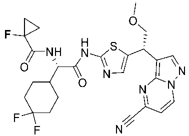 | 562.2 | **673** | 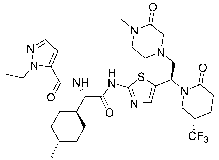 | 681.3 |
| **168** | 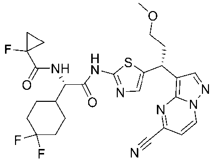 | 576.2 | **674** | 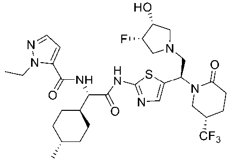 | 672.3 |
| **169** | 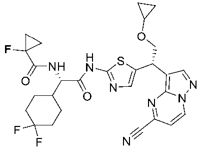 | 588.2 | **675** | 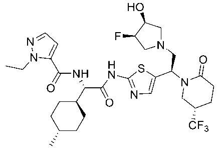 | 672.3 |
| **170** | 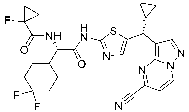 | 558.2 | **676** | 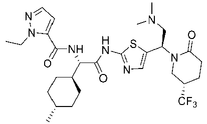 | 612.3 |
| **171** | 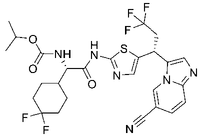 | 599.2 | **677** | 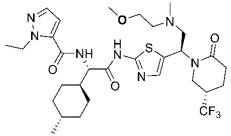 | 656.3 |
| **172** | 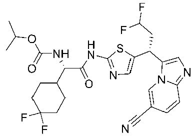 | 581.2 | **678** | 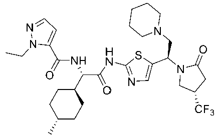 | 638.3 |
| **173** | 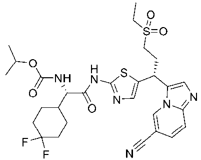 | 637.2 | **679** | 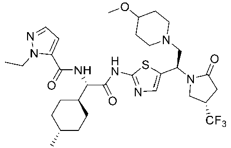 | 668.3 |
| **174** | 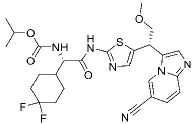 | 561.2 | **680** | 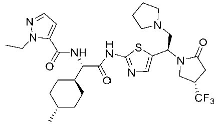 | 624.3 |
| **175** | 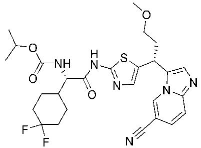 | 575.2 | **681** | 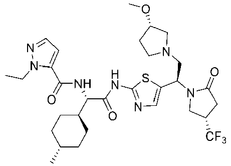 | 654.3 |
| **176** | 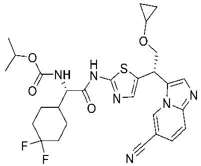 | 587.2 | **682** | 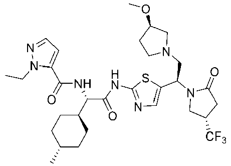 | 654.3 |
| **177** | 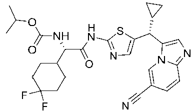 | 557.2 | **683** | 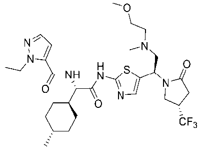 | 642.3 |
| **178** | 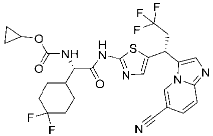 | 597.2 | **684** | 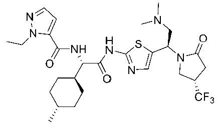 | 598.3 |
| **179** | 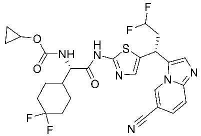 | 579.2 | **685** | 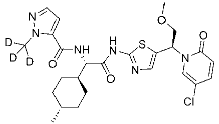 | 550.2 |
| **180** | 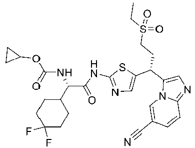 | 635.2 | **686** | 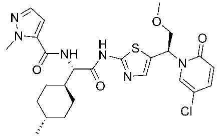 | 547.2 |
| **181** | 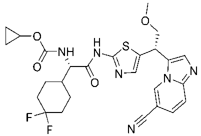 | 559.2 | **687** | 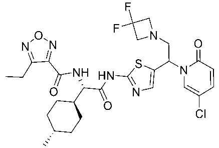 | 624.2 |
| **182** | 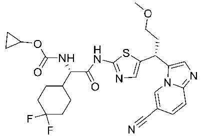 | 573.2 | **688** | 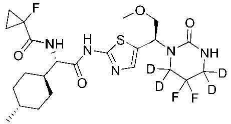 | 536.2 |
| **183** | 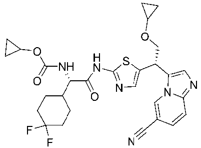 | 585.2 | **689** | 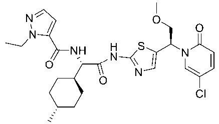 | 561.2 |
| **184** | 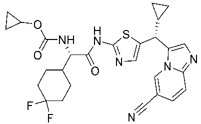 | 555.2 | **690** | 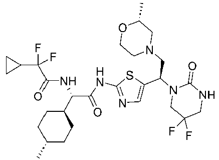 | 633.3 |
| **185** | 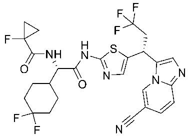 | 599.2 | **691** | 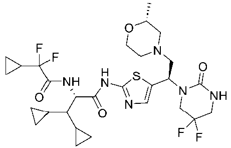 | 631.3 |
| **186** | 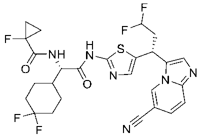 | 581.2 | **692** | 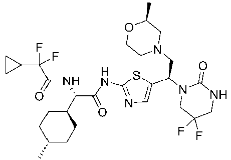 | 633.3 |
| **187** | 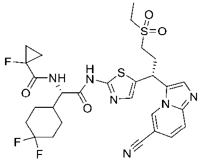 | 637.2 | **693** | 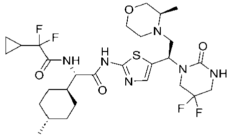 | 633.3 |
| **188** | 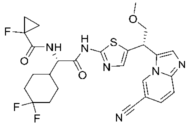 | 561.2 | **694** | 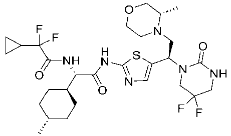 | 633.3 |
| **189** | 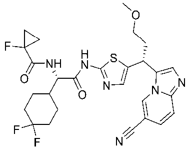 | 575.2 | **695** | 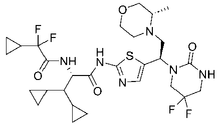 | 631.3 |
| **190** | 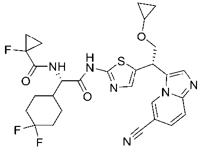 | 587.2 | **696** | 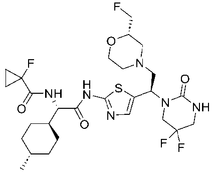 | 619.3 |
| **191** | 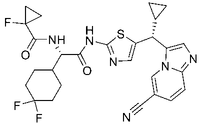 | 557.2 | **697** | 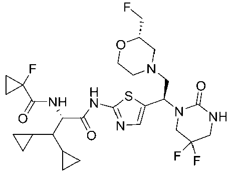 | 617.2 |
| **192** | 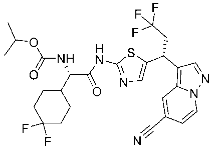 | 599.2 | **698** | 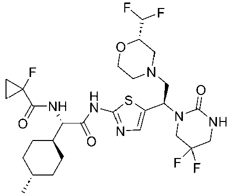 | 637.2 |
| **193** | 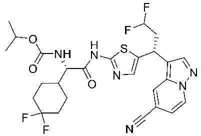 | 581.2 | **699** | 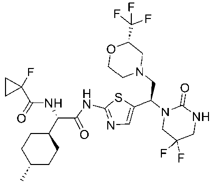 | 655.2 |
| **194** | 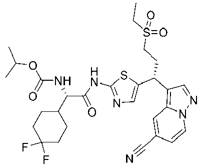 | 637.2 | **700** | 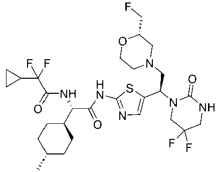 | 651.3 |
| **195** | 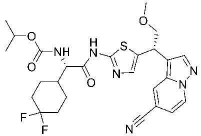 | 561.2 | **701** | 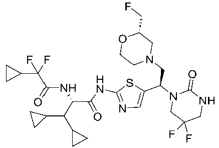 | 649.3 |
| **196** | 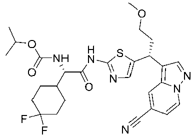 | 575.2 | **702** | 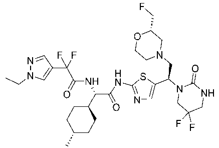 | 705.3 |
| **197** | 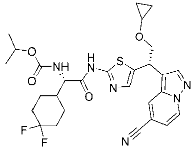 | 587.2 | **703** | 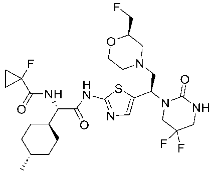 | 619.3 |
| **198** | 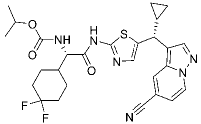 | 557.2 | **704** | 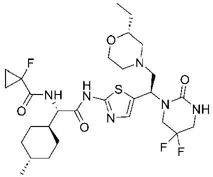 | 615.3 |
| **199** | 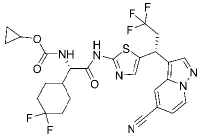 | 597.1 | **705** | 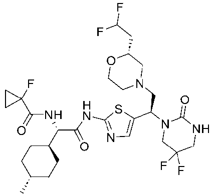 | 651.3 |
| **200** | 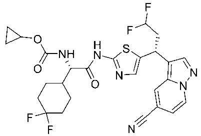 | 579.2 | **706** | 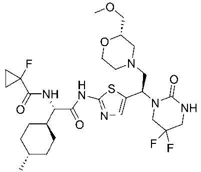 | 631.3 |
| **201** | 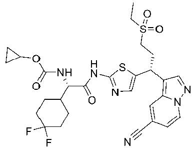 | 635.2 | **707** | 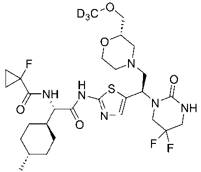 | 634.3 |
| **202** | 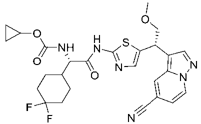 | 559.2 | **708** | 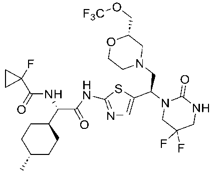 | 685.3 |
| **203** | 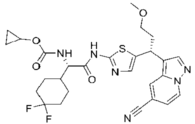 | 573.2 | **709** | 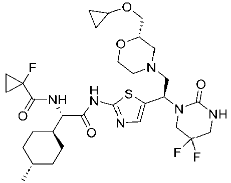 | 657.3 |
| **204** | 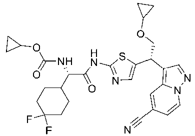 | 585.2 | **710** | 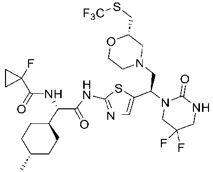 | 701.2 |
| **205** | 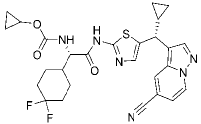 | 555.2 | **711** | 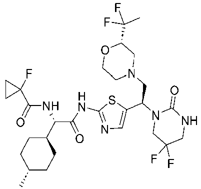 | 651.3 |
| **206** | 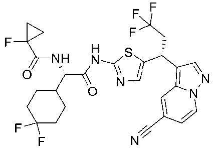 | 599.2 | **712** | 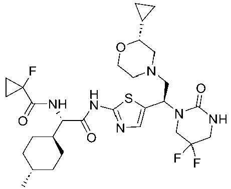 | 627.3 |
| **207** | 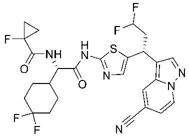 | 581.2 | **713** | 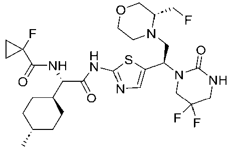 | 619.3 |
| **208** | 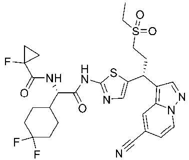 | 637.2 | **714** | 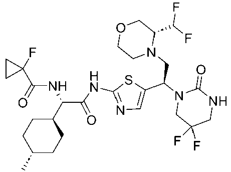 | 637.2 |
| **209** | 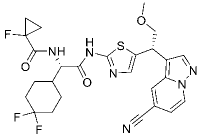 | 561.2 | **715** | 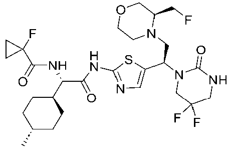 | 619.3 |
| **210** | 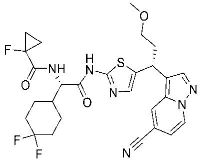 | 575.2 | **716** | 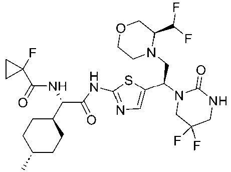 | 637.3 |
| **211** | 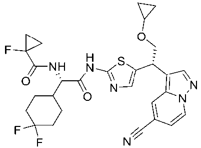 | 587.2 | **717** | 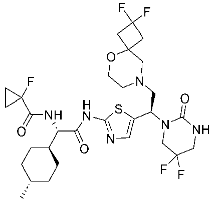 | 663.3 |
| **212** | 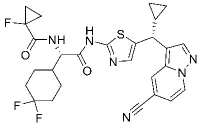 | 557.2 | **718** | 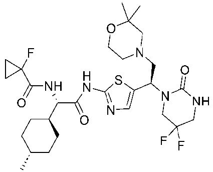 | 615.3 |
| **213** | 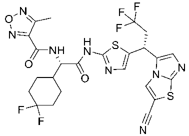 | 629.1 | **719** | 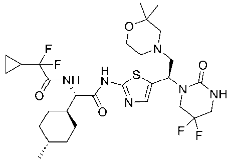 | 647.3 |
| **214** | 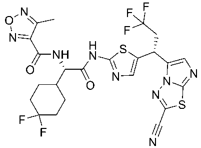 | 630.1 | **720** | 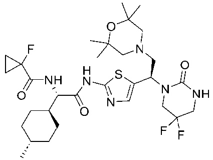 | 643.3 |
| **215** | 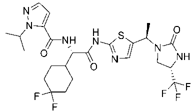 | 592.2 | **721** | 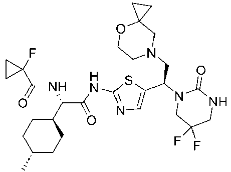 | 613.3 |
| **216** | 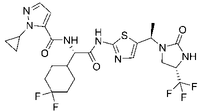 | 590.2 | **722** | 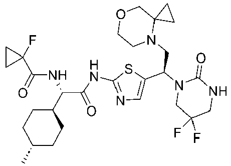 | 613.3 |
| **217** | 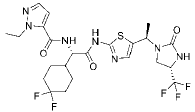 | 578.2 | **723** | 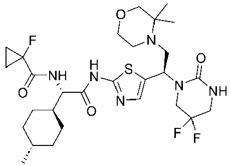 | 615.3 |
| **218** | 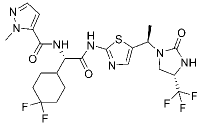 | 564.2 | **724** | 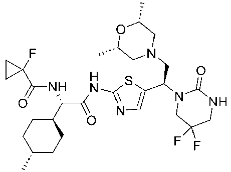 | 615.3 |
| **219** | 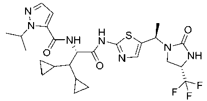 | 568.2 | **725** | 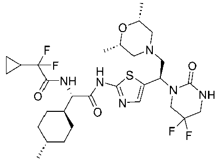 | 647.3 |
| **220** | 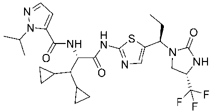 | 582.2 | **726** | 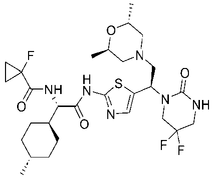 | 615.3 |
| **221** | 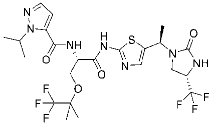 | 614.2 | **727** | 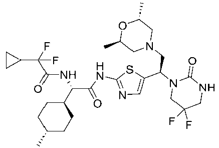 | 647.3 |
| **222** | 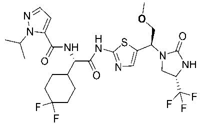 | 622.2 | **728** | 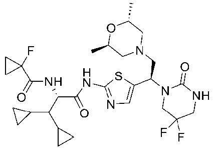 | 613.3 |
| **223** | 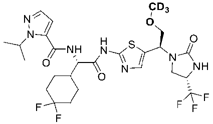 | 625.2 | **729** | 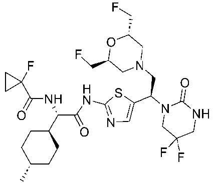 | 651.3 |
| **224** | 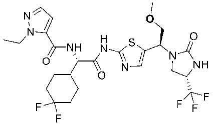 | 608.2 | **730** | 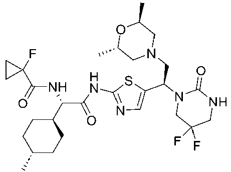 | 615.3 |
| **225** | 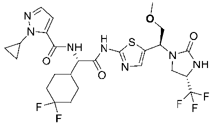 | 620.2 | **731** | 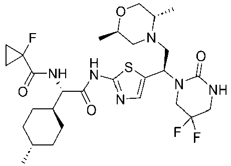 | 615.3 |
| **226** | 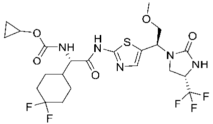 | 570.2 | **732** | 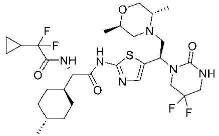 | 647.3 |
| **227** | 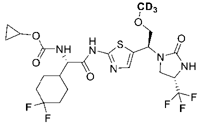 | 573.2 | **733** | 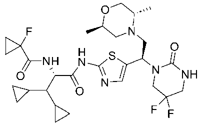 | 613.3 |
| **228** | 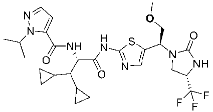 | 598.2 | **734** | 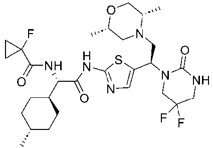 | 615.3 |
| **229** | 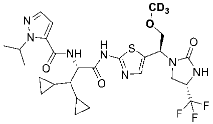 | 601.2 | **735** | 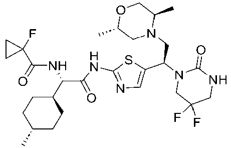 | 615.3 |
| **230** | 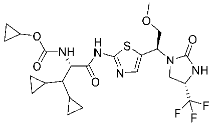 | 546.2 | **736** | 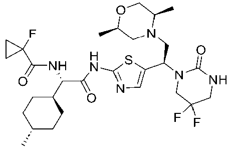 | 615.3 |
| **231** | 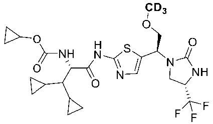 | 549.2 | **737** | 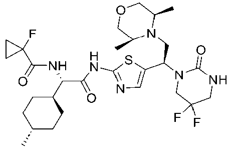 | 615.3 |
| **232** | 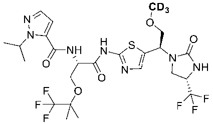 | 647.2 | **738** | 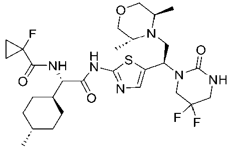 | 615.3 |
| **233** | 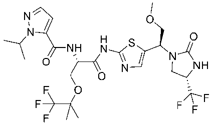 | 644.2 | **739** | 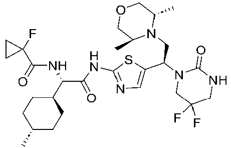 | 615.3 |
| **234** | 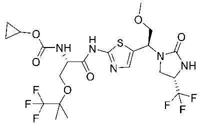 | 592.2 | **740** | 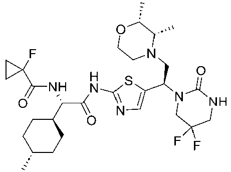 | 615.3 |
| **235** | 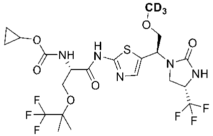 | 595.2 | **741** | 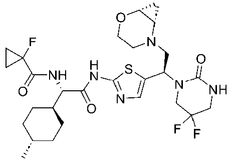 | 599.3 |
| **236** | 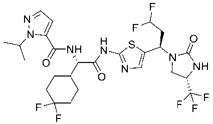 | 642.2 | **742** | 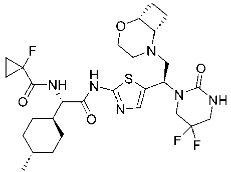 | 613.3 |
| **237** | 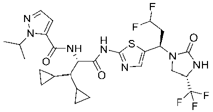 | 618.2 | **743** | 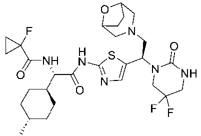 | 613.3 |
| **238** | 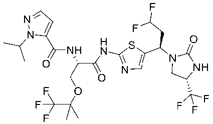 | 664.2 | **744** | 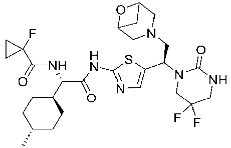 | 599.3 |
| **239** | 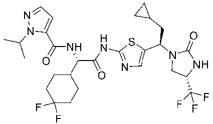 | 632.2 | **745** | 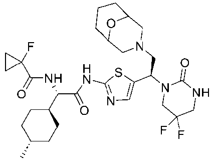 | 627.3 |
| **240** | 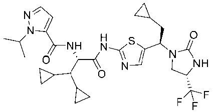 | 608.3 | **746** | 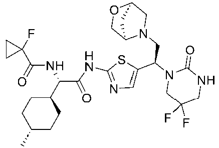 | 599.3 |
| **241** | 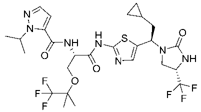 | 654.2 | **747** | 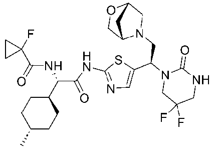 | 599.3 |
| **242** | 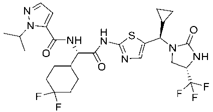 | 618.2 | **748** | 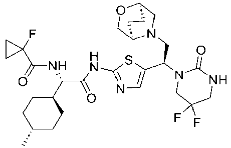 | 613.3 |
| **243** | 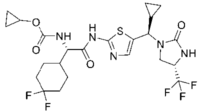 | 566.2 | **749** | 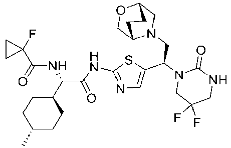 | 613.3 |
| **244** | 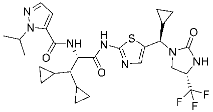 | 594.2 | **750** | 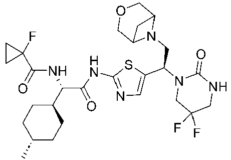 | 599.3 |
| **245** | 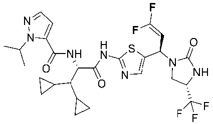 | 616.2 | **751** | 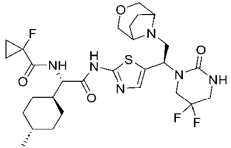 | 613.3 |
| **246** | 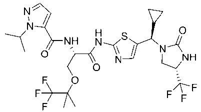 | 640.2 | **752** | 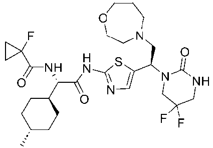 | 601.3 |
| **247** | 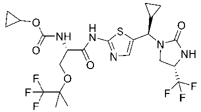 | 588.2 | **753** | 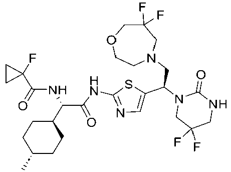 | 637.3 |
| **248** | 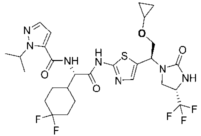 | 648.2 | **754** | 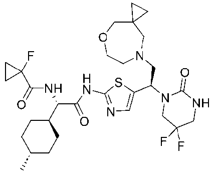 | 627.3 |
| **249** | 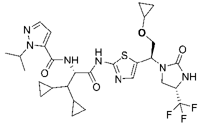 | 624.2 | **755** | 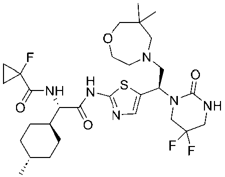 | 629.3 |
| **250** | 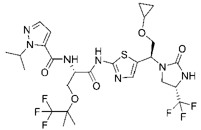 | 670.2 | **756** | 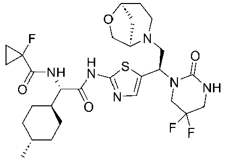 | 613.3 |
| **251** | 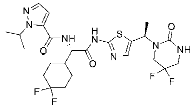 | 574.2 | **757** | 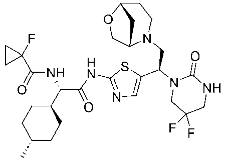 | 613.3 |
| **252** | 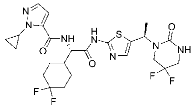 | 572.2 | **758** | 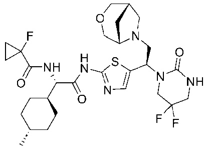 | 613.3 |
| **253** | 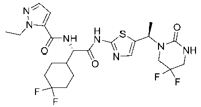 | 560.2 | **759** | 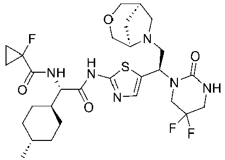 | 613.3 |
| **254** | 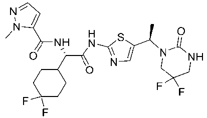 | 546.2 | **760** | 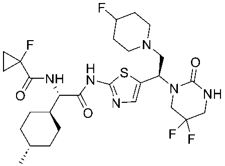 | 603.3 |
| **255** | 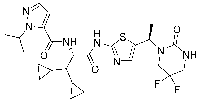 | 550.2 | **761** | 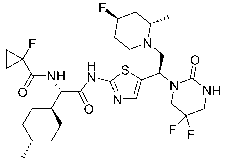 | 617.3 |
| **256** | 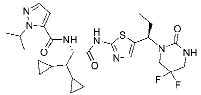 | 564.2 | **762** | 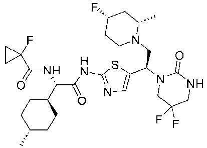 | 617.3 |
| **257** | 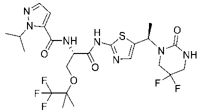 | 596.2 | **763** | 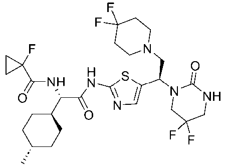 | 621.3 |
| **258** | 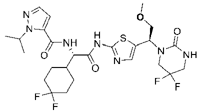 | 604.2 | **764** | 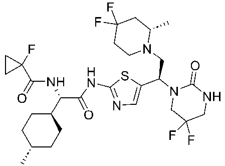 | 635.3 |
| **259** | 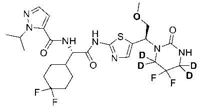 | 608.2 | **765** | 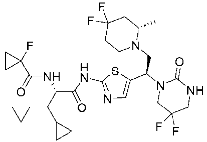 | 633.3 |
| **260** | 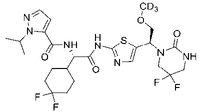 | 607.2 | **766** | 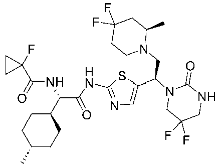 | 635.3 |
| **261** | 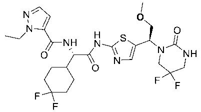 | 590.2 | **767** | 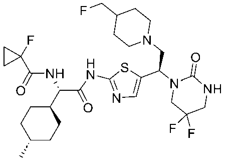 | 617.3 |
| **262** | 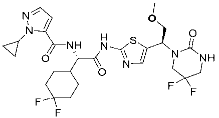 | 602.2 | **768** | 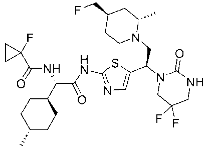 | 631.3 |
| **263** | 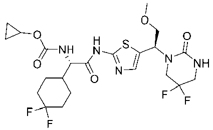 | 552.2 | **769** | 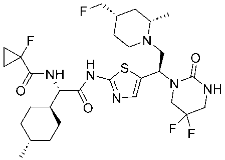 | 631.3 |
| **264** | 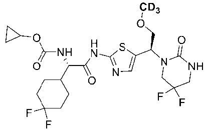 | 555.2 | **770** | 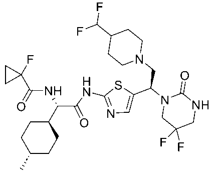 | 635.3 |
| **265** | 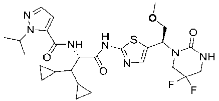 | 580.2 | **771** | 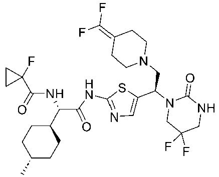 | 633.3 |
| **266** | 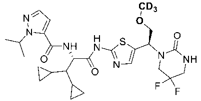 | 583.3 | **772** | 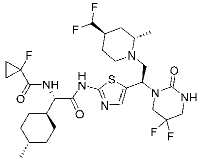 | 649.3 |
| **267** | 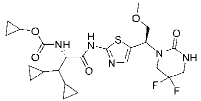 | 528.2 | **773** | 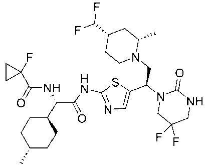 | 649.3 |
| **268** | 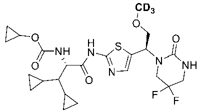 | 531.2 | **774** | 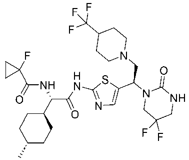 | 653.3 |
| **269** | 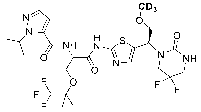 | 629.2 | **775** | 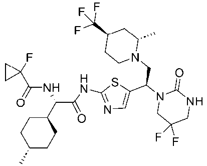 | 667.3 |
| **270** | 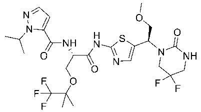 | 626.2 | **776** | 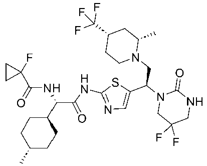 | 667.3 |
| **271** | 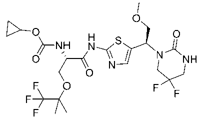 | 574.2 | **777** | 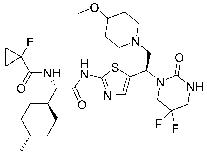 | 615.3 |
| **272** | 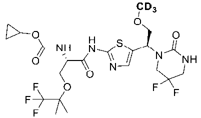 | 577.2 | **778** | 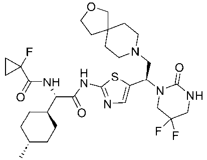 | 641.3 |
| **273** | 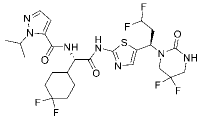 | 624.2 | **779** | 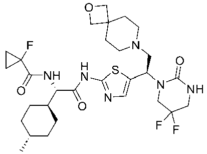 | 627.3 |
| **274** | 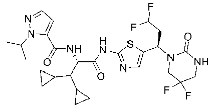 | 600.2 | **780** | 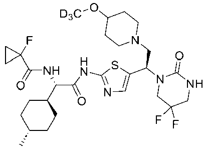 | 618.3 |
| **275** | 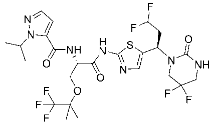 | 646.2 | **781** | 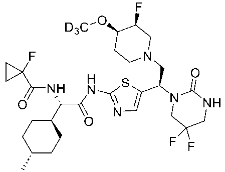 | 636.3 |
| **276** | 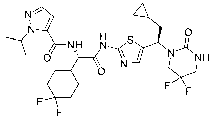 | 614.2 | **782** | 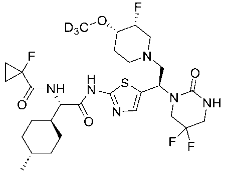 | 636.3 |
| **277** | 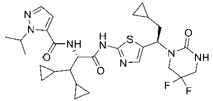 | 590.3 | **783** | 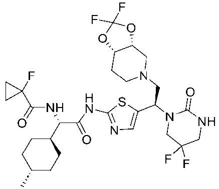 | 665.3 |
| **278** | 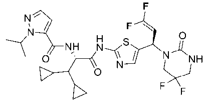 | 598.2 | **784** | 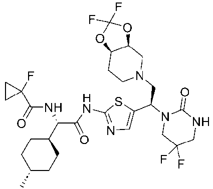 | 665.3 |
| **279** | 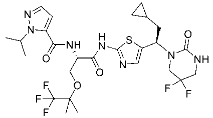 | 636.2 | **785** | 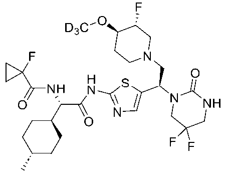 | 636.3 |
| **280** | 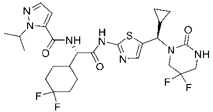 | 600.2 | **786** | 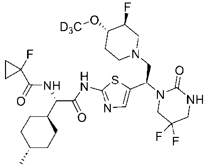 | 636.3 |
| **281** | 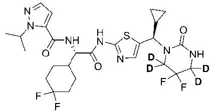 | 604.3 | **787** | 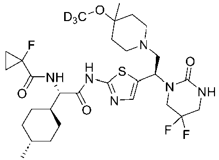 | 632.3 |
| **282** | 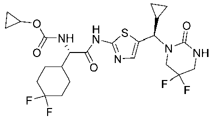 | 548.2 | **788** | 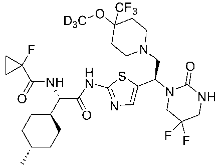 | 686.3 |
| **283** | 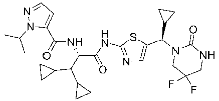 | 576.3 | **789** | 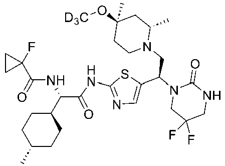 | 646.3 |
| **284** | 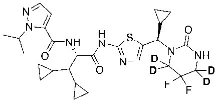 | 580.3 | **790** | 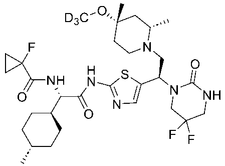 | 646.3 |
| **285** | 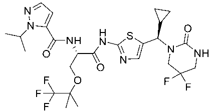 | 622.2 | **791** | 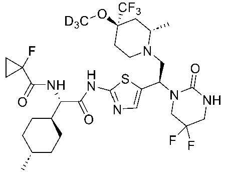 | 700.3 |
| **286** | 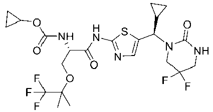 | 570.2 | **792** | 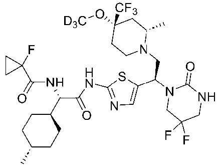 | 700.3 |
| **287** | 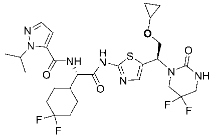 | 630.2 | **793** | 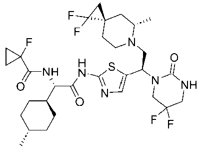 | 661.3 |
| **288** | 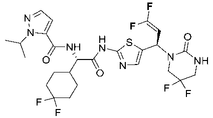 | 622.2 | **794** | 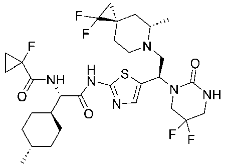 | 661.3 |
| **289** | 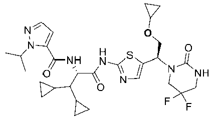 | 606.3 | **795** | 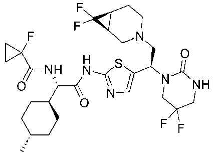 | 633.3 |
| **290** | 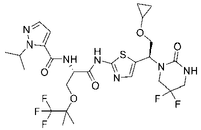 | 652.2 | **796** | 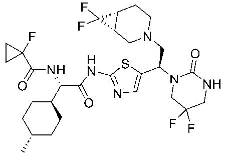 | 633.3 |
| **291** | 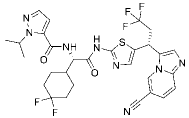 | 649.2 | **797** | 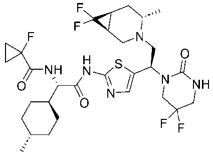 | 647.3 |
| **292** | 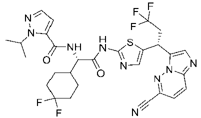 | 650.2 | **798** | 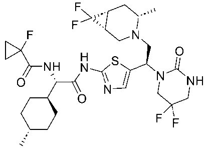 | 647.3 |
| **293** | 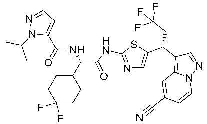 | 649.2 | **799** | 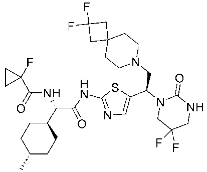 | 661.3 |
| **294** | 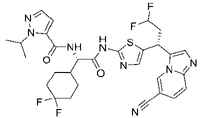 | 631.2 | **800** | 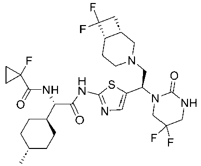 | 647.3 |
| **295** | 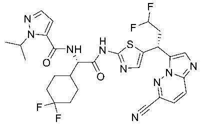 | 632.2 | **801** | 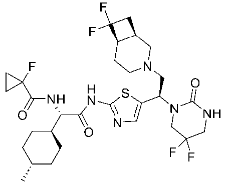 | 647.3 |
| **296** | 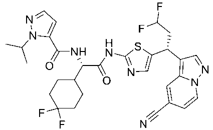 | 631.2 | **802** | 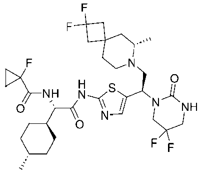 | 675.3 |
| **297** | 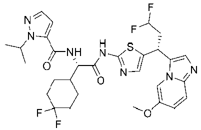 | 636.2 | **803** | 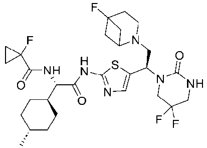 | 615.3 |
| **298** | 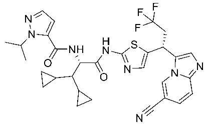 | 625.2 | **804** | 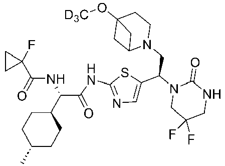 | 630.3 |
| **299** | 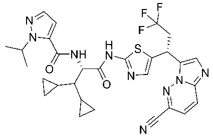 | 626.2 | **805** | 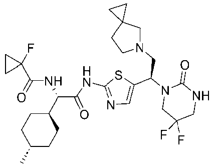 | 597.3 |
| **300** | 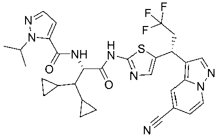 | 625.2 | **806** | 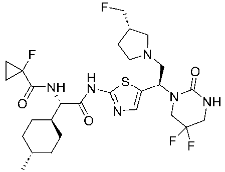 | 603.3 |
| **301** | 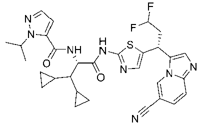 | 607.2 | **807** | 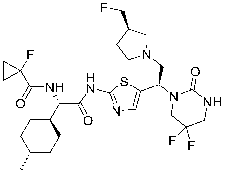 | 603.3 |
| **302** | 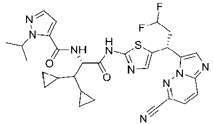 | 608.2 | **808** | 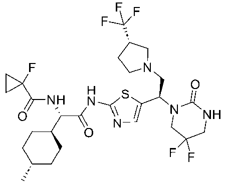 | 639.3 |
| **303** | 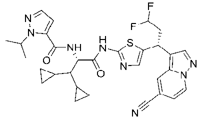 | 607.2 | **809** | 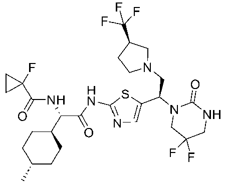 | 639.3 |
| **304** | 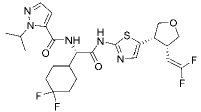 | 544.2 | **810** | 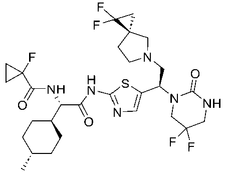 | 633.3 |
| **305** | 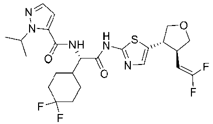 | 544.2 | **811** | 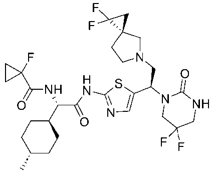 | 633.3 |
| **306** | 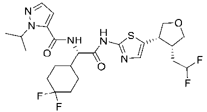 | 546.2 | **812** | 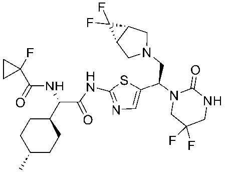 | 619.2 |
| **307** | 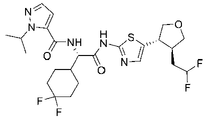 | 546.2 | **813** | 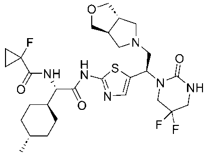 | 613.3 |
| **308** | 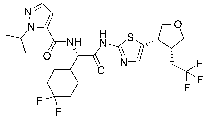 | 564.2 | **814** | 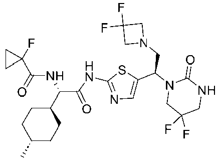 | 593.2 |
| **309** | 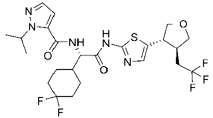 | 564.2 | **815** | 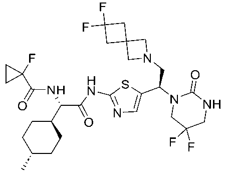 | 633.3 |
| **310** | 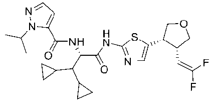 | 520.2 | **816** | 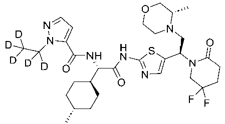 | 641.3 |
| **311** | 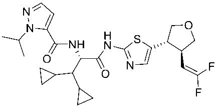 | 520.2 | **817** | 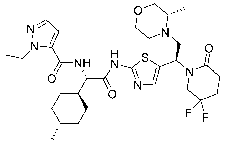 | 636.3 |
| **312** | 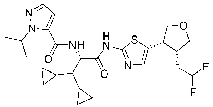 | 522.2 | **818** | 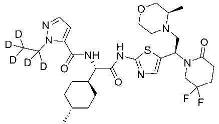 | 641.3 |
| **313** | 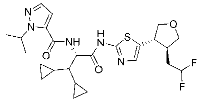 | 522.2 | **819** | 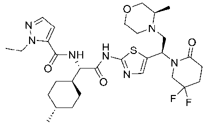 | 636.3 |
| **314** | 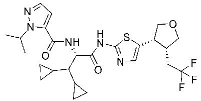 | 540.2 | **820** | 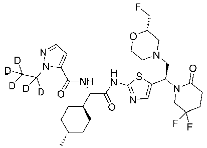 | 659.3 |
| **315** | 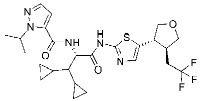 | 540.2 | **821** | 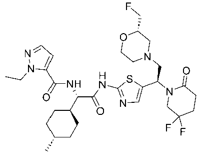 | 654.3 |
| **316** | 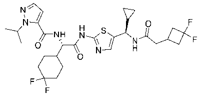 | 613.3 | **822** | 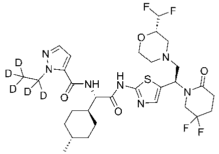 | 677.3 |
| **317** | 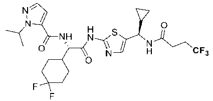 | 605.2 | **823** | 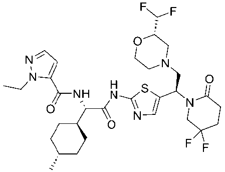 | 672.3 |
| **318** | 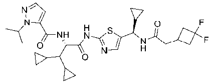 | 589.3 | **824** | 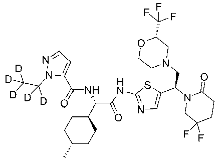 | 695.3 |
| **319** | 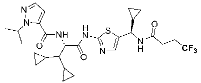 | 581.2 | **825** | 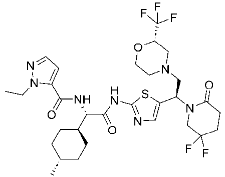 | 690.3 |
| **320** | 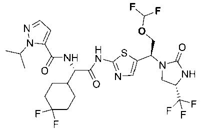 | 658.2 | **826** | 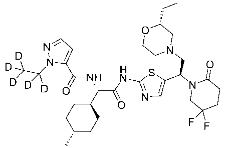 | 655.3 |
| **321** | 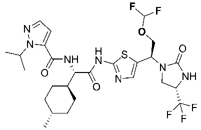 | 636.2 | **827** | 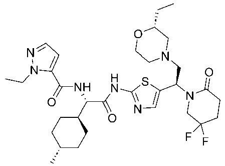 | 650.3 |
| **322** | 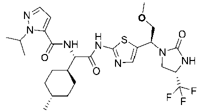 | 600.2 | **828** | 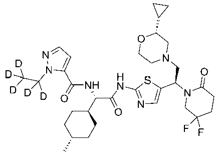 | 667.3 |
| **323** | 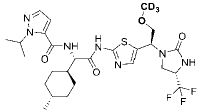 | 603.3 | **829** | 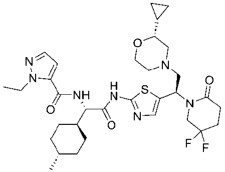 | 662.3 |
| **324** | 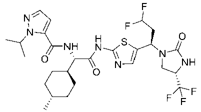 | 620.2 | **830** | 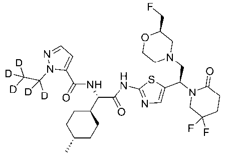 | 659.3 |
| **325** | 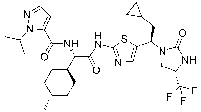 | 610.3 | **831** | 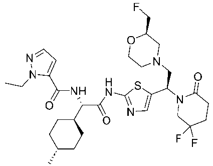 | 654.3 |
| **326** | 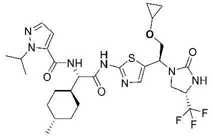 | 626.3 | **832** | 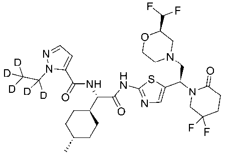 | 677.3 |
| **327** | 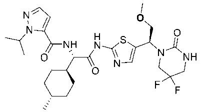 | 582.3 | **833** | 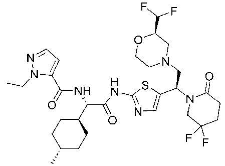 | 672.3 |
| **328** | 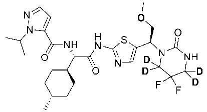 | 586.3 | **834** | 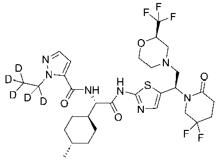 | 695.3 |
| **329** | 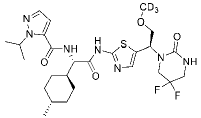 | 585.3 | **835** | 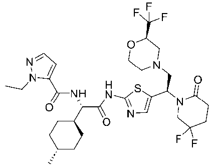 | 690.3 |
| **330** | 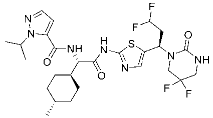 | 602.2 | **836** | 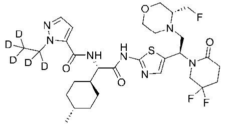 | 659.3 |
| **331** | 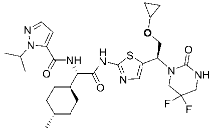 | 608.3 | **837** | 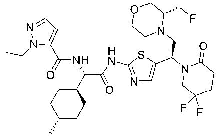 | 654.3 |
| **332** | 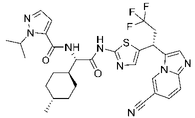 | 627.2 | **838** | 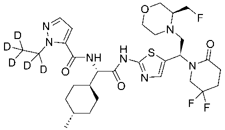 | 659.3 |
| **333** | 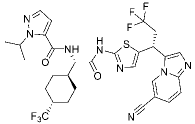 | 681.2 | **839** | 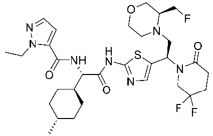 | 654.3 |
| **334** | 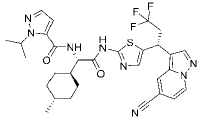 | 627.2 | **840** | 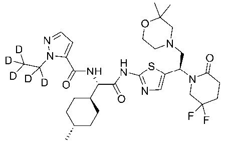 | 655.3 |
| **335** | 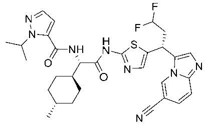 | 609.2 | **841** | 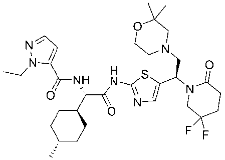 | 650.3 |
| **336** | 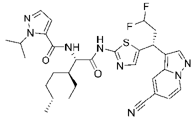 | 609.2 | **842** | 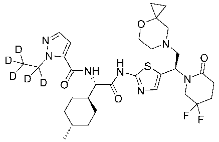 | 653.3 |
| **337** | 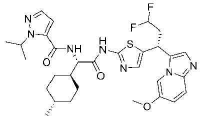 | 614.3 | **843** | 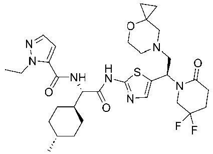 | 648.3 |
| **338** | 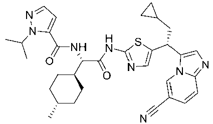 | 599.3 | **844** | 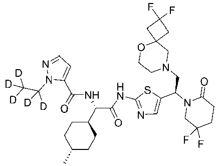 | 703.3 |
| **339** | 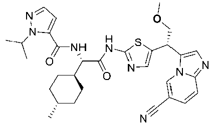 | 589.3 | **845** | 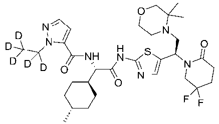 | 655.3 |
| **340** | 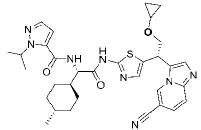 | 615.3 | **846** | 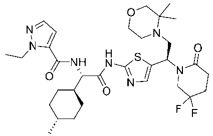 | 650.3 |
| **341** | 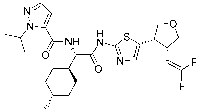 | 522.2 | **847** | 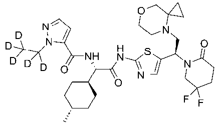 | 653.3 |
| **342** | 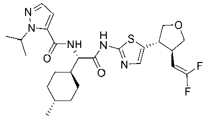 | 522.2 | **848** | 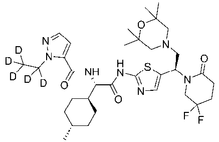 | 683.3 |
| **343** | 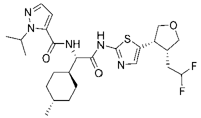 | 524.2 | **849** | 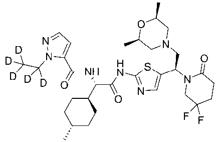 | 655.3 |
| **344** | 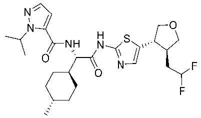 | 524.2 | **850** | 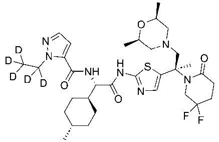 | 669.3 |
| **345** | 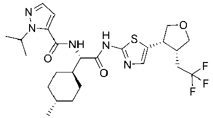 | 542.2 | **851** | 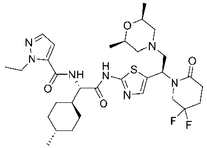 | 650.3 |
| **346** | 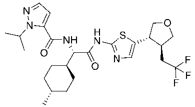 | 542.2 | **852** | 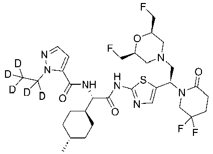 | 691.3 |
| **347** | 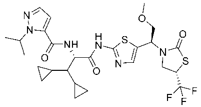 | 615.2 | **853** | 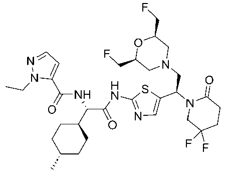 | 686.3 |
| **348** | 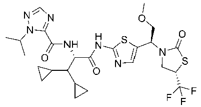 | 616.2 | **854** | 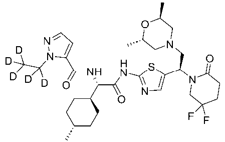 | 655.3 |
| **349** | 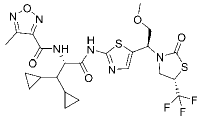 | 589.2 | **855** | 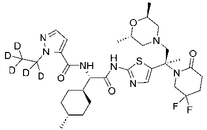 | 669.3 |
| **350** | 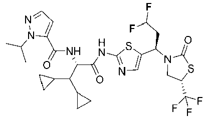 | 635.2 | **856** | 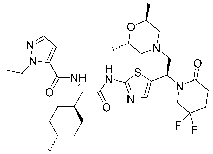 | 650.3 |
| **351** | 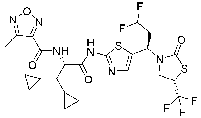 | 609.2 | **857** | 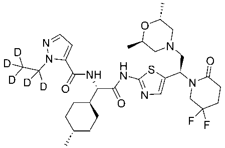 | 655.3 |
| **352** | 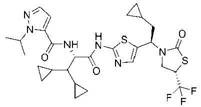 | 625.2 | **858** | 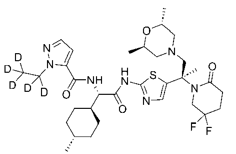 | 669.3 |
| **353** | 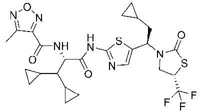 | 599.2 | **859** | 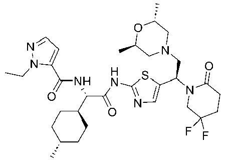 | 650.3 |
| **354** | 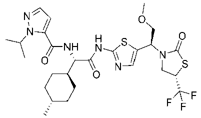 | 617.2 | **860** | 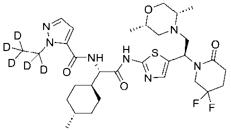 | 655.3 |
| **355** | 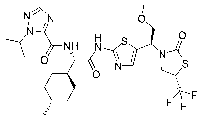 | 618.2 | **861** | 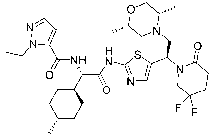 | 650.3 |
| **356** | 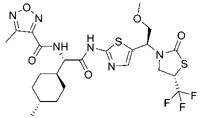 | 591.2 | **862** | 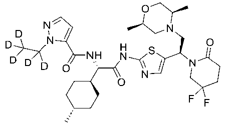 | 655.3 |
| **357** | 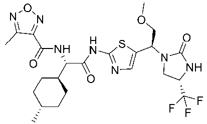 | 574.2 | **863** | 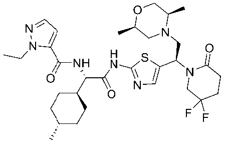 | 650.3 |
| **358** | 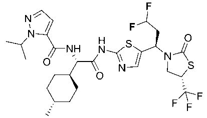 | 637.2 | **864** | 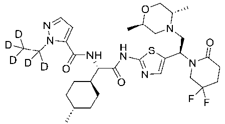 | 655.3 |
| **359** | 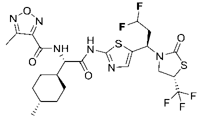 | 611.2 | **865** | 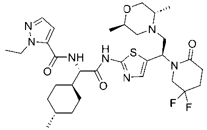 | 650.3 |
| **360** | 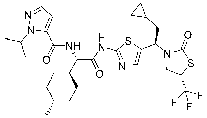 | 627.2 | **866** | 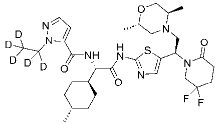 | 655.3 |
| **361** | 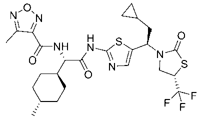 | 601.2 | **867** | 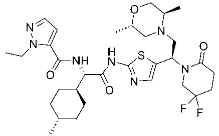 | 650.3 |
| **362** | 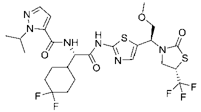 | 639.2 | **868** | 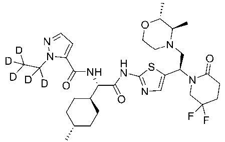 | 655.3 |
| **363** | 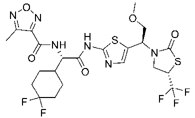 | 613.1 | **869** | 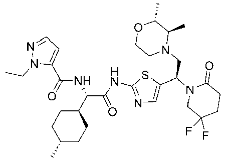 | 650.3 |
| **364** | 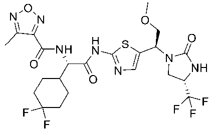 | 596.2 | **870** | 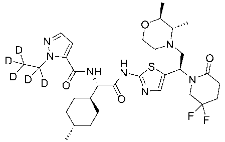 | 655.3 |
| **365** | 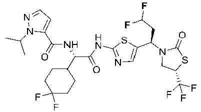 | 659.2 | **871** | 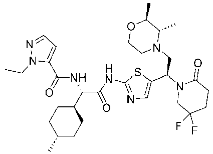 | 650.3 |
| **366** | 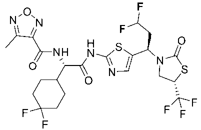 | 633.1 | **872** | 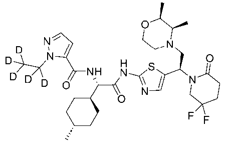 | 655.3 |
| **367** | 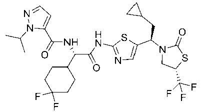 | 649.2 | **873** | 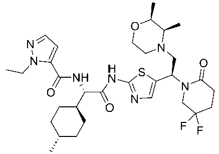 | 650.3 |
| **368** | 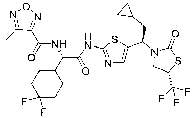 | 623.2 | **874** | 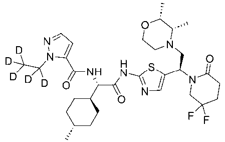 | 655.3 |
| **369** | 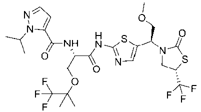 | 661.2 | **875** | 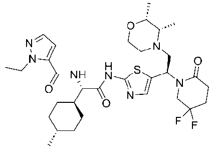 | 650.3 |
| **370** | 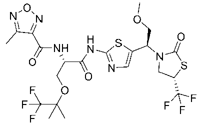 | 635.1 | **876** | 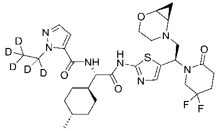 | 639.3 |
| **371** | 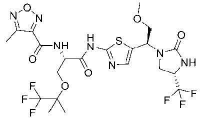 | 618.2 | **877** | 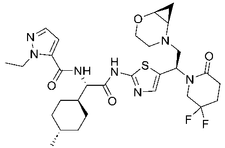 | 634.3 |
| **372** | 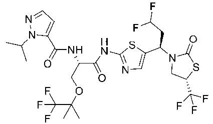 | 681.2 | **878** | 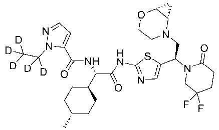 | 639.3 |
| **373** | 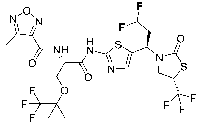 | 655.1 | **879** | 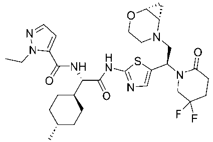 | 634.3 |
| **374** | 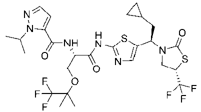 | 671.2 | **880** | 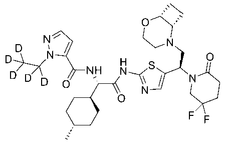 | 653.3 |
| **375** | 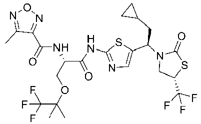 | 645.1 | **881** | 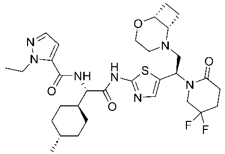 | 648.3 |
| **376** | 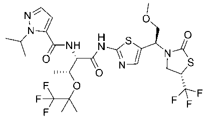 | 675.2 | **882** | 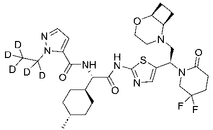 | 653.3 |
| **377** | 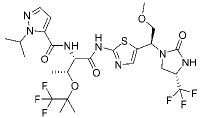 | 658.2 | **883** | 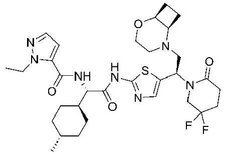 | 648.3 |
| **378** | 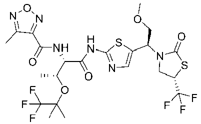 | 649.1 | **884** | 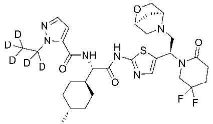 | 639.3 |
| **379** | 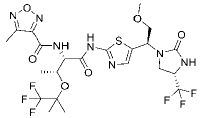 | 632.2 | **885** | 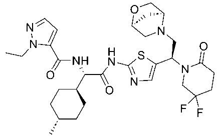 | 634.3 |
| **380** | 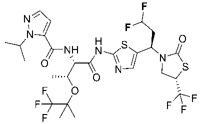 | 695.2 | **886** | 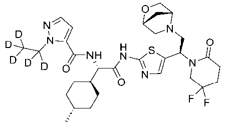 | 639.3 |
| **381** | 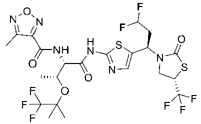 | 669.1 | **887** | 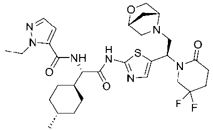 | 634.3 |
| **382** | 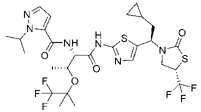 | 685.2 | **888** | 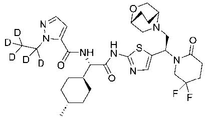 | 653.3 |
| **383** | 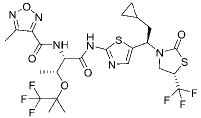 | 659.1 | **889** | 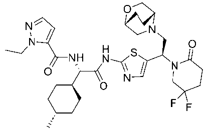 | 648.3 |
| **384** | 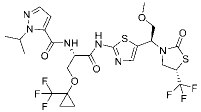 | 659.2 | **890** | 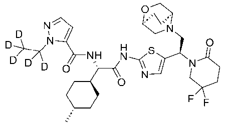 | 653.3 |
| **385** | 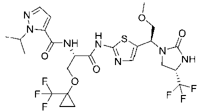 | 642.2 | **891** | 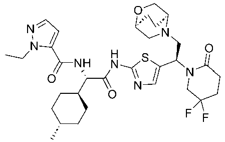 | 648.3 |
| **386** | 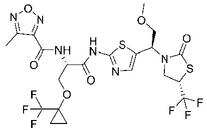 | 633.1 | **892** | 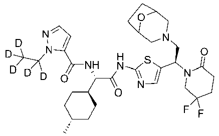 | 653.3 |
| **387** | 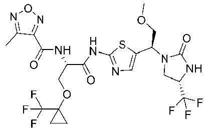 | 616.1 | **893** | 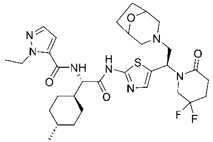 | 648.3 |
| **388** | 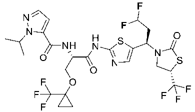 | 679.1 | **894** | 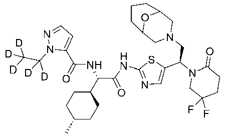 | 667.3 |
| **389** | 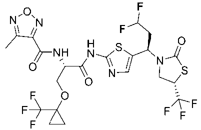 | 653.1 | **895** | 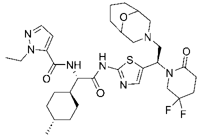 | 662.3 |
| **390** | 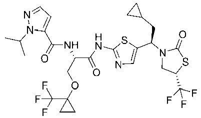 | 669.2 | **896** | 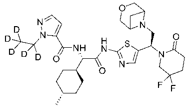 | 639.3 |
| **391** | 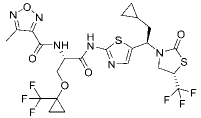 | 643.1 | **897** | 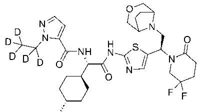 | 653.3 |
| **392** | 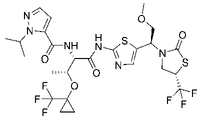 | 673.2 | **898** | 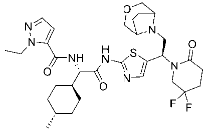 | 648.3 |
| **393** | 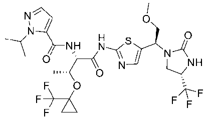 | 656.2 | **899** | 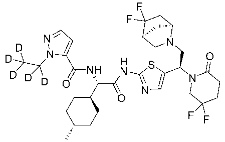 | 673.3 |
| **394** | 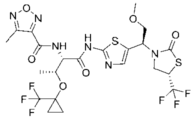 | 647.1 | **900** | 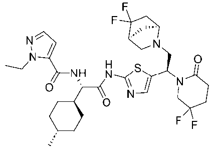 | 668.3 |
| **395** | 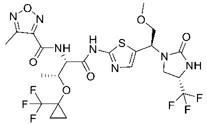 | 630.2 | **901** | 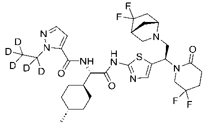 | 673.3 |
| **396** | 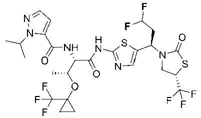 | 693.2 | **902** | 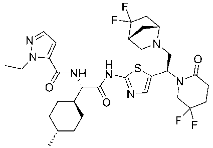 | 668.3 |
| **397** | 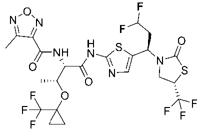 | 667.1 | **903** | 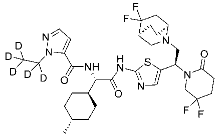 | 687.3 |
| **398** | 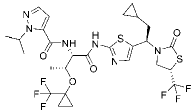 | 683.2 | **904** | 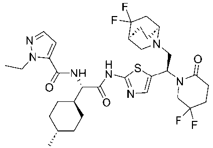 | 682.3 |
| **399** | 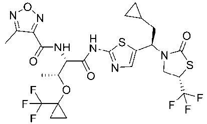 | 657.1 | **905** | 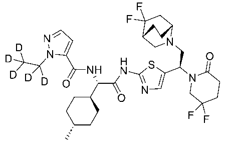 | 687.3 |
| **400** | 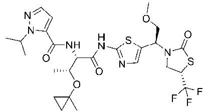 | 619.2 | **906** | 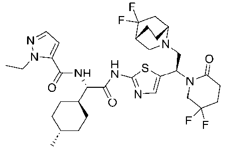 | 682.3 |
| **401** | 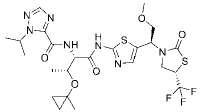 | 620.2 | **907** | 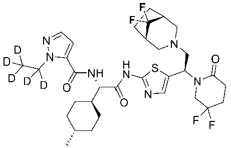 | 687.3 |
| **402** | 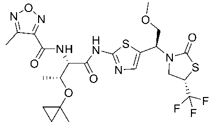 | 593.1 | **908** | 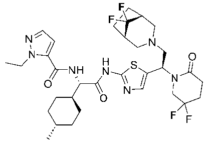 | 682.3 |
| **403** | 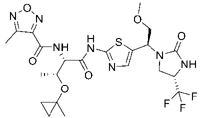 | 576.2 | **909** | 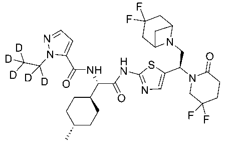 | 673.3 |
| **404** | 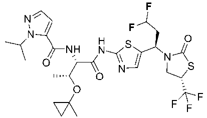 | 639.2 | **910** | 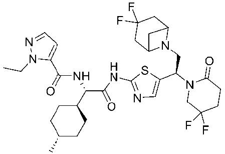 | 668.3 |
| **405** | 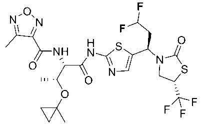 | 613.1 | **911** | 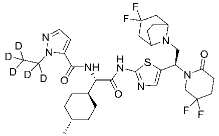 | 687.3 |
| **406** | 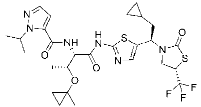 | 629.2 | **912** | 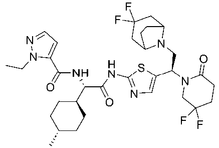 | 682.3 |
| **407** | 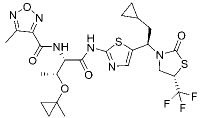 | 603.2 | **913** | 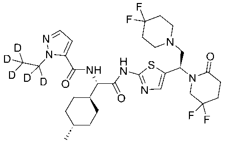 | 661.3 |
| **408** | 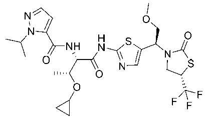 | 605.2 | **914** | 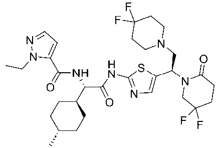 | 656.3 |
| **409** | 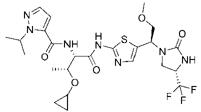 | 588.2 | **915** | 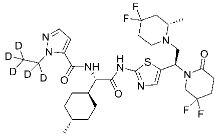 | 675.3 |
| **410** | 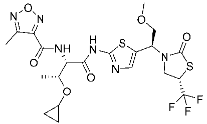 | 579.1 | **916** | 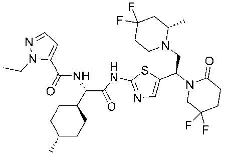 | 670.3 |
| **411** | 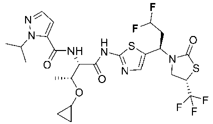 | 625.2 | **917** | 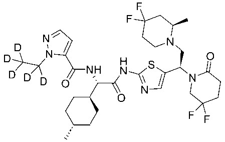 | 675.3 |
| **412** | 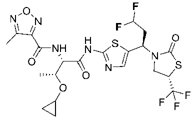 | 599.1 | **918** | 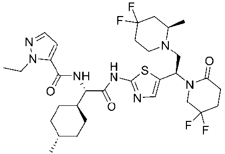 | 670.3 |
| **413** | 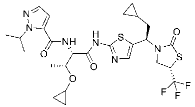 | 615.2 | **919** | 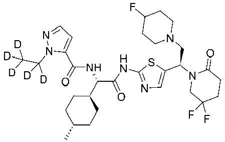 | 643.3 |
| **414** | 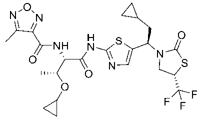 | 589.1 | **920** | 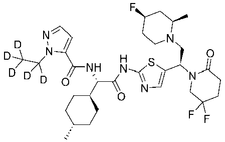 | 657.3 |
| **415** | 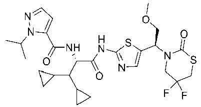 | 597.2 | **921** | 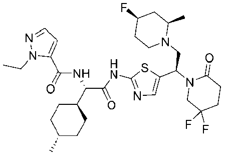 | 652.3 |
| **416** | 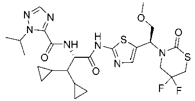 | 598.2 | **922** | 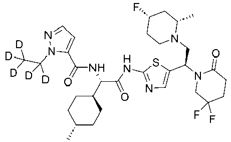 | 657.3 |
| **417** | 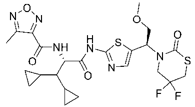 | 571.2 | **923** | 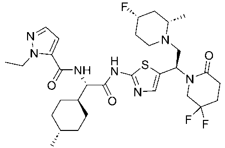 | 652.3 |
| **418** | 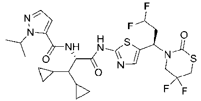 | 617.2 | **924** | 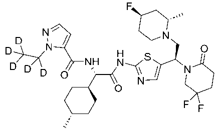 | 657.3 |
| **419** | 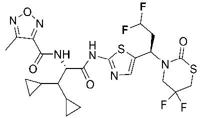 | 591.1 | **925** | 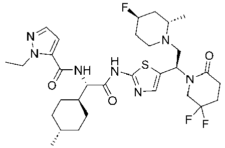 | 652.3 |
| **420** | 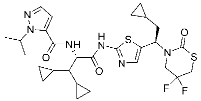 | 607.2 | **926** | 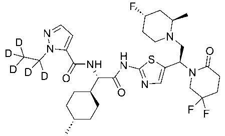 | 657.3 |
| **421** | 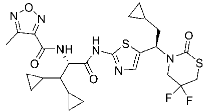 | 581.2 | **927** | 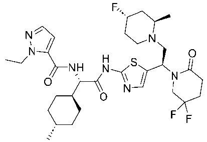 | 652.3 |
| **422** | 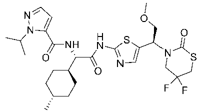 | 599.2 | **928** | 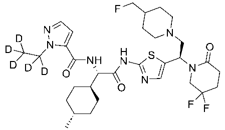 | 657.3 |
| **423** | 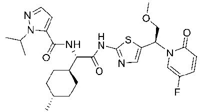 | 559.2 | **929** | 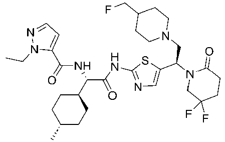 | 652.3 |
| **424** | 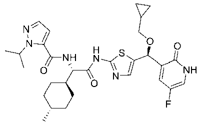 | 585.3 | **930** | 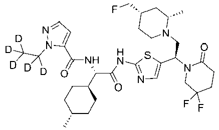 | 671.3 |
| **425** | 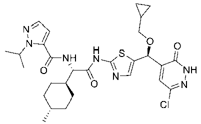 | 602.2 | **931** | 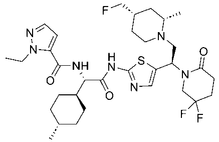 | 666.3 |
| **426** | 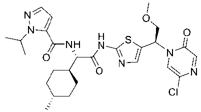 | 576.2 | **932** | 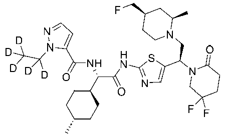 | 671.3 |
| **427** | 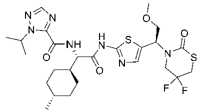 | 600.2 | **933** | 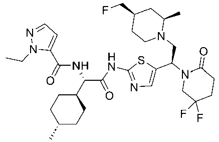 | 666.3 |
| **428** | 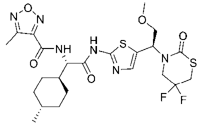 | 573.2 | **934** | 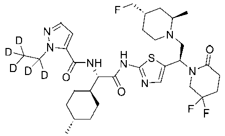 | 671.3 |
| **429** | 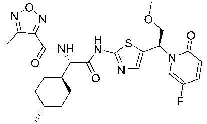 | 533.2 | **935** | 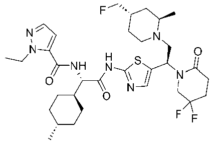 | 666.3 |
| **430** | 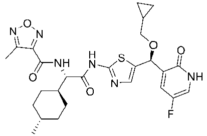 | 559.2 | **936** | 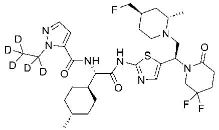 | 671.3 |
| **431** | 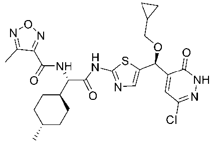 | 576.2 | **937** | 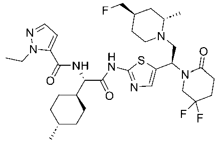 | 666.3 |
| **432** | 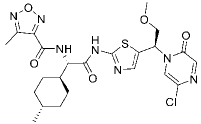 | 550.2 | **938** | 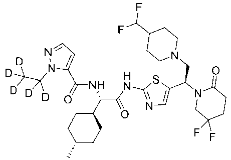 | 675.3 |
| **433** | 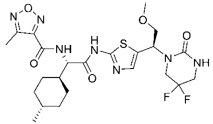 | 556.2 | **939** | 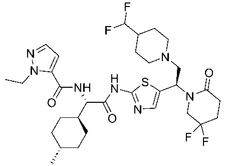 | 670.3 |
| **434** | 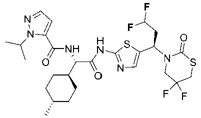 | 619.2 | **940** | 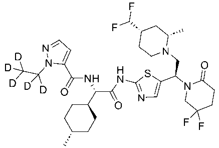 | 689.3 |
| **435** | 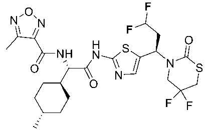 | 593.2 | **941** | 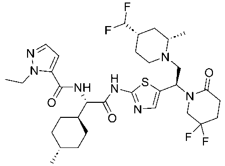 | 684.3 |
| **436** | 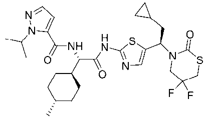 | 609.2 | **942** | 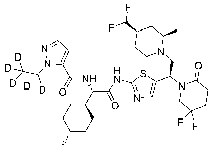 | 689.3 |
| **437** | 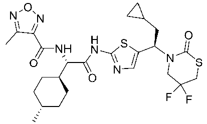 | 583.2 | **943** | 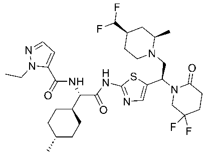 | 684.3 |
| **438** | 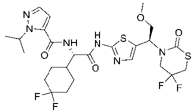 | 621.2 | **944** | 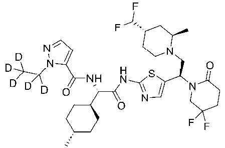 | 689.3 |
| **439** | 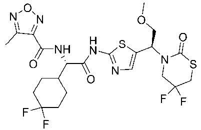 | 595.1 | **945** | 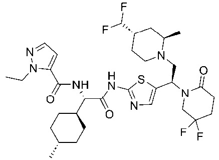 | 684.3 |
| **440** | 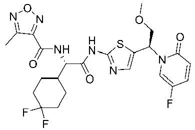 | 555.2 | **946** | 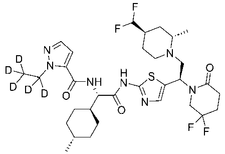 | 689.3 |
| **441** | 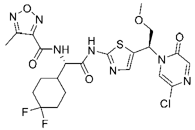 | 572.1 | **947** | 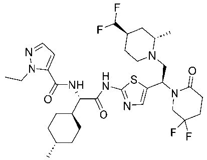 | 684.3 |
| **442** | 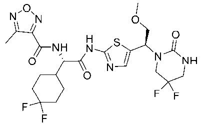 | 578.2 | **948** | 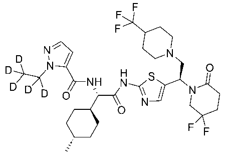 | 693.3 |
| **443** | 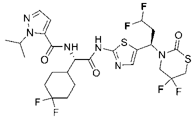 | 641.2 | **949** | 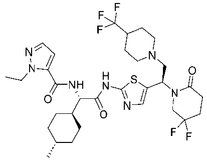 | 688.3 |
| **444** | 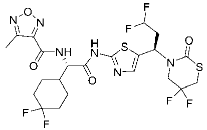 | 615.1 | **950** | 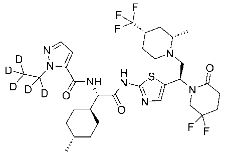 | 707.3 |
| **445** | 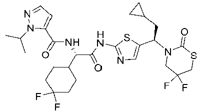 | 631.2 | **951** | 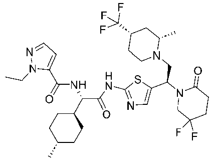 | 702.3 |
| **446** | 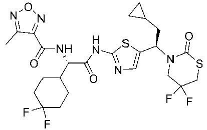 | 605.2 | **952** | 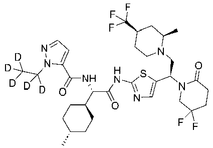 | 707.3 |
| **447** | 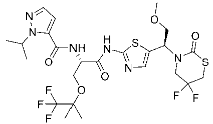 | 643.2 | **953** | 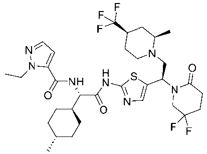 | 702.3 |
| **448** | 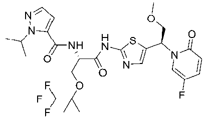 | 603.2 | **954** | 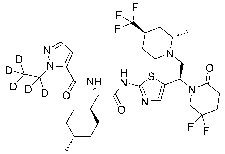 | 707.3 |
| **449** | 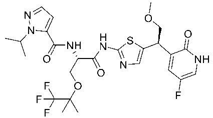 | 603.2 | **955** | 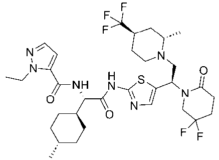 | 702.3 |
| **450** | 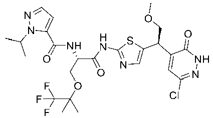 | 620.2 | **956** | 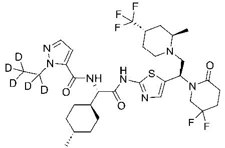 | 707.3 |
| **451** | 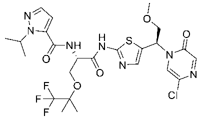 | 620.2 | **957** | 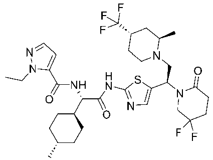 | 702.3 |
| **452** | 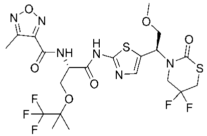 | 617.1 | **958** | 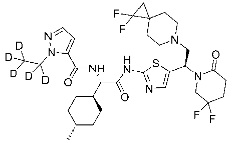 | 687.3 |
| **453** | 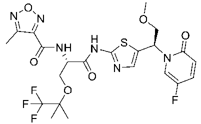 | 577.1 | **959** | 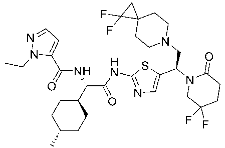 | 682.3 |
| **454** | 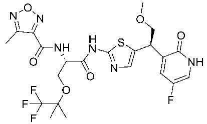 | 577.1 | **960** | 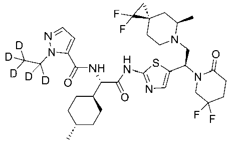 | 701.2 |
| **455** | 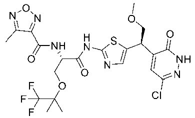 | 594.1 | **961** | 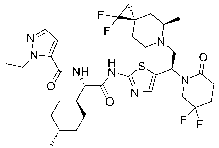 | 696.2 |
| **456** | 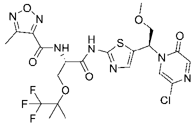 | 594.1 | **962** | 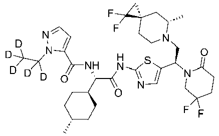 | 701.2 |
| **457** | 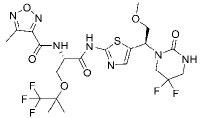 | 600.2 | **963** | 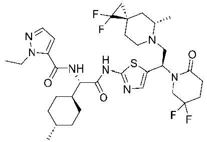 | 696.2 |
| **458** | 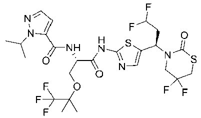 | 663.2 | **964** | 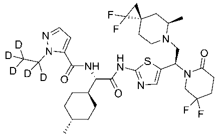 | 701.2 |
| **459** | 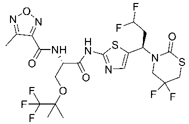 | 637.1 | **965** | 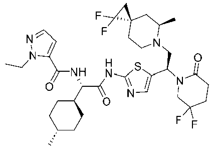 | 696.2 |
| **460** | 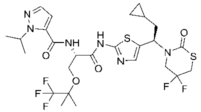 | 653.2 | **966** | 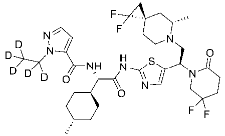 | 701.2 |
| **461** | 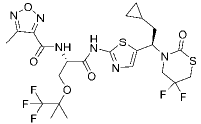 | 627.1 | **967** | 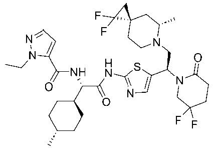 | 696.2 |
| **462** | 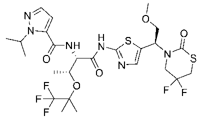 | 657.2 | **968** | 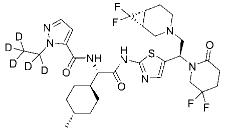 | 673.3 |
| **463** | 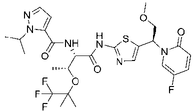 | 617.2 | **969** | 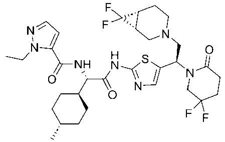 | 668.3 |
| **464** | 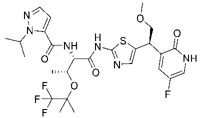 | 617.2 | **970** | 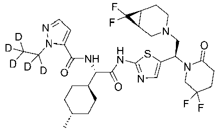 | 673.3 |
| **465** | 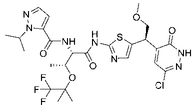 | 634.2 | **971** | 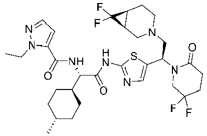 | 668.3 |
| **466** | 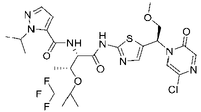 | 634.2 | **972** | 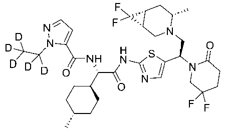 | 687.3 |
| **467** | 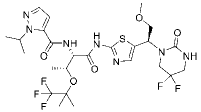 | 640.2 | **973** | 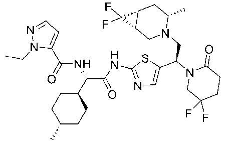 | 682.3 |
| **468** | 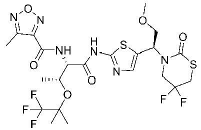 | 631.1 | **974** | 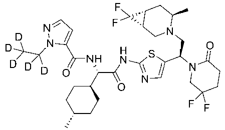 | 687.3 |
| **469** | 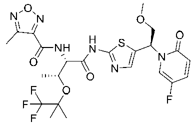 | 591.2 | **975** | 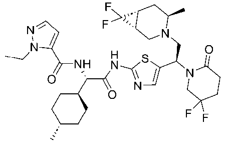 | 682.3 |
| **470** | 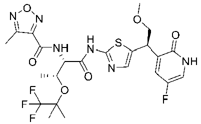 | 591.2 | **976** | 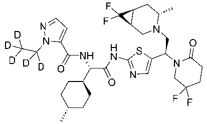 | 687.3 |
| **471** | 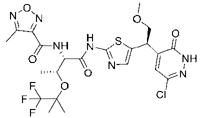 | 608.1 | **977** | 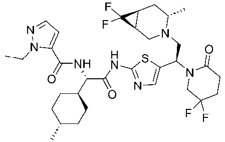 | 682.3 |
| **472** | 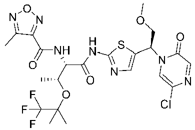 | 608.1 | **978** | 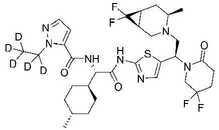 | 687.3 |
| **473** | 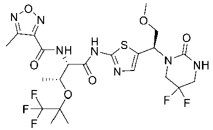 | 614.2 | **979** | 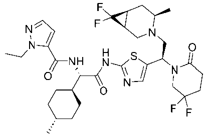 | 682.3 |
| **474** | 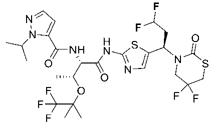 | 677.2 | **980** | 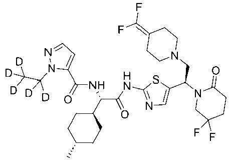 | 673.3 |
| **475** | 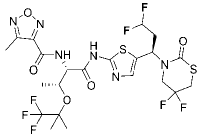 | 651.1 | **981** | 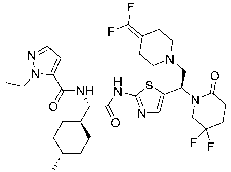 | 668.3 |
| **476** | 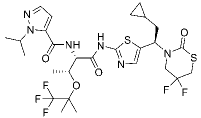 | 667.2 | **982** | 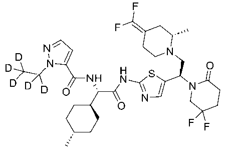 | 687.3 |
| **477** | 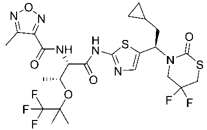 | 641.2 | **983** | 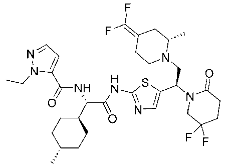 | 682.3 |
| **478** | 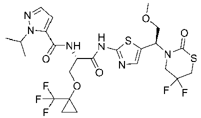 | 641.2 | **984** | 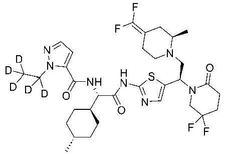 | 687.3 |
| **479** | 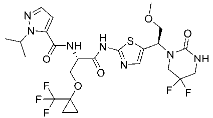 | 624.2 | **985** | 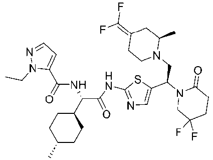 | 682.3 |
| **480** | 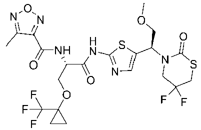 | 615.1 | **986** | 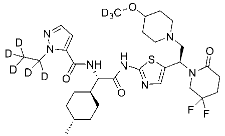 | 658.3 |
| **481** | 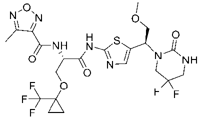 | 598.1 | **987** | 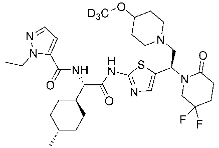 | 653.3 |
| **482** | 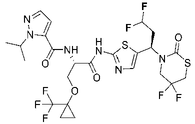 | 661.1 | **988** | 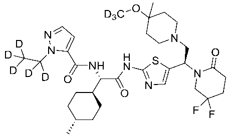 | 672.3 |
| **483** | 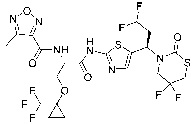 | 635.2 | **989** | 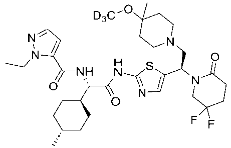 | 667.3 |
| **484** | 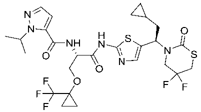 | 651.2 | **990** | 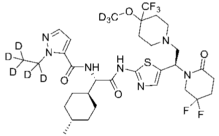 | 726.3 |
| **485** | 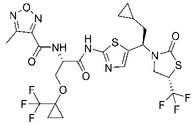 | 643.1 | **991** | 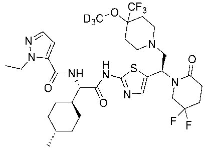 | 721.3 |
| **486** | 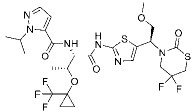 | 655.2 | **992** | 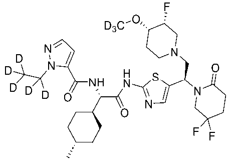 | 676.3 |
| **487** | 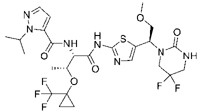 | 638.2 | **993** | 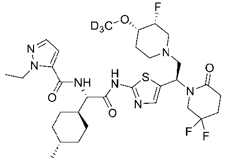 | 671.3 |
| **488** | 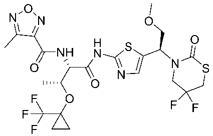 | 629.1 | **994** | 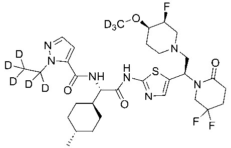 | 676.3 |
| **489** | 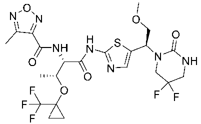 | 612.2 | **995** | 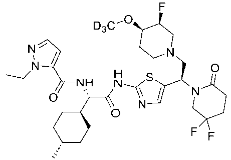 | 671.3 |
| **490** | 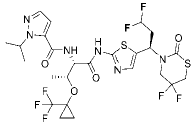 | 675.2 | **996** | 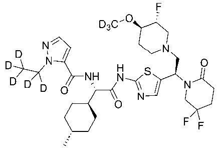 | 676.3 |
| **491** | 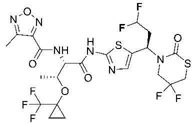 | 649.1 | **997** | 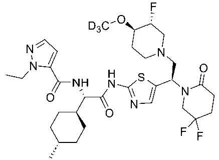 | 671.3 |
| **492** | 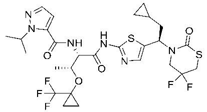 | 665.2 | **998** | 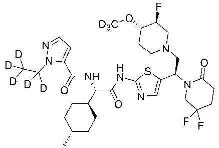 | 676.3 |
| **493** | 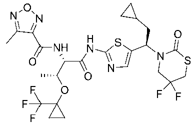 | 639.1 | **999** | 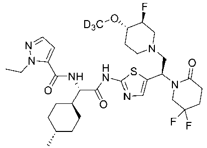 | 671.3 |
| **494** | 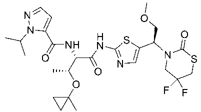 | 601.2 | **1000** | 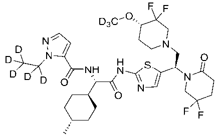 | 694.3 |
| **495** | 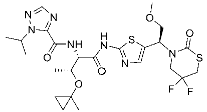 | 602.2 | **1001** | 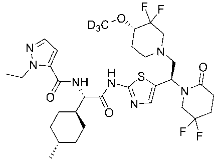 | 689.3 |
| **496** | 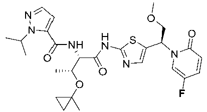 | 561.2 | **1002** | 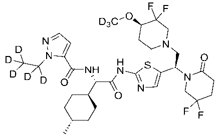 | 694.3 |
| **497** | 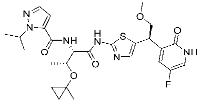 | 561.2 | **1003** | 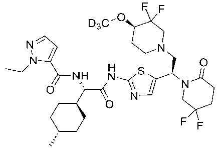 | 689.3 |
| **498** | 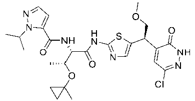 | 578.2 | **1004** | 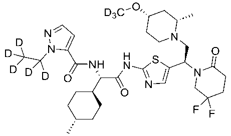 | 672.3 |
| **499** | 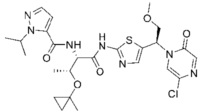 | 578.2 | **1005** | 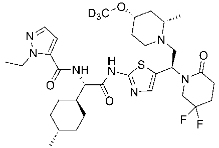 | 667.3 |
| **500** | 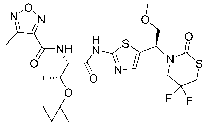 | 575.1 | **1006** | 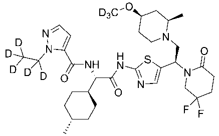 | 672.3 |
| **501** | 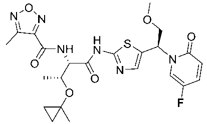 | 535.2 | **1007** | 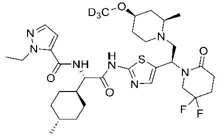 | 667.3 |
| **502** | 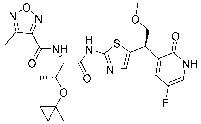 | 535.2 | **1008** | 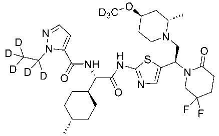 | 672.3 |
| **503** | 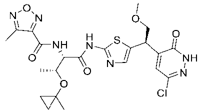 | 552.1 | **1009** | 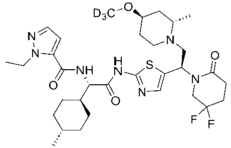 | 667.3 |
| **504** | 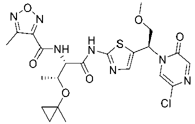 | 552.1 | **1010** | 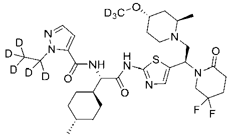 | 672.3 |
| **505** | 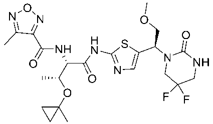 | 558.2 | **1011** | 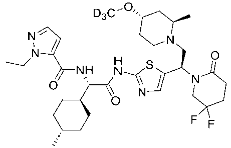 | 667.3 |
| **506** | 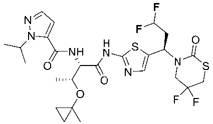 | 621.2 | **1012** | 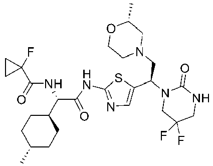 | 601.3 |
| **507** | 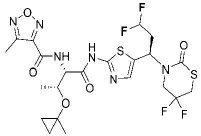 | 595.1 | **1013** | 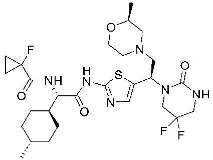 | 601.3 |
| **508** | 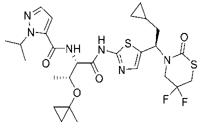 | 611.2 | **1014** | 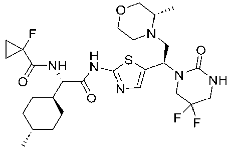 | 601.3 |
| **509** | 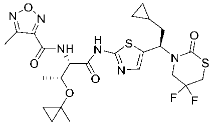 | 585.2 | **1015** | 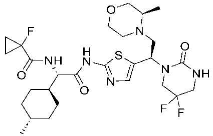 | 601.3 |
| **510** | 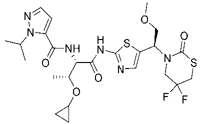 | 587.2 | **1016** | 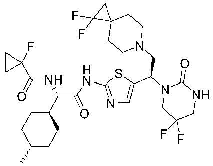 | 647.3 |
| **1017** | 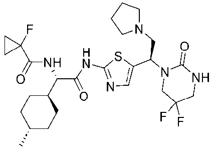 | 571.3 | **1018** | 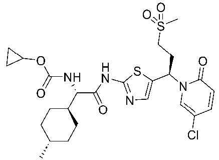 | 585.2 |
| **1019** | 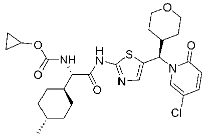 | 563.2 | **1020** | 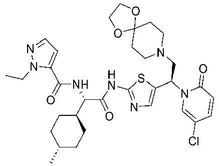 | 672.3 |
| **1021** | 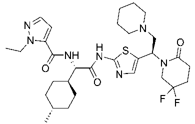 | 620.3 | **1022** | 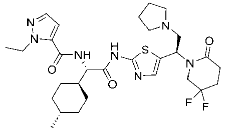 | 606.3 |
| **1023** | 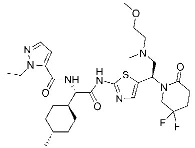 | 624.3 | **1024** | 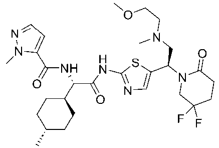 | 610.3 |
| **1025** | 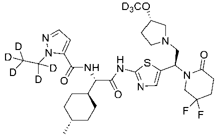 | 644.3 | **1026** | 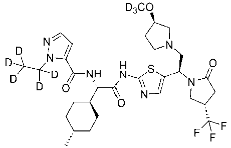 | 662.3 |
| **1027** | 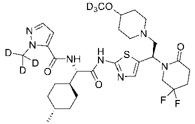 | 642.3 | **1028** | 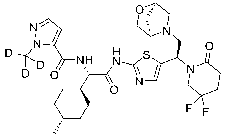 | 623.3 |
| **1029** | 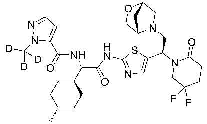 | 623.3 | **1030** | 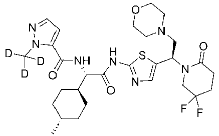 | 611.3 |
| **1031** | 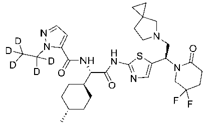 | 637.3 | **1032** | 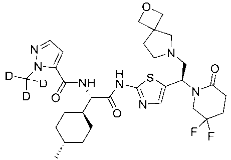 | 637.3 |
| **1033** | 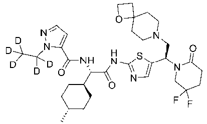 | 667.3 | **1034** | 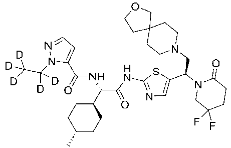 | 681.4 |
| **1035** | 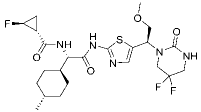 | 532.2 | **1036** | 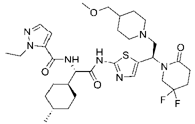 | 664.3 |

Example 327 can also be synthesised according to the following steps.

### Step 1: preparation of tert-butyl(5-(2-methoxyacetyl)thiazol-2-yl)carbamate

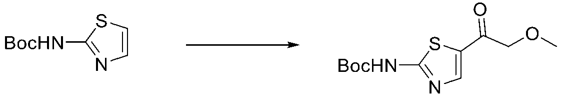

Tert-butylthiazole-2-carbamate (10.3 g, 51.43 mmol) was dissolved in dried tetrahydrofuran (200 mL). The reaction system was maintained under dry nitrogen and cooled to -78°C. An n-butyllithium solution (127.5 mmol, 51 mL, 2.5 M in n-hexane) was slowly added dropwise. After the dropwise addition, the reaction solution was further stirred at -78°C for 30 minutes. N,2-dimethoxy-N-methylacetamide (19.17 g, 144.01 mmol) was added. The reaction solution was further stirred at -78°C for 2 hours. In a reaction flask, the reaction was quenched with a saturated aqueous ammonium chloride solution, and a white solid precipitated. The resulting precipitate was collected by filtration under reduced pressure and dried under reduced pressure to obtain the title compound (5.8 g, 41%). MS m/z (ESI): 273.1 [M+H]⁺.

### Step 2: preparation of tert-butyl(5-(1-((3-amino-2,2-difluoropropyl)amino)-2-methoxyethyl)thiazol-2-yl)carbamate

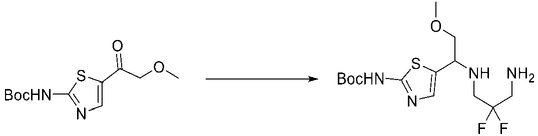

Tert-butyl(5-(2-methoxyacetyl)thiazol-2-yl)carbamate (5 g, 18.36 mmol) and 2,2-difluoropropane-1,3-diamine dihydrochloride (6.72 g, 36.72 mmol) were dissolved in a mixed solvent of DMA (15 mL) and methanol (8 mL), and triethylamine (8.36 g, 82.62 mmol) was added. The reaction mixture was heated to 75°C, and the reaction was stirred for 12 hours and cooled to room temperature. Acetic acid (6.61 g, 110.16 mmol) and sodium cyanoborohydride (6.92 g, 110.16 mmol) were added, and the reaction was heated to 40°C and stirred for 6 hours. The reaction solution was diluted with ethyl acetate, and washed sequentially with a saturated aqueous sodium carbonate solution and a saturated sodium chloride aqueous solution. The organic phase was separated, dried over anhydrous sodium sulphate, filtered and then concentrated under reduced pressure to remove the organic solvent, and the resulting residue was separated by column chromatography to obtain the title compound (3.6 g, 54%). MS m/z (ESI): 367.2 [M+H]⁺.

### Step 3: preparation of tert-butyl(5-(1-(5,5-difluoro-2-carbonyltetrahydropyrimidin-1(2H)-yl)-2-methoxyethyl)thiazol-2-yl)carbamate

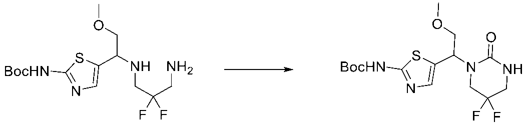

Tert-butyl(5-(1-((3-amino-2,2-difluoropropyl)amino)-2-methoxyethyl)thiazol-2-yl)carbamate (0.56 g, 1.47 mmol) was dissolved in tetrahydrofuran (6 mL). N,N'-carbonyldiimidazole (477 mg, 2.94 mmol) was added. The reaction solution was stirred and reacted at 30°C for 1 hour, and a white solid precipitated. The resulting precipitate was filtered, and the filter cake was washed with a small amount of ethyl acetate, collected and dried under reduced pressure to obtain the title compound (340 mg, 57%). MS m/z (ESI) : 393.1[M+H]⁺.

### Step 4: preparation of 1-(1-(2-aminothiazol-5-yl)-2-methoxyethyl)-5,5-difluorotetrahydropyrimidin-2(1H)-one hydrochloride

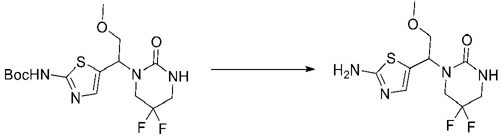

Tert-butyl(5-(1-(5,5-difluoro-2-carbonyltetrahydropyrimidin-1(2H)-yl)-2-methoxyethyl)thiazol-2-yl)carbamate (120 mg, 0.293 mmol) was dissolved in dichloromethane (1 mL). 4 M hydrogen chloride in dioxane (3 mL) was added. The reaction solution was stirred and reacted at room temperature for 8 hours and concentrated under reduced pressure to remove the solvent, so as to obtain the title compound (96 mg, 99%), which was directly used in the next step. MS m/z (ESI): 293.1 [M+H]⁺.

### Step 5: preparation of tert-butyl((1S)-2-((5-(1-(5,5-difluoro-2-carbonyltetrahydropyrimidin-1(2H)-yl)-2-methoxyethyl)thiazol-2-yl)amino)-1-((trans)-4-methylcyclohexyl)-2-carbonylethyl)carbamate

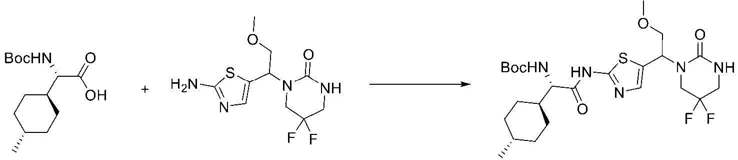

(S)-2-((tert-butoxycarbonyl)amino)-2-((trans)-4-methylcyclohexyl)acetic acid (115 mg, 0.42 mmol) was dissolved in DMF (3 mL). HATU (159.89 mg, 0.42 mmol) and DIPEA (274 mg, 2.12 mmol) were sequentially added. The reaction was stirred at room temperature for 10 minutes, and 1-(1-(2-aminothiazol-5-yl)-2-methoxyethyl)-5,5-difluorotetrahydropyrimidin-2(1H)-one hydrochloride (96 mg, 0.293 mmol) was added. The reaction solution was further stirred and reacted at room temperature for 1 hour, diluted with ethyl acetate and washed with a saturated sodium chloride aqueous solution. The organic phase was separated, dried over anhydrous sodium sulphate, filtered and then concentrated under reduced pressure to remove the organic solvent, and the resulting residue was separated by column chromatography to obtain the title compound (120 mg, 75%). MS m/z (ESI): 546.2 [M+H]⁺.

### Step 6: preparation of (2S)-2-amino-N-(5-(1-(5,5-difluoro-2-carbonyltetrahydropyrimidin-1(2H)-yl)-2-methoxyethyl)thiazol-2-yl)-2-((trans)-4-methylcyclohexyl)acetamide hydrochloride

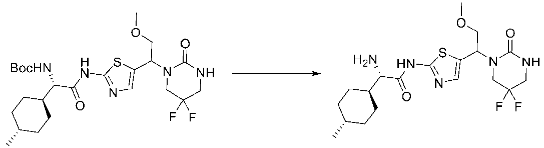

Tert-butyl((1S)-2-((5-(1-(5,5-difluoro-2-carbonyltetrahydropyrimidin-1(2H)-yl)-2-methoxyethyl)thiazol-2-yl)amino)-1-((trans)-4-methylcyclohexyl)-2-carbonylethyl)carbamate (116 mg, 0.22 mmol) was dissolved in dichloromethane (0.5 mL). 4 M hydrogen chloride in dioxane (3 mL) was added. The reaction solution was stirred and reacted at room temperature for 1 hour and concentrated under reduced pressure to remove the solvent, so as to obtain the title compound (103 mg, 100%). MS m/z (ESI): 446.2 [M+H]⁺.

### Step 7: preparation of N-((1S)-2-((5-(1-(5,5-difluoro-2-carbonyltetrahydropyrimidin-1(2H)-yl)-2-methoxyethyl)thiazol-2-yl)amino)-1-((trans)-4-methylcyclohexyl)-2-carbonylethyl)- 1-isopropyl-1H-pyrazole-5-carboxamide

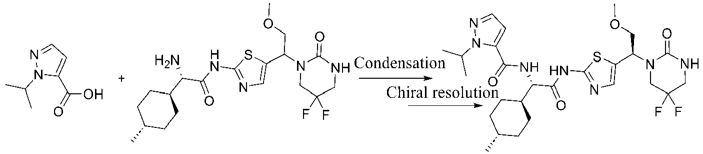

1-isopropyl-1H-pyrazole-5-carboxylic acid (56 mg, 0.36 mmol) was dissolved in DMF (2 mL). HATU (137 mg, 0.36 mmol) and DIPEA (146 mg, 1.13 mmol) were sequentially added. The reaction solution was stirred at room temperature for 5 minutes, and (2S)-2-amino-N-(5-(1-(5,5-difluoro-2-carbonyltetrahydropyrimidin-1(2H)-yl)-2-methoxyethyl)thiazol-2-yl)-2-((trans)-4-methylcyclohexyl)acetamide hydrochloride (102 mg, 0.212 mmol) was added. The reaction was further stirred at room temperature for 1 hour. The reaction solution was diluted with ethyl acetate and washed with a saturated sodium chloride aqueous solution. The organic phase was separated, dried over anhydrous sodium sulphate, filtered and then concentrated under reduced pressure to remove the organic solvent, and the residue was separated by column chromatography and then subjected to chiral HPLC resolution to obtain the title compound (38 mg, 29%). MS m/z (ESI): 582.3 [M+H]⁺.

### Example 556 can also be synthesised according to the following steps

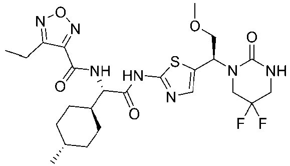

4-ethyl-1,2,5-oxadiazole-3-carboxylic acid (32 mg, 0.22 mmol) was dissolved in DMF (2 mL). HATU (84 mg, 0.22 mmol) and DIPEA (57 mg, 0.44 mmol) were sequentially added. The reaction was stirred at room temperature for 5 minutes, and (2S)-2-amino-N-(5-(1-(5,5-difluoro-2-carbonyltetrahydropyrimidin-1(2H)-yl)-2-methoxyethyl)thiazol-2-yl)-2-((trans)-4-methylcyclohexyl)acetamide hydrochloride (68 mg, 0.14 mmol) was added. The mixture was further stirred at room temperature for 1 hour. The reaction solution was diluted with ethyl acetate and washed with a saturated sodium chloride aqueous solution. The organic phase was separated, dried over anhydrous sodium sulphate, filtered and then concentrated under reduced pressure to remove the organic solvent, and the residue was subjected to column chromatography and chiral resolution to obtain the title compound (15 mg, 18%). MS m/z (ESI):570.2 [M+H]⁺.

### Example 557 can also be synthesised according to the following steps

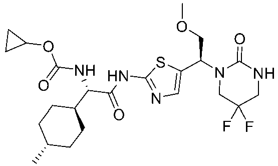

(2S)-2-amino-N-(5-(1-(5,5-difluoro-2-carbonyltetrahydropyrimidin-1(2H)-yl)-2-methoxyethyl)thiazol-2-yl)-2-((trans)-4-methylcyclohexyl)acetamide (95 mg, 213.23 µmol) was dissolved in dichloromethane (5 mL). The mixture was cooled to 0°C in an ice-water bath. DIPEA (100 mg, 0.77 mmol) and cyclopropyl chloroformate (33 mg, 0.27 mmol) were sequentially added, and the mixture was further stirred at 0°C for 1 hour. The reaction solution was diluted with dichloromethane and washed with a saturated sodium chloride aqueous solution. The organic phase was separated, dried over anhydrous sodium sulphate, filtered and then concentrated under reduced pressure to remove the organic solvent, and the residue was sequentially separated by column chromatography and chiral resolution to obtain the title compound (38 mg, 33.6%). MS m/z (ESI): 530.2 [M+H]⁺.

### Example 568 can also be synthesised according to the following steps

### Step 1: preparation of tert-butyl ((2S)-1-((5-(1-(5,5-difluoro-2-carbonyltetrahydropyrimidin-1(2H)-yl)-2-methoxyethyl)thiazol-2-yl)amino)-1-carbonyl-3-((1,1,1-trifluoro-2-methylpropan-2-yl)oxy)propan-2-yl)carbamate

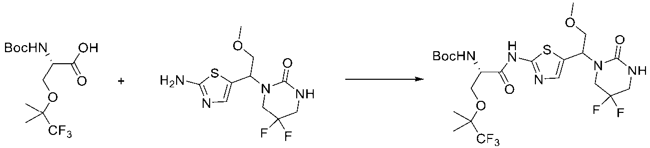

To a solution of N-(tert-butoxycarbonyl)-oxy-(1,1,1-trifluoro-2-methylpropan-2-yl)-L-serine (173 mg, 0.55 mmol) and 1-(1-(2-aminothiazol-5-yl)-2-methoxyethyl)-5,5-difluorotetrahydropyrimidin-2(1H)-one (80 mg, 0.27 mmol) in DMF (10 mL) were sequentially added DIPEA (106 mg, 0.82 mmol, 143 µL) and HATU (207 mg, 0.55 mmol). The reaction solution was stirred at room temperature for 12 hours, diluted with ethyl acetate and washed sequentially with a saturated aqueous ammonium chloride solution and a saturated sodium chloride aqueous solution. The organic phase was separated, dried over anhydrous sodium sulphate, filtered and then concentrated under reduced pressure to remove the organic solvent, and the residue was separated by column chromatography to obtain the title compound (131 mg, 81%). MS m/z (ESI): 590.2 [M+H]⁺.

### Step 2: preparation of N-((S)-1-((5-((R)-1-(5,5-difluoro-2-carbonyltetrahydropyrimidin-1(2H)-yl)-2-methoxyethyl)thiazol-2-yl)amino)-1-carbonyl-3-((1,1,1-trifluoro-2-methylpropan-2-yl)oxy)propan-2-yl)-4-ethyl-1,2,5-oxadiazole-3-carboxamide

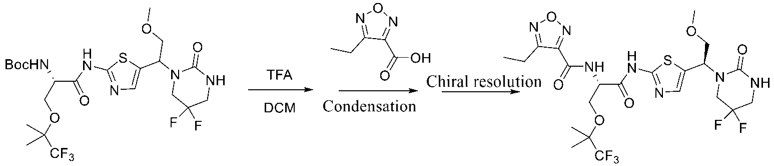

Tert-butyl ((2S)-1-((5-(1-(5,5-difluoro-2-carbonyltetrahydropyrimidin-1(2H)-yl)-2-methoxyethyl)thiazol-2-yl)amino)-1-carbonyl-3-((1,1,1-trifluoro-2-methylpropan-2-yl)oxy)propan-2-yl)carbamate (131 mg, 0.22 mmol) was dissolved in dichloromethane (5 mL). Trifluoroacetic acid (5 mL) was added, and the reaction was stirred at room temperature for 1 hour and concentrated under reduced pressure to remove the organic solvent. The residue was dissolved in ethyl acetate, and washed sequentially with a saturated sodium bicarbonate aqueous solution and a saturated sodium chloride aqueous solution. The organic phase was separated, dried over anhydrous sodium sulphate, filtered and then concentrated under reduced pressure to remove the organic solvent. The resulting residue was dissolved in DMF (5 mL). DIPEA (287 mg, 2.22 mmol, 387 µL), 4-ethyl-1,2,5-oxadiazole-3-carboxylic acid (32 mg, 0.22 mmol) and HATU (168 mg, 0.44 mmol) were sequentially added, and the reaction was stirred at room temperature for 12 hours. The reaction was diluted with ethyl acetate, and washed sequentially with a saturated aqueous ammonium chloride solution and a saturated sodium chloride aqueous solution. The organic phase was separated, dried over anhydrous sodium sulphate, filtered and then concentrated under reduced pressure to remove the organic solvent, and the residue was sequentially separated by column chromatography and subjected to chiral HPLC resolution to obtain the title compound (41 mg, 30%).

¹H NMR (400 MHz, DMSO-*d⁶*) *δ* 12.43 (s, 1H), 9.34 (d, *J =* 7.5 Hz, 1H), 7.39 (s, 1H), 6.91 (s, 1H), 5.70 (t, *J =* 6.7 Hz, 1H), 4.89 (dd, *J =* 12.8, 7.0 Hz, 1H), 4.05-3.70 (m, 4H), 3.70-3.39 (m, 4H), 3.32 (s, 3H) , 2.92 (q, *J=* 7.5 Hz, 2H), 1.31 (t, *J =* 4.4 Hz, 6H), 1.26 (d, *J =* 3.4 Hz, 3H); MS m/z (ESI): 614.2 [M+H]⁺.

### Example 276 can also be synthesised according to the following steps

### Step 1: synthesis of tert-butyl(5-(2-cyclopropylacetyl)thiazol-2-yl)carbamate

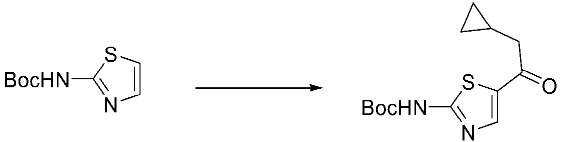

Tert-butyl thiazol-2-ylcarbamate (12 g, 60 mmol) was dissolved in tetrahydrofuran (80 mL). The mixture was cooled to -78°C. To the reaction solution was added dropwise n-butyllithium (60 mL, 2.5 M, 150 mmol), and the mixture was further stirred at -78°C for 1 hour. 2-Cyclopropyl-N-methoxy-N-methylacetamide (21.45 g, 150 mmol) was added, and the mixture was further stirred at -78°C for 1 hour. To the reaction solution was added an aqueous ammonium chloride solution (25 mL), and the mixture was extracted with ethyl acetate (100 mL×3). The organic phases were combined, and washed with a saturated sodium chloride aqueous solution (100 mL). The organic phase was separated, dried over anhydrous sodium sulphate, filtered and then concentrated under reduced pressure to remove the organic solvent, and the residue was separated by column chromatography to obtain the title compound (7.7 g, 46%). MS m/z (ESI):283.1 [M+H]⁺.

### Step 2: synthesis of tert-butyl(5-(1-((3-amino-2,2-difluoropropyl)amino)-2-cyclopropylethyl)thiazol-2-yl)carbamate

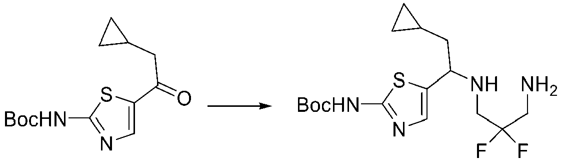

Tert-butyl(5-(2-cyclopropylacetyl)thiazol-2-yl)carbamate (3 g, 10.62 mmol), 2,2-difluoropropane-1,3-diamine dihydrochloride (3.89 g, 21 mmol) and triethylamine (5.37 g, 53 mmol) were dissolved in a mixed solution of N,N-dimethylacetamide and methanol (33 mL, V/V = 10:1), and the reaction was stirred at 75°C for 12 hours, and cooled to room temperature. Under stirring, sodium cyanoborohydride (4.67 g, 74 mmol) and acetic acid (4.46 g, 74 mmol) were sequentially added. The mixture was further heated to 40°C and reacted for 12 hours. The reaction was cooled and then concentrated under reduced pressure to remove methanol. The reaction solution was diluted with water and extracted with ethyl acetate. The organic phases were combined, and washed with a saturated sodium bicarbonate aqueous solution and a saturated sodium chloride aqueous solution. The organic phase was dried over anhydrous sodium sulphate, filtered and then concentrated under reduced pressure to remove the organic solvent, and the residue was separated by column chromatography to obtain the title compound (0.8 g, 20%). MS m/z (ESI): 377.2 [M+H]⁺

### Step 3: synthesis of tert-butyl(5-(2-cyclopropyl-1-(5,5-difluoro-2-oxotetrahydropyrimidin-1(2H)-yl)ethyl)thiazol-2-yl)carbamate

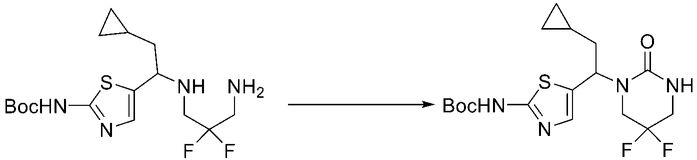

Tert-butyl(5-(1-((3-amino-2,2-difluoropropyl)amino)-2-cyclopropylethyl)thiazol-2-yl)carbamate (0.8 g, 2.13 mmol) was dissolved in tetrahydrofuran (8 mL). Carbonyldiimidazole (861 mg, 5.31 mmol) was added, and the mixture was stirred at room temperature for 1 hour. The reaction solution was diluted with water, and extracted three times with ethyl acetate. The organic phases were combined, and washed with a saturated sodium chloride aqueous solution. The organic phase was separated, dried over anhydrous sodium sulphate, filtered and then concentrated under reduced pressure to remove the organic solvent. The resulting residue was separated by column chromatography to obtain the title compound (570 mg, 67%). MS m/z (ESI): 403.2 [M+H]⁺.

### Step 4: synthesis of 1-(1-(2-aminothiazol-5-yl)-2-cyclopropylethyl)-5,5-difluorotetrahydropyrimidin-2(1H)-one hydrochloride

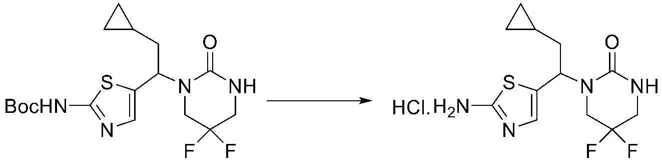

1-(1-(2-aminothiazol-5-yl)-2-cyclopropylethyl)-5,5-difluorotetrahydropyrimidin-2(1H)-one (70 mg, 0.17 mmol) was dissolved in 4 M hydrogen chloride in dioxane (5 mL). The reaction was stirred at 45°C for 12 hours. The reaction was cooled to room temperature and concentrated under reduced pressure to obtain the title compound (58 mg, 98%). MS m/z (ESI): 303.1 [M+H]⁺

### Step 5: synthesis of tert-butyl((1S)-2-((5-(2-cyclopropyl-1-(5,5-difluoro-2-carbonyltetrahydropyrimidin-1(2H)-yl)ethyl)thiazol-2-yl)amino)-1-(4,4-difluorocyclohexyl)-2-carbonylethyl)carbamate

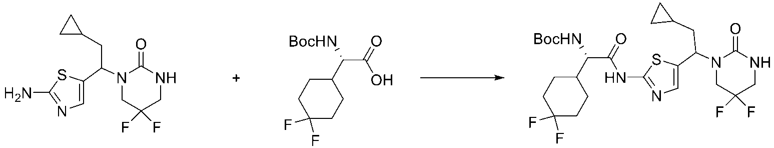

(S)-2-((tertbutoxycarbonyl)amino)-2-(4,4-difluorocyclohexyl)acetic acid (109 mg, 0.37 mmol), HATU (210 mg, 0.56 mmol) and DIPEA (120 mg, 0.93 mmol) were dissolved in N,N-dimethylacetamide (5 mL). The mixture was stirred at room temperature for 5 minutes. 1-(1-(2-aminothiazol-5-yl)-2-cyclopropylethyl)-5,5-difluorotetrahydropyrimidin-2(1H)-one hydrochloride (58 mg, 0.17 mmol) was added, and the mixture was reacted at room temperature for 0.5 hours. The reaction solution was diluted with water and extracted with ethyl acetate. The organic phases were combined, and washed with a saturated sodium chloride aqueous solution. The organic phase was dried over anhydrous sodium sulphate, filtered and then concentrated under reduced pressure to remove the organic solvent, and the residue was separated by column chromatography to obtain the title compound (75 mg, 75%). MS m/z (ESI): 578.2 [M+H]⁺.

### Step 6: synthesis of (2S)-2-amino-N-(5-(2-cyclopropyl-1-(5,5-difluoro-2-carbonyltetrahydropyrimidin-1(2H)-yl)ethyl)thiazol-2-yl)-2-(4,4-difluorocyclohexyl)acetamide hydrochloride

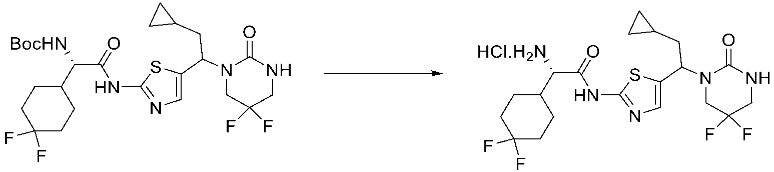

Tert-butyl((1S)-2-((5-(2-cyclopropyl-1-(5,5-difluoro-2-carbonyltetrahydropyrimidin-1(2H)-yl)ethyl)thiazol-2-yl)amino)-1-(4,4-difluorocyclohexyl)-2-carbonylethyl)carbamate (75 mg, 0.13 mmol) was dissolved in 4 M hydrogen chloride in dioxane (5 mL). The reaction was stirred at room temperature for 2 hours and concentrated under reduced pressure to remove the solvent, so as to obtain the title compound (66 mg, 99%). MS m/z (ESI): 478.2 [M+H]⁺

### Step 7: synthesis of N-((S)-2-((5-((R)-2-cyclopropyl-1-(5,5-difluoro-2-carbonyltetrahydropyrimidin-1(2H)-yl)ethyl)thiazol-2-yl)amino)-1-(4,4-difluorocyclohexyl)-2-carbonylethyl)-1-isopropyl-1H-pyrazole-5-carboxamide

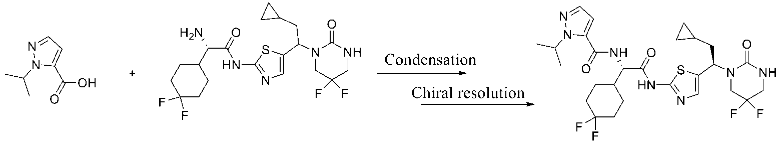

1-isopropyl-1H-pyrazole-5-carboxylic acid (30 mg, 0.19 mmol), HATU (122 mg, 0.32 mmol) and DIPEA (50 mg, 0.39 mmol) were dissolved in N,N-dimethylacetamide (5 mL). The mixture was stirred at room temperature for 5 minutes. (2S)-2-amino-N-(5-(2-cyclopropyl-1-(5,5-difluoro-2-carbonyltetrahydropyrimidin-1(2H)-yl)ethyl)thiazol-2-yl)-2-(4,4-difluorocyclohexyl)acetamide hydrochloride (66 mg, 0.13 mmol) was added, and the resulting reaction solution was further reacted at room temperature for 0.5 hours. The reaction solution was quenched with water and extracted with ethyl acetate. The organic phases were combined and washed with a saturated sodium chloride aqueous solution (50 mL). The organic phase was dried over anhydrous sodium sulphate, filtered and then concentrated under reduced pressure to remove the organic solvent, and the residue was sequentially subjected to column chromatography and chiral HPLC resolution to obtain the title compound (27 mg, 34%).

¹H NMR (400 MHz, DMSO-*d*₆) δ 12.31 (s, 1H), 8.70 (d, *J =* 7.6 Hz, 1H), 7.51 (d, *J =* 2.0 Hz, 1H), 7.38 (s, 1H), 6.94 (d, *J =* 2.0 Hz, 1H), 6.83 (d, *J =* 3.0 Hz, 1H), 5.59- 5.55 (m, 1H), 5.37-5.31 (m, 1H), 4.54 (t, *J =* 8.2 Hz, 1H), 3.60-3.43 (m, 3H), 3.27-3.21 (m, 1H), 2.11-1.87 (m, 4H), 1.85-1.52 (m, 3H), 1.36-1.32 (m, 8H), 1.26-1.22 (m, 2H), 0.75-0.65 (m, 1H), 0.43-0.38 (m, 2H), 0.16-0.11 (m, 2H); MS m/z (ESI) : 614.2 [M+H]⁺.

### Example 259 can also be synthesised according to the following steps

### Step 1: synthesis of tert-butyl 5-(1-((3-amino-2,2-difluoro-1,1,3,3-d₄)amino)-2-methoxyethylthiazol-2-yl)carbamate

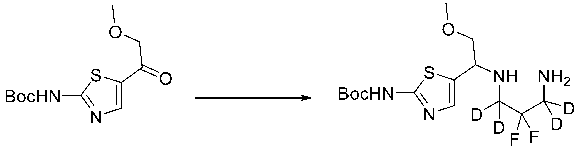

Tert-butyl(5-(2-methoxyacetyl)thiazol-2-yl)carbamate (500 mg, 1.84 mmol), 2,2-difluoropropane-1,1,3,3-*d*₄-1,3-diamine dihydrochloride (687 mg, 3.67 mmol) and triethylamine (927 mg, 9.18 mmol) were dissolved in a mixed solution of N,N-dimethylacetamide and methanol (22 mL, V/V = 10:1), and the mixture was stirred at 75°C for 12 hours. The reaction was cooled to room temperature, and sodium cyanoborohydride (808 mg, 12.85 mmol) and acetic acid (771 mg, 12.85 mmol) were added. The mixture was heated to 40°C and stirred and reacted for 12 hours. The reaction was cooled and then concentrated under reduced pressure to remove methanol. The residue was diluted with water and extracted with ethyl acetate. The organic phases were combined, and washed with a saturated sodium chloride aqueous solution. The organic phase was dried over anhydrous sodium sulphate, filtered and then concentrated under reduced pressure to remove the organic solvent, and the residue was separated by column chromatography to obtain the title compound (400 mg, 59%). MS m/z (ESI): 371.2 [M+H]⁺.

### Step 2: synthesis of tert-butyl 5-(1-(5,5-difluoro-2-oxotetrahydropyrimidin-1(2H)-yl-4,4,6-d₄)-2-methoxyethyl)thiazol-2-yl)carbamate

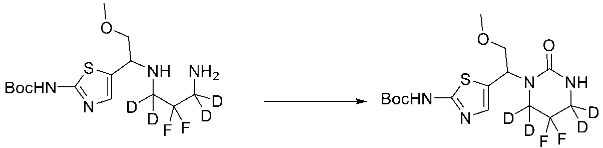

Tert-butyl 5-(1-((3-amino-2,2-difluoro-1,1,3,3-*d*₄)amino)-2-methoxyethylthiazol-2-yl)carbamate (400 mg, 1.1 mmol) was dissolved in tetrahydrofuran (8 mL). At room temperature, carbonyldiimidazole (350 mg, 2.2 mmol) was added. The mixture was stirred at room temperature for 1 hour. The reaction solution was diluted with an aqueous solution and extracted with ethyl acetate. The organic phases were combined, and washed with a saturated sodium chloride aqueous solution. The organic phase was dried over anhydrous sodium sulphate, filtered and then concentrated under reduced pressure to remove the organic solvent, and the residue was separated by column chromatography to obtain the title compound (180 mg, 42%). MS m/z (ESI): 397.2 [M+H]⁺.

### Step 3: synthesis of 1-(1-(2-aminothiazol-5-yl)-2-methoxyethyl)-5,5-difluorotetrahydropyrimidin-2(1H)-one 4,4,6,6-d₄ hydrochloride

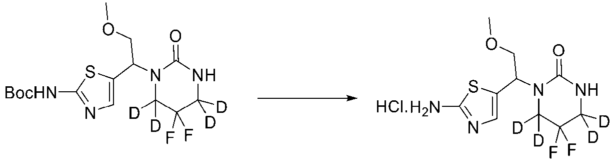

Tert-butyl 5-(1-(5,5-difluoro-2-oxotetrahydropyrimidin-1(2H)-yl-4,4,6-*d*₄)-2-methoxyethyl)thiazol-2-yl)carbamate (180 mg, 0.45 mmol) was dissolved in 4 M hydrogen chloride in dioxane (5 mL). The reaction was stirred at room temperature for 12 hours and concentrated under reduced pressure to remove the solvent, so as to obtain the title compound (140 mg, 93%). MS m/z (ESI): 297.1 [M+H]⁺.

### Step 4: synthesis of tert-butyl((1S)-2-((5-(1-(5,5-difluoro-2-carbonyltetrahydropyrimidin-1(2H)-yl-4,4,6,6-d₄)-2-methoxyethyl)thiazol-2-yl)amino)-1-(4,4-difluorocyclohexyl)-2-carbonylethyl)carbamate

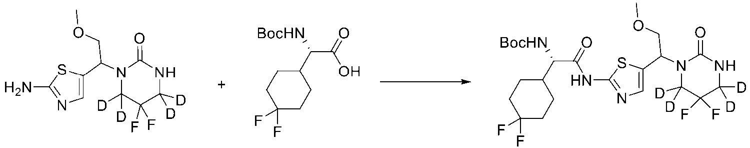

(S)-2-((tertbutoxycarbonyl)amino)-2-(4,4-difluorocyclohexyl)acetic acid (247 mg, 0.84 mmol), HATU (481 mg, 1.27 mmol) and DIPEA (272 mg, 2.11 mmol) were dissolved in N,N-dimethylacetamide (5 mL). The mixture was stirred at room temperature for 5 minutes. 1-(1-(2-aminothiazol-5-yl)-2-methoxyethyl)-5,5-difluorotetrahydropyrimidin-2(1H)-one 4,4,6,6*-d*₄ hydrochloride (140 mg, 0.42 mmol) was added, and the mixture was reacted at room temperature for 0.5 hours. The reaction solution was diluted with an aqueous solution and extracted with ethyl acetate. The organic phases were combined, and washed with a saturated sodium chloride aqueous solution. The organic phase was dried over anhydrous sodium sulphate, filtered and then concentrated under reduced pressure to remove the organic solvent, and the residue was separated by column chromatography to obtain the title compound (200 mg, 83%). MS m/z (ESI): 572.2 [M+H]⁺.

### Step 5: synthesis of (2S)-2-amino-N-(5-(1-(5,5-difluoro-2-carbonyltetrahydropyrimidin-1(2H)-yl-4,4,6,6-d₄)-2-methoxyethyl)thiazol-2-yl)-2-(4,4-difluorocyclohexyl)acetamide hydrochloride

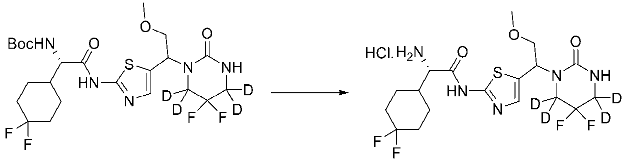

Tert-butyl((1S)-2-((5-(1-(5,5-difluoro-2-carbonyltetrahydropyrimidin-1(2H)-yl-4,4,6,6-*d*₄)-2-methoxyethyl)thiazol-2-yl)amino)-1-(4,4-difluorocyclohexyl)-2-carbonylethyl)carbamate (200 mg, 0.35 mmol) was dissolved in 4 M hydrogen chloride in dioxane (20 mL). The reaction was stirred at room temperature for 2 hours and concentrated under reduced pressure to remove the solvent, so as to obtain the title compound (165 mg, 93%). MS m/z (ESI): 472.2 [M+H]⁺.

### Step 6: synthesis of N-((S)-2-((5-((R)-1-(5,5-difluoro-2-carbonyltetrahydropyrimidin-1(2H)-yl-4,4,6,6-d₄)-2-methoxyethyl)thiazol-2-yl)amino)-1-(4,4-difluorocyclohexyl)-2-carbonylethyl)-1-isopropyl-1H-pyrazole-5-carboxamide

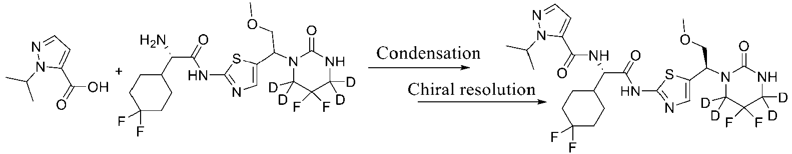

1-isopropyl-1H-pyrazole-5-carboxylic acid (80 mg, 0.52 mmol), HATU (247 mg, 0.65 mmol) and DIPEA (126 mg, 0.98 mmol) were dissolved in N,N-dimethylacetamide (5 mL). The mixture was stirred at room temperature for 5 minutes. (2S)-2-amino-N-(5-(1-(5,5-difluoro-2-carbonyltetrahydropyrimidin-1(2H)-yl-4,4,6,6-*d*₄)-2-methoxyethyl)thiazol-2-yl)-2-(4,4-difluorocyclohexyl)acetamide hydrochloride (165 mg, 0.33 mmol) was added. The reaction solution was further stirred and reacted at room temperature for 0.5 hours. The reaction solution was quenched with an aqueous solution and extracted with ethyl acetate. The organic phases were combined, and washed with a saturated sodium chloride aqueous solution. The organic phase was dried over anhydrous sodium sulphate, filtered and then concentrated under reduced pressure to remove the organic solvent, and the residue was sequentially separated by reversed-phase HPLC and subjected to chiral HPLC resolution to obtain the title compound (73 mg, 43%).

¹H NMR (400 MHz, DMSO-*d*₆) *δ* 12.35 (s, 1H), 8.70 (d, *J =* 7.5 Hz, 1H), 7.51 (d, *J =* 2.0 Hz, 1H), 7.38 (d, *J =* 1.1 Hz, 1H), 6.94 (d, *J =* 2.0 Hz, 1H), 6.88 (s, 1H), 5.68 (t, *J=* 6.8 Hz, 1H), 5.38-5.31 (m, 1H), 4.55 (t, *J* = 8.2 Hz, 1H), 4.22 (t, *J=* 6.6 Hz, 1H), 3.88-3.72 (m, 2H), 3.31 (s, 3H), 2.11-1.96 (m, 3H), 1.93 - 1.72 (m, 2H), 1.66-1.57 (m, 1H), 1.40-1.30 (m, 6H), 1.26-1.18 (m, 1H), 0.91 (t, *J =* 7.4 Hz, 1H); MS m/z (ESI) :608.2 [M+H]⁺.

### Example 569 can also be synthesised according to the following steps

### Step 1: preparation of tert-butyl(5-(1-hydroxyl-2-methoxyethyl)thiazol-2-yl)carbamate

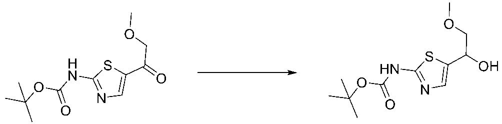

Tert-butyl(5-(2-methoxyacetyl)thiazol-2-yl)carbamate (1.0 g, 3.7 mmol) was dissolved in a tetrahydrofuran/methanol solution (15 mL, V/V=1:1), and the mixture was cooled to 0°C in an ice-water bath. Sodium borohydride (278 mg, 7.36 mmol) was added in batches, and the reaction was stirred at 0°C for 1 hour and concentrated under reduced pressure to remove the organic solvent. The residue was diluted with water and extracted three times with dichloromethane. The organic phases were combined and washed with a saturated sodium chloride aqueous solution. The organic phase was separated, dried over anhydrous sodium sulphate, filtered and then concentrated under reduced pressure to remove the organic solvent, and the residue was separated by column chromatography to obtain the title compound (1.0 g, 99%). MS m/z (ES⁺): 275.1 [M+H]⁺.

### Step 2: preparation of tert-butyl(5-(1-(5-chloro-2-carbonylpyridin-1(2H)-yl)-2-methoxyethyl)thiazol-2-yl)carbamate

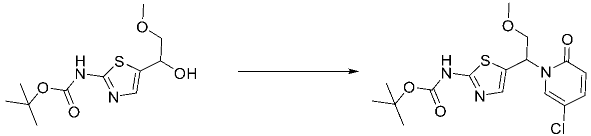

Tert-butyl(5-(1-hydroxyl-2-methoxyethyl)thiazol-2-yl)carbamate (750 mg, 2.7 mmol), 5-chloropyridin-2(1H)-one (118 mg, 0.9 mmol) and methyl trifluoromethanesulfonate (33 mg, 0.2 mmol) were added to toluene (10 mL). The mixture was heated to 100°C, stirred for 2 hours and concentrated under reduced pressure to remove the organic solvent, and the residue was separated by column chromatography to obtain the title compound (68 mg, 20%). MS m/z (ES⁺): 386.1 [M+H]⁺.

### Step 3: preparation of 1-(1-(2-aminothiazol-5-yl)-2-methoxyethyl)-5-chloropyridin-2(1H)-one hydrochloride

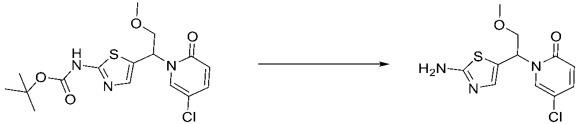

Tert-butyl(5-(1-(5-chloro-2-carbonylpyridin-1(2H)-yl)-2-methoxyethyl)thiazol-2-yl)carbamate (68 mg, 0.18 mmol) was dissolved in dichloromethane (3 mL). 4 M hydrogen chloride in dioxane (3 mL) was added, and the reaction was stirred at room temperature for 12 hours and concentrated under reduced pressure to remove the organic solvent, so as to obtain the title compound (57 mg). MS m/z (ESI): 286.1 [M+H]⁺.

### Step 4: preparation of tert-butyl((1S)-2-((5-(1-(5-chloro-2-carbonylpyridin-1(2H)-yl)-2-methoxyethyl)thiazol-2-yl)amino)-1-((trans)-4-methylcyclohexyl)-2-carbonylethyl)carbamate

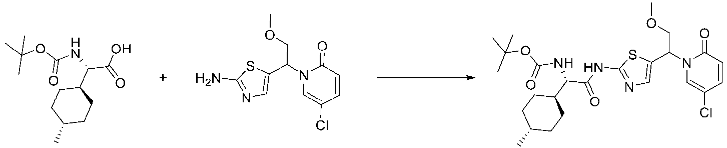

(S)-2-((tert-butoxycarbonyl)amino)-2-((trans)-4-methylcyclohexyl)acetic acid (48 mg, 0.18 mmol) and 1-(1-(2-aminothiazol-5-yl)-2-methoxyethyl)-5-chloropyridin-2(1H)-one hydrochloride (57 mg, 0.18 mmol) was dissolved in DMF (5 mL). Triethylamine (54 mg, 0.53 mmol) and PyBOP (138 mg, 0.27 mmol) were added, and the mixture was stirred at room temperature for 2 hours. The reaction solution was diluted with water, and extracted three times with ethyl acetate. The organic phases were combined and washed with a saturated sodium chloride aqueous solution. The organic phase was dried over anhydrous sodium sulphate, filtered and then concentrated under reduced pressure to remove the organic solvent, and the residue was separated by column chromatography to obtain the title compound (60 mg, 62%). MS m/z (ESI): 539.2 [M+H]⁺.

### Step 5: preparation of (2S)-2-amino-N-(5-(1-(5-chloro-2-carbonylpyridin-1(2H)-yl)-2-methoxyethyl)thiazol-2-yl)-2-((trans)-4-methylcyclohexyl)acetamide hydrochloride

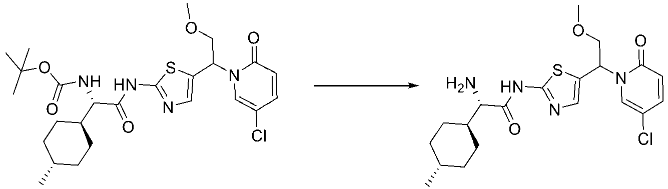

Tert-butyl((1S)-2-((5-(1-(5-chloro-2-carbonylpyridin-1(2H)-yl)-2-methoxyethyl)thiazol-2-yl)amino)-1-((trans)-4-methylcyclohexyl)-2-carbonylethyl)carbamate (60 mg, 0.11 mmol) was dissolved in dichloromethane (3 mL). 4 M hydrogen chloride in dioxane (3 mL) was added, and the reaction was stirred at room temperature for 1 hour and concentrated under reduced pressure to remove the organic solvent, so as to obtain the title compound (52 mg). MS m/z (ESI): 439.2 [M+H]⁺.

### Step 6: preparation of N-((S)-2-((5-((R)-1-(5-chloro-2-carbonylpyridin-1(2H)-yl)-2-methoxyethyl)thiazol-2-yl)amino)-1-((trans)-4-methylcyclohexyl)-2-carbonylethyl)-4-methyl-1,2,5-oxadiazole-3-carboxamide

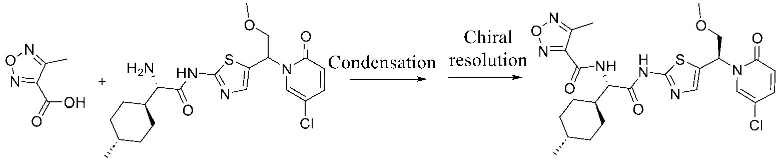

4-methyl-1,2,5-oxadiazole-3-carboxylic acid (14.3 mg, 0.11 mmol) and (2S)-2-amino-N-(5-(1-(5-chloro-2-carbonylpyridin-1(2H)-yl)-2-methoxyethyl)thiazol-2-yl)-2-((trans)-4-methylcyclohexyl)acetamide (49 mg, 0.11 mmol) were dissolved in DMF (3 mL). Triethylamine (34 mg, 0.34 mmol) and PyBOP (88 mg, 0.17 mmol) were sequentially added, and the mixture was stirred at room temperature for 2 hours. The reaction solution was diluted with water, and extracted three times with ethyl acetate. The organic phases were combined and washed with a saturated sodium chloride aqueous solution. The organic phase was dried over anhydrous sodium sulphate, filtered and then concentrated under reduced pressure to remove the organic solvent, and the residue was sequentially separated by reversed-phase HPLC and subjected to chiral HPLC resolution to obtain the title compound (14 mg, 23%).

¹H NMR (400 MHz, DMSO-*d*₆) *δ* 12.58-12.28 (m, 1H), 9.29 (d, *J =* 7.8 Hz, 1H), 7.94 (d, *J =* 5.8 Hz, 1H), 7.56 (s, 1H), 7.50-7.45 (m, 1H), 6.48 (d, *J =* 9.6 Hz, 1H), 6.30-6.24 (m, 1H), 4.52 (t, *J =* 7.8 Hz, 1H), 4.27-4.21 (m, 1H), 3.94-3.86 (m, 1H), 3.30 (s, 3H), 2.45 (s, 3H), 1.85-1.76 (m, 2H), 1.71-1.63 (m, 2H), 1.51-1.44 (m, 1H), 1.23-0.99 (m, 4H), 0.93-0.87 (m, 1H), 0.84 (d, *J =* 6.4 Hz, 3H); MS m/z (ESI): 549.2 [M+H]⁺.

### Example 560 can also be synthesised according to the following steps

### Step 1: preparation of methyl 3-(2-aminothiazol-5-yl)-3-(6-bromoimidazo[1,2-a]pyridin-3-yl)propanoate

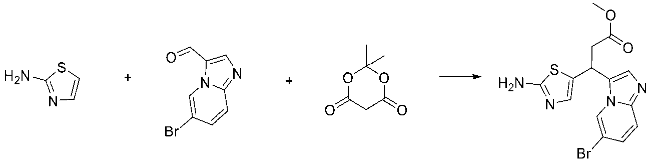

Thiazol-2-amine (2.03 g, 20.3 mmol), 6-bromoimidazo[1,2-a]pyridine-3-carbaldehyde (5.0 g, 22.3 mmol) and 2,2-dimethyl-1,3-dioxane-4,6-dione (2.93 g, 20.3 mmol) were dissolved in a mixed solvent of DMF/methanol (100 mL, V/V=1: 1). The reaction was heated to 85°C in a sealed tube, stirred for 10 hours and concentrated under reduced pressure to remove the organic solvent. The resulting crude product was slurried with ethyl acetate to obtain the title compound (5.1 g, 66%). MS m/z (ES⁺): 381.0 [M+H]⁺.

### Step 2: preparation of methyl 3-(6-bromoimidazo[1,2-a]pyridin-3-yl)-3-(2-((S)-2-((tert-butoxycarbonyl)amino)-2-(4,4-difluorocyclohexyl)acetamido)thiazol-5-yl)propanoate

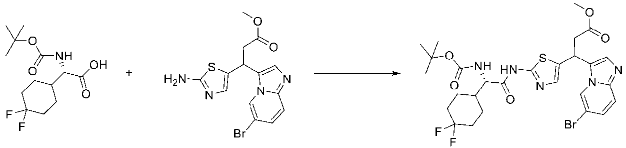

(S)-2-((tert-butoxycarbonyl)amino)-2-(4,4-difluorocyclohexyl)acetic acid (1.0 g, 3.41 mmol) and methyl 3-(2-aminothiazol-5-yl)-3-(6-bromoimidazo[1,2-a]pyridin-3-yl)propanoate (1.6 g, 4.10 mmol) were dissolved in DMF (15 mL). Triethylamine (1.03 g, 10.23 mmol) and HATU (1.94 g, 5.11 mmol) were sequentially added. The reaction was stirred at room temperature for 10 hours. The reaction solution was quenched with water, and extracted three times with ethyl acetate. The organic phases were combined and washed with a saturated sodium chloride aqueous solution. The organic phase was dried over anhydrous sodium sulphate, filtered and then concentrated under reduced pressure to remove the organic solvent, and the residue was separated by column chromatography to obtain the title compound (1.6 g, 72%). MS m/z (ESI): 656.1 [M+H]⁺.

### Step 3: preparation of methyl 3-(2-((S)-2-amino-2-(4,4-difluorocyclohexyl)acetamido)thiazol-5-yl)-3-(6-bromoimidazo[1,2-a]pyridin-3-yl)propanoate hydrochloride

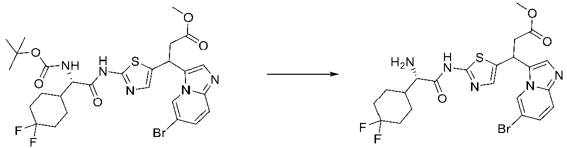

Methyl 3-(6-bromoimidazo[1,2-a]pyridin-3-yl)-3-(2-((S)-2-((tert-butoxycarbonyl)amino)-2-(4,4-difluorocyclohexyl)acetamido)thiazol-5-yl)propanoate (858 mg, 1.31 mmol) was dissolved in dichloromethane (8 mL). 4 M hydrogen chloride in dioxane (8 mL) was added, and the reaction was stirred and reacted at room temperature for 1 hour and concentrated under reduced pressure to remove the organic solvent, so as to obtain the title compound (776 mg, 100%). MS m/z (ESI): 556.1 [M+H]⁺.

### Step 4: preparation of methyl 3-(6-bromoimidazo[1,2-a]pyridin-3-yl)-3-(2-((S)-2-(4,4-difluorocyclohexyl)-2-(1-isopropyl-1H-pyrazole-5-carboxamido<oxalamide>)acetamido)thiazol-5-yl)propanoate

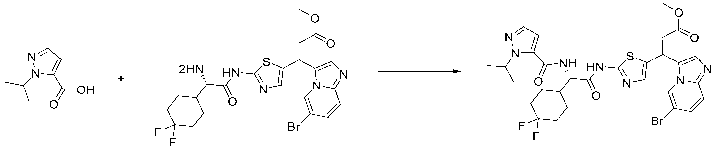

1-isopropyl-1H-pyrazole-5-carboxylic acid (202 mg, 1.31 mmol) and methyl 3-(2-((S)-2-amino-2-(4,4-difluorocyclohexyl)acetamido)thiazol-5-yl)-3-(6-bromoimidazo[1,2-a]pyridin-3-yl)propanoate (727 mg, 1.31 mmol) were dissolved in DMF (10 mL). Triethylamine (397 mg, 3.93 mmol) and PyBOP (1.02 g, 1.97 mmol) were sequentially added, and the mixture was stirred at room temperature for 2 hours. The reaction solution was diluted with water, and extracted three times with ethyl acetate. The organic phases were combined and washed with a saturated sodium chloride aqueous solution. The organic phase was dried over anhydrous sodium sulphate, filtered and then concentrated under reduced pressure to remove the organic solvent, and the residue was separated by column chromatography to obtain the title compound (762 mg, 84%). MS m/z (ESI): 692.1 [M+H]⁺.

### Step 5: preparation of methyl(S)-3-(6-cyanoimidazo[1,2-a]pyridin-3-yl)-3-(2-((S)-2-(4,4-difluorocyclohexyl)-2-(1-isopropyl-1H-pyrazole-5-carboxamido<oxalamide>)acetamido)thiazol-5-yl)propanoate

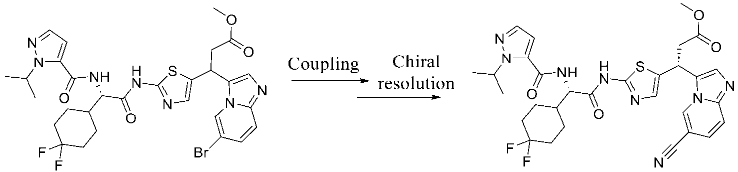

Methyl 3-(6-bromoimidazo[1,2-a]pyridin-3-yl)-3-(2-((S)-2-(4,4-difluorocyclohexyl)-2-(1-isopropyl-1H-pyrazole-5-carboxamido<oxalamide>)acetamido)thiazol-5-yl)propanoate (500 mg, 0.72 mmol), zinc cyanide (127.5 mg, 1.09 mmol) and tetrakis(triphenylphosphine)palladium (418 mg, 0.36 mmol) were dissolved in DMF (10 mL). The reaction solution was sealed and purged with dry nitrogen three times, and then heated to 110°C by microwave and reacted for 1 hour. The reaction solution was cooled to room temperature, diluted with ethyl acetate and washed with a saturated sodium chloride aqueous solution. The organic phase was dried over anhydrous sodium sulphate, filtered and then concentrated under reduced pressure to remove the organic solvent, and the residue was sequentially separated by column chromatography and subjected to chiral HPLC resolution to obtain the title compound (140 mg, 31%). MS m/z (ESI): 639.2 [M+H]⁺.

### Step 6: preparation of (S)-3-(6-cyanoimidazo[1,2-a]pyridin-3-yl)-3-(2-((S)-2-(4,4-difluorocyclohexyl)-2-(1-isopropyl-1H-pyrazole-5-carboxamido<oxalamide>)acetamido)thiazol-5-yl)propanoic acid

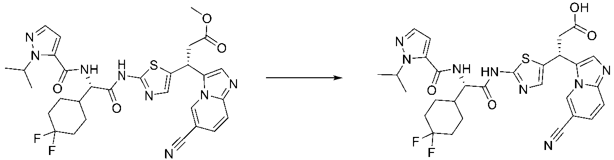

Methyl(S)-3-(6-cyanoimidazo[1,2-a]pyridin-3-yl)-3-(2-((S)-2-(4,4-difluorocyclohexyl)-2-(1-isopropyl-1H-pyrazole-5-carboxamido<oxalamide>)acetamido)thiazol-5-yl)propanoate (140 mg, 0.22 mmol) was dissolved in tetrahydrofuran/water (10 mL, V/V=1:1). Lithium hydroxide (6.3 mg, 0.26 mmol) was added, and the mixture was stirred at room temperature for 1 hour. The reaction solution was diluted with water, adjusted to pH 6 with 1 N hydrochloric acid, and extracted three times with ethyl acetate. The organic phases were combined and washed with a saturated sodium chloride aqueous solution. The organic phase was dried over anhydrous sodium sulphate, filtered and then concentrated under reduced pressure to remove the organic solvent, and the residue was separated by HPLC to obtain the title compound (77 mg, 56%).

¹H NMR (400 MHz, DMSO-*d⁶*) *δ* 9.25 (s, 1H), 8.64 (d, *J =* 7.6 Hz, 1H), 7.74 (s, 1H), 7.66 (d, *J =* 9.4 Hz, 1H), 7.44-7.39 (m, 2H), 7.37 (s, 1H), 6.85 (d, *J =* 6.4 Hz, 1H), 5.31-5.22 (m, 1H), 5.15 (t, *J =* 7.8 Hz, 1H), 4.41 (t, *J =* 8.2 Hz, 1H), 3.15-3.07 (m, 1H), 3.01-2.92 (m, 1H), 1.98-1.77 (m, 5H), 1.74-1.55 (m, 3H), 1.48-1.39 (m, 1H), 1.26 (t, *J =* 7.0 Hz, 6H); MS m/z (ESI): 625.2 [M+H]⁺.

### Example 564 can also be synthesised according to the following steps

### Step 1: preparation of tert-butyl(5-(3-methoxypropionyl)thiazol-2-yl)carbamate

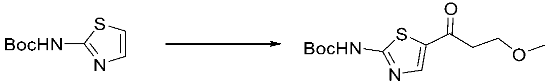

Tert-butyl thiazol-2-ylcarbamate (10.20 g, 50.93 mmol) was dissolved in tetrahydrofuran (200 mL). The mixture was cooled to -78°C, and an n-butyllithium solution (51 mL, 127.5 mmol, 2.5 M in n-hexane) was slowly added dropwise. After the dropwise addition, the reaction solution was further stirred at -78°C for 30 minutes. N,3-dimethoxy-N-methylpropanamide (14.99 g, 101.87 mmol) was added. The reaction solution was further stirred at -78°C for 2 hours. The reaction was quenched with a saturated ammonium chloride, and a white solid precipitated. The resulting precipitate was filtered under reduced pressure, and the filter cake was washed with water, collected and dried under reduced pressure to obtain the title compound (6.1 g, 42%). MS m/z (ESI): 287.1 [M+H]⁺.

### Step 2: preparation of tert-butyl(5-(1-(((S)-2-amino-3,3,3-trifluoropropyl)amino)-3-methoxypropyl)thiazol-2-yl)carbamate

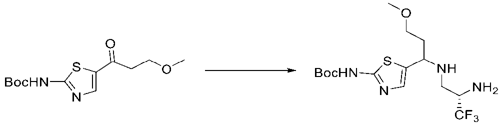

Tert-butyl(5-(3-methoxypropionyl)thiazol-2-yl)carbamate (1.0 g, 3.49 mmol) and (S)-3,3,3-trifluoropropane-1,2-diamine dihydrochloride (1.40 g, 6.98 mmol) were dissolved in a mixed solvent of DMA (10 mL) and methanol (5 mL). Triethylamine (1.67 g, 16.52 mmol) was added, and the mixture was heated to 75°C, stirred for 12 hours and cooled to room temperature. Acetic acid (1.32 g, 22.03 mmol) and sodium cyanoborohydride (1.38 g, 22.03 mmol) were added. The reaction was heated to 40°C, stirred for 6 hours, cooled to room temperature, diluted with ethyl acetate and washed with a saturated sodium chloride aqueous solution. The organic phase was separated, dried over anhydrous sodium sulphate, filtered and then concentrated under reduced pressure to remove the organic solvent, and the residue was subjected to column chromatography to obtain the title compound (660 mg, 47%). MS m/z (ESI): 399.2 [M+H]⁺.

### Step 3: preparation of tert-butyl(5-(3-methoxy-1-((S)-2-carbonyl-4-(trifluoromethyl)imidazolidin-1-yl)propyl)thiazol-2-yl)carbamate

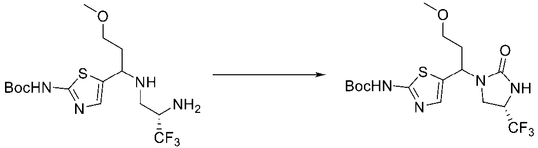

Tert-butyl(5-(1-(((S)-2-amino-3,3,3-trifluoropropyl)amino)-3-methoxypropyl)thiazol-2-yl)carbamate (500 mg, 1.25 mmol) was dissolved in tetrahydrofuran (6 mL). N,N'-carbonyldiimidazole (407 mg, 2.51 mmol) was added. The mixture was stirred at 30°C for 1 hour, and a white solid precipitated. The resulting precipitate was filtered under reduced pressure and collected, and the filter cake was washed with a small amount of ethyl acetate, collected and dried under reduced pressure to obtain the title compound (310 mg, 58%). MS m/z (ESI): 425.1 [M+H]⁺.

### Step 4: preparation of (4S)-1-(1-(2-aminothiazol-5-yl)-3-methoxypropyl)-4-(trifluoromethyl)imidazolidin-2-one hydrochloride

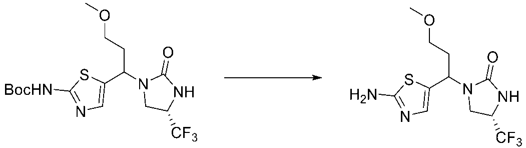

Tert-butyl(5-(3-methoxy-1-((S)-2-carbonyl-4-(trifluoromethyl)imidazolidin-1-yl)propyl)thiazol-2-yl)carbamate (150 mg, 0.35 mmol) was dissolved in dichloromethane (1 mL). 4 M hydrogen chloride in dioxane (3 mL) was added, and the mixture was stirred at room temperature for 8 hours and concentrated under reduced pressure to obtain the title compound (125 mg). MS m/z (ESI): 325.1 [M+H]⁺.

### Step 5: preparation of tert-butyl((1S)-1-cyclohexyl-2-((5-(3-methoxy-1-((S)-2-carbonyl-4-(trifluoromethyl)imidazolidin-1-yl)propyl)thiazol-2-yl)amino)-2-carbonylethyl)carbamate

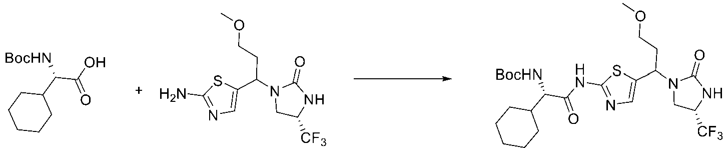

(S)-2-((tert-butoxycarbonyl)amino)-2-cyclohexyl acetic acid (125 mg, 0.49 mmol) was dissolved in DMF (3 mL). HATU (186.31 mg, 0.49 mmol) and DIPEA (274 mg, 2.12 mmol) were sequentially added, and the mixture was stirred at room temperature for 10 minutes. (4S)-1-(1-(2-aminothiazol-5-yl)-3-methoxypropyl)-4-(trifluoromethyl)imidazolidin-2-one hydrochloride (110 mg, 0.305 mmol) was added, and the reaction was further stirred at room temperature for 1 hour. The reaction solution was diluted with ethyl acetate and washed with a saturated sodium chloride aqueous solution. The organic phase was separated, dried over anhydrous sodium sulphate, filtered and then concentrated under reduced pressure to remove the organic solvent, and the residue was subjected to column chromatography to obtain the title compound (125 mg, 73%). MS m/z (ESI): 564.2 [M+H]⁺.

### Step 6: preparation of (2S)-2-amino-2-cyclohexyl-N-(5-(3-methoxy-1-((S)-2-carbonyl-4-(trifluoromethyl)imidazolidin-1-yl)propyl)thiazol-2-yl)acetamide hydrochloride

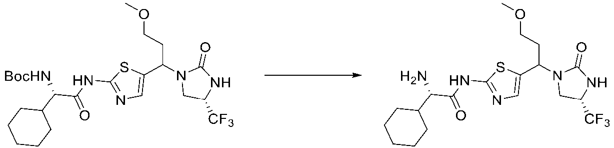

Tert-butyl((1S)-1-cyclohexyl-2-((5-(3-methoxy-1-((S)-2-carbonyl-4-(trifluoromethyl)imidazolidin-1-yl)propyl)thiazol-2-yl)amino)-2-carbonylethyl)carbamate (125 mg, 0.22 mmol) was dissolved in dichloromethane (0.5 mL). 4 M hydrogen chloride in dioxane (3 mL) was added, and the mixture was stirred at room temperature for 1 hour and concentrated under reduced pressure to remove the organic solvent, so as to obtain the title compound (111 mg, 100%). MS m/z (ESI): 464.2 [M+H]⁺.

### Step 7: preparation of N-((1S)-1-cyclohexyl-2-((5-(3-methoxy-1-((S)-2-carbonyl-4-(trifluoromethyl)imidazolidin-1-yl)propyl)thiazol-2-yl)amino)-2-carbonylethyl)-1-isopropyl-1H-pyrazole-5-carboxamide

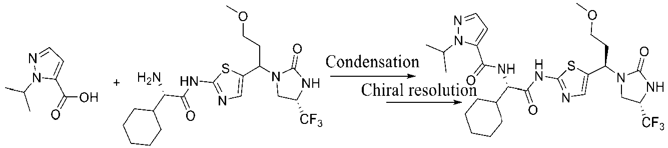

1-isopropyl-1H-pyrazole-5-carboxylic acid (54 mg, 0.35 mmol) was dissolved in DMF (3 mL). HATU (137 mg, 0.36 mmol) and DIPEA (146 mg, 1.13 mmol) were sequentially added, and the mixture was stirred at room temperature for 5 minutes. (2S)-2-amino-2-cyclohexyl-N-(5-(3-methoxy-1-((S)-2-carbonyl-4-(trifluoromethyl)imidazolidin-1-yl)propyl)thiazol-2-yl)acetamide hydrochloride (109 mg, 0.218 mmol) was added, and the mixture was further stirred at room temperature for 1 hour. The reaction solution was diluted with ethyl acetate and washed with a saturated sodium chloride aqueous solution. The organic phase was separated, dried over anhydrous sodium sulphate, filtered and then concentrated under reduced pressure to remove the organic solvent, and the residue was subjected to column chromatography and chiral HPLC resolution to obtain the title compound (23.5 mg, 18%). MS m/z (ESI): 600.3 [M+H]⁺.

### Example 559 can also be synthesised according to the following steps

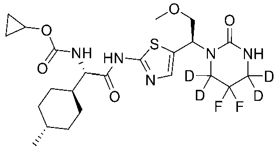

At 0°C, (2S)-2-amino-N-(5-(1-(5,5-difluoro-2-carbonyltetrahydropyrimidin-1(2H)-yl-4,4,6,6-d4)-2-methoxyethyl)thiazol-2-yl)-2-((1r,4S)-4-methylcyclohexyl)acetamide hydrochloride (95 mg, 0.195 mmol) and DIPEA (100 mg, 0.77 mmol) were dissolved in dichloromethane (5 mL). A solution of cyclopropyl chloroformate (33 mg, 0.27 mmol) in dichloromethane (1 mL) was slowly added, and the mixture was further stirred at 0°C for 1 hour. The reaction solution was diluted with dichloromethane and washed with a saturated sodium chloride aqueous solution. The organic phase was dried over anhydrous sodium sulphate, filtered and concentrated under reduced pressure, and the residue was subjected to column chromatography and chiral resolution to obtain the title compound (33 mg, 31.6%).

¹H NMR (400 MHz, DMSO-*d₆*) *δ* 12.13 (s, 1H), 7.45 (d, *J =* 8.0 Hz, 1H), 7.36 (s, 1H), 6.86 (s, 1H), 5.68 (t*, J =* 6.8 Hz, 1H), 4.09 (t, *J =* 7.8 Hz, 1H), 3.95-3.88 (m, 1H), 3.82-3.70 (m, 2H), 3.31 (s, 3H), 1.72-1.52 (m, 4H), 1.44-0.93 (m, 4H), 0.87-0.84 (m, 1H), 0.83-0.80 (m, 3H), 0.79-0.74 (m, 1H), 0.71-0.42 (m, 4H); MS m/z (ESI): 534.2 [M+H]^{+.}

### Example 574 can also be synthesised according to the following steps

### Step 1: preparation of N-methoxy-N-methyl-2-morpholinoacetamide

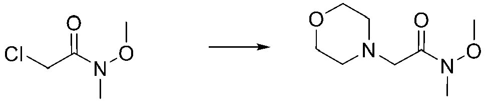

At room temperature, 2-chloro-N-methoxy-N-methylacetamide (10.0 g, 72.6 mmol) and morpholine (8.2 g, 94.3 mmol) were dissolved in acetonitrile (100 mL). Potassium carbonate (20.0 g, 147 mmol) was added. The mixture was stirred at room temperature for 10 hours, filtered and then concentrated under reduced pressure to remove the organic solvent, and the residue was separated by column chromatography to obtain the title compound (11.1 g, 82%). MS m/z (ES⁺): 189.1 [M+H]⁺.

### Step 2: preparation of tert-butyl(5-(2-morpholinoacetyl)thiazol-2-yl)carbamate

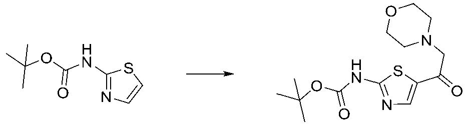

At room temperature, tert-butyl thiazol-2-ylcarbamate (11.7 g, 58.5 mmol) was dissolved in tetrahydrofuran (150 mL). The mixture was cooled to -78°C, and n-butyllithium (58.5 mL, 146.3 mmol) was added dropwise. After the dropwise addition, the mixture was stirred at -78°C for 1 hour. A solution of N-methoxy-N-methyl-2-morpholinoacetamide (11.0 g, 58.5 mmol) dissolved in tetrahydrofuran (20 mL) was added dropwise. After the dropwise addition, the mixture was stirred at -78°C for 1 hour. A saturated ammonium chloride solution was added, and the mixture was extracted three times with ethyl acetate. The organic phases were combined and washed with a saturated sodium chloride aqueous solution. The organic phase was dried over anhydrous sodium sulphate, filtered and then concentrated under reduced pressure to remove the organic solvent, and the residue was separated by column chromatography to obtain the title compound (7.7 g, 40%). MS m/z (ES⁺): 328.1 [M+H]⁺.

### Step 3: preparation of tert-butyl(5-(1-hydroxyl-2-morpholinoethyl)thiazol-2-yl)carbamate

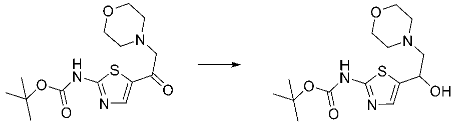

At room temperature, tert-butyl(5-(2-morpholinoacetyl)thiazol-2-yl)carbamate (7.7 g, 23.5 mmol) was dissolved in a tetrahydrofuran/methanol solution (50 mL). The mixture was cooled to 0°C, and sodium borohydride (1.7 g, 47.0 mmol) was added in batches. The mixture was stirred at 0°C for 1 hour and concentrated under reduced pressure to remove the organic solvent. Water was added, and the mixture was extracted three times with dichloromethane. The organic phases were combined and washed with a saturated sodium chloride aqueous solution. The organic phase was dried over anhydrous sodium sulphate, filtered and then concentrated under reduced pressure to remove the organic solvent, and the residue was separated by column chromatography to obtain the title compound (6.2 g, 81%). MS m/z (ES⁺): 330.1 [M+H]⁺.

### Step 4: preparation of tert-butyl(5-(1-(5-chloro-2-carbonylpyridin-1(2H)-yl)-2-morpholinoethyl)thiazol-2-yl)carbamate

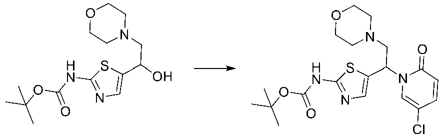

At room temperature, tert-butyl(5-(1-hydroxyl-2-morpholinoethyl)thiazol-2-yl)carbamate (2.0 g, 6.1 mmol) and 5-chloropyridin-2(1H)-one (395.0 mg, 3.1 mmol) were dissolved in a toluene solution (30 mL). Methyl trifluoromethanesulfonate (246.1 mg, 1.5 mmol) was added dropwise. The mixture was heated to 110°C, stirred for 5 hours and concentrated under reduced pressure to remove the organic solvent, and the residue was separated by column chromatography to obtain the title compound (1.1 g, 81%). MS m/z (ES⁺): 441.1 [M+H]⁺.

### Step 5: preparation of 1-(1-(2-aminothiazol-5-yl)-2-morpholinoethyl)-5-chloropyridin-2(1H)-one

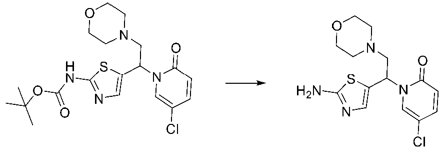

To a solution of tert-butyl(5-(1-(5-chloro-2-carbonylpyridin-1(2H)-yl)-2-morpholinoethyl)thiazol-2-yl)carbamate (1.1 g, 2.5 mmol) in dichloromethane (6 mL) was added hydrogen chloride in dioxane (6 mL). The mixture was stirred overnight at room temperature and concentrated under reduced pressure to remove the organic solvent, so as to obtain the title compound (crude hydrochloride, 850 mg), which was directly used in the next step. MS m/z (ESI): 341.1 [M+H]⁺.

### Step 6: preparation of tert-butyl((S)-2-((5-((R)-1-(5-chloro-2-carbonylpyridin-1(2H)-yl)-2-morpholinoethyl)thiazol-2-yl)amino)-1-((1r,4S)-4-methylcyclohexyl)-2-carbonylethyl)carbamate

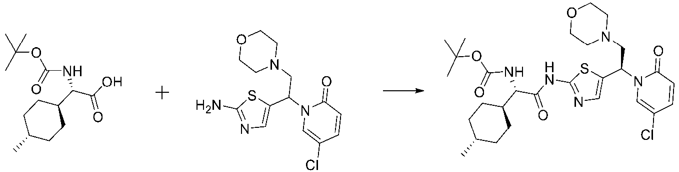

At room temperature, (S)-2-((tert-butoxycarbonyl)amino)-2-((1r,4S)-4-methylcyclohexyl)acetic acid (1.36 g, 5.0 mmol) and 1-(1-(2-aminothiazol-5-yl)-2-morpholinoethyl)-5-chloropyridin-2(1H)-one (850 mg, 2.5 mmol) were dissolved in N,N-dimethylformamide (10 mL). Triethylamine (757.5 mg, 7.5 mmol) and benzotriazol-1-yl-oxy-tripyrrolidinophosphonium hexafluorophosphate (2.6 g, 5.0 mmol) were added, and the mixture was stirred at room temperature for 2 hours. Water was added, and the mixture was extracted three times with ethyl acetate. The organic phases were combined and washed with a saturated sodium chloride aqueous solution. The organic phase was dried over anhydrous sodium sulphate, filtered and then concentrated under reduced pressure to remove the organic solvent, and the residue was separated by column chromatography and chiral resolution to obtain the title compound (291 mg, 20%). MS m/z (ESI): 594.1 [M+H]⁺.

### Step 7: preparation of (S)-2-amino-N-(5-((R)-1-(5-chloro-2-carbonylpyridin-1(2H)-yl)-2-morpholinoethyl)thiazol-2-yl)-2-((1r,4S)-4-methylcyclohexyl)acetamide

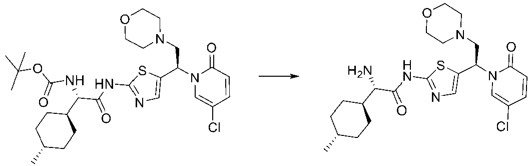

To a solution of tert-butyl((S)-2-((5-((R)-1-(5-chloro-2-carbonylpyridin-1(2H)-yl)-2-morpholinoethyl)thiazol-2-yl)amino)-1-((1r,4S)-4-methylcyclohexyl)-2-carbonylethyl)carbamate (291 mg, 0.49 mmol) in dichloromethane (5 mL) was added hydrogen chloride in dioxane (5 mL), and the mixture was stirred at room temperature for 1 hour and concentrated under reduced pressure to remove the organic solvent, so as to obtain the title compound (crude hydrochloride, 241 mg), which was directly used in the next step. MS m/z (ESI): 494.1 [M+H]⁺.

### Step 8: synthesis of methyl 1-(ethyl-ds)-1H-pyrazole-5-carboxylate

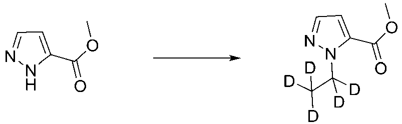

Methyl 1H-pyrazole-5-carboxylate (9 g, 71.43 mmol) and 1-bromoethane-1,1,2,2,2-*d*₅ (9.8 g, 86 mmol) were dissolved in acetonitrile (200 mL). Caesium carbonate (35 g, 107.36 mmol) was added, and the mixture was reacted at room temperature for 2 hours. An aqueous solution (200 mL) was added, and the mixture was extracted with ethyl acetate (100 mL×3). The organic phases were combined, washed with a saturated sodium chloride solution (50 mL). The organic phase was separated, dried over anhydrous sodium sulphate, filtered and then concentrated under reduced pressure to remove the organic solvent, and the residue was separated by column chromatography to obtain the title compound (4 g, 35%).

¹H NMR (400 MHz, CDCl₃) δ 7.48 (d, *J =* 2.0 Hz, 1H), 6.83 (d, *J =* 2.0 Hz, 1H), 3.89 (s, 3H). MS m/z (ESI): 160.1 [M+H]⁺.

### Step 9: synthesis of 1-(ethyl-d₅)-1H-pyrazole-5-carboxylic acid

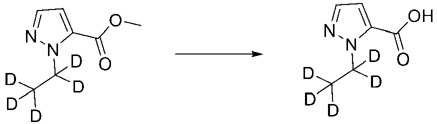

Methyl 1-(ethyl-*d*₅)-1H-pyrazole-5-carboxylate (4 g, 25.16 mmol) was dissolved in a mixed solution (44 mL) of methanol and water (V/V = 8: 3). Lithium hydroxide (2 g, 83.33 mmol) was added, and the mixture was stirred at room temperature for 3 hours. The reaction solution was concentrated under reduced pressure to remove methanol, adjusted to pH 5-6 by dropwise adding 2 M hydrochloric acid and filtered to obtain the title compound (2.9 g, 81 %). MS m/z (ESI): 146.1 [M+H]⁺.

### Step 10: preparation of N-((S)-2-((5-((R)-1-(5-chloro-2-carbonylpyridin-1(2H)-yl)-2-morpholinoethyl)thiazol-2-yl)amino)-1-((1r,4S)-4-methylcyclohexyl)-2-carbonylethyl)-1-(ethyl-d₅)-1H-pyrazole-5-carboxamide

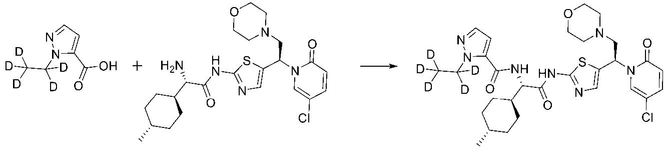

At room temperature, 1-(ethyl-*d*₅)-1H-pyrazole-5-carboxylic acid (72.5 mg, 0.50 mmol) and (S)-2-amino-N-(5-((R)-1-(5-chloro-2-carbonylpyridin-1(2H)-yl)-2-morpholinoethyl)thiazol-2-yl)-2-((1r,4S)-4-methylcyclohexyl)acetamide (123 mg, 0.25 mmol) were dissolved in DMF (5 mL). Triethylamine (75.7 mg, 0.75 mmol) and benzotriazol-1-yl-oxy-tripyrrolidinophosphonium hexafluorophosphate (260 mg, 0.50 mmol) were added, and the mixture was stirred at room temperature for 2 hours. Water was added, and the mixture was extracted three times with ethyl acetate. The organic phases were combined and washed with a saturated sodium chloride aqueous solution. The organic phase was dried over anhydrous sodium sulphate, filtered and then concentrated under reduced pressure to remove the organic solvent, and the residue was separated by column chromatography to obtain the title compound (49 mg, 32%).

¹H NMR (400 MHz, DMSO-*d*⁶) *δ* 12.56-12.33 (m, 1H), 8.58 (d, *J =* 7.6 Hz, 1H), 7.97 (s, 1H), 7.75-7.52 (m, 1H), 7.50-7.45 (m, 1H), 7.00 (s, 1H), 6.56-6.42 (m, 1H), 6.41-6.29 (m, 1H), 4.44 (t, *J =* 8.0 Hz, 1H), 4.13-3.91 (m, 1H), 3.61-3.48 (m, 4H), 3.22-3.13 (m, 1H), 2.46-2.29 (m, 4H), 1.95-1.76 (m, 2H), 1.69-1.60 (m, 2H), 1.48-1.39 (m, 1H), 1.33-1.10 (m, 3H), 1.09-0.96 (m, 1H), 0.94-0.84 (m, 2H), 0.86 (t, *J=* 7.4 Hz, 3H); MS m/z (ESI): 621.2 [M+H]⁺.

### Example 577-1 can also be synthesised according to the following steps

### Step 1: preparation of 2-(4-acetylpiperazin-1-yl)-N-methoxy-N-methylacetamide

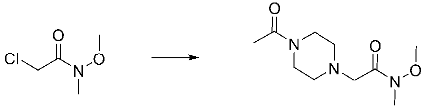

At room temperature, 2-chloro-N-methoxy-N-methylacetamide (5.0 g, 36.3 mmol) and 1-(piperazin-1-yl)ethan-1-one (6.0 g, 47.3 mmol) were dissolved in acetonitrile (50 mL). Potassium carbonate (10.0 g, 72.6 mmol) was added. The mixture was stirred at room temperature for 10 hours, filtered and then concentrated under reduced pressure to remove the organic solvent, and the residue was separated by column chromatography to obtain the title compound (7.5 g, 90%). MS m/z (ES⁺): 230.1 [M+H]⁺.

### Step 2: preparation of tert-butyl(5-(2-(4-acetylpiperazin-1-yl)acetyl)thiazol-2-yl)carbamate

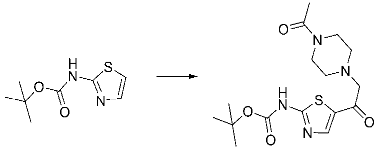

At room temperature, tert-butyl thiazol-2-ylcarbamate (6.6 g, 32.7 mmol) was dissolved in tetrahydrofuran (1100 mL). The mixture was cooled to -78°C, and n-butyllithium (32.8 mL, 81.8 mmol) was added dropwise. After the dropwise addition, the mixture was stirred at -78°C for 1 hour. A solution of 2-(4-acetylpiperazin-1-yl)-N-methoxy-N-methylacetamide (7.5 g, 32.7 mmol) dissolved in tetrahydrofuran (10 mL) was added dropwise. After the dropwise addition, the mixture was stirred at - 78°C for 1 hour. A saturated ammonium chloride solution was added, and the mixture was extracted three times with ethyl acetate. The organic phases were combined and washed with a saturated sodium chloride aqueous solution. The organic phase was dried over anhydrous sodium sulphate, filtered and then concentrated under reduced pressure to remove the organic solvent, and the residue was separated by column chromatography to obtain the title compound (4.2 g, 35%). MS m/z (ES⁺): 369.1 [M+H]⁺.

### Step 3: preparation of tert-butyl(5-(2-(4-acetylpiperazin-1-yl)-1-hydroxyethyl)thiazol-2-yl)carbamate

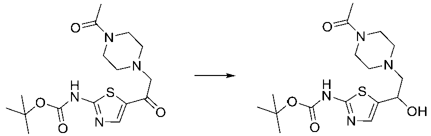

At room temperature, tert-butyl(5-(2-(4-acetylpiperazin-1-yl)acetyl)thiazol-2-yl)carbamate (4.2 g, 11.4 mmol) was dissolved in a tetrahydrofuran/methanol solution (50 mL). The mixture was cooled to 0°C, and sodium borohydride (845 mg, 22.8 mmol) was added in batches. The mixture was stirred at 0°C for 1 hour and concentrated under reduced pressure to remove the organic solvent. Water was added, and the mixture was extracted three times with dichloromethane. The organic phases were combined and washed with a saturated sodium chloride aqueous solution. The organic phase was dried over anhydrous sodium sulphate, filtered and then concentrated under reduced pressure to remove the organic solvent, and the residue was separated by column chromatography to obtain the title compound (3.2 g, 76%). MS m/z (ES⁺): 371.1 [M+H]⁺.

### Step 4: preparation of tert-butyl(5-(2-(4-acetylpiperazin-1-yl)-1-(5-chloro-2-carbonylpyridin-1(2H)-yl)ethyl)thiazol-2-yl)carbamate

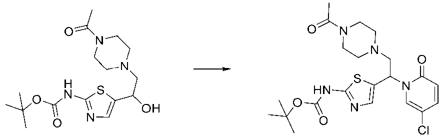

At room temperature, tert-butyl(5-(2-(4-acetylpiperazin-1-yl)-1-hydroxyethyl)thiazol-2-yl)carbamate (1.6 g, 4.3 mmol) and 5-chloropyridin-2(1H)-one (280.0 mg, 2.2 mmol) were dissolved in a toluene solution (20 mL). Methyl trifluoromethanesulfonate (180.4 mg, 1.1 mmol) was added dropwise. The mixture was heated to 110°C, stirred for 5 hours and concentrated under reduced pressure to remove the organic solvent, and the residue was separated by column chromatography to obtain the title compound (323 mg, 31%). MS m/z (ES⁺): 482.1 [M+H]⁺.

### Step 5: preparation of 1-(2-(4-acetylpiperazin-1-yl)-1-(2-aminothiazol-5-yl)ethyl)-5-chloropyridin-2(1H)-one

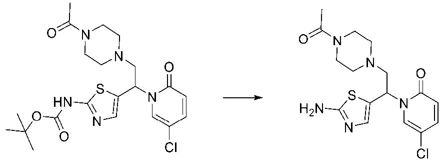

To a solution of tert-butyl(5-(2-(4-acetylpiperazin-1-yl)-1-(5-chloro-2-carbonylpyridin-1(2H)-yl)ethyl)thiazol-2-yl)carbamate (323 mg, 0.7 mmol) in dichloromethane (4 mL) was added hydrogen chloride in dioxane (4 mL). The mixture was stirred overnight at room temperature and concentrated under reduced pressure to remove the organic solvent, so as to obtain the title compound (crude hydrochloride, 255 mg), which was directly used in the next step. MS m/z (ESI): 382.1 [M+H]⁺.

### Step 6: preparation of tert-butyl((1S)-2-((5-(2-(4-acetylpiperazin-1-yl)-1-(5-chloro-2-carbonylpyridin-1(2H)-yl)ethyl)thiazol-2-yl)amino)-1-((1r,4S)-4-methylcyclohexyl)-2-carbonylethyl)carbamate

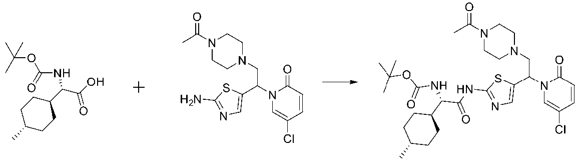

At room temperature, (S)-2-((tert-butoxycarbonyl)amino)-2-((1r,4S)-4-methylcyclohexyl)acetic acid (364 mg, 1.34 mmol) and 1-(2-(4-acetylpiperazin-1-yl)-1-(2-aminothiazol-5-yl)ethyl)-5-chloropyridin-2(1H)-one (255 mg, 0.67 mmol) were dissolved in N,N-dimethylformamide (6 mL). Triethylamine (202 mg, 2.0 mmol) and benzotriazol-1-yl-oxy-tripyrrolidinophosphonium hexafluorophosphate (697 mg, 1.34 mmol) were added, and the mixture was stirred at room temperature for 2 hours. Water was added, and the mixture was extracted three times with ethyl acetate. The organic phases were combined and washed with a saturated sodium chloride aqueous solution. The organic phase was dried over anhydrous sodium sulphate, filtered and then concentrated under reduced pressure to remove the organic solvent, and the residue was separated by column chromatography to obtain the title compound (304 mg, 72%). MS m/z (ESI): 635.1 [M+H]⁺.

### Step 7: preparation of (2S)-N-(5-(2-(4-acetylpiperazin-1-yl)-1-(5-chloro-2-carbonylpyridin-1(2H)-yl)ethyl)thiazol-2-yl)-2-amino-2-((1r,4S)-4-methylcyclohexyl)acetamide

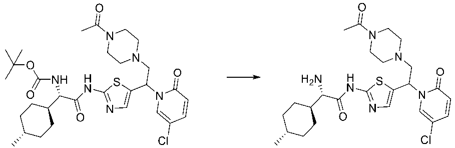

To a solution of tert-butyl((1S)-2-((5-(2-(4-acetylpiperazin-1-yl)-1-(5-chloro-2-carbonylpyridin-1(2H)-yl)ethyl)thiazol-2-yl)amino)-1-((1r,4S)-4-methylcyclohexyl)-2-carbonylethyl)carbamate (304 mg, 0.48 mmol) in dichloromethane (3 mL) was added hydrogen chloride in dioxane (3 mL), and the mixture was stirred at room temperature for 1 hour and concentrated under reduced pressure to remove the organic solvent, so as to obtain the title compound (crude hydrochloride, 256 mg), which was directly used in the next step. MS m/z (ESI): 535.1 [M+H]⁺.

### Step 8: preparation of N-((1S)-2-((5-(2-(4-acetylpiperazin-1-yl)-1-(5-chloro-2-carbonylpyridin-1(2H)-yl)ethyl)thiazol-2-yl)amino)-1-((1r,4S)-4-methylcyclohexyl)-2-carbonylethyl)-1-(ethyl-d₅)-1H-pyrazole-5-carboxamide

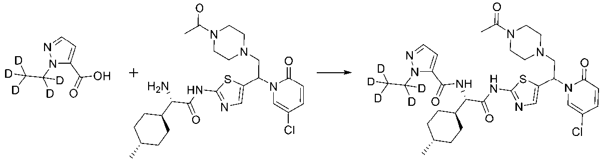

At room temperature, 1-(ethyl-*d*₅)-1H-pyrazole-5-carboxylic acid (139.2 mg, 0.96 mmol) and (2S)-N-(5-(2-(4-acetylpiperazin-1-yl)-1-(5-chloro-2-carbonylpyridin-1(2H)-yl)ethyl)thiazol-2-yl)-2-amino-2-((1r,4S)-4-methylcyclohexyl)acetamide (256.0 mg, 0.48 mmol) were dissolved in N,N-dimethylformamide (5 mL). Triethylamine (145.4 mg, 1.44 mmol) and benzotriazol-1-yl-oxy-tripyrrolidinophosphonium hexafluorophosphate (499.2 mg, 0.96 mmol) were added, and the mixture was stirred at room temperature for 2 hours. Water was added, and the mixture was extracted three times with ethyl acetate. The organic phases were combined and washed with a saturated sodium chloride aqueous solution. The organic phase was dried over anhydrous sodium sulphate, filtered and then concentrated under reduced pressure to remove the organic solvent, and the residue was separated by column chromatography to obtain the title compound (58 mg, 18%).

¹H NMR (400 MHz, DMSO-*d*₆) *δ* 12.45-12.12 (m, 1H), 8.50 (d, *J =* 7.6 Hz, 1H), 7.91-7.84 (m, 1H), 7.45-7.36 (m, 3H), 6.96-6.90 (m, 1H), 6.40 (d, *J* = 9.4 Hz, 1H), 4.37 (t, *J =* 8.0 Hz, 1H), 2.99-2.87 (m, 4H), 2.40-2.25 (m, 2H), 1.96-1.91 (m, 1H), 1.89 (s, 3H), 1.80-1.71 (m, 2H), 1.69-1.64 (m, 2H), 1.63-1.54 (m, 2H), 1.41-1.34 (m, 1H), 1.25-1.14 (m, 4H), 1.12-1.05 (m, 1H), 0.99-0.91 (m, 1H), 0.87-0.79 (m, 1H), 0.78 (d, *J =* 6.6 Hz, 3H).MS m/z (ESI): 662.3 [M+H]⁺.

### Example 586 can also be synthesised according to the following steps

### Step 1: synthesis of tert-butyl(5-(2-methoxyacetyl)thiazol-2-yl)carbamate

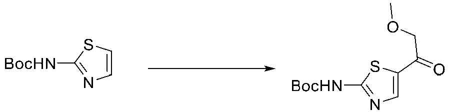

Tert-butyl thiazol-2-ylcarbamate (20 g, 100 mmol) was dissolved in tetrahydrofuran (300 mL). At -78°C, n-butyllithium (100 mL, 2.5 M, 250 mmol) was added, and the mixture was stirred for 1 hour. N,2-dimethoxy-N-methylacetamide (33 g, 250 mmol) was added at -78°C, and the mixture was stirred at -78°C for 2 hours. The mixture was quenched with a saturated ammonium chloride solution (100 mL) at -78°C, and then water (200 mL) was added, which resulted in the precipitation of some products, which were collected by filtration. The filtrate was extracted with ethyl acetate (200 mL×3). The organic phases were combined, washed with a saturated sodium chloride solution (300 mL), dried over anhydrous sodium sulphate, filtered, concentrated under reduced pressure and then slurried with ethyl acetate (150 mL) to obtain the title compound (16 g, 59%). MS m/z (ESI):273.1 [M+H]⁺.

### Step 2: synthesis of tert-butyl(5-(1-hydroxyl-2-methoxyethyl)thiazol-2-yl)carbamate

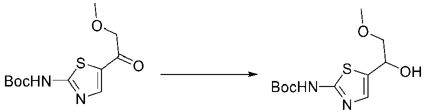

Tert-butyl(5-(2-methoxyacetyl)thiazol-2-yl)carbamate (16 g, 93 mmol) was dissolved in a mixed solution (120 mL) of tetrahydrofuran and methanol (V/V = 1: 1). Sodium borohydride (7 g, 185 mmol) was added in batches at 0°C, and the mixture was stirred at room temperature for 2 hours and concentrated under reduced pressure to remove most of the methanol. Water (50 mL) was added, and the mixture was extracted with dichloromethane (200 mL×3). The organic phases were combined and washed with a saturated sodium chloride solution (200 mL). The organic phase was dried over anhydrous sodium sulphate, filtered and then concentrated under reduced pressure to remove the organic solvent, and the residue was separated by column chromatography to obtain the title compound (16 g, 99%). MS m/z (ESI):275.1 [M+H]⁺.

### Step 3: synthesis of tert-butyl(5-(1-(5-chloro-2-carbonylpyridin-1(2H)-yl)-2-methoxyethyl)thiazol-2-yl)carbamate

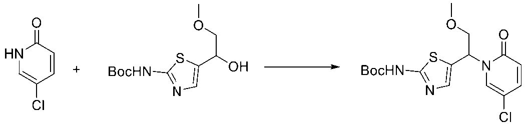

5-chloropyridin-2(1H)-one (4 g, 30.88 mmol) and tert-butyl(5-(1-hydroxyl-2-methoxyethyl)thiazol-2-yl)carbamate (17 g, 62.04 mmol) were dissolved in toluene (140 mL). Methyl trifluoromethanesulfonate (1 g, 6.10 mmol) was added, and the mixture was stirred at 100°C for 12 hours and concentrated under reduced pressure. The solid was dissolved in dichloromethane and methanol. The mixture was further concentrated under reduced pressure, and the residue was separated by silica gel column chromatography to obtain the title compound (9 g, 76%). MS m/z (ESI):386.1 [M+H]⁺.

### Step 4: synthesis of (R)-1-(1-(2-aminothiazol-5-yl)-2-methoxyethyl)-5-chloropyridin-2(1H)-one

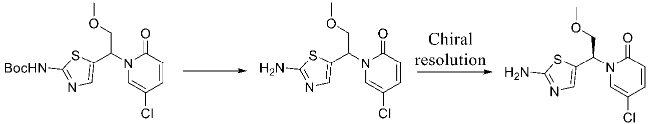

Tert-butyl(5-(1-(5-chloro-2-carbonylpyridin-1(2H)-yl)-2-methoxyethyl)thiazol-2-yl)carbamate (9 g, 23.38 mmol) was dissolved in 4 M hydrogen chloride in dioxane (100 mL). At 45°C, the mixture was stirred for 12 hours. The reaction was cooled to room temperature and concentrated under reduced pressure to obtain the 1-(1-(2-aminothiazol-5-yl)-2-methoxyethyl)-5-chloropyridin-2(1H)-one (crude hydrochloride). A saturated sodium bicarbonate solution (100 mL) was added, and the mixture was extracted with ethyl acetate (10 mL×3). The organic phases were combined, washed with a saturated sodium chloride solution (200 mL), dried over anhydrous sodium sulphate, filtered and concentrated under reduced pressure. The residue was subjected to chiral resolution to obtain the title compound (3 g, 45%). MS m/z (ESI): 286.1[M+H]⁺.

### Step 5: synthesis of tert-butyl((S)-2-((5-((R)-1-(5-chloro-2-carbonylpyridin-1(2H)-yl)-2-methoxyethyl)thiazol-2-yl)amino)-1-((1r,4S)-4-methylcyclohexyl)-2-carbonylethyl)carbamate

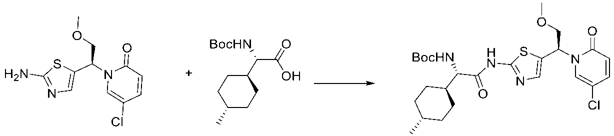

(S)-2-((tert-butoxycarbonyl)amino)-2-((1r,4S)-4-methylcyclohexyl)acetic acid (1.9 g, 7 mmol), HATU (4 g, 10.53 mmol) and DIPEA (2.3 g, 17.83 mmol) were dissolved in N,N-dimethylacetamide (25 mL). The mixture was stirred at room temperature for 10 minutes. (R)-1-(1-(2-aminothiazol-5-yl)-2-methoxyethyl)-5-chloropyridin-2(1H)-one (1 g, 3.5 mmol) was added, and the mixture was reacted at room temperature for 12 hours. An aqueous solution (40 mL) was added, and the mixture was extracted with ethyl acetate (50 mL×3). The organic phases were combined, washed with a saturated sodium chloride solution (50 mL), dried over anhydrous sodium sulphate, filtered and concentrated under reduced pressure, and the residue was separated by silica gel column chromatography to obtain the title compound (1.5 g, 80%). MS m/z (ESI): 539.2 [M+H]⁺.

### Step 6: synthesis of (S)-2-amino-N-(5-((R)-1-(5-chloro-2-carbonylpyridin-1(2H)-yl)-2-methoxyethyl)thiazol-2-yl)-2-((1r,4S)-4-methylcyclohexyl)acetamide

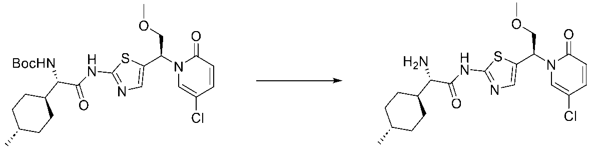

Tert-butyl((S)-2-((5-((R)-1-(5-chloro-2-carbonylpyridin-1(2H)-yl)-2-methoxyethyl)thiazol-2-yl)amino)-1-((1r,4S)-4-methylcyclohexyl)-2-carbonylethyl)carbamate (1.5 g, 2.78 mmol) was dissolved in 4 M hydrogen chloride in dioxane (50 mL). The mixture was stirred at 25°C for 2 hours. The reaction solution was concentrated under reduced pressure to obtain the crude hydrochloride, and a saturated sodium bicarbonate solution (40 mL) was added. The mixture was extracted with ethyl acetate (50 mL×3). The organic phases were combined, washed with a saturated sodium chloride solution (60 mL), dried over anhydrous sodium sulphate, filtered and concentrated under reduced pressure, and the residue was separated by silica gel column chromatography to obtain the title compound (1 g, 82%). MS m/z (ESI): 539.2 [M+H]⁺

### Step 7: synthesis of methyl 1-(ethyl-d₅)-1H-pyrazole-5-carboxylate

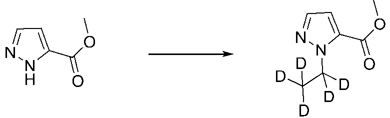

Methyl 1H-pyrazole-5-carboxylate (9 g, 71.43 mmol) and 1-bromoethane-1,1,2,2,2-*d*₅ (9.8 g, 86 mmol) were dissolved in acetonitrile (200 mL). Caesium carbonate (35 g, 107.36 mmol) was added, and the mixture was reacted at room temperature for 2 hours. An aqueous solution (200 mL) was added, and the mixture was extracted with ethyl acetate (100 mL×3). The organic phases were combined, washed with a saturated sodium chloride solution (50 mL). The organic phase was separated, dried over anhydrous sodium sulphate, filtered and then concentrated under reduced pressure to remove the organic solvent, and the residue was separated by column chromatography to obtain the title compound (4 g, 35%).

¹H NMR (400 MHz, CDCl₃) δ 7.48 (d, *J =* 2.0 Hz, 1H), 6.83 (d, *J =* 2.0 Hz, 1H), 3.89 (s, 3H). MS m/z (ESI): 160.1 [M+H]⁺.

### Step 8: synthesis of 1-(ethyl-d₅)-1H-pyrazole-5-carboxylic acid

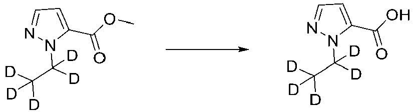

Methyl 1-(ethyl-*d*₅)-1H-pyrazole-5-carboxylate (4 g, 25.16 mmol) was dissolved in a mixed solution (44 mL) of methanol and water (V/V = 8: 3). Lithium hydroxide (2 g, 83.33 mmol) was added, and the mixture was stirred at room temperature for 3 hours. The reaction solution was concentrated under reduced pressure to remove methanol, adjusted to pH 5-6 by dropwise adding 2 M hydrochloric acid and filtered to obtain the title compound (2.9 g, 81 %). MS m/z (ESI): 146.1 [M+H]⁺.

### Step 9: synthesis of N-((S)-2-((5-((R)-1-(5-chloro-2-carbonylpyridin-1(2H)-yl)-2-methoxyethyl)thiazol-2-yl)amino)-1-((1r,4S)-4-methylcyclohexyl)-2-carbonylethyl)-1-(ethyl-d₅)-1H-pyrazole-5-carboxamide

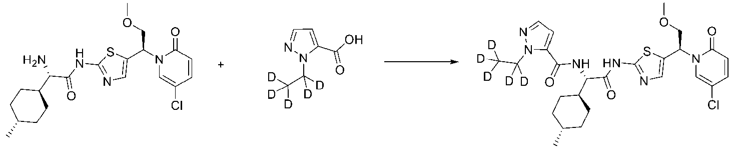

1-(ethyl-*d*₅)-1H-pyrazole-5-carboxylic acid (66 mg, 0.45 mmol), HATU (260 mg, 0.68 mmol) and DIPEA (147 mg, 1.14 mmol) were dissolved in N,N-dimethylacetamide (5 mL). The mixture was stirred at room temperature for 5 minutes. (S)-2-amino-N-(5-((R)-1-(5-chloro-2-carbonylpyridin-1(2H)-yl)-2-methoxyethyl)thiazol-2-yl)-2-((1r,4S)-4-methylcyclohexyl)acetamide (100 mg, 0.23 mmol) was added, and the mixture was reacted at room temperature for 1 hour. An aqueous solution (25 mL) was added, and the mixture was extracted with ethyl acetate (10 mL×3). The organic phases were combined, washed with a saturated sodium chloride solution (50 mL), dried over anhydrous sodium sulphate, filtered and concentrated under reduced pressure, and the residue was separated by preparative liquid chromatography to obtain the title compound (87 mg, 67%).

¹H NMR (400 MHz, DMSO-*d*₆) δ 12.39 (s, 1H), 8.57 (d, *J =* 7.5 Hz, 1H), 7.94 (d, *J =* 2.9 Hz, 1H), 7.55 (s, 1H), 7.52-7.40 (m, 2H), 7.00 (d, *J =* 2.1 Hz, 1H), 6.47 (d, *J* = 9.7 Hz, 1H), 6.28-6.25 (m, 1H), 4.43 (t, *J =* 8.1 Hz, 1H), 4.25-4.21 (m 1H), 3.91-3.82 (m, 1H), 3.30 (s, 3H), 1.85-1.76(m, 2H), 1.63-1.70 (m, 2H), 1.47-1.41(m, 1H), 1.32-1.10 (m, 2H), 1.08-0.99 (m, 1H), 0.91-0.75 (m, 5H); MS m/z (ESI) :566.2 [M+H]⁺.

### Example 588 can also be synthesised according to the following steps

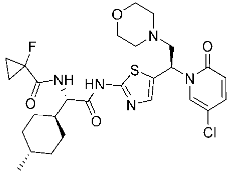

At room temperature, 1-fluorocyclopropane-1-carboxylic acid (52.0 mg, 0.50 mmol) and (S)-2-amino-N-(5-((R)-1-(5-chloro-2-carbonylpyridin-1(2H)-yl)-2-morpholinoethyl)thiazol-2-yl)-2-((1r,4S)-4-methylcyclohexyl)acetamide (123.0 mg, 0.25 mmol) were dissolved in N,N-dimethylformamide (5 mL). Triethylamine (75.7 mg, 0.75 mmol) and benzotriazol-1-yl-oxy-tripyrrolidinophosphonium hexafluorophosphate (260.0 mg, 0.50 mmol) were added, and the mixture was stirred at room temperature for 2 hours. Water was added, and the mixture was extracted three times with ethyl acetate. The organic phases were combined and washed with a saturated sodium chloride aqueous solution. The organic phase was dried over anhydrous sodium sulphate, filtered and then concentrated under reduced pressure to remove the organic solvent, and the residue was separated by column chromatography to obtain the title compound (31.4 mg, 22%).

¹H NMR (400 MHz, DMSO-*d⁶*) *δ* 12.35-12.24 (m, 1H), 8.27 (d, *J =* 7.8 Hz, 1H), 7.90 (s, 1H), 7.59-7.48 (m, 1H), 7.44 (d, *J =* 9.6 Hz, 1H), 6.44 (d, *J =* 9.8 Hz, 1H), 4.29 (t, *J =* 8.2 Hz, 1H), 4.01-3.85 (m, 1H),3.67-3.43 (m, 4H), 2.53-2.44 (m, 2H), 1.98-1.87 (m, 1H), 1.78-1.69 (m, 2H), 1.64-1.53 (m, 3H), 1.37-1.30 (m, 1H), 1.29-1.15 (m, 5H), 1.14-1.01 (m, 3H), 0.94-0.87 (m, 1H), 0.86-0.79 (m, 2H), 0.77 (d, *J =* 6.2 Hz, 3H); MS m/z (ESI): 580.1 [M+H]⁺.

### Example 592 can also be synthesised according to the following steps

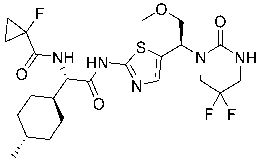

At room temperature, 1-fluorocyclopropane-1-carboxylic acid (37.5 mg, 0.36 mmol) was dissolved in DMF (2 mL). HATU (137 mg, 0.36 mmol) and DIPEA (146 mg, 1.13 mmol) were sequentially added, and the mixture was stirred at room temperature for 5 minutes. (2S)-2-amino-N-(5-(1-(5,5-difluoro-2-carbonyltetrahydropyrimidin-1(2H)-yl)-2-methoxyethyl)thiazol-2-yl)-2-((1r,4S)-4-methylcyclohexyl)acetamide hydrochloride (101 mg, 0.21 mmol) was added, and the mixture was further stirred for 1 hour. The reaction solution was diluted with ethyl acetate and washed with a saturated sodium chloride aqueous solution. The organic phase was dried over anhydrous sodium sulphate, filtered and concentrated under reduced pressure, and the residue was subjected to column chromatography and chiral resolution to obtain the title compound (33 mg, 30%).

¹H NMR (400 MHz, DMSO-*d₆*) δ 12.22 (s, 1H), 8.32 (m, 1H), 7.37 (s, 1H), 6.89 (s, 1H), 5.68 (t, *J =* 6.8 Hz, 1H), 4.37 (d, *J =* 8.6 Hz, 1H), 3.85 -3.70 (m, 2H), 3.64-3.35 (m, 4H), 3.31 (s, 3H), 1.83-1.73 (m, 2H), 1.70-1.62 (m, 2H), 1.46-1.38 (m, 1H), 1.35-1.11 (m, 7H), 1.03-0.85 (m, 2H), 0.83-0.79 (s, 3H) ; MS m/z (ESI): 532.2 [M+H]⁺.

### Example 601 can also be synthesised according to the following steps

### Step 1: preparation of tert-butyl N-[5-[1-[(3-amino-2,2-difluoro-propyl)amino]-2-morpholino-ethyl]thiazol-2-yl]carbamate

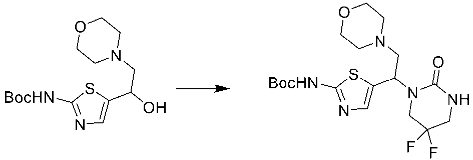

To a solution of tert-butyl N-[5-(1-hydroxyl-2-morpholino-ethyl)thiazol-2-yl]carbamate (2 g, 6.07 mmol) in dichloromethane (20 mL) were added methanesulfonic anhydride (1.59 g, 9.11 mmol), DIPEA (2.35 g, 18.21 mmol, 3.17 mL) and 4-dimethylaminopyridine (148.34 mg, 1.21 mmol). The mixture was stirred at room temperature for 12 h and washed with a saturated sodium chloride solution. The aqueous phase was extracted three times with dichloromethane. The organic phases were combined, dried over anhydrous sodium sulphate and concentrated under reduced pressure, and the residue was dissolved in acetonitrile (20 mL). 2,2-difluoropropane-1,3-diamine hydrochloride (668 mg, 3.65 mmol, 2CL) and DIPEA (2.35 g, 18.21 mmol, 3.17 mL) were sequentially added, and the mixture was heated to 60°C, stirred for 12 h and concentrated under reduced pressure to remove the solvent. The residue was dispersed in ethyl acetate, washed with a saturated sodium chloride solution, dried over anhydrous sodium sulphate, filtered, concentrated under reduced pressure and then separated by column chromatography to obtain the title compound (1.1 g, 43%). MS m/z (ESI):422.2 [M+H]⁺.

### Step 2: preparation of tert-butyl N-[5-[1-(5,5-difluoro-2-carbonyl-hexahydropyrimidin-1-yl)-2-morpholino-ethyl]thiazol-2-yl]carbamate

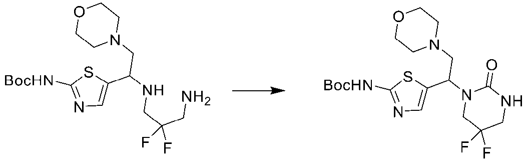

To a solution of tert-butyl N-[5-[1-[(3-amino-2,2-difluoro-propyl)amino]-2-morpholino-ethyl]thiazol-2-yl]carbamate (1.1 g, 2.61 mmol) in tetrahydrofuran (50 mL) was added CDI (1.06 g, 6.52 mmol), and the mixture was stirred at room temperature for 2 h. The reaction was quenched with a 5 M sodium hydroxide aqueous solution (10 mL), washed with a saturated sodium chloride solution and extracted three times with ethyl acetate. The organic phases were combined, dried over anhydrous sodium sulphate, filtered and concentrated under reduced pressure, and the residue was separated by column chromatography to obtain the title compound (400 mg, 34%). MS m/z (ESI):448.2 [M+H]⁺.

### Step 3: preparation of tert-butyl N-[(1S)-2-[[5-[1-(5,5-difluoro-2-carbonyl-hexahydropyrimidin-1-yl)-2-morpholino-ethyl]thiazol-2-yl]amino]-1-(4-methylcyclohexyl)-2-carbonyl-ethyl]carbamate

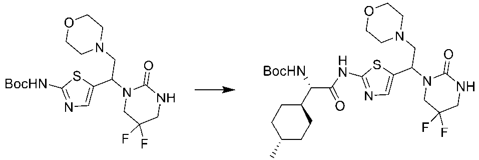

To a solution of tert-butyl N-[5-[1-(5,5-difluoro-2-carbonyl-hexahydropyrimidin-1-yl)-2-morpholino-ethyl]thiazol-2-yl]carbamate (400 mg, 893.86 µmol) in methanol (2 mL) was added 4 M hydrogen chloride dioxane (10 mL), and the mixture was stirred at room temperature for 8 h and concentrated under reduced pressure to remove the solvent. To the residue were added tert-butyl N-[(1S)-2-amino-1-(4-methylcyclohexyl)-2-carbonyl-ethyl]carbamate (241.67 mg, 893.86 µmol), DIPEA (115.52 mg, 893.86 µmol, 155.69 µL), DMF (10 mL) and HATU (337.22 mg, 893.86 µmol), and the mixture was stirred at room temperature for 12 h. An aqueous ammonium chloride solution (25 mL) was added, and the mixture was washed with a saturated sodium chloride solution and extracted three times with ethyl acetate. The organic phases were combined, dried over anhydrous sodium sulphate, filtered and concentrated under reduced pressure, and the residue was separated by column chromatography to obtain the title compound (350 mg, 65%). MS m/z (ESI):601.3[M+H]⁺.

### Step 4: preparation of N-[(1S)-2-[[5-[1-(5,5-difluoro-2-carbonyl-hexahydropyrimidin-1-yl)-2-morpholino-ethyl]thiazol-2-yl]amino]-1-(4-methylcyclohexyl)-2-carbonyl-ethyl]-1-fluoro-cyclopropanecarboxamide

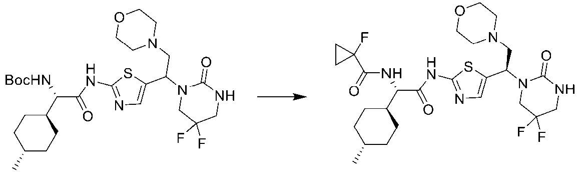

To a solution of tert-butyl N-[(1S)-2-[[5-[1-(5,5-difluoro-2-carbonyl-hexahydropyrimidin-1-yl)-2-morpholino-ethyl]thiazol-2-yl]amino]-1-(4-methylcyclohexyl)-2-carbonyl-ethyl]carbamate (350 mg, 582.63 µmol) in methanol (2 mL) was added 4 M hydrogen chloride dioxane (10 mL), and the mixture was stirred at room temperature for 1 h and concentrated under reduced pressure to remove the solvent. The residue was dissolved in DMF (10 mL). 1-Fluoro-cyclopropylcarboxylic acid (91 mg, 874 µmol), DIPEA (377 mg, 2.9 mmol, 508 µL) and HATU (330 mg, 874 µmol) were sequentially added, and the mixture was stirred at room temperature for 2 h. An aqueous ammonium chloride solution (25 mL) was added, and the mixture was washed with a saturated sodium chloride solution and extracted three times with ethyl acetate. The organic phases were combined, dried over anhydrous sodium sulphate, filtered and concentrated under reduced pressure, and the residue was subjected to chiral resolution to obtain the title compound (100 mg, 29%).

¹H NMR (400 MHz, DMSO-*d₆)* δ 12.17 (s, 1H), 8.33 (d, *J =* 8.1 Hz, 1H), 7.35 (s, 1H), 6.87 (s, 1H), 5.71 (t, *J =* 7.8 Hz, 1H), 4.39 (t, *J =* 8.3 Hz, 1H), 3.66-3.43 (m, 7H), 2.83 (dd, *J =* 12.6, 9.1 Hz, 1H), 2.73 (dd, *J =* 12.7, 7.1 Hz, 1H), 2.47-2.38 (m,3H), 1.79 (d, *J =* 9.6 Hz, 2H), 1.67 (t, *J =* 9.0 Hz, 2H), 1.50-1.07 (m, 8H), 1.03-0.76 (m, 7H); MS m/z (ESI):587.2 [M+H]⁺.

### Example 613 can also be synthesised according to the following steps

### Step 1: preparation of tert-butyl(5-(1-hydroxyl-2-methoxyethyl)thiazol-2-yl)carbamate

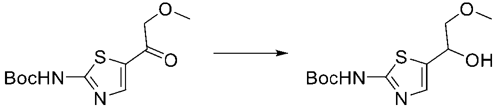

At room temperature, tert-butyl(5-(2-methoxyacetyl)thiazol-2-yl)carbamate (2 g, 7.34 mmol) was dissolved in methanol (35 mL). Sodium borohydride (278 mg, 7.34 mmol) was added, and the mixture was stirred at room temperature for 1 hour and concentrated under reduced pressure. The residue was subjected to column chromatography to obtain the title compound (1.81 g, 90%). MS m/z (ESI): 275.1 [M+H]^{+.}

### Step 2: preparation of 1-(2-((tert-butoxycarbonyl)amino)thiazol-5-yl)-2-methoxyethyl 4-methylbenzenesulfonate

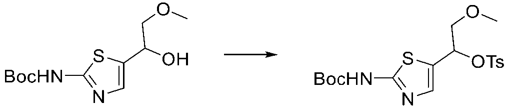

Under cooling at 0°C, tert-butyl(5-(1-hydroxyl-2-methoxyethyl)thiazol-2-yl)carbamate (650 mg, 2.37 mmol) was dissolved in dichloromethane (15 mL), and p-toluenesulfonyl chloride (903 mg, 4.74 mmol), 4-dimethylaminopyridine (27 mg, 218.71 µmol) and DIPEA (612 mg, 1.46 mmol) were added. The mixture was warmed to room temperature and stirred for 6 hours. The reaction solution was diluted with dichloromethane and washed with a saturated sodium chloride aqueous solution. The organic phase was dried over anhydrous sodium sulphate, filtered and then concentrated under reduced pressure to remove the organic solvent, and the residue was separated by column chromatography to obtain the title compound (960 mg), which was directly used in the next step. MS m/z (ESI): 429.1 [M+H]^{+.}

### Step 3: preparation of tert-butyl(5-(1-(5,5-difluoro-2-carbonylpiperidin-1-yl)-2-methoxyethyl)thiazol-2-yl)carbamate

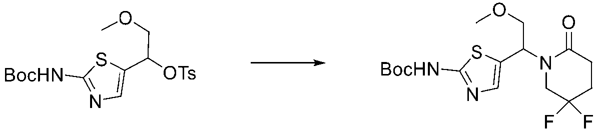

At room temperature, 5,5-difluoropiperidin-2-one (378 mg, 2.8 mmol) was dissolved in tetrahydrofuran (10 mL). Sodium hydride (224 mg, 5.6 mmol, 60 wt%) was added, and the mixture was stirred at room temperature for 1 hour. 1-(2-((tert-butoxycarbonyl)amino)thiazol-5-yl)-2-methoxyethyl 4-methylbenzenesulfonate (600 mg, 1.4 mmol) was added, and the mixture was heated to 60°C and stirred for 12 hours. The reaction was cooled to room temperature and quenched with a saturated ammonium chloride solution. The reaction solution was diluted with ethyl acetate and washed with a saturated sodium chloride solution, and the phases were separated. The organic phase was dried over anhydrous sodium sulphate, filtered and then concentrated under reduced pressure to remove the organic solvent, and the residue was subjected to column chromatography to obtain the title compound (190 mg, 34.7%). MS m/z (ESI): 392.1 [M+H]^{+.}

### Step 4: preparation of 1-(1-(2-aminothiazol-5-yl)-2-methoxyethyl)-5,5-difluoropiperidin-2-one hydrochloride

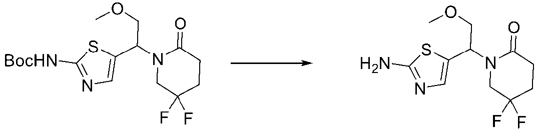

Tert-butyl(5-(1-(5,5-difluoro-2-carbonylpiperidin-1-yl)-2-methoxyethyl)thiazol-2-yl)carbamate (0.12 g, 0.306 mmol) was dissolved in dichloromethane (1 mL). 4 M hydrogen chloride in dioxane (3 mL) was added, and the mixture was stirred at room temperature for 8 hours and concentrated under reduced pressure to obtain the title compound (hydrochloride, 99 mg), which was directly used in the next step. MS m/z (ESI): 292.1 [M+H]⁺.

### Step 5: preparation of tert-butyl((1S)-2-((5-(1-(5,5-difluoro-2-carbonylpiperidin-1-yl)-2-methoxyethyl)thiazol-2-yl)amino)-1-((1r,4S)-4-methylcyclohexyl)-2-carbonylethyl)carbamate

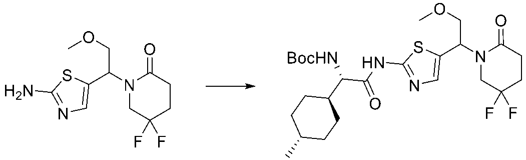

At room temperature, (S)-2-((tert-butoxycarbonyl)amino)-2-((1r,4S)-4-methylcyclohexyl)acetic acid (115 mg, 0.42 mmol) was dissolved in DMF (3 mL). HATU (159.89 mg, 0.42 mmol) and DIPEA (274 mg, 2.12 mmol) were sequentially added, and the mixture was stirred at room temperature for 10 minutes. 1-(1-(2-aminothiazol-5-yl)-2-methoxyethyl)-5,5-difluoropiperidin-2-one hydrochloride (99 mg, 0.30 mmol) was added, and the mixture was further stirred for 1 hour. The reaction solution was diluted with ethyl acetate and washed with a saturated sodium chloride aqueous solution. The organic phase was dried over anhydrous sodium sulphate, filtered and concentrated under reduced pressure, and the residue was subjected to normal-phase column chromatography to obtain the title compound tert-butyl((1S)-2-((5-(1-(5,5-difluoro-2-carbonylpiperidin-1-yl)-2-methoxyethyl)thiazol-2-yl)amino)-1-((1r,4S)-4-methylcyclohexyl)-2-carbonylethyl)carbamate (120 mg, 73%). MS m/z (ESI): 545.2 [M+H]⁺.

### Step 6: preparation of (2S)-2-amino-N-(5-(1-(5,5-difluoro-2-carbonylpiperidin-1-yl)-2-methoxyethyl)thiazol-2-yl)-2-((1r,4S)-4-methylcyclohexyl)acetamide hydrochloride

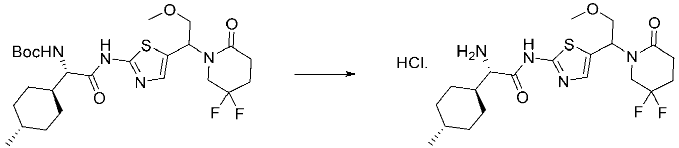

Tert-butyl((1S)-2-((5-(1-(5,5-difluoro-2-carbonylpiperidin-1-yl)-2-methoxyethyl)thiazol-2-yl)amino)-1-((1r,4S)-4-methylcyclohexyl)-2-carbonylethyl)carbamate (116 mg, 0.213 mmol) was dissolved in dichloromethane (0.5 mL). 4 M hydrogen chloride in dioxane (3 mL) was added, and the mixture was stirred at room temperature for 1 hour and concentrated under reduced pressure to obtain the title compound (hydrochloride, 101 mg), which was directly used in the next step. MS m/z (ESI): 445.2 [M+H]⁺.

### Step 7: preparation of N-((S)-2-((5-((R)-1-(5,5-difluoro-2-carbonylpiperidin-1-yl)-2-methoxyethyl)thiazol-2-yl)amino)-1-((1r,4S)-4-methylcyclohexyl)-2-carbonylethyl)-1-(ethyl-d5)-1H-pyrazole-5-carboxamide

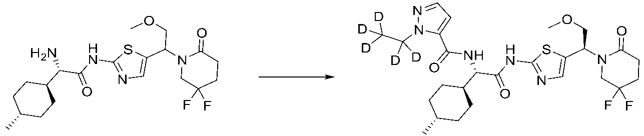

1-(ethyl-d5)-1H-pyrazole-5-carboxylic acid (52 mg, 0.36 mmol) was dissolved in DMF (2 mL). HATU (137 mg, 0.36 mmol) and DIPEA (146 mg, 1.13 mmol) were sequentially added, and the mixture was stirred at room temperature for 5 minutes. (2S)-2-amino-N-(5-(1-(5,5-difluoro-2-carbonylpiperidin-1-yl)-2-methoxyethyl)thiazol-2-yl)-2-((1r,4S)-4-methylcyclohexyl)acetamide hydrochloride (101 mg, 0.21 mmol) was added, and the mixture was further stirred for 1 hour. The reaction solution was diluted with ethyl acetate and washed with a saturated sodium chloride aqueous solution. The organic phase was dried over anhydrous sodium sulphate, filtered and concentrated under reduced pressure, and the residue was sequentially subjected to column chromatography and chiral resolution to obtain the title compound (36 mg, 30%).

¹H NMR (400 MHz, DMSO-*d₆*) *δ* 12.26 (s, 1H), 8.55 (d, *J =* 7.6 Hz, 1H), 7.48-7.46 (m, 1H), 7.41 (s, 1H), 7.01-6.98 (m, 1H), 5.85 (t, *J =* 6.6 Hz, 1H), 4.44 (t, *J =* 8.0 Hz, 1H), 3.87-3.82 (m, 1H), 3.76-3.71 (m, 1H), 3.70-3.60 (m, 1H), 3.54-3.45 (m, 1H), 3.30 (s, 3H), 2.8-2.19 (m, 2H), 1.87-1.74 (m, 2H), 1.71-1.61 (m, 2H), 1.49-1.40 (m, 1H), 1.39 -0.95 (m, 4H), 0.93-0.88 (m, 1H), 0.87-0.84 (m, 3H), 0.83-0.77(m, 2H) ; MS m/z (ESI): 572.2 [M+H]⁺.

### Example 624 can also be synthesised according to the following steps

### Step 1: preparation of 1-(1-(2-aminothiazol-5-yl)-2-hydroxyethyl)-5,5-difluoropiperidin-2-one

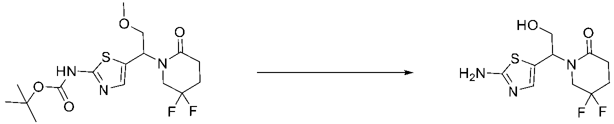

Tert-butyl(5-(1-(5,5-difluoro-2-oxopiperidin-1-yl)-2-methoxyethyl)thiazol-2-yl)carbamate (30.0 g, 76.7 mmol) was dissolved in dichloromethane (1 L). The mixture was cooled to -78°C. Boron tribromide (153.5 mL, 307 mmol, 2 M in dichloromethane) was slowly added dropwise. After the dropwise addition, the mixture was gradually warmed to room temperature and stirred for 2 hours. LCMS indicated the complete consumption of the starting material. The reaction system was cooled to 0°C. The reaction was quenched by slowly dropwise adding methanol (50 mL) and concentrated under reduced pressure to remove the organic solvent, so as to obtain the title compound as a crude, which was directly used in the next reaction. MS m/z (ESI): 278.1 [M+H]⁺.

### Step 2: preparation of tert-butyl(5-(1-(5,5-difluoro-2-oxopiperidin-1-yl)-2-hydroxyethyl)thiazol-2-yl)carbamate

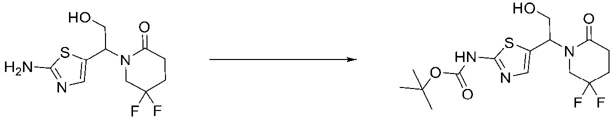

The crude 1-(1-(2-aminothiazol-5-yl)-2-hydroxyethyl)-5,5-difluoropiperidin-2-one was dissolved in methanol (800 mL). N,N-diisopropylethylamine (49.7 g, 383.5 mmol) and di-tert-butyl dicarbonate (100.0 g, 460.2 mmol) were sequentially added, and the mixture was heated to 60°C and stirred for 5 hours. The reaction system was cooled to 0°C, and ammonia water (20 mL) was slowly added dropwise. The mixture was concentrated under reduced pressure to remove the organic solvent, and the crude was dissolved in ethyl acetate. The mixture was washed sequentially with a saturated sodium bicarbonate solution and a saturated sodium chloride solution, dried over anhydrous sodium sulphate, filtered and then concentrated to remove the organic solvent, and the residue was purified by column chromatography to obtain the title compound (23.7 g, overall yield over two steps: 82%). MS m/z (ESI): 378.1 [M+H]⁺.

### Step 3: preparation of tert-butyl(5-(1-(5,5-difluoro-2-oxopiperidin-1-yl)-2-oxoethyl)thiazol-2-yl)carbamate

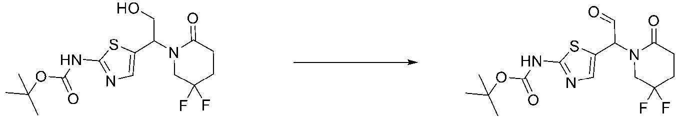

Tert-butyl(5-(1-(5,5-difluoro-2-oxopiperidin-1-yl)-2-hydroxyethyl)thiazol-2-yl)carbamate (6.4 g, 17.0 mmol) was dissolved in a mixed solvent of dichloromethane/dimethyl sulfoxide (120 mL/30 mL), and the mixture was cooled to 0°C. Triethylamine (6.9 g, 68.0 mmol) was added, and pyridine sulphur trioxide (10.8 g, 68.0 mmol) was added in batches. The mixture was stirred at 0°C for 1 hour. The reaction was quenched with water and extracted with ethyl acetate. The organic phase was washed sequentially with a saturated sodium bicarbonate solution and a saturated sodium chloride solution, dried over anhydrous sodium sulphate, filtered and then concentrated to remove the organic solvent, and the crude was purified by column chromatography to obtain the title compound (5.5 g, 86%). MS m/z (ESI): 376.1 [M+H]⁺.

### Step 4: preparation of tert-butyl(5-(1-(5,5-difluoro-2-oxopiperidin-1-yl)-2-morpholinoethyl)thiazol-2-yl)carbamate

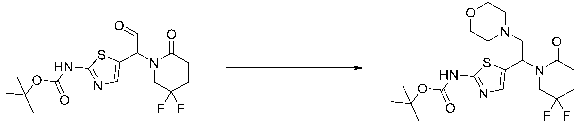

Tert-butyl(5-(1-(5,5-difluoro-2-oxopiperidin-1-yl)-2-oxoethyl)thiazol-2-yl)carbamate (500 mg, 1.3 mmol) was dissolved in 1,2-dichloroethane (20 mL). Morpholine (340 mg, 3.9 mmol) was added, and 2 drops of acetic acid was added dropwise. The mixture was stirred at room temperature for 2 hours, and sodium triacetoxyborohydride (826 mg, 3.9 mmol) was added. The mixture was stirred at room temperature for 16 hours. The reaction was quenched with a saturated sodium bicarbonate solution and extracted with dichloromethane. The organic phase was washed with a saturated sodium chloride solution, dried over anhydrous sodium sulphate, filtered and then concentrated to remove the organic solvent, and the crude was purified by column chromatography to obtain the title compound (492 mg, 85%). MS m/z (ESI): 447.2 [M+H]⁺.

### Step 5: preparation of 1-(1-(2-aminothiazol-5-yl)-2-morpholinoethyl)-5,5-difluoropiperidin-2-one

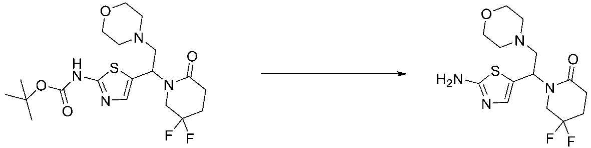

Tert-butyl(5-(1-(5,5-difluoro-2-oxopiperidin-1-yl)-2-morpholinoethyl)thiazole-2-)carbamate (492 mg, 1.1 mmol) was dissolved in dichloromethane (3 mL). 4 M hydrogen chloride in dioxane (6 mL) was added, and the reaction was stirred at 45°C for 8 hours and concentrated under reduced pressure to remove the solvent, so as to obtain the title compound as a crude, which was directly used in the next reaction. MS m/z (ESI): 347.1 [M+H]⁺.

### Step 6: preparation of tert-butyl((1S)-2-((5-(1-(5,5-difluoro-2-oxopiperidin-1-yl)-2-morpholinoethyl)thiazol-2-yl)amino)-1-((1r,4S)-4-methylcyclohexyl)-2-oxoethyl)carbamate

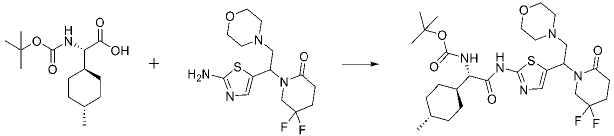

The crude (S)-2-((tert-butoxycarbonyl)amino)-2-((trans)-4-methylcyclohexyl)acetic acid (597 mg, 2.2 mmol) and 1-(1-(2-aminothiazol-5-yl)-2-morpholinoethyl)-5,5-difluoropiperidin-2-one was dissolved in N,N-dimethylformamide (6 mL). Triethylamine (333.3 mg, 3.3 mmol) and benzotriazol-1-yl-oxy-tripyrrolidinophosphonium hexafluorophosphate (1.2 g, 2.2 mmol) were sequentially added. The reaction was stirred at room temperature for 16 hours. The reaction solution was diluted with ethyl acetate and washed with a saturated sodium chloride aqueous solution, and the phases were separated. The organic phase was dried over anhydrous sodium sulphate, filtered and then concentrated to remove the organic solvent, and the resulting residue was separated by column chromatography to obtain the title compound (528 mg, overall yield over two steps: 80%). MS m/z (ESI): 600.3 [M+H]⁺.

### Step 7: preparation of (2S)-2-amino-N-(5-(1-(5,5-difluoro-2-oxopiperidin-1-yl)-2-morpholinoethyl)thiazol-2-yl)-2-((1r,4S)-4-methylcyclohexyl)acetamide

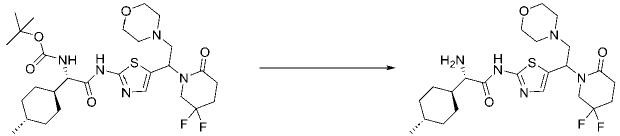

Tert-butyl((1S)-2-((5-(1-(5,5-difluoro-2-oxopiperidin-1-yl)-2-morpholinoethyl)thiazol-2-yl)amino)-1-((1r,4S)-4-methylcyclohexyl)-2-oxoethyl)carbamate (528 mg, 0.88 mmol) was dissolved in dichloromethane (3 mL). 4 M hydrogen chloride in dioxane (3 mL) was added. The reaction solution was stirred and reacted at room temperature for 1 hour and concentrated under reduced pressure to remove the solvent, so as to obtain the title compound as a crude, which was directly used in the next reaction. MS m/z (ESI): 500.1 [M+H]⁺.

### Step 8: preparation of N-((S)-2-((5-((R)-1-(5,5-difluoro-2-oxopiperidin-1-yl)-2-morpholinoethyl)thiazol-2-yl)amino)-1-((1r,4S)-4-methylcyclohexyl)-2-oxoethyl)-1-(ethyl-d5)-1H-pyrazole-5-carboxamide

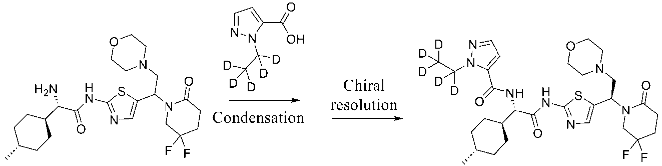

The crude 1-(ethyl-*d*₅)-1*H*-pyrazole-5-carboxylic acid (256 mg, 1.76 mmol) and (2S)-2-amino-N-(5-(1-(5,5-difluoro-2-oxopiperidin-1-yl)-2-morpholinoethyl) thiazol-2-yl)-2-((1r,4S)-4-methylcyclohexyl)acetamide was dissolved in N,N-dimethylformamide (6 mL). Triethylamine (253 mg, 2.5 mmol) and benzotriazol-1-yl-oxy-tripyrrolidinophosphonium hexafluorophosphate (885 mg, 1.7 mmol) were sequentially added. The reaction was stirred at room temperature for 1 hour. The mixture was diluted with ethyl acetate and washed with a saturated sodium chloride solution, and the phases were separated. The organic phase was dried over anhydrous sodium sulphate, filtered and concentrated under reduced pressure to remove the organic solvent. The resulting residue was subjected to column chromatography and chiral resolution to obtain the title compound (107 mg, overall yield over two steps: 19%).

¹H NMR(400 MHz, DMSO-*d₆*) δ 12.27 (s, 1H), 8.57 (d, *J =* 8.0 Hz, 1H), 7.48 (d, *J =* 2.0 Hz, 1H), 7.40 (s, 1H), 7.00 (d, *J =* 2.0 Hz, 1H), 5.89 (t, *J =* 8.0 Hz, 1H), 4.46 (t, *J =* 8.2 Hz, 1H), 3.71-3.38 (m, 5H), 3.29-3.26 (m, 1H), 2.93-2.83(m, 1H), 2.76-2.65(m, 1H), 2.47-2.36 (m, 4H), 2.33-2.23 (m, 3H), 1.89-1.73 (m, 2H), 1.72-1.63 (m, 2H), 1.52 -1.41(m, 1H), 1.39-0.94 (m, 4H), 0.92-0.78 (m, 5H);MS m/z (ESI): 627.3 [M+H]⁺.

### Example 627 can also be synthesised according to the following steps

### Step 1: preparation of tert-butyl(5-(1-(5,5-difluoro-2-oxopiperidin-1-yl)-2-((R)-2-methylmorpholino)ethyl)thiazol-2-yl)carbamate

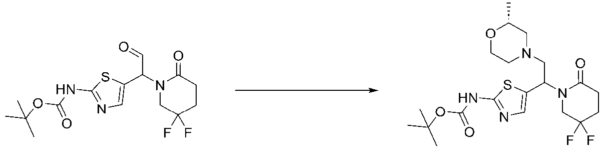

Tert-butyl(5-(1-(5,5-difluoro-2-oxopiperidin-1-yl)-2-oxoethyl)thiazol-2-yl)carbamate (500 mg, 1.3 mmol) was dissolved in 1,2-dichloroethane (20 mL). (R)-2-methylmorpholine (395 mg, 3.9 mmol) was added, and 2 drops of acetic acid was added dropwise. The mixture was stirred at room temperature for 2 hours, and sodium triacetoxyborohydride (826 mg, 3.9 mmol) was added. The mixture was stirred at room temperature for 16 hours. The reaction was quenched with a saturated sodium bicarbonate solution and extracted with dichloromethane. The organic phase was washed with a saturated sodium chloride solution, dried over anhydrous sodium sulphate, filtered and then concentrated to remove the organic solvent, and the crude was purified by column chromatography to obtain the title compound (495 mg, 81%). MS m/z (ESI): 461.2 [M+H]⁺.

### Step 2: preparation of 1-(1-(2-aminothiazol-5-yl)-2-((R)-2-methylmorpholino)ethyl)-5,5-difluoropiperidin-2-one

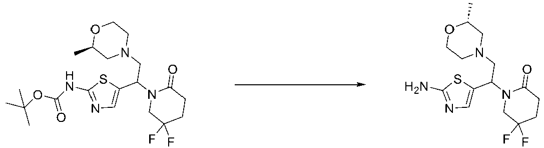

Tert-butyl(5-(1-(5,5-difluoro-2-oxopiperidin-1-yl)-2-((R)-2-methylmorpholino)ethyl)thiazol-2-yl)carbamate (495 mg, 1.1 mmol) was dissolved in dichloromethane (3 mL). 4 M hydrogen chloride in dioxane (6 mL) was added, and the reaction was stirred at 45°C for 8 hours and concentrated under reduced pressure to remove the solvent, so as to obtain the title compound as a crude, which was directly used in the next reaction. MS m/z (ESI): 361.1 [M+H]⁺.

### Step 3: preparation of tert-butyl((1S)-2-((5-(1-(5,5-difluoro-2-oxopiperidin-1-yl)-2-((R)-2-methylmorpholino)ethyl)thiazol-2-yl)amino)-1-((1r,4S)-4-methylcyclohexyl)-2-oxoethyl)carbamate

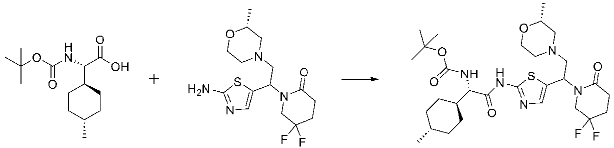

(S)-2-((tert-butoxycarbonyl)amino)-2-((trans)-4-methylcyclohexyl)acetic acid (597 mg, 2.2 mmol) and 1-(1-(2-aminothiazol-5-yl)-2-((R)-2-methylmorpholino) ethyl)-5,5-difluoropiperidin-2-one (385 mg, 1.1 mmol) were dissolved in N,N-dimethylformamide (6 mL). Triethylamine (333.3 mg, 3.3 mmol) and benzotriazol-1-yl-oxy-tripyrrolidinophosphonium hexafluorophosphate (1.2 g, 2.2 mmol) were sequentially added. The reaction was stirred at room temperature for 16 hours. The reaction solution was diluted with ethyl acetate and washed with a saturated sodium chloride aqueous solution, and the phases were separated. The organic phase was dried over anhydrous sodium sulphate, filtered and then concentrated to remove the organic solvent, and the resulting residue was separated by column chromatography to obtain the title compound (520 mg, overall yield over two steps: 77%). MS m/z (ESI): 614.3 [M+H]⁺.

### Step 4: preparation of (2S)-2-amino-N-(5-(1-(5,5-difluoro-2-oxopiperidin-1-yl)-2-((R)-2-methylmorpholino)ethyl)thiazol-2-yl)-2-((1r,4S)-4-methylcyclohexyl)acetamide

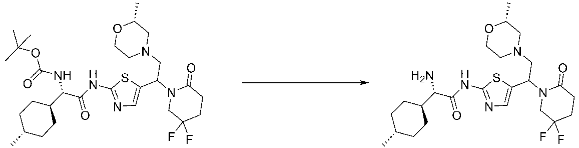

Tert-butyl((1S)-2-((5-(1-(5,5-difluoro-2-oxopiperidin-1-yl)-2-((R)-2-methylmorpholino)ethyl)thiazol-2-yl)amino)-1-((1r,4S)-4-methylcyclohexyl)-2-oxoethyl)carbamate (520 mg, 0.85 mmol) was dissolved in dichloromethane (3 mL). 4 M hydrogen chloride in dioxane (3 mL) was added. The reaction solution was stirred and reacted at room temperature for 1 hour and concentrated under reduced pressure to remove the solvent, so as to obtain the title compound as a crude, which was directly used in the next reaction. MS m/z (ESI): 514.1 [M+H]⁺.

### Step 5: preparation of N-((S)-2-((5-((R)-1-(5,5-difluoro-2-oxopiperidin-1-yl)-2-((R)-2-methylmorpholino)ethyl)thiazol-2-yl)amino)-1-((1r,4S)-4-methylcyclohexyl)-2-oxoethyl)-1-(ethyl-d₅)-1H-pyrazole-5-carboxamide

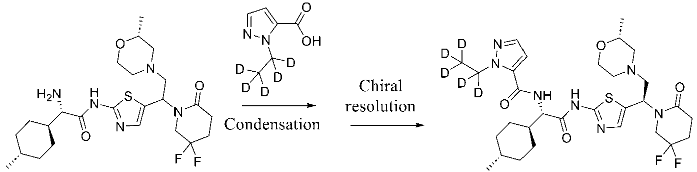

The crude 1-(ethyl-*d*₅)-1*H*-pyrazole-5-carboxylic acid (247 mg, 1.7 mmol) and the crude (2S)-2-amino-N-(5-(1-(5,5-difluoro-2-oxopiperidin-1-yl)-2-((R)-2-methylmorpholino)ethyl)thiazol-2-yl)-2-((1r,4S)-4-methylcyclohexyl)acetamide were dissolved in N,N-dimethylformamide (6 mL). Triethylamine (253 mg, 2.5 mmol) and benzotriazol-1-yl-oxy-tripyrrolidinophosphonium hexafluorophosphate (885 mg, 1.7 mmol) were sequentially added. The reaction was stirred at room temperature for 1 hour. The mixture was diluted with ethyl acetate and washed with a saturated sodium chloride solution, and the phases were separated. The organic phase was dried over anhydrous sodium sulphate, filtered and then concentrated under reduced pressure to remove the organic solvent. The resulting residue was subjected to column chromatography and chiral resolution to obtain the title compound (94 mg, overall yield over two steps: 17%).

¹H NMR (400 MHz, DMSO-*d⁶*) δ 12.30 (s, 1H), 8.60 (d, *J* = 7.6 Hz, 1H), 7.48 (d, *J =* 2.0 Hz, 1H), 7.43-7.38 (m, 1H), 7.05-6.99 (m, 1H), 5.89 (t, *J =* 8.0 Hz, 1H), 4.46 (t, *J =* 8.2 Hz, 1H), 3.78-3.71 (m, 1H), 3.67-3.58 (m, 1H), 3.50-3.40 (m, 3H), 2.92-2.84 (m, 1H), 2.78 (*d, J =* 10.8 Hz, 1H), 2.74-2.67 (m, 2H), 2.56-2.51 (m, 1H), 2.36-2.24 (m, 2H), 2.20-2.11 (m, 1H), 1.87-1.74 (m, 3H), 1.72-1.64 (m, 2H), 1.50-1.42 (m, 1H), 1.33-1.14 (m, 2H), 1.09-0.97 (m, 4H), 0.93-0.78 (m, 6H); MS m/z (ESI): 641.3 [M+H]⁺.

### Example 685 can also be synthesised according to the following steps

### Step 1: synthesis of methyl 1-(methyl-d₃)-1H-pyrazole-5-carboxylate

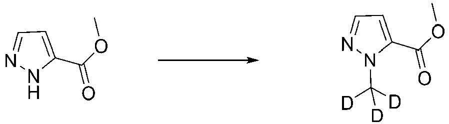

Methyl 1H-pyrazole-5-carboxylate (10 g, 79.30 mmol) and iodomethane-*d*₃ (14 g, 96.55 mmol) were dissolved in acetonitrile (200 mL). Caesium carbonate (39 g, 120 mmol) was added, and the mixture was reacted at room temperature for 2 hours. An aqueous solution (200 mL) was added, and the mixture was extracted with ethyl acetate (100 mL×3). The organic phases were combined, washed with a saturated sodium chloride solution (50 mL), dried over anhydrous sodium sulphate, filtered and concentrated under reduced pressure, and the residue was separated by silica gel column chromatography to obtain the title compound (4.3 g, 38%).

¹ H NMR (400 MHz, CDCl₃) δ 7.47 (d, *J =* 2.0 Hz, 1H), 6.84 (d, *J =* 2.1 Hz, 1H), 3.89 (s, 3H);MS m/z (ESI): 144 [M+H]⁺.

### Step 2: synthesis of 1-(methyl-d₃)-1H-pyrazole-5-carboxylic acid

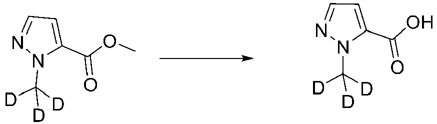

Methyl 1-(methyl-*d*₃)-1H-pyrazole-5-carboxylate (4.3 g, 30.10 mmol) was dissolved in a mixed solution (44 mL) of methanol and water (V/V=8:3), and lithium hydroxide (2 g, 83.33 mmol) was added. The mixture was stirred at room temperature for 3 hours. The reaction solution was concentrated to remove the organic solvent, adjusted to pH 5-6 by dropwise adding 2 M hydrochloric acid and filtered to obtain the title compound (3.2 g, 82 %). MS m/z (ESI): 130[M+H]⁺.

### Step 3: synthesis of N-((S)-2-((5-((R)-1-(5-chloro-2-carbonylpyridin-1(2H)-yl)-2-methoxyethyl)thiazol-2-yl)amino)-1-((1r,4S)-4-methylcyclohexyl)-2-carbonylethyl)-1-(methyl-d₃)-1H-pyrazole-5-carboxamide

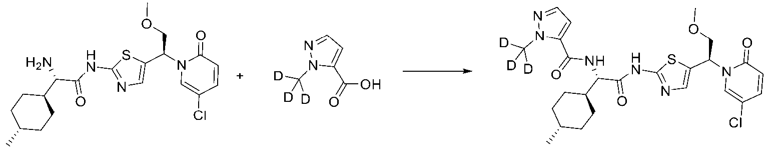

1-(methyl-*d*₃)-1H-pyrazole-5-carboxylic acid (59 mg, 0.46 mmol), HATU (260 mg, 0.68 mmol) and DIPEA (147 mg, 1.14 mmol) were dissolved in N,N-dimethylacetamide (5 mL). The mixture was stirred at room temperature for 5 minutes. (S)-2-amino-N-(5-((R)-1-(5-chloro-2-carbonylpyridin-1(2H)-yl)-2-methoxyethyl)thiazol-2-yl)-2-((1r,4S)-4-methylcyclohexyl)acetamide (100 mg, 0.23 mmol) was added, and the mixture was reacted at room temperature for 1 hour. An aqueous solution (25 mL) was added, and the mixture was extracted with ethyl acetate (10 mL×3). The organic phases were combined, washed with a saturated sodium chloride solution (50 mL), dried over anhydrous sodium sulphate, filtered and concentrated under reduced pressure, and the residue was separated by preparative liquid chromatography to obtain the title compound (64 mg, 51%).

¹H NMR (400 MHz, DMSO-*d*₆) δ 12.41 (s, 1H), 8.57 (d, *J =* 7.5 Hz, 1H), 7.94 (d, *J =* 2.8 Hz, 1H), 7.55 (s, 1H), 7.49-7.46 (m, 2H), 7.04 (d, *J =* 2.1 Hz, 1H), 6.47 (d, J = 9.7 Hz, 1H), 6.28-6.25 (m, 1H), 4.44 (t, *J =* 8.1 Hz, 1H), 4.25-4.20 (m, 1H), 3.91-3.87 (m, 1H), 3.30 (s, 3H), 1.91-1.76 (m, 2H), 1.70-1.62 (s, 2H), 1.44 (d, *J =* 12.5 Hz, 1H), 1.35-1.10 (m, 2H), 1.06-0.95 (m, 1H), 0.90-0.75 (m, 5H); MS m/z (ESI) :550.2 [M+H]⁺.

### Example 686 can also be synthesised according to the following steps

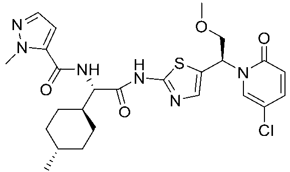

1-methyl-1H-pyrazole-5-carboxylic acid (58 mg, 0.46 mmol), HATU (260 mg, 0.68 mmol) and DIPEA (147 mg, 1.14 mmol) were dissolved in N,N-dimethylacetamide (5 mL). The mixture was stirred at room temperature for 5 minutes. (S)-2-amino-N-(5-((R)-1-(5-chloro-2-carbonylpyridin-1(2H)-yl)-2-methoxyethyl)thiazol-2-yl)-2-((1r,4S)-4-methylcyclohexyl)acetamide (100 mg, 0.23 mmol) was added, and the mixture was reacted at room temperature for 1 hour. An aqueous solution (25 mL) was added, and the mixture was extracted with ethyl acetate (10 mL×3). The organic phases were combined, washed with a saturated sodium chloride solution (50 mL), dried over anhydrous sodium sulphate, filtered and concentrated under reduced pressure, and the residue was separated by preparative liquid chromatography to obtain the title compound (72 mg, 58%). MS m/z (ESI) :547.2 [M+H]⁺.

### Example 687 can also be synthesised according to the following steps

### Step 1: preparation of 2-(3,3-difluoroazetidin-1-yl)-N-methoxy-N-methylacetamide

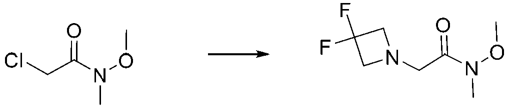

At room temperature, 2-chloro-N-methoxy-N-methylacetamide (5.0 g, 36.5 mmol) and 3,3-difluoroazetidin hydrochloride (5.2 g, 40.2 mmol) were dissolved in acetonitrile (50 mL). Potassium carbonate (10.0 g, 72.5 mmol) was added. The mixture was stirred at room temperature for 10 hours, filtered and then concentrated under reduced pressure to remove the organic solvent, and the residue was separated by column chromatography to obtain the title compound (5.7 g, 81%). MS m/z (ES⁺): 195.1 [M+H]⁺.

### Step 2: preparation of tert-butyl(5-(2-(3,3-difluoroazetidin-1-yl)acetyl)thiazol-2-yl)carbamate

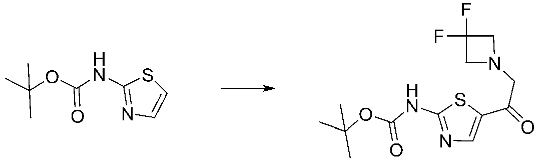

At room temperature, tert-butyl thiazol-2-ylcarbamate (1.0 g, 5.0 mmol) was dissolved in tetrahydrofuran (20 mL). The mixture was cooled to -78°C, and n-butyllithium (5.0 mL, 12.5 mmol) was added dropwise. After the dropwise addition, the mixture was stirred at -78°C for 1 hour. A solution of 2-(3.3-difluoroazetidin-1-yl)-N-methoxy-N-methylacetamide (1.5 g, 7.5 mmol) dissolved in tetrahydrofuran (3 mL) was added dropwise. After the dropwise addition, the mixture was stirred at -78°C for 1 hour. A saturated ammonium chloride solution was added, and the mixture was extracted three times with ethyl acetate. The organic phases were combined and washed with a saturated sodium chloride aqueous solution. The organic phase was dried over anhydrous sodium sulphate, filtered and then concentrated under reduced pressure to remove the organic solvent, and the residue was separated by column chromatography to obtain the title compound (1.3 g, 78%). MS m/z (ES⁺): 334.1 [M+H]⁺.

### Step 3: preparation of tert-butyl(5-(2-(3,3-difluoroazetidin-1-yl)-1-hydroxyethyl)thiazol-2-yl)carbamate

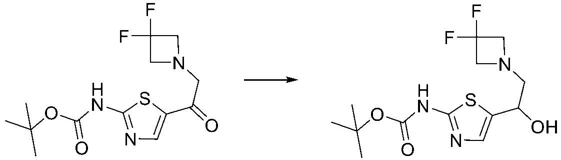

At room temperature, tert-butyl(5-(2-(3,3-difluoroazetidin-1-yl)acetyl)thiazol-2-yl)carbamate (1.3 g, 3.9 mmol) was dissolved in a tetrahydrofuran/methanol solution (15 mL). The mixture was cooled to 0°C, and sodium borohydride (288.6 mg, 7.8 mmol) was added in batches. The mixture was stirred at 0°C for 1 hour and concentrated under reduced pressure to remove the organic solvent. Water was added, and the mixture was extracted three times with dichloromethane. The organic phases were combined and washed with a saturated sodium chloride aqueous solution. The organic phase was dried over anhydrous sodium sulphate, filtered and then concentrated under reduced pressure to remove the organic solvent, and the residue was separated by column chromatography to obtain the title compound (1.2 g, 92%). MS m/z (ES⁺): 336.1 [M+H]⁺.

### Step 4: preparation of tert-butyl(5-(1-(5-chloro-2-carbonylpyridin-1(2H)-yl)-2-(3,3-difluoroazetidin-1-yl)ethyl)thiazol-2-yl)carbamate

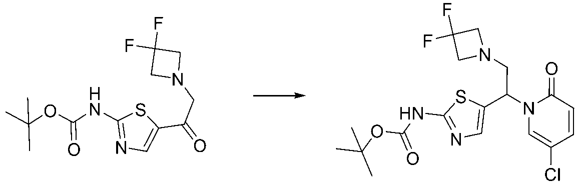

At room temperature, tert-butyl(5-(1-hydroxyl-2-methoxyethyl)thiazol-2-yl)carbamate (670 mg, 2.0 mmol) and 5-chloropyridin-2(1H)-one (129.5 mg, 1.0 mmol) were dissolved in a toluene solution (10 mL). Methyl trifluoromethanesulfonate (82 mg, 0.5 mmol) was added dropwise. The mixture was heated to 110°C, stirred for 5 hours and concentrated under reduced pressure to remove the organic solvent, and the residue was separated by column chromatography to obtain the title compound (137 mg, 31%). MS m/z (ES⁺): 447.1 [M+H]⁺.

### Step 5: preparation of 1-(1-(2-aminothiazol-5-yl)-2-(3,3-difluoroazetidin-1-yl)ethyl)-5-chloropyridin-2(1H)-one

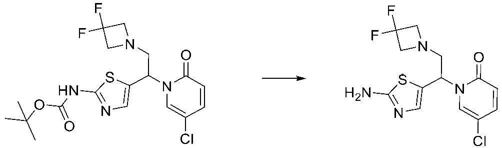

To a solution of tert-butyl(5-(1-(5-chloro-2-carbonylpyridin-1(2H)-yl)-2-(3,3-difluoroazetidin-1-yl)ethyl)thiazol-2-yl)carbamate (137 mg, 0.31 mmol) in dichloromethane (3 mL) was added hydrogen chloride in dioxane (3 mL). The mixture was stirred overnight at room temperature and concentrated under reduced pressure to remove the organic solvent, so as to obtain the title compound (crude, 106 mg), which was directly used in the next step. MS m/z (ESI): 347.1 [M+H]⁺.

### Step 6: preparation of tert-butyl((1S)-2-((5-(1-(5-chloro-2-carbonylpyridin-1(2H)-yl)-2-(3,3-difluoroazetidin-1-yl)ethyl)thiazol-2-yl)amino)-1-((1r,4S)-4-methylcyclohexyl)-2-carbonylethyl)carbamate

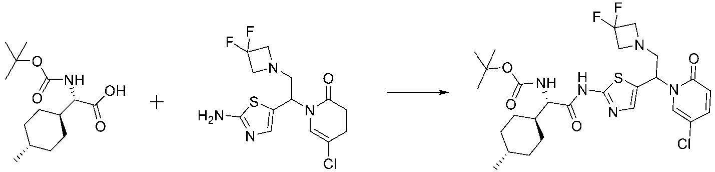

At room temperature, (S)-2-((tert-butoxycarbonyl)amino)-2-((1r,4S)-4-methylcyclohexyl)acetic acid (83.6 mg, 0.31 mmol) and 1-(1-(2-aminothiazol-5-yl)-2-(3,3-difluoroazetidin-1-yl)ethyl)-5-chloropyridin-2(1H)-one (106 mg, 0.31 mmol) were dissolved in N,N-dimethylformamide (5 mL). Triethylamine (93.3 mg, 0.92 mmol) and benzotriazol-1-yl-oxy-tripyrrolidinophosphonium hexafluorophosphate (240.2 mg, 0.46 mmol) were added, and the mixture was stirred at room temperature for 2 hours. Water was added, and the mixture was extracted three times with ethyl acetate. The organic phases were combined and washed with a saturated sodium chloride aqueous solution. The organic phase was dried over anhydrous sodium sulphate, filtered and then concentrated under reduced pressure to remove the organic solvent, and the residue was separated by column chromatography to obtain the title compound (180 mg, 96%). MS m/z (ESI): 600.1 [M+H]⁺.

### Step 7: preparation of (2S)-2-amino-N-(5-(1-(5-chloro-2-carbonylpyridin-1(2H)-yl)-2-(3,3-difluoroazetidin-1-yl)ethyl)thiazol-2-yl)-2-((1r,4S)-4-methylcyclohexyl)acetamide

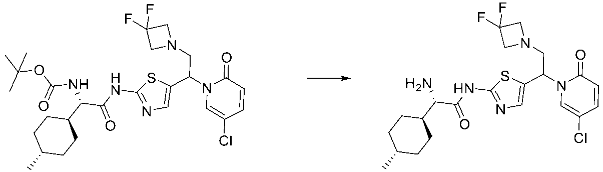

To a solution of tert-butyl((1S)-2-((5-(1-(5-chloro-2-carbonylpyridin-1(2H)-yl)-2-(3,3-difluoroazetidin-1-yl)ethyl)thiazol-2-yl)amino)-1-((1r,4S)-4-methylcyclohexyl)-2-carbonylethyl)carbamate (180 mg, 0.3 mmol) in dichloromethane (5 mL) was added hydrogen chloride in dioxane (5 mL), and the mixture was stirred at room temperature for 1 hour and concentrated under reduced pressure to remove the organic solvent, so as to obtain the title compound (crude hydrochloride, 150 mg), which was directly used in the next reaction. MS m/z (ESI): 500.1 [M+H]⁺.

### Step 8: preparation of N-((1S)-2-((5-(1-(5-chloro-2-carbonylpyridin-1(2H)-yl)-2-(3,3-difluoroazetidin-1-yl)ethyl)thiazol-2-yl)amino)-1-((1r,4S)-4-methylcyclohexyl)-2-carbonylethyl)-4-ethyl-1,2,5-oxadiazole-3-carboxamide

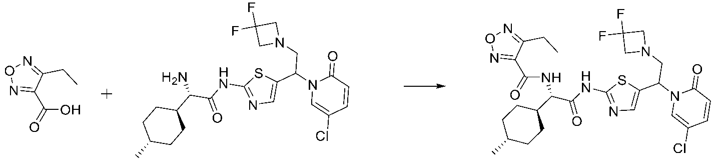

At room temperature, 4-ethyl-1,2,5-oxadiazole-3-carboxylic acid (21.3 mg, 0.15 mmol) and (2S)-2-amino-N-(5-(1-(5-chloro-2-carbonylpyridin-1(2H)-yl)-2-(3,3-difluoroazetidin-1-yl)ethyl)thiazol-2-yl)-2-((1r,4S)-4-methylcyclohexyl)acetamide (75.0 mg, 0.15 mmol) were dissolved in N,N-dimethylformamide (3 mL). Triethylamine (45.4 mg, 0.45 mmol) and benzotriazol-1-yl-oxy-tripyrrolidinophosphonium hexafluorophosphate (119.6 mg, 0.23 mmol) were added, and the mixture was stirred at room temperature for 2 hours. Water was added, and the mixture was extracted three times with ethyl acetate. The organic phases were combined and washed with a saturated sodium chloride aqueous solution. The organic phase was dried over anhydrous sodium sulphate, filtered and then concentrated under reduced pressure to remove the organic solvent, and the residue was separated by column chromatography to obtain the title compound (9.1 mg, 10%).

¹H NMR (400 MHz, DMSO-*d⁶*) *δ* 12.86-11.96 (m, 1H), 9.30 (d, *J* = 7.6 Hz, 1H), 7.94-7.87 (m, 1H), 7.55-7.51 (m, 1H), 7.50-7.44 (m, 1H), 6.47 (d, *J =* 9.6 Hz, 1H), 4.52 (t, *J* = 7.8 Hz, 1H), 3.73-3.54 (m, 5H), 3.26-3.19 (m, 1H), 2.91-2.84 (m, 2H), 2.03-1.96 (m, 1H), 1.84-1.75 (m, 2H), 1.71-1.62 (m, 2H), 1.51-1.44 (m, 1H), 1.29-1.19 (m, 7H), 1.08-0.99 (m, 1H), 0.84 (d, *J =* 6.4 Hz, 3H); MS m/z (ESI): 624.2 [M+H]⁺.

### Example 688 can also be synthesised according to the following steps

### Step 1: synthesis of 2,2-difluoromalonamide

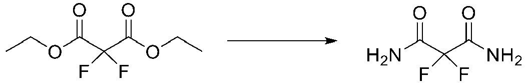

Diethyl-2,2-difluoromalonate (20 g, 102 mmol) was dissolved in methanol (40 mL). An ammonia methanol solution (180 mL, 7 M, 1260 mmol) was added at 0°C, and the mixture was stirred at room temperature for 12 hours. The reaction solution was concentrated under reduced pressure to obtain the title compound (13 g, 92%). MS m/z (ESI):139.1 [M+H]⁺.

### Step 2: synthesis of 2,2-difluoropropane-1,1,3,3-d4-1,3-diamine dihydrochloride

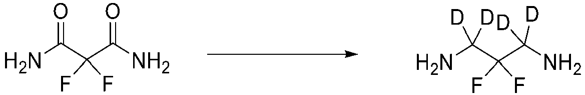

2,2-difluoromalonamide (4 g, 29 mmol) was dissolved in tetrahydrofuran (40 mL). A solution of borane-d₃ in tetrahydrofuran (145 mL, 1 M, 145 mmol) was added at 0°C, and the mixture was stirred at 75°C for 12 hours. The reaction was quenched by slowly adding methanol (200 ml) at 0°C and concentrated under reduced pressure. Hydrogen chloride in methanol (40 mL, 4 M, 160 mmol) was added, and the mixture was stirred at 25°C for 5 hours and filtered to obtain the title compound (4.5 g, 83%).

¹H NMR (400 MHz, DMSO-*d₆*) *δ* 8.91 (s, 4H);MS m/z (ESI):115.1 [M+H]⁺.

### Step 3: preparation of tert-butyl(5-(1-((3-amino-2,2-difluoropropyl-1,1,3,3-d4)amino)-2-methoxyethyl)thiazol-2-yl)carbamate

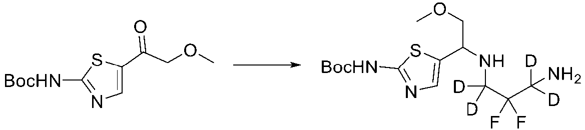

At room temperature, tert-butyl(5-(2-methoxyacetyl)thiazol-2-yl)carbamate (3 g, 11.02 mmol) and 2,2-difluoropropane-1,1,3,3-d₄-1,3-diamine hydrochloride (4.12 g, 22.03 mmol) were dissolved in a mixed solvent of DMA (15 mL) and methanol (8 mL). Triethylamine (5.01 g, 49.57 mmol) was added. The reaction solution was heated to 75°C and stirred for 12 hours, and then cooled to room temperature and stirred for 2 hours. Acetic acid (3.97 g, 66.1 mmol) and sodium cyanoborohydride (4.15 g, 66.1 mmol) were sequentially added, and the mixture was heated to 40°C and stirred for 6 hours. The reaction solution was diluted with ethyl acetate and washed sequentially with a saturated sodium carbonate solution and a saturated sodium chloride aqueous solution. The organic phase was dried over anhydrous sodium sulphate, filtered and concentrated under reduced pressure, and the residue was subjected to column chromatography to obtain the title compound (2.28 g, 55.9%). MS m/z (ESI): 371.2 [M+H]⁺.

### Step 4: preparation of tert-butyl(5-(1-(5,5-difluoro-2-carbonyltetrahydropyrimidin-1(2H)-yl-4,4,6,6-d4)-2-methoxyethyl)thiazol-2-yl)carbamate

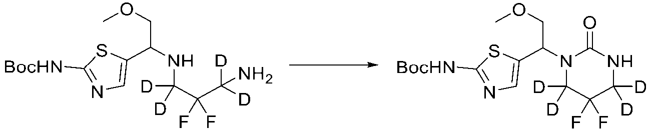

At room temperature, tert-butyl(5-(1-((3-amino-2,2-difluoropropyl-1,1,3,3-d4)amino)-2-methoxyethyl)thiazol-2-yl)carbamate (0.59 g, 1.59 mmol) was dissolved in tetrahydrofuran (6 mL). N,N'-carbonyldiimidazole (477 mg, 2.94 mmol) was added. The mixture was heated to 30°C and stirred for 1 hour, and a white solid precipitated. The resulting precipitate was filtered under reduced pressure, and the filter cake was washed with a small amount of ethyl acetate and dried to obtain the title compound tert-butyl(5-(1-(5,5-difluoro-2-carbonyltetrahydropyrimidin-1(2H)-yl-4,4,6,6-d4)-2-methoxyethyl)thiazol-2-yl)carbamate (360 mg, 57%). MS m/z (ESI): 397.1 [M+H]⁺.

### Step 5: preparation of 1-(1-(2-aminothiazol-5-yl)-2-methoxyethyl)-5,5-difluorotetrahydropyrimidin-2(1H)-one-4,4,6,6-d4 hydrochloride

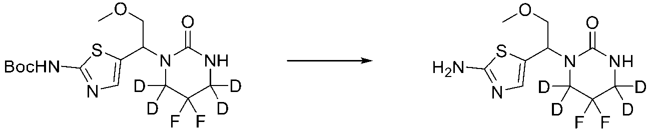

In an ice bath, tert-butyl(5-(1-(5,5-difluoro-2-carbonyltetrahydropyrimidin-1(2H)-yl-4,4,6,6-d4)-2-methoxyethyl)thiazol-2-yl)carbamate (150 mg, 0.38 mmol) was dissolved in dichloromethane (1 mL). 4 M hydrogen chloride in dioxane (3 mL) was added, and the mixture was warmed to room temperature, stirred for 8 hours and concentrated under reduced pressure to obtain the title compound (hydrochloride, 123 mg), which was directly used in the next step. MS m/z (ESI): 297.1 [M+H]⁺.

### Step 6: preparation of tert-butyl((1S)-2-((5-(1-(5,5-difluoro-2-carbonyltetrahydropyrimidin-1(2H)-yl-4,4,6,6-d4)-2-methoxyethyl)thiazol-2-yl)amino)-1-((1r,4S)-4-methylcyclohexyl)-2-carbonylethyl)carbamate

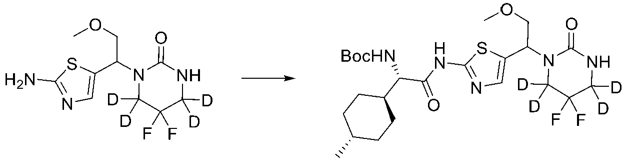

At room temperature, (S)-2-((tert-butoxycarbonyl)amino)-2-((1r,4S)-4-methylcyclohexyl)acetic acid (115 mg, 0.42 mmol) was dissolved in DMF (3 mL). HATU (159.89 mg, 0.42 mmol) and DIPEA (274 mg, 2.12 mmol) were sequentially added, and the mixture was stirred at room temperature for 10 minutes. 1-(1-(2-aminothiazol-5-yl)-2-methoxyethyl)-5,5-difluorotetrahydropyrimidin-2(1H)-one-4,4,6,6-d4 hydrochloride (110 mg, 0.33 mmol) was added, and the mixture was further stirred at room temperature for 1 hour. The reaction solution was diluted with ethyl acetate and washed with a saturated sodium chloride aqueous solution. The organic phase was dried over anhydrous sodium sulphate, filtered and concentrated under reduced pressure, and the residue was subjected to column chromatography to obtain the title compound (126 mg, 69.3%). MS m/z (ESI): 550.2 [M+H]⁺.

### Step 7: preparation of (2S)-2-amino-N-(5-(1-(5,5-difluoro-2-carbonyltetrahydropyrimidin-1(2H)-yl-4,4,6,6-d4)-2-methoxyethyl)thiazol-2-yl)-2-((1r,4S)-4-methylcyclohexyl)acetamide hydrochloride

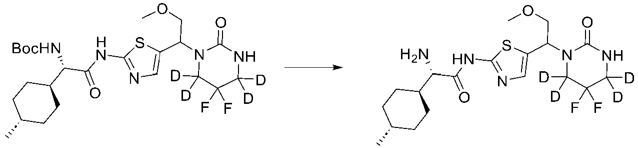

In an ice bath, tert-butyl((1S)-2-((5-(1-(5,5-difluoro-2-carbonyltetrahydropyrimidin-1(2H)-yl-4,4,6,6-d4)-2-methoxyethyl)thiazol-2-yl)amino)-1-((1r,4S)-4-methylcyclohexyl)-2-carbonylethyl)carbamate (126 mg, 0.23 mmol) was dissolved in dichloromethane (0.5 mL). 4 M hydrogen chloride in dioxane (3 mL) was added, and the mixture was warmed to room temperature, stirred for 1 hour and concentrated under reduced pressure to obtain the title compound (hydrochloride, 106 mg), which was directly used in the next step. MS m/z (ESI): 450.2 [M+H]⁺.

### Step 8: preparation of N-((S)-2-((5-((R)-1-(5,5-difluoro-2-carbonyltetrahydropyrimidin-1(2H)-yl-4,4,6,6-d4)-2-methoxyethyl)thiazol-2-yl)amino)-1-((1r,4S)-4-methylcyclohexyl)-2-carbonylethyl)-1-fluorocyclopropane-1-carboxamide

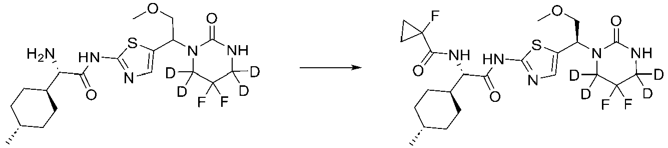

At room temperature, 1-fluorocyclopropane-1-carboxylic acid (35.3 mg, 0.34 mmol) was dissolved in DMF (2 mL). HATU (129.3 mg, 0.34 mmol) and DIPEA (142.2 mg, 1.1 mmol) were sequentially added, and the mixture was stirred at room temperature for 5 minutes. (2S)-2-amino-N-(5-(1-(5,5-difluoro-2-carbonyltetrahydropyrimidin-1(2H)-yl-4,4,6,6-d4)-2-methoxyethyl)thiazol-2-yl)-2-((1r,4S)-4-methylcyclohexyl)acetamide hydrochloride (103 mg, 0.21 mmol) was added, and the mixture was further stirred for 1 hour. The reaction solution was diluted with ethyl acetate and washed with a saturated sodium chloride aqueous solution. The organic phase was dried over anhydrous sodium sulphate, filtered and concentrated under reduced pressure, and the residue was subjected to column chromatography and chiral resolution to obtain the title compound (21.5 mg, 19%).

¹H NMR (400 MHz, DMSO-*d₆*) *δ* 12.20 (s, 1H), 8.34 (d, *J=* 8.0 Hz, 1H), 7.36 (s, 1H), 6.87-6.85 (m, 1H), 5.68 (t, *J* = 6.8 Hz, 1H), 4.37 (t, *J* = 8.6 Hz, 1H), 3.85-3.68 (m, 2H), 3.31 (s, 3H), 1.83-1.72 (m, 2H), 1.71-1.60 (m, 2H), 1.46-1.38 (m, 1H), 1.36 -1.25 (m, 3H), 1.22-1.10 (m, 3H), 1.0-0.92 (m, 2H), 0.90-0.85 (m, 1H), 0.82-0.79 (s, 3H) ; MS m/z (ESI): 536.2 [M+H]⁺.

### Example 689 can also be synthesised according to the following steps

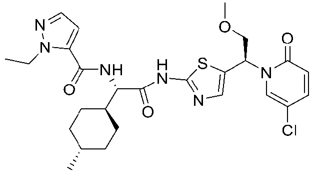

1-ethyl-1H-pyrazole-5-carboxylic acid (64 mg, 0.46 mmol), HATU (260 mg, 0.68 mmol) and DIPEA (147 mg, 1.14 mmol) were dissolved in N,N-dimethylacetamide (5 mL). The mixture was stirred at room temperature for 5 minutes. (S)-2-amino-N-(5-((R)-1-(5-chloro-2-carbonylpyridin-1(2H)-yl)-2-methoxyethyl)thiazol-2-yl)-2-((1r,4S)-4-methylcyclohexyl)acetamide (100 mg, 0.23 mmol) was added, and the mixture was reacted at room temperature for 1 hour. An aqueous solution (25 mL) was added, and the mixture was extracted with ethyl acetate (10 mL×3). The organic phases were combined, washed with a saturated sodium chloride solution (50 mL), dried over anhydrous sodium sulphate, filtered and concentrated under reduced pressure, and the residue was separated by preparative liquid chromatography to obtain the title compound (78 mg, 61%).

¹H NMR (400 MHz, DMSO-*d*₆) *δ* 12.40 (s, 1H), 8.58 (d, *J =* 7.5 Hz, 1H), 7.94 (d, *J =* 2.8 Hz, 1H), 7.56 (d, *J=* 0.9 Hz, 1H), 7.51-7.42 (m, 2H), 7.00 (d, *J =* 2.1 Hz, 1H), 6.47 (d, *J =* 9.7 Hz, 1H), 6.28-6.25 (m, 1H), 4.49-4.21 (m, 3H), 4.25-4.21 (m, 1H), 3.91-3.87 (m, 1H), 3.30 (s, 3H), 1.91-1.73 (m, 2H), 1.63-1.70 (m, 2H), 1.45 (d, *J* = 12.7 Hz, 1H), 1.29-0.98 (m, 5H), 1.08-0.98 (m, 1H), 0.91-0.79 (m, 5H); MS m/z (ESI) :561.2 [M+H]⁺.

### Example 696 can also be synthesised according to the following steps

### Step 1: preparation of tert-butyl(S)-2-((((trifluoromethyl)sulfonyl)oxy) methyl)morpholine-4-carboxylate

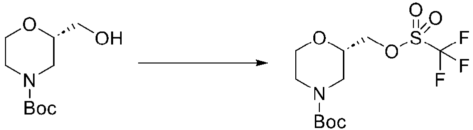

Tert-butyl(2S)-2-(hydroxymethyl)morpholine-4-carboxylate (75 g, 345.21 mmol) and pyridine (41 g, 517.81 mmol) were dissolved in dichloromethane (600 mL). The mixture was cooled to -25°C, and a trifluoromethanesulfonic anhydride solution (116.88 g, 414.25 mmol) in dichloromethane (150 mL) was added dropwise. The mixture was further stirred at -25°C for 1 hour, and the reaction was quenched with 1 N hydrochloric acid solution. The dichloromethane layer was washed with water and a saturated sodium chloride solution, dried over anhydrous sodium sulphate, filtered and concentrated to obtain the residue as the title compound crude, which was directly used in the next reaction. MS m/z (ESI): 350.1[M+H]⁺.

### Step 2: preparation of tert-butyl(S)-2-(fluoromethyl)morpholine-4-carboxylate

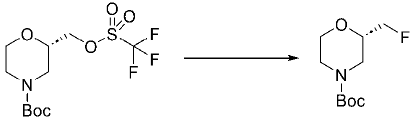

The crude tert-butyl(S)-2-((((trifluoromethyl)sulfonyl)oxy) methyl)morpholine-4-carboxylate was dissolved in tetrahydrofuran (300 mL). Under nitrogen, 1 M tetrabutylammonium fluoride in tetrahydrofuran (520 mL) was added dropwise. After the dropwise addition, the reaction was stirred at room temperature for 1 hour and concentrated under reduced pressure to remove the solvent, and the residue was separated by column chromatography to obtain the title compound (69.3 g, overall yield over two steps: 91%). MS m/z (ESI): 220.1[M+H]⁺.

### Step 3: preparation of (S)-2-(fluoromethyl)morpholine

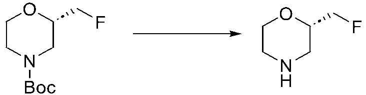

At 0°C, tert-butyl(S)-2-(fluoromethyl)morpholine-4-carboxylate (69.3 g, 316 mmol) was dissolved in 4 M hydrogen chloride in dioxane (200 mL). The mixture was transferred to room temperature, stirred for 1 hour and concentrated under reduced pressure to remove the solvent, and the residue was slurried with ethyl acetate and filtered. The filter residue was collected and dried under reduced pressure to obtain the title compound (44.8 g, 91%). MS m/z (ESI): 120.1[M+H]⁺.

### Step 4: preparation of tert-butyl(5-(1-(5,5-difluoro-2-oxotetrahydropyrimidin-1(2H)-yl)-2-hydroxyethyl)thiazol-2-yl)carbamate

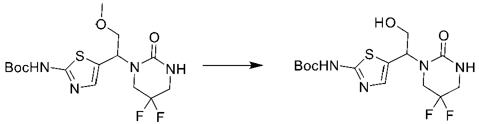

Under cooling in an ice bath, tert-butyl(5-(1-(5,5-difluoro-2-oxotetrahydropyrimidin-1(2H)-yl)-2-methoxyethyl)thiazol-2-yl)carbamate (30 g, 76.45 mmol) was dissolved in dichloromethane (1 L). With stirring, a solution of boron tribromide in dichloromethane (2 M, 300 mL) was slowly added dropwise. The reaction solution was transferred to room temperature, stirred for 24 hours and cooled to -78°C. The reaction was quenched by dropwise adding methanol (500 mL) and concentrated under reduced pressure to remove the organic solvent. To the resulting residue were sequentially added methanol (500 mL), DIPEA (98.80 g, 764.49 mmol, 133.16 mL) and di-tert-butyl dicarbonate (38.68 g, 382.24 mmol). The reaction solution was heated at 60°C, stirred for 6 h and cooled to room temperature. The reaction solution was poured into ice water (3 L), and a white solid precipitated. The resulting precipitate was filtered to collect the filter cake, which was dried under reduced pressure to obtain the title compound (22.5 g, 78%) as a white solid. MS m/z (ESI): 379.1 [M+H]⁺.

### Step 5: preparation of tert-butyl(5-(1-(5,5-difluoro-2-oxotetrahydropyrimidin-1(2H)-yl)-2-oxoethyl)thiazol-2-yl)carbamate

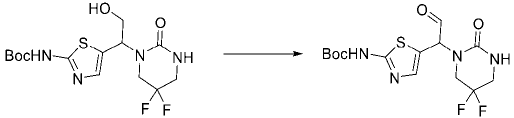

At 3°C, tert-butyl(5-(1-(5,5-difluoro-2-oxotetrahydropyrimidin-1(2H)-yl)-2-hydroxyethyl)thiazol-2-yl)carbamate (7 g, 18.50 mmol) was dispersed in a mixed solvent of dimethyl sulfoxide (40 mL) and ethyl acetate (40 mL). With stirring, triethylamine (7.49 g, 74.00 mmol, 10.32 mL) and pyridine sulphur trioxide (11.78 g, 74.00 mmol) were sequentially added. The mixture was further stirred at 3°C for 30 minutes. The reaction solution was diluted with ethyl acetate (200 mL), and washed sequentially with a 10% citric acid solution and a saturated sodium chloride aqueous solution, and the phases were separated. The organic phase was dried over anhydrous sodium sulphate, filtered and then concentrated under reduced pressure to remove the organic solvent, so as to obtain the title compound as a crude (5.6 g), which was directly used in the next reaction without further purification. MS m/z (ESI): 377.1 [M+H]⁺.

### Step 6: preparation of tert-butyl(5-(1-(5,5-difluoro-2-oxotetrahydropyrimidin-1(2H)-yl)-2-((S)-2-(fluoromethyl)morpholino)ethyl)thiazol-2-yl)carbamate

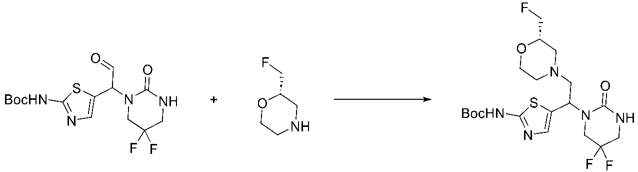

Tert-butyl(5-(1-(5,5-difluoro-2-oxotetrahydropyrimidin-1(2H)-yl)-2-oxoethyl)thiazol-2-yl)carbamate (500 mg, 1.33 mmol), 3-(S)-3-methylmorpholine (620 mg, 3.99 mmol) and DIPEA (504 mg, 3.9 mmol) were dissolved in DCE (20 mL). At 30°C the reaction was stirred for 2 hours. Acetic acid (1 mL) was added, and the reaction was further stirred at 30°C for 12 hours. Sodium triacetoxyborohydride (1.4 g, 6.65 mmol) was added, and the reaction was stirred at room temperature for 1 hour and concentrated under reduced pressure to remove the solvent. The residue was dissolved in ethyl acetate, and washed sequentially with a saturated aqueous sodium carbonate solution and a saturated sodium chloride aqueous solution. The organic phase was separated, dried over anhydrous sodium sulphate, filtered and then concentrated under reduced pressure to remove the organic solvent, and the resulting residue was separated by column chromatography to obtain the title compound (364 mg, 57%). MS m/z (ESI): 480.2 [M+H]+.

### Step 7: preparation of 1-((R)-1-(2-aminothiazol-5-yl)-2-((S)-2-(fluoromethyl)morpholino)ethyl)-5,5-difluorotetrahydropyrimidin-2(1H)-one

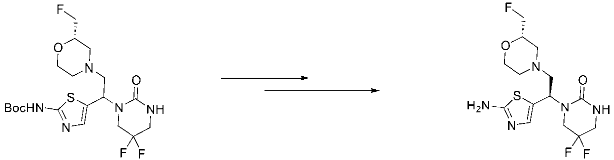

Tert-butyl(5-(1-(5,5-difluoro-2-oxotetrahydropyrimidin-1(2H)-yl)-2-((S)-2-(fluoromethyl)morpholino)ethyl)thiazol-2-yl)carbamate (364 mg, 0.76 mmol) was dissolved in methanol (2 mL). 4 M hydrogen chloride in dioxane (10 mL) was added. The reaction solution was stirred and reacted at 50°C for 4 hours and concentrated under reduced pressure to remove the solvent, and the residue was subjected to chiral resolution to obtain the title compound (130 mg, 45%). MS m/z (ESI): 380.2 [M+H]+.

### Step 8: preparation of tert-butyl((S)-2-((5-((R)-1-(5,5-difluoro-2-oxotetrahydropyrimidin-1(2H)-yl)-2-((S)-2-(fluoromethyl)morpholino)ethyl)thiazol-2-yl)amino)-1-((1r,4S)-4-methylcyclohexyl)-2-oxoethyl)carbamate

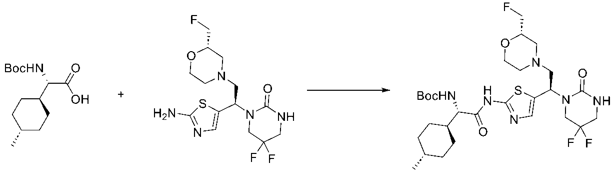

(S)-2-((tert-butoxycarbonyl)amino)-2-((trans)-4-methylcyclohexyl)acetic acid (184 mg, 0.68 mmol) was dissolved in DMF (10 mL). HATU (259 mg, 0.68 mmol) and DIPEA (220 mg, 1.7 mmol) were sequentially added, and the mixture was stirred at room temperature for 10 minutes. 1-((R)-1-(2-aminothiazol-5-yl)-2-((S)-2-(fluoromethyl)morpholino)ethyl)-5,5-difluorotetrahydropyrimidin-2(1H)-one (130 mg, 0.34 mmol) was added. The reaction solution was further stirred and reacted at 40°C for 1 hour. The mixture was cooled to room temperature, diluted with ethyl acetate, washed with a saturated sodium chloride aqueous solution, dried over anhydrous sodium sulphate, filtered and concentrated under reduced pressure, and the residue was separated by column chromatography to obtain the title compound (194 mg, 90%). MS m/z (ESI):633.3 [M+H]⁺.

### Step 9: preparation of (S)-2-amino-N-(5-((R)-1-(5,5-difluoro-2-oxotetrahydropyrimidin-1(2H)-yl)-2-((S)-2-(fluoromethyl)morpholino)ethyl)thiazol-2-yl)-2-((1r,4S)-4-methylcyclohexyl)acetamide

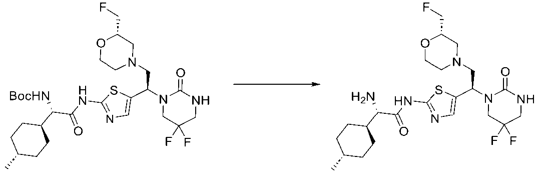

Tert-butyl((S)-2-((5-((R)-1-(5,5-difluoro-2-oxotetrahydropyrimidin-1(2H)-yl)-2-((S)-3-methylmorpholino)ethyl)thiazol-2-yl)amino)-1-((1r,4S)-4-methylcyclohexyl)-2-oxoethyl)carbamate (194 mg, 0.306 mmol) was dissolved in dichloromethane (2 mL). 4 M hydrogen chloride in dioxane (10 mL) was added. The reaction solution was stirred and reacted at room temperature for 1 hour and concentrated under reduced pressure to remove the solvent, so as to obtain the title compound as a crude, which was directly used in the next reaction. MS m/z (ESI):533.3[M+H]⁺.

### Step 10: preparation of N-((S)-2-((5-((R)-1-(5,5-difluoro-2-oxotetrahydropyrimidin-1(2H)-yl)-2-((S)-2-(fluoromethyl)morpholino)ethyl)thiazol-2-yl)amino)-1-((1r,4S)-4-methylcyclohexyl)-2-oxoethyl)-1-fluorocyclopropane-1-carboxamide

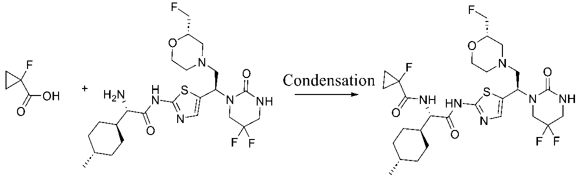

1-fluorocyclopropanecarboxylic acid (64 mg, 0.612 mmol) was dissolved in DMF (10 mL). HATU (233 mg, 0.612 mmol) and DIPEA (237 mg, 1.836 mmol) were sequentially added. The reaction solution was stirred at room temperature for 5 minutes, and the crude (S)-2-amino-N-(5-((R)-1-(5,5-difluoro-2-oxotetrahydropyrimidin-1(2H)-yl)-2-((S)-2-(fluoromethyl)morpholino)ethyl)thiazol-2-yl)-2-((1r,4S)-4-methylcyclohexyl)acetamide was added. The reaction was further stirred at room temperature for 1 hour. The reaction solution was diluted with ethyl acetate, washed with a saturated sodium chloride aqueous solution, dried over anhydrous sodium sulphate, filtered and concentrated under reduced pressure, and the residue was separated by column chromatography to obtain the title compound (150 mg, overall yield over two steps: 80%).

¹H NMR(400 MHz, DMSO-*d₆*) *δ* 12.17 (s, 1H), 8.32 (d, *J =* 8.0 Hz, 1H), 7.36 (d, *J* = 1.2 Hz, 1H), 6.87 (d, *J* = 3.0 Hz, 1H), 5.72 (t, *J* = 8.0 Hz, 1H), 4.40 (dd, *J* = 47.5, 4.4 Hz, 2H), 4.38 (d, *J* = 8.0 Hz, 1H), 3.85-3.78 (m, 1H), 3.70-3.44 (m, 5H), 3.42-3.38 (m, 1H), 2.91-2.82 (m, 2H), 2.80-2.65 (m, 2H), 2.22 (td, *J* = 11.2, 3.2 Hz, 1H), 1.98 (t, *J=* 10.2 Hz, 1H), 1.86-1.72 (m, 2H), 1.71-1.58 (m, 2H), 1.48-1.39 (m, 1H), 1.36-1.22 (m, 3H), 1.21-1.07(m, 3H), 1.01-0.91 (m, 1H), 0.90-0.86 (m, 1H), 0.85-0.77 (m, 4H) ;MS m/z (ESI): 619.3 [M+H]⁺.

### Example 736 can also be synthesised according to the following steps

### Step 1: preparation of tert-butyl(5-(1-(5,5-difluoro-2-oxotetrahydropyrimidin-1(2H)-yl)-2-((2R,5R)-2,5-dimethylmorpholino)ethyl)thiazol-2-yl)carbamate

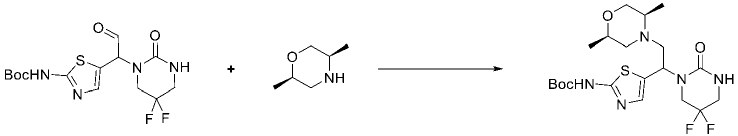

Tert-butyl(5-(1-(5,5-difluoro-2-oxotetrahydropyrimidin-1(2H)-yl)-2-oxoethyl)thiazol-2-yl)carbamate (0.8 g, 2.13 mmol) and (2R,5R)-2,5-dimethylmorpholine (0.97 g, 6.40 mmol) were dissolved in 1,2-dichloroethane (15 mL). Triethylamine (1.1 g, 10.90 mmol) was added, and the reaction mixture was heated to 30°C, and stirred and reacted for 3 hours. Acetic acid (0.9 g, 15 mmol) and sodium triacetoxyborohydride (2.3 g, 10.85 mmol) were added, and the reaction was further stirred at 30°C for 2 hours. The reaction solution was concentrated, diluted with ethyl acetate, washed sequentially with a saturated aqueous sodium carbonate solution and a saturated sodium chloride aqueous solution, dried over anhydrous sodium sulphate, filtered and concentrated under reduced pressure, and the resulting residue was separated by column chromatography to obtain the title compound (0.95 g, 94%). MS m/z (ESI): 476.2 [M+H]⁺.

### Step 2: preparation of 1-(1-(2-aminothiazol-5-yl)-2-((2R,5R)-2,5-dimethylmorpholino)ethyl)-5,5-difluorotetrahydropyrimidin-2(1H)-one

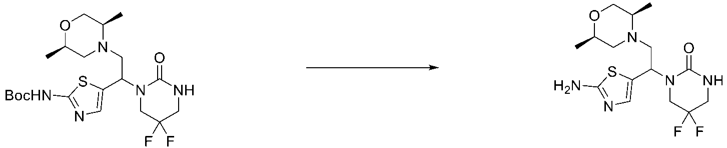

Tert-butyl(5-(1-(5,5-difluoro-2-oxotetrahydropyrimidin-1(2H)-yl)-2-((2R,5R)-2,5-dimethylmorpholino)ethyl)thiazol-2-yl)carbamate (0.95 g, 2.0 mmol) was dissolved in methanol (5 mL). 4 M hydrogen chloride in dioxane (15 mL) was added. The reaction solution was stirred and reacted at 45°C for 3 hours and concentrated under reduced pressure to remove the solvent, so as to obtain the title compound as a crude, which was directly used in the next reaction. MS m/z (ESI): 376.2 [M+H]⁺.

### Step 3: preparation of tert-butyl((1S)-2-((5-(1-(5,5-difluoro-2-oxotetrahydropyrimidin-1(2H)-yl)-2-((2R,5R)-2,5-dimethylmorpholino)ethyl)thiazol-2-yl)amino)-1-((1r,4S)-4-methylcyclohexyl)-2-oxoethyl)carbamate

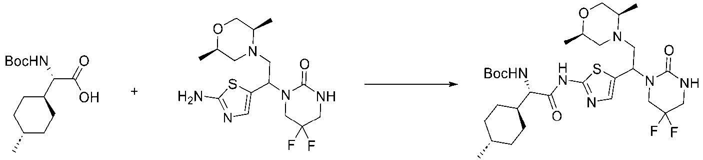

(S)-2-((tert-butoxycarbonyl)amino)-2-((trans)-4-methylcyclohexyl)acetic acid (1.1 g, 4.0 mmol) was dissolved in N,N-dimethylformamide (10 mL). HATU (1.52 g, 4.0 mmol) and N,N-diisopropylethylamine (2.6 g, 20.0 mmol) were sequentially added. The reaction was stirred at room temperature for 30 minutes. 1-(1-(2-aminothiazol-5-yl)-2-((2R,5R)-2,5-dimethylmorpholino)ethyl)-5,5-difluorotetrahydropyrimidin-2(1H)-one was added. The reaction solution was stirred and reacted at 40°C for 1 hour. The mixture was diluted with ethyl acetate, washed with a saturated sodium chloride aqueous solution, dried over anhydrous sodium sulphate, filtered and concentrated under reduced pressure, and the resulting residue was separated by column chromatography to obtain the title compound (600 mg, overall yield over two steps: 48%). MS m/z (ESI): 629.3 [M+H]⁺.

### Step 4: preparation of (2S)-2-amino-N-(5-(1-(5,5-difluoro-2-oxotetrahydropyrimidin-1(2H)-yl)-2-((2R,5R)-2,5-dimethylmorpholino)ethyl)thiazol-2-yl)-2-((1r,4S)-4-methylcyclohexyl)acetamide

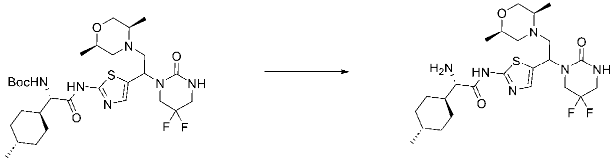

Tert-butyl((1S)-2-((5-(1-(5,5-difluoro-2-oxotetrahydropyrimidin-1(2H)-yl)-2-((2R,5R)-2,5-dimethylmorpholino)ethyl)thiazol-2-yl)amino)-1-((1r,4S)-4-methylcyclohexyl)-2-oxoethyl)carbamate (600 mg, 0.96 mmol) was dissolved in 4 M hydrogen chloride in dioxane (15 mL), and the reaction was stirred at 30°C for 30 minutes and concentrated under reduced pressure to remove the solvent, so as to obtain the title compound as a crude, which was directly used in the next step. MS m/z (ESI): 529.3 [M+H]⁺.

### Step 5: preparation of N-((S)-2-((5-((R)-1-(5,5-difluoro-2-oxotetrahydropyrimidin-1(2H)-yl)-2-((2R,5R)-2,5-dimethylmorpholino)ethyl)thiazol-2-yl)amino)-1-((1r,4S)-4-methylcyclohexyl)-2-oxoethyl)-1-fluorocyclopropane-1-carboxamide

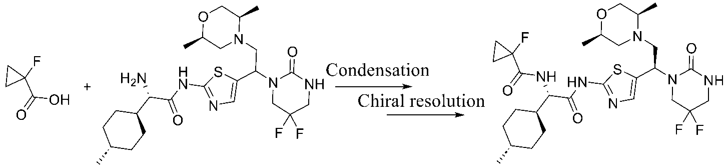

1-fluorocyclopropane-1-carboxylic acid (200 mg, 1.92 mmol) was dissolved in N,N-dimethylformamide (10 mL). HATU (738 mg, 1.92 mmol) and N,N-diisopropylethylamine (621 mg, 4.8 mmol) were sequentially added. The reaction solution was stirred at room temperature for 25 minutes, and the crude (2S)-2-amino-N-(5-(1-(5,5-difluoro-2-oxotetrahydropyrimidin-1(2H)-yl)-2-((2R,5R)-2,5-dimethylmorpholino)ethyl)thiazol-2-yl)-2-((1r,4S)-4-methylcyclohexyl)acetamide was added. The reaction was further stirred at room temperature for 1 hour. The reaction solution was diluted with ethyl acetate, washed with a saturated sodium chloride aqueous solution, dried over anhydrous sodium sulphate, filtered and concentrated under reduced pressure, and the residue was separated by column chromatography and subjected to chiral resolution to obtain the title compound (105 mg, overall yield over two steps: 18%).

¹H NMR (400 MHz, DMSO-*d*₆) *δ* 12.19 (s, 1H), 8.36 (d, *J* = 8.1 Hz, 1H), 7.37 *(d, J=* 1.1 Hz, 1H), 6.87 *(d, J =* 3.3 Hz, 1H), 5.67 (*t, J =* 8.1 Hz, 1H), 4.38 (*t, J =* 8.4 Hz, 1H), 3.63-3.40 (m, 6H), 3.38-3.30 (m, 1H), 2.90-2.85 (m, 1H), 2.81-2.67 (m, 2H),2.59-2.50 (m, 1H), 2.24 (t, *J =* 10.7 Hz, 1H), 1.81-1.64 (m, 5H), 1.44-1.12 (m, 6H), 1.05-0.98 (m, 7H), 0.92-0.79 (m, 5H). MS m/z (ESI): 615.3 [M+H]⁺.

### Example 763 can also be synthesised according to the following steps

### Step 1: preparation of tert-butyl(5-(1-(5,5-difluoro-2-oxotetrahydropyrimidin-1(2H)-yl)-2-(4,4-difluoropiperidin-1-yl)ethyl)thiazol-2-yl)carbamate

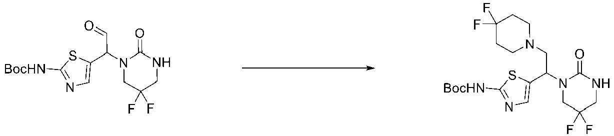

Tert-butyl(5-(1-(5,5-difluoro-2-oxotetrahydropyrimidin-1(2H)-yl)-2-oxoethyl)thiazol-2-yl)carbamate (5 g, 13.28 mmol) and 4,4-difluoropiperidine (4.83 g, 39.85 mmol) were dissolved in DCE (80 mL). The reaction mixture was heated to 30°C, stirred and reacted for 3 hours, and cooled to room temperature. Acetic acid (6.6 mL) and sodium triacetylborohydride (4.67 g, 22.05 mmol) were added, and the reaction was stirred at room temperature for 1 hour. The reaction solution was diluted with ethyl acetate, and washed sequentially with a saturated aqueous sodium carbonate solution and a saturated sodium chloride aqueous solution. The organic phase was separated, dried over anhydrous sodium sulphate, filtered and then concentrated under reduced pressure to remove the organic solvent, and the resulting residue was separated by column chromatography to obtain the title compound (3.6 g, 56%). MS m/z (ESI): 482.2 [M+H]⁺.

### Step 2: preparation of 1-(1-(2-aminothiazol-5-yl)-2-(4,4-difluoropiperidin-1-yl)ethyl)-5,5-difluorotetrahydropyrimidin-2(1H)-one

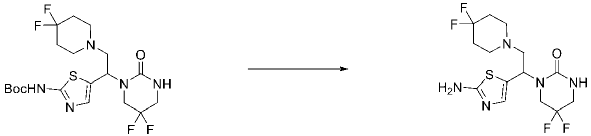

Tert-butyl(5-(1-(5,5-difluoro-2-oxotetrahydropyrimidin-1(2H)-yl)-2-(4,4-difluoropiperidin-1-yl)ethyl)thiazol-2-yl)carbamate (120 mg, 0.25 mmol) was dissolved in methanol (1 mL). 4 M hydrogen chloride in dioxane (3 mL) was added. The reaction solution was stirred and reacted at room temperature for 8 hours and concentrated under reduced pressure to remove the solvent, so as to obtain the title compound as a crude (113 mg), which was directly used in the next step. MS m/z (ESI): 382.2 [M+H]⁺.

### Step 3: preparation of tert-butyl((1S)-2-((5-(1-(5,5-difluoro-2-oxotetrahydropyrimidin-1(2H)-yl)-2-(4,4-difluoropiperidin-1-yl)ethyl)thiazol-2-yl)amino)-1-((1r,4S)-4-methylcyclohexyl)-2-oxoethyl)carbamate

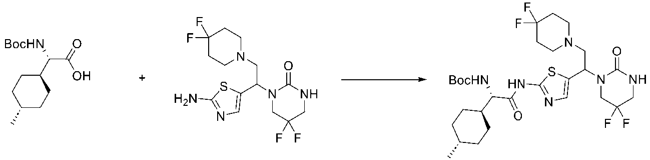

(S)-2-((tert-butoxycarbonyl)amino)-2-((trans)-4-methylcyclohexyl)acetic acid (115 mg, 0.42 mmol) was dissolved in DMF (3 mL). HATU (160 mg, 0.42 mmol) and DIPEA (274 mg, 2.12 mmol) were sequentially added. The reaction was stirred at room temperature for 10 minutes, and the crude 1-(1-(2-aminothiazol-5-yl)-2-(4,4-difluoropiperidin-1-yl)ethyl)-5,5-difluorotetrahydropyrimidin-2(1H)-one was added. The reaction solution was further stirred and reacted at room temperature for 1 hour, diluted with ethyl acetate and washed with a saturated sodium chloride aqueous solution. The organic phase was separated, dried over anhydrous sodium sulphate, filtered and then concentrated under reduced pressure to remove the organic solvent, and the resulting residue was separated by column chromatography to obtain the title compound (116 mg, overall yield over two steps: 73%). MS m/z (ESI): 635.2 [M+H]⁺.

### Step 4: preparation of (2S)-2-amino-N-(5-(1-(5,5-difluoro-2-oxotetrahydropyrimidin-1(2H)-yl)-2-(4,4-difluoropiperidin-1-yl)ethyl)thiazol-2-yl)-2-((1r,4S)-4-methylcyclohexyl)acetamide

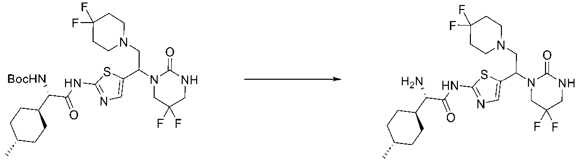

Tert-butyl((1S)-2-((5-(1-(5,5-difluoro-2-oxotetrahydropyrimidin-1(2H)-yl)-2-(4,4-difluoropiperidin-1-yl)ethyl)thiazol-2-yl)amino)-1-((1r,4S)-4-methylcyclohexyl)-2-oxoethyl)carbamate (116 mg, 0.18 mmol) was dissolved in methanol (2 mL). 4 M hydrogen chloride in dioxane (5 mL) was added. The reaction solution was stirred and reacted at room temperature for 1 hour and concentrated under reduced pressure to remove the solvent, so as to obtain the title compound as a crude (110 mg), which was directly used in the next step. MS m/z (ESI): 535.2 [M+H]⁺.

### Step 5: preparation of N-((S)-2-((5-((R)-1-(5,5-difluoro-2-oxotetrahydropyrimidin-1(2H)-yl)-2-(4,4-difluoropiperidin-1-yl)ethyl)thiazol-2-yl)amino)-1-((1r,4S)-4-methylcyclohexyl)-2-oxoethyl)-1-fluorocyclopropane-1-carboxamide

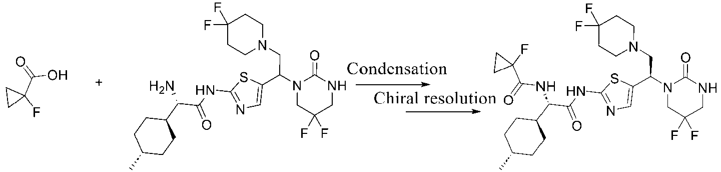

1-fluorocyclopropane-1-carboxylic acid (37 mg, 0.36 mmol) was dissolved in DMF (2 mL). HATU (137 mg, 0.36 mmol) and DIPEA (146 mg, 1.13 mmol) were sequentially added. The reaction solution was stirred at room temperature for 5 minutes. (2S)-2-amino-N-(5-(1-(5,5-difluoro-2-oxotetrahydropyrimidin-1(2H)-yl)-2-(4,4-difluoropiperidin-1-yl)ethyl)thiazol-2-yl)-2-((1r,4S)-4-methylcyclohexyl)acetamide (110 mg, 0.18 mmol) was added. The reaction was further stirred at room temperature for 1 hour. The reaction solution was diluted with ethyl acetate and washed with a saturated sodium chloride aqueous solution. The organic phase was separated, dried over anhydrous sodium sulphate, filtered and then concentrated under reduced pressure to remove the organic solvent, and the residue was separated by column chromatography and then subjected to chiral HPLC resolution to obtain the title compound (22 mg, overall yield over two steps: 20%).

¹H NMR (400 MHz, DMSO-*d₆*) *δ* 12.19 (s, 1H), 8.36 (d, *J =* 8.2 Hz, 1H), 7.35 (d, *J =* 1.2 Hz, 1H), 6.89 (d, *J =* 3.0 Hz, 1H), 5.70 (t, *J =* 8.0 Hz, 1H), 4.38 (t, *J =* 8.4 Hz, 1H), 3.65-3.44 (m, 3H), 3.43-3.39 (m, 1H), 2.95-2.86 (m, 1H), 2.85-2.75 (m, 1H), 2.72- 2.54 (m, 4H), 2.05-1.86 (m, 4H), 1.85-1.73 (m, 2H), 1.70-1.60 (m, 2H), 1.46-1.37 (m, 1H), 1.36-1.22 (m, 3H), 1.20-1.07(m, 3H), 1.02-0.86 (m, 2H), 0.86-0.75 (m, 4H) ; MS m/z (ESI): 621.3 [M+H]⁺.

### Example 774

### Step 1: preparation of tert-butyl(5-(1-(5,5-difluoro-2-oxotetrahydropyrimidin-1(2H)-yl)-2-(4-(trifluoromethyl)piperidin-1-yl)ethyl)thiazol-2-yl)carbamate

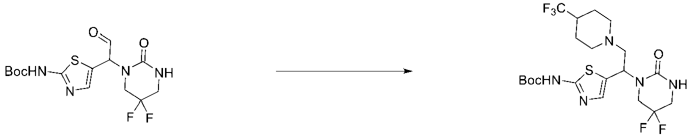

Tert-butyl(5-(1-(5,5-difluoro-2-oxotetrahydropyrimidin-1(2H)-yl)-2-oxoethyl)thiazol-2-yl)carbamate (5.8 g, 15.41 mmol) and 4-(trifluoromethyl)piperidine (7.08 g, 46.23 mmol) were dissolved in ethanol (60 mL). The reaction mixture was heated to 50°C, and stirred and reacted for 2 hours. Acetic acid (2.78 g, 46.23 mmol) and sodium cyanoborohydride (4.84 g, 77.05 mmol) were added. The reaction was stirred at 50°C for 6 hours and concentrated under reduced pressure to remove the solvent, and the residue was dissolved in ethyl acetate. The mixture was washed sequentially with a saturated aqueous sodium carbonate solution and a saturated sodium chloride aqueous solution, dried over anhydrous sodium sulphate, filtered and concentrated under reduced pressure, and the residue was separated by column chromatography to obtain the title compound (6.1 g, 77%). MS m/z (ESI): 514.2 [M+H]⁺.

### Step 2: preparation of (R)-1-(1-(2-aminothiazol-5-yl)-2-(4-(trifluoromethyl)piperidin-1-yl)ethyl)-5,5-difluorotetrahydropyrimidin-2(1H)-one

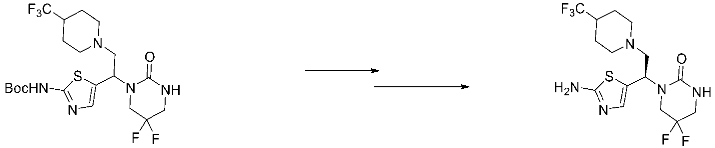

Tert-butyl(5-(1-(5,5-difluoro-2-oxotetrahydropyrimidin-1(2H)-yl)-2-(4-(trifluoromethyl)piperidin-1-yl)ethyl)thiazol-2-yl)carbamate (6.1 g, 11.88 mmol) was dissolved in methanol (10 mL). 4 M hydrogen chloride in dioxane (30 mL) was added. The reaction solution was stirred and reacted at room temperature for 8 hours and concentrated under reduced pressure to remove the solvent, and the residue was subjected to chiral resolution to obtain the title compound (2.21 g, 45%). MS m/z (ESI): 414.1 [M+H]⁺.

### Step 3: preparation of tert-butyl((S)-2-((5-((R)-1-(5,5-difluoro-2-oxotetrahydropyrimidin-1(2H)-yl)-2-(4-(trifluoromethyl)piperidin-1-yl)ethyl)thiazol-2-yl)amino)-1-((1r,4S)-4-methylcyclohexyl)-2-oxoethyl)carbamate

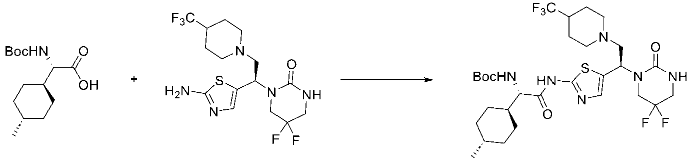

(S)-2-((tert-butoxycarbonyl)amino)-2-((trans)-4-methylcyclohexyl)acetic acid (1.74 g, 6.41 mmol) was dissolved in DMF (20 mL). HATU (2.44 g, 6.41 mmol) and DIPEA (2.07 g, 16.04 mmol) were sequentially added, and the mixture was stirred at room temperature for 10 minutes. (R)-1-(1-(2-aminothiazol-5-yl)-2-(4-(trifluoromethyl)piperidin-1-yl)ethyl)-5,5-difluorotetrahydropyrimidin-2(1H)-one (2.21 g, 5.35 mmol) was added. The reaction solution was stirred and reacted at 40°C for 1 hour. The mixture was diluted with ethyl acetate, washed with a saturated sodium chloride aqueous solution, dried over anhydrous sodium sulphate, filtered and concentrated under reduced pressure, and the residue was separated by column chromatography to obtain the title compound (3.11 g, 87%). MS m/z (ESI): 667.3 [M+H]⁺.

### Step 4: preparation of (S)-2-amino-N-(5-((R)-1-(5,5-difluoro-2-oxotetrahydropyrimidin-1(2H)-yl)-2-(4-(trifluoromethyl)piperidin-1-yl)ethyl)thiazol-2-yl)-2-((1r,4S)-4-methylcyclohexyl)acetamide

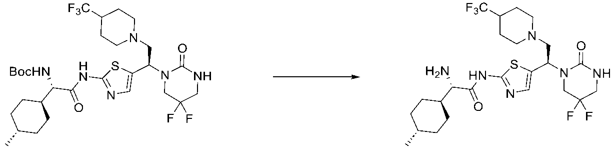

Tert-butyl((S)-2-((5-((R)-1-(5,5-difluoro-2-oxotetrahydropyrimidin-1(2H)-yl)-2-(4-(trifluoromethyl)piperidin-1-yl)ethyl)thiazol-2-yl)amino)-1-((1r,4S)-4-methylcyclohexyl)-2-oxoethyl)carbamate (3.11 g, 4.66 mmol) was dissolved in dichloromethane (10 mL). 4 M hydrogen chloride in dioxane (30 mL) was added. The reaction solution was stirred and reacted at room temperature for 1 hour and concentrated under reduced pressure to remove the solvent, so as to obtain the title compound as a crude, which was directly used in the next reaction. MS m/z (ESI): 567.3 [M+H]⁺.

### Step 5: preparation of N-((S)-2-((5-((R)-1-(5,5-difluoro-2-oxotetrahydropyrimidin-1(2H)-yl)-2-(4-(trifluoromethyl)piperidin-1-yl)ethyl)thiazol-2-yl)amino)-1-((1r,4S)-4-methylcyclohexyl)-2-oxoethyl)-1-fluorocyclopropane-1-carboxamide

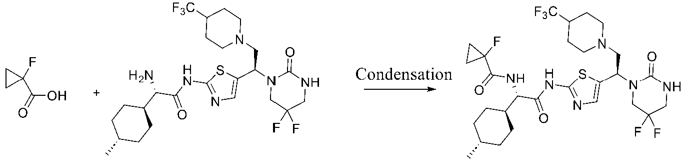

1-fluorocyclopropanecarboxylic acid (539 mg, 5.18 mmol) was dissolved in DMF (20 mL). HATU (1.97 g, 5.18 mmol) and DIPEA (2.79 g, 21.58 mmol) were sequentially added. The reaction solution was stirred at room temperature for 5 minutes, and the crude (S)-2-amino-N-(5-((R)-1-(5,5-difluoro-2-oxotetrahydropyrimidin-1(2H)-yl)-2-(4-(trifluoromethyl)piperidin-1-yl)ethyl)thiazol-2-yl)-2-((1r,4S)-4-methylcyclohexyl)acetamide was added. The reaction was further stirred at room temperature for 1 hour. The reaction solution was diluted with ethyl acetate, washed with a saturated sodium chloride aqueous solution, dried over anhydrous sodium sulphate, filtered and concentrated under reduced pressure, and the residue was separated by column chromatography to obtain the title compound (2.41 g, overall yield over two steps: 79%).

¹H NMR (400 MHz, DMSO-*d₆*) *δ* 12.18 (s, 1H), 8.37 (d, *J* = 8.1 Hz, 1H), 7.34 (d, *J* = 1.2 Hz, 1H), 6.89 (s, 1H), 5.69 (t, *J* = 7.9 Hz, 1H), 4.37 (t, *J* = 8.4 Hz, 1H), 3.64-3.44 (m, 4H), 3.07-2.95 (m, 2H), 2.90-2.64 (m, 2H), 2.38-2.16 (m, 1H), 2.16-1.98 (m, 2H), 1.83-1.72 (m, 4H), 1.67 (t, *J* = 11.0 Hz, 2H), 1.53-1.07 (m, 10H), 0.99-0.84 (m, 5H). MS m/z (ESI): 653.3 [M+H]⁺.

### Example 820 can also be synthesised according to the following steps

### Step 1: preparation of tert-butyl(5-(1-(5,5-difluoro-2-oxopiperidin-1-yl)-2-((S)-2-(fluoromethyl)morpholino)ethyl)thiazol-2-yl)carbamate

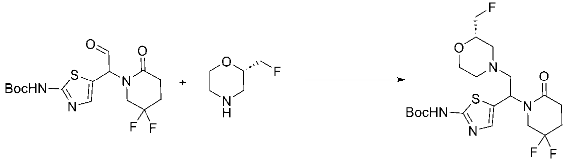

Tert-butyl N-[5-[1-(5,5-difluoro-2-oxo-1-piperidyl)-2-oxo-ethyl]thiazol-2-yl]carbamate (500 mg, 1.33 mmol) and (2S)-2-(fluoromethyl)morpholine (622 mg, 4.0 mmol) were dissolved in methanol (20 mL). N,N-diisopropylethylamine (516 mg, 4.0 mmol) was added and dissolved at room temperature with stirring. Acetic acid (4 mL) was added, and the reaction was heated to 50°C and stirred for 12 hours. Sodium cyanoborohydride (419 mg, 6.66 mmol) was added, and the mixture was further stirred for 1 hour and concentrated under reduced pressure to remove the solvent. The residue was dissolved in ethyl acetate, washed with a saturated sodium carbonate solution and a saturated sodium chloride solution, dried over anhydrous sodium sulphate, filtered and then concentrated under reduced pressure to remove the organic solvent, so as to obtain the title compound as a crude, which was directly used in the next reaction. MS m/z (ESI): 479.2[M+H]⁺.

### Step 2: preparation of 1-(1-(2-aminothiazol-5-yl)-2-((S)-2-(fluoromethyl)morpholino)ethyl)-5,5-difluoropiperidin-2-one

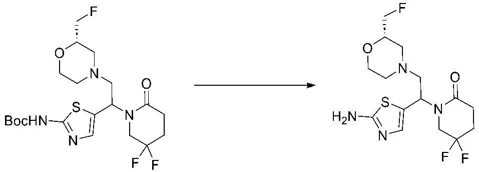

The crude tert-butyl(5-(1-(5,5-difluoro-2-oxopiperidin-1-yl)-2-((S)-2-(fluoromethyl)morpholino)ethyl)thiazol-2-yl)carbamate was dissolved in 4 M hydrogen chloride in dioxane (30 mL). The mixture was heated to 50°C, and the reaction was stirred for 2 hours and concentrated under reduced pressure to remove the solvent, so as to obtain the title compound as a crude, which was directly used in the next reaction. MS m/z (ESI): 379.1[M+H]⁺.

### Step 3: preparation of tert-butyl((1S)-2-((5-(1-(5,5-difluoro-2-oxopiperidin-1-yl)-2-((S)-2-(fluoromethyl)morpholino)ethyl)thiazol-2-yl)amino)-1-((1r,4S)-4-methylcyclohexyl)-2-oxoethyl)carbamate

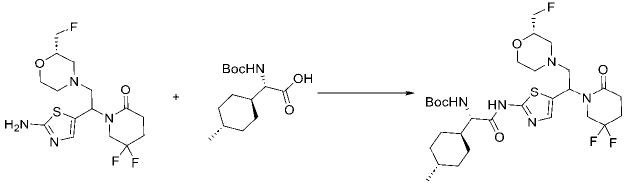

(2S)-2-(tert-butoxycarbonylamino)-2-(4-methylcyclohexyl)acetic acid (722 mg, 2.66 mmol) was dissolved in N,N-dimethylformamide (15 mL). HATU (1.00 g, 2.66 mmol) and N,N-diisopropylethylamine (859 mg, 6.65 mmol, 1.16 mL) were sequentially added, and the mixture was stirred at room temperature for 20 minutes. The crude 1-(1-(2-aminothiazol-5-yl)-2-((S)-2-(fluoromethyl)morpholino)ethyl)-5,5-difluoropiperidin-2-one was added, and the reaction was heated to 40°C and stirred for 1 hour. The mixture was diluted with ethyl acetate, washed with a saturated sodium carbonate solution and a saturated sodium chloride solution, dried over anhydrous sodium sulphate, filtered and concentrated under reduced pressure to obtain the residue as the title compound crude. MS m/z (ESI): 632.3[M+H]⁺.

### Step 4: preparation of (2S)-2-amino-N-(5-(1-(5,5-difluoro-2-oxopiperidin-1-yl)-2-((S)-2-(fluoromethyl)morpholino)ethyl)thiazol-2-yl)-2-((1r,4S)-4-methylcyclohexyl)acetamide

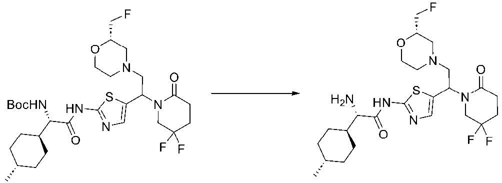

The crude tert-butyl((1S)-2-((5-(1-(5,5-difluoro-2-oxopiperidin-1-yl)-2-((S)-2-(fluoromethyl)morpholino)ethyl)thiazol-2-yl)amino)-1-((1r,4S)-4-methylcyclohexyl)-2-oxoethyl)carbamate was dissolved in 4 M hydrogen chloride in dioxane (20 mL). The mixture was stirred at room temperature for 30 minutes and concentrated under reduced pressure to remove the solvent, so as to obtain the title compound as a crude, which was directly used in the next reaction. MS m/z (ESI): 532.3[M+H]⁺.

### Step 5: preparation of N-((S)-2-((5-((R)-1-(5,5-difluoro-2-oxopiperidin-1-yl)-2-((S)-2-(fluoromethyl)morpholino)ethyl)thiazol-2-yl)amino)-1-((1r,4S)-4-methylcyclohexyl)-2-oxoethyl)-1-(ethyl-d5)-1H-pyrazole-5-carboxamide

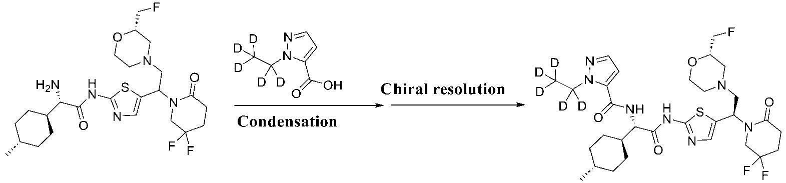

2-(ethyl-*d₅*)pyrazole-3-carboxylic acid (385 mg, 2.65 mmol) was dissolved in N,N-dimethylformamide (15 mL). HATU (1.00 g, 2.65 mmol) and N,N-diisopropylethylamine (857 mg, 6.63 mmol) were sequentially added, and the mixture was stirred at room temperature for 10 minutes. The crude (2S)-2-amino-N-(5-(1-(5,5-difluoro-2-oxopiperidin-1-yl)-2-((S)-2-(fluoromethyl)morpholino)ethyl)thiazol-2-yl)-2-((1r,4S)-4-methylcyclohexyl)acetamide was added, and the mixture was stirred at room temperature for 30 minutes. The mixture was diluted with ethyl acetate, washed with a saturated sodium carbonate solution and a saturated sodium chloride solution, dried over anhydrous sodium sulphate, filtered and concentrated under reduced pressure, and the residue was separated by HPLC and subjected to chiral resolution to obtain the title compound (101 mg, overall yield over five steps: 12%).

¹H NMR (400 MHz, DMSO-*d₆*) *δ* 12.28 (s, 1H), 8.58 (s, 1H), 7.53 - 7.45 (m, 1H), 7.41 (d, *J* = 5.5 Hz, 1H), 7.01 (dt, *J =* 5.1, 2.1 Hz, 1H), 5.90 (d, *J* = 7.7 Hz, 1H), 4.47 (d, *J* = 9.4 Hz, 2H), 4.33 (s, 1H), 3.81 (s, 1H), 3.63 (s, 2H), 3.49 (d, *J* = 13.9 Hz, 2H), 2.93 (s, 1H), 2.74 (*t, J =* 20.5 Hz, 3H), 2.31 (s, 4H), 1.99 (s, 1H), 1.92-1.75 (m, 2H), 1.69 (s, 2H), 1.46 (s, 1H), 1.38-1.13 (m, 3H), 1.04 (s, 1H), 0.86 (t, *J =* 5.7 Hz, 5H); MS m/z (ESI): 659.3[M+H]⁺.

### Example 849 can also be synthesised according to the following steps

### Step 1: preparation of tert-butyl(5-(1-(5,5-difluoro-2-oxopiperidin-1-yl)-2-((2R,6S)-2,6-dimethylmorpholino)ethyl)thiazol-2-yl)carbamate

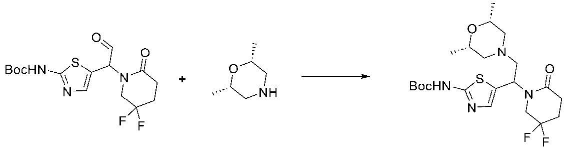

Tert-butyl N-[5-[1-(5,5-difluoro-2-oxo-1-piperidyl)-2-oxo-ethyl]thiazol-2-yl]carbamate (500 mg, 1.33 mmol) and cis-2,6-dimethylmorpholine (461 mg, 4.00 mmol) were dissolved in a mixed solvent of acetic acid (4 mL) and methanol (20 mL). The reaction was heated to 50°C and stirred for 12 hours. Sodium cyanoborohydride (419 mg, 6.66 mmol) was added, and the mixture was further stirred for 1 hour and concentrated under reduced pressure to remove the solvent. The residue was dissolved in ethyl acetate. The mixture was washed sequentially with a saturated sodium carbonate solution and a saturated sodium chloride solution, dried over anhydrous sodium sulphate, filtered and concentrated to obtain the residue as the title compound crude, which was directly used in the next reaction. MS m/z (ESI): 475.2[M+H]⁺.

### Step 2: preparation of 1-(1-(2-aminothiazol-5-yl)-2-((2R,6S)-2,6-dimethylmorpholino)ethyl)-5,5-difluoropiperidin-2-one

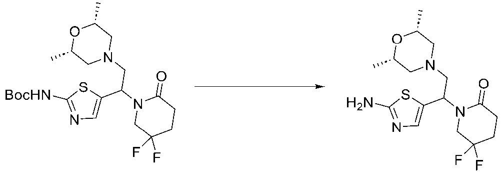

The crude tert-butyl(5-(1-(5,5-difluoro-2-oxopiperidin-1-yl)-2-((2R,6S)-2,6-dimethylmorpholino)ethyl)thiazol-2-yl)carbamate was dissolved in 4 M hydrogen chloride in dioxane (30 mL). The mixture was heated to 50°C, and the reaction was stirred for 2 hours and concentrated under reduced pressure to remove the solvent, so as to obtain the title compound as a crude, which was directly used in the next reaction. MS m/z (ESI): 375.2[M+H]⁺.

### Step 3: preparation of tert-butyl((1S)-2-((5-(1-(5,5-difluoro-2-oxopiperidin-1-yl)-2-((2R,6S)-2,6-dimethylmorpholino)ethyl)thiazol-2-yl)amino)-1-((1r,4S)-4-methylcyclohexyl)-2-oxoethyl)carbamate

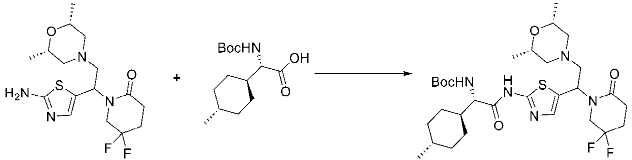

(2S)-2-(tert-butoxycarbonylamino)-2-(4-methylcyclohexyl)acetic acid (722 mg, 2.66 mmol) was dissolved in N,N-dimethylformamide (15 mL). HATU (1.00 g, 2.66 mmol) and N,N-diisopropylethylamine (860 mg, 6.65 mmol) were sequentially added, and the mixture was stirred at room temperature for 20 minutes. The crude 1-(1-(2-aminothiazol-5-yl)-2-((2R,6S)-2,6-dimethylmorpholino)ethyl)-5,5-difluoropiperidin-2-one was added, and the reaction was transferred to 40°C and stirred for 1 hour. The mixture was diluted with ethyl acetate, washed sequentially with a saturated sodium carbonate solution and a saturated sodium chloride solution, dried over anhydrous sodium sulphate, filtered and concentrated under reduced pressure to obtain the title compound as a crude, which was directly used in the next reaction. MS m/z (ESI): 628.3[M+H]⁺.

### Step 4: preparation of (2S)-2-amino-N-(5-(1-(5,5-difluoro-2-oxopiperidin-1-yl)-2-((2R,6S)-2,6-dimethylmorpholino)ethyl)thiazol-2-yl)-2-((1r,4S)-4-methylcyclohexyl)acetamide

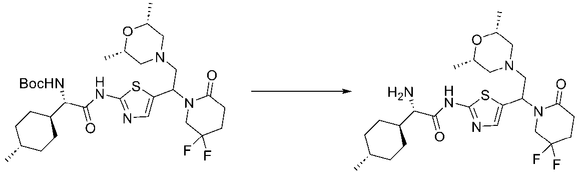

The crude tert-butyl((1S)-2-((5-(1-(5,5-difluoro-2-oxopiperidin-1-yl)-2-((2R,6S)-2,6-dimethylmorpholino)ethyl)thiazol-2-yl)amino)-1-((1r,4S)-4-methylcyclohexyl)-2-oxoethyl)carbamate was dissolved in 4 M hydrogen chloride in dioxane (20 mL). The mixture was stirred at room temperature for 30 minutes and concentrated under reduced pressure to remove the solvent, so as to obtain the title compound as a crude, which was directly used in the next reaction. MS m/z (ESI): 528.3[M+H]⁺.

### Step 5: preparation of N-((S)-2-((5-((R)-1-(5,5-difluoro-2-oxopiperidin-1-yl)-2-((2R,6S)-2,6-dimethylmorpholino)ethyl)thiazol-2-yl)amino)-1-((1r,4S)-4-methylcyclohexyl)-2-oxoethyl)-1-(ethyl-d5)-1H-pyrazole-5-carboxamide

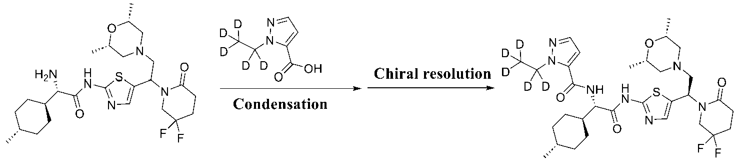

2-(ethyl-*d₅*) pyrazole-3-carboxylic acid (386 mg, 2.65 mmol) was dissolved in N,N-dimethylformamide (15 mL). HATU (1.00 g, 2.65 mmol) and N,N-diisopropylethylamine (860 mg, 6.64 mmol) were sequentially added, and the mixture was stirred at room temperature for 20 minutes. The crude (2S)-2-amino-N-(5-(1-(5,5-difluoro-2-oxopiperidin-1-yl)-2-((2R,6S)-2,6-dimethylmorpholino)ethyl)thiazol-2-yl)-2-((1r,4S)-4-methylcyclohexyl)acetamide was added, and the mixture was further stirred for 30 minutes. The reaction solution was diluted with ethyl acetate, washed with a saturated sodium carbonate solution and a saturated sodium chloride solution, dried over anhydrous sodium sulphate, filtered and concentrated under reduced pressure, and the residue was separated by HPLC and subjected to chiral resolution to obtain the title compound (153 mg, overall yield over five steps: 18%).

¹H NMR (400 MHz, DMSO-*d₆*) *δ* 12.26 (s, 1H), 8.57 (d, *J =* 7.6 Hz, 1H), 7.48 (t, *J =* 1.5 Hz, 1H), 7.38 (s, 1H), 7.03-6.98 (m, 1H), 5.89 (t, *J =* 8.0 Hz, 1H), 4.46 (t, *J = 8.2* Hz, 1H), 3.71-3.57 (m, 1H), 3.57-3.37 (m, 3H), 2.89-2.64 (m, 4H), 2.30 (dq, *J* = 15.7, 7.4 Hz, 2H), 1.80 (dt, *J* = 21.1, 11.4 Hz, 3H), 1.69 (t, *J* = 10.4 Hz, 3H), 1.46 (d, *J =* 12.4 Hz, 1H), 1.21 (dt, *J* = 25.1, 15.0 Hz, 3H), 1.03 (t, *J* = 6.6 Hz, 7H), 0.86 (t, *J* = 9.0 Hz, 6H); MS m/z (ESI): 655.3[M+H]⁺.

Example 854 can also be synthesised according to the following steps

### Step 1: preparation of tert-butyl(5-(1-(5,5-difluoro-2-oxopiperidin-1-yl)-2-((2S,6S)-2,6-dimethylmorpholino)ethyl)thiazol-2-yl)carbamate

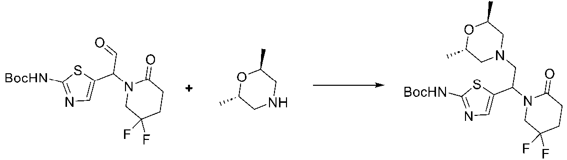

Tert-butyl N-[5-[1-(5,5-difluoro-2-oxo-1-piperidyl)-2-oxo-ethyl]thiazol-2-yl]carbamate (500 mg, 1.33 mmol) and (2S,6S)-2,6-dimethylmorpholine (460 mg, 4.00 mmol) were dissolved in a mixed solvent of acetic acid (4 mL) and methanol (20 mL). The reaction was heated to 50°C and stirred for 3 hours. Sodium cyanoborohydride (419 mg, 6.66 mmol) was added, and the mixture was further stirred for 30 minutes and concentrated under reduced pressure to remove the solvent. The residue was dissolved in ethyl acetate. The mixture was washed sequentially with a saturated sodium carbonate solution and a saturated sodium chloride solution, dried over anhydrous sodium sulphate, filtered and concentrated under reduced pressure to obtain the residue as the title compound crude, which was directly used in the next reaction. MS m/z (ESI): 475.2[M+H]⁺.

### Step 2: preparation of 1-(1-(2-aminothiazol-5-yl)-2-((2S,6S)-2,6-dimethylmorpholino)ethyl)-5,5-difluoropiperidin-2-one

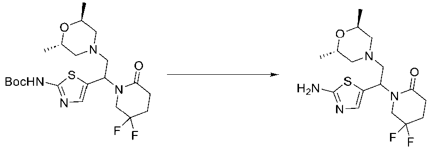

The crude tert-butyl(5-(1-(5,5-difluoro-2-oxopiperidin-1-yl)-2-((2S,6S)-2,6-dimethylmorpholino)ethyl)thiazol-2-yl)carbamate was dissolved in 4 M hydrogen chloride in dioxane (30 mL). The mixture was heated to 50°C, and the reaction was stirred for 2 hours and concentrated under reduced pressure to remove the solvent, so as to obtain the title compound as a crude, which was directly used in the next reaction. MS m/z (ESI): 375.2[M+H]⁺.

### Step 3: preparation of tert-butyl((1S)-2-((5-(1-(5,5-difluoro-2-oxopiperidin-1-yl)-2-((2S,6S)-2,6-dimethylmorpholino)ethyl)thiazol-2-yl)amino)-1-((1r,4S)-4-methylcyclohexyl)-2-oxoethyl)carbamate

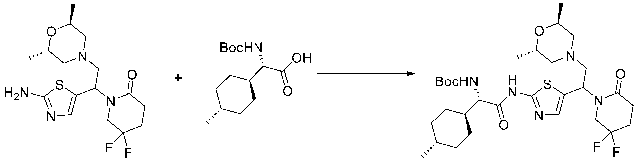

(2S)-2-(tert-butoxycarbonylamino)-2-(4-methylcyclohexyl)acetic acid (722 mg, 2.66 mmol) was dissolved in N,N-dimethylformamide (15 mL). HATU (1.00 g, 2.66 mmol) and N,N-diisopropylethylamine (860 mg, 6.65 mmol) were sequentially added, and the mixture was stirred at room temperature for 20 minutes, and mixed with the crude 1-(1-(2-aminothiazol-5-yl)-2-((2S,6S)-2,6-dimethylmorpholino)ethyl)-5,5-difluoropiperidin-2-one. The mixture was transferred to 40°C and stirred for 1 hour. The resultant was cooled to room temperature, diluted with ethyl acetate, washed sequentially with a saturated sodium carbonate solution and a saturated sodium chloride solution, dried over anhydrous sodium sulphate, filtered and concentrated under reduced pressure to obtain the title compound as a crude. MS m/z (ESI): 628.3[M+H]⁺.

### Step 4: preparation of (2S)-2-amino-N-(5-(1-(5,5-difluoro-2-oxopiperidin-1-yl)-2-((2S,6S)-2,6-dimethylmorpholino)ethyl)thiazol-2-yl)-2-((1r,4S)-4-methylcyclohexyl)acetamide

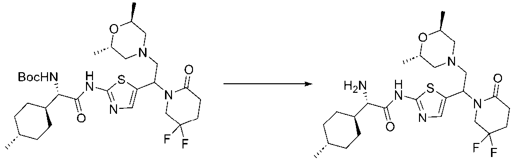

The crude tert-butyl ((1S)-2-((5-(1-(5,5-difluoro-2-oxopiperidin-1-yl)-2-((2S,6S)-2,6-dimethylmorpholino)ethyl)thiazol-2-yl)amino)-1-((1r,4S)-4-methylcyclohexyl)-2-oxoethyl)carbamate was dissolved in 4 M hydrogen chloride in dioxane (30 mL). The mixture was stirred at room temperature for 30 minutes and concentrated under reduced pressure to remove the solvent, so as to obtain the title compound as a crude, which was directly used in the next reaction. MS m/z (ESI): 528.3[M+H]⁺.

### Step 5: preparation of N-((S)-2-((5-((R)-1-(5,5-difluoro-2-oxopiperidin-1-yl)-2-((2S,6S)-2,6-dimethylmorpholino)ethyl)thiazol-2-yl)amino)-1-((1r,4S)-4-methylcyclohexyl)-2-oxoethyl)-1-(ethyl-d5)-1H-pyrazole-5-carboxamide

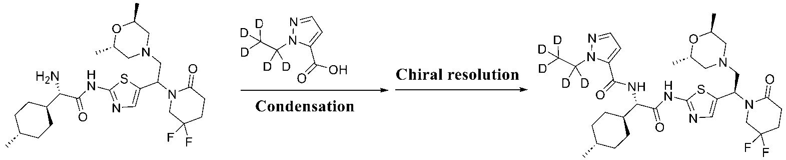

2-(ethyl-*d₅*)pyrazole-3-carboxylic acid (386 mg, 2.65 mmol) was dissolved in N,N-dimethylformamide (15 mL). HATU (1.00 g, 2.65 mmol) and N,N-diisopropylethylamine (860 mg, 6.64 mmol) were sequentially added, and the mixture was stirred at room temperature for 20 minutes. The crude (2S)-2-amino-N-(5-(1-(5,5-difluoro-2-oxopiperidin-1-yl)-2-((2S,6S)-2,6-dimethylmorpholino)ethyl)thiazol-2-yl)-2-((1r,4S)-4-methylcyclohexyl)acetamide was added, and the mixture was further stirred for 30 minutes. The mixture was diluted with ethyl acetate, washed with a saturated sodium carbonate solution and a saturated sodium chloride solution, dried over anhydrous sodium sulphate, filtered and concentrated under reduced pressure, and the residue was subjected to HPLC and chiral resolution to obtain the title compound (150 mg, overall yield over five steps: 17%).

¹H NMR (400 MHz, DMSO-*d₆*) δ 12.28 (s, 1H), 8.58 (d, *J* = 7.7 Hz, 1H), 7.48 (d, *J =* 2.0 Hz, 1H), 7.39 (s, 1H), 7.00 (d, *J* = 2.1 Hz, 1H), 5.95 (dd, *J* = 10.5, 6.2 Hz, 1H), 4.45 (t, *J* = 8.2 Hz, 1H), 3.92-3.80 (m, 2H), 3.67 (ddd, *J* = 17.7, 12.9, 10.0 Hz, 1H), 3.43 (q, *J* = 12.3 Hz, 1H), 2.92 (dd, *J* = 12.7, 10.5 Hz, 1H), 2.56 (dd, *J* = 12.8, 6.4 Hz, 2H), 2.52-2.38 (m, 2H), 2.38-2.26 (m, 1H), 2.20 (dd, *J* = 11.0, 5.7 Hz, 2H), 1.82 (dd, *J* = 20.8, 12.2 Hz, 2H), 1.68 (s, 2H), 1.46 (d, *J* = 12.8 Hz, 1H), 1.26-1.12 (m, 4H), 1.09 (d, *J* = 6.4 Hz, 7H), 0.94-0.78 (m, 5H); MS m/z (ESI): 655.3[M+H]⁺.

### Example 857 can also be synthesised according to the following steps

### Step 1: preparation of tert-butyl(5-(1-(5,5-difluoro-2-oxopiperidin-1-yl)-2-((2R,6R)-2,6-dimethylmorpholino)ethyl)thiazol-2-yl)carbamate

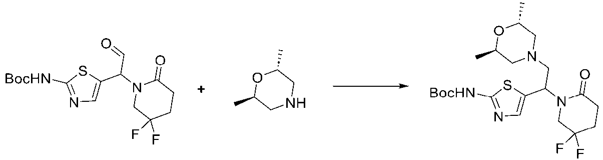

Tert-butyl N-[5-[1-(5,5-difluoro-2-oxo-1-piperidyl)-2-oxo-ethyl]thiazol-2-yl]carbamate (500 mg, 1.33 mmol) and (2R,6R)-2,6-dimethylmorpholine (460 mg, 4.00 mmol) were dissolved in a mixed solvent of acetic acid (4 mL) and methanol (20 mL). The reaction was heated to 50°C and stirred for 12 hours. Sodium cyanoborohydride (419 mg, 6.66 mmol) was added, and the mixture was further stirred for 1 hour and concentrated under reduced pressure to remove the solvent. The residue was dissolved in ethyl acetate. The mixture was washed sequentially with a saturated sodium carbonate solution and a saturated sodium chloride solution, dried over anhydrous sodium sulphate, filtered and concentrated under reduced pressure to obtain the residue as the title compound crude, which was directly used in the next reaction.

MS m/z (ESI): 475.2[M+H]⁺.

### Step 2: preparation of 1-(1-(2-aminothiazol-5-yl)-2-((2R,6R)-2,6-dimethylmorpholino)ethyl)-5,5-difluoropiperidin-2-one

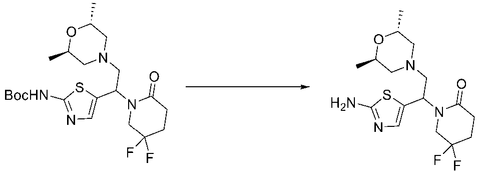

The crude tert-butyl(5-(1-(5,5-difluoro-2-oxopiperidin-1-yl)-2-((2R,6R)-2,6-dimethylmorpholino)ethyl)thiazol-2-yl)carbamate was dissolved in 4 M hydrogen chloride in dioxane (30 mL). The mixture was heated to 50°C, and the reaction was stirred for 3 hours and concentrated under reduced pressure to remove the solvent, so as to obtain the title compound as a crude, which was directly used in the next reaction. MS m/z (ESI): 375.2[M+H]⁺.

### Step 3: preparation of tert-butyl((1S)-2-((5-(1-(5,5-difluoro-2-oxopiperidin-1-yl)-2-((2R,6R)-2,6-dimethylmorpholino)ethyl)thiazol-2-yl)amino)-1-((1r,4S)-4-methylcyclohexyl)-2-oxoethyl)carbamate

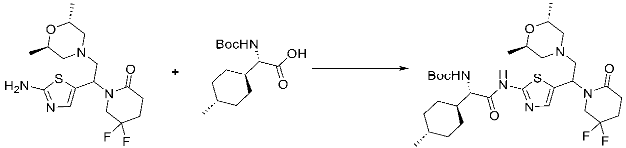

(2S)-2-(tert-butoxycarbonylamino)-2-(4-methylcyclohexyl)acetic acid (722 mg, 2.66 mmol) was dissolved in N,N-dimethylformamide (15 mL). HATU (1.00 g, 2.66 mmol) and N,N-diisopropylethylamine (860 mg, 6.65 mmol) were sequentially added, and the mixture was stirred at room temperature for 20 minutes. The crude 1-(1-(2-aminothiazol-5-yl)-2-((2R,6R)-2,6-dimethylmorpholino)ethyl)-5,5-difluoropiperidin-2-one was added, and the reaction was stirred at 40°C for 1 hour. The mixture was cooled to room temperature, diluted with ethyl acetate, washed with a saturated sodium carbonate solution and a saturated sodium chloride solution, dried over anhydrous sodium sulphate, filtered and concentrated under reduced pressure to obtain the residue as the title compound crude, which was directly used in the next reaction. MS m/z (ESI): 628.3[M+H]⁺.

### Step 4: preparation of (2S)-2-amino-N-(5-(1-(5,5-difluoro-2-oxopiperidin-1-yl)-2-((2R,6R)-2,6-dimethylmorpholino)ethyl)thiazol-2-yl)-2-((1r,4S)-4-methylcyclohexyl)acetamide

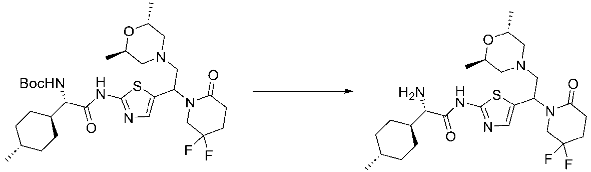

The crude tert-butyl ((1S)-2-((5-(1-(5,5-difluoro-2-oxopiperidin-1-yl)-2-((2R,6R)-2,6-dimethylmorpholino)ethyl)thiazol-2-yl)amino)-1-((1r,4S)-4-methylcyclohexyl)-2-oxoethyl)carbamate was dissolved in 4 M hydrogen chloride in dioxane (20 mL). The mixture was stirred at room temperature for 30 minutes and concentrated under reduced pressure to remove the solvent, so as to obtain the title compound as a crude, which was directly used in the next reaction. MS m/z (ESI): 528.3[M+H]⁺.

### Step 5: preparation of N-((S)-2-((5-((R)-1-(5,5-difluoro-2-oxopiperidin-1-yl)-2-((2R,6R)-2,6-dimethylmorpholino)ethyl)thiazol-2-yl)amino)-1-((1r,4S)-4-methylcyclohexyl)-2-oxoethyl)-1-(ethyl-d5)-1H-pyrazole-5-carboxamide

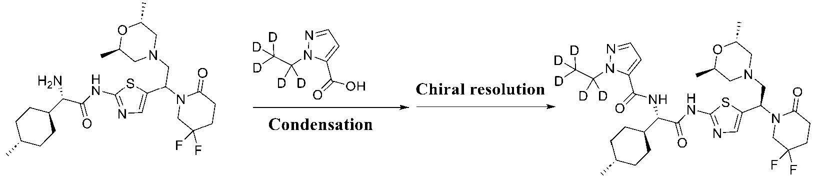

2-(ethyl-*d₅*)pyrazole-3-carboxylic acid (386 mg, 2.65 mmol) was dissolved in N,N-dimethylformamide (15 mL). HATU (1.00 g, 2.65 mmol) and N,N-diisopropylethylamine (860 mg, 6.64 mmol) were sequentially added, and the mixture was stirred at room temperature for 20 minutes. The crude (2S)-2-amino-N-(5-(1-(5,5-difluoro-2-oxopiperidin-1-yl)-2-((2R,6R)-2,6-dimethylmorpholino)ethyl)thiazol-2-yl)-2-((1r,4S)-4-methylcyclohexyl)acetamide was added, and the mixture was stirred at room temperature for 30 minutes. The mixture was diluted with ethyl acetate, washed with a saturated sodium carbonate solution and a saturated sodium chloride solution, dried over anhydrous sodium sulphate, filtered and concentrated under reduced pressure, and the residue was purified by HPLC and subjected to chiral resolution to obtain the title compound (169 mg, overall yield over five steps: 19%).

¹H NMR (400 MHz, DMSO-*d₆*) δ 12.27 (s, 1H), 8.57 (d, *J =* 7.7 Hz, 1H), 7.48 (d, *J* = 2.1 Hz, 1H), 7.38 (d, *J* = 1.1 Hz, 1H), 7.01 (d, *J* = 2.1 Hz, 1H), 5.88 (t, *J* = 8.0 Hz, 1H), 4.46 (t, *J* = 8.2 Hz, 1H), 3.90-3.80 (m, 2H), 3.70-3.56 (m, 1H), 3.45 (q, *J* = 12.4 Hz, 1H), 2.89-2.79 (m, 1H), 2.61 (dd, *J* = 12.8, 6.6 Hz, 1H), 2.54-2.49 (m, 1H), 2.38-2.18 (m, 3H), 2.17-2.08 (m, 2H), 1.81 (t, *J =* 15.6 Hz, 2H), 1.68 (s, 2H), 1.46 (d, *J =* 11.7 Hz, 1H), 1.35-1.15 (m, 4H), 1.10-0.97 (m, 7H), 0.85 (d, *J* = 6.5 Hz, 5H); MS m/z (ESI): 655.3[M+H]⁺.

### Example 860 can also be synthesised according to the following steps

### Step 1: preparation of tert-butyl(5-(1-(5,5-difluoro-2-oxopiperidin-1-yl)-2-((2S,5S)-2,5-dimethylmorpholino)ethyl)thiazol-2-yl)carbamate

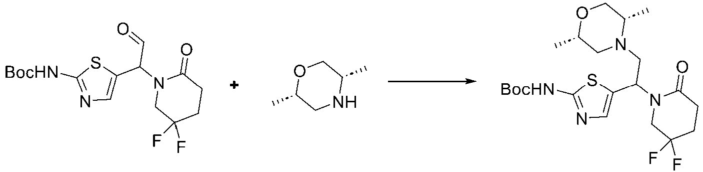

Tert-butyl N-[5-[1-(5,5-difluoro-2-oxo-1-piperidyl)-2-oxo-ethyl]thiazol-2-yl]carbamate (500 mg, 1.33 mmol) and (2S,5S)-2,5-dimethylmorpholine (797 mg, 6.93 mmol) were dissolved in a mixed solvent of acetic acid (2 mL) and 1,2-dichloroethane (15 mL). The reaction was transferred to 30°C and stirred for 15 hours. Sodium triacetoxyborohydride (1.41 g, 6.66 mmol) was added. The mixture was further stirred for 1 hour and concentrated under reduced pressure to remove the solvent, and the residue was dissolved in ethyl acetate. The mixture was washed sequentially with a saturated sodium carbonate solution and a saturated sodium chloride solution, dried over anhydrous sodium sulphate, filtered and concentrated, and the residue was separated by silica gel column chromatography to obtain the title compound (340 mg, 54%). MS m/z (ESI): 475.2[M+H]⁺.

### Step 2: preparation of 1-(1-(2-aminothiazol-5-yl)-2-((2S,5S)-2,5-dimethylmorpholino)ethyl)-5,5-difluoropiperidin-2-one

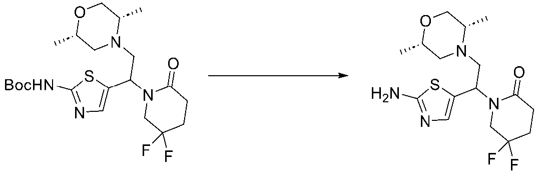

Tert-butyl(5-(1-(5,5-difluoro-2-oxopiperidin-1-yl)-2-((2S,5S)-2,5-dimethylmorpholino)ethyl)thiazol-2-yl)carbamate (340 mg, 716.45 µmol) was dissolved in 4 M hydrogen chloride in dioxane (20 mL). The reaction was transferred to 50°C, stirred for 3 hours and concentrated under reduced pressure to remove the solvent, so as to obtain the title compound crude, which was directly used in the next reaction. MS m/z (ESI): 375.2[M+H]⁺.

### Step 3: preparation of tert-butyl((1S)-2-((5-(1-(5,5-difluoro-2-oxopiperidin-1-yl)-2-((2S,5S)-2,5-dimethylmorpholino)ethyl)thiazol-2-yl)amino)-1-((1r,4S)-4-methylcyclohexyl)-2-oxoethyl)carbamate

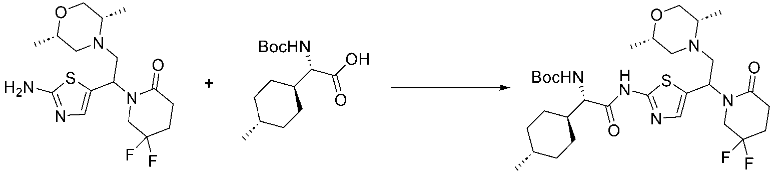

(2S)-2-(tert-butoxycarbonylamino)-2-(4-methylcyclohexyl)acetic acid (388 mg, 1.43 mmol) was dissolved in N,N-dimethylformamide (15 mL). HATU (544 mg, 1.43 mmol) and N,N-diisopropylethylamine (463 mg, 3.58 mmol) were sequentially added, and the mixture was stirred at room temperature for 30 minutes. 1-(1-(2-aminothiazol-5-yl)-2-((2S,5S)-2,5-dimethylmorpholino)ethyl)-5,5-difluoropiperidin-2-one was added. The reaction was transferred to 40°C and stirred for 1 hour. The reaction solution was cooled to room temperature, diluted with ethyl acetate, washed with a saturated sodium carbonate solution and a saturated sodium chloride solution, dried over anhydrous sodium sulphate, filtered and then concentrated under reduced pressure to remove the organic solvent, so as to obtain the residue as the title compound crude, which was directly used in the next reaction. MS m/z (ESI): 628.3[M+H]⁺.

### Step 4: preparation of (2S)-2-amino-N-(5-(1-(5,5-difluoro-2-oxopiperidin-1-yl)-2-((2S,5S)-2,5-dimethylmorpholino)ethyl)thiazol-2-yl)-2-((1r,4S)-4-methylcyclohexyl)acetamide

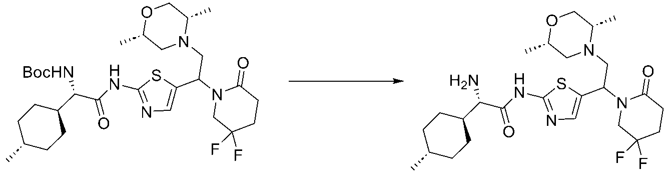

The crude tert-butyl((1S)-2-((5-(1-(5,5-difluoro-2-oxopiperidin-1-yl)-2-((2S,55)-2,5-dimethylmorpholino)ethyl)thiazol-2-yl)amino)-1-((1r,4S)-4-methylcyclohexyl)-2-oxoethyl)carbamate was dissolved in 4 M hydrogen chloride in dioxane (20 mL). The mixture was stirred at room temperature for 30 minutes and concentrated under reduced pressure to obtain the title compound as a crude, which was directly used in the next reaction. MS m/z (ESI): 528.3[M+H]⁺.

### Step 5: preparation of N-((S)-2-((5-((R)-1-(5,5-difluoro-2-oxopiperidin-1-yl)-2-((2S,5S)-2,5-dimethylmorpholino)ethyl)thiazol-2-yl)amino)-1-((1r,4S)-4-methylcyclohexyl)-2-oxoethyl)-1-(ethyl-d5)-1H-pyrazole-5-carboxamide

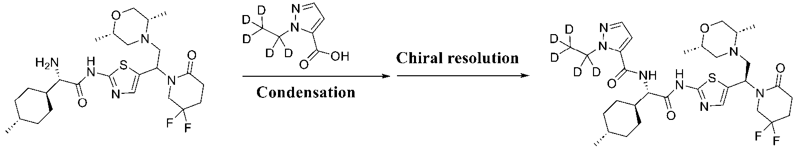

2-(ethyl-*d₅*)pyrazole-3-carboxylic acid (208 mg, 1.43 mmol) was dissolved in N,N-dimethylformamide (15 mL). HATU (544 mg, 1.43 mmol) and N,N-diisopropylethylamine (462 mg, 3.57 mmol) were sequentially added, and the mixture was stirred at room temperature for 10 minutes. (2S)-2-amino-N-(5-(1-(5,5-difluoro-2-oxopiperidin-1-yl)-2-((2S,5S)-2,5-dimethylmorpholino)ethyl)thiazol-2-yl)-2-((1r,4S)-4-methylcyclohexyl)acetamide was added, and the mixture was further stirred at room temperature for 30 minutes. The mixture was diluted with ethyl acetate, washed with a saturated sodium carbonate solution and a saturated sodium chloride solution, dried over anhydrous sodium sulphate, filtered and then concentrated under reduced pressure to remove the organic solvent, and the residue was separated by HPLC and subjected to chiral resolution to obtain the title compound (98 mg, overall yield over four steps: 21%).

¹H NMR (400 MHz, DMSO-*d₆*) δ 12.24 (s, 1H), 8.58 (d, *J* = 7.7 Hz, 1H), 7.48 (d, *J* = 2.1 Hz, 1H), 7.34 (d, *J* = 1.2 Hz, 1H), 7.01 (d, *J* = 2.1 Hz, 1H), 5.79 (t, *J* = 8.0 Hz, 1H), 4.46 (t, *J* = 8.2 Hz, 1H), 3.74-3.41 (m, 5H), 2.87 (dd, *J* = 12.9, 8.0 Hz, 1H), 2.76 (dd, *J* = 12.5, 8.2 Hz, 2H), 2.54-2.46 (m, 1H), 2.32 (dt, *J* = 16.9, 9.0 Hz, 3H), 1.82 *(t, J=* 16.2 Hz, 2H), 1.68 (s, 2H), 1.46 (d, *J* = 12.3 Hz, 1H), 1.30-1.10 (m, 4H), 1.02 (dd, *J* = 33.3, 6.4 Hz, 7H), 0.86 (t, *J* = 8.9 Hz, 5H); MS m/z (ESI): 655.3[M+H]⁺.

### Example 862 can also be synthesised according to the following steps

### Step 1: preparation of tert-butyl(5-(1-(5,5-difluoro-2-oxopiperidin-1-yl)-2-((2R,5R)-2,5-dimethylmorpholino)ethyl)thiazol-2-yl)carbamate

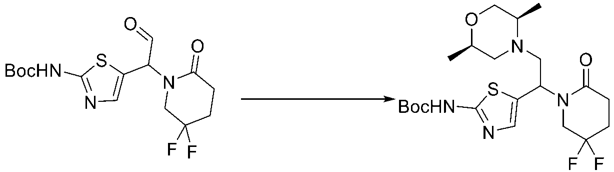

Tert-butyl(5-(1-(5,5-difluoro-2-oxopiperidin-1-yl)-2-oxoethyl)thiazol-2-yl) (1 g, 2.7 mmol) and (2R,5R)-2,5-dimethylmorpholine (1.2 g, 7.9 mmol) were dissolved in 1,2-dichloroethane (20 mL). Triethylamine (1.4 g, 13.9 mmol) was added, and the reaction mixture was heated to 30°C, and stirred and reacted for 3 hours. Acetic acid (1.2 g, 20 mmol) and sodium triacetoxyborohydride (2.8 g, 13.2 mmol) were added, and the reaction was stirred at 30°C for 2 hours. The reaction solution was concentrated, diluted with ethyl acetate, and washed sequentially with a saturated aqueous sodium carbonate solution and a saturated sodium chloride aqueous solution. The organic phase was separated, dried over anhydrous sodium sulphate, filtered and then concentrated under reduced pressure to remove the organic solvent, and the resulting residue was separated by column chromatography to obtain the title compound (1.21 g, 94%). MS m/z (ESI): 475.2 [M+H]⁺.

### Step 2: preparation of 1-(1-(2-aminothiazol-5-yl)-2-((2R,5R)-2,5-dimethylmorpholino)ethyl)-5,5-difluoropiperidin-2-one

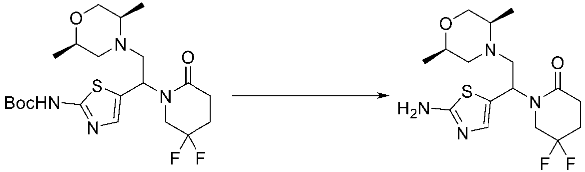

Tert-butyl(5-(1-(5,5-difluoro-2-oxopiperidin-1-yl)-2-((2R,5R)-2,5-dimethylmorpholino)ethyl)thiazol-2-yl)carbamate (1.21 g, 2.55 mmol) was dissolved in methanol (5 mL). 4 M hydrogen chloride in dioxane (15 mL) was added. The reaction solution was stirred and reacted at 45°C for 3 hours and concentrated under reduced pressure to remove the solvent, so as to obtain the title compound as a crude, which was directly used in the next reaction. MS m/z (ESI): 375.2 [M+H]⁺.

### Step 3: preparation of tert-butyl((1S)-2-((5-(1-(5,5-difluoro-2-oxopiperidin-1-yl)-2-((2R,5R)-2,5-dimethylmorpholino)ethyl)thiazol-2-yl)amino)-1-((1r,4S)-4-methylcyclohexyl)-2-oxoethyl)carbamate

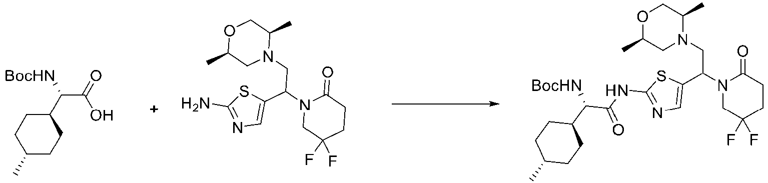

(S)-2-((tert-butoxycarbonyl)amino)-2-((trans)-4-methylcyclohexyl)acetic acid (1.38 g, 5.1 mmol) was dissolved in N,N-dimethylformamide (10 mL). HATU (1.94 g, 5.1 mmol) and N,N-diisopropylethylamine (1.66 g, 12.8 mmol) were sequentially added. The reaction was stirred at room temperature for 30 minutes. 1-(1-(2-aminothiazol-5-yl)-2-((2R,5R)-2,5-dimethylmorpholino)ethyl)-5,5-difluoropiperidin-2-one was added. The reaction solution was further stirred and reacted at room temperature for 1 hour, diluted with ethyl acetate and washed with a saturated sodium chloride aqueous solution. The organic phase was separated, dried over anhydrous sodium sulphate, filtered and then concentrated under reduced pressure to remove the organic solvent, and the resulting residue was separated by column chromatography to obtain the title compound (550 mg, overall yield over two steps: 35%). MS m/z (ESI): 628.3 [M+H]⁺.

### Step 4: preparation of (2S)-2-amino-N-(5-(1-(5,5-difluoro-2-oxopiperidin-1-yl)-2-((2R,5R)-2,5-dimethylmorpholino)ethyl)thiazol-2-yl)-2-((1r,4S)-4-methylcyclohexyl)acetamide

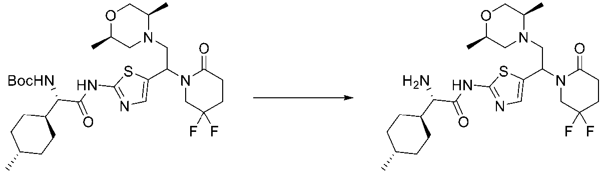

Tert-butyl((1S)-2-((5-(1-(5,5-difluoro-2-oxopiperidin-1-yl)-2-((2R,5R)-2,5-dimethylmorpholino)ethyl)thiazol-2-yl)amino)-1-((1r,4S)-4-methylcyclohexyl)-2-oxoethyl)carbamate (550 mg, 0.88 mmol) was dissolved in methanol (5 mL). 4 M hydrogen chloride in dioxane (15 mL) was added. The reaction solution was stirred and reacted at 30°C for 30 minutes and concentrated under reduced pressure to remove the solvent, so as to obtain the title compound as a crude, which was directly used in the next step. MS m/z (ESI): 528.3 [M+H]⁺.

### Step 5: preparation of N-((S)-2-((5-((R)-1-(5,5-difluoro-2-oxopiperidin-1-yl)-2-((2R,5R)-2,5-dimethylmorpholino)ethyl)thiazol-2-yl)amino)-1-((1r,4S)-4-methylcyclohexyl)-2-oxoethyl)-1-(ethyl-d5)-1H-pyrazole-5-carboxamide

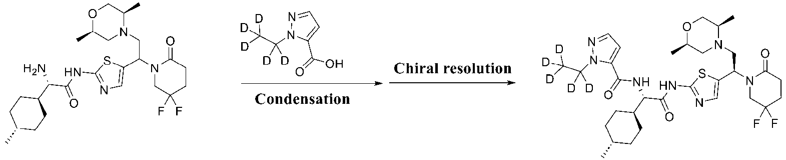

1-(ethyl-d5)-1H-pyrazole-5-carboxylic acid (255 mg, 1.76 mmol) was dissolved in N,N-dimethylformamide (7 mL). HATU (669 mg, 1.76 mmol) and N,N-diisopropylethylamine (569 mg, 4.4 mmol) were sequentially added. The reaction solution was stirred at room temperature for 25 minutes. The crude (2S)-2-amino-N-(5-(1-(5,5-difluoro-2-oxopiperidin-1-yl)-2-((2R,5R)-2,5-dimethylmorpholino)ethyl)thiazol-2-yl)-2-((1r,4S)-4-methylcyclohexyl)acetamide was added. The reaction was further stirred at room temperature for 1 hour. The reaction solution was diluted with ethyl acetate and washed with a saturated sodium chloride aqueous solution. The organic phase was separated, dried over anhydrous sodium sulphate, filtered and then concentrated under reduced pressure to remove the organic solvent, and the residue was separated by column chromatography and then subjected to chiral resolution to obtain the title compound (95 mg, overall yield over two steps: 17%).

¹H NMR (400 MHz, DMSO-*d*₆) δ 12.30 (s, 1H), 8.60 (d, *J* = 7.6 Hz, 1H), 7.48 (d, *J* = 2.0 Hz, 1H), 7.42 (s, 1H), 7.01 (d, *J* = 2.1 Hz, 1H), 5.87-5.83 (m, 1H), 4.45 (t, *J=* 8.2 Hz, 1H), 3.65-3.47 (m, 3H), 3.43-3.30 (m, 2H), 2.95-2.90 (m, 1H), 2.85-2.67 (m, 2H), 2.56-2.44 (m, 4H), 2.37-2.18 (m, 3H), 1.91-1.61 (m, 4H), 1.46 (d, *J* = 12.7 Hz, 1H), 1.36-1.11 (m, 2H), 1.06-0.96 (m, 6H), 0.91-0.80 (m, 5H); MS m/z (ESI): 655.3 [M+H]⁺.

### Example 863 can also be synthesised according to the following steps

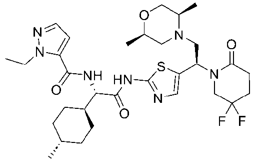

1-ethyl-1H-pyrazole-5-carboxylic acid (294 mg, 2.1 mmol) was dissolved in N,N-dimethylformamide (7 mL). HATU (798 mg, 2.1 mmol) and N,N-diisopropylethylamine (673 mg, 5.2 mmol) were sequentially added. The reaction solution was stirred at room temperature for 25 minutes. (2S)-2-amino-N-(5-(1-(5,5-difluoro-2-oxopiperidin-1-yl)-2-((2R,5R)-2,5-dimethylmorpholino)ethyl)thiazol-2-yl)-2-((1r,4S)-4-methylcyclohexyl)acetamide (631 mg, 1.05 mmol) was added, and the mixture was further stirred and reacted at room temperature for 1 hour. The reaction solution was diluted with ethyl acetate and washed with a saturated sodium chloride aqueous solution. The organic phase was separated, dried over anhydrous sodium sulphate, filtered and concentrated under reduced pressure to remove the organic solvent, and the residue was separated by column chromatography and then subjected to chiral resolution to obtain the title compound (111 mg, 16%).

¹H NMR (400 MHz, DMSO-*d*₆) δ 12.30 (s, 1H), 8.60 (d, *J= 7.7* Hz, 1H), 7.48 *(d, J =* 2.0 Hz, 1H), 7.42 *(d, J=* 1.0 Hz, 1H), 7.01 (d, *J* = 2.1 Hz, 1H), 5.88-5.82 (m, 1H), 4.63-4.41 (m, 3H), 3.67-3.48 (m, 3H), 3.48-3.30 (m, 2H), 2.99-2.90 (m, 1H), 2.85-2.64 (m, 2H), 2.55-2.45 (m, 3H), 2.39-2.17 (m, 3H), 1.91-1.60 (m, 4H), 1.46 (d, *J* = 12.7 Hz, 1H), 1.36-1.12 (m, 5H), 1.10-0.95 (m, 7H), 0.93-0.70 (m, 5H). MS m/z (ESI): 650.3 [M+H]⁺.

### Example 864 can also be synthesised according to the following steps

### Step 1: preparation of tert-butyl(5-(1-(5,5-difluoro-2-oxopiperidin-1-yl)-2-((2R,55)-2,5-dimethylmorpholino)ethyl)thiazol-2-yl)carbamate

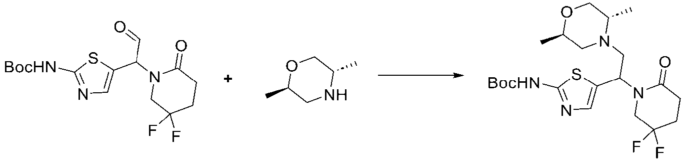

Tert-butyl N-[5-[1-(5,5-difluoro-2-oxo-1-piperidyl)-2-oxo-ethyl]thiazol-2-yl]carbamate (500 mg, 1.33 mmol) and (2R,5S)-2,5-dimethylmorpholine (460 mg, 4.00 mmol) were dissolved in a mixed solvent of acetic acid (0.8 mL) and 1,2-dichloroethane (15 mL). The mixture was stirred at 30°C for 3 hours, and sodium triacetoxyborohydride (1.41 g, 6.66 mmol) was added. The mixture was further stirred for 1 hour and concentrated under reduced pressure to remove the solvent, and the residue was dissolved in ethyl acetate. The mixture was washed with a saturated sodium carbonate solution and a saturated sodium chloride solution, dried over anhydrous sodium sulphate, filtered and then concentrated under reduced pressure to remove the organic solvent, and the residue was separated by silica gel column chromatography to obtain the title compound (199 mg, 31%). MS m/z (ESI): 475.2 [M+H]⁺.

### Step 2: preparation of 1-(1-(2-aminothiazol-5-yl)-2-((2R,5S)-2,5-dimethylmorpholino)ethyl)-5,5-difluoropiperidin-2-one

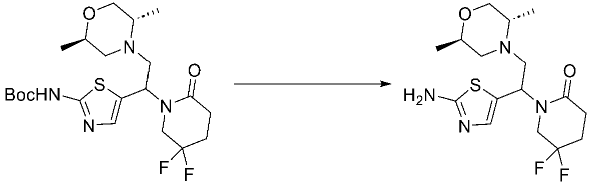

Tert-butyl(5-(1-(5,5-difluoro-2-oxopiperidin-1-yl)-2-((2R,5S)-2,5-dimethylmorpholino)ethyl)thiazol-2-yl)carbamate (199 mg, 419.33 µmol) was dissolved in 4 M hydrogen chloride in dioxane (20 mL). The reaction was transferred to 50°C, stirred for 2 hours and concentrated under reduced pressure to remove the solvent, so as to obtain the title compound crude, which was directly used in the next reaction. MS m/z (ESI): 375.2 [M+H]⁺.

### Step 3: preparation of tert-butyl((1S)-2-((5-(1-(5,5-difluoro-2-oxopiperidin-1-yl)-2-((2R,5S)-2,5-dimethylmorpholino)ethyl)thiazol-2-yl)amino)-1-((1r,4S)-4-methylcyclohexyl)-2-oxoethyl)carbamate

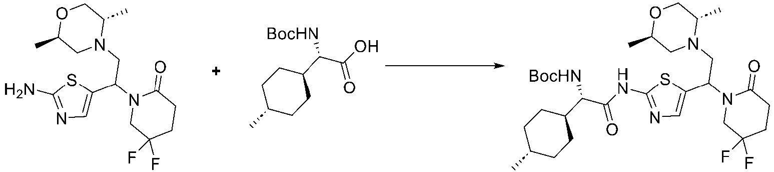

(2S)-2-(tert-butoxycarbonylamino)-2-(4-methylcyclohexyl)acetic acid (228 mg, 839 µmol) was dissolved in N,N-dimethylformamide (15 mL). HATU (319 mg, 839 µmol) and N,N-diisopropylethylamine (271 mg, 2.10 mmol) were sequentially added, and the mixture was stirred at room temperature for 20 minutes. The crude 1-(1-(2-aminothiazol-5-yl)-2-((2R,5S)-2,5-dimethylmorpholino)ethyl)-5,5-difluoropiperidin-2-one was added, and the reaction was stirred at 40°C for 1 hour. The mixture was diluted with ethyl acetate, washed with a saturated sodium carbonate solution and a saturated sodium chloride solution, dried over anhydrous sodium sulphate, filtered and then concentrated under reduced pressure to remove the organic solvent, so as to obtain the title compound as a crude, which was directly used in the next reaction. MS m/z (ESI): 628.3[M+H]⁺.

### Step 4: preparation of (2S)-2-amino-N-(5-(1-(5,5-difluoro-2-oxopiperidin-1-yl)-2-((2R,5S)-2,5-dimethylmorpholino)ethyl)thiazol-2-yl)-2-((1r,4S)-4-methylcyclohexyl)acetamide

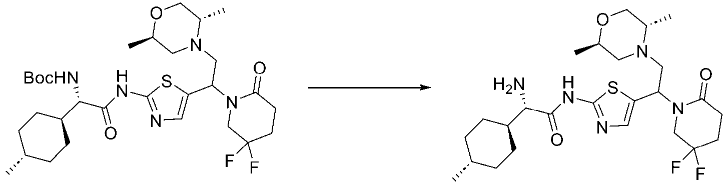

The crude tert-butyl ((1S)-2-((5-(1-(5,5-difluoro-2-oxopiperidin-1-yl)-2-((2R,5S)-2,5-dimethylmorpholino)ethyl)thiazol-2-yl)amino)-1-((1r,4S)-4-methylcyclohexyl)-2-oxoethyl)carbamate was dissolved in 4 M hydrogen chloride in dioxane (20 mL). The mixture was stirred at room temperature for 1 hour and concentrated under reduced pressure to remove the solvent, so as to obtain the title compound, which was directly used in the next reaction. MS m/z (ESI): 528.3[M+H]⁺.

### Step 5: preparation of N-((S)-2-((5-((R)-1-(5,5-difluoro-2-oxopiperidin-1-yl)-2-((2R,5S)-2,5-dimethylmorpholino)ethyl)thiazol-2-yl)amino)-1-((1r,4S)-4-methylcyclohexyl)-2-oxoethyl)-1-(ethyl-d5)-1H-pyrazole-5-carboxamide

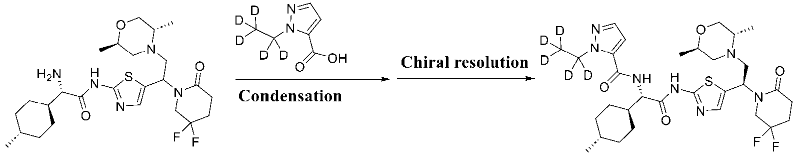

2-(ethyl-d5)pyrazole-3-carboxylic acid (121 mg, 836 µmol) was dissolved in N,N-dimethylformamide (15 mL). HATU (318 mg, 836 µmol) and N,N-diisopropylethylamine (270 mg, 2.09 mmol) were sequentially added, and the mixture was stirred at room temperature for 10 minutes. The crude (2S)-2-amino-N-(5-(1-(5,5-difluoro-2-oxopiperidin-1-yl)-2-((2R,5S)-2,5-dimethylmorpholino)ethyl)thiazol-2-yl)-2-((1r,4S)-4-methylcyclohexyl)acetamide was added, and the mixture was stirred at room temperature for 30 minutes. The mixture was diluted with ethyl acetate, washed with a saturated sodium carbonate solution and a saturated sodium chloride solution, dried over anhydrous sodium sulphate, filtered and then concentrated under reduced pressure to remove the organic solvent, and the residue was separated by HPLC and subjected to chiral resolution to obtain the title compound (92 mg, overall yield over four steps: 34%).

¹H NMR (400 MHz, DMSO-*d₆*) δ 12.30 (s, 1H), 8.59 (d, *J =* 7.7 Hz, 1H), 7.48 (d, *J* = 2.2 Hz, 2H), 7.00 (d, *J* = 2.0 Hz, 1H), 5.89 (d, *J* = 6.9 Hz, 1H), 4.45 (t, *J* = 8.1 Hz, 1H), 3.73 - 3.57 (m, 2H), 3.42 (dt, *J* = 19.4, 12.7 Hz, 3H), 3.04 (t, *J* = 10.6 Hz, 1H), 2.88 (d, *J* = 10.7 Hz, 1H), 2.57-2.43 (m, 1H),2.39-2.24 (m, 2H), 2.24 (s, 2H), 1.81 (*q, J =* 11.6 Hz, 3H), 1.68 (s, 3H), 1.46 *(d, J =* 12.6 Hz, 1H), 1.30-1.12 (m, 2H), 1.01 (d, *J =* 6.3 Hz, 4H), 0.95 (d, *J=* 6.1 Hz, 3H), 0.85 (d, *J* = 6.4 Hz, 5H); MS m/z (ESI): 655.3[M+H]⁺.

### Example 1012 can also be synthesised according to the following steps

### Step 1: preparation of tert-butyl(5-(1-(5,5-difluoro-2-oxotetrahydropyrimidin-1(2H)-yl)-2-((R)-2-methylmorpholino)ethyl)thiazol-2-yl)carbamate

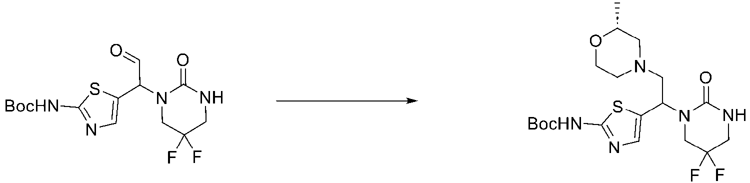

Tert-butyl(5-(1-(5,5-difluoro-2-oxotetrahydropyrimidin-1(2H)-yl)-2-oxoethyl)thiazol-2-yl)carbamate (1 g, 2.66 mmol) and (R)-2-methylmorpholine (0.81 g, 7.97 mmol) were dissolved in 1,2-dichloroethane (15 mL) and acetic acid (1.5 mL). The reaction mixture was heated to 30°C, stirred and reacted for 3 hours, and cooled to room temperature. Sodium triacetylborohydride (0.93 g, 4.38 mmol) was added, and the reaction was stirred at room temperature for 1 hour and concentrated under reduced pressure to remove the solvent. The residue was dissolved in ethyl acetate, and washed sequentially with a saturated sodium carbonate solution and a saturated sodium chloride aqueous solution. The organic phase was separated, dried over anhydrous sodium sulphate, filtered and then concentrated under reduced pressure to remove the organic solvent, and the resulting residue was separated by column chromatography to obtain the title compound (0.89 g, 72%). MS m/z (ESI): 462.2 [M+H]⁺.

### Step 2: preparation of 1-((R)-1-(2-aminothiazol-5-yl)-2-((R)-2-methylmorpholino)ethyl)-5,5-difluorotetrahydropyrimidin-2(1H)-one

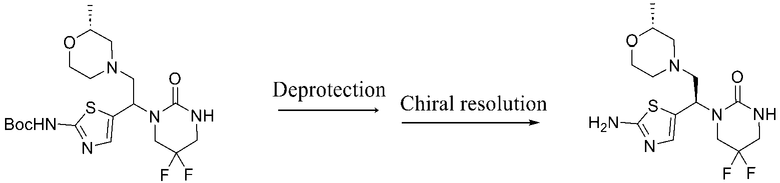

Tert-butyl(5-(1-(5,5-difluoro-2-oxotetrahydropyrimidin-1(2H)-yl)-2-((R)-2-methylmorpholino)ethyl)thiazol-2-yl)carbamate (890 mg, 1.93 mmol) was dissolved in methanol (1 mL). 4 M hydrogen chloride in dioxane (10 mL) was added. The reaction solution was stirred and reacted at room temperature for 8 hours and concentrated under reduced pressure to remove the solvent, and then the solid was dissolved in methanol. The mixture was adjusted to pH 7-8 by adding triethylamine and concentrated under reduced pressure to obtain the crude, which was subjected to chiral resolution (chromatographic column: Daicel chemical-bonded chiral column, model: ChiralPak AD-H, specification: 50 mm*250 mm, 10 µm, mobile phase: n-hexane : ethanol : methanol : diethylamine=60 : 20 : 20 : 0.1, detection wavelength: 214 nm, column temperature: 25°C, and isocratic elution for 20 min; the title compound was the first eluting peak with a retention time of 11.56 min) to obtain the title compound (260 mg, 37%).

¹H NMR (400 MHz, MeOD-*d₆*) *δ* 6.85 (d, *J* = 1.3 Hz, 1H), 5.71 (dd, *J* = 10.0, 6.0 Hz, 1H), 3.81 (ddd, *J =* 11.3, 3.4, 1.6 Hz, 1H), 3.69-3.59 (m, 1H), 3.63-3.53 (m, 2H), 3.57-3.40 (m, 2H), 3.43-3.32 (m, 1H), 2.96 *(dt, J =* 11.1, 2.0 Hz, 1H), 2.85 (dd, *J =* 12.9, 10.0 Hz, 1H), 2.76-2.61 (m, 2H), 2.30 (td, *J =* 11.4, 3.3 Hz, 1H), 1.80 (dd, *J =* 11.1, 9.8 Hz, 1H), 1.11 (d, *J =* 6.3 Hz, 3H); MS m/z (ESI): 362.1 [M+H]⁺.

### Step 3: preparation of tert-butyl((S)-2-((5-((R)-1-(5,5-difluoro-2-oxotetrahydropyrimidin-1(2H)-yl)-2-((R)-2-methylmorpholino)ethyl)thiazol-2-yl)amino)-1-((1r,4S)-4-methylcyclohexyl)-2-oxoethyl)carbamate

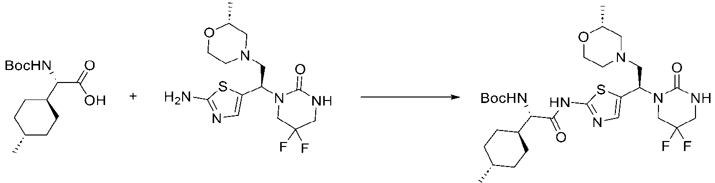

(S)-2-((tert-butoxycarbonyl)amino)-2-((trans)-4-methylcyclohexyl)acetic acid (114 mg, 0.42 mmol) was dissolved in DMF (3 mL). HATU (160 mg, 0.42 mmol) and DIPEA (274 mg, 2.12 mmol) were sequentially added. The reaction was stirred at room temperature for 10 minutes. 1-((R)-1-(2-aminothiazol-5-yl)-2-((R)-2-methylmorpholino)ethyl)-5,5-difluorotetrahydropyrimidin-2(1H)-one (138 mg, 0.38 mmol) was added. The reaction solution was further stirred and reacted at room temperature for 1 hour, diluted with ethyl acetate and washed with a saturated sodium chloride aqueous solution. The organic phase was separated, dried over anhydrous sodium sulphate, filtered and then concentrated under reduced pressure to remove the organic solvent, and the resulting residue was separated by column chromatography to obtain the title compound (160 mg, 68%). MS m/z (ESI):615.3 [M+H]⁺.

### Step 4: preparation of (S)-2-amino-N-(5-((R)-1-(5,5-difluoro-2-oxotetrahydropyrimidin-1(2H)-yl)-2-((R)-2-methylmorpholino)ethyl)thiazol-2-yl)-2-((1r,4S)-4-methylcyclohexyl)acetamide

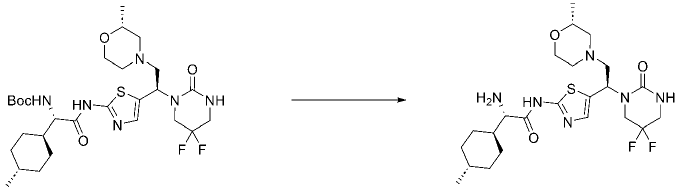

Tert-butyl((S)-2-((5-((R)-1-(5,5-difluoro-2-oxotetrahydropyrimidin-1(2H)-yl)-2-((R)-2-methylmorpholino)ethyl)thiazol-2-yl)amino)-1-((1r,4S)-4-methylcyclohexyl)-2-oxoethyl)carbamate (160 mg, 0.26 mmol) was dissolved in methanol (1.5 mL). 4 M hydrogen chloride in dioxane (5 mL) was added. The reaction solution was stirred and reacted at room temperature for 1 hour and concentrated under reduced pressure to remove the solvent, so as to obtain the title compound as a crude. MS m/z (ESI): 515.2 [M+H]⁺.

### Step 5: preparation of N-((S)-2-((5-((R)-1-(5,5-difluoro-2-oxotetrahydropyrimidin-1(2H)-yl)-2-((R)-2-methylmorpholino)ethyl)thiazol-2-yl)amino)-1-((1r,4S)-4-methylcyclohexyl)-2-oxoethyl)-1-fluorocyclopropane-1-carboxamide

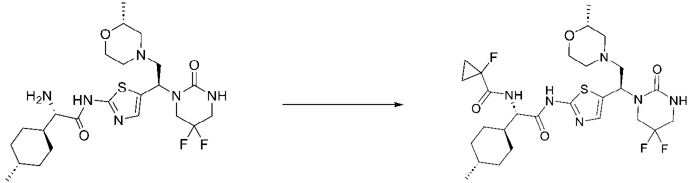

1-fluorocyclopropane-1-carboxylic acid (38 mg, 0.36 mmol) was dissolved in DMF (5 mL). HATU (137 mg, 0.36 mmol) and DIPEA (146 mg, 1.13 mmol) were sequentially added. The reaction solution was stirred at room temperature for 5 minutes. The crude (S)-2-amino-N-(5-((R)-1-(5,5-difluoro-2-oxotetrahydropyrimidin-1(2H)-yl)-2-((R)-2-methylmorpholino)ethyl)thiazol-2-yl)-2-((1r,4S)-4-methylcyclohexyl)acetamide from the previous step was added. The reaction was further stirred at room temperature for 1 hour. The reaction solution was diluted with ethyl acetate and washed with a saturated sodium chloride aqueous solution. The organic phase was separated, dried over anhydrous sodium sulphate, filtered and then concentrated under reduced pressure to remove the organic solvent, and the residue was separated by column chromatography and then separated by preparative reversed-phase HPLC to obtain the title compound (28 mg, overall yield over two steps: 18%).

¹H NMR (400 MHz, DMSO-*d₆*) *δ* 12.03 (s, 1H), 8.35 (d, *J* = 8.0 Hz, 1H), 7.35 (d, *J* = 1.2 Hz, 1H), 6.89 (d, *J* = 3.0 Hz, 1H), 5.71 (t, *J* = 7.8 Hz, 1H), 4.38 (t, *J* = 8.4 Hz, 1H), 3.80-3.67 (m, 1H), 3.63-3.36 (m, 6H), 2.90-2.75 (m, 2H), 2.75-2.62 (m, 2H), 2.15 (td, *J =*11.2*,* 3.2 Hz, 1H), 1.85-1.73 (m, 3H), 1.72-1.61 (m, 2H), 1.43 (d, *J= 12.8* Hz, 1H), 1.37-1.22 (m, 3H), 1.21-1.10 (m, 3H), 1.09-0.95 (m, 4H), 0.99-0.78 (m, 5H); MS m/z (ESI): 601.3 [M+H]⁺.

### Example 1014 can also be synthesised according to the following steps

### Step 1: preparation of tert-butyl(5-(1-(5,5-difluoro-2-oxotetrahydropyrimidin-1(2H)-yl)-2-((S)-3-methylmorpholino)ethyl)thiazol-2-yl)carbamate

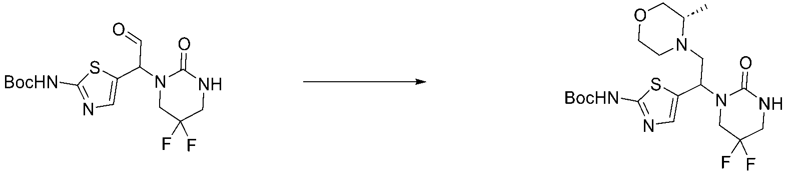

Tert-butyl(5-(1-(5,5-difluoro-2-oxotetrahydropyrimidin-1(2H)-yl)-2-oxoethyl)thiazol-2-yl)carbamate (6.10 g, 16.21 mmol) and 3-(S)-3-methylmorpholine (4.92 g, 48.62 mmol) were dissolved in ethanol (60 mL). The mixture was heated to 50°C, and stirred and reacted for 2 hours. Acetic acid (2.92 g, 48.62 mmol) and sodium cyanoborohydride (5.09 g, 81.04 mmol) were added, and the reaction was stirred at 50°C for 6 hours and concentrated under reduced pressure to remove the solvent. The residue was dissolved in ethyl acetate, and washed sequentially with a saturated aqueous sodium carbonate solution and a saturated sodium chloride aqueous solution. The organic phase was separated, dried over anhydrous sodium sulphate, filtered and then concentrated under reduced pressure to remove the organic solvent, and the resulting residue was separated by column chromatography to obtain the title compound (4.8 g, 64%). MS m/z (ESI): 462.2 [M+H]+.

### Step 2: preparation of 1-((R)-1-(2-aminothiazol-5-yl)-2-((S)-3-methylmorpholino)ethyl)-5,5-difluorotetrahydropyrimidin-2(1H)-one

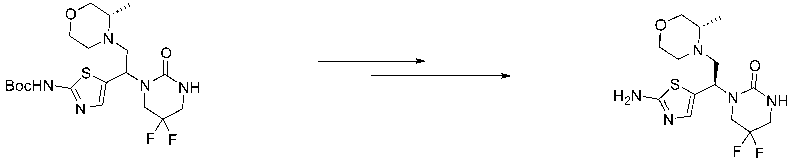

Tert-butyl(5-(1-(5,5-difluoro-2-oxotetrahydropyrimidin-1(2H)-yl)-2-((S)-3-methylmorpholino)ethyl)thiazol-2-yl)carbamate (4.8 g, 10.4 mmol) was dissolved in methanol (10 mL). 4 M hydrogen chloride in dioxane (30 mL) was added. The reaction solution was stirred and reacted at 50°C for 4 hours and concentrated under reduced pressure to remove the solvent, and the residue was subjected to chiral resolution to obtain the title compound (1.71 g, 45%). MS m/z (ESI): 362.2 [M+H]+.

### Step 3: preparation of tert-butyl((S)-2-((5-((R)-1-(5,5-difluoro-2-oxotetrahydropyrimidin-1(2H)-yl)-2-((S)-3-methylmorpholino)ethyl)thiazol-2-yl)amino)-1-((1r,4S)-4-methylcyclohexyl)-2-oxoethyl)carbamate

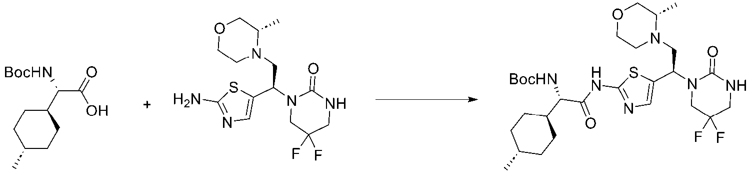

(S)-2-((tert-butoxycarbonyl)amino)-2-((trans)-4-methylcyclohexyl)acetic acid (1.54 g, 5.68 mmol) was dissolved in DMF (20 mL). HATU (2.16 g, 5.68 mmol) and DIPEA (1.83 g, 14.19 mmol) were sequentially added, and the mixture was stirred at room temperature for 10 minutes. 1-((R)-1-(2-aminothiazol-5-yl)-2-((S)-3-methylmorpholino)ethyl)-5,5-difluorotetrahydropyrimidin-2(1H)-one (1.71 g, 4.73 mmol) was added. The reaction solution was further stirred and reacted at 40°C for 1 hour. The mixture was cooled to room temperature, diluted with ethyl acetate, washed with a saturated sodium chloride aqueous solution, dried over anhydrous sodium sulphate, filtered and concentrated under reduced pressure, and the residue was separated by column chromatography to obtain the title compound (2.61 g, 90%). MS m/z (ESI):615.3 [M+H]⁺.

### Step 4: preparation of (S)-2-amino-N-(5-((R)-1-(5,5-difluoro-2-oxotetrahydropyrimidin-1(2H)-yl)-2-((S)-3-methylmorpholino)ethyl)thiazol-2-yl)-2-((1r,4S)-4-methylcyclohexyl)acetamide

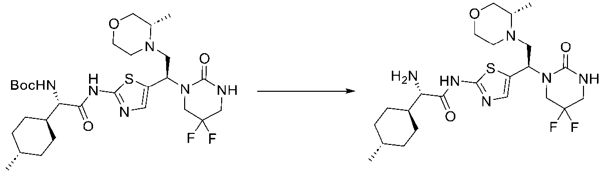

Tert-butyl((S)-2-((5-((R)-1-(5,5-difluoro-2-oxotetrahydropyrimidin-1(2H)-yl)-2-((S)-3-methylmorpholino)ethyl)thiazol-2-yl)amino)-1-((1r,4S)-4-methylcyclohexyl)-2-oxoethyl)carbamate (2.61 g, 4.25 mmol) was dissolved in dichloromethane (10 mL). 4 M hydrogen chloride in dioxane (30 mL) was added. The reaction solution was stirred and reacted at room temperature for 1 hour and concentrated under reduced pressure to remove the solvent, so as to obtain the title compound as a crude, which was directly used in the next reaction. MS m/z (ESI):515.3[M+H]⁺.

### Step 5: preparation of N-((S)-2-((5-((R)-1-(5,5-difluoro-2-oxotetrahydropyrimidin-1(2H)-yl)-2-((S)-3-methylmorpholino)ethyl)thiazol-2-yl)amino)-1-((1r,4S)-4-methylcyclohexyl)-2-oxoethyl)-1-fluorocyclopropane-1-carboxamide

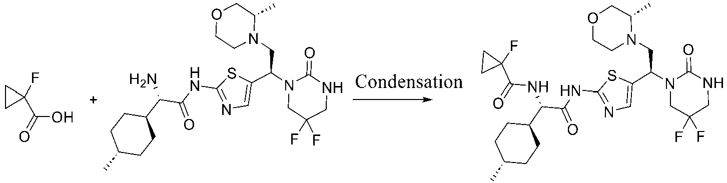

1-fluorocyclopropanecarboxylic acid (492 mg, 4.72 mmol) was dissolved in DMF (20 mL). HATU (1.79 g, 4.72 mmol) and DIPEA (1.52 g, 11.79 mmol) were sequentially added. The reaction solution was stirred at room temperature for 5 minutes. The crude (S)-2-amino-N-(5-((R)-1-(5,5-difluoro-2-oxotetrahydropyrimidin-1(2H)-yl)-2-((S)-3-methylmorpholino)ethyl)thiazol-2-yl)-2-((1r,4S)-4-methylcyclohexyl)acetamide was added. The reaction was further stirred at room temperature for 1 hour. The reaction solution was diluted with ethyl acetate, washed with a saturated sodium chloride aqueous solution, dried over anhydrous sodium sulphate, filtered and concentrated under reduced pressure, and the residue was separated by column chromatography to obtain the title compound (2.11 g, overall yield over two steps: 83%).

¹H NMR (400 MHz, DMSO-*d₆*) δ 12.20 (s, 1H), 8.37 (d, *J =* 8.1 Hz, 1H), 7.39 (d, *J* = 1.1 Hz, 1H), 6.88 (d, *J =* 3.1 Hz, 1H), 5.71 (dd, *J =* 10.2, 5.8 Hz, 1H), 4.37 (t, *J= 8.4* Hz, 1H), 3.70-3.36 (m, 8H), 3.22-3.07 (m, 2H), 2.87-2.79 (m, 1H), 2.48-2.36 (m, 1H), 2.25-2.18 (m, 1H), 1.85-1.72 (m, 2H), 1.70-1.61 (m, 2H), 1.47-1.06 (m, 7H), 1.01-0.92 (m, 4H), 0.89-0.77 (m, 5H); MS m/z (ESI): 601.3 [M+H]⁺.

The following examples can also be synthesised with reference to Examples 327, 574, 624 or 696, wherein the ¹H NMR data are as shown in the table below:

| Example | ¹H NMR |
|---|---|
| 628 | ¹H NMR (400 MHz, DMSO-*d₆*) δ 12.26 (s, 1H), 8.55 (d, *J =* 7.7 Hz, 1H), 7.48 (d, *J =* 2.0 Hz, 1H), 7.39 (s, 1H), 7.00 (d, *J* = 2.1 Hz, 1H), 5.89 (t, *J* = 8.1 Hz, 1H), 4.46 (t, *J =* 8.2 Hz, 1H), 3.72 (d, *J* = 11.5 Hz, 1H), 3.63 (dd, *J =* 16.5, 11.7 Hz, 1H), 3.52-3.35 (m, 3H), 2.97-2.81 (m, 1H), 2.80-2.66 (m, 3H), 2.37-2.23 (m, 3H), 2.06 (td, *J =* 11.3, 3.2 Hz, 1H), 1.85 (dd, *J =* 19.4, 9.4 Hz, 3H), 1.68 (s, 2H), 1.46 (d, *J =* 12.6 Hz, 1H), 1.38-1.11 (m, 3H), 1.04 (d, *J* = 6.3 Hz, 4H), 0.85 (d, *J* = 6.5 Hz, 5H) |
| 634 | ¹H NMR (400 MHz, DMSO-*d*₆) δ 12.35 (s, 1H), 8.60 (d, *J* = 7.6 Hz, 1H), 7.93 (d, *J* = 2.9 Hz, 1H), 7.62-7.33 (m, 3H), 7.01 (d, *J* = 2.1 Hz, 1H), 6.47 (d, *J* = 9.7 Hz, 1H), 6.20 (s, 1H), 4.56-4.32 (m, 3H), 3.25-3.18 (m, 4H), 3.15-3.10 (m, 1H), 2.97-2.92 (m, 1H), 2.75-2.65 (m, 3H), 2.28-2.13 (m, 2H), 1.91-1.60 (m, 7H), 1.44 (d, *J* = 12.7 Hz, 1H), 1.35-1.15 (m, 5H), 1.07-0.98 (m, 1H), 0.91-0.79 (m, 5H). |
| 644 | ¹H NMR (400 MHz, DMSO-*d₆*) *δ* 12.38 (s, 1H), 8.60 (d, *J* = 7.6 Hz, 1H), 7.97 (d, *J* = 3.0 Hz, 1H), 7.52 (s, 1H), 7.48 (d, *J* = 2.1 Hz, 1H), 7.47-7.43 (m, 1H), 7.00 (d, *J =* 2.0 Hz, 1H), 6.49-6.43 (m, 1H), 4.46-4.39 (m, 3H), 3.43-3.29 (m, 2H), 2.78-2.71 (m, 1H), 2.17 (s, 6H), 1.88-1.80 (m, 1H), 1.79-1.72 (m, 1H), 1.71-1.69 (m, 2H), 1.49-1.38 (m, 1H), 1.27-1.21 (m, 4H), 1.20-1.13 (m, 1H), 1.09-0.97 (m, 1H), 0.82-0.76 (m, 5H). |
| 646 | ¹H NMR (400 MHz, DMSO-*d₆*) δ 12.24 (s, 1H), 8.57 (d, *J =* 7.7 Hz, 1H), 7.48 (d, *J =* 2.0 Hz, 1H), 7.36 (d, *J* = 1.1 Hz, 1H), 7.01 (d, *J* = 2.1 Hz, 1H), 5.84 (t, *J* = 8.0 Hz, 1H), 4.51-4.38 (m, 3H), 3.70-3.55 (m, 1H), 3.45 (q, *J* = 12.5 Hz, 1H), 3.21 (s, 3H), 3.15 (dt, *J* = 8.8, 4.6 Hz, 1H), 2.82 (dd, *J* = 12.8, 8.9 Hz, 1H), 2.75-2.64 (m, 3H), 2.35-2.11(m, 4H), 1.89-1.73 (m, 4H), 1.68 (s, 2H), 1.52-1.32 (m, 3H), 1.33-0.95 (m, 7H), 0.85 (d, *J* = 6.5 Hz, 6H). |
| 648 | ¹H NMR (400 MHz, DMSO-*d₆*) δ 12.19 (s, 1H), 8.56 (d, *J* = 7.7 Hz, 1H), 7.48 (d, *J =* 2.0 Hz, 1H), 7.39 (d, *J* = 1.1 Hz, 1H), 7.01 (d, *J* = 2.0 Hz, 1H), 5.78 (t, *J* = 7.9 Hz, 1H), 4.44 (dt, *J =* 12.1, 7.4 Hz, 3H), 3.86 (tt, *J* = 6.9, 3.4 Hz, 1H), 3.64 (ddd, *J* = 17.9, 13.0, 10.1 Hz, 1H), 3.45 (*q, J =* 12.2 Hz, 1H), 3.15 (s, 3H), 3.01-2.76 (m, 3H), 2.60 (q, *J* = 7.6 Hz, 1H), 2.47 (d, *J* = 3.3 Hz, 1H), 2.28 (tt, *J* = 14.7, 7.2 Hz, 2H), 1.94 (*dq, J =* 14.2, 7.3 Hz, 1H), 1.88-1.72 (m, 2H), 1.73-1.58 (m, 3H), 1.46 (d, *J* = 12.7 Hz, 1H), 1.37-0.96 (m, 8H), 0.86 (t, *J =* 9.2 Hz, 6H). |
| 650 | ¹H NMR (400 MHz, DMSO-*d₆*) δ 12.27 (s, 1H), 8.57 (d, *J* = 7.6 Hz, 1H), 7.48 (d, *J =* 2.0 Hz, 1H), 7.39 (s, 1H), 7.00 (d, *J* = 2.0 Hz, 1H), 5.80 (t, *J =* 8.0 Hz, 1H), 4.49 -4.38 (m, 3H), 3.86 (dt, *J* = 6.9, 3.6 Hz, 1H), 3.72-3.57 (m, 1H), 3.44 (q, *J* = 12.2 Hz, 1H), 3.15 (s, 3H), 2.99 (dd, *J* = 12.4, 8.9 Hz, 1H), 2.87-2.72 (m, 2H), 2.64-2.50 (m, 2H), 2.26 (dq, *J* = 15.3, 7.0 Hz, 2H), 1.93 (dt, *J* = 13.5, 7.2 Hz, 1H), 1.81 (dd, *J* = 22.2, 11.4 Hz, 2H), 1.67 (s, 3H), 1.46 (d, *J* = 12.6 Hz, 1H), 1.37-0.95 (m, 8H), 0.85 (d, *J* = 6.4 Hz, 6H). |
| 656 | ¹H NMR(400 MHz, DMSO-*d₆*) *δ*12.29 (s, 1H), 8.59 (d, *J =* 7.6 Hz, 1H), 7.48 (d, *J =* 2.0 Hz, 1H), 7.39 (s, 1H), 7.01 (d, *J* = 2.0 Hz, 1H), 5.85 (t, *J =* 8.0 Hz, 1H), 4.43 (m, 3H), 3.69-3.55 (m, 1H), 3.50-3.39 (m, 1H), 2.90-2.78 (m, 1H), 2.69-2.53 (m, 2H), 2.37-2.22 (m, 2H), 2.20 (s, 6H), 1.87-1.73 (m, 2H), 1.72-1.61 (m, 2H), 1.51-1.39 (m, 1H), 1.37-1.21 (m, 4H), 1.20-1.13(m, 1H), 1.12-0.90 (m, 2H), 0.89-0.77 (m, 5H). |
| 663 | ¹H NMR (400 MHz, DMSO-*d₆*) δ12.35 (s, 1H), 12.07 (s, 1H), 8.60 (d, *J* = 7.6 Hz, 1H), 7.92 (*d, J =* 2.9 Hz, 1H), 7.52-7.40 (m, 3H), 7.01 (d, *J* = 2.0 Hz, 1H), 6.47 (d, *J =* 9.7 Hz, 1H), 6.21 (s, 1H), 4.53-4.33 (m, 3H), 3.22 (dd, *J =* 13.1, 9.1 Hz, 1H), 2.94 (dd, *J =* 13.1, 6.5 Hz, 1H), 2.85-2.75 (m, 2H), 2.21-2.11 (m, 2H), 2.10-2.00 (m, 1H), 1.88-1.58 (m, 6H), 1.47-1.35 (m, 3H), 1.31-1.10 (m, 5H), 1.09- 0.96 (m, 1H), 0.91-0.78 (m, 5H). |
| 700 | ¹H NMR(400 MHz, DMSO-*d₆*) *δ* 12.23(s, 1H), 8.76 (d, *J* = 8.0 Hz, 1H), 7.36 (d, *J =* 1.2 Hz, 1H), 6.86 (d, *J* = 3.0 Hz, 1H), 5.71 (t, *J* =8.0 Hz, 1H), 4.39 (dd, *J =* 47.5, 4.5 Hz, 2H), 4.35 (t, *J* = 8.4 Hz, 1H), 3.85-3.75 (m, 1H), 3.72-3.41 (m, 5H), 3.40-3.32 (m, 1H), 2.90-2.81 (m, 2H), 2.78-2.65 (m, 2H), 2.22 (td, *J* =11.2, 3.2 Hz, 1H), 1.98 (*t, J =* 10.6 Hz, 1H), 1.85-1.71 (m, 2H), 1.70-1.52 (m, 3H), 1.45-1.34 (m, 1H), 1.32-1.20 (m, 1H), 1.19-1.05 (m, 1H), 1.01-0.88 (m, 1H), 0.87-0.75 (m, 5H), 0.69- 0.60 (m, 4H) |
| 703 | ¹H NMR (400 MHz, DMSO-*d*₆) δ 12.15 (s, 1H), 8.36 (d, *J* = 8.1 Hz, 1H), 7.35 (d, *J* = 1.1 Hz, 1H), 6.90 (d, *J* = 3.4 Hz, 1H), 5.73 (t, *J* = 8.1 Hz, 1H), 4.53-4.43 (m, 1H), 4.40-4.33 (m, 2H), 3.83-3.81 (m, 1H), 3.70-3.45 (m, 4H), 3.40-3.33 (m, 1H), 2.90-2.73 (m, 4H), 2.17-2.01 (m, 2H), 1.81-1.64 (m, 4H), 1.49-1.05 (m, 8H), 1.02-0.78 (m, 6H) |
| 704 | ¹H NMR (400 MHz, DMSO-*d₆*) *δ* 12.18 (s, 1H), 8.36 (d, *J =* 8.2 Hz, 1H), 7.35 (s, 1H), 6.90 (s, 1H), 5.72 (t, *J* = 8.2 Hz, 1H), 4.37 (t, *J* = 8.4 Hz, 2H), 3.62-3.51 (m, 2H), 3.50-3.44 (m, 2H), 3.42-3.36 (m, 1H), 3.28-3.21 (m, 1H), 3.03-2.98 (m, 1H), 2.97-2.89 (m, 2H), 2.87-2.79 (m, 1H), 2.73-2.66 (m, 1H), 2.19-2.12 (m, 1H), 1.84-1.77 (m, 3H), 1.76-1.71 (m, 2H), 1.70-1.62 (m, 2H), 1.46-1.36 (m, 1H), 1.36-1.23 (m, 3H), 1.18-1.13 (m, 5H), 0.89-0.82 (m, 6H) |
| 718 | ¹H NMR (400 MHz, DMSO-*d*₆) δ 12.18 (s, 1H), 8.36 (d, *J* = 8.1 Hz, 1H), 7.34 (d, *J =* 1.1 Hz, 1H), 6.90 (d, *J* = 2.9 Hz, 1H), 5.91-5.36 (m, 1H), 4.38 (t, *J* = 8.4 Hz, 1H), 3.63-3.45 (m, 5H), 3.37-3.33 (m, 1H), 2.95-2.79 (m, 3H), 2.85-2.79 (m, 1H), 2.64-2.59 (m, 1H), 2.43-2.33 (m, 3H), 2.18-2.16 (m, 1H), 1.81-1.64 (m, 4H), 1.44-1.24 (m, 3H), 1.19-1.04 (m, 8H), 0.95-0.73 (m, 5H) |
| 721 | ¹H NMR (400 MHz, DMSO-*d₆*) *δ* 12.18 (s, 1H), 8.37 (d, *J =* 8.0 Hz, 1H), 7.34 (s, 1H), 6.91 (s, 1H), 5.70 (t, *J* = 8.2 Hz, 1H), 4.37 (t, *J* = 8.4 Hz, 1H), 3.64-3.59 (m, 2H), 3.58-3.45 (m, 2H), 2.95-2.89 (m, 1H), 2.88-2.81 (m, 1H), 2.74-2.66 (m, 1H), 2.57-2.51 (m, 1H), 2.47-2.39 (m, 1H), 1.83-1.76 (m, 2H), 1.70-1.63 (m, 2H), 1.45-1.39 (m, 1H), 1.35-1.25 (m, 3H), 1.21-1.11 (m, 5H), 1.01-1.89 (m, 1H), 0.89-0.78 (m, 6H), 0.66-0.55 (m, 2H), 0.54-0.49 (m, 1H), 0.48-0.42 (m, 1H) |
| 723 | ¹H NMR (400 MHz, DMSO-*d₆*) *δ* 12.18 (s, 1H), 8.36 (d, *J =* 8.2 Hz, 1H), 7.41 (s, 1H), 6.85 (s, 1H), 5.55 (t, *J =* 7.8 Hz, 1H), 4.38 (t, *J =* 8.4 Hz, 1H), 3.68-3.57 (m, 2H), 3.55-3.47 (m, 2H), 3.47-3.39 (m, 2H), 3.27-3.22 (m, 1H), 3.19-3.13 (m, 1H), 2.95-2.83 (m, 1H), 2.73-2.65 (m, 1H), 2.63-2.56 (m, 1H), 1.83-1.74 (m, 2H), 1.70-1.59 (m, 2H), 1.46-1.38 (m, 1H), 1.36-1.33 (m, 1H), 1.31-1.27 (m, 1H), 1.25-1.22 (m, 2H), 1.21-1.11 (m, 3H), 1.01-0.93 (m, 7H), 0.89-0.79 (m, 5H) |
| 724 | ¹H NMR (400 MHz, DMSO-*d₆*) δ 12.13 (s, 1H), 8.37 (dd, *J =* 8.2, 1.7 Hz, 1H), 7.34 (d, *J =* 1.2 Hz, 1H), 6.91 (d, *J =* 2.8 Hz, 1H), 5.72 (t, *J =* 8.0 Hz, 1H), 4.38 (t, *J=* 8.4 Hz, 1H), 3.66-3.44 (m, 6H), 2.87-2.65 (m, 4H), 1.86 - 1.60 (m, 6H), 1.53-1.39 (m, 1H), 1.37 -1.10 (m, 7H), 1.04 (dd, *J =* 6.3, 4.0 Hz, 6H), 0.99-0.84 (m, 5H). |
| 725 | ¹H NMR (400 MHz, DMSO-*d₆*) δ 12.20 (s, 1H), 8.72 (d, *J =* 7.8 Hz, 1H), 7.28 (s, 1H), 6.82 (d, *J* = 3.3 Hz, 1H), 5.65 (t, *J =* 8.0 Hz, 1H), 4.27 (t, *J =* 8.2 Hz, 1H), 3.55 - 3.35 (m, 6H), 2.88-2.57 (m, 4H), 1.90-1.47 (m, 7H), 1.33 (d, *J =* 12.6 Hz, 1H), 1.26- 1.02 (m, 3H), 0.97 (dd, *J =* 6.3, 4.0 Hz, 6H), 0.91-0.68 (m, 5H), 0.63-0.54 (m, 4H) |
| 726 | ¹H NMR(400 MHz, DMSO-*d₆*) *δ* 12.12 (s, 1H), 8.37 (d, *J =* 8.0 Hz, 1H), 7.34 (d, *J* = 1.2 Hz, 1H), 6.93 (d, *J* = 3.0 Hz, 1H), 5.71 (t, *J* = 8.0 Hz, 1H), 4.38 (t, *J* = 8.4 Hz, 1H), 3.92-3.84(m, 2H), 3.63-3.40 (m, 4H) , 2.81-2.70 (m, 1H), 2.68-2.59 (m, 1H), 2.58-2.53 (m, 2H), 2.20-2.07 (m, 2H), 1.87-1.73 (m, 2H), 1.71-1.60 (m, 2H), 1.47-1.38 (m, 1H), 1.36-1.22 (m, 3H), 1.21-1.13 (m, 3H), 1.12-1.07 (m, 6H), 0.96-0.92 (m, 1H), 0.91-0.86 (m, 1H), 0.86-0.75(m, 4H) |
| 727 | ¹H NMR (400 MHz, DMSO-*d₆*) *δ* 12.25 (s, 1H), 8.80 (d, *J =* 8.0 Hz, 1H), 7.34 (d, *J* = 1.2 Hz, 1H), 6.93 (d, *J =* 3.0 Hz, 1H), 5.70 (t, *J =* 8.0 Hz, 1H), 4.35 (t, *J* = 8.4 Hz, 1H), 3.92-3.81 (m, 2H), 3.62-3.43 (m, 4H), 2.81-2.71 (m, 1H), 2.68-2.59 (m, 1H), 2.58-2.52 (m, 2H), 2.18-2.08 (m, 2H), 1.84-1.72 (m, 2H), 1.71-1.54 (m, 3H), 1.46-1.37 (m, 1H), 1.32-1.21 (m, 1H), 1.20-1.07 (m, 7H), 1.01-0.93 (m, 1H), 0.90-0.78 (m, 5H), 0.70-0.56 (m, 4H) |
| 730 | ¹H NMR (400 MHz, DMSO-*d₆*) δ 12.16 (s, 1H), 8.36 (d, *J =* 8.1 Hz, 1H), 7.33 (d, *J =* 1.1 Hz, 1H), 6.90 (d, *J =* 2.9 Hz, 1H), 5.97-5.59 (m, 1H), 4.37 (t, *J =* 8.4 Hz, 1H), 3.92-3.88 (m, 2H), 3.65-3.45 (m, 3H), 3.39-3.31 (m, 2H), 2.85-2.79 (m, 1H), 2.61-2.56 (m, 1H), 2.44-2.42 (m, 2H), 2.26-2.22 (m, 2H), 1.80-1.64 (m, 5H), 1.44-1.24 (m, 4H), 1.21-1.03 (m, 8H), 0.95-0.79 (m, 5H) |
| 731 | ¹H NMR (400 MHz, DMSO-*d₆*) *δ* 12.22 (s, 1H), 8.36 (d, *J =* 8.2 Hz, 1H), 7.42 (s, 1H), 6.87 (s, 1H), 5.78-5.70 (m, 1H), 4.37 (t, *J =* 8.4 Hz, 1H), 3.65-3.60 (m, 1H), 3.59 -3.41 (m, 4H), 3.40-3.35 (m, 1H), 3.07 (t, *J =* 10.6 Hz, 1H), 2.99-2.88 (m, 2H), 2.38-2.31 (m, 1H), 2.29-2.20 (m, 1H), 1.85-1.75 (m, 3H), 1.70-1.62 (m, 2H), 1.45-1.39 (m, 1H), 1.36-1.32 (m, 1H), 1.31-1.22 (m, 3H), 1.21-1.11 (m, 4H), 1.02 (d, *J =* 6.2 Hz, 3H), 0.96 (d, *J =* 6.2 Hz, 3H), 0.91-0.86 (m, 1H), 0.84 (d, *J =* 6.4 Hz, 3H) |
| 734 | ¹H NMR (400 MHz, DMSO-*d₆*) *δ* 12.14 (s, 1H), 8.36 (d, *J* = 8.2 Hz, 1H), 7.30 (d, *J* = 1.2 Hz, 1H), 6.90 (d, *J =* 3.0 Hz, 1H), 5.61 (t, *J* = 8.0 Hz, 1H), 4.38 (t, *J* = 8.4 Hz, 1H), 3.68-3.57 (m, 2H), 3.56-3.42 (m, 5H), 2.95 -2.77 (m, 2H), 2.75-2.64 (m, 1H), 2.49-2.45 (m, 1H), 2.39-2.30 (m, 1H), 1.86-1.73 (m, 2H), 1.72-1.58(m, 2H), 1.48-1.37(m, 1H), 1.36- 1.22 (m, 3H), 1.21-1.10(m, 3H), 1.07 (d, *J* = 6.2 Hz, 3H), 0.99 (d, *J =* 6.4 Hz, 3H), 0.96-0.89 (m, 1H), 0.88-0.86 (m, 1H), 0.85-0.76 (m, 4H) |
| 739 | ¹H NMR (400 MHz, DMSO-*d₆*) *δ* 12.19 (s, 1H), 8.36 (d, *J =* 8.2 Hz, 1H), 7.40 (s, 1H), 6.87 (s, 1H), 5.63 (t, *J =* 8.2 Hz, 1H), 4.37 (t, *J =* 8.4 Hz, 1H), 3.70-3.61 (m, 1H), 3.59-3.53 (m, 2H), 3.61-3.40 (m, 3H), 3.28-3.22 (m, 2H), 3.09-3.02 (m, 1H), 2.88-2.81 (m, 2H), 2.70-2.64 (m, 1H), 1.83-1.76 (m, 2H), 1.71-1.62 (m, 2H), 1.45-1.39 (m, 1H), 1.36-1.31 (m, 1H), 1.30-1.27 (m, 1H), 1.27-1.22 (m, 1H), 1.20-1.11 (m, 3H), 0.99-0.93 (m, 7H), 0.88-0.79 (m, 5H) |
| 743 | ¹H NMR (400 MHz, DMSO-*d₆*) δ 12.20 (s, 1H), 8.38 (d, *J =* 8.4 Hz, 1H), 7.33 (d, *J* = 1.2 Hz, 1H), 6.94 (d, *J =* 2.7 Hz, 1H), 5.80 -5.60 (m, 1H), 4.38 (t, *J =* 8.4 Hz, 1H), 4.20 (t, *J =* 6.2 Hz, 2H), 3.62 -3.42 (m, 4H), 2.82-2.74 (m, 1H), 2.71 (d, *J* = 10.4 Hz, 1H), 2.64-2.57 (m, 2H), 2.34 (d, *J=* 8.7 Hz, 1H), 2.18 (d, *J* = 8.7Hz, 1H), 1.91-1.73 (m, 4H), 1.72-1.60 (m, 4H), 1.42 (d, *J =* 12.7 Hz, 1H), 1.36-1.06 (m, 7H), 1.02-0.84 (m, 5H). |
| 746 | ¹H NMR(400 MHz, DMSO-*d₆*) *δ* 12.15 (s, 1H), 8.30 (d, *J =* 8.0 Hz, 1H), 7.34 (d, *J* = 1.2 Hz, 1H), 6.83 (d, *J =* 3.0 Hz, 1H), 5.50 (t, *J =* 8.0 Hz, 1H), 4.42-4.33 (m, 2H), 3.86-3.81(m, 1H), 3.65-3.44 (m, 5H), 3.43-3.38 (m, 1H), 3.07-2.98 (m, 1H), 2.95-2.86 (m, 1H), 2.84-2.73 (m, 1H), 2.53-2.51(m, 1H),1.86-1.62 (m, 5H), 1.61-1.55 (m, 1H), 1.48-1.38 (m, 1H), 1.37-1.23 (m, 3H), 1.22-1.09 (m, 3H), 1.03-0.90 (m, 1H), 0.88-0.86 (m, 1H), 0.85-0.76 (m, 4H) |
| 747 | ¹H NMR(400 MHz, DMSO-*d₆*) *δ* 12.11 (s, 1H), 8.30 (d, *J =* 8.2 Hz, 1H), 7.33 (d, *J =* 1.2 Hz, 1H), 6.83 (d, *J =* 3.0 Hz, 1H), 5.50 (t, *J =* 8.0 Hz, 1H), 4.42-4.33 (m, 2H), 3.86-3.81 (m, 1H), 3.63-3.44 (m, 5H), 3.43-3.38 (m, 1H),3.06-2.98 (m, 1H), 2.91-2.86 (m, 1H), 2.81-2.75 (m, 1H), 2.53-2.51(m, 1H), 1.87-1.61 (m, 5H), 1.60-1.52 (m, 1H), 1.49-1.39 (m, 1H), 1.38-1.22 (m, 3H), 1.21-1.08 (m, 3H), 1.04-0.90 (m, 1H), 0.89-0.85 (m, 1H), 0.85-0.76 (m, 4H) |
| 760 | ¹H NMR(400 MHz, DMSO-*d₆*) *δ* 12.18 (s, 1H), 8.36 (d, *J =* 8.2 Hz, 1H), 7.33 (d, *J* = 1.2 Hz, 1H), 6.89 (d, *J =* 3.0 Hz, 1H), 5.67 (t, *J =* 8.0 Hz, 1H), 4.81-4.56 (m, 1H), 4.38 (t, *J =* 8.4 Hz, 1H), 3.65-3.43(m, 4H), 2.85-2.70 (m, 2H), 2.68-2.55 (m, 2H), 2.47-2.32 (m, 2H), 1.94-1.76 (m, 4H), 1.75-1.56 (m, 4H), 1.47-1.37 (m, 1H), 1.36-1.22 (m, 3H), 1.22-1.06 (m, 3H), 1.05-0.92(m, 1H), 0.92-0.74 (m, 5H) |
| 764 | ¹H NMR (400 MHz, DMSO-*d₆*) *δ* 12.20 (s, 1H), 8.36 (d, *J =* 8.2 Hz, 1H), 7.39 (d, *J =* 1.1 Hz, 1H), 6.89 (s, 1H), 5.69 (t, *J =* 7.9 Hz, 1H), 4.37 (t, *J =* 8.4 Hz, 1H), 3.71-3.44 (m, 3H), 3.44 -3.35 (m, 1H), 3.17 (dd, *J =* 13.3, 9.8 Hz, 1H), 3.03 (d, *J* = 11.5 Hz, 1H), 2.71-2.60 (m, 2H), 2.42-2.29 (m, 1H), 2.13-1.57 (m, 8H), 1.44-1.40 (m, 1H),1.36-1.07 (m, 10H),1.01-0.84 (m, 5H). |
| 767 | ¹H NMR(400 MHz, DMSO-*d₆*) *δ* 12.17 (s, 1H), 8.37 (d, *J =* 8.0 Hz, 1H), 7.33 (d, *J* = 1.2 Hz, 1H), 6.87 (d, *J =* 3.0 Hz, 1H), 5.68 (t, *J* = 7.8 Hz, 1H), 4.38 (t, *J* = 8.0 Hz, 1H),4.27 (dd, *J =* 47.6, 5.4 Hz, 2H), 3.65-3.43 (m, 4H), 3.01-2.86(m, 2H), 2.81-2.67 (m, 2H), 2.13-1.92(m, 2H), 1.84-1.71 (m, 2H), 1.70-1.56 (m, 5H), 1.46-1.37 (m, 1H), 1.36-1.24 (m, 3H), 1.23-1.07 (m, 5H), 1.04-0.87 (m, 2H), 0.86-0.77 (m, 4H) |
| 770 | ¹H NMR (400 MHz, DMSO-*d₆*) δ 12.18 (s, 1H), 8.52-8.05 (m, 1H), 7.33 (d, *J* = 1.2 Hz, 1H), 6.89 (s, 1H), 5.68 (t, *J* = 7.8 Hz, 1H), 4.37 (t, *J =* 8.4 Hz, 1H), 3.68 -3.42 (m, 4H), 3.03-2.89 (m, 2H), 2.81-2.67 (m, 2H), 2.09-1.94 (m, 2H), 1.84-1.58 (m, 8H), 1.48-1.08 (m, 10H), 1.02-0.84 (m, 5H) |
| 777 | ¹H NMR (400 MHz, DMSO-*d₆*) δ 12.17 (s, 1H), 8.37 (d, *J* = 8.1 Hz, 1H), 7.31 (d, *J* = 1.2 Hz, 1H), 6.89 (d, *J* = 3.0 Hz, 1H), 5.65 (t, *J* = 7.9 Hz, 1H), 4.38 (t, *J* = 8.4 Hz, 1H), 3.64 -3.44 (m, 3H), 3.19-3.12 (m, 1H), 2.82-2.65 (m, 4H), 2.27-2.11 (m, 2H), 1.87-1.73 (m, 4H), 1.67 (t, *J =* 11.2 Hz, 2H), 1.47-1.06 (m, 13H), 1.04-0.77 (m, 6H). |
| 780 | ¹H NMR (400 MHz, DMSO-*d₆*) δ 12.19 (s, 1H), 8.37 (dd, *J* = 8.1, 1.6 Hz, 1H), 7.33 (d, *J* = 1.2 Hz, 1H), 6.91 (s, 1H), 5.68 (t, *J* = 8.0 Hz, 1H), 4.39 (t, *J=* 8.4 Hz, 1H), 3.72 -3.44 (m, 3H), 3.20-3.09 (m, 1H), 2.86-2.66 (m, 4H), 2.21-2.09 (s, 2H), 1.89-1.73 (m, 4H), 1.74-1.63 (m, 2H), 1.54 - 1.08 (m, 10H), 1.05-0.84 (m, 6H) |
| 781 | ¹H NMR (400 MHz, DMSO-*d₆*) *δ* 12.19 (s, 1H), 8.36 (d, *J =* 8.2 Hz, 1H), 7.35 (s, 1H), 6.88 (s, 1H), 5.66 (t, *J* = 8.0 Hz, 1H), 4.82-4.65 (m, 1H), 4.37 (t, *J* = 8.4 Hz, 1H), 3.65 -3.53 (m, 1H), 3.50-3.42 (m, 2H), 3.41-3.35 (m, 1H), 2.97-2.83 (m, 2H), 2.82-2.76 (m, 1H), 2.64-2.57 (m, 1H), 2.38-2.30 (m, 1H), 1.84-1.72 (m, 3H), 1.72-1.59 (m, 4H), 1.45-1.38 (m, 1H), 1.35-1.32 (m, 1H), 1.31-1.28 (mz, 1H), 1.27-1.23 (m, 1H), 1.21-1.11 (m, 3H), 1.01-0.92 (m, 1H), 0.90-0.76 (m, 6H) |
| 782 | ¹H NMR (400 MHz, DMSO-*d₆*) *δ* 12.19 (s, 1H), 8.36 (d, *J* = 8.2 Hz, 1H), 7.33 (s, 1H), 6.90 (s, 1H), 5.67 (t, *J =* 7.8 Hz, 1H), 4.82-4.65 (m, 1H), 4.38 (t, *J =* 8.4 Hz, 1H), 3.60-3.52 (m, 1H), 3.51-3.43 (m, 2H), 3.42-3.36 (m, 1H), 2.93-2.81 (m, 2H), 2.80-2.69 (m, 2H), 2.29-2.20 (m, 1H), 1.84-1.74 (m, 3H), 1.71-1.60 (m, 4H), 1.46-1.38 (m, 1H), 1.36-1.32 (m, 1H), 1.31-1.27 (m, 1H), 1.27-1.23 (m, 1H), 1.22-1.09 (m, 3H), 1.02-0.93 (m, 1H), 0.90-0.79 (m, 6H) |
| 787 | ¹H NMR (400 MHz, DMSO-*d₆*) δ 12.12 (s, 1H), 8.30 (dd, *J =* 8.2, 1.6 Hz, 1H), 7.26 (d, *J* = 1.2 Hz, 1H), 6.83 (d, *J* = 3.1 Hz, 1H), 5.63 (t, *J =* 8.0 Hz, 1H), 4.31 (t, *J* = 8.4 Hz, 1H), 3.61-3.38 (m, 6H), 2.79-2.64 (m, 2H), 1.79-1.67 (m, 2H), 1.66-1.50 (m, 4H), 1.49 -1.31 (m, 4H), 1.30-1.01 (m, 10H), 0.98-0.72 (m, 6H). |
| 816 | ¹H NMR (400 MHz, DMSO-*d₆*) δ 12.27 (s, 1H), 8.56 (d, *J =* 7.7 Hz, 1H), 7.48 (d, *J* = 2.0 Hz, 1H), 7.45 (s, 1H), 7.00 (d, *J* = 2.0 Hz, 1H), 5.87 (dd, *J =* 10.1, 5.8 Hz, 1H), 4.46 (t, *J =* 8.2 Hz, 1H), 3.72-3.61 (m, 2H), 3.58 (dd, *J* = 11.0, 3.0 Hz, 1H), 3.44 (d, *J* = 6.3 Hz, 1H), 3.23 (dd, *J* = 13.1, 10.5 Hz, 2H), 3.12 (dd, *J* = 11.0, 7.9 Hz, 1H), 2.82-2.75 (m, 1H), 2.50-2.42 (s, 1H), 2.39-2.17 (m, 4H), 1.82 (t, *J* = 16.0 Hz, 2H), 1.68 (s, 2H), 1.46 (d, *J =* 12.3 Hz, 1H), 1.35-1.12 (m, 3H), 1.04 (d, *J =* 12.5 Hz, 1H), 0.96 (d, *J =* 6.3 Hz, 3H), 0.92 - 0.78 (m, 6H) |
| 818 | ¹H NMR (400 MHz, DMSO-*d₆*) *δ* 12.28 (s, 1H), 8.60 (d, *J =* 7.8 Hz, 1H), 7.48 (d, *J* = 2.0 Hz, 1H), 7.42 (s, 1H), 7.01 (d, *J* = 2.0 Hz, 1H), 5.81 (t, *J =* 7.6 Hz, 1H), 4.46 (t, *J* = 8.2 Hz, 1H), 3.73-3.61 (m, 1H), 3.59-3.41 (m, 5H), 3.25-3.18 (m, 1H), 3.16-3.08 (m, 1H), 2.76-2.69 (m, 1H), 2.63-2.57 (m, 1H), 2.34-2.23 (m, 3H), 1.87-1.76 (m, 2H), 1.71-1.64 (m, 2H), 1.48-1.42 (m, 1H), 1.33-1.22 (m, 2H), 1.21-1.13 (m, 1H), 1.09-1.01 (m, 1H), 0.98 (d, *J* = 6.4 Hz, 3H), 0.92-0.79 (m, 6H). |
| 913 | ¹H NMR (400 MHz, DMSO-*d₆*) δ 12.19 (s, 1H), 8.58 (d, *J* = 7.7 Hz, 1H), 7.48 (d, *J* = 2.0 Hz, 1H), 7.40 (d, *J* = 1.0 Hz, 1H), 7.01 (d, *J* = 2.1 Hz, 1H), 5.93-5.84 (m, 1H), 4.46 (t, *J* = 8.2 Hz, 1H), 3.64 (dt, *J =* 15.7, 12.4 Hz, 1H), 3.44 (q, *J* = 12.4 Hz, 1H), 2.98 (dd, *J =* 13.0, 9.6 Hz, 1H), 2.79 (dd, *J =* 13.0, 6.7 Hz, 1H), 2.59 (ddd, *J =* 27.0, 9.5, 4.7 Hz, 4H), 2.30 (tt, *J =* 14.4, 7.2 Hz, 2H), 1.92 (dp, *J* = 21.3, 5.7 Hz, 4H), 1.84-1.75 (m, 2H), 1.68 (s, 2H), 1.46 (d, *J* = 12.6 Hz, 1H), 1.27-1.11 (m, 4H), 1.11-0.97 (m, 1H), 0.86 (t, *J* = 9.2 Hz, 5H) |
| 919 | ¹H NMR (400 MHz, DMSO-*d₆*) *δ* 12.27 (s, 1H), 8.60 (d, *J =* 7.8 Hz, 1H), 7.48 (d, *J* = 2.2 Hz, 1H), 7.38 (s, 1H), 7.01 (d, *J =* 2.0 Hz, 1H), 5.86 (t, *J =* 8.0 Hz, 1H), 4.78-4.56 (m, 1H), 4.46 (t, *J =* 8.2 Hz, 1H), 3.70-3.57 (m, 1H), 3.50-3.39 (m, 1H), 2.91-2.82 (m, 1H), 2.76-2.68 (m, 1H), 2.68-2.57 (m, 2H), 2.46-2.39 (m, 1H), 2.36-2.24 (m, 3H), 1.88-1.77 (m, 4H), 1.73-1.64 (m, 4H), 1.51-1.42 (m, 1H), 1.33-1.22 (m, 2H), 1.22-1.13 (m, 1H), 1.09-0.97 (m, 1H), 0.92-0.79 (m, 6H) |
| 928 | ¹H NMR (400 MHz, DMSO-*d₆*) δ 12.27 (s, 1H), 8.59 (d, *J* = 7.7 Hz, 1H), 7.48 (d, *J* = 2.0 Hz, 1H), 7.38 (d, *J* = 1.1 Hz, 1H), 7.01 (d, *J* = 2.0 Hz, 1H), 5.86 (t, *J* = 8.0 Hz, 1H), 4.45 (t, *J* = 8.2 Hz, 1H), 4.32 (d, *J* = 5.7 Hz, 1H), 4.20 (d, *J=* 5.6 Hz, 1H), 3.73-3.55 (m, 1H), 3.49-3.31 (m, 1H), 2.96-2.88 (m, 2H), 2.86-2.80 (m, 1H), 2.74-2.67 (m, 1H), 2.53-2.51 (m, 3H), 2.36-2.23 (m, 2H), 2.12-2.04 (m, 1H), 2.01-1.95 (m, 1H), 1.88-1.75 (m, 2H), 1.72-1.56 (m, 5H), 1.46 (d, *J* = 12.6 Hz, 1H), 1.35-1.154 (m, 3H), 1.08-0.99 (m, 1H), 0.92-0.80 (m, 5H) |
| 938 | ¹H NMR (400 MHz, DMSO-*d₆*) δ 12.28 (s, 1H), 8.59 (d, *J* = 7.7 Hz, 1H), 7.48 (d, *J* = 2.0 Hz, 1H), 7.38 (d, *J* = 1.1 Hz, 1H), 7.01 (d, *J* = 2.1 Hz, 1H), 6.12-5.57 (m, 2H), 4.45 (t, *J* = 8.2 Hz, 1H), 3.70-3.56 (m, 1H), 3.49-3.40 (m, 1H), 2.95-2.90 (m, 2H), 2.88-2.82 (m, 1H), 2.74-2.67 (m, 1H), 2.55-2.45 (m, 4H), 2.35-2.20 (m, 2H), 2.10-2.04 (m, 1H), 1.97-1.94 (m, 1H), 1.89-1.60 (m, 6H), 1.46 (d, *J* = 12.6 Hz, 1H), 1.40-1.11 (m, 3H), 1.08-0.99 (m, 1H), 0.92-0.80 (m, 5H) |
| 948 | ¹H NMR (400 MHz, DMSO-*d₆*) δ 12.28 (s, 1H), 8.59 (d, *J =* 7.6 Hz, 1H), 7.48 (d*, J* = 2.0 Hz, 1H), 7.38 *(d, J* = 1.1 Hz, 1H), 7.01 (d, *J* = 2.1 Hz, 1H), 5.87 (t, *J* = 8.0 Hz, 1H), 4.45 (t, *J* = 8.2 Hz, 1H), 3.74-3.54 (m, 1H), 3.44-3.39 (m, 1H), 2.97-2.94 (m, 2H), 2.91-2.85 (m, 1H), 2.73-2.67 (m, 1H), 2.56-2.48 (m, 3H), 2.35-2.23 (m, 3H), 2.15-2.09 (m, 1H), 2.04-1.98 (m, 1H), 1.88-1.60 (m, 6H), 1.53-1.34 (m, 2H), 1.34-1.10 (m, 2H), 1.11-0.97 (m, 1H), 0.92-0.84 (m, 5H) |
| 958 | ¹H NMR (400 MHz, DMSO-*d₆*) δ 12.26 (s, 1H), 8.56 (d, *J* = 7.7 Hz, 1H), 7.48 (d, *J* = 2.0 Hz, 1H), 7.38 (d, *J =* 1.1 Hz, 1H), 7.00 (d, *J =* 2.1 Hz, 1H), 5.88 (t, *J* = 8.0 Hz, 1H), 4.46 (t, *J* = 8.2 Hz, 1H), 3.72-3.57 (m, 1H), 3.52-3.34 (m, 2H), 2.89 (dd, *J* = 12.9, 9.1 Hz, 1H), 2.75 (dd, *J =* 12.8, 7.0 Hz, 1H), 2.54 (d, *J* = 6.9 Hz, 2H), 2.30 (tt, *J* = 14.9, 7.3 Hz, 3H), 1.82 (t, *J =* 15.2 Hz, 2H), 1.68 (s, 2H), 1.58 (d, *J =* 6.4 Hz, 2H), 1.47 (d, *J =* 15.3 Hz, 3H), 1.21 (q, *J =* 8.4 Hz, 5H), 1.04 (q, *J=* 11.8 Hz, 1H), 0.85 (d, *J* = 6.5 Hz, 6H) |
| 968 | ¹H NMR (400 MHz, DMSO-*d₆*) δ 12.29 (s, 1H), 8.59 (d, *J =* 7.7 Hz, 1H), 7.48 (d, *J* = 2.0 Hz, 1H), 7.39 (d, *J =* 1.1 Hz, 1H), 7.00 (d, *J =* 2.0 Hz, 1H), 5.95-5.86 (m, 1H), 4.45 (t, *J =* 8.2 Hz, 1H), 3.67-3.52 (m, 1H), 3.41 (q, *J =* 11.7 Hz, 1H), 2.93 (dd, *J =* 12.9, 10.2 Hz, 1H), 2.76-2.60 (m, 3H), 2.35-2.17(m, 5H), 1.92-1.72 (m, 5H), 1.67 (s, 2H), 1.45 (d, *J =* 12.6 Hz, 1H), 1.28-1.12 (m, 4H), 1.03 (q, *J =* 12.2 Hz, 1H), 0.85 (d, *J =* 6.4 Hz, 5H) |
| 970 | ¹H NMR (400 MHz, DMSO-*d₆*) δ 12.30 (s, 1H), 8.58 (d, *J =* 7.7 Hz, 1H), 7.48 (d, *J* = 2.0 Hz, 1H), 7.39 *(d, J=* 1.1 Hz, 1H), 7.00 (d, *J* = 2.1 Hz, 1H), 5.88 (dd, *J* = 10.1, 6.2 Hz, 1H), 4.45 (t, *J =* 8.2 Hz, 1H), 3.65-3.50 (m, 1H), 3.40 (q, *J =* 11.5 Hz, 2H), 2.87 (dd, *J =* 13.0, 10.2 Hz, 1H), 2.74 (s, 2H), 2.65 (dd, *J =* 13.0, 6.2 Hz, 1H), 2.40-2.17 (m, 3H), 2.08 (s, 1H), 1.81 (dq, *J =* 22.4, 11.5 Hz, 5H), 1.68 (s, 1H), 1.62-1.54 (m, 1H), 1.45 (d, *J* = 12.6 Hz, 1H), 1.35-1.12 (m, 4H), 1.03 (q, *J =* 11.8 Hz, 1H), 0.85 (d, *J =* 6.5 Hz, 5H). |
| 980 | ¹H NMR (400 MHz, DMSO-*d₆*) δ12.22 (s, 1H), 8.58 (d, *J =* 7.6 Hz, 1H), 7.48 (d, *J* = 2.0 Hz, 1H), 7.39 (d, *J =* 1.1 Hz, 1H), 7.01 (d, *J =* 2.1 Hz, 1H), 5.88 (t, *J* = 8.0 Hz, 1H), 4.45 (t, *J* = 8.2 Hz, 1H), 3.69-3.59 (m, 1H), 3.49-3.40 (m, 1H), 2.92-2.86 (m, 1H), 2.78-2.70 (m, 1H), 2.54-2.43(m, 6H), 2.35-2.24 (m, 2H), 2.13-2.10 (m, 4H), 1.88-1.68 (m, 5H), 1.48-1.44 (m, 1H), 1.29-0.96 (m, 2H), 0.92-0.80 (m, 5H). |
| 986 | ¹H NMR (400 MHz, DMSO-*d₆*) δ 12.24 (s, 1H), 8.56 (d, *J* = 7.7 Hz, 1H), 7.48 (d, *J* = 2.0 Hz, 1H), 7.35 (d, *J =* 1.1 Hz, 1H), 7.01 (d, *J =* 2.0 Hz, 1H), 5.83 (t, *J* = 8.1 Hz, 1H), 4.46 (t, *J =* 8.2 Hz, 1H), 3.70-3.55 (m, 1H), 3.45 (q, *J =* 12.4 Hz, 1H), 3.15 (dt, *J =* 8.8, 4.6 Hz, 1H), 2.87-2.66 (m, 4H), 2.33-2.09 (m, 4H), 1.80 (d, *J =* 13.5 Hz, 4H), 1.68 (s, 2H), 1.54-1.33 (m, 3H), 1.33-1.11 (m, 3H), 1.04 (q, *J* = 11.9 Hz, 1H), 0.85 (d, *J =* 6.5 Hz, 6H). |
| 988 | ¹H NMR (400 MHz, DMSO-*d₆*) δ 12.27 (s, 1H), 8.59 (d, *J =* 7.7 Hz, 1H), 7.48 (d, *J* = 2.0 Hz, 1H), 7.37 (d, *J =* 1.1 Hz, 1H), 7.01 (d, *J =* 2.1 Hz, 1H), 5.84 (t, *J* = 8.0 Hz, 1H), 4.45 (t, *J* = 8.2 Hz, 1H), 3.71-3.56 (m, 1H), 3.49-3.40 (m, 1H), 2.84-2.79 (m, 1H), 2.73-2.68 (m, 1H), 2.56-2.46 (m, 6H), 2.43-2.20 (m, 3H), 1.86-1.75 (m, 2H), 1.69-1.61 (m, 4H), 1.48-1.37 (m, 3H), 1.35-1.12 (m, 1H), 1.06-0.99 (m, 4H), 0.92-0.80 (m, 5H) |
| 992 | ¹H NMR (400 MHz, DMSO-*d₆*) *δ* 12.29 (s, 1H), 8.60 (d, *J =* 7.8 Hz, 1H), 7.48 (d, *J* = 2.0 Hz, 1H), 7.38 (s, 1H), 7.01 (d, *J* = 2.2 Hz, 1H), 5.86 (d, *J* = 8.0 Hz, 1H), 4.82-4.64 (m, 1H), 4.45 (t, *J =* 8.2 Hz, 1H), 3.67-3.55 (m, 1H), 3.49-3.40 (m, 1H), 2.97-2.85 (m, 2H), 2.80-2.64 (m, 2H), 2.34-2.19 (m, 4H), 1.87-1.75 (m, 2H), 1.74-1.57 (m, 5H), 1.50-1.41 (m, 1H), 1.34-1.11 (m, 3H), 1.08-0.97 (m, 1H), 0.94-0.76 (m, 6H). |
| 1013 | ¹H NMR (400 MHz, DMSO-*d₆*) δ 12.20 (s, 1H), 8.37 (d, *J =* 8.1 Hz, 1H), 7.39 (d, *J =* 1.1 Hz, 1H), 6.88 (d, *J =* 3.1 Hz, 1H), 5.71 (dd, *J =* 10.2, 5.8 Hz, 1H), 4.37 (t, *J =* 8.4 Hz, 1H), 3.75 -3.35 (m, 8H), 3.24-3.07 (m, 2H), 2.87-2.81 (m, 1H), 2.46-2.38 (m, 1H), 2.27-2.18 (m, 1H), 1.83-1.73 (m, 2H), 1.67 (t, *J* = 10.9 Hz, 2H), 1.42 (d, *J =* 12.7 Hz, 1H), 1.38-1.22 (m, 3H), 1.22 -1.07 (m, 3H), 1.02-0.77 (m, 9H). |
| 1015 | ¹H NMR(400 MHz, DMSO-*d₆*) *δ* 12.17 (s, 1H), 8.36 (d, *J =* 8.0 Hz, 1H), 7.38 (d, *J* = 1.2 Hz, 1H), 6.89 (d, *J =* 3.0 Hz, 1H), 5.64 (t, *J* = 7.8 Hz, 1H), 4.38 (t, *J* = 8.0 Hz, 1H), 3.67-3.54 (m, 3H), 3.53-3.43 (m, 3H), 3.22-3.11(m, 2H), 2.78-2.65(m, 1H), 2.59-2.52 (m, 2H), 2.33-2.26 (m, 1H) 1.86-1.72 (m, 2H), 1.71-1.60 (m, 2H), 1.46-1.38 (m, 1H), 1.36-1.21 (m, 4H), 1.21-1.12 (m, 3H), 0.99 (d, *J* = 6.2 Hz, 3H), 0.95-0.88 (m, 1H), 0.88-0.76 (m, 5H) |
| 1016 | ¹H NMR (400 MHz, DMSO-*d₆*) δ 12.18 (s, 1H), 8.38 (d, *J =* 8.1 Hz, 1H), 7.33 (s, 1H), 6.88 (s, 1H), 5.67 (s, 1H), 4.37 (s, 1H), 3.69-3.43 (m, 3H), 2.80-2.62 (m, 2H), 2.43-2.21 (m, 2H), 1.83-1.74 (m, 3H), 1.70-1.60 (m, 4H), 1.51-1.38 (m, 3H), 1.37-1.05 (m, 11H), 1.01- 0.84 (m, 5H) |
| 1018 | ¹H NMR (400 MHz, DMSO-*d₆*) δ 12.29 (s, 1H), 8.00 (s, 1H), 7.65 (s, 1H), 7.48 (dd, *J* = 9.7, 2.8 Hz, 1H), 6.48 (d, *J* = 9.7 Hz, 1H), 6.16 (t, *J* = 7.7 Hz, 1H), 4.07 (t, *J =* 7.9 Hz, 1H), 3.93-3.89 (m, 1H), 3.17-3.05 (m, 2H), 3.02 (s, 3H), 2.77-2.60 (m, 2H), 1.71-1.51 (m, 4H), 1.44 -0.92 (m, 6H), 0.85-0.73 (m, 5H), 0.55-0.61 (m,3H) |
| 1019 | ¹H NMR (400 MHz, DMSO-*d₆*) δ 12.24 (s, 1H), 8.04 (d, *J =* 2.9 Hz, 1H), 7.63 (s, 1H), 7.54-7.42 (m, 2H), 6.45 (d, *J =* 9.8 Hz, 1H), 4.07 (t, *J =* 7.8 Hz, 1H), 3.97-3.88 (m, 1H), 3.83 (d, *J* = 10.6 Hz, 2H), 3.27 (q, *J =* 10.6 Hz, 2H), 2.82 (s, 1H), 1.74-1.52 (m, 4H), 1.39 (d, *J =* 12.9 Hz, 1H), 1.27-1.08 (m, 7H), 1.00 (q, *J* = 11.4 Hz, 1H), 0.86-0.74 (m, 5H), 0.64-0.49 (m, 4H) |
| 1020 | ¹H NMR (400 MHz, DMSO-*d₆*) δ 12.33 (s, 1H), 8.60 (d, *J =* 7.6 Hz, 1H), 7.93 (d, *J* = 2.9 Hz, 1H), 7.50-7.42 (m, 3H), 7.01 (d, *J =* 2.0 Hz, 1H), 6.47 (d, *J =* 9.7 Hz, 1H), 6.20 (s, 1H), 4.50-4.32 (m, 3H), 3.83 (s, 4H), 3.29-3.23 (m, 1H), 3.02-2.98 (m, 1H), 2.52-2.62 (m, 4H),1.85-1.75 (m, 2H), 1.70-1.60 (m, 2H), 1.55-1.50 (m, 4H), 1.46-1.40 (m, 1H), 1.34 -0.96 (m, 5H), 0.90-0.75 (m, 6H) |
| 1021 | ¹H NMR (400 MHz, DMSO-*d₆*) δ 12.25 (s, 1H), 8.58 (d, *J =* 7.8 Hz, 1H), 7.48 (d, *J =* 2.0 Hz, 1H), 7.35 (s, 1H), 7.01 (d, *J =* 2.0 Hz, 1H), 5.85 (t, *J =* 7.8 Hz, 1H), 4.52-4.31 (m, 3H), 3.70-3.56 (m, 1H), 3.54-3.38 (m, 1H), 2.84-2.72 (m, 1H), 2.72-2.63 (m, 1H), 2.55-2.52 (m, 1H), 2.44-2.36 (m, 3H), 2.33-2.20 (m, 2H), 1.88-1.74 (m, 2H), 1.73-1.62 (m, 2H), 1.55-1.42 (m, 5H), 1.41-1.29 (m, 3H), 1.29-1.22 (m, 4H), 1.22-1.12 (m, 1H), 1.10-1.01 (m, 1H), 0.99-0.70 (m, 6H) |
| 1022 | ¹H NMR (400 MHz, DMSO-*d₆*) δ 12.27 (s, 1H), 8.58 (d, *J =* 7.6 Hz, 1H), 7.49 (d, *J* = 2.0 Hz, 1H), 7.39 (s, 1H), 7.01 (d, *J =* 2.0 Hz, 1H), 5.81 (t, *J =* 7.8 Hz, 1H), 4.51-4.35 (m, 3H), 3.75-3.59 (m, 1H), 3.52-3.44 (m, 1H), 3.01-2.93 (m, 1H), 2.90-2.81 (m, 1H), 2.63-2.53 (m, 3H), 2.39-2.19 (m, 3H), 1.91-1.74 (m, 2H), 1.73-1.60 (m, 6H), 1.52-1.40 (m, 1H), 1.36-1.22 (m, 5H), 1.22-0.97 (m, 3H), 0.94-0.79 (m, 5H) |
| 1023 | ¹H NMR (400 MHz, DMSO-*d₆*) δ 12.27 (s, 1H), 8.58 (d, *J =* 7.6 Hz, 1H), 7.49 (d, *J* = 2.0 Hz, 1H), 7.39 (s, 1H), 7.00 (d, *J =* 2.0 Hz, 1H), 5.85 (t, *J =* 7.9 Hz, 1H), 4.49-4.38 (m, 3H), 3.73-3.62 (m, 1H), 3.50-3.42 (m, 3H), 3.22 (s, 3H), 3.02-2.91 (m, 1H), 2.79-2.69 (m, 1H), 2.65-2.55 (m, 2H), 2.35-2.26 (m, 2H), 2.25 (s, 3H), 1.89-1.73 (m, 2H), 1.73-1.61 (m, 2H), 1.51-1.38 (m, 1H), 1.37-1.13 (m, 6H), 1.12-0.91 (m, 2H), 0.90-0.74 (m, 5H) |
| 1024 | ¹H NMR (400 MHz, DMSO-*d₆*) δ 12.01 (s, 1H), 8.58 (d, *J =* 7.6 Hz, 1H), 7.47 (d, *J* = 2.0 Hz, 1H), 7.39 (s, 1H), 7.05 (d, *J =* 2.0 Hz, 1H), 5.92-5.72 (m, 1H), 4.45 (t, *J* = 8.2 Hz, 1H), 4.01 (s, 3H), 3.73-3.61 (m, 1H), 3.46-3.38 (m, 3H), 3.22 (s, 3H), 3.01-2.93 (m, 1H), 2.77-2.68 (m, 1H), 2.64-2.55 (m, 2H), 2.35-2.18 (m, 5H), 1.91-1.73 (m, 2H), 1.73-1.61(m, 2H), 1.51-1.41 (m, 1H), 1.34-1.15 (m, 2H), 1.15-1.07 (m, 2H), 1.06-0.97 (m, 1H), 0.91-0.80 (m, 5H) |
| 1025 | ¹H NMR (400 MHz, DMSO-*d₆*) δ 12.26 (s, 1H), 8.56 (d, *J =* 7.6 Hz, 1H), 7.48 (d, *J =* 2.0 Hz, 1H), 7.39 (s, 1H), 7.01 (d, *J* = 2.0 Hz, 1H), 5.79 (t, *J* = 7.8 Hz, 1H), 4.46 (t, *J* = 8.0 Hz, 1H), 3.92-3.79 (m, 1H), 3.72-3.56 (m, 1H), 3.53-3.39 (m, 1H), 3.01-2.90 (m, 1H), 2.90-2.76 (m, 2H), 2.64-2.57 (m, 1H), 2.57-2.53 (m, 1H), 2.48-2.41 (m, 1H), 2.37-2.17 (m, 2H), 2.00-1.59 (m, 6H), 1.52-1.41 (m, 1H), 1.39-0.96 (m, 4H), 0.94-0.76 (m, 6H) |
| 1027 | ¹H NMR (400 MHz, DMSO-*d₆*) δ 12.27 (s, 1H), 8.58 (d, *J =* 7.8 Hz, 1H), 7.47 (d, *J =* 2.1 Hz, 1H), 7.35 (s, 1H), 7.05 (d, *J* = 2.1 Hz, 1H), 5.83 (t, *J* = 8.0 Hz, 1H), 4.47 (t, *J =* 8.3 Hz, 1H), 3.71-3.56 (m, 1H), 3.45 (q, *J =* 12.2 Hz, 1H), 3.15 (p, *J =* 4.5 Hz, 1H), 2.91-2.64 (m, 4H), 2.38-2.07 (m, 4H), 1.90-1.73 (m, 4H), 1.68 (s, 2H), 1.51-1.33 (m, 3H), 1.32-1.11 (m, 3H), 1.03 (q, J = 11.1 Hz, 1H), 0.85 (d, J = 6.5 Hz, 6H) |
| 1028 | ¹H NMR (400 MHz, DMSO-*d₆*) δ 12.29 (s, 1H), 8.57 (d, *J =* 7.8 Hz, 1H), 7.48-7.44 (m, 1H), 7.35-7.32 (m, 1H), 7.05 (d, *J =* 6.4 Hz, 1H), 5.63 (t, *J* = 7.8 Hz, 1H), 4.46 (t, *J =* 8.2 Hz, 1H), 4.36-4.32 (m, 1H), 3.80 (d, *J =* 7.4 Hz, 1H), 3.71-3.60 (m, 1H), 3.54-3.44 (m, 3H), 3.03-2.97 (m, 2H), 2.89-2.82 (m, 2H), 2.57-2.52 (m, 1H), 2.44 (d, *J =* 9.6 Hz, 1H), 2.37-2.26 (m, 2H), 1.88-1.76 (m, 2H), 1.75-1.65 (m, 2H), 1.62-1.57 (m, 1H), 1.49-1.42 (m, 1H), 1.33-1.16 (m, 2H), 1.14 (d, *J =* 7.2 Hz, 2H), 1.08-0.97 (m, 1H), 0.93-0.79 (m, 4H) |
| 1029 | ¹H NMR (400 MHz, DMSO-*d₆*) δ 12.25 (s, 1H), 8.58 (d, *J =* 7.6 Hz, 1H), 7.47 (d, *J* = 2.1 Hz, 1H), 7.38 (d, *J =* 1.1 Hz, 1H), 7.05 (d, *J =* 2.1 Hz, 1H), 5.69 (t, J = 7.8 Hz, 1H), 4.46 (t, *J* = 8.3 Hz, 1H), 4.34 (s, 1H), 3.81 (d, *J =* 6.6 Hz, 1H), 3.72-3.57 (m, 1H), 3.52 (d, *J =* 6.3 Hz, 2H), 3.44 (q, *J =* 12.3 Hz, 1H), 3.06-2.91 (m, 2H), 2.74 (d, *J =* 9.6 Hz, 1H), 2.53-2.49 (m, 2H), 2.37-2.21 (m, 2H), 1.82 (dd, *J* = 22.1, 10.7 Hz, 2H), 1.70 (d, *J =* 9.3 Hz, 3H), 1.57 (d, *J =* 9.4 Hz, 1H), 1.46 (d, *J =* 12.4 Hz, 1H), 1.37-1.12 (m, 3H), 1.03 (q*, J =* 11.2 Hz, 1H), 0.85 (d, *J =* 6.5 Hz, 5H). |
| 1030 | ¹H NMR (400 MHz, DMSO-*d₆*) δ 12.30 (s, 1H), 8.58 (d, *J* = 7.8 Hz, 1H), 7.47 (d, *J* = 2.1 Hz, 1H), 7.40 (d, *J =* 1.1 Hz, 1H), 7.05 (d, *J =* 2.1 Hz, 1H), 5.89 (t, *J* = 8.0 Hz, 1H), 4.46 (t, *J* = 8.3 Hz, 1H), 3.74-3.38 (m, 6H), 2.97-2.82 (m, 1H), 2.72 (dd, *J =* 12.8, 6.8 Hz, 1H), 2.58-2.38 (m, 5H), 2.30 (tt, *J =* 15.3, 7.4 Hz, 2H), 1.82 (dd, *J=* 20.3, 11.9 Hz, 2H), 1.68 (s, 2H), 1.46 (d, *J =* 12.4 Hz, 1H), 1.37-1.13 (m, 3H), 1.03 (q, *J =* 10.7 Hz, 1H), 0.85 (d, J = 6.5 Hz, 5H) |
| 1031 | ¹H NMR (400 MHz, DMSO-*d₆*) δ 12.28 (s, 1H), 8.58 (d, *J =* 7.6 Hz, 1H), 7.49-7.46 (m, 1H), 7.42-7.38 (m, 1H), 7.01 (d, *J =* 6.6 Hz, 1H), 5.83-5.74 (m, 1H), 4.45 (t, *J =* 8.2 Hz, 1H), 3.74-3.60 (m, 1H), 3.53-3.43 (m, 2H), 3.01-2.86 (m, 2H), 2.80-2.67 (m, 2H), 2.36-2.25 (m, 2H), 1.90-1.78 (m, 2H), 1.75-1.63 (m, 4H), 1.50-1.43 (m, 1H), 1.34-1.19 (m, 2H), 1.16 (t, *J =* 7.2 Hz, 2H), 1.09-0.97 (m, 1H), 0.94-0.78 (m, 6H), 0.55-0.44 (m, 4H) |
| 1032 | ¹H NMR (400 MHz, DMSO-*d₆*) δ 12.25 (s, 1H), 8.55 (d, *J* = 7.8 Hz, 1H), 7.47 (d, *J* = 2.0 Hz, 1H), 7.38 (s, 1H), 7.04 (d, *J =* 2.0 Hz, 1H), 5.81 (t, *J* = 8.0 Hz, 1H), 4.57-4.31 (m, 5H), 3.70-3.55 (m, 1H), 3.50-3.37 (m, 1H), 3.06-2.95 (m, 1H), 2.93-2.85 (m, 1H), 2.84-2.70 (m, 2H), 2.65-2.56 (m, 1H), 2.55-2.53 (m, 1H), 2.38-2.17 (m, 2H), 2.10-1.91 (m, 2H), 1.89-1.62 (m, 4H), 1.51-1.40 (m, 1H), 1.37-0.89 (m, 5H), 0.89-0.72 (m, 5H) |
| 1033 | ¹H NMR (400 MHz, DMSO-*d₆*) δ12.28 (s, 1H), 8.58 (d, *J =* 7.7 Hz, 1H), 7.48 (d, *J* = 2.0 Hz, 1H), 7.40 (s, 1H), 7.01 (d, *J =* 2.0 Hz, 1H), 5.92-5.86 (m, 1H), 5.33 (s, 1H), 4.47-4.39 (m, 2H), 3.69-3.59 (m, 1H), 3.48-3.43 (m, 3H), 2.98-2.80 (m, 4H), 2.60-2.50 (m, 2H), 2.33-2.22 (m, 2H), 2.12-2.04 (m, 2H), 2.02-1.94 (m, 2H), 1.88-1.74 (m, 2H), 1.72-1.64 (m, 3H), 1.50-1.40 (m, 1H), 1.35-1.15 (m, 3H), 1.10-0.95 (m, 1H), 0.90-0.75 (m, 5H) |
| 1035 | ¹H NMR (400 MHz, DMSO-*d₆*) δ 12.22 (s, 1H), 8.56 (d, *J =* 7.9 Hz, 1H), 7.36 (s, 1H), 6.91 (s, 1H), 5.69 (t, *J =* 6.9 Hz, 1H), 4.83-4.58 (m, 1H), 4.36 (t, *J =* 7.8 Hz, 1H), 3.81 (dd, *J* = 10.4, 7.9 Hz, 1H), 3.73 (dd, *J =* 10.4, 5.9 Hz, 1H), 3.66-3.35 (m, 4H), 3.31 (s, 3H), 2.37-2.23 (m, 1H), 1.67 (dt, *J* = 24.9, 11.9 Hz, 4H), 1.50-1.31 (m, 2H), 1.23 (d, *J =* 6.3 Hz, 1H), 1.16-0.94 (m, 3H), 0.84 (d, *J* = 6.5 Hz, 5H) |
| 1036 | ¹H NMR (400 MHz, DMSO-*d₆*) δ 12.23 (s, 1H), 8.55 (d, *J =* 7.8 Hz, 1H), 7.48 (d, *J* = 2.3 Hz, 1H), 7.36 (s, 1H), 7.00 (d, *J =* 2.0 Hz, 1H), 5.84 (t, *J* = 8.1 Hz, 1H), 4.44 (p, *J =* 7.9 Hz, 3H), 3.70-3.54 (m, 1H), 3.45 (q, *J =* 13.0 Hz, 1H), 3.21 (s, 3H), 3.15 (d, *J =* 6.5 Hz, 2H), 2.88 (s, 2H), 2.84-2.76 (m, 1H), 2.70 (dd, *J* = 13.3, 7.8 Hz, 1H), 2.30 (dd, *J =* 13.6, 6.6 Hz, 2H), 2.00 (dt, *J =* 34.0, 11.1 Hz, 2H), 1.82 (t, *J =* 15.1 Hz, 2H), 1.73-1.54 (m, 4H), 1.47 (d, *J =* 12.0 Hz, 2H), 1.35-0.97 (m, 10H), 0.85 (d, *J =* 6.5 Hz, 5H) |

### Biological assay and evaluation

The present invention will be further described and explained below in conjunction with test examples, and these examples are not meant to limit the scope of the present invention.

### Test example 1 Assay for competitive binding effect between compounds of the present invention and human IL-17 receptor for ligand

1. Experimental objective: to quantitatively determine the blocking effect of the compound on the binding between human IL-17RA and IL-17A proteins using competitive ELISA under in vitro conditions.
2. Experimental instruments and reagents:
2.1 Instruments:

| **Name of instrument** | **Manufacturer** | **Specification and model** |
|---|---|---|
| -80°C refrigerator | Thermo Fisher | 905 |
| Medical refrigerator (2-8°C) | Haier | HYC-940 |
| Haier low-temperature refrigerator | Haier | DW-25L262 |
| Microcentrifuge | IKA | IKA MINI GS025 |
| EnVision plate reader | PerkinElmer | 2105-0020 |
| Ultra-pure water purification system | Thermo Fisher | Pacific TII |
| Digital microplate shaker | Thermo Fisher | 88882006 |

2.2 Reagents:
3. Experimental method: IL-17A at 0.0625 µg/mL was coated in a 96-well plate at 4°C overnight at 100 µL per well. The plate was washed with PBST (300 µL/well x 4), and then blocked at 24°C for 2 h by adding 300 µL of ELISA Blocker blocking buffer to each well. The plate was further washed under the same conditions. 80 µL of PBS solution containing 1% BSA and 10 µL of 10× test compound solution were added to each well and mixed uniformly, and then the mixture was incubated at room temperature for 1 hour. After that, 10 µL of IL-17RA solution was added to a final concentration of 5 nM and mixed uniformly, and then the mixture was incubated at 24°C for 1 h. The corresponding concentration and volume of DMSO solution without compounds was added to positive control wells, and 1% BSA solution without IL-17RA was added to negative control wells.

| **Reagents and materials** | **Manufacturer** | **Cat. No.** |
|---|---|---|
| Recombinant Human IL-17A Protein | R&D | BT7955-025/CF |
| IL17RA Protein, human, Recombinant (hFc Tag) | R&D | 177-IR-100 |
| Anti-Human IgG, Fc gamma Fragment Specific, HRP-Linked Antibody | CST | 32935 |
| DMSO | Sigma | D2650-100ML |
| PBS | Gibco | 10010-023 |
| ELISA Blocker blocking buffer | Thermo Fisher | N502 |
| 96-well V-bottom plate (PS) | Corning | 3896 |
| Clear Polystyrene Microplates | R&D | DY990 |
| Blocker BSA (10%) in PBS | Thermo Fisher | 37525 |
| 5×ELISA/ELISPOT diluent | Thermo Fisher | 00-4202-56 |
| PBS Tween-20 (20×) | Thermo Fisher | 28352 |
| TMB ELISA Substrate | Abcam | ab171523 |
| STOP Solution | CST | 7002P6 |
| Reagent reservoir | Corning | 4870 |
| ELISA Plate Sealers | R&D | DY992 |

After the plate was washed four times, 100 µL of the anti-Fc tag HRP-conjugated antibody was added, and the mixture was incubated at 37°C for 1 h. After the plate was washed four times, 100 µL of TMB substrate was added to each well, and the mixture was incubated at 37°C in the dark. After colour development was completed, the reaction was terminated by adding 100 µL of stop solution to each well, and the absorbance was measured at wavelengths of 450 nm and 570 nm using a microplate reader.

### 4. Experimental data processing method:

1) Inhibition rate (%): The original data (OD450-OD570) was calculated according to the following formula to obtain the inhibition rate.
   Inhibition rate% = [(average value of positive control wells - value of sample wells)/(average value of positive control wells - average value of negative control wells)] × 100, where the positive control wells are wells without compounds, and the negative control wells are wells without IL-17RA protein solution.
2) Curve fitting: Log(inhibitor) vs. response -- Variable slope (four parameters) in GraphPad Prism 8 was used for fitting equation analysis of the compound concentration and the corresponding inhibition rate, and the curve was fitted to obtain the IC₅₀ value of the compound.
   The fitting calculation equation was Y = Bottom + (Top - Bottom) / (1 + 10^((LogIC₅₀ - X) * HillSlope)).

### 5. Experimental results and conclusions:

**Table 1**

| Example | IC₅₀ (nM) | Example | IC₅₀ (nM) |
|---|---|---|---|
| 521 | 1.3 | 731 | 6.6 |
| 522 | 6.2 | 734 | 2.9 |
| 524 | 3.6 | 736 | 3.6 |
| 532 | 7.9 | 739 | 5.0 |
| 556 | 1.8 | 743 | 6.0 |
| 559 | 4.3 | 746 | 6.0 |
| 569 | 5.0 | 747 | 7.0 |
| 592 | 6.4 | 760 | 7.0 |
| 601 | 4.1 | 763 | 6.0 |
| 696 | 5.0 | 764 | 7.0 |
| 688 | 5.1 | 770 | 7.0 |
| 700 | 3.4 | 777 | 8.0 |
| 703 | 7.5 | 780 | 10.0 |
| 704 | 4.9 | 781 | 5.0 |
| 718 | 5.0 | 782 | 4.0 |
| 721 | 4.0 | 787 | 9.0 |
| 723 | 5.0 | 1012 | 6.0 |
| 724 | 6.0 | 1013 | 9.0 |
| 725 | 5.6 | 1014 | 7.0 |
| 726 | 4.8 | 1015 | 4.0 |
| 727 | 3.3 | 1018 | 10.0 |
| 730 | 6.7 | | |

6. Conclusion: the compounds of the present invention exhibit an excellent competitive binding effect for the ligand compared to the human IL-17 receptor.

### Test example 2 Assay for inhibitory effect of compounds of the present invention against Groα secretion by HT-29 cells

Experimental objective: to measure the inhibitory effect of the compounds against Groα secretion by HT-29 cells, thereby evaluating the inhibition of the compounds on IL-17A pathway.

### Experimental instruments and reagents:

### Instrument:

| | | |
|---|---|---|
| **Name of instrument** | **Manufacturer** | **Specification and model** |

| Centrifuge | Eppendorf | 5810R |
|---|---|---|
| CO2 incubator | Thermo | 311 |
| Biosafety cabinet | Shanghai Boxun Industry Co., Ltd | BSC-1300IIA2 |
| Microplate reader | PerkinElmer | Envision2105 |

### Reagents:

Experimental method: A cell suspension at 50,000 cells/ml was prepared, and 200 µl of the cell suspension was added to each well of a 96-well plate, i.e., 10,000 cells/well. The plate was cultured overnight. In the 96-well V-bottom plate, 5 mM compound solution was serially diluted 3-fold using DMSO, and the compound from each well was diluted 100-fold using a complete culture medium. IL-17A protein (R&D, #BT7955-025/CF) with a stock concentration of 100 µg/ml was diluted 333.33-fold using a complete culture medium. The compound and IL-17A were mixed at a volume ratio of 1:1 and incubated at 37°C for 30 minutes. 50 µl of a mixture of the compound and IL-17A was added to cell wells, and the final concentration of IL-17A protein was 30 ng/ml.

| **Reagents and materials** | **Manufacturer** | **Cat. No.** |
|---|---|---|
| DMSO | Sigma | D2650-100ML |
| Human Groα Valukin Elisa kit | Biotechne | VAL139 |
| HT-29 | ATCC | HTB-38 |
| 96-well V-bottom plate (PS) | Coming | 3894 |
| 96-well plate | Corning | 3599 |
| IL-17A | R&D | BT7955-025/CF |
| Gibco^{™} Foetal Bovine Serum | ThermoFisher | 10091-148 |
| RPMI 1640 | Gibco | 22400-089 |

The mixture was incubated in a 37°C, 5% CO₂ cell culture incubator for 48 hours. The concentration of Groα in the supernatant was determined by ELISA method using Human Groα Valukin Elisa kit (Biotechne, # VAL139), and the absorbance intensity at 450 nm was measured using a microplate reader (PE, EnVision2105). Data were analysed using GraphPad Prism 8.3.0.

Experimental data processing method: Data were analysed using GraphPad Prism 8.3.0.

Inhibition rate% = [(average value of positive control wells - value of sample wells)/(average value of positive control wells - average value of negative control wells)] × 100%, where the sample wells are wells containing cells plus compounds at different concentration gradients, the positive control wells are wells containing cells plus IL-17A, and the negative control wells are wells containing culture medium plus IL-17A.

Curve fitting: The IC₅₀ value was obtained by curve fitting based on the inhibition rate (%) at each concentration using the log(inhibitor) vs. responseVariable slope (four parameters) in GraphPad Prism 6.0, and the calculation equation was Y = Bottom + (Top-Bottom)/(1+10^((LogIC₅₀-X)*HillSlope)).

Experimental results and conclusions:

**Table 2**

| Example | IC₅₀ (nM) | Example | IC₅₀ (nM) |
|---|---|---|---|
| 259 | 5.5 | 774 | 5.0 |
| 327 | 2.5 | 777 | 5.3 |
| 521 | 2.0 | 780 | 10.0 |
| 524 | 9.3 | 781 | 10.0 |
| 532 | 7.9 | 782 | 4.0 |
| 540-1 | 8.0 | 787 | 8.9 |
| 541 | 6.8 | 816 | 4.6 |
| 556 | 4.6 | 818 | 2.6 |
| 564 | 5.9 | 820 | 3.0 |
| 565 | 4.0 | 849 | 4.0 |
| 574 | 5.0 | 854 | 9.0 |
| 586-1 | 8.4 | 857 | 2.4 |
| 586 | 8.1 | 860 | 2.0 |
| 601 | 7.5 | 862 | 2.7 |
| 613 | 5.6 | 863 | 3.2 |
| 624 | 4.3 | 864 | 3.0 |
| 627 | 6.3 | 913 | 3.5 |
| 628 | 6.7 | 919 | 4.0 |
| 634 | 2.5 | 928 | 4.0 |
| 644 | 3.8 | 938 | 4.4 |
| 646 | 8.0 | 948 | 6.5 |
| 648 | 9.6 | 958 | 2.3 |
| 650 | 6.0 | 968 | 3.0 |
| 663 | 1.6 | 970 | 4.0 |
| 689 | 5.2 | 980 | 5.0 |
| 696 | 2.0 | 986 | 9.0 |
| 700 | 2.6 | 988 | 8.8 |
| 703 | 7.0 | 992 | 3.5 |
| 704 | 6.5 | 994 | 9.0 |
| 718 | 8.0 | 1012 | 3.0 |
| 723 | 4.0 | 1013 | 9.0 |
| 724 | 4.0 | 1014 | 8.0 |
| 725 | 2.5 | 1015 | 6.0 |
| 726 | 1.8 | 1016 | 2.0 |
| 727 | 1.2 | 1020 | 2.5 |
| 731 | 6.0 | 1021 | 8.5 |
| 734 | 2.0 | 1022 | 10.0 |
| 736 | 1.0 | 1023 | 10.0 |
| 739 | 3.0 | 1024 | 7.0 |
| 743 | 6.0 | 1025 | 10.0 |
| 746 | 3.0 | 1026 | 8.0 |
| 747 | 3.0 | 1028 | 7.0 |
| 760 | 5.7 | 1029 | 6.0 |
| 763 | 4.5 | 1030 | 7.0 |
| 764 | 7.0 | 1032 | 8.0 |
| 767 | 7.0 | 1034 | 6.0 |
| 770 | 3.4 | 1036 | 6.0 |

Conclusion: the compounds of the present invention exhibit an excellent inhibitory effect on Groα secretion by HT-29 cells.

### Test example 3. Pharmacokinetic assay in Wistar rats

1. **Experimental objective:** to study the pharmacokinetic behaviours of the compounds of the present invention in the body (plasma) of Wistar rats as test animals after oral administration at a dose of 30 mg/kg.
2. **Experimental scheme**
2.1 **Test drugs:** the examples of the present invention, prepared in house.
2.2 **Test animals:** Wistar rats (female, 3 rats per group), from Beijing Vital River Laboratory Animal Technology Co., Ltd., with the animal production license no. (SCXK (Jing) 2021-0011).
2.3 **Drug preparation:** Formulation of drug for oral administration: 30% PEG 400+70% (10% Solutol) in water

Solutol HS15 was melted in a water bath at 50-60°C, and then 50 mL of the melted Solutol HS15 was added to 450 mL of ultrapure water. The mixture was stirred and sonicated to obtain 500 mL of 10% Solutol HS15 solution. The compound was weighed, and according to the total administration volume, 30% PEG solution was first added. The mixture was shaken uniformly and sonicated until a clear solution was obtained. Subsequently, 70% (10% Solutol) in water was added, resulting in a colourless and clear solution with a concentration of 3.0 mg/mL.

2.4 **Administration scheme:** After overnight fasting, 3 female Wistar rats per group were administered orally (PO) at a dose of 30 mg/kg with an administration volume of 10 mL/kg.

2.5 **Sample collection:** At 0.25 h, 0.5 h, 1 h, 2 h, 4 h, 6 h, 8 h and 24 h after oral administration to the rats, 0.2 mL of blood was collected from the jugular vein, placed in an EDTA-K₂ test tube, and centrifuged at 4°C at 6000 rpm for 6 min to separate plasma, which was stored at -80°C.

### 2.6 Sample treatment:

1) To 50 uL of plasma sample was added 250 uL of acetonitrile for precipitation, and the mixture was mixed and then centrifuged at 4°C at 4000 rpm for 20 minutes.
2) The treated supernatant solution was taken and subjected to LC/MS/MS to analyse the concentration of the test compounds. LC/MS/MS analysis instrument: AB Sciex API-4000 Qtrap-Shimadzu Controller-CBM20A.

### 2.7 Liquid phase analysis:

• Chromatographic column: Waters Xbridge C18 3.5 um 2.1*50 mm
• Mobile phase: solution A: 0.1% formic acid in water, and solution B: 0.1% formic acid in acetonitrile
• Flow rate: 0.6 mL/min
• Elution time: 0-3.5 minutes, with eluents as follows:

| Time/min | Solution A | Solution B |
|---|---|---|
| 0.01 | 98% | 2% |
| 0.30 | 98% | 2% |
| 1.20 | 2% | 98% |
| 2.50 | 2% | 98% |
| 2.51 | 98% | 2% |
| 3.50 | 98% | 2% |

**3. Experimental results and analysis:** The results of pharmacokinetic experiments in rats are shown in Table 3 (pharmacokinetic parameters) below.

**Table 3**

| Example No. | Pharmacokinetic experiment (PO, 30 mg/kg) | | | |
|---|---|---|---|---|
| | Time to peak | Plasma concentration | Area under the curve | Half-life |
| | tₘₐₓ (h) | Cₘₐₓ (ng/mL) | AUC₀₋ₜ (ng/mL*h) | t_{1/2} (h) |
| 556 | 2.0 | 2389 | 22077 | 2.3 |
| 559 | 2.0 | 2190 | 12726 | 2.7 |
| 586 | 2.0 | 1750 | 15299 | 4.3 |
| 613 | 2.0 | 3303 | 20957 | 2.6 |
| 624 | 1.0 | 3403 | 10951 | 1.0 |
| 627 | 4.0 | 2527 | 22061 | 3.2 |
| 628 | 4.0 | 2273 | 20876 | 3.3 |
| 646 | 2.0 | 3833 | 19810 | 2.4 |
| 731 | 2.0 | 1840 | 14284 | 2.3 |
| 736 | 2.0 | 1310 | 10795 | 2.4 |
| 739 | 2.0 | 1943 | 10346 | 2.5 |
| 763 | 4.0 | 1477 | 15915 | 3.4 |
| 764 | 4.0 | 1090 | 11681 | 4.1 |
| 770 | 4.0 | 1350 | 11566 | 3.5 |
| 774 | 4.0 | 2307 | 24398 | 4.5 |
| 787 | 2.0 | 2330 | 18784 | 2.4 |
| 816 | 4.0 | 2537 | 28402 | 3.8 |
| 818 | 2.0 | 2647 | 15455 | 3.1 |
| 849 | 2.0 | 2477 | 15505 | 2.8 |
| 854 | 1.0 | 2167 | 14865 | 3.0 |
| 857 | 2.0 | 2290 | 15928 | 3.5 |
| 864 | 4.0 | 1600 | 15652 | 3.7 |
| 928 | 4.0 | 2160 | 18946 | 4.5 |
| 938 | 4.0 | 1243 | 12513 | 6.0 |
| 948 | 4.0 | 1713 | 18832 | 9.3 |
| 958 | 4.0 | 1500 | 13165 | 9.4 |
| 992 | 2.0 | 4927 | 24645 | 2.9 |
| 994 | 4.0 | 2000 | 13135 | 2.4 |
| 1012 | 2.0 | 2220 | 14212 | 2.3 |
| 1014 | 4.0 | 1673 | 12777 | 2.4 |

4. Experimental conclusions: As can be seen from the results of pharmacokinetic experiments in rats in the table, the examples of the present invention exhibit excellent absorption and metabolic properties, and have significantly improved exposure (AUC) and plasma concentration.

### Test example 4. Pharmacokinetic determination in beagle dogs

1. **Experimental objective:** to study the pharmacokinetic behaviours of the compounds of the present invention in the body (plasma) of beagle dogs as test animals after oral administration at a dose of 3 mg/kg.
2. **Experimental scheme**
2.1 **Test drugs:** the examples of the present invention, prepared in house.
2.2 **Test animals:** beagle dogs (male, 3 dogs per group), from Guangxi Grand Forest Scientific Primate Company., Ltd., with the animal production license no.(SCXK (Gui) 2021-0004).
2.3 **Drug preparation:** Formulation of drug for oral administration: 30% PEG 400+70% (10% Solutol) in water

Solutol HS15 was melted in a water bath at 50-60°C, and then 50 mL of the melted Solutol HS15 was added to 450 mL of ultrapure water. The mixture was stirred and sonicated to obtain 500 mL of 10% Solutol HS15 solution. The compound was weighed, and according to the total administration volume, 30% PEG solution was first added. The mixture was shaken uniformly and sonicated until a clear solution was obtained. Subsequently, 70% (10% Solutol) in water was added, resulting in a colourless and clear solution with a concentration of 0.6 mg/mL.

2.4 **Administration scheme:** After overnight fasting, 3 male beagle dogs per group were administered orally (PO) at a dose of 3 mg/kg with an administration volume of 5 mL/kg.

2.5 **Sample collection:** At 0.25 h, 0.5 h, 1 h, 2 h, 4 h, 6 h, 8 h and 24 h after oral administration to the beagle dogs, blood was collected from the forelimb vein or other suitable vein. Each sample was collected in an amount of approximately 1 mL, anticoagulated with K2-EDTA and placed on ice. Blood samples were collected and placed on ice, and plasma was separated by centrifugation within 1 hour (the centrifugation was performed at 2-8°C at 2200 g for 10 minutes). Plasma samples were stored in a -80°C refrigerator before analysis, and the remaining plasma samples were stored in the -80°C refrigerator again after analysis.

### 2.6 Sample treatment:

1) To 40 uL of plasma sample was added 400 uL of methanol for precipitation, and the mixture was mixed and then centrifuged at 4000 rpm for 10 minutes.
2) The treated supernatant solution was taken and subjected to LC/MS/MS to analyse the concentration of the test compounds. LC/MS/MS analysis instrument: AB Sciex LC-MS/MS-29 (TQ6500+).

### 2.7 Liquid phase analysis:

• Chromatographic column: ACQUITY UPLC HSS T3 1.8 um 2.1*50 mm
• Mobile phase: solution A: 0.1% formic acid in water, and solution B: 0.1% formic acid in acetonitrile.
• Flow rate: 0.6 mL/min
• Elution time: 0-1.4 min, with eluents as follows:

| Time/min | Solution A | Solution B |
|---|---|---|
| 0.01 | 90% | 10% |
| 0.60 | 10% | 90% |
| 1.10 | 10% | 90% |
| 1.11 | 90% | 10% |
| 1.40 | 90% | 10% |

2.7 **Experimental results and conclusions:** the examples of the present invention exhibit excellent absorption and metabolic properties, and have excellent exposure and half-life; and the half-life of the examples of the present invention is more than 5 hours, and some examples even have a half-life of more than 6 hours.

### Test example 5 Pharmacological efficacy of compounds in 5% imiquimod-induced psoriasis model in Wistar rats

1. **Experimental objective:** to evaluate the pharmacological efficacy of the compounds in a 5% imiquimod-induced psoriasis model in Wistar rats
2. **Experimental scheme**
2.1 **Test drugs:** the examples of the present invention, prepared in house.
2.2 **Test animals:** Wistar rats (female, 5 rats per group, 6-7 weeks old), from Beijing Vital River Laboratory Animal Technology Co., Ltd., with ad libitum access to standard laboratory diet and water.
2.3 **Reagents:** 5% imiquimod cream (Sichuan Mingxin Pharmaceutical Co., Ltd.), dexamethasone (Shanghai Titan Pharmaceuticals), anti-IL-17A mouse mAb (BioXcell), Veetin hair removal cream (for sensitive skin), polyethylene glycol 400 (Sinopharm Chemical Reagent Co., Ltd.), Kolliphor^{®} HS15 (Sigma-Aldrich), and PBS pH 7.4 (1X, Gibco)
2.4 **Administration:** administration volume: 10 mL/kg; administration dosage for test compound group: 5-100 (e.g., 7.5, 15, 30, 50 or 100) mg/kg.
2.5 **Experimental scheme**

| No. | Model | 5% IMQ dosage (mg) | Administr ation volume (mL/kg) | Administra tion dosage (mg/kg) | Administration route/frequency | Number of animals |
|---|---|---|---|---|---|---|
| 1 | Normal group | - | - | - | - | 5 |
| 2 | Vehicle control group* | 75 | 10 | - | PO, BID*7 days | 5 |
| 3 | Dexamethasone group** | 75 | 10 | 3 | PO, QD*7 days | 5 |
| 4 | Anti-IL-17A antibody group*** | 75 | 10 | 30 | SC, BIW*1 week | 5 |

| | | | | | | |
|---|---|---|---|---|---|---|
| Notes: *30%PEG400 + 70% (10% Solutol) in water; **0.5% CMC-Na/1% Tween80; *** PBS | | | | | | |

2.6 **Experimental procedure and endpoint collection**
1. After 2-3 days of acclimatization, the dorsal skin of the rats in the modelling area was shaved and treated with an appropriate amount of depilatory cream (3 cm × 3 cm). The cream was thoroughly rinsed off with clean water within 5-10 minutes of application.
2. 2-5 days after depilation (depending on the actual condition of the dorsal skin after depilation), animals with complete hair removal and no skin damage were selected and grouped based on their body weight on that day.
3. From Day 1 to Day 6 of the experiment, animals were weighed daily and dosed according to the scheme (QD/BID, administration volume: 10 mL/kg). The dorsal skin in the modelling area was scored daily using the Psoriasis Area and Severity Index (PASI). The daily total PASI score was the sum of the scores for erythema, scaling and thickening (refer to Table 4 for scoring criteria). One hour after the administration, 75 mg of 5% imiquimod was applied to the dorsal modelling area.

**Table 4. PASI scoring criteria**

| Score | Erythema | Scaling | Thickening |
|---|---|---|---|
| 0 | None | None | None |
| 1 | Mild | Mild | Mild |
| 2 | Moderate | Moderate | Moderate |
| 3 | Severe | Severe | Severe |
| 4 | Very severe | Very severe | Very severe |

4. On Day 7 of the experiment, animals were weighed, and the dorsal skin in the modelling area was subjected to PASI scoring and photographed, wherein the animals in the test compound group were administrated sequentially, and at 0.5 h, 1 h, 2 h, 4 h, 6 h, 8 h and 24 h after the administration, blood samples were collected via jugular vein puncture (alternating veins, approximately 120 µL per sample) for plasma separation.
5. The collected blood samples were transferred to centrifuge tubes containing EDTA-K2, gently inverted 3-4 times manually, placed on ice, and centrifuged at 8000 rpm for 5 minutes. The plasma obtained by the centrifugation was transferred to new, pre-labelled centrifuge tubes. Approximately 50 uL was allocated for PK analysis, while the remaining plasma samples were flash-frozen on dry ice and then stored in the -80°C refrigerator.
6. After the blood collection at 8 h, the animals in the test compound group were euthanized. One section of the lesional dorsal skin was collected, weighed, minced, and transferred to a new grinding tube for determining the skin/plasma concentration ratio.
2.7 **Primary indicators:** D1-D7 body weight and PASI scores of dorsal skin, with photography at the endpoint.
2.8 **Experimental results and conclusions:** The examples of the present invention can effectively ameliorate psoriasis symptoms in the 5% imiquimod-induced psoriasis model in Wistar rats, demonstrating significant improvement in dorsal skin PASI scores. The antibody group achieves a therapeutic effect of 40%-50% on D7. Certain examples of the present invention, administered orally, can achieve a comparable technical effect to the antibody group.

## Claims

1. A compound as represented by formula (I-A), or a stereoisomer or pharmaceutically acceptable salt thereof: 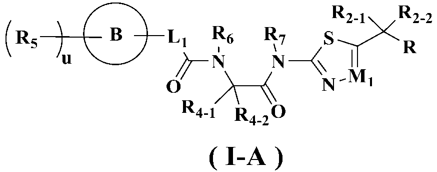 **characterised in that**:
M₁ is selected from N or CR₃;
L₁ is selected from a bond, -O-, -NH-, -CO-, -CONH-, C₁₋₃ alkylene, C₂₋₄ alkenylene, C₂₋₄ alkynylene, -O-C₁₋₃ alkylene-, -S-C₁₋₃ alkylene-, -C₁₋₃ alkylene-NH-, -C₁₋₃ alkylene-C(O)NH-, -C₁₋₃ alkylene-NHC(O)-, -C₁₋₃ alkylene-S(O)ᵣ₁-, -C₁₋₃ alkylene-NHS(O)ᵣ₂- or -C₁₋₃ alkylene-S(O)ᵣ₃NH-; the -NH-, -CONH-, C₁₋₃ alkylene, C₂₋₄ alkenylene, C₂₋₄ alkynylene, -O-C₁₋₃ alkylene-, -S-C₁₋₃ alkylene-, -C₁₋₃ alkylene-NH-, -C₁₋₃ alkylene-C(O)NH-, -C₁₋₃ alkylene-NHC(O)-, -C₁₋₃ alkylene-S(O)ᵣ₁-, -C₁₋₃ alkylene-NHS(O)ᵣ₂- or -C₁₋₃ alkylene-S(O)ᵣ₃NH- is optionally substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl; a bond is preferred;
ring B is selected from C₃₋₁₂ cycloalkyl, 3- to 12-membered heterocyclyl, C₆₋₁₄ aryl or 5- to 14-membered heteroaryl; or ring B is absent;
R is selected from C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₁₂ cycloalkyl, 3- to 12-membered heterocyclyl, C₆₋₁₄ aryl or 5- to 14-membered heteroaryl, wherein the C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₁₂ cycloalkyl, 3- to 12-membered heterocyclyl, C₆₋₁₄ aryl or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
alternatively, R₂₋₁ and R₂₋₂ are connected to form C₃₋₈ cycloalkyl, 3- to 8-membered heterocyclyl, C₆₋₁₀ aryl or 5- to 10-membered heteroaryl, wherein the C₃₋₈ cycloalkyl, 3- to 8-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
alternatively, R and R₂₋₂ are connected to form C₃₋₈ cycloalkyl, 3- to 8-membered heterocyclyl, C₆₋₁₀ aryl or 5- to 10-membered heteroaryl, wherein the C₃₋₈ cycloalkyl, 3- to 8-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₃ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, - (CH₂)ₒ₁ORₒ₁, -(CH₂)ₒ₂C(O)R_{c2}, -(CH₂)ₒ₃NHC(O)R_{c3}, -(CH₂)ₒ₄C(O)NHR_{c4}, - (CH₂)ₒ₅NR_{c5}R_{c6}, -(CH₂)ₒ₆S(O)ₒ₇R_{c7}, -(CH₂)ₒ₈S(O)₂NHR_{c8} or -(CH₂)ₒ₉NHS(O)₂Rₒ₉, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₄₋₁ and R₄₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₚ₁OR₄₁, -(CH₂)ₚ₂C(O)R_{d2}, - (CH₂)ₚ₃NHC(O)R_{d3}, -(CH₂)ₚ₄C(O)NHR_{d4}, -(CH₂)ₚ₅NR_{d5}R_{d6}, -(CH₂)ₚ₆S(O)ₚ₇P_{d7}, - (CH₂)ₚ₈S(O)₂NHR_{d8} or -(CH₂)ₚ₉NHS(O)₂R_{d9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
alternatively, R₄₋₁ and R₄₋₂ are connected to form C₃₋₈ cycloalkyl, 3- to 8-membered heterocyclyl, C₆₋₁₀ aryl or 5- to 10-membered heteroaryl, wherein the C₃₋₈ cycloalkyl, 3- to 8-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₅ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, - (CH₂)_{q1}ORₑ₁, -(CH₂)_{q2}C(O)Rₑ₂, -(CH₂)_{q3}NHC(O)Rₑ₃, -(CH₂)_{q4}C(O)NHRₑ₄, - (CH₂)_{q5}NRₑ₅Rₑ₆, -(CH₂)_{q6}S(O)_{q7}Rₑ₇, -(CH₂)_{q8}S(O)₂NHRₑ₈ or -(CH₂)_{q9}NHS(O)₂Rₑ₉, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₆ and R₇ are each independently selected from hydrogen, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₁₂ cycloalkyl, 3- to 12-membered heterocyclyl, C₆₋₁₄ aryl or 5- to 14-membered heteroaryl, wherein the C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₁₂ cycloalkyl, 3- to 12-membered heterocyclyl, C₆₋₁₄ aryl or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
Rₐ₁, Rₐ₂, Rₐ₃, Rₐ₄, Rₐ₅, Rₐ₆, Rₐ₇, Rₐ₈ and Rₐ₉ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{b1}, R_{b2}, R_{b3}, R_{b4}, R_{b5}, R_{b6}, R_{b7}, R_{b8} and R_{b9} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{c1}, R_{c2}, R_{c3}, R_{c4}, R_{c5}, R_{c6}, R_{c7}, R_{c8} and R_{c9} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{d1}, R_{d2}, R_{d3}, R_{d4}, R_{d5}, R_{d6}, R_{d7}, R_{d8} and Rₐ₉ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
Rₑ₁, Rₑ₂, Rₑ₃, Rₑ₄, Rₑ₅, Rₑ₆, Rₑ₇, Rₑ₈ and Rₑ₉ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
u is 0, 1, 2, 3, 4 or 5;
m1, m2, m3, m4, m5, m6, m8 and m9 are each independently selected from 0, 1, 2, 3, 4, 5 or 6;
n1, n2, n3, n4, n5, n6, n8 and n9 are each independently selected from 0, 1, 2, 3, 4, 5 or 6;
o1, o2, o3, o4, o5, o6, o8 and o9 are each independently selected from 0, 1, 2, 3, 4, 5 or 6;
p1, p2, p3, p4, p5, p6, p8 and p9 are each independently selected from 0, 1, 2, 3, 4, 5 or 6;
q1, q2, q3, q4, q5, q6, q8 and q9 are each independently selected from 0, 1, 2, 3, 4, 5 or 6;
m7, n7, o7, p7 and q7 are each independently selected from 0, 1, 2 or 3;
r1, r2 and r3 are each independently selected from 0, 1 or 2.

2. The compound or the stereoisomer or pharmaceutically acceptable salt thereof according to claim 1, **characterised in that** R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, - (CH₂)ₙ₁OR_{b1}, -(CH₂)ₙ₂C(O)R_{b2}, -(CH₂)ₙ₃NHC(O)R_{b3}, -(CH₂)ₙ₄C(O)NHR_{b4}, - (CH₂)ₙ₅NR_{b5}R_{b6}, -(CH₂)ₙ₆S(O)ₙ₇R_{b7}, -(CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, carboxyl, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl, wherein the C₃₋₈ cycloalkyl, 3- to 8-membered heterocyclyl, C₆₋₁₀ aryl and 5- to 10-membered heteroaryl substituents are optionally further substituted with one or more of hydrogen, deuterium, halogen, amino, hydroxyl, cyano, nitro, carboxyl, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl.

3. The compound or the stereoisomer or pharmaceutically acceptable salt thereof according to claim 1 or 2, **characterised in that** the compound is as represented by formula (I-A-1) 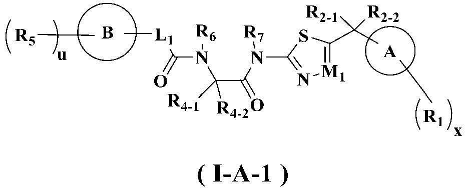 wherein
ring A is selected from C₃₋₁₂ cycloalkyl, 3- to 12-membered heterocyclyl, C₆₋₁₄ aryl or 5- to 14-membered heteroaryl;
R₁ is independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₘ₁ORₐ₁, -(CH₂)ₘ₂C(O)Rₐ₂, -(CH₂)ₘ₃NHC(O)Rₐ₃, - (CH₂)ₘ₄C(O)NHRₐ₄, -(CH₂)ₘ₅NRₐ₅Rₐ₆, -(CH₂)ₘ₆S(O)ₘ₇Rₐ₇, -(CH₂)ₘ₈S(O)₂NHRₐ₈ or -(CH₂)ₘ₉NHS(O)₂Rₐ₉, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5-to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
M₁ is selected from N or CR₃;
L₁ is selected from a bond, -O-, -NH-, -CO-, -CONH-, C₁₋₃ alkylene, C₂₋₄ alkenylene, C₂₋₄ alkynylene, -O-C₁₋₃ alkylene-, -S-C₁₋₃ alkylene-, -C₁₋₃ alkylene-NH-, -C₁₋₃ alkylene-C(O)NH-, -C₁₋₃ alkylene-NHC(O)-, -C₁₋₃ alkylene-S(O)ᵣ₁-, -C₁₋₃ alkylene-NHS(O)ᵣ₂- or -C₁₋₃ alkylene-S(O)ᵣ₃NH-; the -NH-, -CONH-, C₁₋₃ alkylene, C₂₋₄ alkenylene, C₂₋₄ alkynylene, -O-C₁₋₃ alkylene-, -S-C₁₋₃ alkylene-, -C₁₋₃ alkylene-NH-, -C₁₋₃ alkylene-C(O)NH-, -C₁₋₃ alkylene-NHC(O)-, -C₁₋₃ alkylene-S(O)ᵣ₁-, -C₁₋₃ alkylene-NHS(O)ᵣ₂- or -C₁₋₃ alkylene-S(O)ᵣ₃NH- is optionally substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl; a bond is preferred;
ring B is selected from C₃₋₁₂ cycloalkyl, 3- to 12-membered heterocyclyl, C₆₋₁₄ aryl or 5- to 14-membered heteroaryl; or ring B is absent;
R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₁₂ cycloalkyl, 3- to 12-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₁₂ cycloalkyl, 3- to 12-membered heterocyclyl, C₆₋₁₀ aryl or 5- to 10-membered heteroaryl may optionally be further substituted with one or more of hydrogen, deuterium, halogen, amino, hydroxyl, cyano, nitro, carboxyl, oxo, thio, =CF₂, =CHF, =NOC₁₋₆ alkyl, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₁₂ cycloalkyl, 3- to 12-membered heterocyclyl, C₆₋₁₀ aryl and 5- to 10-membered heteroaryl; the C₃₋₁₂ cycloalkyl, 3- to 12-membered heterocyclyl, C₆₋₁₀ aryl and 5- to 10-membered heteroaryl substituents are optionally further substituted with one or more of hydrogen, deuterium, halogen, amino, hydroxyl, cyano, nitro, carboxyl, oxo, thio, =CF₂, =CHF, =NOC₁₋₆ alkyl, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ heteroalkyl, C₁₋₆ deuteroheteroalkyl, C₁₋₆ haloheteroalkyl, C₁₋₆ hydroxyalkyl, C₃₋₈ cycloalkyl, 3- to 8-membered heterocyclyl, C₆₋₁₀ aryl and 5- to 10-membered heteroaryl; the C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ heteroalkyl, C₁₋₆ deuteroheteroalkyl, C₁₋₆ haloheteroalkyl, C₁₋₆ hydroxyalkyl, C₃₋₈ cycloalkyl, 3- to 8-membered heterocyclyl, C₆₋₁₀ aryl and 5- to 10-membered heteroaryl are optionally further substituted with one or more of hydrogen, deuterium, halogen, amino, hydroxyl, cyano, nitro, carboxyl, oxo, thio, =CF₂, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ heteroalkyl, C₁₋₆ deuteroheteroalkyl, C₁₋₆ haloheteroalkyl, C₁₋₆ hydroxyalkyl, C₃₋₈ cycloalkyl, 3- to 8-membered heterocyclyl, C₆₋₁₀ aryl and 5- to 10-membered heteroaryl;
preferably, R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, carboxyl, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl, wherein the C₃₋₈ cycloalkyl, 3- to 8-membered heterocyclyl, C₆₋₁₀ aryl and 5- to 10-membered heteroaryl substituents are optionally further substituted with one or more of hydrogen, deuterium, halogen, amino, hydroxyl, cyano, nitro, carboxyl, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
preferably, R₂₋₁ and R₂₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
alternatively, R₂₋₁ and R₂₋₂ are connected to form C₃₋₈ cycloalkyl, 3- to 8-membered heterocyclyl, C₆₋₁₀ aryl or 5- to 10-membered heteroaryl, wherein the C₃₋₈ cycloalkyl, 3- to 8-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₃ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, - (CH₂)ₒ₁OR_{c1}, -(CH₂)ₒ₂C(O)R_{c2}, -(CH₂)ₒ₃NHC(O)R_{c3}, -(CH₂)ₒ₄C(O)NHR_{c4}, - (CH₂)ₒ₅NR_{c5}R_{c6}, -(CH₂)ₒ₆S(O)ₒ₇R_{c7}, -(CH₂)ₒ₈S(O)₂NHR_{c8} or -(CH₂)ₒ₉NHS(O)₂R_{c9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₄₋₁ and R₄₋₂ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₚ₁OR_{d1}, -(CH₂)ₚ₂C(O)R_{d2}, - (CH₂)ₚ₃NHC(O)R_{d3}, -(CH₂)ₚ₄C(O)NHR_{d4}, -(CH₂)ₚ₅NR_{d5}R_{d6}, -(CH₂)ₚ₆S(O)ₚ₇R_{d7}, - (CH₂)ₚ₈S(O)₂NHR_{d8} or -(CH₂)ₚ₉NHS(O)₂R_{d9}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
alternatively, R₄₋₁ and R₄₋₂ are connected to form C₃₋₈ cycloalkyl, 3- to 8-membered heterocyclyl, C₆₋₁₀ aryl or 5- to 10-membered heteroaryl, wherein the C₃₋₈ cycloalkyl, 3- to 8-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₅ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, - (CH₂)_{q1}ORₑ₁, -(CH₂)_{q2}C(O)Rₑ₂, -(CH₂)_{q3}NHC(O)Rₑ₃, -(CH₂)_{q4}C(O)NHRₑ₄, - (CH₂)_{q5}NRₑ₅Rₑ₆, -(CH₂)_{q6}S(O)_{q7}Rₑ₇, -(CH₂)_{q8}S(O)₂NHRₑ₈ or -(CH₂)_{q9}NHS(O)₂Rₑ₉, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₆ and R₇ are each independently selected from hydrogen, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₁₂ cycloalkyl, 3- to 12-membered heterocyclyl, C₆₋₁₄ aryl or 5- to 14-membered heteroaryl, wherein the C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₁₂ cycloalkyl, 3- to 12-membered heterocyclyl, C₆₋₁₄ aryl or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
Rₐ₁, Rₐ₂, Rₐ₃, Rₐ₄, Rₐ₅, Rₐ₆, Rₐ₇, Rₐ₈ and Rₐ₉ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{b1}, R_{b2}, R_{b3}, R_{b4}, R_{b5}, R_{b6}, R_{b7}, R_{b8} and R_{b9} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{c1}, R_{c2}, R_{c3}, R_{c4}, R_{c5}, R_{c6}, R_{c7}, R_{c8} and R_{c9} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{d1}, R_{d2}, R_{d3}, R_{d4}, R_{d5}, R_{d6}, R_{d7}, R_{d8} and R_{d9} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
Rₑ₁, Rₑ₂, Rₑ₃, Rₑ₄, Rₑ₅, Rₑ₆, Rₑ₇, Rₑ₈ and Rₑ₉ are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3-to 8-membered heterocyclyl, C₆₋₁₀ aryl or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
u is 0, 1, 2, 3, 4 or 5;
m1, m2, m3, m4, m5, m6, m8 and m9 are each independently selected from 0, 1, 2, 3, 4, 5 or 6;
n1, n2, n3, n4, n5, n6, n8 and n9 are each independently selected from 0, 1, 2, 3, 4, 5 or 6;
o1, o2, o3, o4, o5, o6, o8 and o9 are each independently selected from 0, 1, 2, 3, 4, 5 or 6;
p1, p2, p3, p4, p5, p6, p8 and p9 are each independently selected from 0, 1, 2, 3, 4, 5 or 6;
q1, q2, q3, q4, q5, q6, q8 and q9 are each independently selected from 0, 1, 2, 3, 4, 5 or 6;
m7, n7, o7, p7 and q7 are each independently selected from 0, 1, 2 or 3;
r1, r2 and r3 are each independently selected from 0, 1 or 2;
x is 0, 1, 2, 3, 4 or 5.

4. The compound or the stereoisomer or pharmaceutically acceptable salt thereof according to any one of claims 1-3, **characterised in that** the compound is as represented by formula (VII): 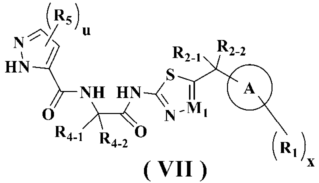 wherein
ring A is selected from C₃₋₁₂ cycloalkyl, 3- to 12-membered heterocyclyl, C₆₋₁₄ aryl or 5- to 14-membered heteroaryl;
R₁ is independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₘ₁ORₐ₁, -(CH₂)ₘ₂C(O)Rₐ₂, -(CH₂)ₘ₃NHC(O)Rₐ₃, - (CH₂)ₘ₄C(O)NHRₐ₄, -(CH₂)ₘ₅NRₐ₅Rₐ₆, -(CH₂)ₘ₆S(O)ₘ₇Rₐ₇, -(CH₂)ₘ₈S(O)₂NHRₐ₈ or -(CH₂)ₘ₉NHS(O)₂Rₐ₉, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5-to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
x is 0, 1, 2, 3, 4 or 5.

5. The compound or the stereoisomer or pharmaceutically acceptable salt thereof according to claim 4, **characterised in that** the compound is as represented by formula (VII-B), 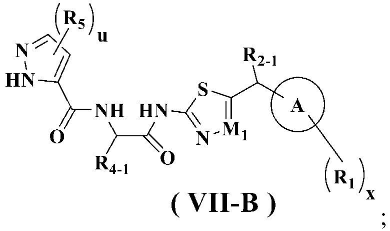
preferably, the compound is as represented by formula (VII-B-1) or (VII-B-2), 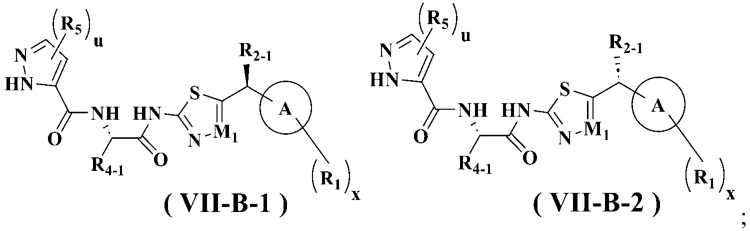
further preferably, the compound is as represented by formula (VII-1) or (VII-2), 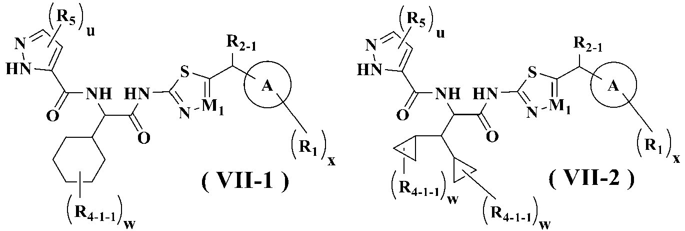
wherein R₄₋₁₋₁ is independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl;
w is selected from 0, 1, 2, 3, 4, 5 or 6;
further preferably, the compound is as represented by formula (VII-1-1), (VII-1-2), (VII-2-1) or (VII-2-2),

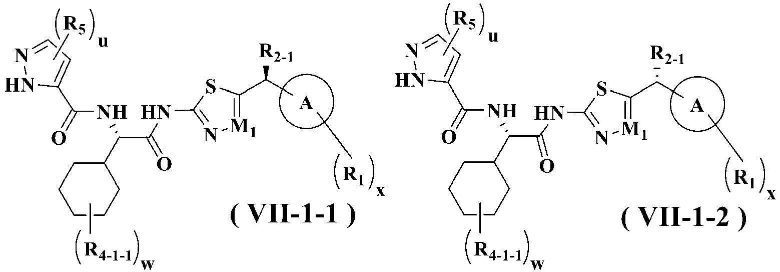

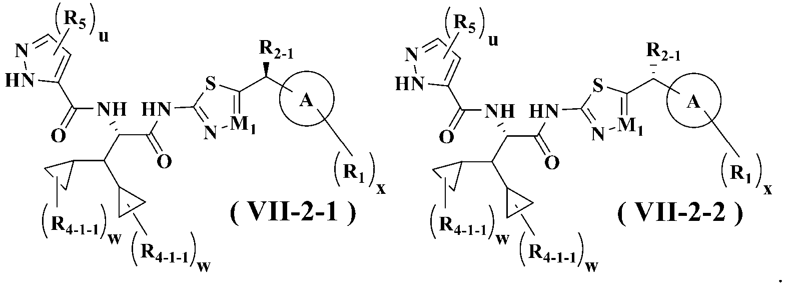

6. The compound or the stereoisomer or pharmaceutically acceptable salt thereof according to any one of claims 2-5, **characterised in that**
ring A is selected from C₃₋₈ cycloalkyl, 3- to 10-membered heterocyclyl, C₆₋₁₀ aryl or 5- to 10-membered heteroaryl;
preferably 5- to 6-membered heterocyclyl, 5- to 6-membered heteroaryl, 5- to 6-membered heteroaryl fused 5- to 6-membered heteroaryl, 5- to 6-membered heteroaryl fused phenyl, or 5- to 6-membered heteroaryl fused 5- to 6-membered heterocyclyl;
more preferably 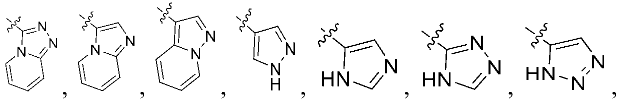 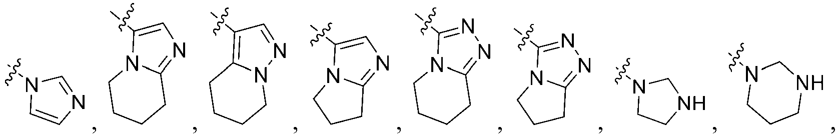 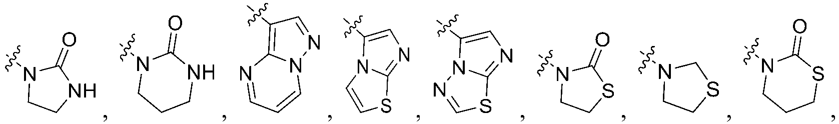 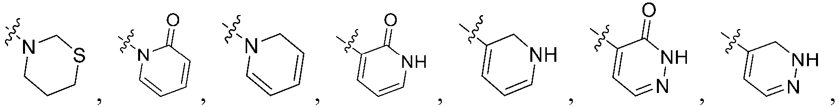 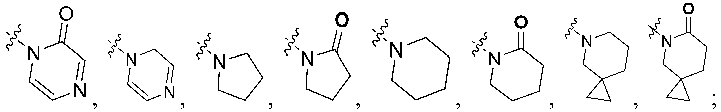
more preferably 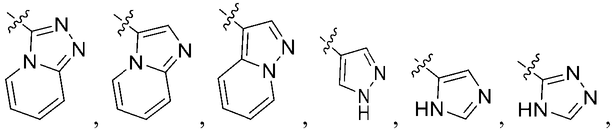 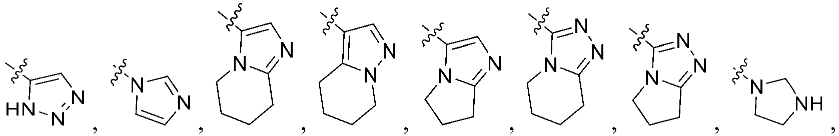 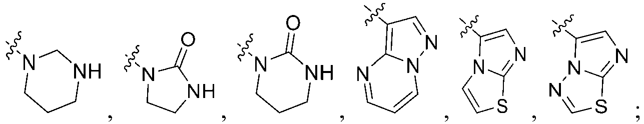
more preferably

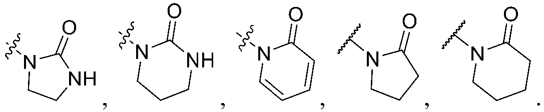

7. The compound or the stereoisomer or pharmaceutically acceptable salt thereof according to any one of claims 1-3, **characterised in that** the compound is as represented by formula (IV) or formula (IV-A), 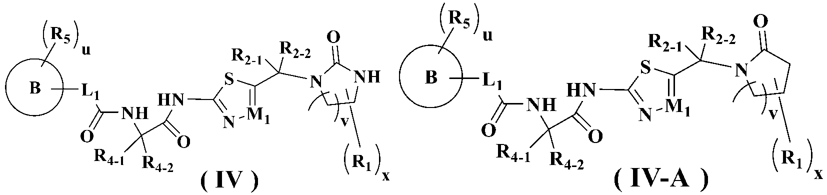 wherein
ring B is selected from C₃₋₁₂ cycloalkyl, 3- to 12-membered heterocyclyl, C₆₋₁₄ aryl or 5- to 14-membered heteroaryl; or ring B is absent, and 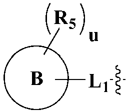 is R₅ - L₁- 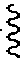 -;
R₁ is independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -(CH₂)ₘ₁ORₐ₁, -(CH₂)ₘ₂C(O)Rₐ₂, -(CH₂)ₘ₃NHC(O)Rₐ₃, - (CH₂)ₘ₄C(O)NHRₐ₄, -(CH₂)ₘ₅NRₐ₅Rₐ₆, -(CH₂)ₘ₆S(O)ₘ₇Rₐ₇, -(CH₂)ₘ₈S(O)₂NHRₐ₈ or -(CH₂)ₘ₉NHS(O)₂Rₐ₉, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5-to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
x is 0, 1, 2, 3, 4 or 5;
v is 1, 2, 3 or 4.

8. The compound or the stereoisomer or pharmaceutically acceptable salt thereof according to claim 7, **characterised in that** the compound is as represented by formula (IV-1), formula (IV-2), formula (IV-A-1) or formula (IV-A-2), 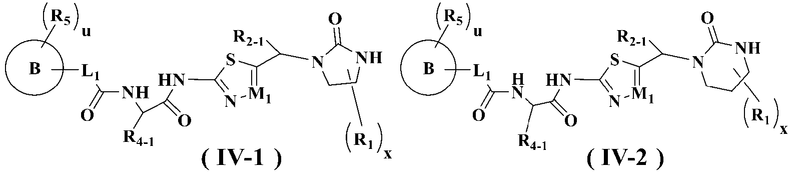 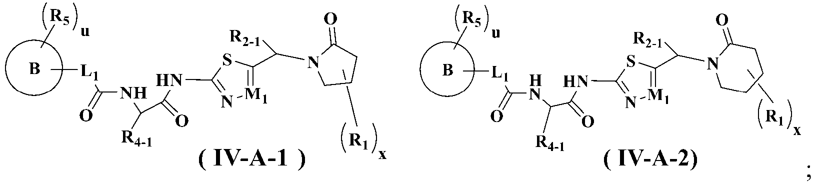 preferably, the compound is as represented by formula (IV-1-1), formula (IV-2-1), formula (IV-A-3) or formula (IV-A-4),

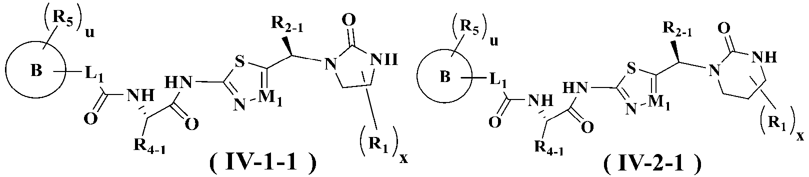

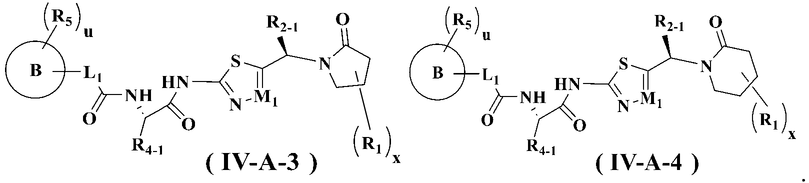

9. The compound or the stereoisomer or pharmaceutically acceptable salt thereof according to any one of claims 1-3, **characterised in that** the compound is as represented by formula (I-AAA) 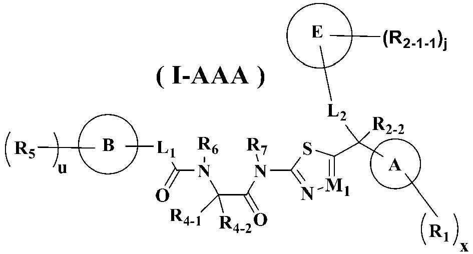
wherein: L₂ is selected from a bond, -O-, -NH-, -CO-, -CONH-, C₁₋₃ alkylene, C₂₋₄ alkenylene, C₂₋₄ alkynylene, -O-C₁₋₃ alkylene-, -S-C₁₋₃ alkylene-, -C₁₋₃ alkylene-NH-, -C₁₋₃ alkylene-C(O)NH-, -C₁₋₃ alkylene-NHC(O)-, -C₁₋₃ alkylene-S(O)ᵣ₄-, -C₁₋₃ alkylene-NHS(O)ᵣ₅- or -C₁₋₃ alkylene-S(O)ᵣ₆NH-; the -NH-, -CONH-, C₁₋₃ alkylene, C₂₋₄ alkenylene, C₂₋₄ alkynylene, -O-C₁₋₃ alkylene-, -S-C₁₋₃ alkylene-, -C₁₋₃ alkylene-NH-, -C₁₋₃ alkylene-C(O)NH-, -C₁₋₃ alkylene-NHC(O)-, -C₁₋₃ alkylene-S(O)ᵣ₄-, -C₁₋₃ alkylene-NHS(O)ᵣ₅- or -C₁₋₃ alkylene-S(O)ᵣ₆NH- is optionally substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl;
ring E is selected from C₃₋₁₂ cycloalkyl, 3- to 12-membered heterocyclyl, C₆₋₁₀ aryl, or 5- to 10-membered heteroaryl, preferably 3- to 12-membered heterocyclyl;
each R₂₋₁₋₁ is independently selected from hydrogen, deuterium, halogen, amino, hydroxyl, cyano, nitro, carboxyl, oxo, thio, =CF₂, =CHF, =NOC₁₋₆ alkyl, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ heteroalkyl, C₁₋₆ deuteroheteroalkyl, C₁₋₆ haloheteroalkyl, C₁₋₆ hydroxyalkyl, C₃₋₈ cycloalkyl, 3- to 8-membered heterocyclyl, C₆₋₁₀ aryl or 5- to 10-membered heteroaryl, wherein the C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ heteroalkyl, C₁₋₆ deuteroheteroalkyl, C₁₋₆ haloheteroalkyl, C₁₋₆ hydroxyalkyl, C₃₋₈ cycloalkyl, 3- to 8-membered heterocyclyl, C₆₋₁₀ aryl and 5- to 10-membered heteroaryl are optionally further substituted with one or more of hydrogen, deuterium, halogen, amino, hydroxyl, cyano, nitro, carboxyl, oxo, thio, =CF₂, =CHF, =NOC₁₋₆ alkyl, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ heteroalkyl, C₁₋₆ deuteroheteroalkyl, C₁₋₆ haloheteroalkyl, C₁₋₆ hydroxyalkyl, C₃₋₈ cycloalkyl, 3- to 8-membered heterocyclyl, C₆₋₁₀ aryl and 5- to 10-membered heteroaryl, and R₂₋₁₋₁ is preferably methyl, ethyl, methoxy, F, =CF₂, -CHF₂, -CH₂F, -CF₃, -CH₂CHF₂, - CHF₂CH₃, -CH₂CF₃, -CH₂OCH₃, -CH₂OCD₃, -CH₂OCF₃, -CH₂O cyclopropyl, - CH₂SCF₃, cyclopropyl, cyclobutyl, OCD₃ or OCF₃;
j is 0, 1, 2, 3, 4 or 5;
r4, r5 and r6 are each independently selected from 0,1 or 2;
v is 1, 2, 3 or 4.

10. The compound or the stereoisomer or pharmaceutically acceptable salt thereof according to claim 9, **characterised in that** the compound is as represented by formula (I-AAA-1) or (I-AAA-2) 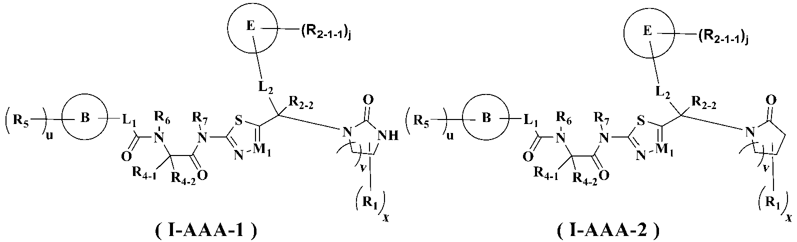 v is 1, 2, 3 or 4.

11. The compound or the stereoisomer or pharmaceutically acceptable salt thereof according to any one of claims 1-3, **characterised in that** the compound is as represented by formula (I-AA-1) or (I-AA-2) 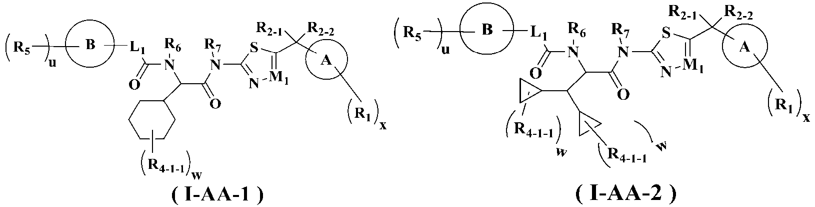
wherein R₄₋₁₋₁ is independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl or 5- to 10-membered heteroaryl;
w is selected from 0, 1, 2, 3, 4, 5 or 6.

12. The compound or the stereoisomer or pharmaceutically acceptable salt thereof according to any one of claims 1-3 and 6-11, **characterised in that**
ring B is selected from C₃₋₆ cycloalkyl, 3- to 8-membered heterocyclyl, C₆₋₁₀ aryl or 5- to 10-membered heteroaryl; or ring B is absent;
preferably, ring B is selected from C₃₋₆ cycloalkyl, phenyl or 5- to 6-membered heteroaryl; or ring B is absent;
more preferably, ring B is selected from 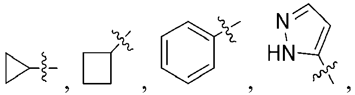 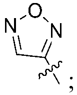 or ring B is absent;
further preferably, ring B is 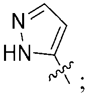 further preferably, ring B is

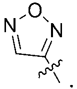

13. The compound or the stereoisomer or pharmaceutically acceptable salt thereof according to any one of claims 1-3, **characterised in that** the compound is as represented by formula (I-AA), (I-AA-3) or (I-AA-4) 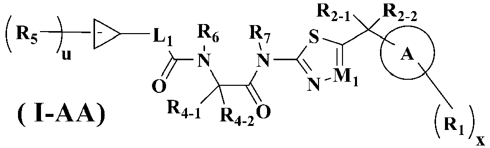 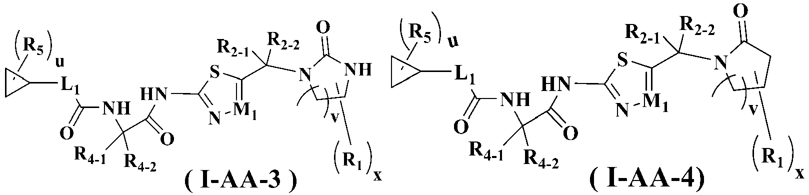 v is 1, 2, 3 or 4.

14. The compound or the stereoisomer or pharmaceutically acceptable salt thereof according to any one of claims 1-13, **characterised in that**
L₁ is selected from a bond, O or-CF₂-; further preferably, L₁ is a bond; further preferably, L₁ is O; further preferably, L₁ is selected from a bond or-CF₂-;
and/or
R₁ is independently selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl, 5- to 6-membered heteroaryl, -(CH₂)ₘ₁ORₐ₁, -(CH₂)ₘ₂C(O)Rₐ₂, -(CH₂)ₘ₃NHC(O)Rₐ₃, - (CH₂)ₘ₄C(O)NHRₐ₄, -(CH₂)ₘ₅NRₐ₅Rₐ₆, -(CH₂)ₘ₆S(O)ₘ₇Rₐ₇, -(CH₂)ₘ₈S(O)₂NHRₐ₈ or -(CH₂)ₘ₉NHS(O)₂Rₐ₉, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5-to 6-membered heteroaryl may optionally be further substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl;
Rₐ₁, Rₐ₂, Rₐ₃, Rₐ₄, Rₐ₅, Rₐ₆, Rₐ₇, Rₐ₈ and Rₐ₉ are each independently selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3-to 6-membered heterocyclyl, C₆₋₁₀ aryl or 5- to 6-membered heteroaryl may optionally be further substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl;
preferably, R₁ is hydrogen, deuterium, oxo, trifluoromethyl, fluoro, chloro, bromo, -CH₂CF₃, cyano, methoxy, -CH=CF₂ or -CH₂CHF₂;
preferably, R₁ is hydrogen, deuterium, oxo, trifluoromethyl, fluoro, -CH₂CF₃, cyano, methoxy, -CH=CF₂ or -CH₂CHF₂;
more preferably, R₁ is oxo, trifluoromethyl, fluoro, -CH₂CF₃ or cyano;
and/or
R₂₋₁ and R₂₋₂ are each independently selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl, 5- to 6-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl may optionally be further substituted with one or more of hydrogen, deuterium, halogen, amino, hydroxyl, cyano, nitro, carboxyl, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl, wherein the C₃₋₆ cycloalkyl, 3- to 6-membered heterocyclyl, C₆₋₁₀ aryl and 5- to 6-membered heteroaryl substituents are optionally further substituted with one or more of hydrogen, deuterium, halogen, amino, hydroxyl, cyano, nitro, carboxyl, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl;
preferably, R₂₋₁ and R₂₋₂ are each independently selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl, 5- to 6-membered heteroaryl, -(CH₂)ₙ₁OR_{b1}, -(CH₂)ₙ₂C(O)R_{b2}, - (CH₂)ₙ₃NHC(O)R_{b3}, -(CH₂)ₙ₄C(O)NHR_{b4}, -(CH₂)ₙ₅NR_{b5}R_{b6}, -(CH₂)ₙ₆S(O)ₙ₇R_{b7}, - (CH₂)ₙ₈S(O)₂NHR_{b8} or -(CH₂)ₙ₉NHS(O)₂R_{b9}, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl may optionally be further substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl;
alternatively, R₂₋₁ and R₂₋₂ are connected to form C₃₋₈ cycloalkyl, 3- to 8-membered heterocyclyl, C₆₋₁₀ aryl or 5- to 10-membered heteroaryl, wherein the C₃₋₈ cycloalkyl, 3- to 8-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl;
R_{b1}, R_{b2}, R_{b3}, R_{b4}, R_{b5}, R_{b6}, R_{b7}, R_{b8} and R_{b9} are each independently selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3-to 6-membered heterocyclyl, C₆₋₁₀ aryl or 5- to 6-membered heteroaryl may optionally be further substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl;
preferably, R₂₋₁ is 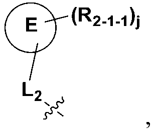 and preferably ring E is 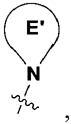 wherein ring E' is selected from 3- to 12-membered heterocyclyl, preferably 3- to 8-membered monocyclic heterocyclyl, 3- to 8-membered fused heterocyclyl, 3- to 8-membered bridged heterocyclyl or 3- to 8-membered spiroheterocyclyl, more preferably 3- to 8-membered monocyclic heterocyclyl, 3- to 8-membered fused heterocyclyl or 3- to 8-membered spiroheterocyclyl, more preferably 5- to 9-membered heterocyclyl, more preferably 5- to 6-membered heterocyclyl; preferably, ring E' is selected from 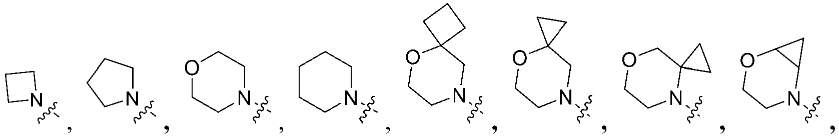 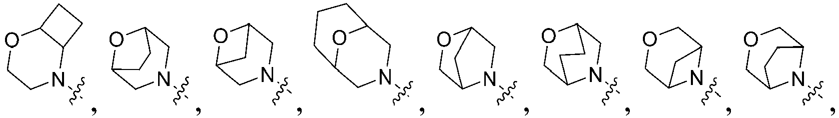 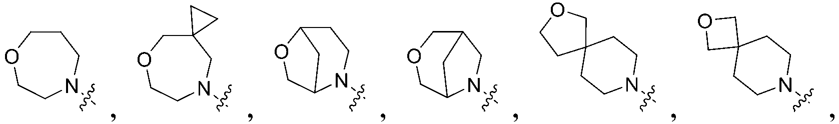 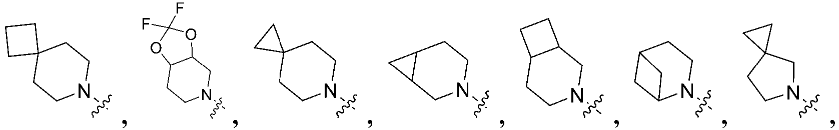 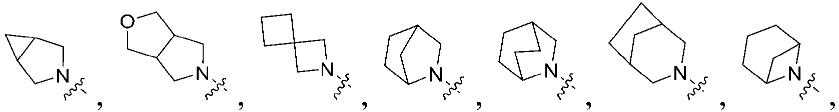 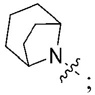
preferably, L₂ is CH₂;
preferably, R₂₋₁ is selected from hydrogen, methyl, 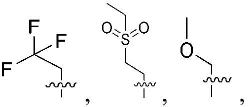 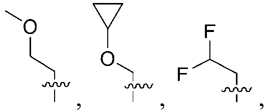 cyclopropyl, -CH=CF₂, -CH₂-cyclopropyl, -OCH₂OCD₃, -CH₂OCD₃, -CH₂OCHF₂, -CH₂COOCH₂CH₃, -CH₂COOH, -CH₂CH₂COOH, 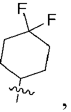 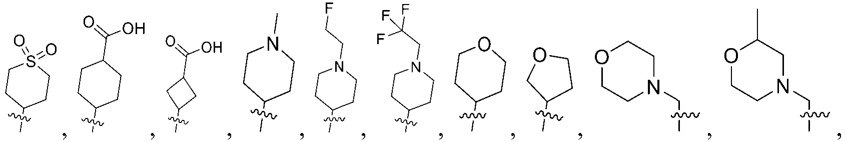 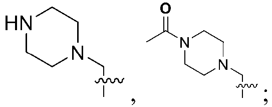
preferably, R₂₋₂ is selected from hydrogen or methyl; preferably, R₂₋₂ is hydrogen;
and/or
R₄₋₁ and R₄₋₂ are each independently selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₈ cycloalkyl, 3- to 6-membered heterocyclyl, C₆₋₁₀ aryl, 5- to 6-membered heteroaryl, -(CH₂)ₚ₁OR_{d1}, -(CH₂)ₚ₂C(O)R_{d2}, - (CH₂)ₚ₃NHC(O)R_{d3}, -(CH₂)ₚ₄C(O)NHR_{d4}, -(CH₂)ₚ₅NR_{d5}R_{d6}, -(CH₂)ₚ₆S(O)ₚ₇R_{d7}, - (CH₂)ₚ₈S(O)₂NHR_{d8} or -(CH₂)ₚ₉NHS(O)₂R_{d9}, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₈ cycloalkyl, 3- to 6-membered heterocyclyl, C₆₋₁₀ aryl or 5- to 6-membered heteroaryl may optionally be further substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl;
alternatively, R₄₋₁ and R₄₋₂ are connected to form C₃₋₈ cycloalkyl, 3- to 8-membered heterocyclyl, C₆₋₁₀ aryl or 5- to 10-membered heteroaryl, wherein the C₃₋₈ cycloalkyl, 3- to 8-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl;
R_{d1}, R_{d2}, R_{d3}, R_{d4}, R_{d5}, R_{d6}, R_{d7}, R_{d8} and R_{d9} are each independently selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3-to 6-membered heterocyclyl, C₆₋₁₀ aryl or 5- to 6-membered heteroaryl may optionally be further substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl;
preferably, R₄₋₁ is selected from 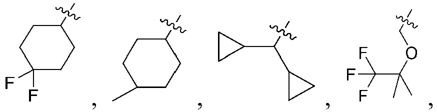 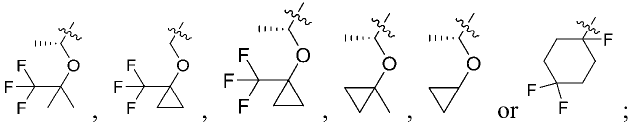 preferably, R₄₋₁ is selected from 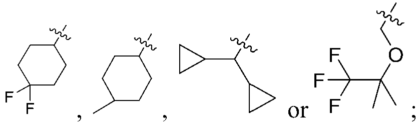 preferably, R₄₋₁ is selected from 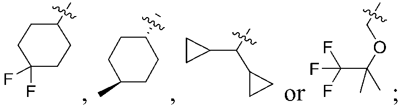
more preferably, R₄₋₁ is selected from 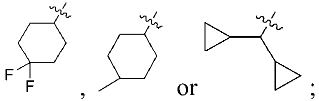 more preferably, R₄₋₁ is selected from 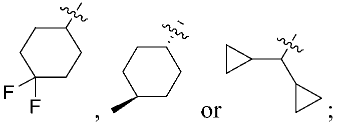 more preferably, R₄₋₁ is 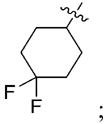 more preferably, R₄₋₁ is 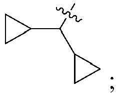 more preferably, R₄₋₁ is 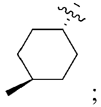
more preferably, R₄₋₂ is hydrogen;
and/or
R₅ is selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl, 5- to 6-membered heteroaryl, - (CH₂)_{q1}ORₑ₁, -(CH₂)_{q2}C(O)Rₑ₂, -(CH₂)_{q3}NHC(O)Rₑ₃, -(CH₂)_{q4}C(O)NHRₑ₄, - (CH₂)_{q5}NRₑ₅Rₑ₆, -(CH₂)_{q6}S(O)_{q7}Rₑ₇, -(CH₂)_{q8}S(O)₂NHRₑ₈ or -(CH₂)_{q9}NHS(O)₂Rₑ₉, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl may optionally be further substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl;
Rₑ₁, Rₑ₂, Rₑ₃, Rₑ₄, Rₑ₅, Rₑ₆, Rₑ₇, Rₑ₈ and Rₑ₉ are each independently selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl or 5- to 6-membered heteroaryl, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3-to 6-membered heterocyclyl, C₆₋₁₀ aryl or 5- to 6-membered heteroaryl may optionally be further substituted with one or more of hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl;
preferably, R₅ is selected from hydrogen, fluoro, methyl, isopropyl, 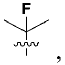 trifluoromethyl, monofluoromethyl, cyano, cyclopropyl, ethyl, deuterated ethyl, cyclobutyl, cyclopentyl, cyclohexyl, 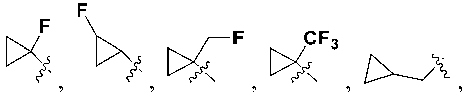 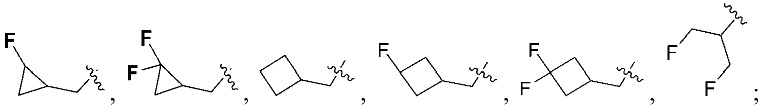
preferably, R₅ is selected from hydrogen, fluoro, methyl, isopropyl, 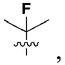 trifluoromethyl, monofluoromethyl, cyano, cyclopropyl or ethyl;
more preferably, R₅ is selected from hydrogen, fluoro, methyl, isopropyl, 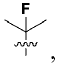 trifluoromethyl, monofluoromethyl or cyano;
and/or
R₄₋₁₋₁ is independently selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ deuteroalkoxy, C₁₋₃ haloalkoxy, C₁₋₃ hydroxyalkyl, C₃₋₆ cycloalkyl, 3- to 6-membered heterocyclyl, C₆₋₁₀ aryl and 5- to 6-membered heteroaryl;
preferably, R₄₋₁₋₁ is independently selected from hydrogen, fluoro or methyl.

15. The compound or the stereoisomer or pharmaceutically acceptable salt thereof according to claim 1, **characterised in that** the compound is as represented by:

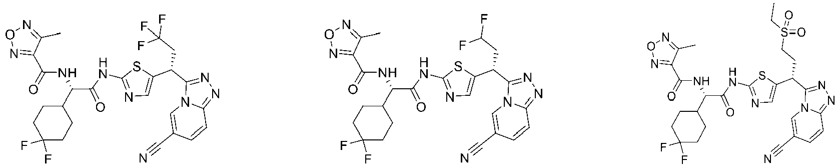

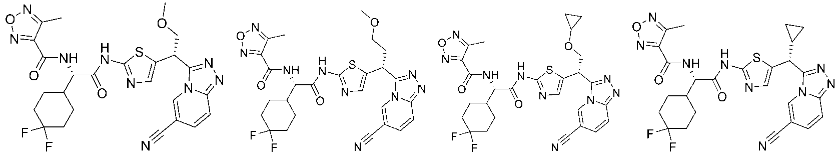

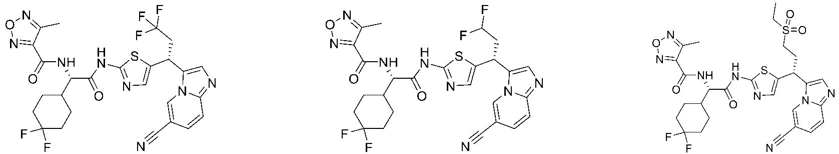

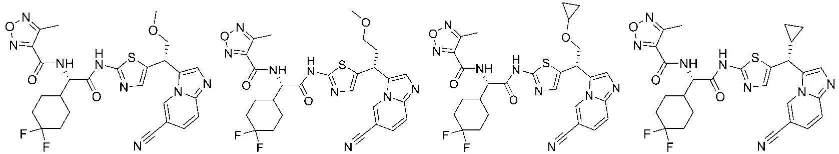

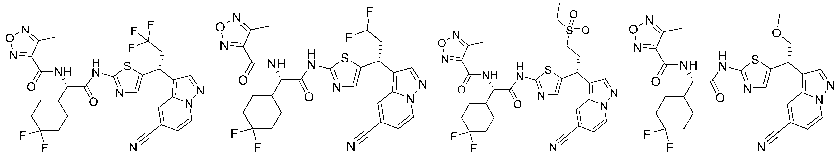

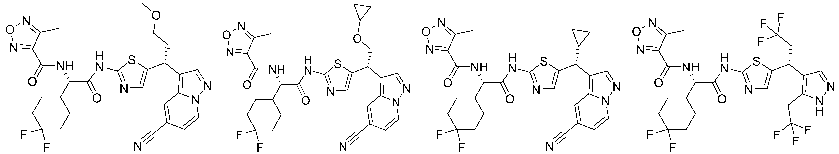

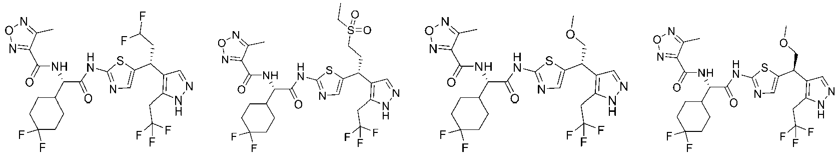

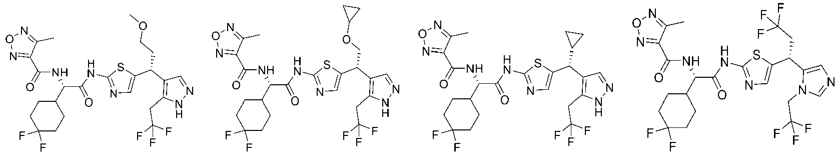

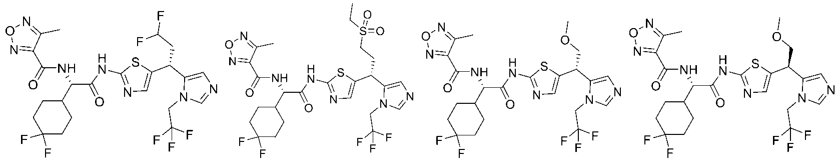

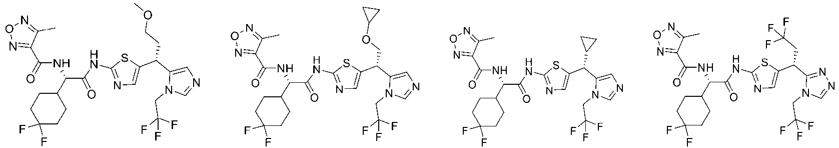

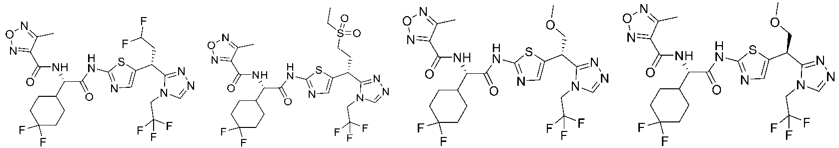

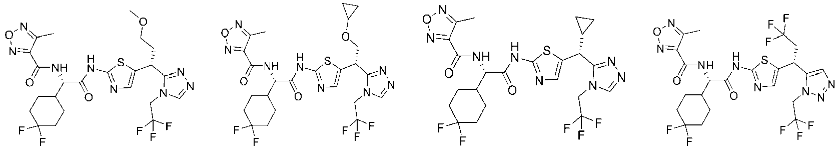

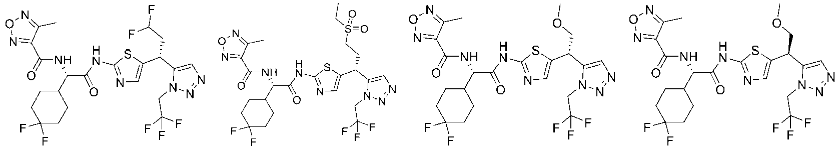

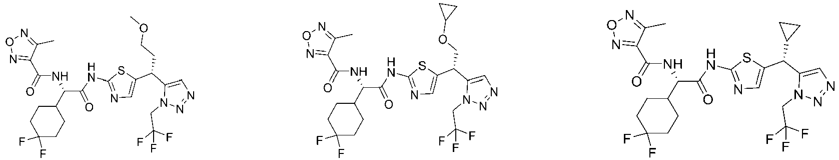

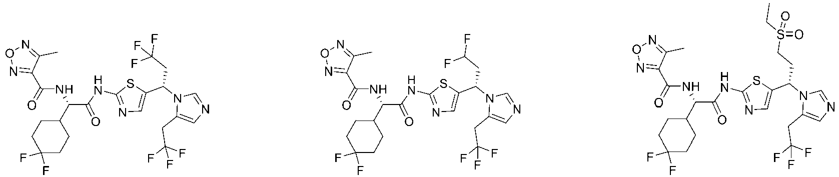

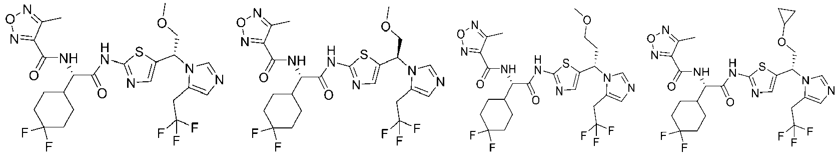

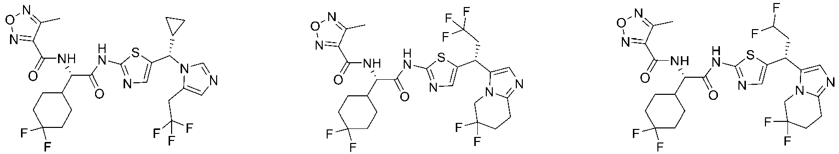

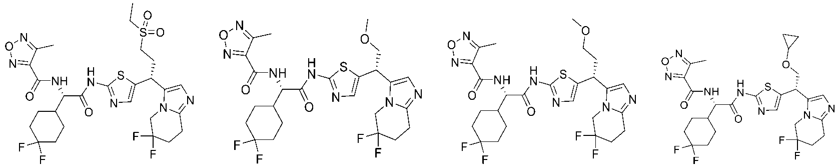

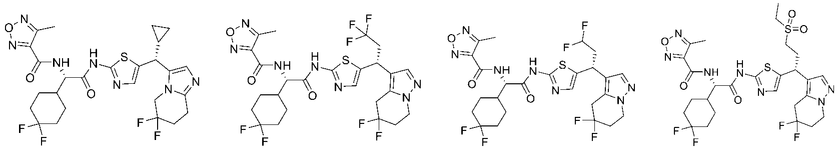

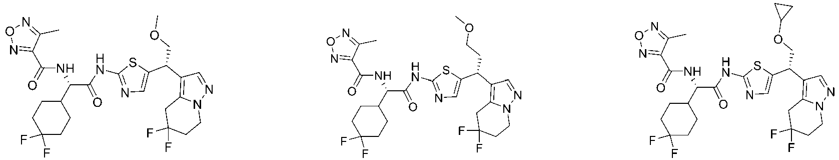

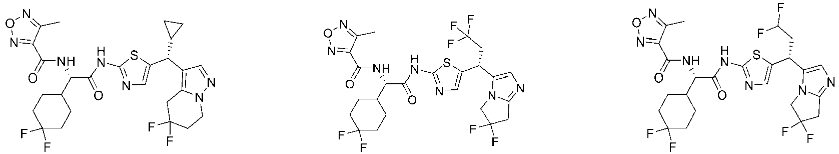

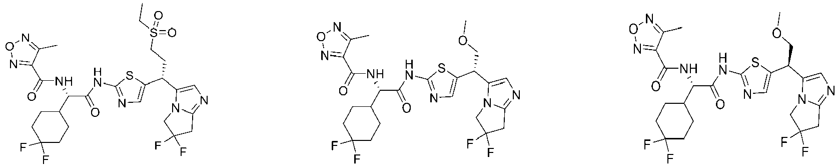

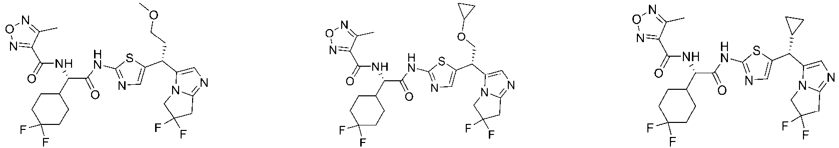

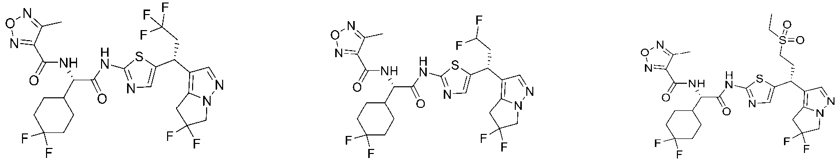

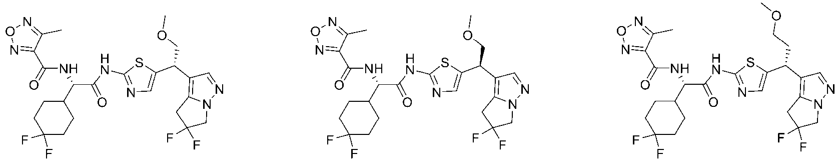

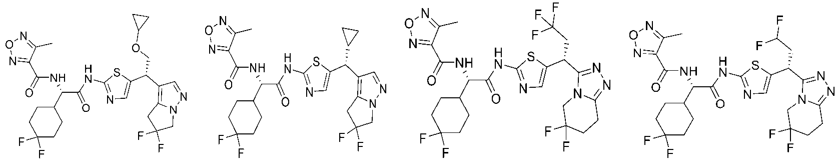

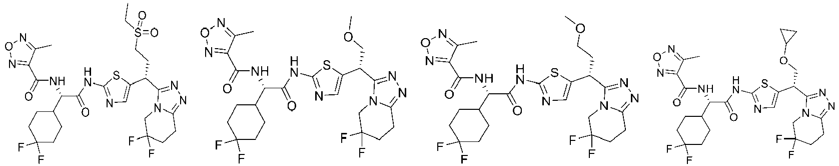

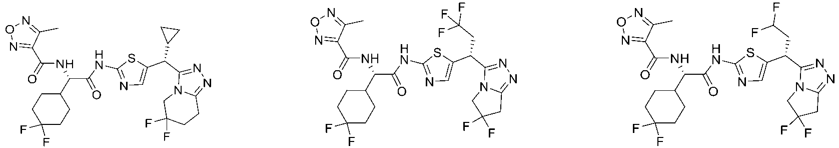

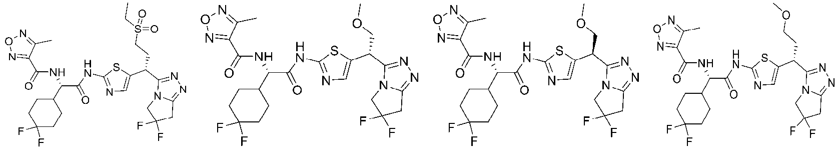

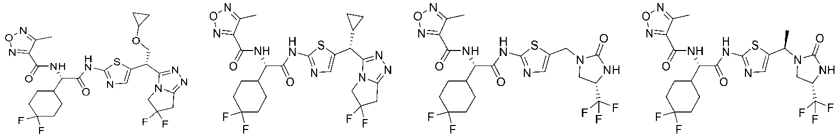

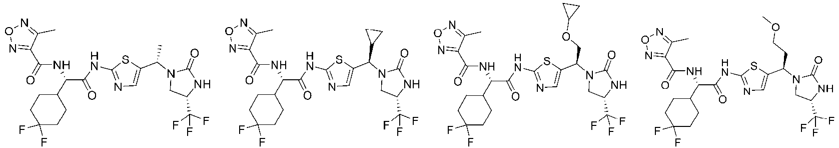

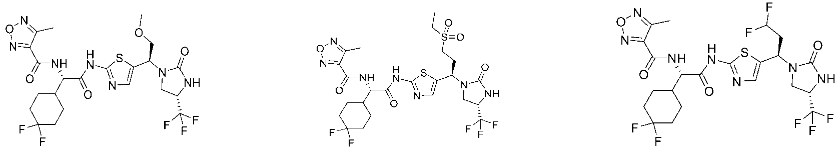

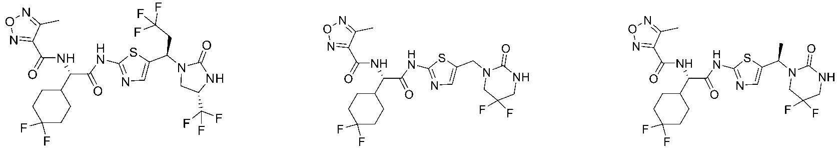

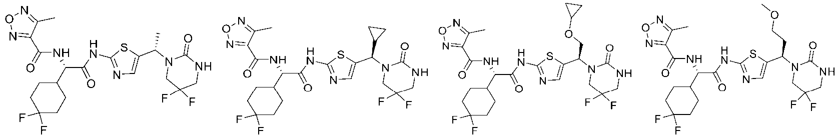

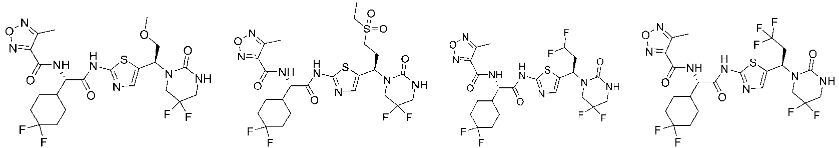

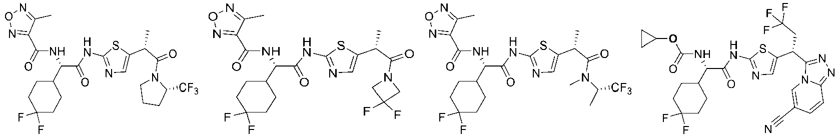

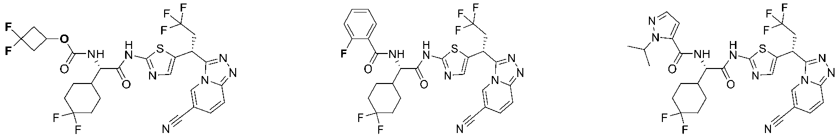

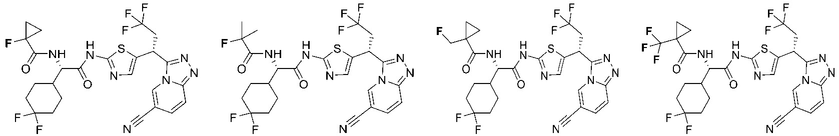

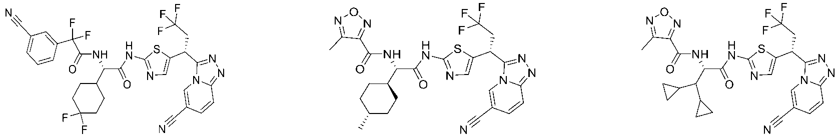

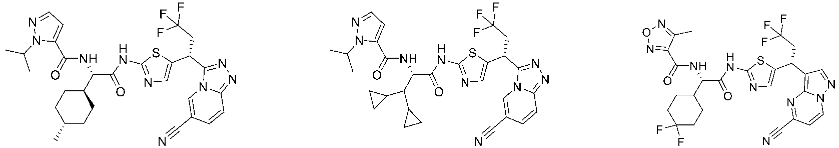

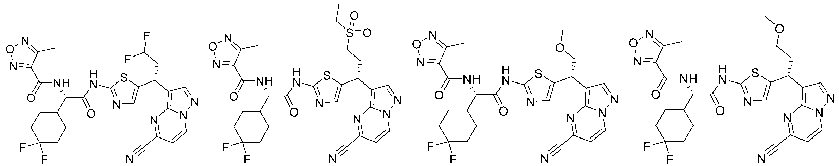

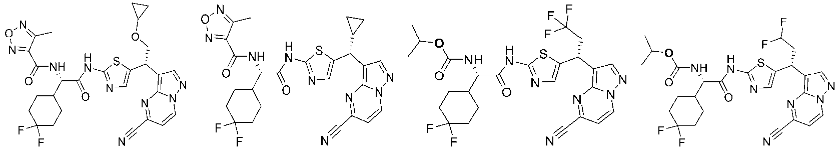

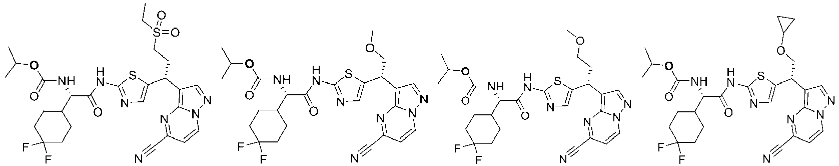

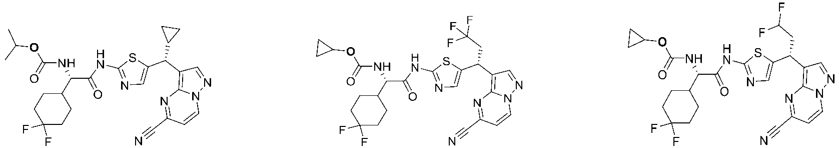

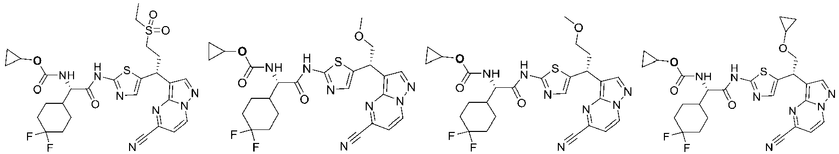

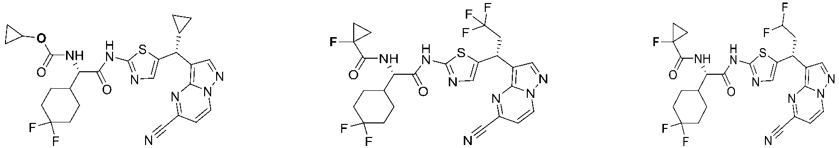

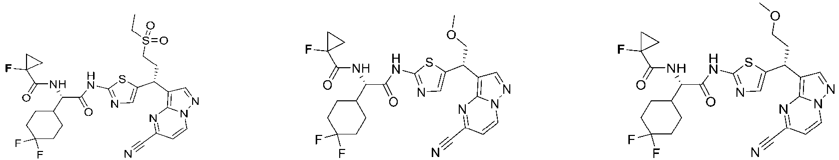

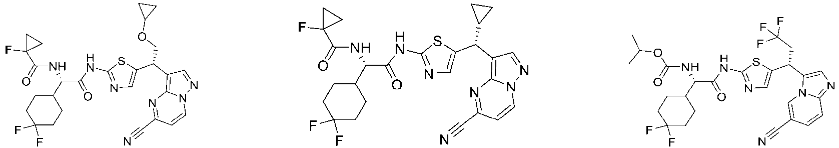

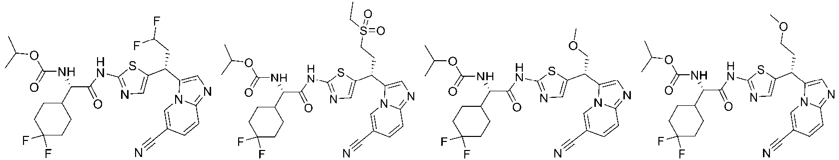

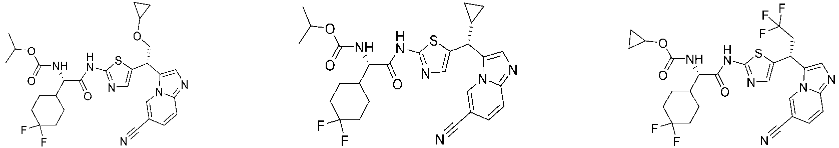

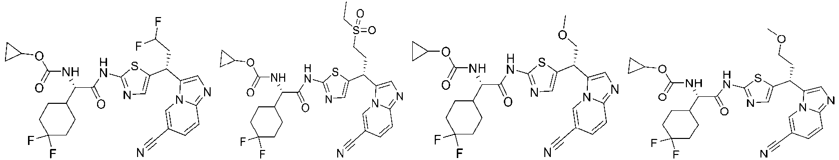

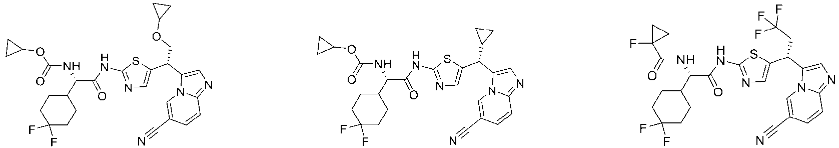

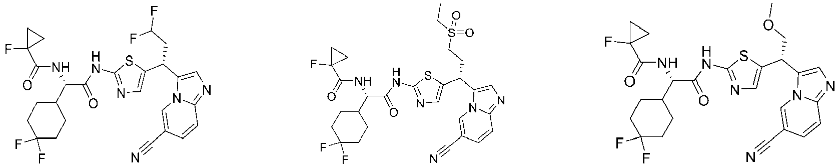

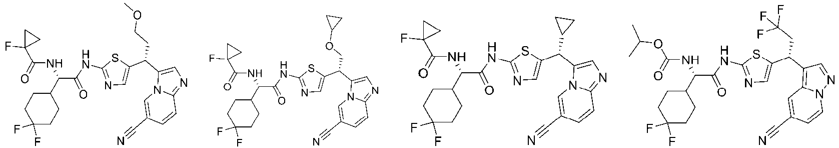

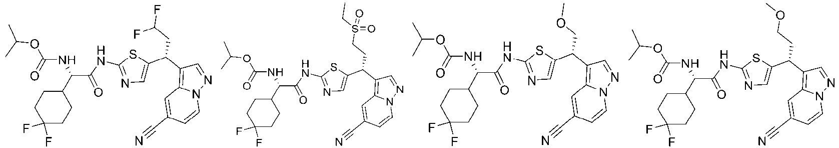

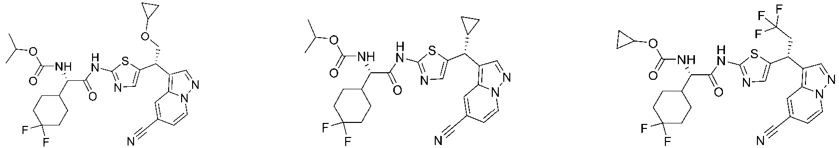

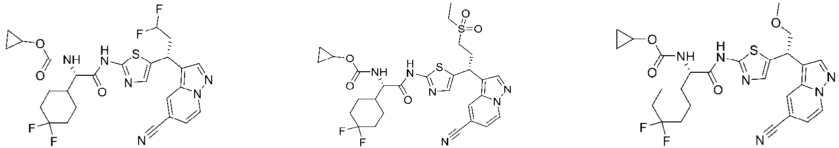

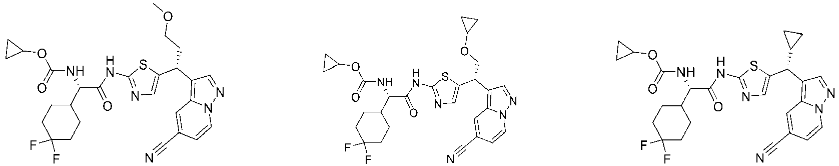

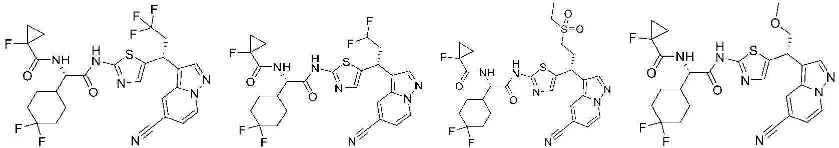

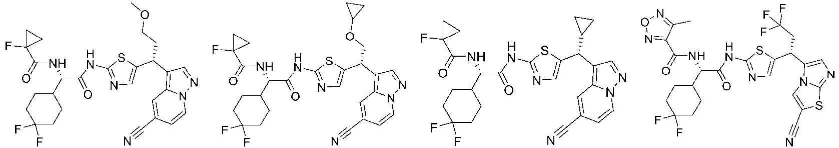

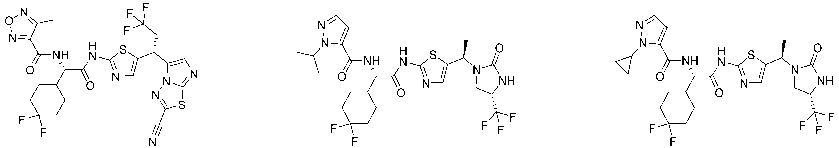

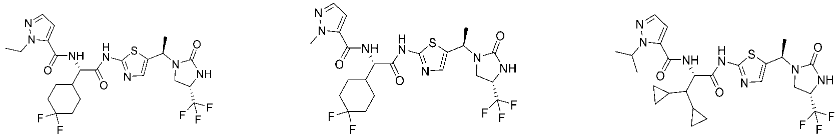

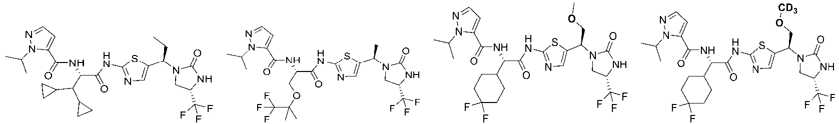

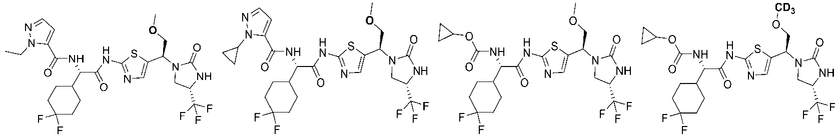

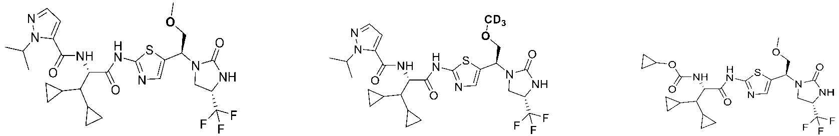

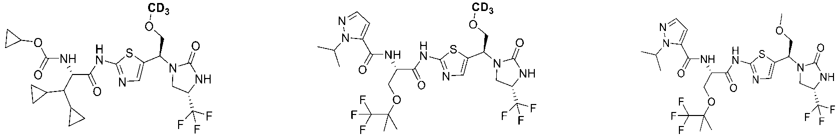

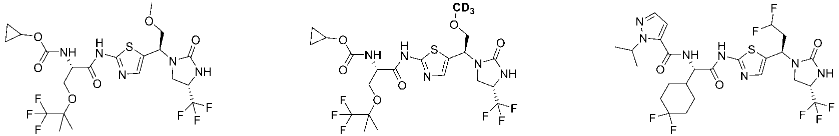

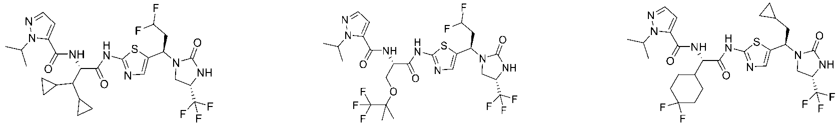

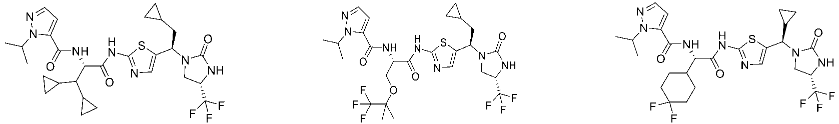

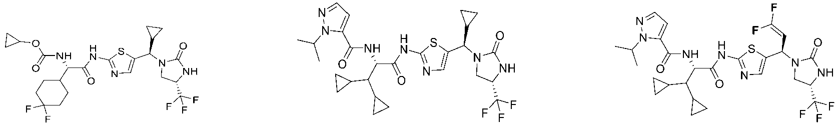

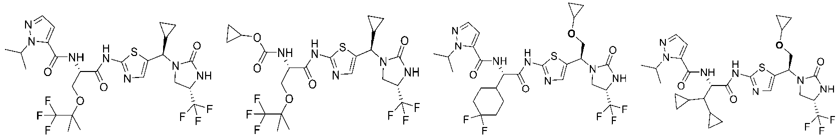

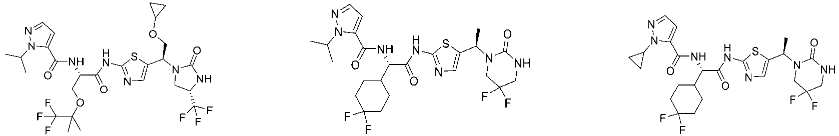

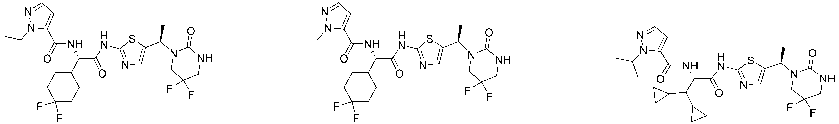

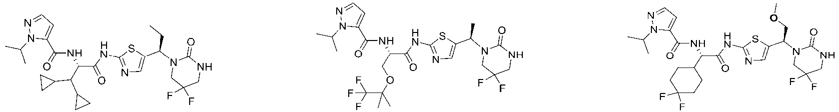

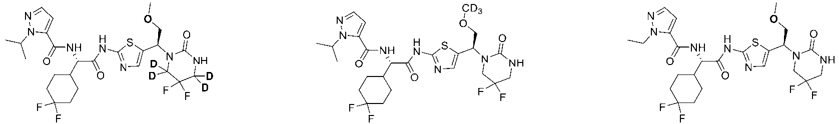

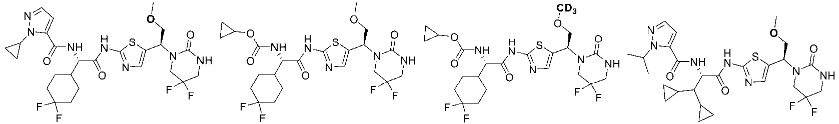

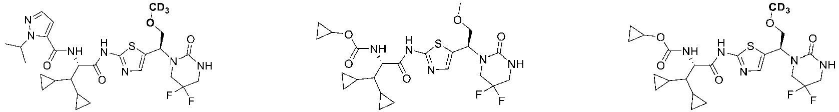

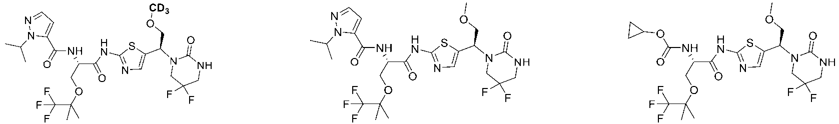

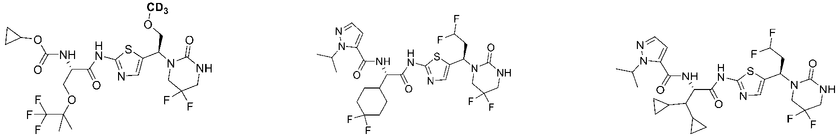

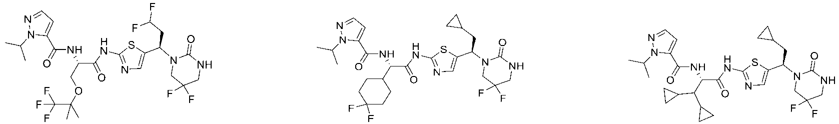

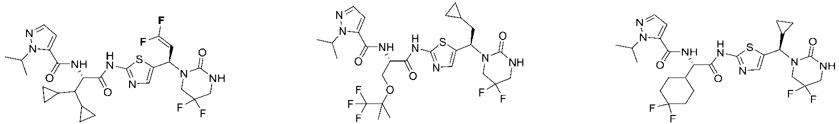

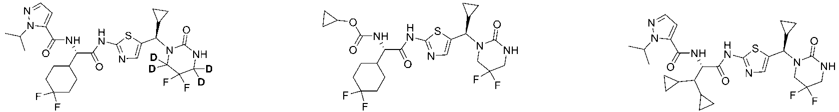

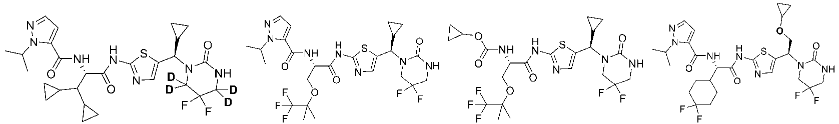

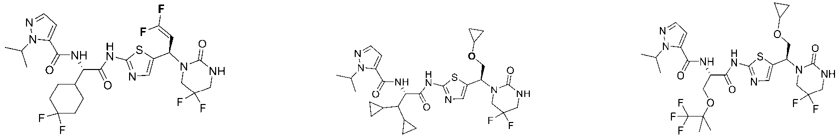

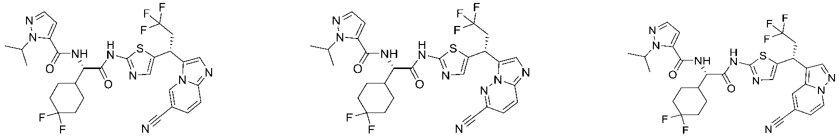

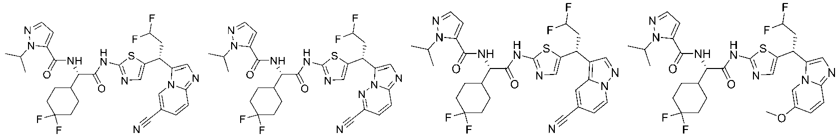

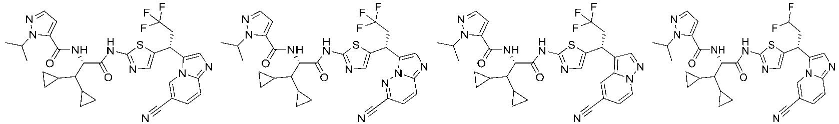

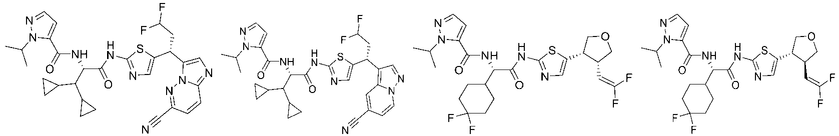

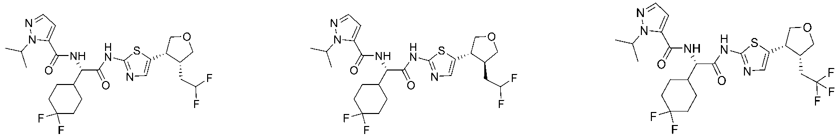

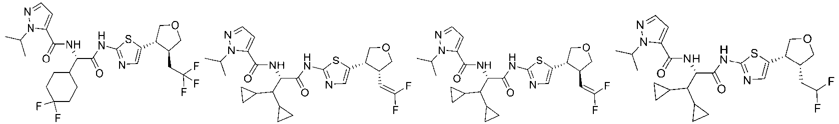

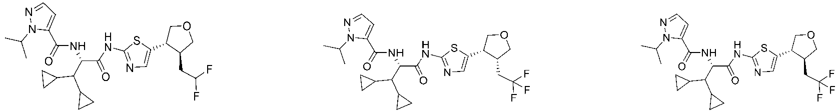

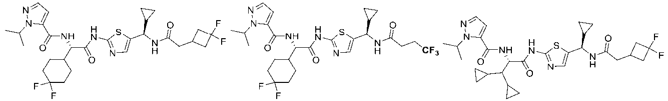

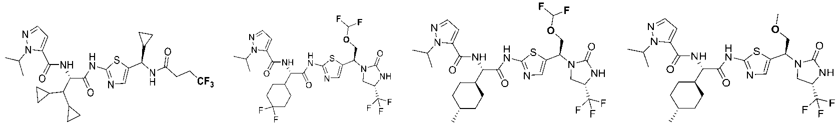

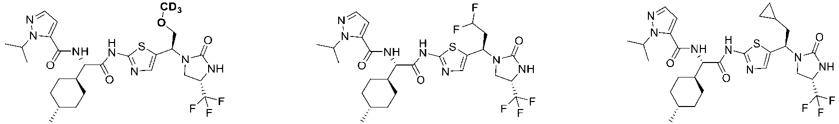

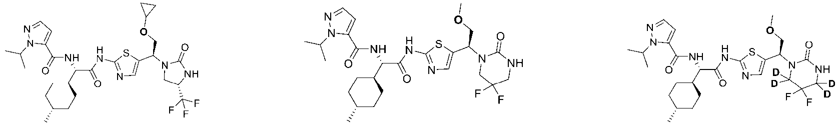

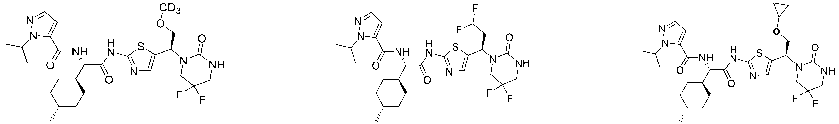

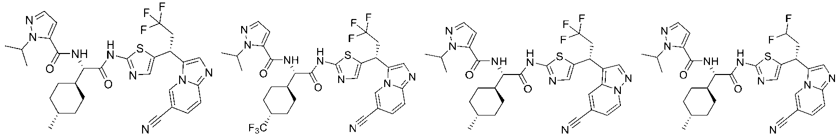

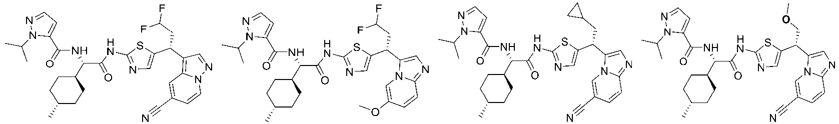

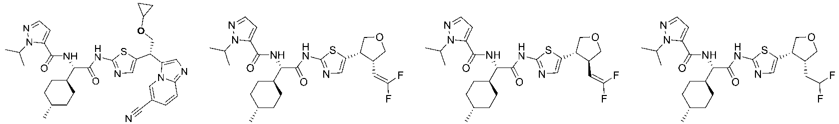

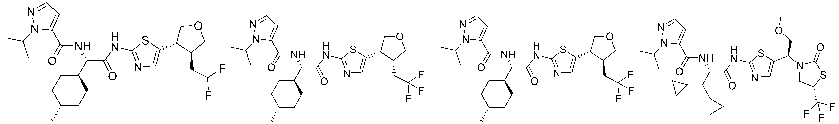

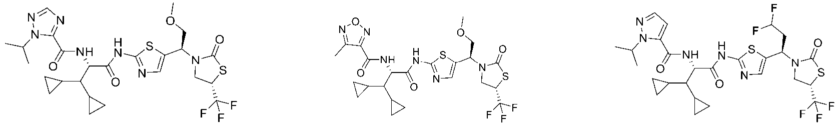

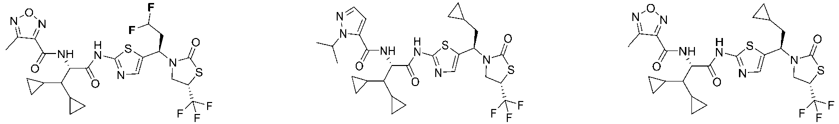

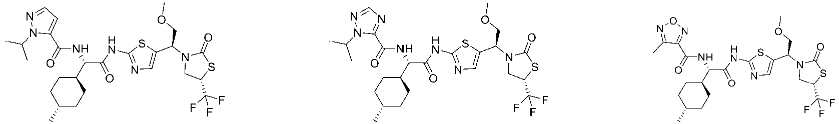

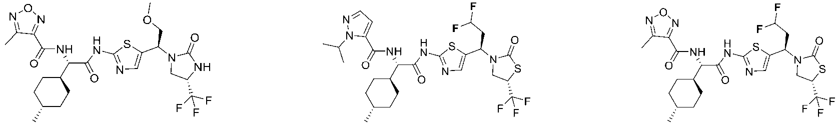

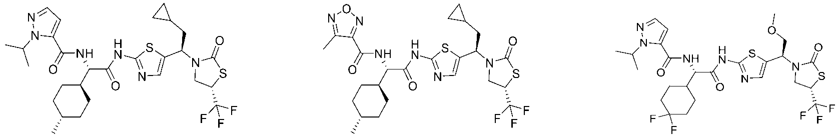

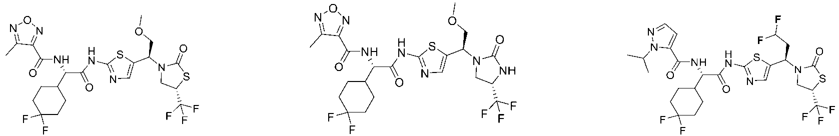

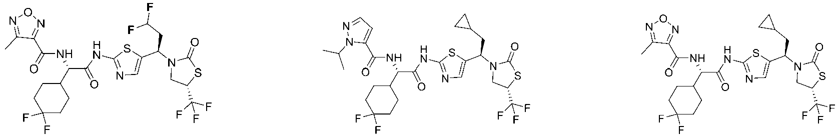

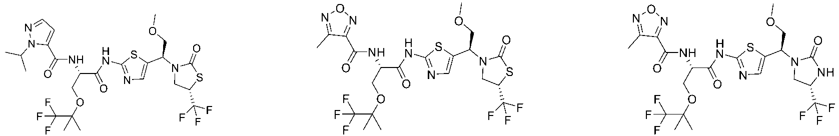

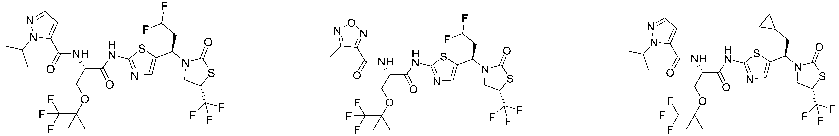

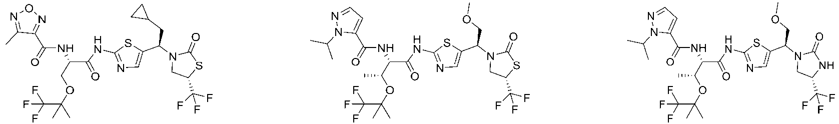

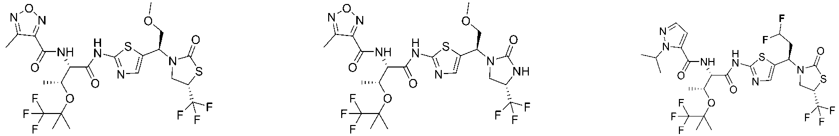

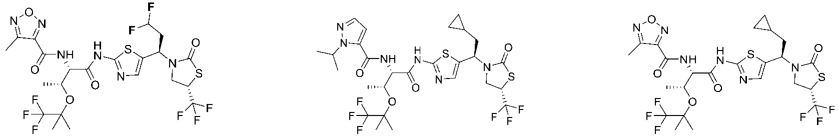

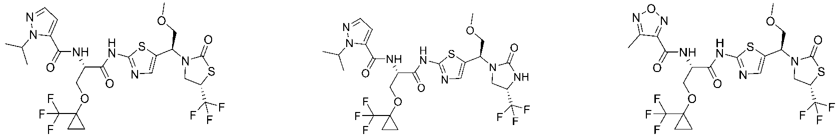

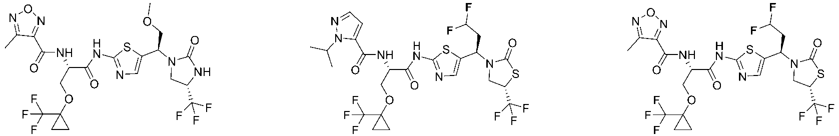

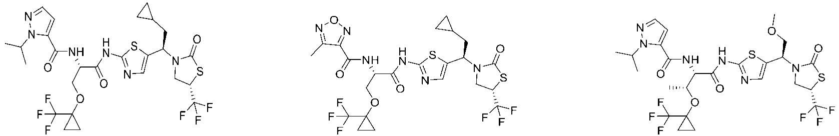

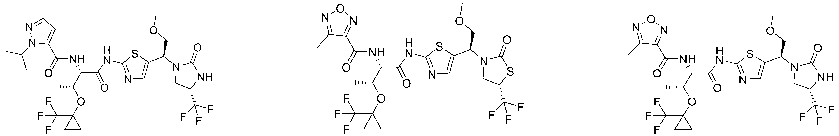

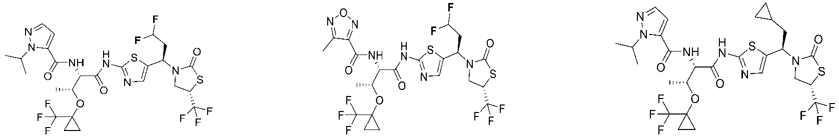

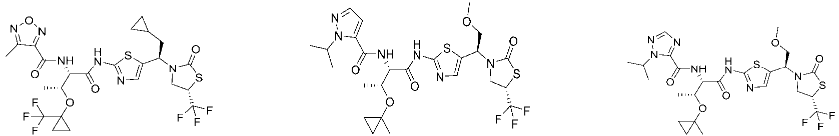

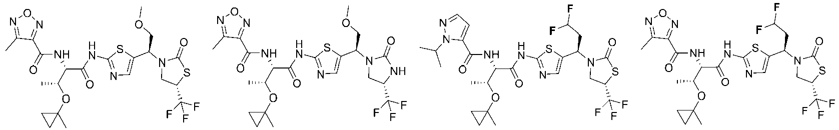

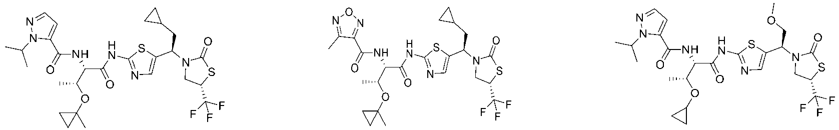

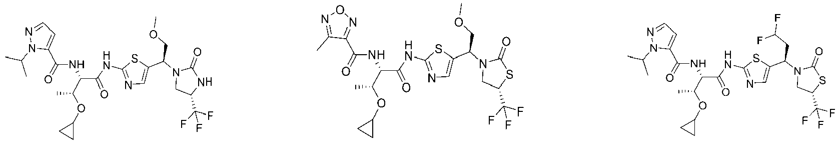

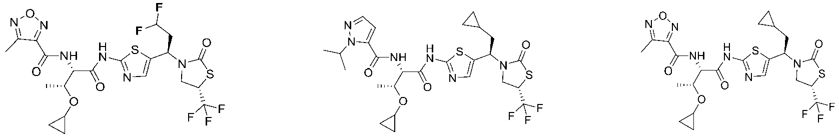

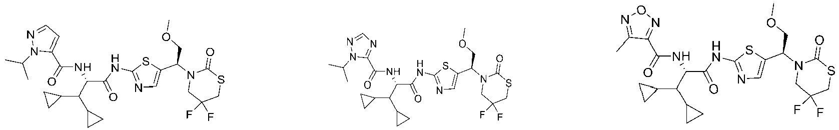

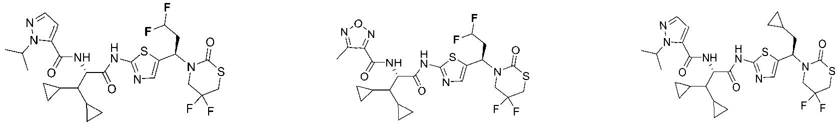

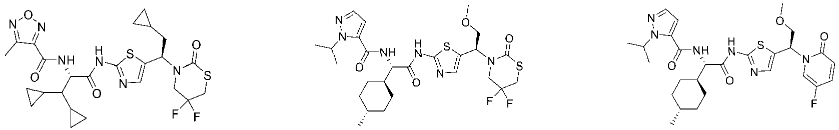

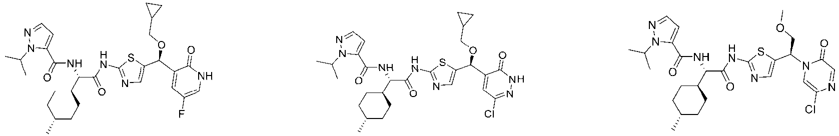

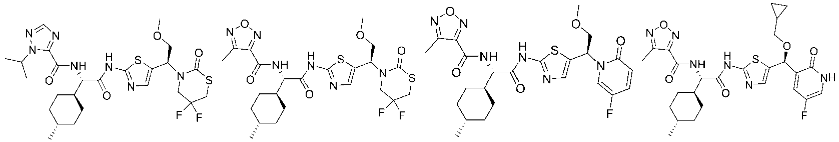

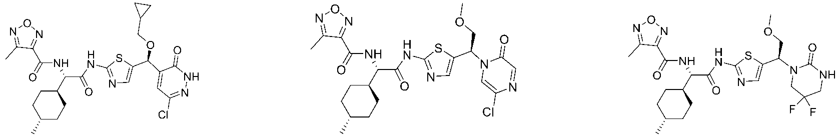

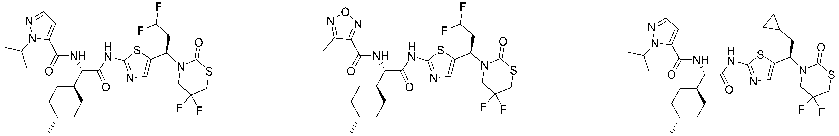

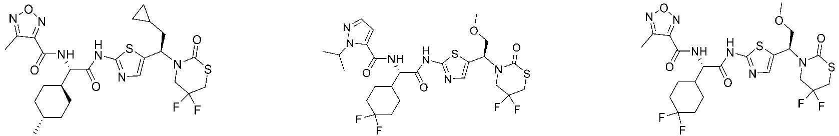

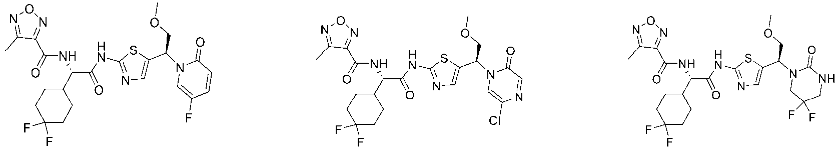

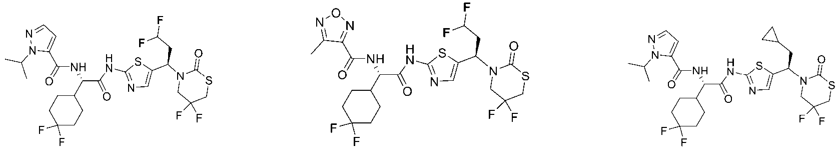

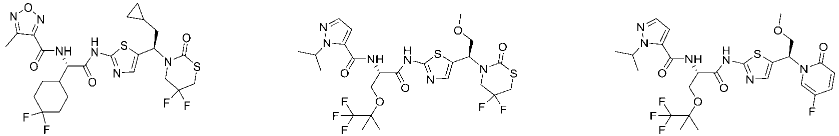

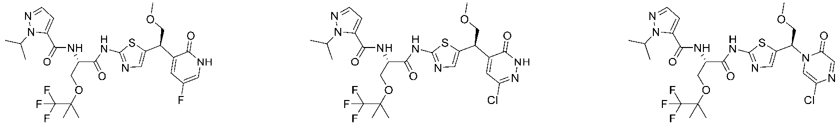

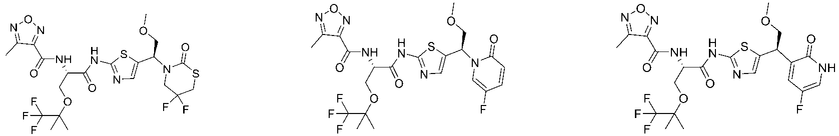

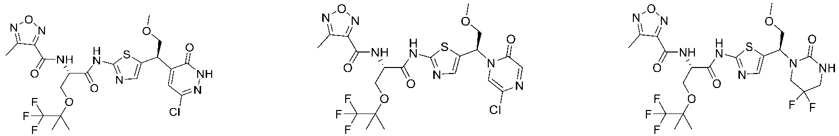

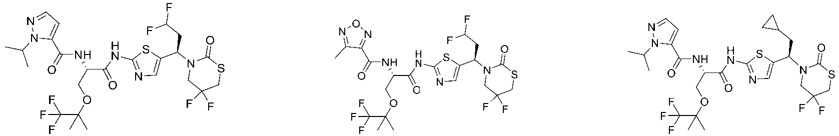

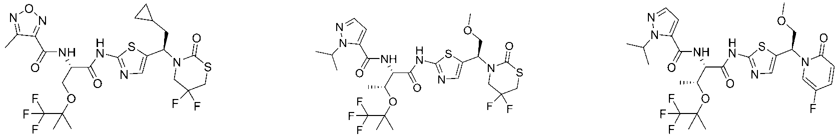

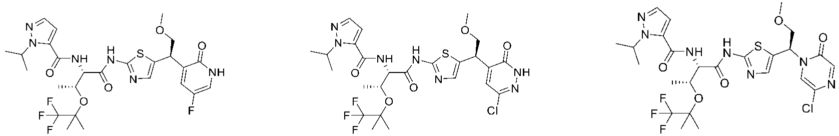

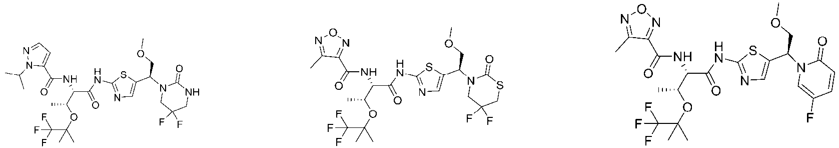

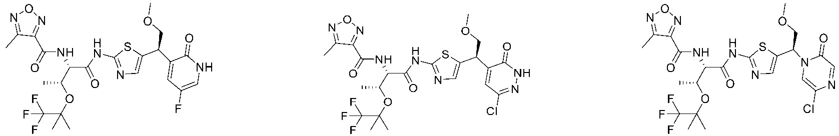

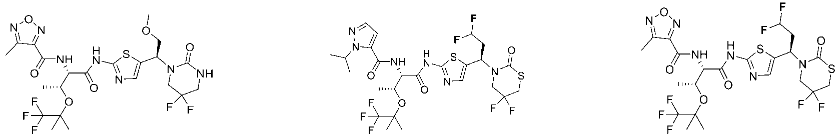

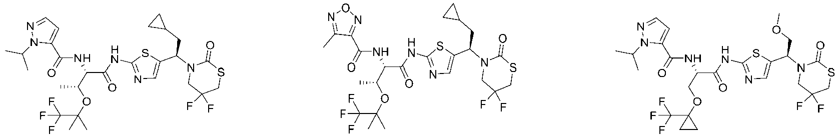

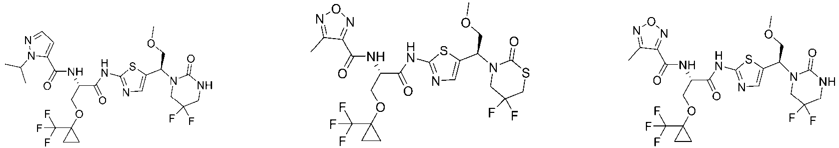

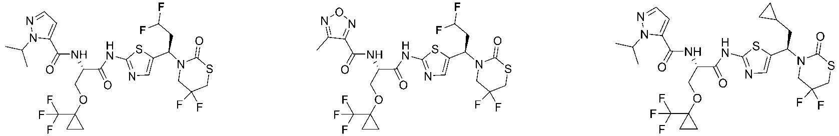

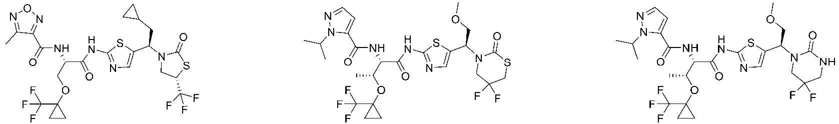

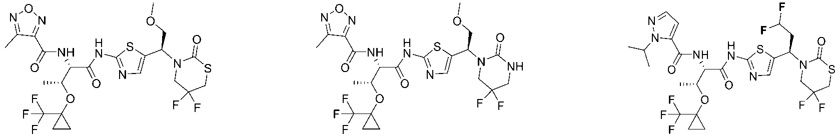

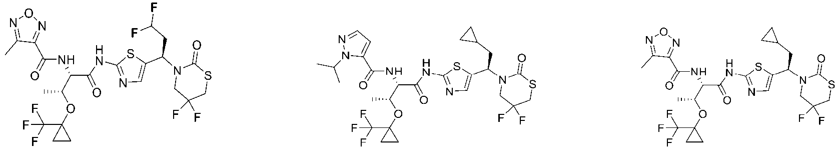

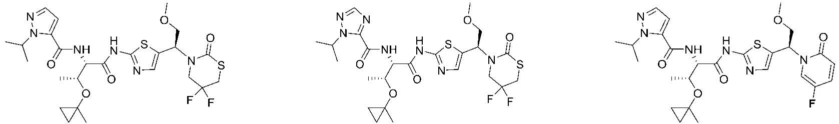

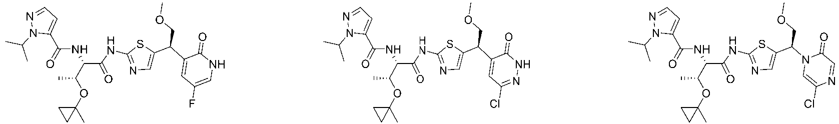

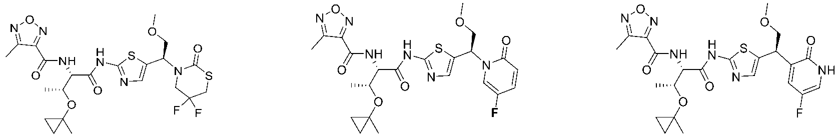

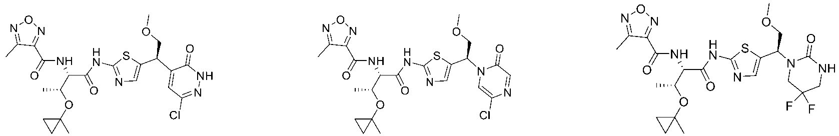

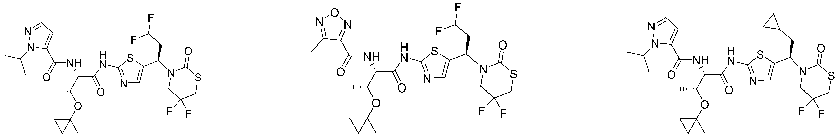

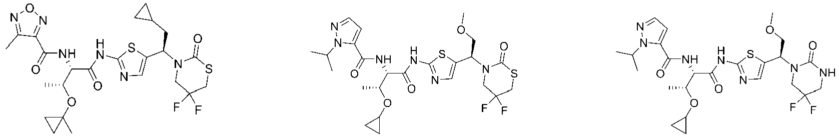

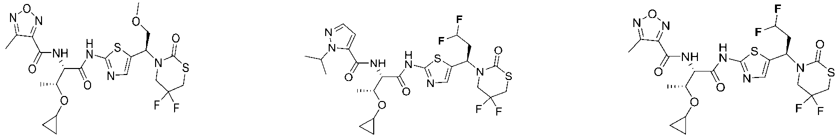

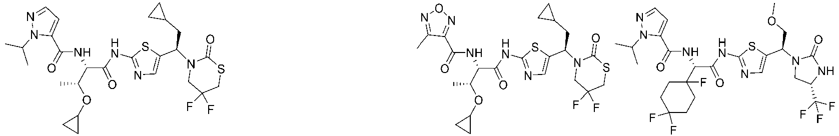

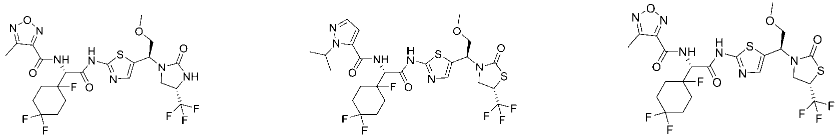

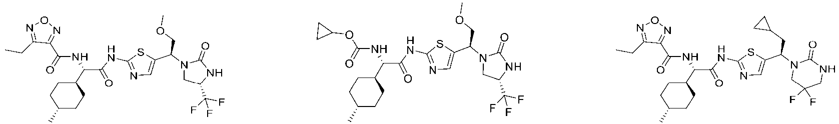

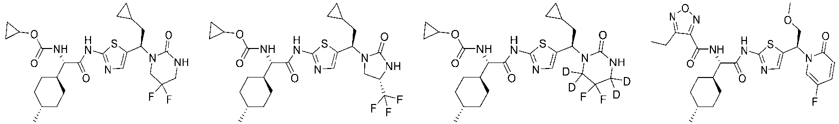

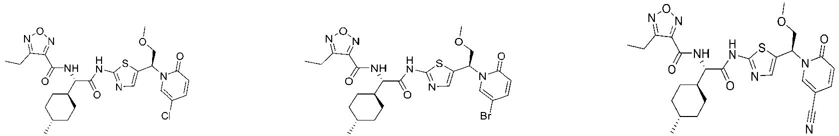

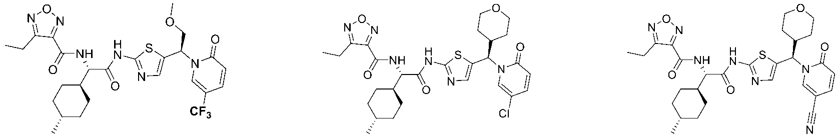

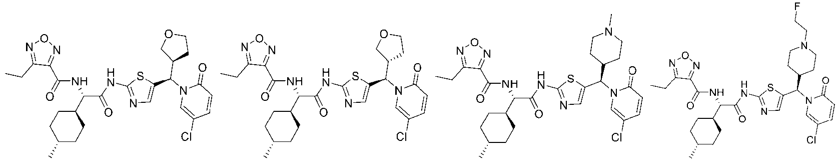

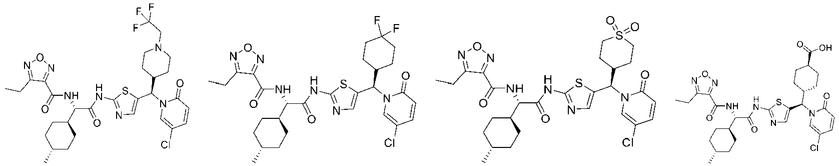

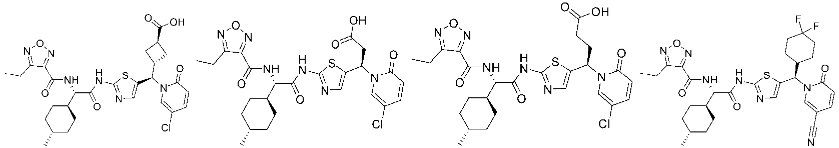

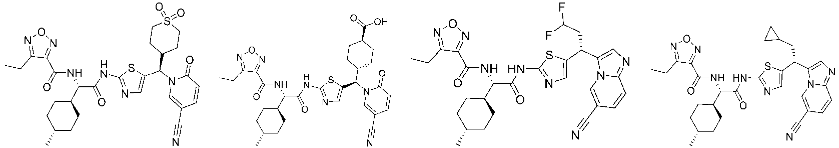

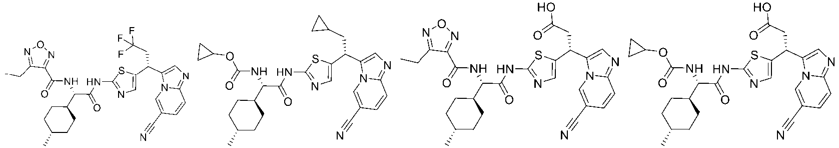

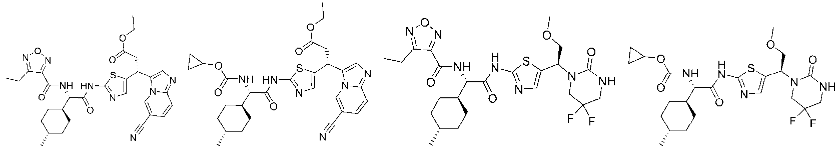

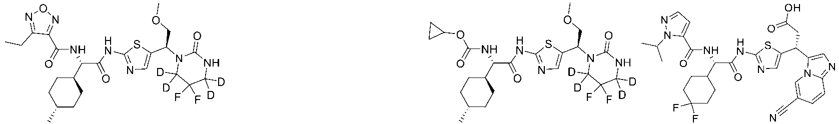

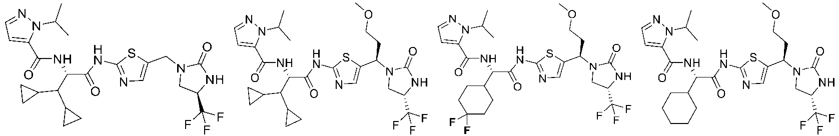

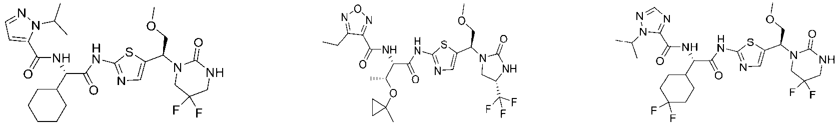

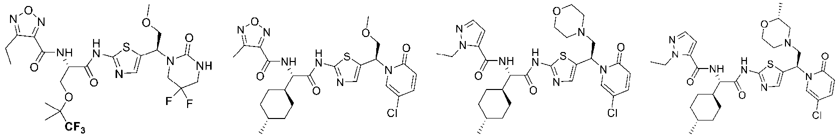

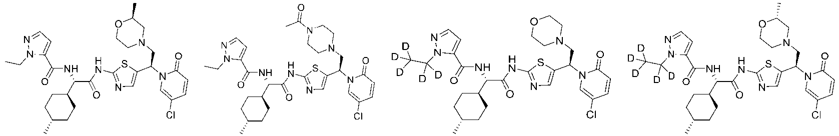

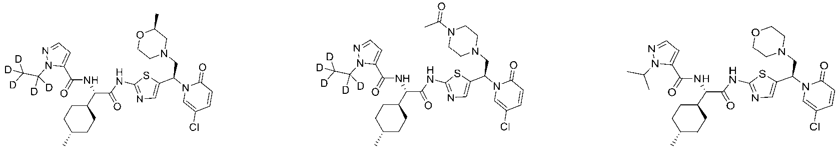

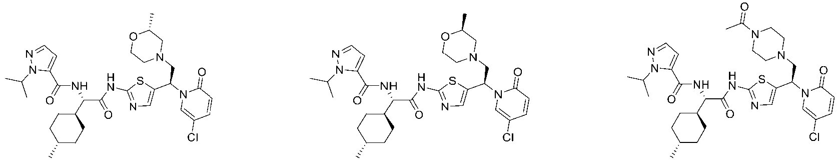

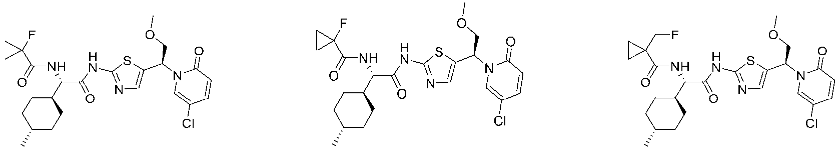

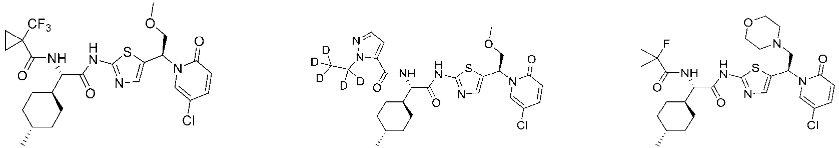

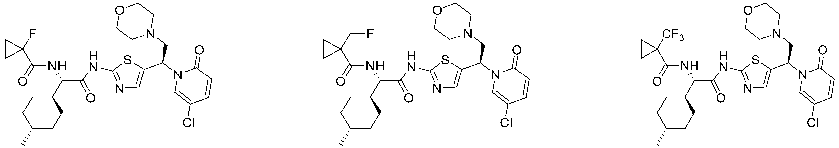

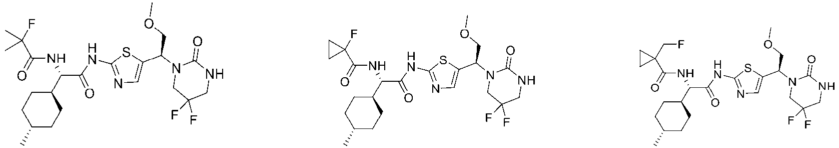

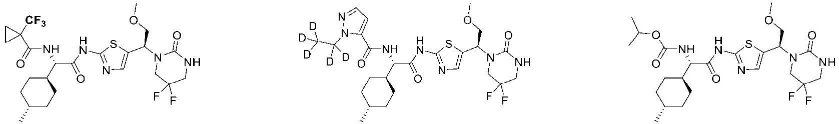

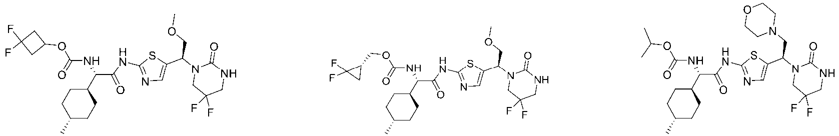

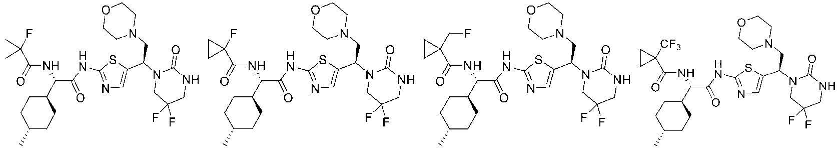

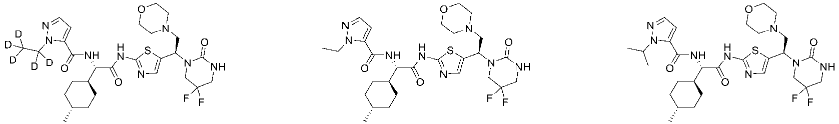

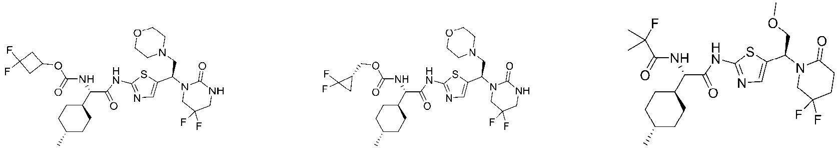

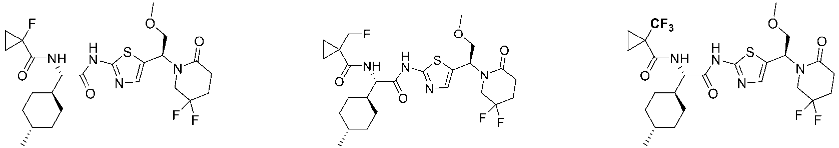

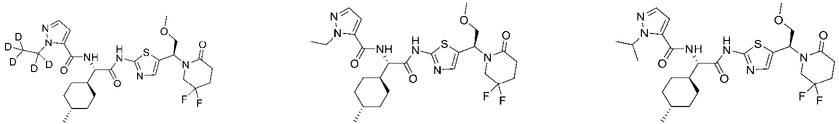

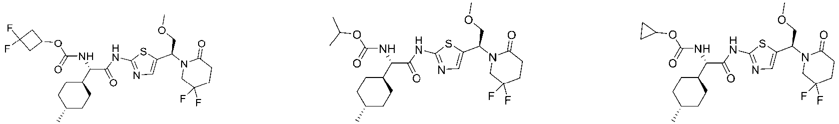

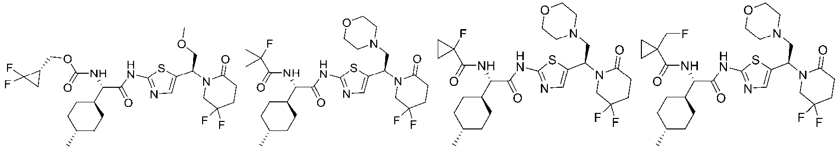

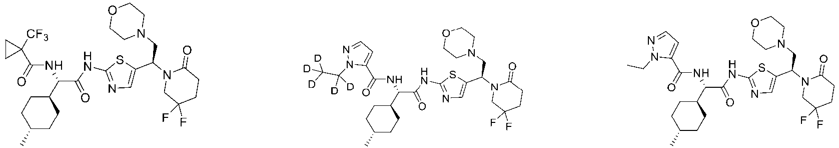

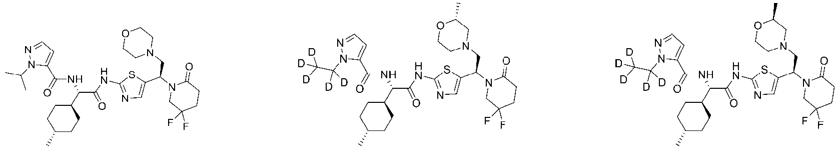

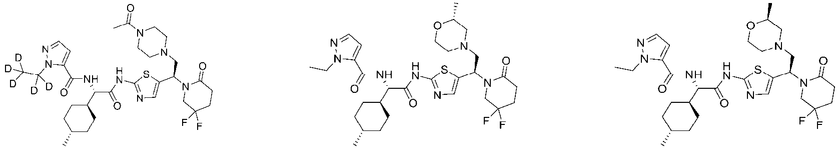

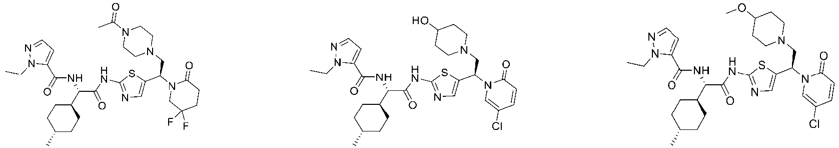

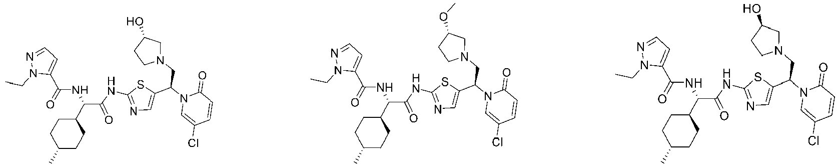

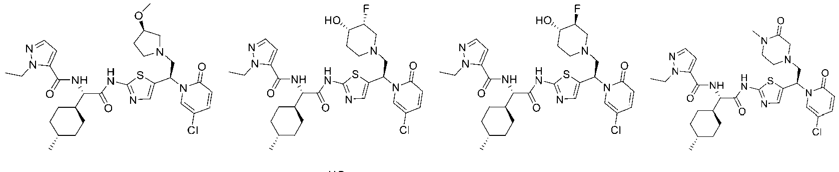

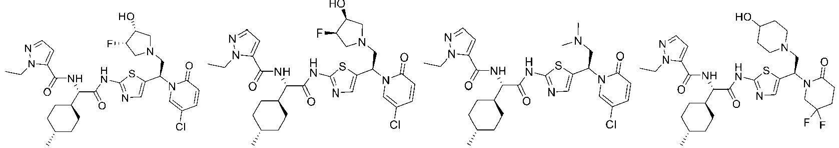

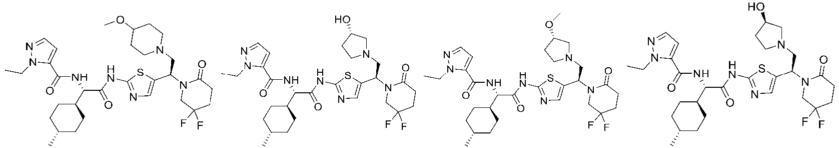

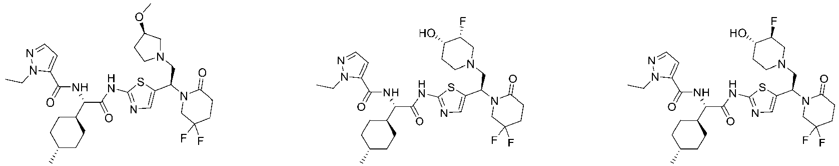

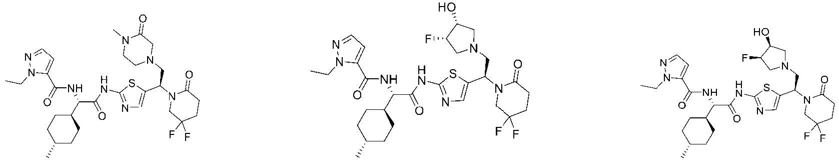

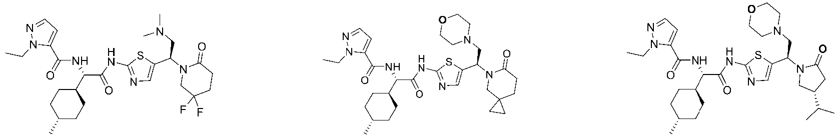

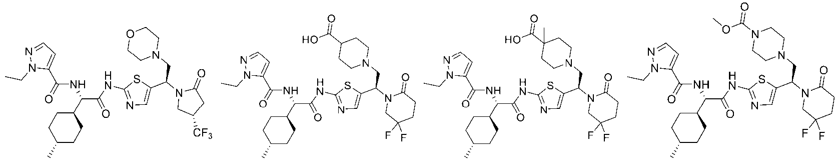

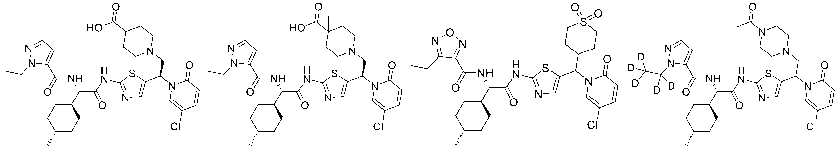

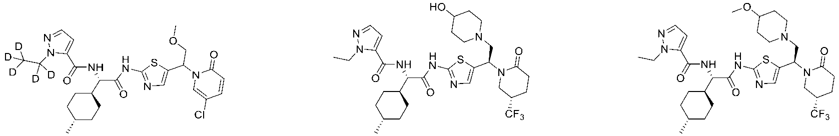

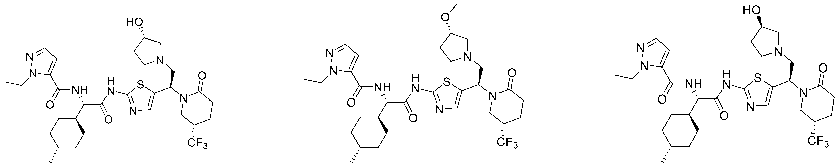

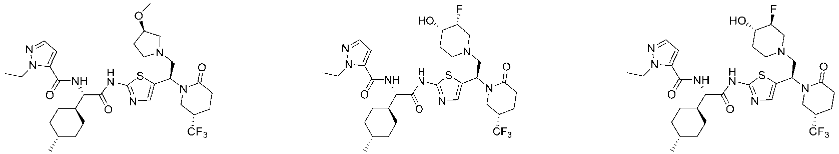

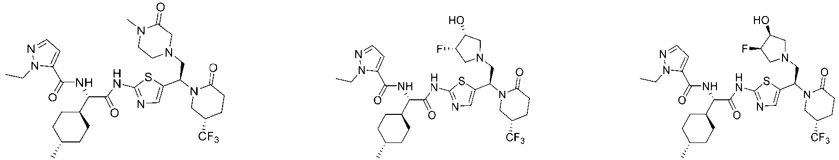

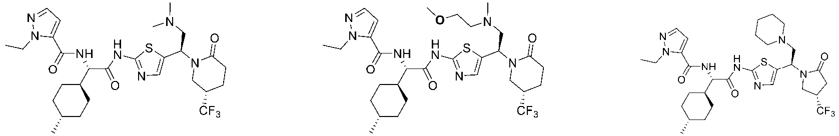

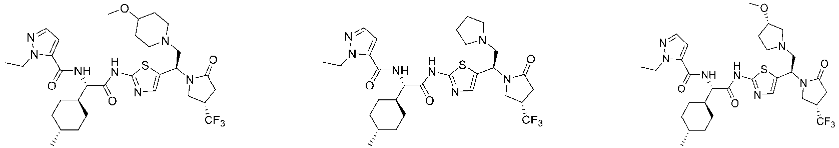

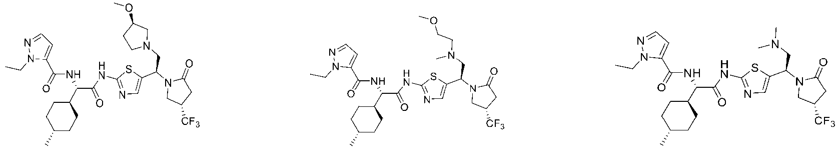

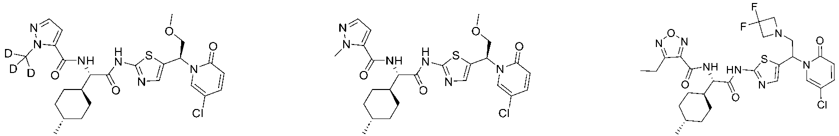

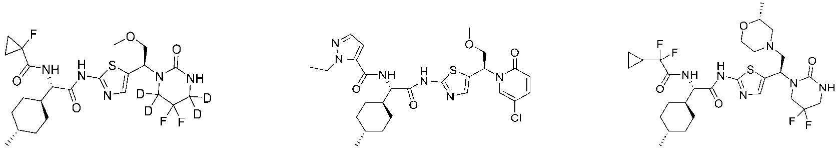

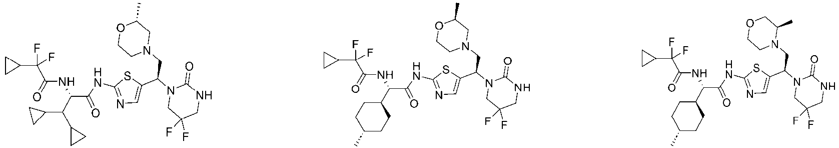

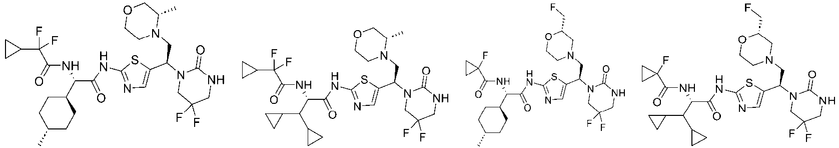

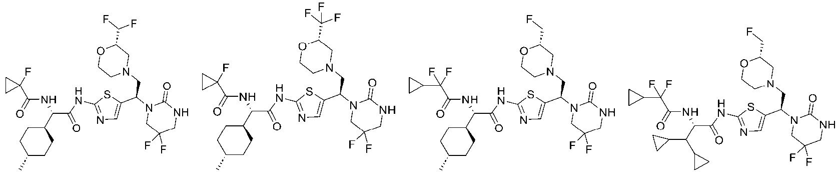

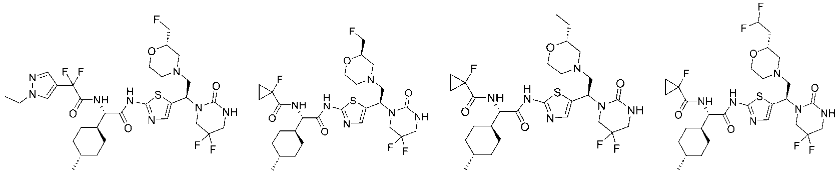

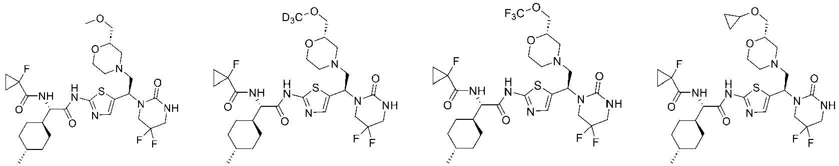

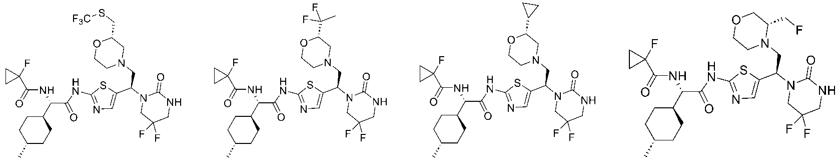

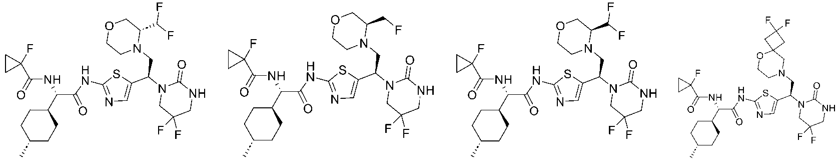

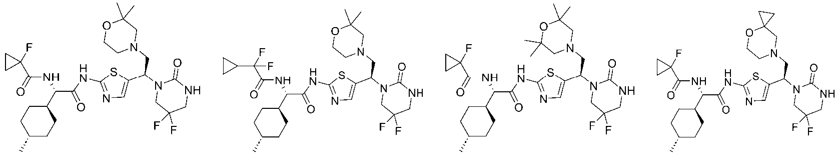

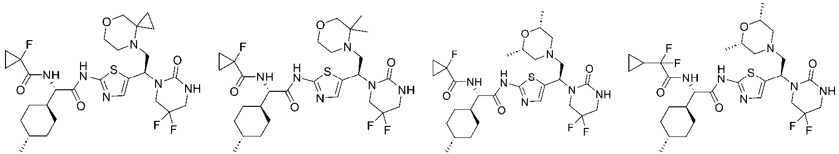

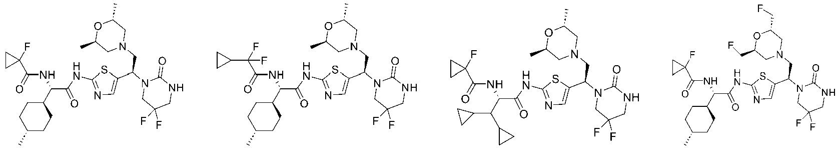

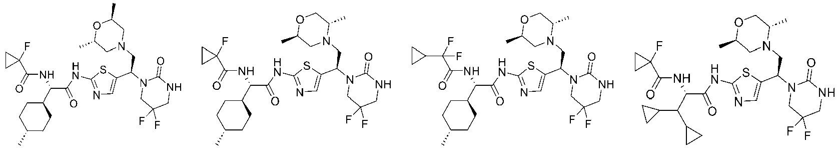

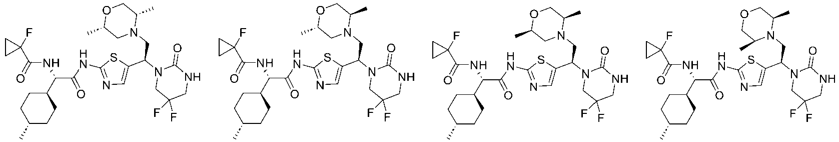

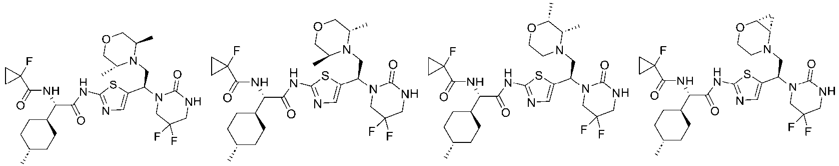

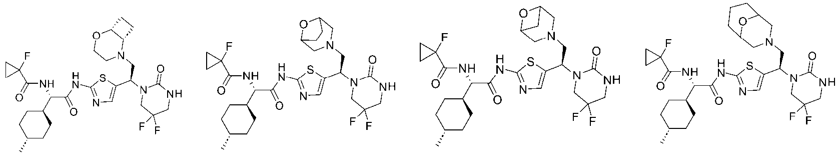

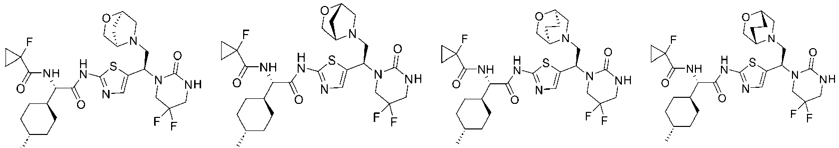

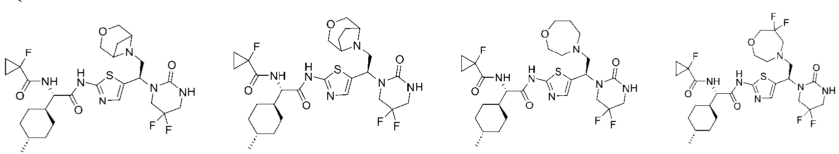

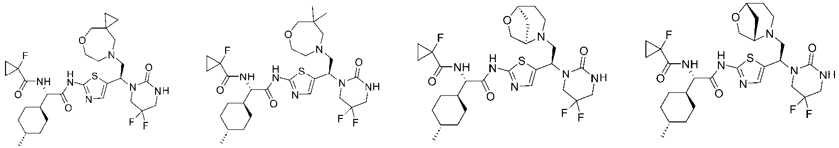

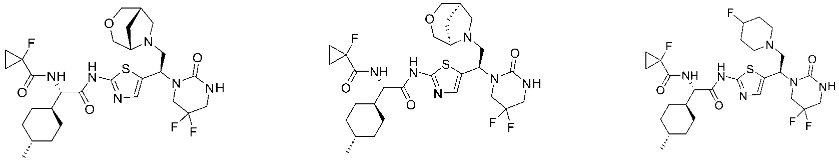

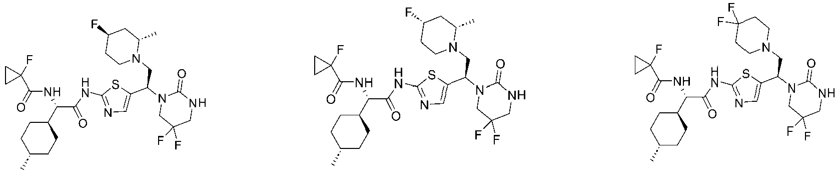

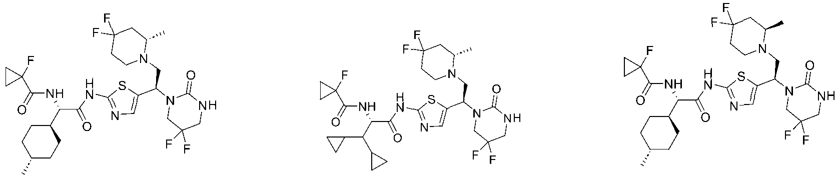

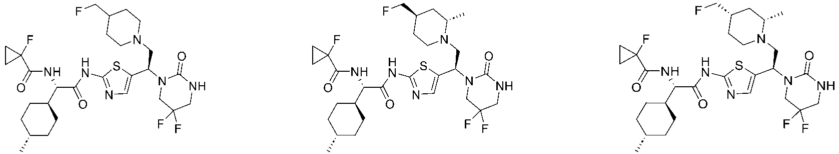

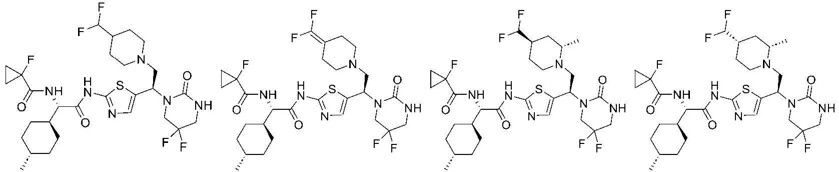

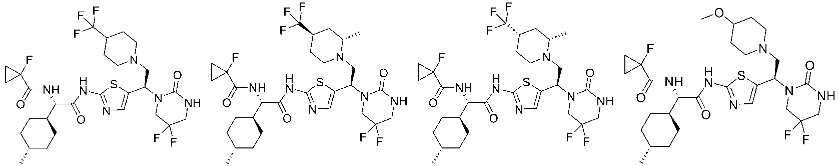

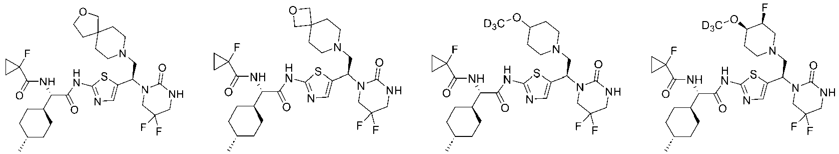

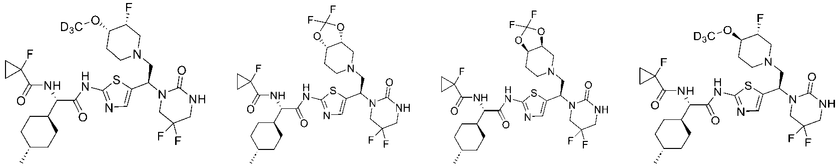

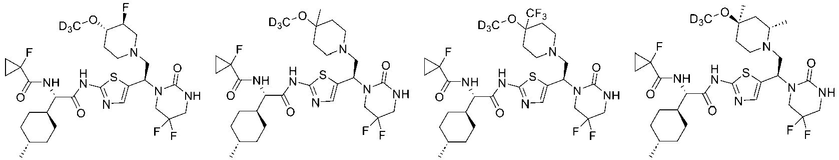

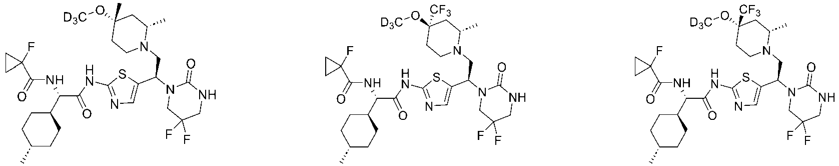

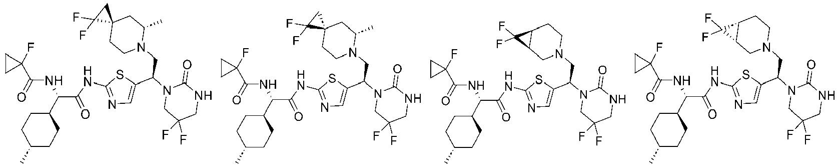

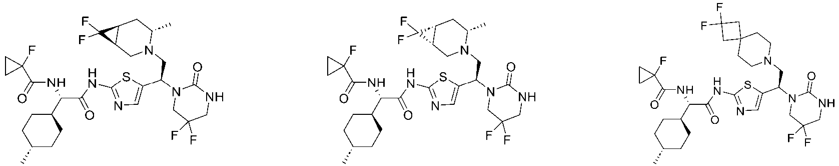

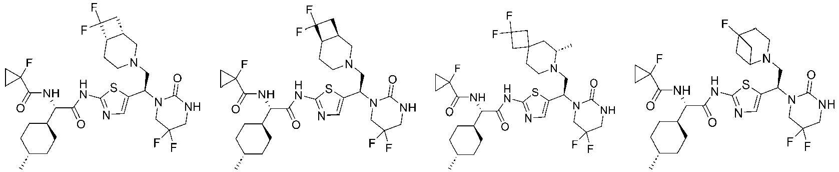

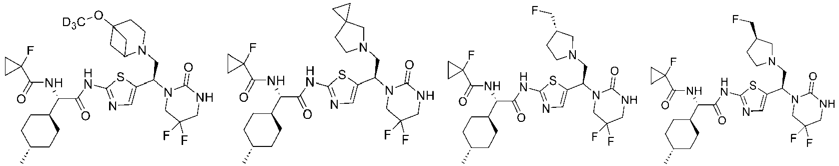

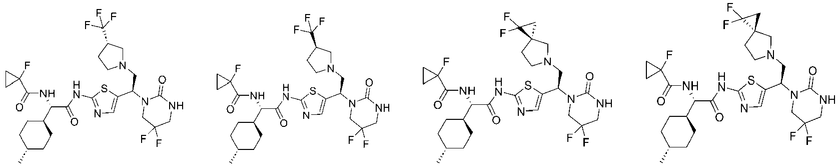

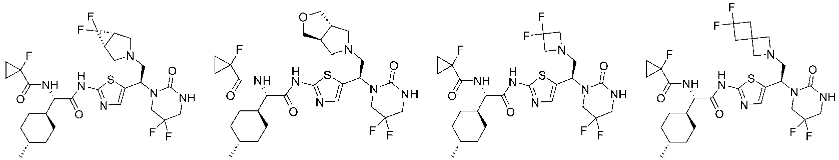

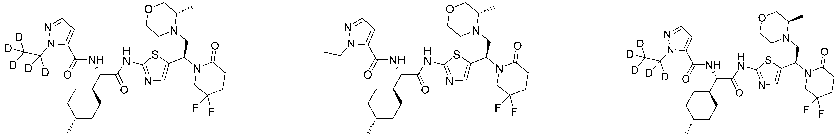

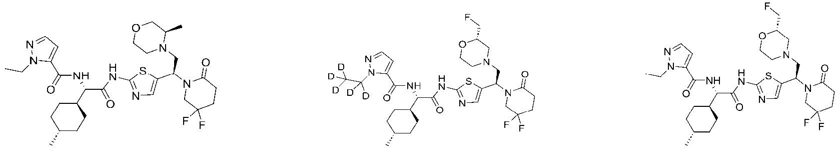

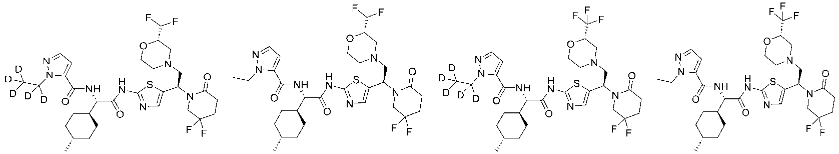

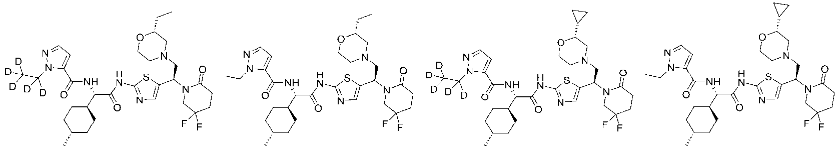

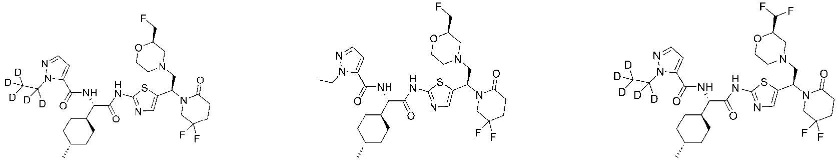

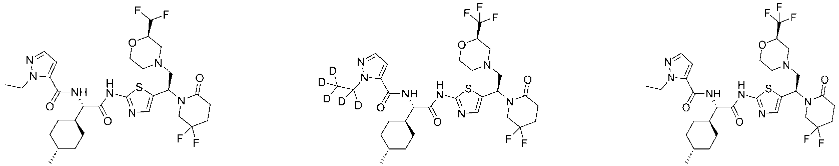

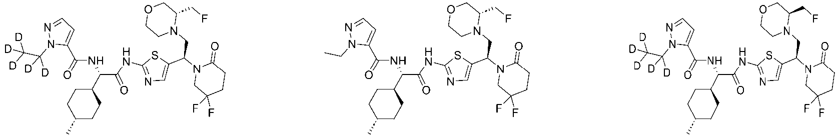

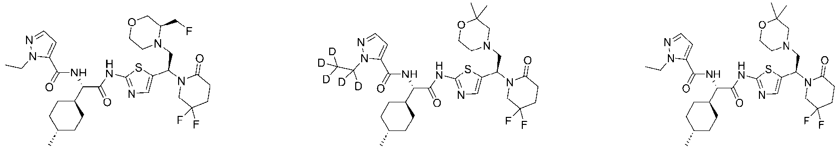

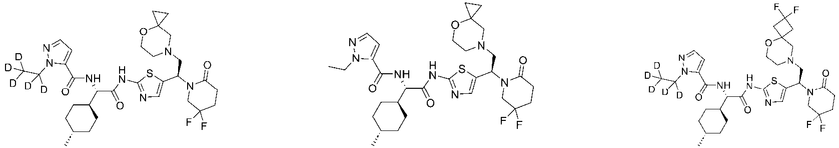

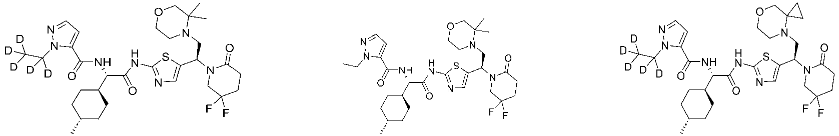

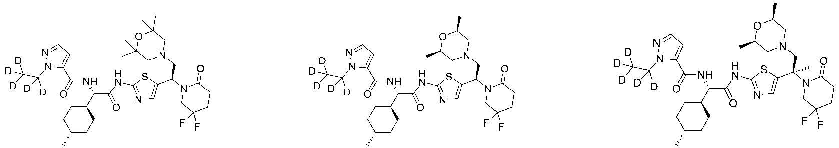

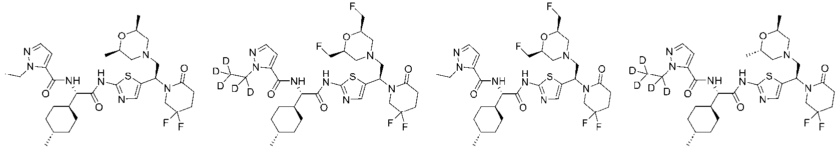

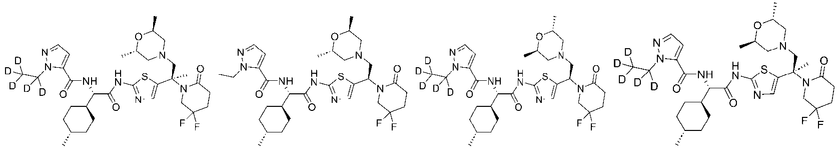

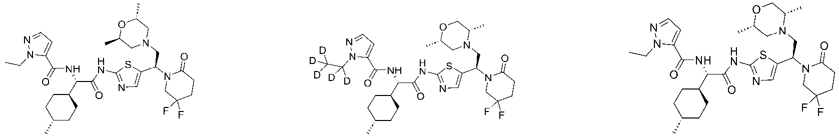

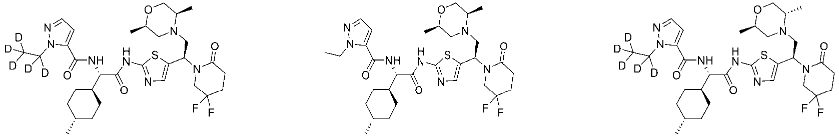

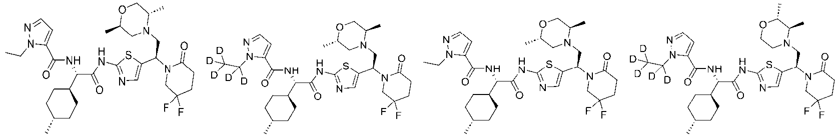

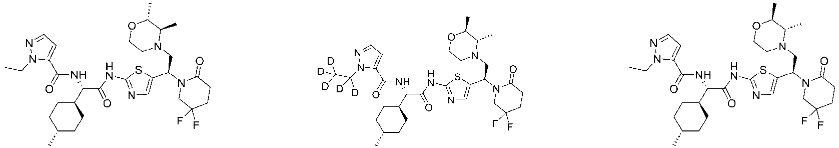

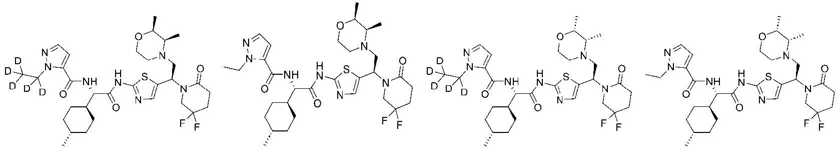

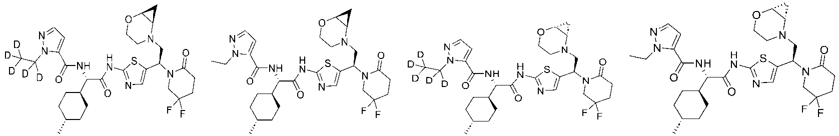

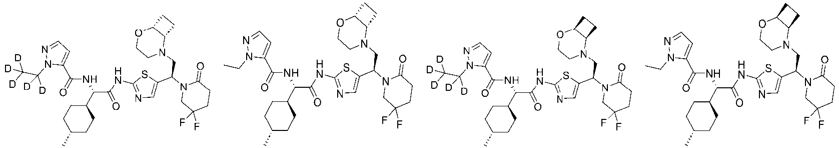

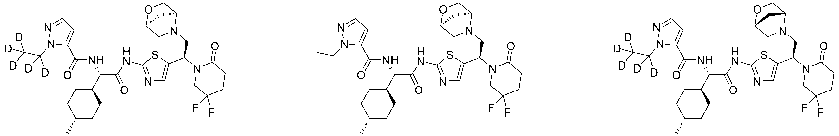

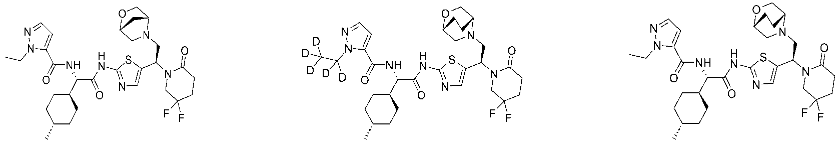

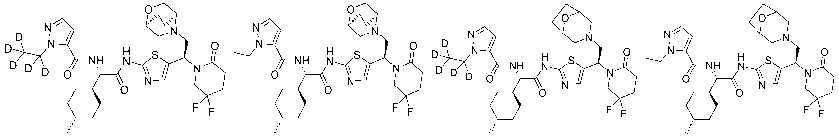

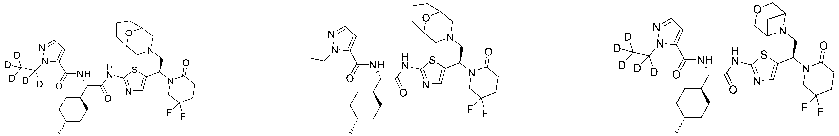

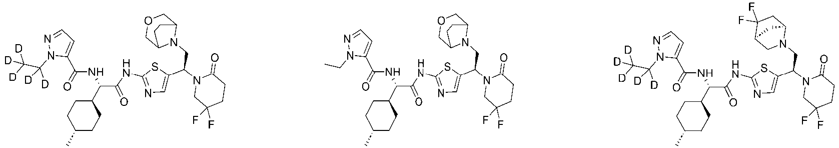

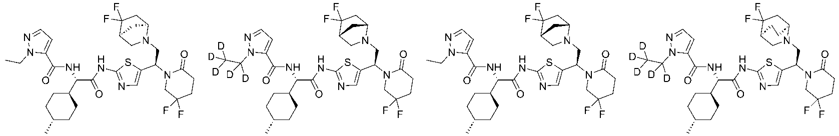

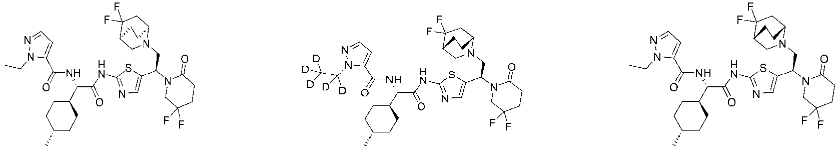

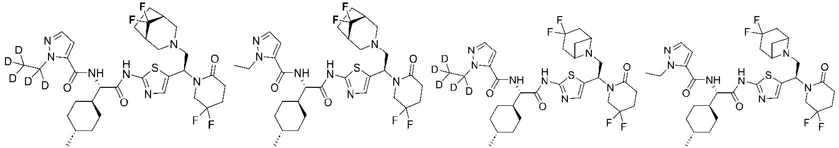

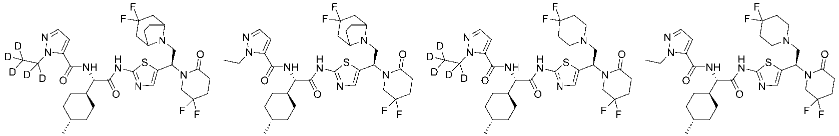

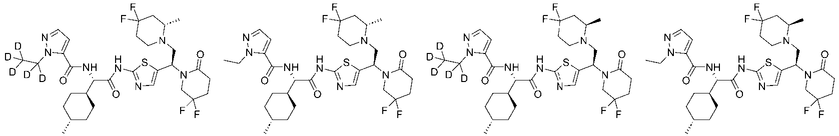

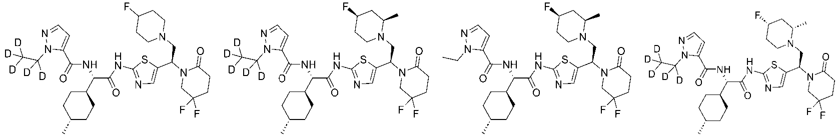

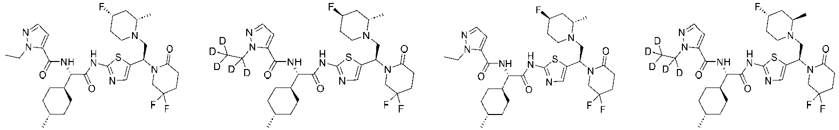

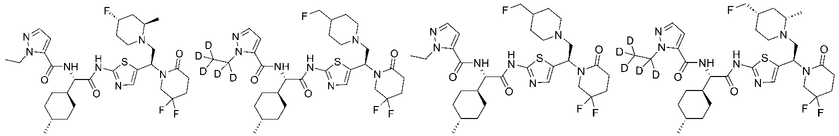

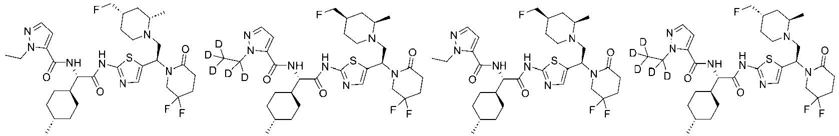

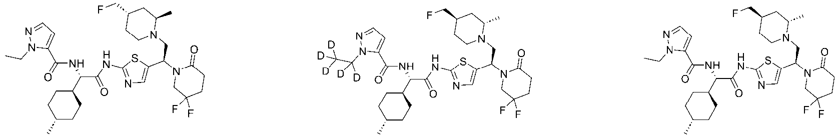

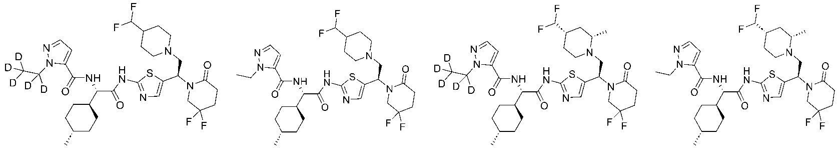

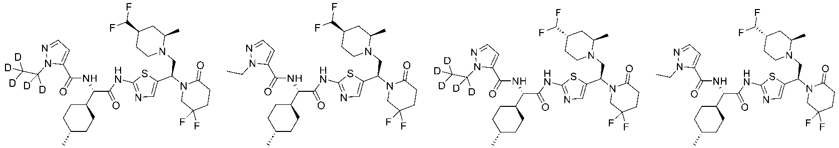

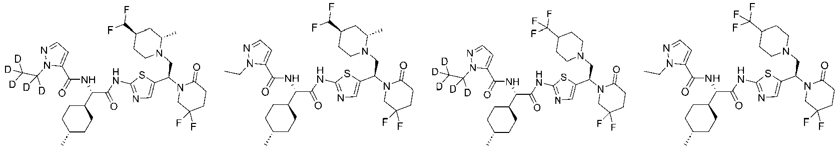

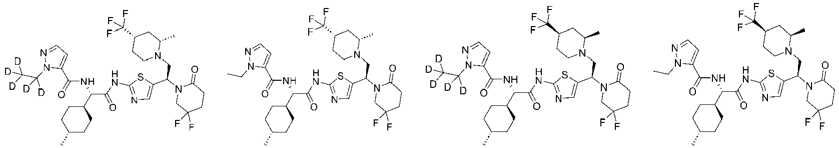

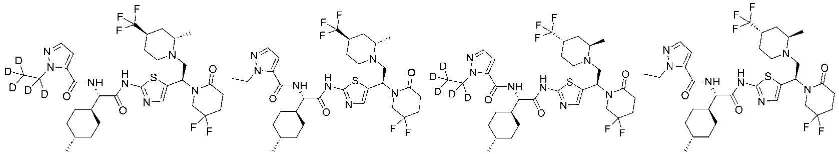

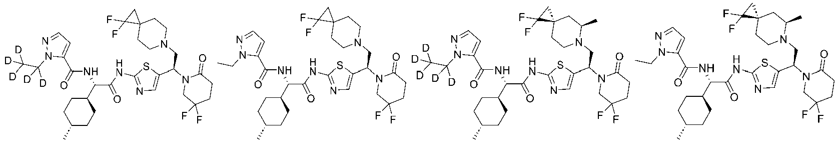

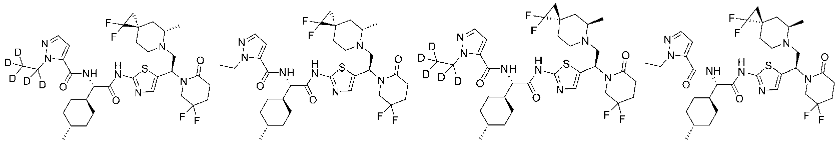

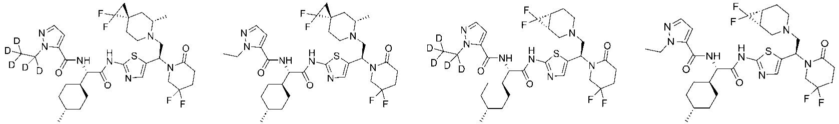

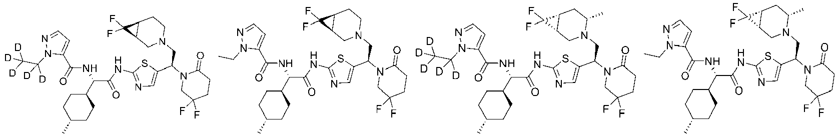

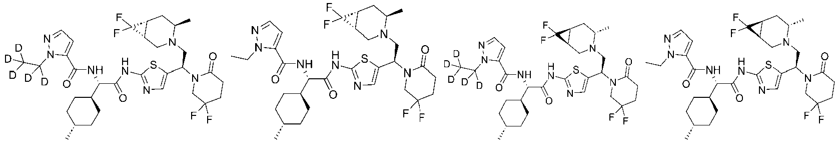

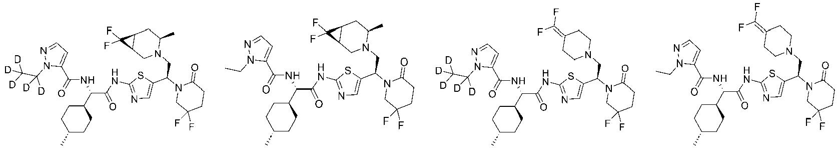

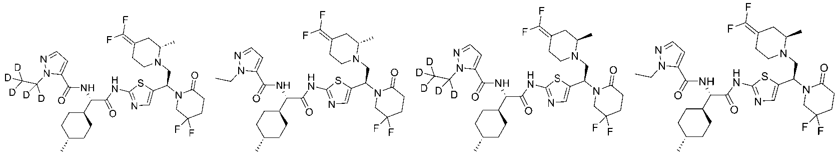

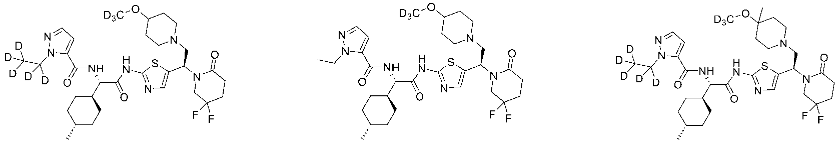

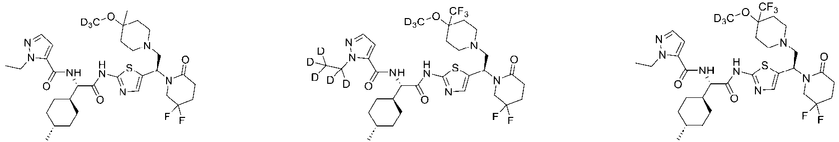

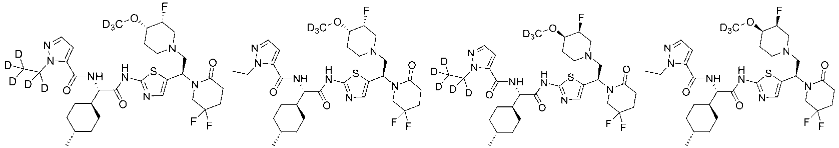

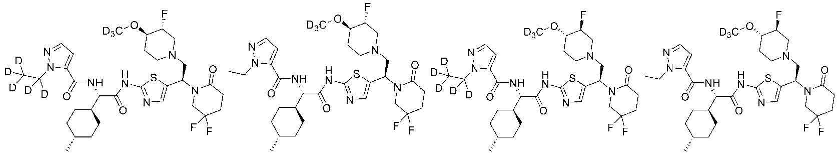

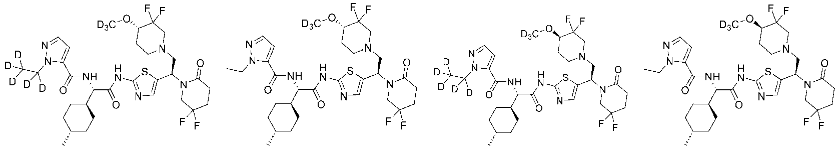

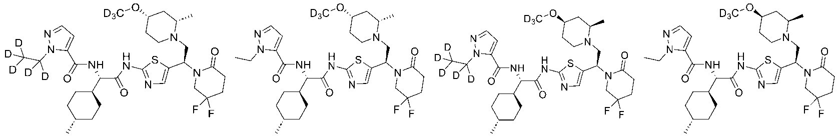

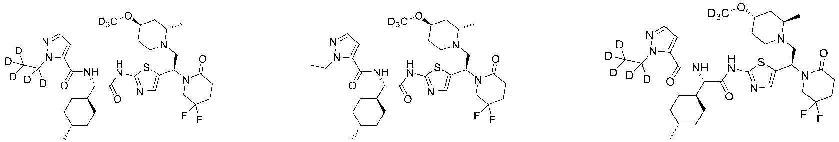

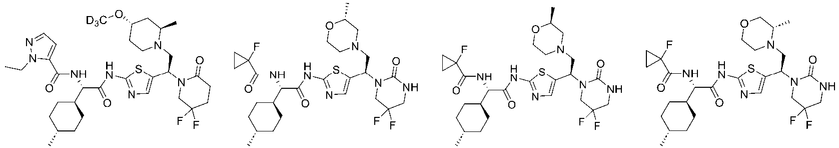

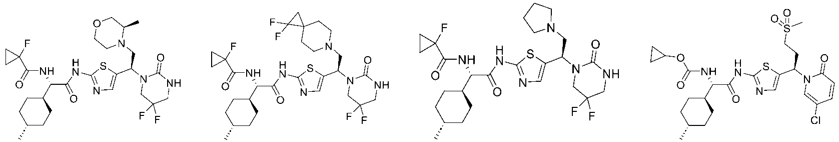

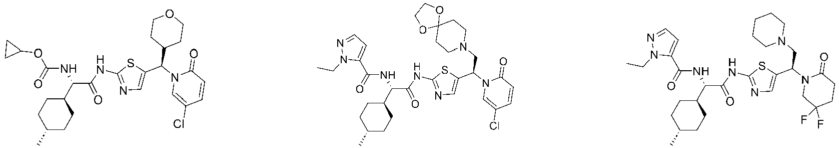

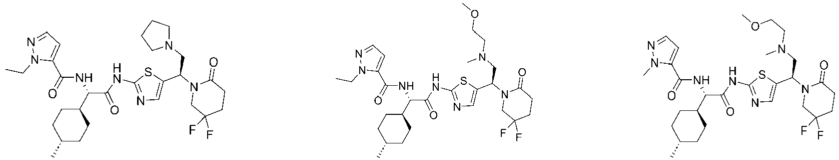

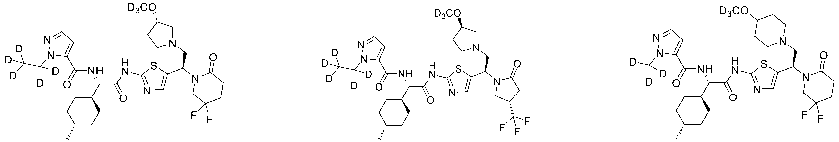

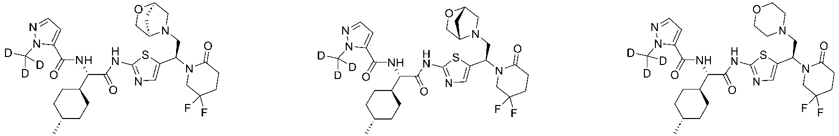

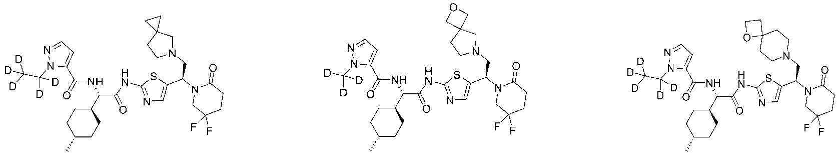

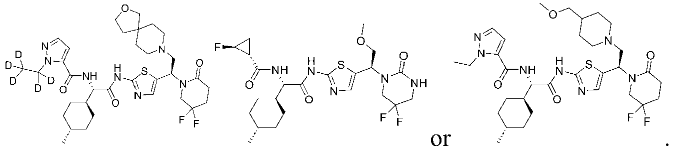

16. A compound as represented by the following general formulas, or a stereoisomer or pharmaceutically acceptable salt thereof, 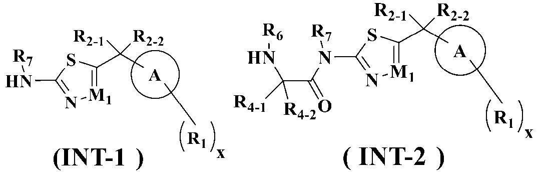 ring A, R₁, R₂₋₁, R₂₋₂, R₄-₁, R₄₋₂, R₆, R₇, x and M₁ are as defined in claim 3.

17. The compound or the stereoisomer or pharmaceutically acceptable salt thereof according to claim 16, **characterised in that** the compound is selected from

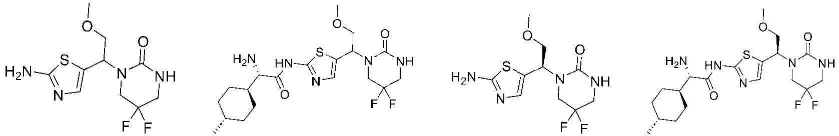

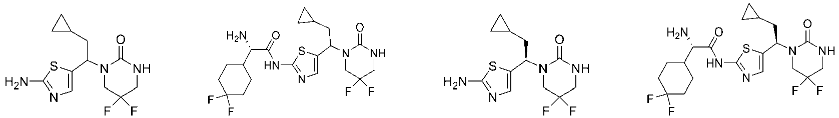

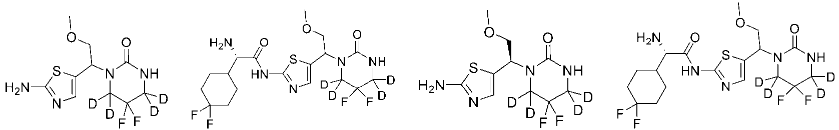

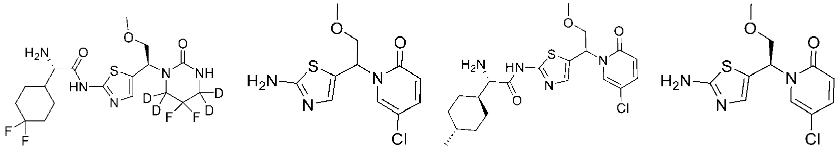

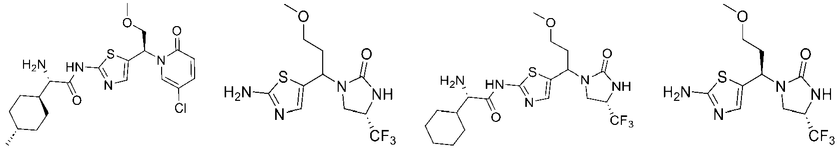

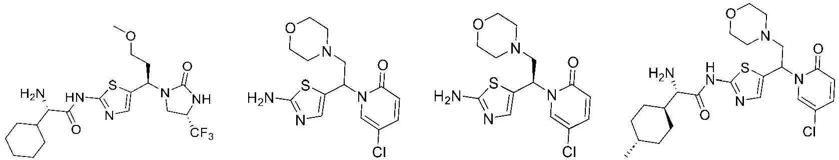

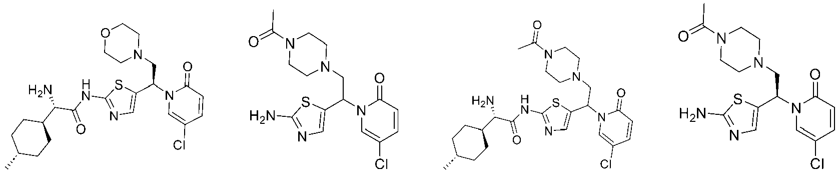

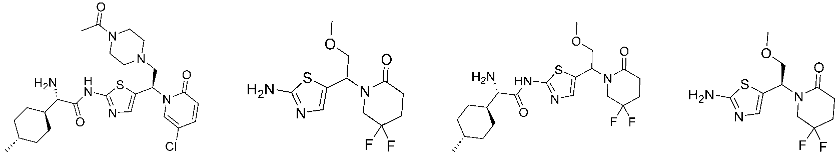

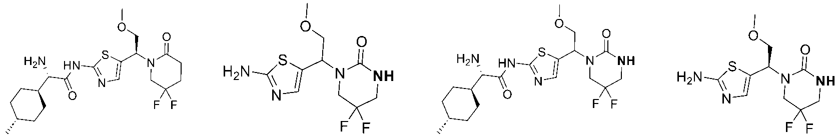

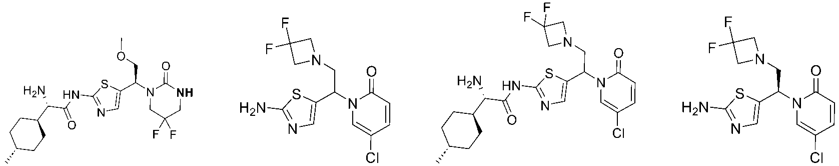

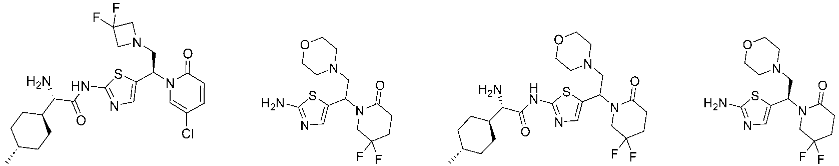

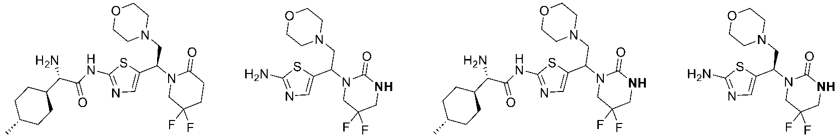

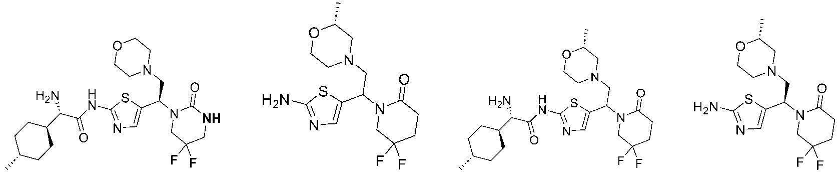

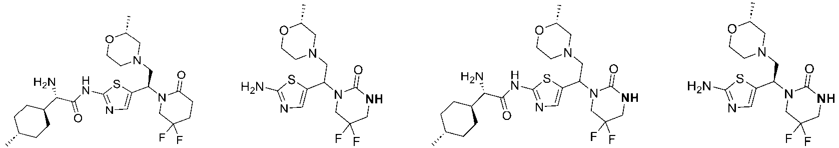

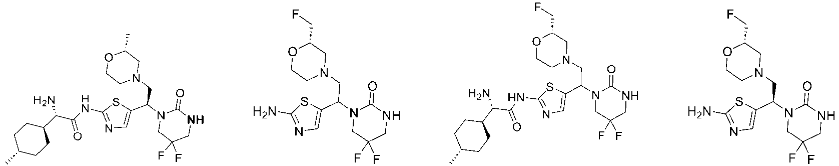

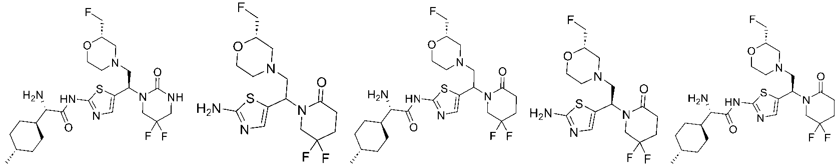

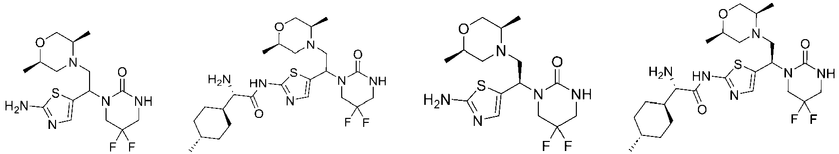

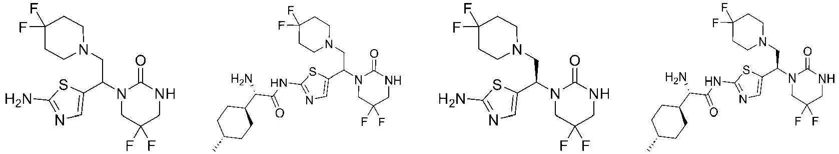

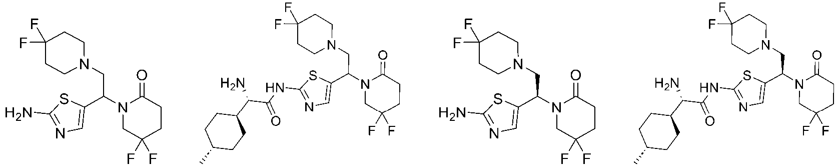

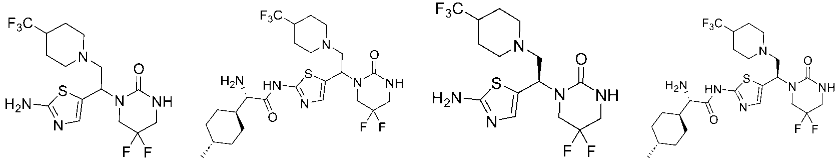

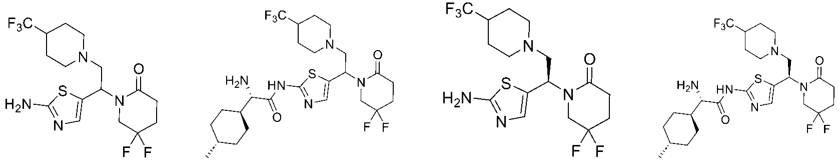

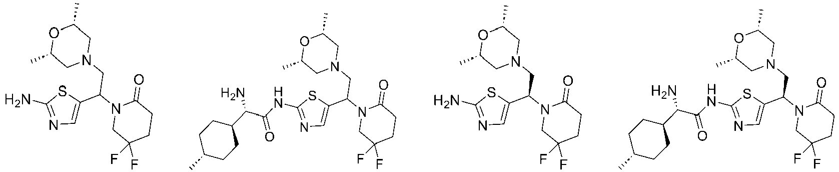

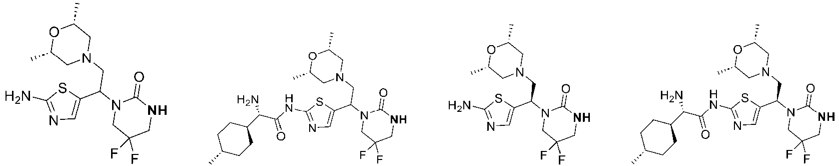

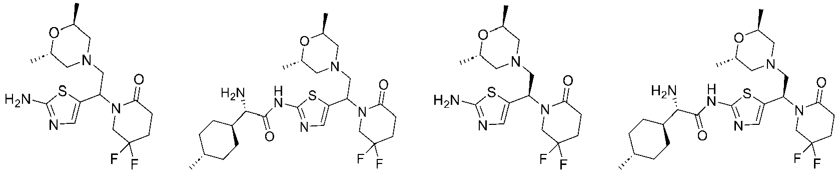

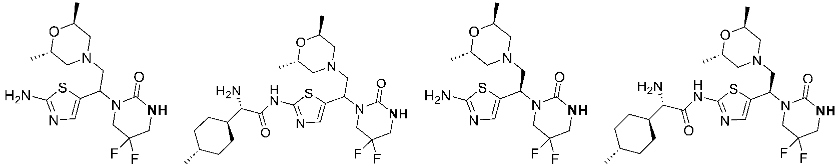

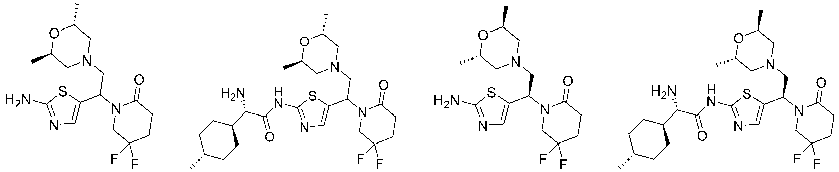

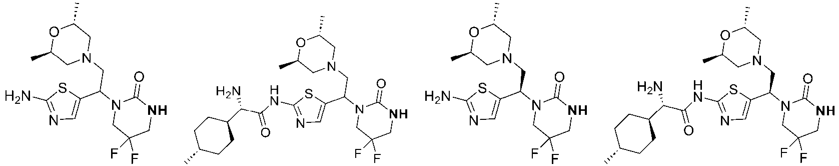

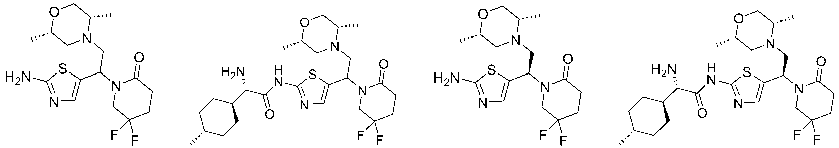

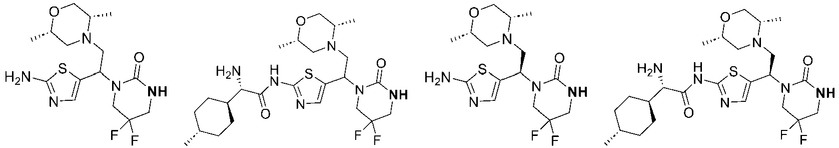

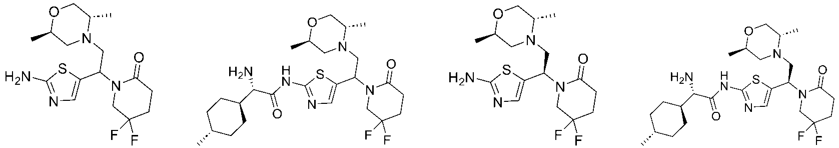

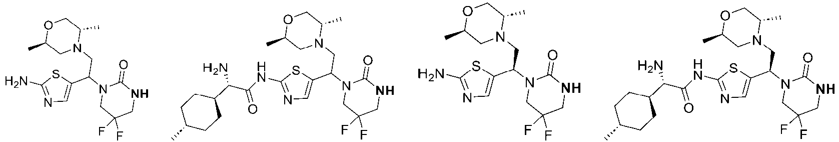

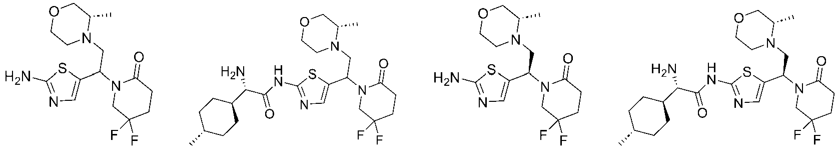

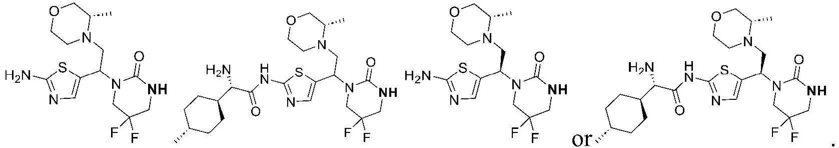

18. A method for preparing the compound as represented by formula (I-A-1), or the stereoisomer or pharmaceutically acceptable salt thereof, **characterised in that** the method comprises reacting a compound as represented by formula (INT-3) with a compound as represented by formula (INT-2) to obtain the compound as represented by formula (I-A-1); 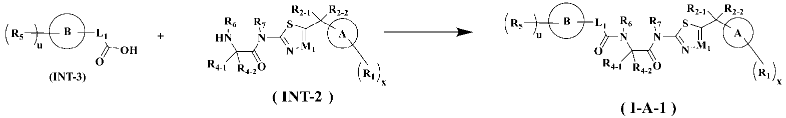
preferably, the reaction is carried out in the presence of a base and a catalyst; the base is an organic base, and the catalyst is an amide condensing agent;
ring A, R₁, R₂₋₁, R₂₋₂, R₄-₁, R₄₋₂, R₆, R₇, x and M₁ are as defined in claim 3.

19. A pharmaceutical composition, **characterised by** comprising a therapeutically effective dose of the compound or the stereoisomer or pharmaceutically acceptable salt thereof according to any one of claims 1-15, and one or more pharmaceutically acceptable carriers, diluents or excipients.

20. Use of the compound or the stereoisomer or pharmaceutically acceptable salt thereof according to any one of claims 1-15 or the pharmaceutical composition according to claim 19 in the preparation of an IL-17 inhibitor drug.

21. Use of the compound or the stereoisomer or pharmaceutically acceptable salt thereof according to any one of claims 1-15 or the pharmaceutical composition according to claim 19 in the preparation of a medicament for treating an autoimmune disease, **characterised in that** the autoimmune disease is preferably selected from psoriasis, plaque psoriasis, guttate psoriasis, inverse psoriasis, pustular psoriasis, erythroderma psoriaticum, psoriatic arthritis, ankylosing spondylitis, hidradenitis suppurativa, rheumatoid arthritis, palmoplantar psoriasis, spondyloarthritis and non-infectious uveitis.
